(19) Europäisches Patentamt
European Patent Office
Office européen des brevets

(11) EP 4 711 371 A1

(12) **EUROPEAN PATENT APPLICATION**
published in accordance with Art. 153(4) EPC

(43) Date of publication:
**18.03.2026 Bulletin 2026/12**

(21) Application number: **24803032.2**

(22) Date of filing: **08.05.2024**

(51) International Patent Classification (IPC):
***C07D 491/22*** $^{(2006.01)}$ ***A61K 31/4745*** $^{(2006.01)}$
***A61P 35/00*** $^{(2006.01)}$

(52) Cooperative Patent Classification (CPC):
**C07D 491/22;** A61K 31/4745; A61P 35/00

(86) International application number:
**PCT/CN2024/091808**

(87) International publication number:
**WO 2024/230752 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.05.2023 CN 202310507097**
**02.11.2023 CN 202311446269**

(71) Applicant: **Gan &Amp Lee Pharmaceuticals Co., Ltd.**
**Beijing 101109 (CN)**

(72) Inventors:
- **SUN, Yingjie**
  **Beijing 101109 (CN)**
- **CUI, Menghan**
  **Beijing 101109 (CN)**
- **JIAO, Jiao**
  **Beijing 101109 (CN)**
- **WANG, Jiaxing**
  **Beijing 101109 (CN)**
- **CHE, Liming**
  **Beijing 101109 (CN)**
- **ZHANG, Guofu**
  **Beijing 101109 (CN)**
- **CHEN, Wen**
  **Beijing 101109 (CN)**

(74) Representative: **Kraus & Lederer PartGmbB**
**Thomas-Wimmer-Ring 15**
**80539 München (DE)**

Remarks:
The complete document including Reference Table(s) and the Sequence Listing(s) can be downloaded from the EPO website

# (54) CAMPTOTHECIN DERIVATIVE, LINKER, LIGAND-DRUG CONJUGATE AND MEDICAL USE THEREOF

(57) Disclosed in the present invention are a new camptothecin derivative, a linker, a ligand-drug conjugate, and a pharmaceutical composition thereof containing the camptothecin derivative or the conjugate, and the corresponding medical use.

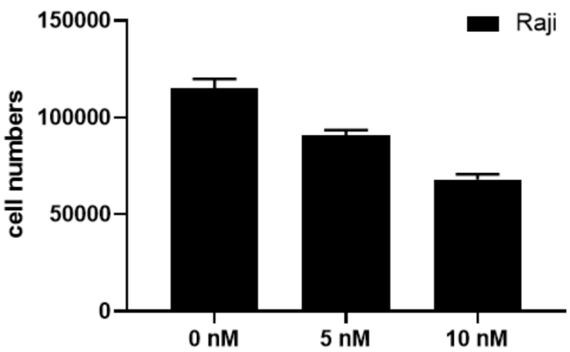


Figure 1

EP 4 711 371 A1

## Description

### Technical Field

The present invention relates to the field of pharmaceutical technology, specifically to camptothecin derivatives, linkers, ligand-drug conjugates, and their pharmaceutical uses in the treatment or prevention of proliferative diseases.

### Background Art

Camptothecin is a water-insoluble cytotoxic alkaloid derived from the Chinese Camptotheca acuminata and the Indian Nothapodytes foetida. Camptothecin and its related analogues are known as potential anticancer agents and have demonstrated therapeutic activity both *in vivo* and *in vitro* studies.

Camptothecin and its analogues are known inhibitors of DNA topoisomerase I. For example, exatecan, a camptothecin analogue, is also a DNA topoisomerase I inhibitor and exhibits potent anticancer activity.

Exatecan, a small-molecule antitumor compound, is a camptothecin derivative known to exert antitumor effects by inhibiting DNA topoisomerase I, developed by Daiichi Sankyo, it advanced to Phase III clinical trials as a single-agent chemotherapy drug, primarily treating bone cancer, prostate cancer, breast cancer, pancreatic cancer, and others. Different with the currently clinically used irinotecan, exatecan does not require enzymatic activation. Furthermore, compared to the active metabolite of Irinotecan-SN-38, and another agent in clinical use-Topotecan, exatecan exhibits stronger topoisomerase I inhibitory activity and demonstrates superior cytotoxic effects against multiple cancer cell lines *in vitro.* Exatecan has not yet been successfully commercialized as a standalone chemotherapy agent, likely due to its high cellular toxicity, which results in the narrow therapeutic window.

Ligand-drug conjugates (ADC), as a novel targeted therapeutics, typically comprises three components: an antibody or antibody-like ligand, a small-molecule drug, and a linker that conjugates the ligand to the drug. ADCs leverage the antibody's specific recognition of antigens to transport the drug molecule to the vicinity of target cells, where it is effectively released to achieve therapeutic effects. In 2000, Pfizer's ADC drug Mylotarg was launched first, bringing this promising and challenging field into the spotlight. Recent years, the pharmaceutical market has witnessed a new wave of enthusiasm for ADC development, with 13 ADC drugs currently approved globally.

The antibody-drug conjugate DS-8201a (brand name: Enhertu), jointly developed and commercialized by Daiichi Sankyo and AstraZeneca, was launched in December 2019. This drug forms an amide derivative by combining idecitabine with hydroxyacetic acid, which is then linked to produce the ADC. As a new-generation antibody-drug conjugate, Enhertu has demonstrated the potential to become a blockbuster drug.

It is highly necessary and urgent to explore and discover camptothecin or idecitabine derivatives with superior antitumor activity, and to enhance the safety and efficacy of small-molecule anticancer compounds in the application of ADC Drugs, thereby obtaining antitumor drugs with superior therapeutic effects.

### SUMMARY

The first aspect of the present invention provides a compound, the compound represented by Formula 1, or a pharmaceutically acceptable salt thereof,

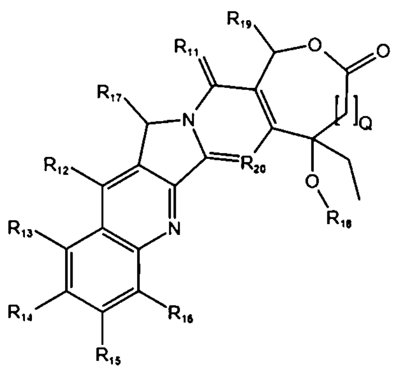

Formula 1

wherein,

each occurrence of $R_{16}$, $R_{17}$, and $R_{19}$ is each independently selected from hydrogen, halogen, $-NO_2$, -CN, -OR, -SR,

$-N(R_a)(R_b)$, $-C(O)R$, $-CO_2R$, $-C(O)C(O)R$, - $C(O)CH_2C(O)R$, $-S(O)R$, $-S(O)_2R$, $-C(O)N(R_a)(R_b)$, $-SO_2N(R_a)(R_b)$, $-OC(O)R$, - $N(R)SO_2R$, and $C_{1-6}$ alkyl, $C_{2-6}$ alkenyl, or $C_{2-6}$ alkynyl, each optionally substituted with R; preferably, each occurrence of $R_{16}$, $R_{17}$, and $R_{19}$ is independently selected from hydrogen and $C_{1-6}$ alkyl optionally substituted with R; more preferably, each occurrence of $R_{16}$, $R_{17}$, and $R_{19}$ is independently selected from hydrogen and $C_{1-6}$ alkyl; more preferably, each occurrence of $R_{16}$, $R_{17}$, and $R_{19}$ is independently hydrogen;

$R_{20}$ is selected from N and $CR_{20a}$;

$R_{20a}$ is selected from hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, $-C(O)CH_2OH$, - S(O)H, $-S(O)_2H$, $-C(O)NH_2$, $-SO_2NH_2$, -OC(O)H, $-N(H)SO_2H$, and $C_{1-6}$ alkyl; preferably, $R_{20a}$ is hydrogen or $C_{1-6}$ alkyl;

$R_{11}$ is selected from O and S;

Q is selected from 0, 1, 2, 3, and 4;

$R_{18}$ is selected from H and $-B-R_3$;

when Q is 0, and $R_{18}$ is $-B-R_3$, wherein $R_{14}$ and $R_{15}$ are each independently selected from hydrogen, halogen, $C_{1-8}$ alkyl, and $C_{1-8}$ haloalkyl; preferably, $R_{14}$ and $R_{15}$ are each independently selected from hydrogen, F, Cl, Br, I, $C_{1-3}$ alkyl, and $C_{1-3}$ haloalkyl; preferably, $R_{14}$ and $R_{15}$ are each independently $-CH_3$ or F; preferably, $R_{14}$ is $-CH_3$, and/or $R_{15}$ is -F;

B is selected from -C(O)- and -P(O)(OH)-; preferably, B is -C(O)-;

$R_3$ is selected from $-(CH_2CH_2O)_aC_{1-6}$ alkyl,

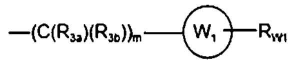

,

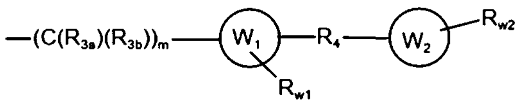

,

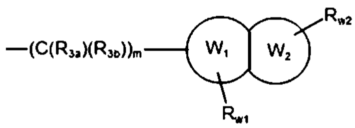

,

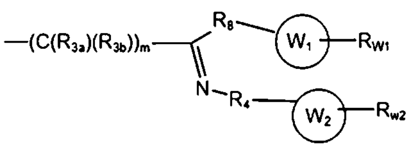

,

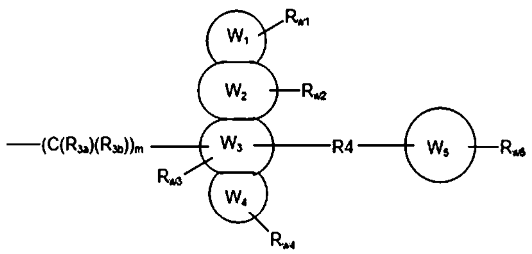

,

and

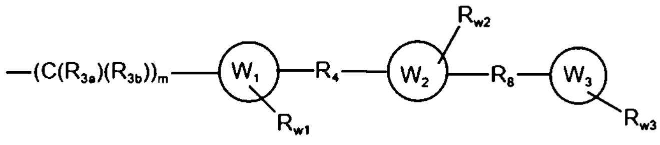

,

wherein when $-(C(R_{3a})(R_{3b}))_m$ - contains methylene units, n methylene units of the $-(C(R_{3a})(R_{3b}))_m-$ are each independently replaced by $-N(R_5)C(O)-$, -C(O)-, -OC(O)-, $-NR_5-$, -O-, -S-, -SO-, $-SO_2-$, $-P(R_5)-$, $-P(=O)(R_5)-$, $-N(R_5)SO_2-$, -C(=S)-, $-C(=NR_5)-$, -N=N-, or -N=CH-;

a is an integer greater than or equal to 1; preferably, a is an integer from 1 to 20; more preferably, a is selected from 1, 2, 3, 4, 5, 6, 7, and 8;

each occurrence of $R_4$ and $R_8$ is each independently a single bond or selected from -O-, $C_{1-8}$ alkylene, $C_{1-8}$ haloalkylene, $-N(R_5)C(O)-$, -C(O)-, -OC(O)-, $-NR_5-$, -S-, -SO-, $-SO_2-$, $-P(R_5)-$, $-P(=O)(R_5)-$, $-N(R_5)SO_2-$, -C(=S)-, $-C(=NR_5)-$, -N=N-, and -N= $CR_5-$;

each occurrence of ring $W_1$, ring $W_2$, ring $W_3$, ring $W_4$, and ring $W_5$ is each independently selected from 6- to 10-membered aryl or arylene, 5- to 10-membered heteroaryl or heteroarylene, 3- to 10-membered cycloalkyl or cycloalkylene, and 3- to 10-membered heterocyclyl or heterocyclylene; preferably, the heteroaryl, heteroarylene, heterocyclylene, and heterocyclyl each independently contain 1, 2, 3, or 4 heteroatoms independently selected from N, O, P, and S; and when $R_3$ is

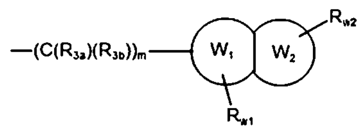

,

$W_1$ and $W_2$ are fused together; when $R_3$ is

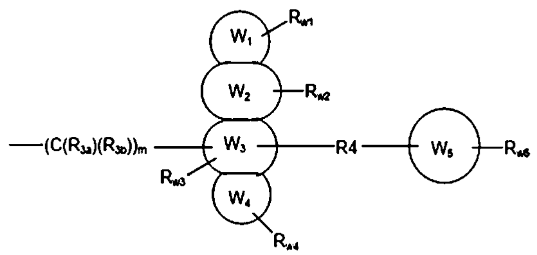

,

$W_1$ and $W_2$ are fused together, $W_2$ and $W_3$ are fused together, and $W_3$ and $W_4$ are fused together;
each occurrence of ring $W_1$, ring $W_2$, ring $W_3$, ring $W_4$, and ring $W_5$ is independently selected from 3- to 10-membered cycloalkyl, cycloalkylene, heterocyclylene, and heterocyclyl, wherein the 3- to 10-membered cycloalkyl, cycloalkylene, heterocyclylene, and heterocyclyl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is independently selected from hydrogen, protium, deuterium, tritium, halogen, =O, =S, $-NO_2$, -CN, -OR, -SR, $-N(R_a)(R_b)$, -C(O)R, - $CO_2R$, -C(O)C(O) R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, and $C_{1-6}$ alkyl optionally substituted with R; preferably, each occurrence of $R_{w1}$, $R_{w2}$, $R_{w3}$, $R_{w4}$, and $R_{w5}$ is independently selected from hydrogen, deuterium, F, Cl, Br, I, hydroxyl, =O, =S, $-NH_2$, $-NO_2$, -CN, -COOH, $-OC_{1-3}$ alkyl, $-C_{1-3}$ alkyl, and $C_{1-3}$ alkylene-hydroxyl;

When ring $W_1$, ring $W_2$, ring $W_3$, ring $W_4$, and ring $W_5$ is independently selected from 6- to 10-membered aryl, 6- to 10-membered arylene, 5- to 10-membered heteroarylene, and 5- to 10-membered heteroaryl, the aryl, arylene, heteroarylene, and heteroaryl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is independently selected from hydrogen, protium, deuterium, tritium, halogen, $-NO_2$, - CN, -OR, -SR, $-N(R_a)(R_b)$, -C(O)R, $-CO_2R$, -C(O)C(O)R, $-C(O)CH_2C(O)R$, -S(O)R, - $S(O)_2R$, $-C(O)N(R_a)(R_b)$, $-SO_2N(R_a)(R_b)$, -OC(O)R, -N(R)SO2R, and $C_{1-6}$ alkyl optionally substituted with R; preferably, each occurrence of $R_{w1}$, $R_{w2}$, $R_{w3}$, $R_{w4}$, and $R_{w5}$ is independently selected from hydrogen, deuterium, F, Cl, Br, I, hydroxyl, $-NH_2$, - $NO_2$, -CN, $-OC_{1-3}$ alkyl, -COOH, and $C_{1-3}$ alkyl;

$R_{13}$ is selected from hydrogen, halogen, $C_{1-8}$ alkyl, and $C_{1-8}$ haloalkyl; preferably, $R_{13}$ is hydrogen; $R_{12}$ is selected from $C_{1-8}$ alkylene-$N(R_a)(R_b)$, $C_{1-8}$ haloalkylene-$N(R_a)(R_b)$, halogen, -NO2, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, and $C_{1-6}$ alkyl optionally substituted with R; preferably, $R_{12}$ is selected from $C_{1-3}$ alkylene-$N(R_a)(R_b)$ and $C_{1-3}$ haloalkylene-$N(R_a)(R_b)$; preferably, $R_{12}$ is selected from $-CH_2N(R_a)(R_b)$; or $R_{13}$ and $R_{12}$ together with the carbon atoms to which they are attached form a saturated or unsaturated 5- to 10-membered cycloalkyl, 5- to 10-membered heterocyclyl containing 1, 2, or 3 heteroatoms independently selected from N, O, P, and S, wherein the cycloalkyl or heterocyclyl is substituted with one or more substituents independently selected from halogen, $-NO_2$, -CN, -OR, -SR, $-N(R_a)(R_b)$, - C(O)R, $-CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, and $C_{1-6}$ alkyl optionally substituted with R; preferably, $R_{13}$ and $R_{12}$ together with the carbon atoms to which they are attached form a 6-membered cycloalkyl, the cycloalkyl is substituted with $-N(R_a)(R_b)$;
each occurrence of $R_{3a}$, $R_{3b}$, and $R_5$ is each independently hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ alkyl optionally substituted with R; and
m and n are each independently an integer greater than or equal to 1, and $m \geq n$; preferably, m and n are each independently 1, 2, 3, 4, 5, 6, 7, 8, 9, or 10, and $m \geq n$; preferably, m is 1, 2, 3, 4, 5, 6, 7, 8, 9, or 10, -n is 0, 1, 2, 3, 4, or 5, and $m \geq n$;
when Q is 1,2,3, or 4, and $R_{18}$ is H, $R_{12}$, $R_{13}$, and $R_{14}$ are each independently selected from hydrogen, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, and $C_{1-6}$ alkyl optionally substituted with R; preferably, $R_{12}$, $R_{13}$, and $R_{14}$ are each independently selected from hydrogen, F, Cl, Br, I, $C_{1-3}$ alkyl, $-NO_2$, - CN, and $-O-C_{1-3}$ alkyl; or $R_{12}$ and $R_{13}$

together with the carbon atoms to which they are attached form a saturated or unsaturated 5- to 10-membered cycloalkyl, or a 5- to 10-membered heterocyclyl containing 1, 2, or 3 heteroatoms independently selected from N, O, and S, the cycloalkyl or heterocyclyl is unsubstituted or substituted with one or more substituents independently selected from halogen, $-NO_2$, -CN, -OR, -SR, - $N(R_a)(R_b)$, -C(O)R, $-CO_2R$, -C(O)C(O)R, $-C(O)CH_2C(O)R$, -S(O)R, $-S(O)_2R$, - $C(O)N(R_a)(R_b)$, $-SO_2N(R_a)(R_b)$, -OC(O)R, $-N(R)SO_2R$, and $C_{1-6}$ alkyl optionally substituted with R; preferably, $R_{12}$ and $R_{13}$ together with the carbon atoms to which they are attached form a saturated or unsaturated 5- to 6-membered heterocyclyl containing two oxygen atoms;

$R_{15}$ is $C_{1-8}$ alkylene-hydroxyl, $C_{1-8}$ alkylene-amino, $-C(R_a)(R_b)-N(R_a)-R_{4c}$, - $C(R_a)(R_b)-N(R_a)-C(O)-R_{4c}$, or $-C(R_a)(R_b)-N(R_a)-C(O)O-R_{4c}$; preferably, $R_{15}$ is $-CH_2-NH_2$, $-CH_2-NH-C(O)-R_{4c}$, $-CH_2-NH-C(O)O-R_{4c}$, or $C_4H_8OH$; preferably, $R_{15}$ is $-CH_2-NH_2$, or $-(CH_2)_4-OH$; and

each occurrence of R, $R_a$, $R_b$, and $R_{4c}$ is independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, $-C(O)CH_2OH$, -S(O)H, $-S(O)_2H$, $-C(O)NH_2$, $-SO_2NH_2$, -OC(O)H, $-N(H)SO_2H$, $C_{1-6}$ alkyl, or $C_{1-6}$ alkylene-hydroxyl; preferably, each occurrence of R, $R_a$, $R_b$, and $R_{4c}$ is independently hydrogen, F, Cl, Br, I, $CH_2OH$, or $C_2H_4OH$;

when Q is 0, $R_{11}$ is S, and $R_{18}$ is H, $R_{12}$ is selected from hydrogen and - $CH_2N(R_{3A})(R_{4A})$;

$R_{14}$ and $R_{15}$ are each independently selected from hydrogen, halogen, $-NO_2$, -CN, -OR', $-N(R_{a1})(R_{b1})$, -C(O)R', $-CO_2R'$, -C(O)C(O)R', $-C(O)CH_2C(O)R'$, - $C(O)N(R_{a1})(R_{b1})$, $-SO_2N(R_{a1})(R_{b1})$, -OC(O)R', $-N(R')SO_2R'$, and $C_{1-6}$ alkyl; preferably, $R_{14}$ and $R_{15}$ are each independently selected from halogen, amino, -OR', and $C_{1-6}$ alkyl; more preferably, $R_{14}$ and $R_{15}$ are each independently selected from fluorine, chlorine, amino, $-O-C_{1-6}$ alkyl, and $C_{1-6}$ alkyl; or $R_{14}$ and $R_{15}$ together form a saturated or unsaturated 4- to 10-membered cycloalkyl, or a 5- or 6-membered heterocyclyl containing 1 to 3 heteroatoms independently selected from N, O, and S; wherein the cycloalkyl or heterocyclyl is unsubstituted or substituted with one or more substituents independently selected from halogen, $-NO_2$, -CN, -OR, -SR, $-N(R_a)(R_b)$, -C(O)R, $-CO_2R$, -C(O)C(O)R, - $C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, or $C_{1-6}$ alkyl, $C_{2-6}$ alkenyl, and $C_{2-6}$ alkynyl, each optionally substituted with R; preferably, the cycloalkyl or heterocyclyl is unsubstituted;

$R_{13}$ is hydrogen, $-C_{1-6}$ alkyl, or $-C_{1-6}$ alkylene-hydroxyl; preferably, $R_{5A}$ is hydrogen, $C_{1-6}$ alkyl, or $-(CH_2)_4$-hydroxyl; each occurrence of $R_{3A}$ and $R_{4A}$ is independently selected from hydrogen, halogen, -OR', $-N(R_{a1})(R_{b1})$, -C(O)R', -C(S)R', $-C(S)C(R_{a1})(R_{b1})OH$, $-C(O)CH_2N(R_{a1})(R_{b1})$, - $CO_2R'$, -C(O)C(O)R', $-C(O)CH_2C(O)R'$, $-C(O)C(R_{a1})(R_{b1})OH$, $-C(O)-C_{3-6}$ cycloalkylene-OH, $-C(O)-C_{1-6}$ alkylene-OH, $-C_{1-6}$ alkylene -OH $-C(O)-C_{3-6}$ cycloalkylene-$C_{1-6}$ alkylene-OH, $-C(O)O-C_{1-6}$ alkylene-OH, -S(O)R', $-S(O)_2R$, - $C(O)N(R_{a1})(R_{b1})$, $-SO_2N(R_{a1})(R_{b1})$, -OC(O)R', $-N(R')SO_2R'$, and $C_{1-6}$ alkyl, $C_{1-6}$ alkenyl, and $C_{1-6}$ alkynyl, each optionally substituted with R'; preferably, $R_{4A}$ is selected from hydrogen or $C_{1-6}$ alkyl;

wherein each occurrence of R', $R_{a1}$, and $R_{b1}$ is independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, -S(O)H, $-S(O)_2H$, $-C(O)NH_2$, $-SO_2NH_2$, -OC(O)H, - $N(H)SO_2H$, $C_{1-6}$ alkyl, $C_{2-6}$ alkenyl, or $C_{2-6}$ alkynyl, 3-, 4-, 5-, 6-, 7-, or 8-membered cycloalkyl, 3- to 8-membered heterocyclyl containing 1, 2, or 3 heteroatoms independently selected from N, O, and S, 6- to 10-membered aryl, or 5- to 10-membered heteroaryl containing 1, 2, or 3 heteroatoms independently selected from N, O, and S; the $C_{1-6}$ alkyl, $C_{2-6}$ alkenyl, $C_{2-6}$ alkynyl, 3-, 4-, 5-, 6-, 7-, 8-membered cycloalkyl, 3- to 8-membered heterocyclyl, 6- to 10-membered aryl, or 5- to 10-membered heteroaryl is unsubstituted or substituted with one or more substituents independently selected from halogen, $-NO_2$, -CN, -OH, $-NH_2$, -C(O)H, $-CO_2H$, -C(O)C(O)H, $-C(O)CH_2C(O)H$, - S(O)H, $-S(O)_2H$, $-C(O)NH_2$, $-SO_2NH_2$, -OC(O)H, $-N(H)SO_2H$, $C_{1-6}$ alkyl, $C_{2-6}$ alkenyl, $C_{1-6}$ alkynyl, and hydroxyl-$C_{1-6}$ alkylene;

when $R_{14}$ is $-CH_3$, $R_{15}$ is fluorine, and $R_{13}$ is hydrogen, $R_{12}$ is

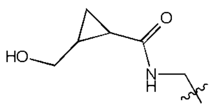,

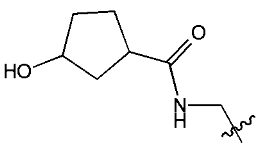, 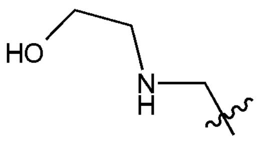; 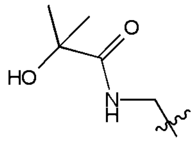, 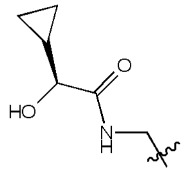,

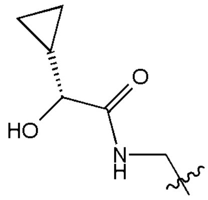, 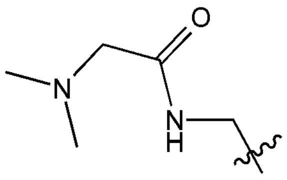, 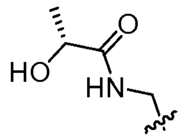, 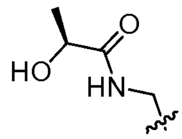,

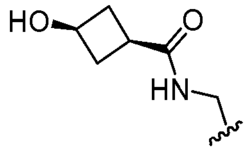 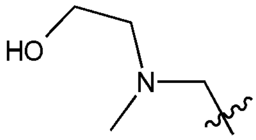; 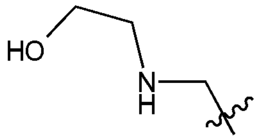, 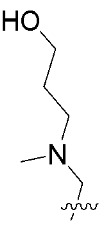, 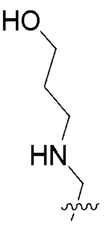,

or

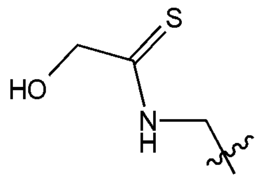,

and
when $R_{14}$ is -O-$CH_3$, $R_{15}$ is fluorine, and $R_{13}$ is hydrogen, $R_{12}$ is not

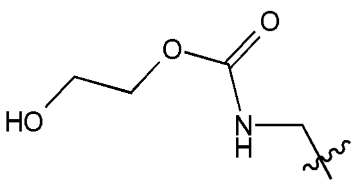, 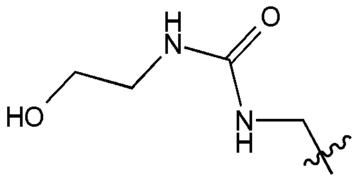, or 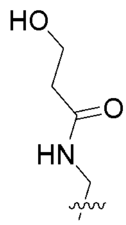;

and
when $R_{14}$ and $R_{15}$ form a 1,4-dioxane, and $R_{13}$ is hydrogen, $R_{12}$ is $-CH_2NH_2$, $-CH_2NHC_2H_4OH$, $-CH_2N(CH_3)C_2H_4OH$, $-CH_2NHC_3H_6OH$,

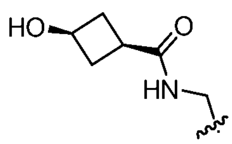,

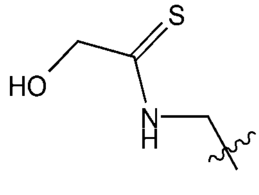, 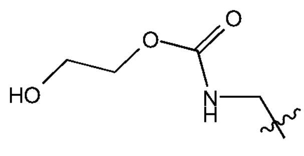, 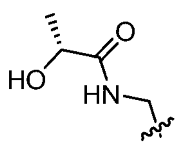, 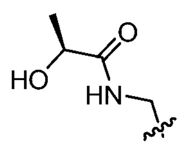,

or

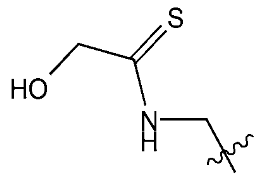

;

and

when $R_{14}$ is fluorine, $R_{15}$ is fluorine, and $R_{13}$ is hydrogen, $R_{12}$ is not hydrogen; and when $R_{14}$ is $-CH_3$, $R_{15}$ is chlorine, and $R_{13}$ is hydrogen, $R_{12}$ is selected from

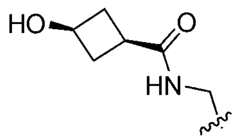

,

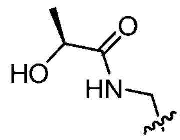

, or

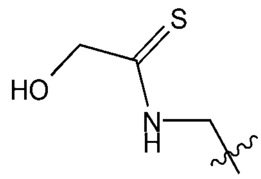

;

and

when $R_{14}$ and $R_{15}$ form a 1,3-dioxolane, and $R_{13}$ is hydrogen, $R_{12}$ is selected from $-CH_2NHC_2H_4OH$, $-CH_2N(CH_3)C_2H_4OH$, $-CH_2NHC_3H_6OH$, or

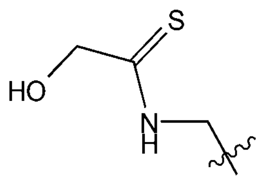

;

and

when $R_{13}$ is selected from $-(CH_2)_4-OH$, $R_{12}$ is hydrogen, $R_{14}$ and $R_{15}$ are fluorine, or $R_{14}$ and $R_{15}$ form a 5-membered or 6-membered heterocyclyl containing two oxygen atoms.

In some embodiments, wherein the structure of Formula 1 is selected from the following structures:

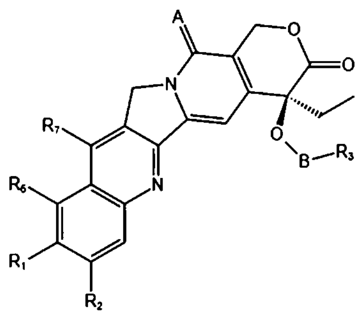

Formula 1A ,

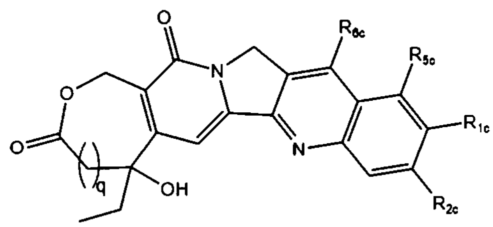

Formula 1B , and

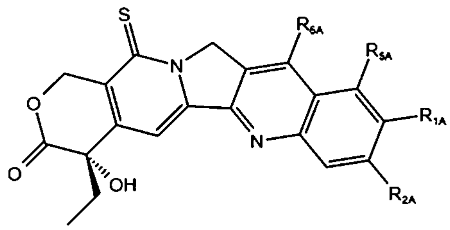

Formula 1C ;

when Formula 1 is Formula 1A:

wherein, $R_1$ and $R_2$ are each independently selected from hydrogen, halogen, $C_{1-8}$ alkyl, and $C_{1-8}$ haloalkyl; preferably, $R_1$ and $R_2$ are each independently selected from hydrogen, F, Cl, Br, I, $C_{1-3}$ alkyl, and $C_{1-3}$ haloalkyl; preferably, $R_1$ and $R_2$ are each independently $-CH_3$, or F; preferably, $R_1$ is $-CH_3$, and/or $R_2$ is F;
A is selected from oxygen and sulfur;
B is selected from -C(O)- and -P(O)(OH)-; preferably, B is -C(O)-;
$R_3$ is selected from $-(CH_2CH_2O)_aC_{1-6}$ alkyl,

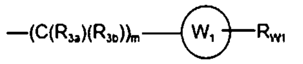
,

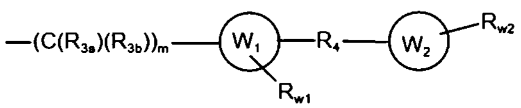
,
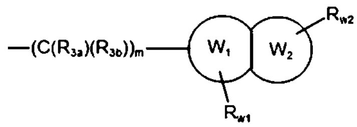
,

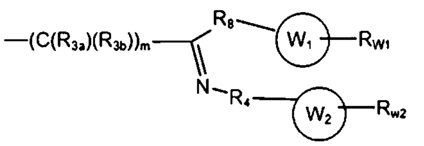
,
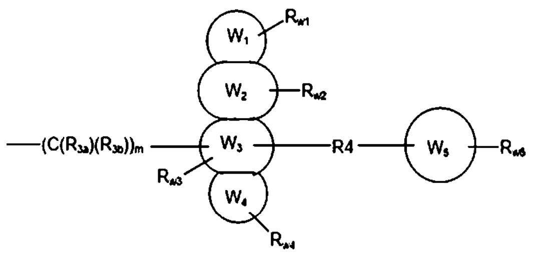
,

and

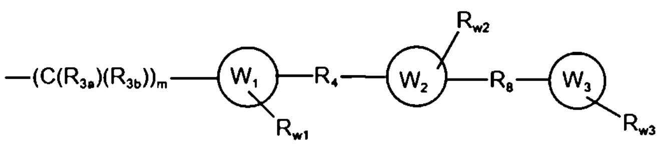
,

wherein when $-(C(R_{3a})(R_{3b}))_m$ - contains methylene units, the n methylene units of the $-(C(R_{3a})(R_{3b}))_m$- are each independently replaced by $-N(R_5)C(O)-$, -C(O)-, -OC(O)-, $-NR_5-$, -O-, -S-, -SO-, $-SO_2-$, $-P(R_5)-$, $-P(=O)(R_5)-$, $-N(R_5)SO_2-$, -C(=S)-, $-C(=NR_5)-$, -N=N-, or -N=CH-;
a is an integer greater than or equal to 1; preferably, a is an integer from 1 to 20; more preferably, a is selected from 1, 2, 3, 4, 5, 6, 7, and 8;
each occurrence of $R_4$ and $R_8$ is independently a single bond or selected from -O-, $C_{1-8}$ alkylene, $C_{1-8}$ haloalkylene, $-N(R_5)C(O)-$, -C(O)-, -OC(O)-, $-NR_5-$, -S-, -SO-, $-SO_2-$, $-P(R_5)-$, $-P(=O)(R_5)-$, $-N(R_5)SO_2-$, -C(=S)-, $-C(=NR_5)-$, -N=N-, and $-N=CR_5-$;
each occurrence of ring $W_1$, ring $W_2$, ring $W_3$, ring $W_4$, and ring $W_5$ is independently selected from 6- to 10-membered aryl or arylene, 5- to 10-membered heteroaryl or heteroarylene, 3- to 10-membered cycloalkyl or cycloalkylene, and 3- to 10-membered heterocyclyl or heterocyclylene; preferably, the heteroaryl, heteroarylene, heterocyclylene, and heterocyclyl each independently contain 1, 2, 3, or 4 heteroatoms independently selected from N, O, P, and S; and

when $R_3$ is

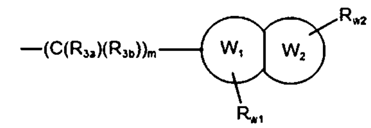

,

$W_1$ and $W_2$ are fused together; when $R_3$ is

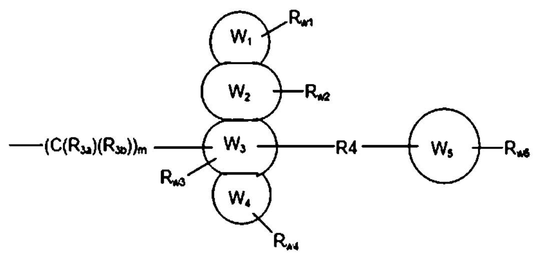

,

$W_1$ and $W_2$ are fused together, $W_2$ and $W_3$ are fused together, and $W_3$ and $W_4$ are fused together;

When each occurrence of ring $W_1$, ring $W_2$, ring $W_3$, ring $W_4$, and ring $W_5$ is independently selected from 3- to 10-membered cycloalkyl, cycloalkylene, heterocyclylene, and heterocyclyl, wherein the 3- to 10-membered cycloalkyl, cycloalkylene, heterocyclylene, and heterocyclyl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is independently selected from hydrogen, protium, deuterium, tritium, halogen, =O, =S, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, and $C_{1-6}$ alkyl optionally substituted with R; preferably, each occurrence of $R_{w1}$, $R_{w2}$, $R_{w3}$, $R_{w4}$, and $R_{w5}$ is independently selected from hydrogen, deuterium, F, Cl, Br, I, hydroxyl, =O, =S, $-NH_2$, $-NO_2$, -CN, -COOH, $-OC_{1-3}$ alkyl, $-C_{1-3}$ alkyl, and $C_{1-3}$ alkylene-hydroxyl;

When each occurrence of ring $W_1$, ring $W_2$, ring $W_3$, ring $W_4$, and ring $W_5$ is independently selected from 6- to 10-membered aryl, 6- to 10-membered arylene, 5- to 10-membered heteroarylene, and 5- to 10-membered heteroaryl, the aryl, arylene, heteroarylene, and heteroaryl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is independently selected from hydrogen, protium, deuterium, tritium, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, and $C_{1-6}$ alkyl optionally substituted with R; preferably, each occurrence of $R_{w1}$, $R_{w2}$, $R_{w3}$, $R_{w4}$, and $R_{w5}$ is independently selected from hydrogen, deuterium, F, Cl, Br, I, hydroxyl, $-NH_2$, $-NO_2$, -CN, $-OC_{1-3}$ alkyl, -COOH, and $C_{1-3}$ alkyl;

$R_6$ is selected from hydrogen, halogen, $C_{1-8}$ alkyl, and $C_{1-8}$ haloalkyl; preferably, $R_6$ is hydrogen; $R_7$ is selected from $C_{1-8}$ alkylene-$N(R_a)(R_b)$, $C_{1-8}$ haloalkylene-$N(R_a)(R_b)$, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, $-O-CH_2C(O)N(R_a)(R_b)$, and $C_{1-6}$ alkyl optionally substituted with R; preferably, $R_7$ is selected from $C_{1-3}$ alkylene-$N(R_a)(R_b)$ and $C_{1-3}$ haloalkylene-$N(R_a)(R_b)$; preferably, $R_7$ is selected from $-CH_2N(R_a)(R_b)$; or $R_6$ and $R_7$ together with the carbon atoms to which they are attached form a saturated or unsaturated 5- to 10-membered cycloalkyl, 5- to 10-membered heterocyclyl containing 1, 2, or 3 heteroatoms independently selected from N, O, P, and S, wherein the cycloalkyl or heterocyclyl is unsubstituted or substituted with one or more substituents independently selected from halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, and $C_{1-6}$ alkyl optionally substituted with R; preferably, $R_6$ and $R_7$ together with the carbon atoms to which they are attached form a saturated or unsaturated 6-membered cycloalkyl, wherein the cycloalkyl is substituted with $-N(R_a)(R_b)$;

each occurrence of $R_{3a}$, $R_{3b}$, and $R_5$ is independently hydrogen, protium, deuterium, tritium, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, or $C_{1-6}$ alkyl optionally substituted with R;

each occurrence of R, $R_a$, and $R_b$ is independently hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, $-C(O)CH_2OH$, -S(O)H, $-S(O)_2H$, $-C(O)NH_2$, $-SO_2NH_2$, -OC(O)H, $-N(H)SO_2H$, $C_{1-6}$ alkylene-hydroxyl, or $C_{1-6}$ alkyl; and

m and n are each independently an integer greater than or equal to 1, and m≥n; preferably, m, and n are each independently 1, 2, 3, 4, 5, 6, 7, 8, 9, or 10, and m≥n; preferably, m is 1, 2, 3, 4, 5, 6, 7, 8, 9, or 10, -n is 0, 1, 2, 3, 4, or 5, and m≥n;

when Formula 1 is Formula 1B:

wherein, q is 1, 2, or 3;

$R_{1c}$, $R_{2c}$, and $R_{5c}$ are each independently selected from hydrogen, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, -S(O)2R, $-C(O)N(R_a)(R_b)$, $-SO_2N(R_a)(R_b)$, -OC(O)R, -N(R)SO2R, and $C_{1-6}$ alkyl optionally substituted with R; preferably, $R_{1c}$, $R_{2c}$, and $R_{5c}$ are each independently selected from hydrogen, F, Cl, Br, I, $C_{1-3}$ alkyl, $-NO_2$, -CN, and $-O-C_{1-3}$ alkyl; or $R_{1c}$ and $R_{2c}$ together with the carbon atoms to which they are attached form a saturated or unsaturated 5- to 10-membered cycloalkyl, or 5- to 10-membered heterocyclyl containing 1, 2, or 3 heteroatoms independently selected from N, O, and S, wherein the cycloalkyl, or heterocyclyl is unsubstituted or substituted with one or more substituents selected from halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, and $C_{1-6}$ alkyl optionally substituted with R; preferably, $R_{1c}$ and $R_{2c}$ together with the carbon atoms to which they are attached form a saturated or unsaturated 5- to 6-membered heterocyclyl containing two oxygen atoms;

$R_{6c}$ is $C_{1-8}$ alkylene-hydroxyl, $C_{1-8}$ alkylene-amino, $-C(R_a)(R_b)-N(R_a)-R_{4c}$, $-C(R_a)(R_b)-N(R_a)-C(O)-R_{4c}$, or $-C(R_a)(R_b)-N(R_a)-C(O)O-R_{4c}$; preferably, $R_{6c}$ is $-CH_2-NH_2$, $-CH_2-NH-C(O)-R_{4c}$, $-CH_2-NH-C(O)O-R_{4c}$, or $C_4H_8OH$; preferably, $R_{6c}$ is $-CH_2-NH_2$, or $-(CH_2)_4-OH$; and

each occurrence of R, $R_a$, $R_b$, and $R_{4c}$ is independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, $-C(O)CH_2OH$, -S(O)H, $-S(O)_2H$, $-C(O)NH_2$, $-SO_2NH_2$, -OC(O)H, $-N(H)SO_2H$, $C_{1-6}$ alkyl, or $C_{1-6}$ alkylene-hydroxyl; preferably, each occurrence of R, $R_a$, $R_b$, and $R_{4c}$ is independently hydrogen, F, Cl, Br, I, $CH_2OH$, or $C_2H_4OH$;

when Formula 1 is Formula 1C:

$R_{6A}$ is selected from hydrogen and $-CH_2N(R_{3A})(R_{4A})$;

$R_{1A}$ and $R_{2A}$ are each independently selected from hydrogen, halogen, $-NO_2$, -CN, -OR', $-N(R_{a1})(R_{b1})$, -C(O)R', $-CO_2R'$, -C(O)C(O)R', $-C(O)CH_2C(O)R'$, - $C(O)N(R_{a1})(R_{b1})$, $-SO_2N(R_{a1})(R_{b1})$, -OC(O)R', $-N(R')SO_2R'$, and $C_{1-6}$ alkyl; preferably, $R_{1A}$ and $R_{2A}$ are each independently selected from halogen, amino, -OR', and $C_{1-6}$ alkyl; more preferably, $R_{1A}$ and $R_{2A}$ are each independently selected from fluorine, chlorine, amino, $-O-C_{1-6}$ alkyl, and $C_{1-6}$ alkyl;

or $R_{1A}$ and $R_{2A}$ together form a saturated or unsaturated 4- to 10- membered cycloalkyl, or 5-, or 6-membered heterocyclyl containing 1 to 3 heteroatoms independently selected from N, O, and S, wherein the cycloalkyl, or heterocyclyl is unsubstituted or substituted with one or more substituents selected from halogen, $-NO_2$, -CN, -OR, -SR, $-N(R_{a1})(R_{b1})$, -C(O)R, $-CO_2R$, -C(O)C(O)R, $-C(O)CH_2C(O)R$, -S(O)R, -S(O)2R, $-C(O)N(R_{a1})(R_{b1})$, -SOZN$(R_{a1})(R_{b1})$, -OC(O)R, -N(R)SO2R, or $C_{1-6}$ alkyl, $C_{1-6}$ alkenyl, and $C_{1-6}$ alkynyl optionally substituted with R; preferably, the cycloalkyl or heterocyclyl is unsubstituted;

$R_{5A}$ is hydrogen, or $C_{1-6}$ alkyl;

each occurrence of $R_{3A}$ and $R_{4A}$ is independently selected from hydrogen, halogen, -OR', $-N(R_{a1})(R_{b1})$, -C(O)R', -C(S)R', $-C(S)C(R_{a1})(R_{b1})OH$, $-C(O)CH_2N(R_{a1})(R_{b1})$, - $CO_2R'$, -C(O)C(O)R', $-C(O)CH_2C(O)R'$, $-C(O)C(R_{a1})(R_{b1})OH$, $-C(O)-C_{3-6}$ cycloalkylene-OH, $-C(O)-C_{1-6}$ alkylene-OH, $-C(O)-C_{3-6}$ cycloalkylene-$C_{1-6}$ alkylene-OH, $-C(O)O-C_{1-6}$ alkylene-OH, -S(O)R', $-S(O)_2R$, $-C(O)N(R_{a1})(R_{b1})$, $-SO_2N(R_{a1})(R_{b1})$, -OC(O)R', $-N(R')SO_2R'$, and $C_{1-6}$ alkyl , $C_{1-6}$ alkenyl, and $C_{1-6}$ alkynyl optionally substituted with R',; preferably, $R_{4A}$ is selected from hydrogen, or $C_{1-6}$ alkyl;

wherein each occurrence of R', $R_{a1}$, and $R_{b1}$ is independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, -S(O)H, $-S(O)_2H$, $-C(O)NH_2$, $-SO_2NH_2$, -OC(O)H, - $N(H)SO_2H$, $C_{1-6}$ alkyl, $C_{2-6}$ alkenyl, or $C_{2-6}$ alkynyl, 3, 4, 5, 6, 7, or 8-membered cycloalkyl, 3- to 8-membered heterocyclyl containing 1, 2, or 3 heteroatoms independently selected from N, O, and S, 6- to 10-membered aryl, or 5- to 10-membered heteroaryl containing 1, 2, or 3 heteroatoms independently selected from N, O, and S; the $C_{1-6}$ alkyl, $C_{2-6}$ alkenyl, $C_{2-6}$ alkynyl, 3-, 4-, 5-, 6-, 7-, 8-membered cycloalkyl, 3- to 8-membered heterocyclyl, 6- to 10-membered aryl, or 5- to 10-membered heteroaryl is unsubstituted or substituted with one or more substituents selected from halogen, $-NO_2$, -CN, -OH, $-NH_2$, -C(O)H, $-CO_2H$, -C(O)C(O)H, $-C(O)CH_2C(O)H$, -S(O)H, $-S(O)_2H$, - $C(O)NH_2$, $-SO_2NH_2$, -OC(O)H, $-N(H)SO_2H$, $C_{1-6}$ alkyl, $C_{2-6}$ alkenyl, or $C_{1-6}$ alkynyl, and hydroxyl-$C_{1-6}$ alkylene;

wherein, when $R_{1A}$ is $-CH_3$, $R_{2A}$ is fluorine, and $R_{5A}$ is hydrogen, $R_{6A}$ is

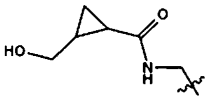, 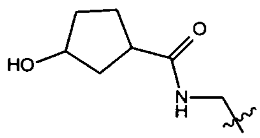, 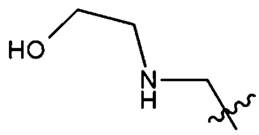; 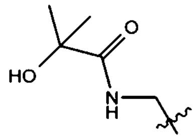,

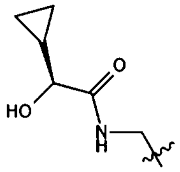, 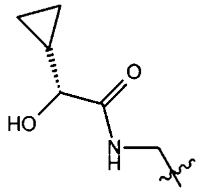, 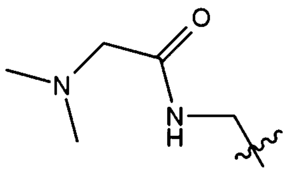, 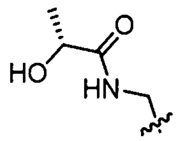,

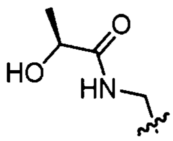 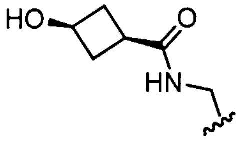 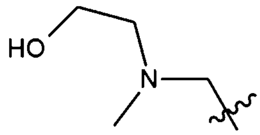; 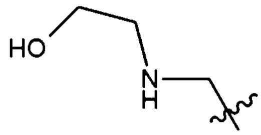,

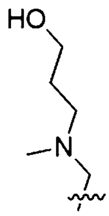, 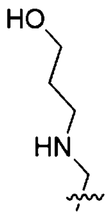, or 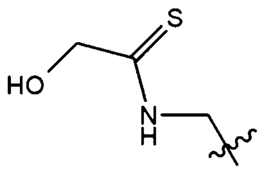;

and

when $R_{1A}$ is $-O-CH_3$, $R_{2A}$ is fluorine, $R_{5A}$ is hydrogen, $R_{6A}$ is not

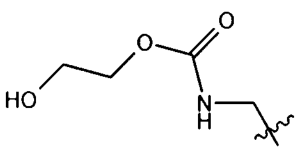, 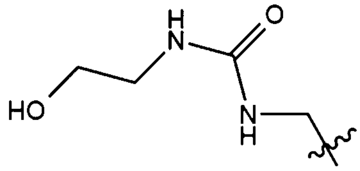, or 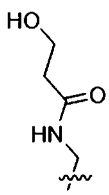;

and

when $R_{1A}$ and $R_{2A}$ form a 1,4-dioxane, and $R_{5A}$ is hydrogen, $R_{6A}$ is $-CH_2NH_2$, $-CH_2NHC_2H_4OH$, $-CH_2N(CH_3)C_2H_4OH$, $-CH_2NHC_3H_6OH$,

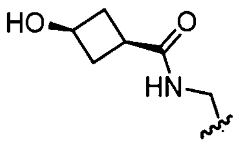,

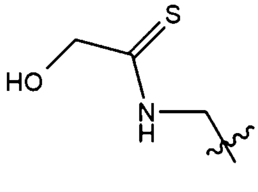, 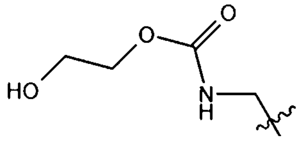, 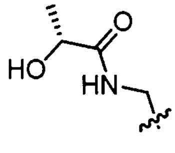, 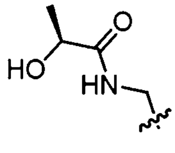,

or

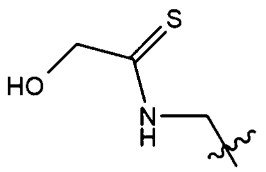

;

and

when $R_{1A}$ is fluorine, $R_{2A}$ is fluorine, and when $R_{5A}$ is hydrogen, $R_{6A}$ is not hydrogen; and
when $R_{1A}$ is $-CH_3$, $R_{2A}$ is chlorine, and when $R_{5A}$ is hydrogen, $R_{6A}$ is selected from

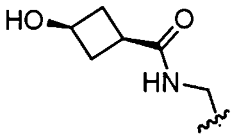

,

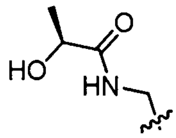

, or

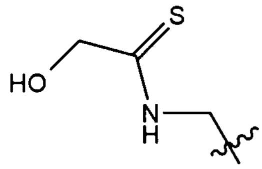

;

and

when $R_{1A}$ and $R_{2A}$ form a 1,3-dioxolane, and $R_{5A}$ is hydrogen, $R_{6A}$ is selected from $-CH_2NHC_2H_4OH$, $-CH_2N(CH_3)C_2H_4OH$, $-CH_2NHC_3H_6OH$, or

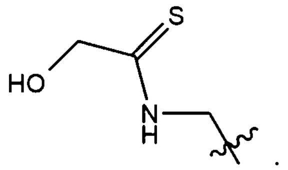

;

wherein, when $R_{5A}$ is selected from $-C_4H_8-OH$, $R_{6A}$ is hydrogen, $R_{1A}$ and $R_{2A}$ are fluorine, or $R_{1A}$ and $R_{2A}$ form a 5- or 6-membered heterocyclyl containing two oxygen atoms.

In some embodiments, each occurrence of ring $W_1$, ring $W_2$, ring $W_3$, ring $W_4$, and ring $W_5$ is independently selected from 6-, or 8-membered aryl, or heteroarylene, 5-, 6-, 7-, or 8-membered heteroaryl or heteroarylene, containing 1, 2, 3, or 4 heteroatoms independently selected from N, O, and S, 4-, 5-, 6-, 7-, or 8-membered cycloalkyl, or cycloalkylene, and 4-, 5-, 6-, 7-, or 8-membered heterocyclyl or heterocyclylene, containing 1, 2, 3, or 4 heteroatoms independently selected from N, O, and S, when each occurrence of ring $W_1$, ring $W_2$, ring $W_3$, ring $W_4$, and ring $W_5$ is independently selected from 4- to 8-membered cycloalkyl, cycloalkylene, heterocyclylene, and heterocyclyl, the 4-, 5-, 6-, 7-, or 8-membered cycloalkyl, cycloalkylene, heterocyclylene, and heterocyclyl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is independently selected from hydrogen, deuterium, F, Cl, Br, I, hydroxyl, =O, =S, $-NH_2$, $-NO_2$, -CN, -COOH, $-OC_{1-3}$ alkyl, $-C_{1-3}$ alkyl, and $C_{1-3}$ alkylene-hydroxyl; when each occurrence of ring $W_1$, ring $W_2$, ring $W_3$, ring $W_4$, and ring $W_5$ is independently selected from 6-, or 8-membered aryl, 6-, or 8-membered arylene, 5-, 6-, 7-, or 8-membered heteroarylene, and heteroaryl, wherein the aryl, arylene, heteroarylene, and heteroaryl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is independently selected from hydrogen, deuterium, F, Cl, Br, I, hydroxyl, $-NH_2$, $-NO_2$, -CN, $-OC_{1-3}$ alkyl, -COOH, and $C_{1-3}$ alky; preferably, each occurrence of ring $W_1$, ring $W_2$, ring $W_3$, ring $W_4$, and ring $W_5$ is independently selected from phenyl or phenylene, pyridinyl, or pyridinylene, pyrazolyl, or pyrazolylene, or

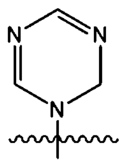

,

optionally substituted with hydrogen, deuterium, F, Cl, Br, I, hydroxyl, - $NH_2$, $-NO_2$, -CN, $-OC_{1-3}$ alky, and -COOH, or phenyl optionally substituted with $C_{1-3}$ alky, or tetrahydropyranyl or tetrahydropyranylene, dioxolanyl or dioxolanylene, tetra-

hydrofuranyl or tetrahydrofuranylene,

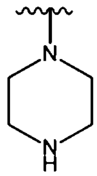 , or 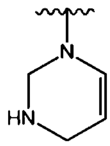 ,

,optionally substituted with hydrogen, deuterium, F, Cl, Br, I, hydroxyl, =O, =S, $-NH_2$, $-NO_2$, - CN, -COOH, $-OC_{1-3}$ alkyl, $-C_{1-3}$ alkyl, or $C_{1-3}$ alkylene-hydroxyl,

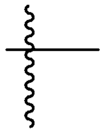

represents the attachment point; and/or

when $R_3$ is

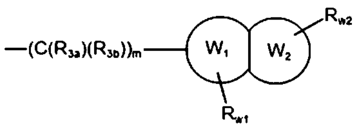 ,

ring $W_1$ and ring $W_2$ are fused to form the following fused ring structure:

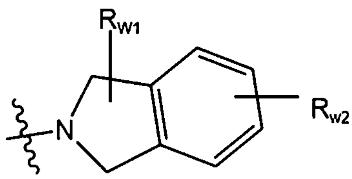 ,

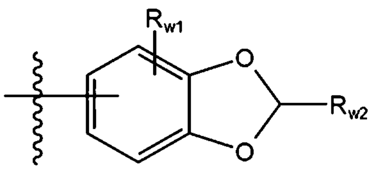 , 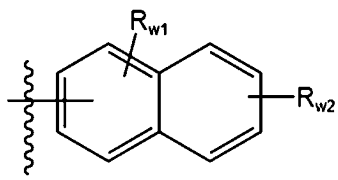

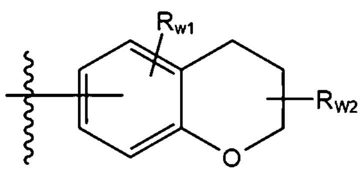 , 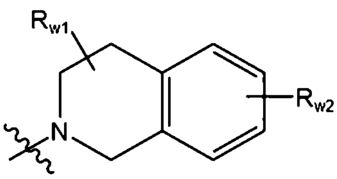 , or 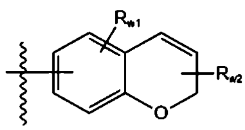 ,

wherein, each occurrence of $R_{w1}$ and $R_{w2}$ is independently selected from hydrogen, deuterium, F, Cl, Br, I, hydroxyl, =O, =S, $-NH_2$, $-NO_2$, -CN, -COOH, $-OC_{1-3}$ alkyl, $-C_{1-3}$ alkyl, and $C_{1-3}$ alkylene-hydroxyl,

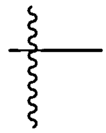

represents the attachment point;

each occurrence of $R_{3A}$ is independently selected from hydrogen, -C(O)R', - C(S)R', -C(S)C($R_{a1}$)($R_{b1}$)OH, -C(O)$CH_2$N($R_{a1}$)($R_{b1}$), -$CO_2$R', -C(O)C($R_{a1}$)($R_{b1}$)OH, - C(O)-$C_{3-6}$ cycloalkylene-OH, -C(O)-$C_{1-6}$ alkylene-OH, -C(O)-$C_{3-6}$ cycloalkylene-$C_{1-6}$ alkylene-OH, -C(O)O-$C_{1-6}$ alkylene-OH, and $C_{1-6}$ alkyl, $C_{1-6}$ alkenyl, and $C_{1-6}$ alkynyl optionally substituted with R'; and/or

preferably, $R_{4A}$ is selected from hydrogen or $C_{1-6}$ alkyl; and/or

each occurrence of R', $R_{a1}$, and $R_{b1}$ is independently hydrogen, fluorine, chlorine, -OH, $C_{1-6}$ alkyl, $C_{2-6}$ alkenyl,or $C_{2-6}$ alkynyl, 3, 4, 5, or 6-membered cycloalkyl, or 3, 4, 5, or 6-membered heterocycloalkyl containing heteroatoms independently selected from N, O, and S, the $C_{1-6}$ alkyl, $C_{2-6}$ alkenyl, $C_{2-6}$ alkynyl, 3, 4, 5, 6-membered cycloalkyl, or 3, 4, 5, or 6-membered heterocyclyl is unsubstituted or substituted with 1 or 2 substituents independently selected from fluorine, chlorine, -$NO_2$, -CN, -OH, - $NH_2$, $C_{1-6}$ alkyl, $C_{2-6}$ alkenyl, $C_{2-6}$ alkynyl, and hydroxyl-$C_{1-6}$ alkylene.

In some embodiments, the compound is selected from:

wherein $R_3$ is selected from the following groups:

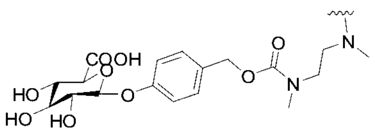, 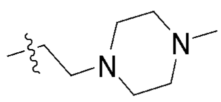, 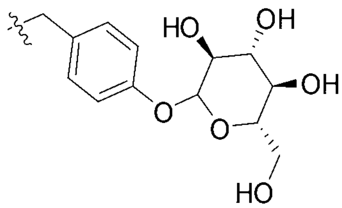,

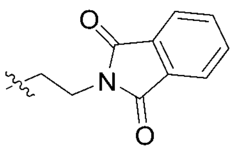, 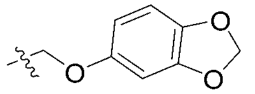, 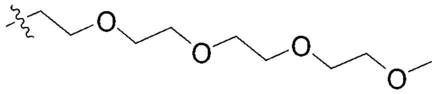,

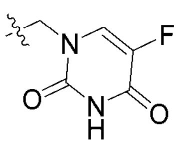, 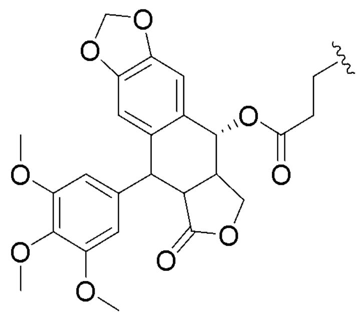, 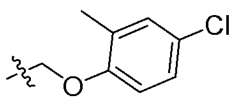, 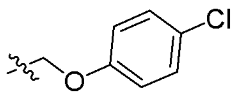,

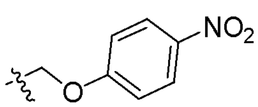, 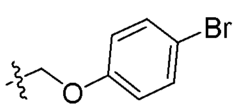, 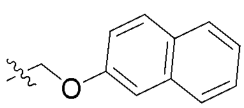,

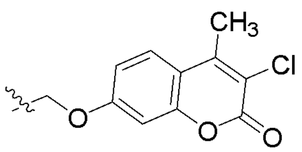, 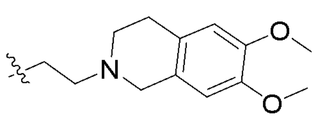, 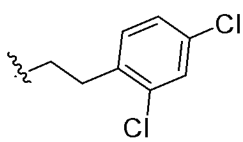, 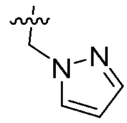,

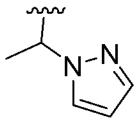, 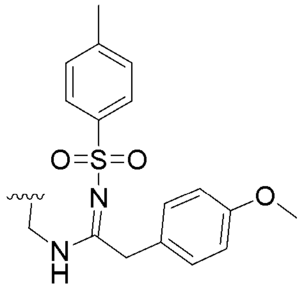, 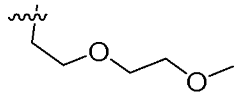,

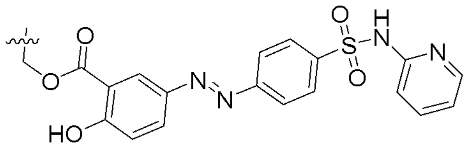, 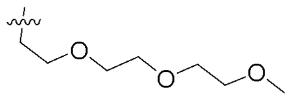,

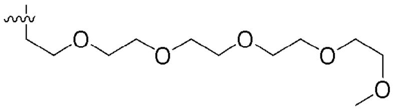, 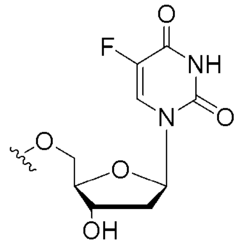, 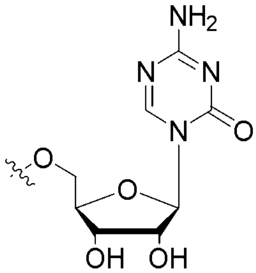,

and

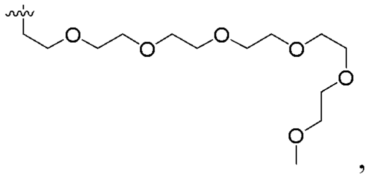,

wherein

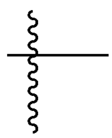

represents the attachment point.

In some embodiments, the compound is selected from:

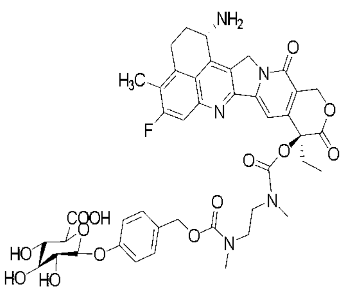 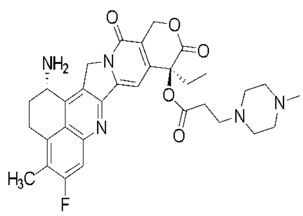

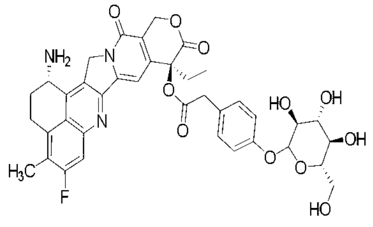
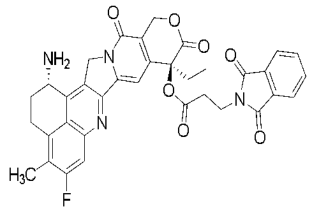

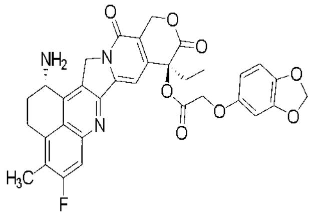
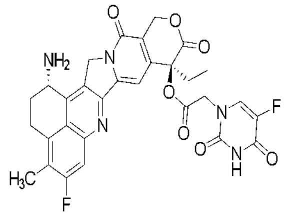

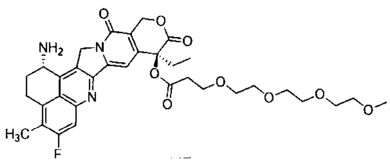
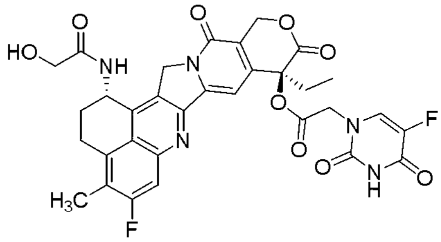

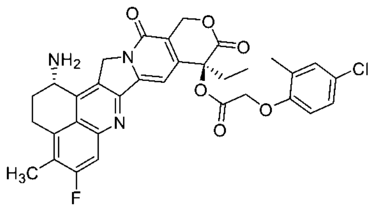
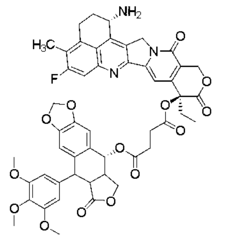

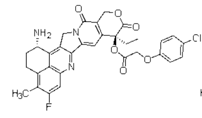
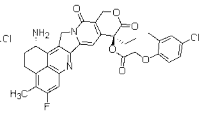
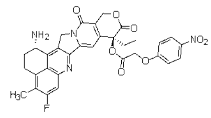

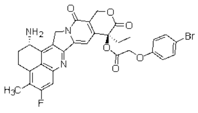
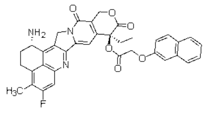
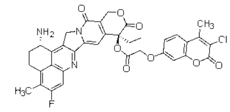

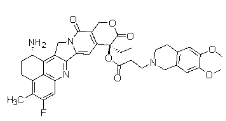

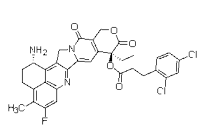
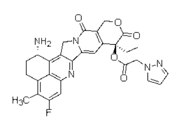
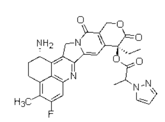

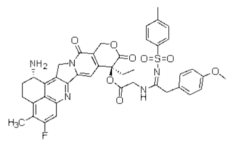
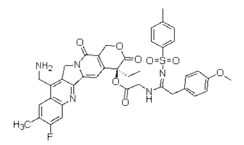
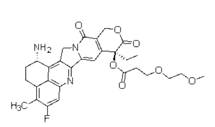

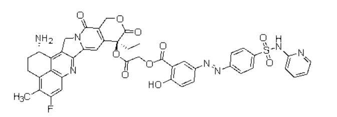
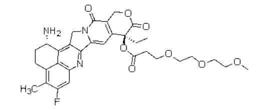

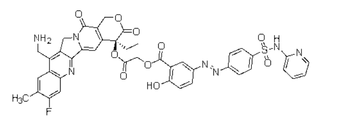
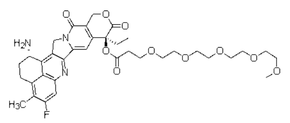

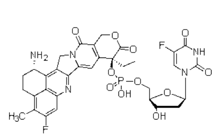
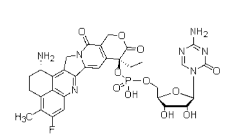
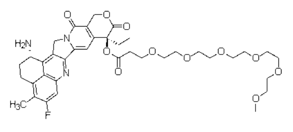

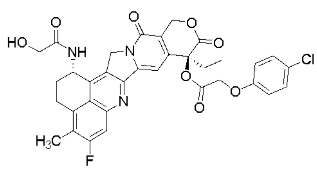
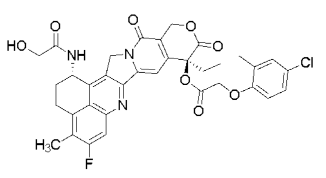
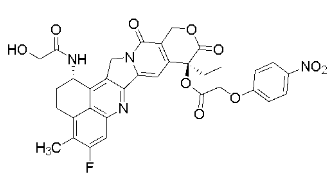

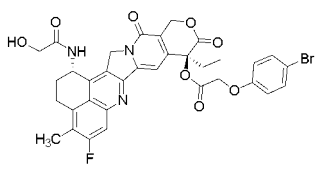
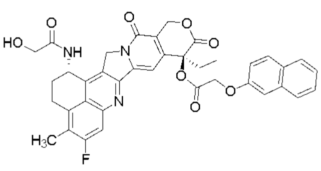
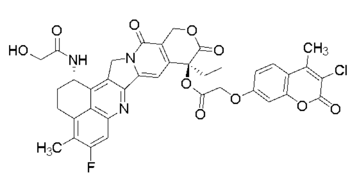

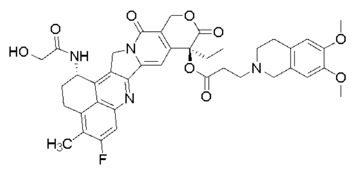

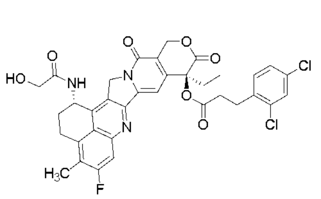
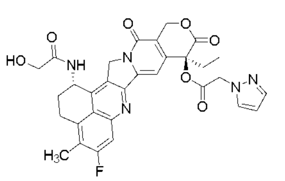
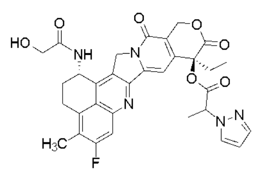

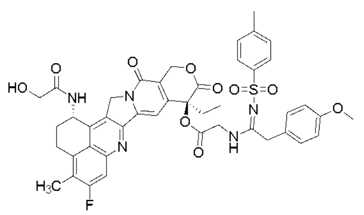
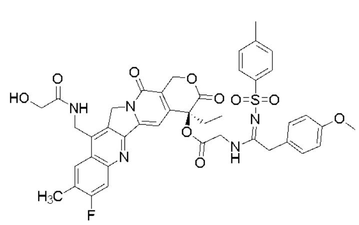

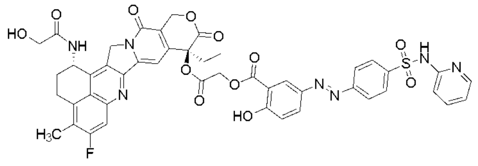

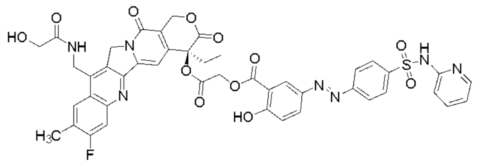

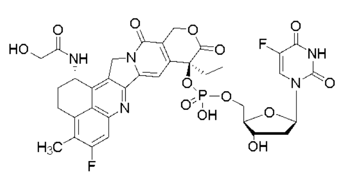
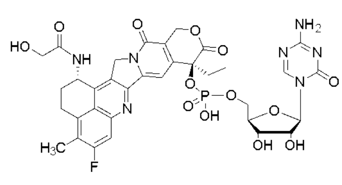

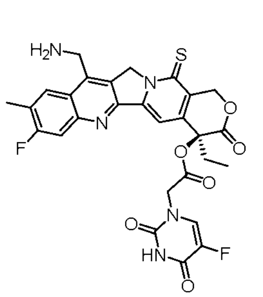
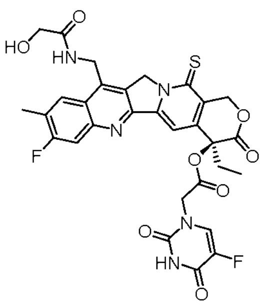

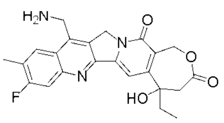
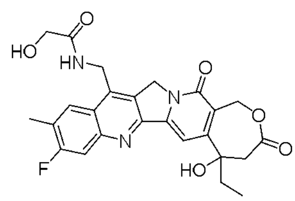
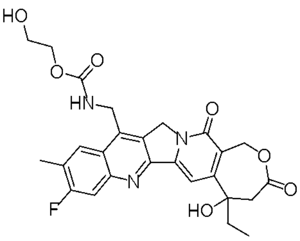

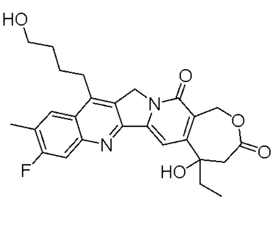
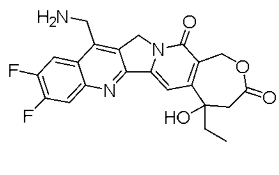
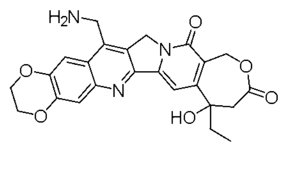

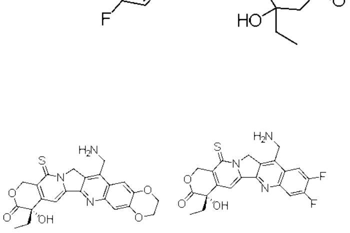
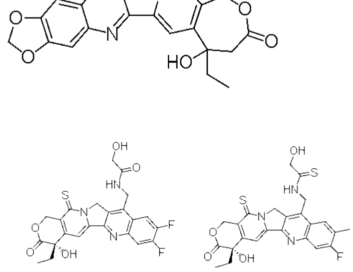

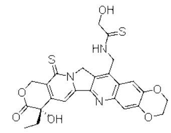
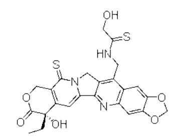
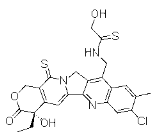
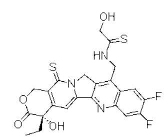

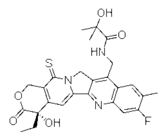
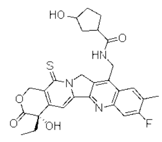
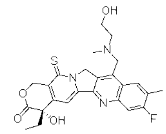

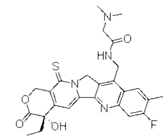
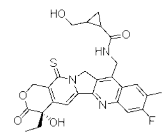
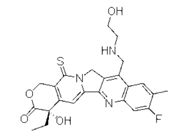

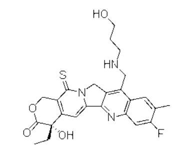
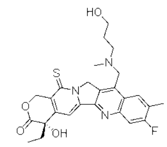

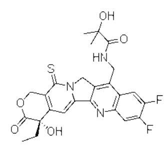
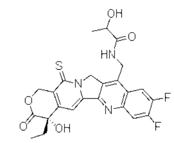
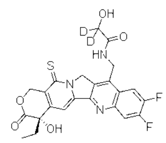
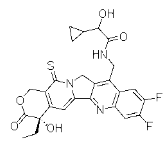

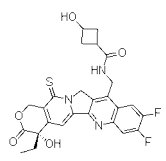
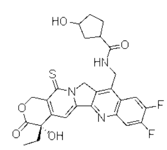
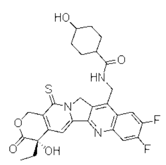
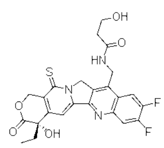

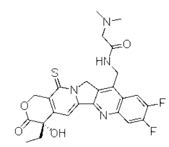
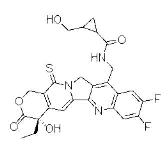
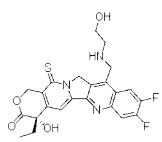
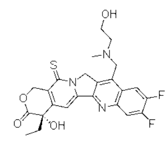

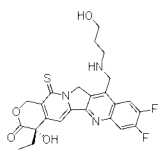
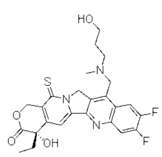
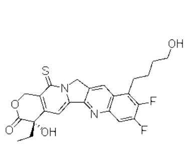
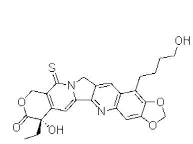

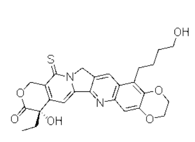
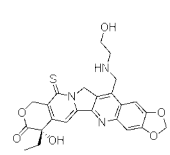
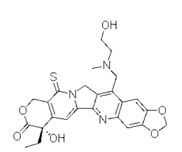
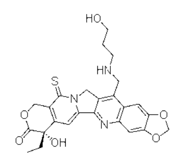

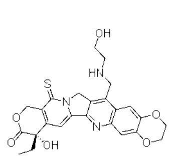
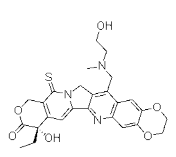
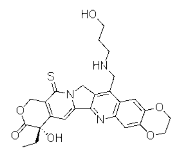

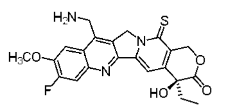
,
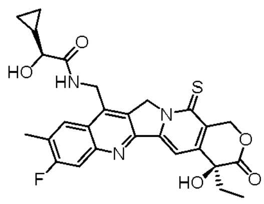
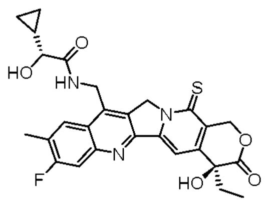

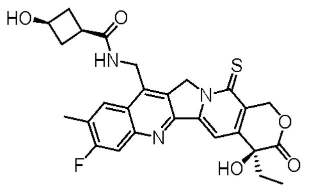
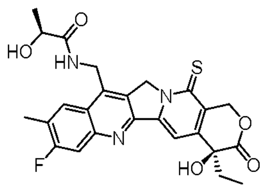
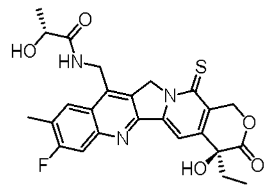

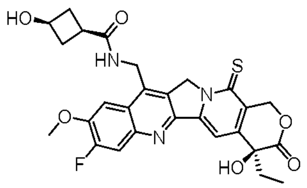
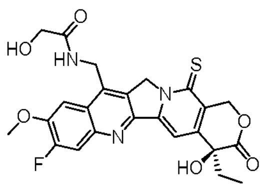
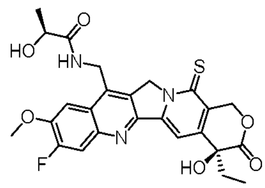
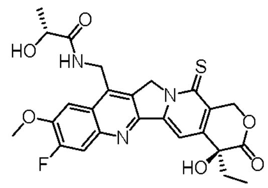

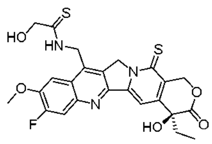
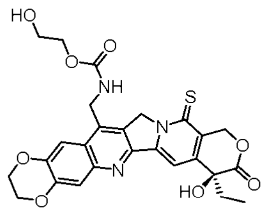
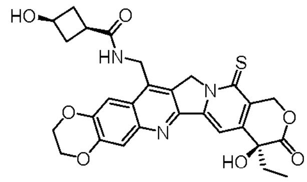
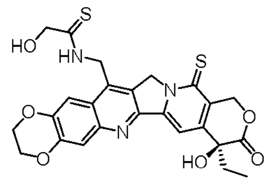

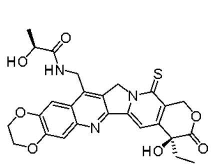
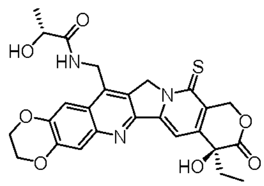
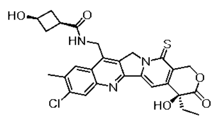
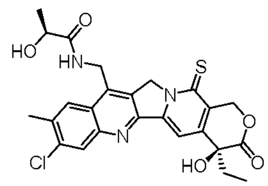

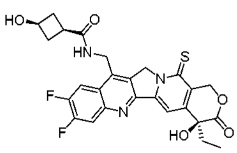
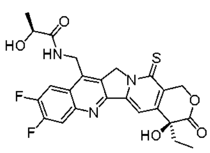
。

In some embodiments, a compound represented by Formula 1A, or a pharmaceutically acceptable salt thereof,

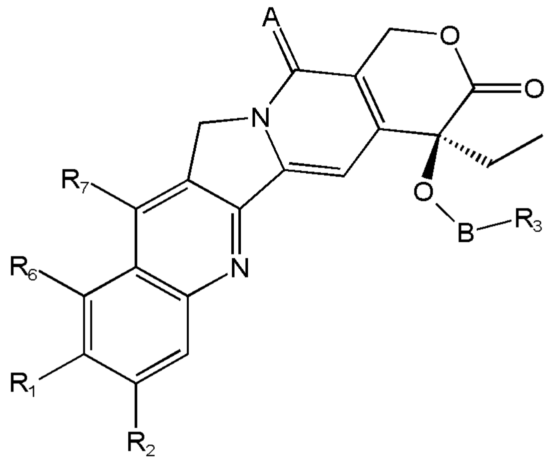

Formula 1A

wherein, $R_1$ and $R_2$ are each independently selected from hydrogen, halogen, $C_{1-8}$ alkyl, and $C_{1-8}$ haloalkyl;
A is selected from oxygen or sulfur;
B is selected from -C(O)- or -P(O)(OH)-;
$R_3$ is selected from $-(C(R_{3a})(R_{3b}))_m-R_{3c}$,

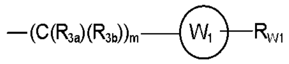

,

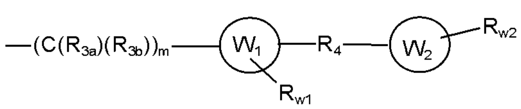

,

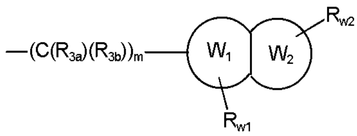

,

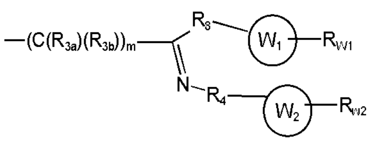

,

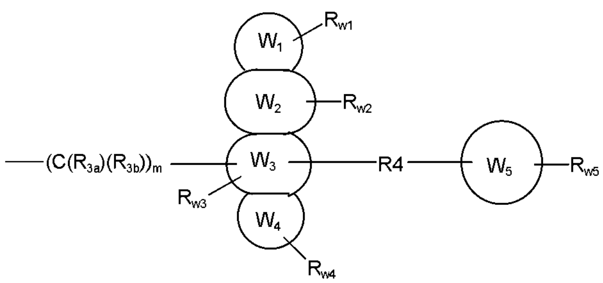

,

or

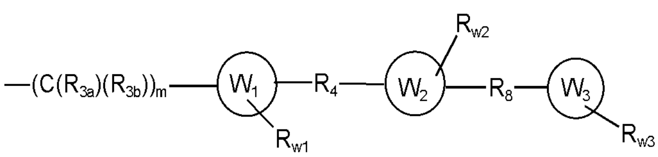

,

wherein when the $-(C(R_{3a})(R_{3b}))_m-$ contains methylene units, the n methylene units of the $-(C(R_{3a})(R_{3b}))_m-$ are each independently replaced by $-N(R_5)C(O)-$, $-C(O)N(R_5)-$, -C(O)-, -OC(O)-, -C(O)O-, $-NR_5-$, -O-, -S-, -SO-, $-SO_2-$, $-P(R_5)-$, $-P(=O)(R_5)-$, $-N(R_5)SO_2-$, $-SO_2N(R_5)-$, -C( = S)-, $-C( = NR_5)-$, -N = N-, -CH = N-, or -N = CH-, when $R_3$ is

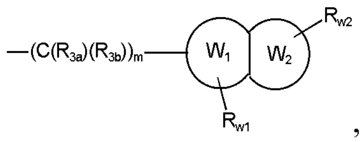

$W_1$ and $W_2$ are fused together;

$R_4$ and $R_8$ are each independently a single bond or selected from -O-, $C_{1-8}$ alkylene, $C_{1-8}$ haloalkylene, $-N(R_5)C(O)-$, $-C(O)N(R_5)-$, -C(O)-, -OC(O)-, -C(O)O-, $-NR_5-$, -S-, - SO-, $-SO_2-$, $-P(R_5)-$, $-P(=O)(R_5)-$, $-N(R_5)SO_2-$, $-SO_2N(R_5)-$, -C(=S)-, $-C(=NR_5)-$, -N =N-, -CH=N-, or -N=CH-;

ring $W_1$, ring $W_2$, ring $W_3$, ring $W_4$, and ring $W_5$ are each independently selected from 6- to 10-membered aryl, 5- to 10-membered heteroaryl, 3- to 10- membered cycloalkyl, and 3- to 10-membered heterocyclyl;

when ring $W_1$, ring $W_2$, ring $W_3$, ring $W_4$, and ring $W_5$ are each independently selected from cycloalkyl or heterocyclyl, the cycloalkyl or heterocyclyl is substituted with one or more $R_{w1}$, $R_{w2}$, $R_{w3}$ , $R_{w4}$, or $R_{w5}$, each occurrence of $R_{w1}$, $R_{w2}$ , $R_{w3}$ , $R_{w4}$, or $R_{w5}$ is independently selected from hydrogen, protium, deuterium, tritium, halogen, = O, = S, $-NO_2$, -CN, -OR, -SR, $-N(R_a)(R_b)$, -C(O)R, $-CO_2R$, -C(O)C(O)R, - $C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, or $C_{1-6}$ aliphatic group optionally substituted with R;

when ring $W_1$, ring $W_2$, ring $W_3$, ring $W_4$, and ring $W_5$ are each independently selected from aryl or heteroaryl, the aryl or heteroaryl is substituted with one or more $R_{w1}$, $R_{w2}$, $R_{w3}$, $R_{w4}$ , or $R_{w5}$, each occurrence of $R_{w1}$, $R_{w2}$, $R_{w3}$, $R_{w4}$ , or $R_{w5}$ is independently selected from hydrogen, protium, deuterium, tritium, halogen, $-NO_2$, - CN, -OR, -SR, $-N(R_a)(R_b)$, -C(O)R, $-CO_2R$, -C(O)C(O)R, $-C(O)CH_2C(O)R$, -S(O)R, - $S(O)_2R$, $-C(O)N(R_a)(R_b)$, $-SO_2N(R_a)(R_b)$, -OC(O)R, $-N(R)SO_2R$, or $C_{1-6}$ aliphatic group optionally substituted with R;

$R_6$ is selected from hydrogen, halogen, $C_{1-8}$ alkyl, and $C_{1-8}$ haloalkyl; preferably, $R_6$ is selected from hydrogen;

$R_7$ is selected from $C_{1-8}$ alkylene-$N(R_a)(R_b)$, $C_{1-8}$ haloalkylene-$N(R_a)(R_b)$, halogen, $-NO_2$, -CN, -OR, -SR, $-N(R_a)(R_b)$, -C(O)R, $-CO_2R$, -C(O)C(O)R, $-C(O)CH_2C(O)R$, - S(O)R, $-S(O)_2R$, $-C(O)N(R_a)(R_b)$, $-SO_2N(R_a)(R_b)$, -OC(O)R, $-N(R)SO_2R$, or $C_{1-6}$ aliphatic group optionally substituted with R; preferably, $R_7$ is selected from $C_{1-8}$ alkylene-$N(R_a)(R_b)$, or $C_{1-a}$ haloalkylene-$N(R_a)(R_b)$; preferably, $R_7$ is selected from - $CH_2N(R_a)(R_b)$;

or $R_6$ and $R_7$ together with the carbon atoms to which they are attached form a saturated or unsaturated 5- to 10-membered cycloalkyl or 6- to 10-membered heterocyclyl containing 1 to 3 heteroatoms independently selected from N, O, and S, wherein the cycloalkyl or heterocyclyl is substituted with one or more substituents independently selected from halogen, $-NO_2$, -CN, -OR, -SR, $-N(R_a)(R_b)$, -C(O)R, - $CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R; preferably, $R_6$ and $R_7$ together with the carbon atoms to which they are attached form a 6-membered cycloalkyl, the cycloalkyl is substituted with $-N(R_a)(R_b)$;

each occurrence of $R_{3a}$, $R_{3b}$, $R_{3c}$, and $R_5$ is independently hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ aliphatic group optionally substituted with R;

each occurrence of R, $R_a$, and $R_b$ is independently hydrogen, protium, deuterium, tritium, halogen, $-NO_2$, -CN, -OH, -SH, $-NH_2$, -C(O)H, $-CO_2H$, -C(O)C(O)H, - $C(O)CH_2C(O)H$, $-C(O)CH_2OH$, -S(O)H, $-S(O)_2H$, $-C(O)NH_2$, $-SO_2NH_2$, -OC(O)H, - $N(H)SO_2H$, or $C_{1-6}$ aliphatic group;

m and n are integers greater than or equal to 0;

when $R_3$ is $-(C(R_{3a})(R_{3b}))_m-R_{3c}$, $-(C(R_{3a})(R_{3b}))_m-$ contains methylene units, the n methylene units of the $-(C(R_{3a})(R_{3b}))_m-$ are each independently replaced by - $N(R_5)C(O)-$, $-C(O)N(R_5)-$, -C(O)-, -OC(O)-, -C(O)O-, $-NR_5-$, -O-, -S-, -SO-, $-SO_2-$, - $P(R_5)-$, $-P(=O)(R_5)-$, $-N(R_5)SO_2-$, $-SO_2N(R_5)-$, -C(=S)-, $-C(=NR_5)-$, -N=N-, -CH= N- or -N=CH-;

preferably, $R_3$ is $-(CH_2)_a(CH_2CH_2O)_b(CH_3)_c$, or $C_{1-8}$ aliphatic group optionally substituted with R, m = a + 3 * b + c, b is not 0, a and b are integers greater than 0; when $R_3$ is

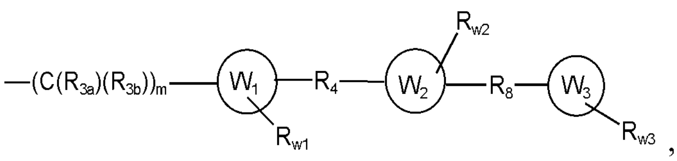

$-(C(R_{3a})(R_{3b}))_m-$ contains methylene units, the n methylene units of the $-(C(R_{3a})(R_{3b}))_m-$ are each independently replaced by $-N(R_5)C(O)-$, $-C(O)N(R_5)-$, -C(O)-, -OC(O)-, -C(O)O-, - $NR_5-$, -O-, or -S-, wherein ring $W_1$, ring $W_2$, and ring $W_3$ are each independently selected from 6- to 10-membered aryl, 5- to 8-membered heteroaryl, 4- to 8-membered cycloalkyl, or 4- to 8-membered heterocyclyl; preferably, ring $W_1$ is selected from phenyl or a 4- to 8-membered heterocyclyl; ring $W_2$ is selected from phenyl or 4- to 8-membered heterocyclyl; ring $W_3$ is selected from

phenyl or 5- to 8-membered heteroaryl; more preferably, ring $W_1$ is phenyl or oxolanyl;
ring $W_2$ is phenyl or oxolanyl; ring $W_3$ is phenyl or pyridinyl;
when ring $W_1$, ring $W_2$, and ring $W_3$ are selected from cycloalkyl or heterocyclyl, the cycloalkyl or heterocyclyl is substituted with one or more $R_{w1}$, $R_{w2}$, or $R_{w3}$, each occurrence of $R_{w1}$, $R_{w2}$, or $R_{w3}$ is independently selected from hydrogen, protium, deuterium, tritium, halogen,=O, =S, $-NO_2$, -CN, -OR, -SR, $-N(R_a)(R_b)$, -C(O)R, - $CO_2R$, -C(O)C(O) R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R; when ring $W_1$, ring $W_2$, or ring $W_3$ is selected from 6- to 10-membered aryl or 5- to 8-membered heteroaryl, the aryl or heteroaryl is substituted with one or more $R_{w1}$, $R_{w2}$, or $R_{w3}$, each occurrence of $R_{w1}$, $R_{w2}$, or $R_{w3}$ is independently selected from hydrogen, protium, deuterium, tritium, halogen, $-NO_2$, -CN, -OR, -SR, $-N(R_a)(R_b)$, - C(O)R, $-CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R; when $R_3$ is

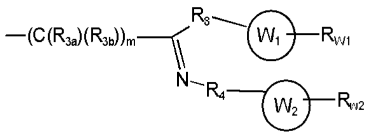
,

and $-(C(R_{3a})(R_{3b}))_m-$ contains methylene units, the n methylene units of the $(C(R_{3a})(R_{3b}))_m-$ are each independently replaced by $-N(R_5)C(O)-$, $-C(O)N(R_5)-$, -C(O)-, -OC(O)-, -C(O)O-, $-NR_5-$, -O-, or -S-; wherein ring $W_1$ is selected from 6- to 10-membered aryl, 4- to 8-membered cycloalkyl, 4- to 8-membered heterocyclyl, and 5- to 8-membered heteroaryl, when ring $W_1$ is selected from 4- to 8-membered cycloalkyl or 4- to 8-membered heterocyclyl, the cycloalkyl or heterocyclyl is substituted with one or more $R_{w1}$, each occurrence of $R_{w1}$ is independently selected from hydrogen, protium, deuterium, tritium, halogen, -(=O), -( = S), $-NO_2$, -CN, -OR, -SR, $-N(R_a)(R_b)$, -C(O)R, $-CO_2R$, -C(O) C(O)R, - $C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, or $C_{1-6}$ aliphatic group optionally substituted with R; when ring $W_1$ is selected from 6- to 10-membered aryl or 5- to 8-membered heteroaryl, the aryl or heteroaryl is substituted with one or more $R_{w1}$, each occurrence of $R_{w1}$ is independently selected from hydrogen, protium, deuterium, tritium, halogen, $-NO_2$, -CN, -OR, -SR, - $N(R_a)(R_b)$, -C(O)R, $-CO_2R$, -C(O)C(O)R, $-C(O)CH_2C(O)R$, -S(O)R, $-S(O)_2R$, - $C(O)N(R_a)(R_b)$, $-SO_2N(R_a)(R_b)$, -OC(O)R, $-N(R)SO_2R$, or $C_{1-6}$ aliphatic group optionally substituted with R;
when $R_3$ is

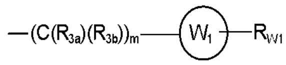
,

and $-(C(R_{3a})(R_{3b}))_m-$ contains methylene units, the n methylene units of the $-(C(R_{3a})(R_{3b}))_m-$ are each independently replaced by - $N(R_5)C(O)-$, $-C(O)N(R_5)-$, -C(O)-, -OC(O)-, -C(O)O-, $-NR_5-$, -O-, -S-, -SO-, $-SO_2-$, - $P(R_5)-$, $-P(=O)(R_5)-$, $-N(R_5)SO_2-$, $-SO_2N(R_5)-$, -C(=S)-, $-C(=NR_5)-$, -N=N-, -CH= N-, or -N=CH-;
preferably, $R_3$ is

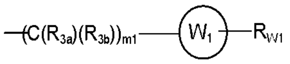
,or
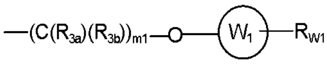
,

m1 is an integer from 1 to 6; ring $W_1$ is selected from 6- to 10-membered aryl, 4- to 8-membered cycloalkyl, 4- to 8-membered saturated or unsaturated heterocyclyl containing 1 to 3 heteroatoms optionally selected from N, O, and S, and 5- to 8-membered heteroaryl containing 1 to 3 heteroatoms optionally selected from N, O, and S;
when ring $W_1$ is selected from 4- to 8-membered cycloalkyl or 4- to 8-membered heterocyclyl, the cycloalkyl or heterocyclyl is substituted with one or more $R_{w1}$, each occurrence of $R_{w1}$ is independently selected from hydrogen, protium, deuterium, tritium, halogen, =O, =S, $-NO_2$, -CN, -OR, -SR, $-N(R_a)(R_b)$, -C(O)R, $-CO_2R$, -C(O)C(O)R, -C(O) $CH_2C(O)R$, -S(O)R, $-S(O)_2R$, $-C(O)N(R_a)(R_b)$, $-SO_2N(R_a)(R_b)$, -OC(O)R, - $N(R)SO_2R$, or a $C_{1-6}$ aliphatic group optionally substituted with R;
when ring $W_1$ is selected from 6- to 10-membered aryl or 5- to 8-membered heteroaryl, the phenyl or 5- to 8-membered heteroaryl is substituted with one or more $R_{w1}$, each occurrence of $R_{w1}$ is independently selected from hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)$ R, -S(O)R, $-S(O)_2R$, $-C(O)N(R_a)(R_b)$, $-SO_2N(R_a)(R_b)$, - OC(O)R, $-N(R)SO_2R$, or $C_{1-6}$ aliphatic group optionally substituted with R;
preferably, when ring $W_1$ is a 5- to 8-membered heteroaryl substituted with one or more $R_{w1}$, the heteroaryl is pyridinyl

or pyrazolyl; when ring $W_1$ is selected from 4- to 8-membered heterocyclyl, containing 1, 2, or 3 heteroatoms, each occurrence of the heteroatoms is independently selected from N, O, or S; preferably, ring $W_1$ is

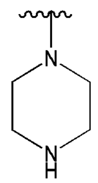

and

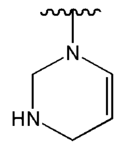

;

ring $W_1$ is substituted with one or more $R_{w1}$, each occurrence of $R_{w1}$ is independently selected from hydrogen, protium, deuterium, tritium, halogen, =O, = S, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, or $C_{1-6}$ aliphatic group optionally substituted with R;
when $R_3$ is

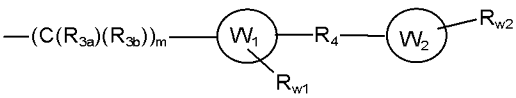

,

and $-(C(R_{3a})(R_{3b}))_m-$ contains methylene units, the n methylene units of the $-(C(R_{3a})(R_{3b}))_m-$ are each independently replaced by $-N(R_5)C(O)-$, $-C(O)N(R_5)-$, -C(O)-, -OC(O)-, -C(O)O-, - $NR_5-$, -O-, -S-, -SO-, $-SO_2-$, $-P(R_5)-$, $-P(=O)(R_5)-$, $-N(R_5)SO_2-$, $-SO_2N(R_5)-$, -C(=S)-, $-C(=NR_5)-$, -N=N-, -CH=N-, or -N=CH-; wherein ring $W_1$ and ring $W_2$ are each independently selected from substituted or unsubstituted 6- to 10-membered aryl, 5- to 8-membered heteroaryl, 4- to 8-membered cycloalkyl, or 4- to 8-membered heterocyclyl; preferably, ring $W_1$ is selected from phenyl or 4- to 8-membered heterocyclyl, substituted with one or more $R_{w1}$, ring $W_2$ is selected from 4- to 8-membered heterocyclyl substituted with one or more $R_{w2}$; preferably, ring $W_1$ is phenyl or oxolanyl substituted with one or more $R_{w1}$ ;
ring $W_2$ is selected from

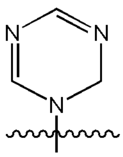

, 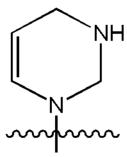

, and 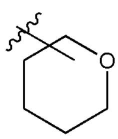

,

and each substituted with one or more $R_{w2}$;
when ring $W_1$ or ring $W_2$ is selected from cycloalkyl or heterocyclyl, the cycloalkyl or heterocyclyl is substituted with one or more $R_{w1}$ or $R_{w2}$, each occurrence of $R_{w1}$ or $R_{w2}$ is independently selected from hydrogen, protium, deuterium, tritium, halogen, = O, = S, $-NO_2$, -CN, -OR, -SR, $-N(R_a)(R_b)$, -C(O)R, $-CO_2R$, -C(O)C(O)R, - $C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, or $C_{1-6}$ aliphatic group optionally substituted with R;
when ring $W_1$ or ring $W_2$ is selected from 6- to 10-membered aryl or 5- to 8-membered heteroaryl, the aryl or heteroaryl is substituted with one or more $R_{w1}$ or $R_{w2}$, each occurrence of $R_{w1}$ or $R_{w2}$ is independently selected from hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ aliphatic group optionally substituted with R, $R_4$ is a single bond or selected from -O-, $C_{1-8}$ alkylene, $C_{1-8}$ haloalkylene, $-N(R_5)C(O)-$, - $C(O)N(R_5)-$, -C(O)-, -OC(O)-, -C(O)O-, $-NR_5-$, -S-, -SO-, $-SO_2-$, $-P(R_5)-$, $-P(=O)(R_5)-$, $-N(R_5)SO_2-$, $-SO_2N(R_5)-$, -C(=S)-, $-C(=NR_5)-$, -N=N-, -CH=N-, or -N=CH-;
when $R_3$ is

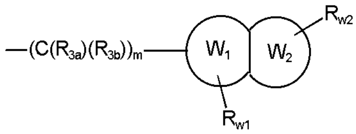

,

$-(C(R_{3a})(R_{3b}))_m-$ contains methylene units, the n methylene units of the $-(C(R_{3a})(R_{3b}))_m-$ are each independently replaced by $-N(R_5)C(O)-$, $-C(O)N(R_5)-$, -C(O)-, -OC(O)-, -C(O)O-, $-NR_5-$, -O-, -S-, -SO-, $-SO_2-$, - $P(R_5)-$, $-P(=O)(R_5)-$, $-N(R_5)SO_2-$, $-SO_2N(R_5)-$, -C(=S)-, $-C(=NR_5)-$, -N=N-, -CH= N-, or -N=CH-; ring $W_1$ and ring $W_2$ are each independently selected from 6- to 10-membered aryl, 5- to 8-membered heteroaryl, 4- to 8-membered cycloalkyl, or 4- to 8-membered heterocyclyl; preferably, ring $W_1$ and ring $W_2$ are fused to form a fused ring structure selected from any one of the following structures:

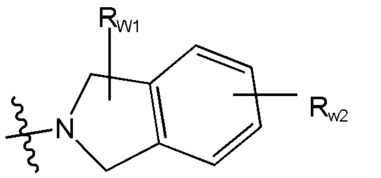

,

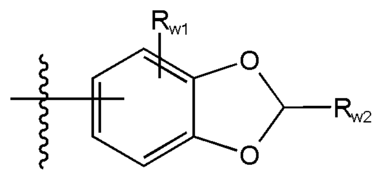

,

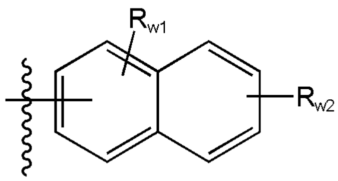

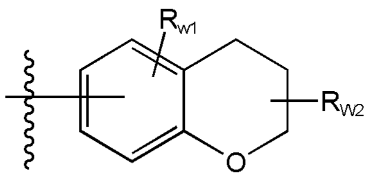

,

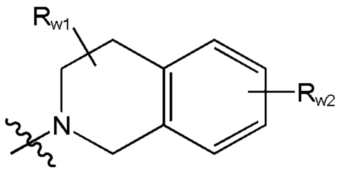

,

and

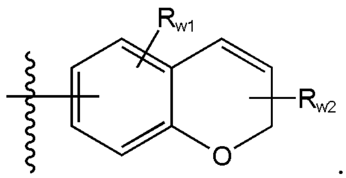

;

when ring $W_1$ or ring $W_2$ is a 4- to 8-membered cycloalkyl or 4- to 8-membered heterocyclyl, the cycloalkyl or heterocyclyl is substituted with one or more $R_{w1}$ or $R_{w2}$, each occurrence of $R_{w1}$ or $R_{w2}$ is independently selected from hydrogen, protium, deuterium, tritium, halogen, =O, =S, $-NO_2$, -CN, -OR, -SR, $-N(R_a)(R_b)$, -C(O)R, - $CO_2R$, -C(O)C(O) R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R;
when ring $W_1$ or ring $W_2$ is a 6- to 10-membered aryl or 5- to 8-membered heteroaryl, the aryl or heteroaryl is substituted with one or more $R_{w1}$ or $R_{w2}$, each occurrence of $R_{w1}$ or $R_{w2}$ is independently selected from hydrogen, protium, deuterium, tritium, halogen, $-NO_2$, -CN, -OR, -SR, $-N(R_a)(R_b)$, -C(O)R, $-CO_2R$, -C(O)C(O)R, - $C(O)CH_2C(O)$ R, -S(O)R, $-S(O)_2R$, $-C(O)N(R_a)(R_b)$, $-SO_2N(R_a)(R_b)$, -OC(O)R, - $N(R)SO_2R$, or $C_{1-6}$ aliphatic group optionally substituted with R;
when $R_3$ is

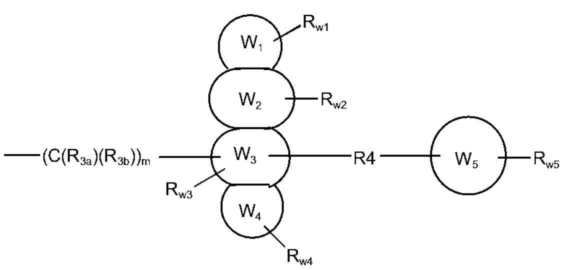

,

$-(C(R_{3a})(R_{3b}))_m$ contains methylene units, the n methylene units of the $-(C(R_{3a})(R_{3b}))_m$- are each independently replaced by $-N(R_5)C(O)-$, $-C(O)N(R_5)-$, -C(O)-, -OC(O)-, -C(O)O-, $-NR_5-$, -O-, -S-, - SO-, $-SO_2-$, $-P(R_5)-$, $-P(=O)(R_5)-$, $-N(R_5)SO_2-$, $-SO_2N(R_5)-$, -C(=S)-, $-C(=NR_5)-$, -N =N-, -CH=N-, or -N=CH-; ring $W_1$, $W_2$, $W_3$, and $W_4$ are fused together in sequence, ring $W_1$ and ring $W_4$ are each independently selected from 4- to 8-membered heterocyclyl, the heterocyclyl is substituted with one or more $R_{w1}$ or $R_{w4}$, each occurrence of $R_{w1}$ or $R_{w4}$ is independently selected from hydrogen, protium, deuterium, tritium, halogen, = O, = S, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ aliphatic group optionally substituted with R; ring $W_5$ and ring $W_2$ is 6- to 10-membered aryl or 5- to 8-membered heteroaryl, the aryl or heteroaryl is substituted with one or more $R_{w2}$, each occurrence of $R_{w2}$ is independently selected from hydrogen, protium, deuterium, tritium, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R; ring $W_3$ is 4- to 8-membered cycloalkyl, the cycloalkyl is substituted with one or more $R_{w3}$, each occurrence of $R_{w3}$ is independently selected from hydrogen, protium, deuterium, tritium, halogen, =O, =S, $-NO_2$, -CN, -OR, -SR, $-N(R_a)(R_b)$, -C(O)R, - $CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R; $R_4$ is a single bond or selected from -O-, $C_{1-8}$ alkylene, $C_{1-8}$ haloalkylene, - $N(R_5)C(O)-$, $-C(O)N(R_5)-$, -C(O)-, -OC(O)-, -C(O) O-, $-NR_5-$, -S-, -SO-, $-SO_2-$, $-P(R_5)-$, $-P(=O)(R_5)-$, $-N(R_5)SO_2-$, $-SO_2N(R_5)-$, -C(=S)-, $-C(=NR_5)-$, -N=N-, -CH=N-, or - N = CH-;

m is an integer from 1 to 20;

n is an integer from 0 to 8.

In some embodiments, a compound represented by Formula 1C, or a pharmaceutically acceptable salt thereof,

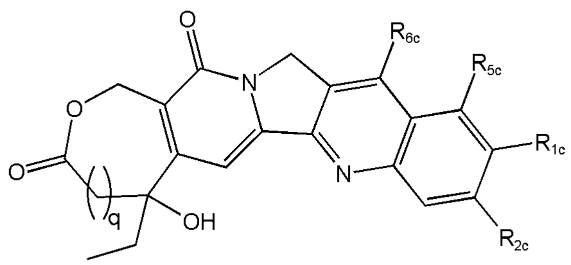

Formula 1C

wherein, q is 1, 2, or 3;

$R_{1c}$, $R_{2c}$, $R_{5c}$ are independently selected from hydrogen, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, - $C(O)N(R_a)(R_b)$, $-SO_2N(R_a)(R_b)$, -OC(O)R, $-N(R)SO_2R$, or $C_{1-6}$ aliphatic group optionally substituted with R;

or $R_{1c}$ and $R_{2c}$ together with the carbon atoms to which they are attached form a saturated or unsaturated 5- to 10-membered cycloalkyl or 6- to 10-membered heterocyclyl containing 1 to 3 heteroatoms independently selected from N, O, and S, wherein the cycloalkyl or heterocyclyl is substituted with one or more substituents independently selected from halogen, $-NO_2$, -CN, -OR, -SR, $-N(R_a)(R_b)$, -C(O)R, - $CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R;

$R_{6c}$ is $C_{1-8}$ alkyl-hydroxyl, $C_{1-8}$ alkylamino, $-C(R_a)(R_b)-N(R_a)-R_{4c}$, $-C(R_a)(R_b)-N(R_a)-C(O)-R_{4c}$, or $-C(R_a)(R_b)-N(R_a)-C(O)O-R_{4c}$;

Each occurence of R, $R_a$, $R_b$, and $R_{4c}$ are independently selected from hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, -S(O)H, $-S(O)_2H$, $-C(O)NH_2$, $-SO_2NH_2$, -OC(O)H, - $N(H)SO_2H$, $C_{1-6}$ aliphatic group, 3- to 8-membered cycloalkyl, 3- to 8-membered heterocyclyl, 6- to 10-membered aryl, or 5- to 10-membered heteroaryl, the $C_{1-6}$ aliphatic group, 3- to 8-membered cycloalkyl, 3- to 8-membered heterocyclyl, 6- to 10-membered aryl, or 5- to 10-membered heteroaryl is unsubstituted or substituted with one or more substituents independently selected from halogen, $-NO_2$, -CN, -OH, -SH, $-NH_2$, -C(O)H, $-CO_2H$, -C(O) C(O)H, $-C(O)CH_2C(O)H$, -S(O)H, $-S(O)_2H$, $-C(O)NH_2$, $-SO_2NH_2$, -OC(O)H, $-N(H)SO_2H$, $C_{1-6}$ aliphatic group, and $C_{1-6}$ alkylene-hydroxyl.

The second aspect of the present invention provides a ligand-drug conjugate represented by Formula 3, or a pharmaceutically acceptable salt thereof,

$Ab-(L-D)_n$

Formula 3;

wherein, Ab is a ligand; L is a linker moiety; D is a drug moiety;
n is any integer or decimal from 1 to 15; preferably, n is any integer or decimal from 1 to 13;preferably, n is any integer or decimal from 3 to 10;
wherein the drug moiety D is selected from the following structures:

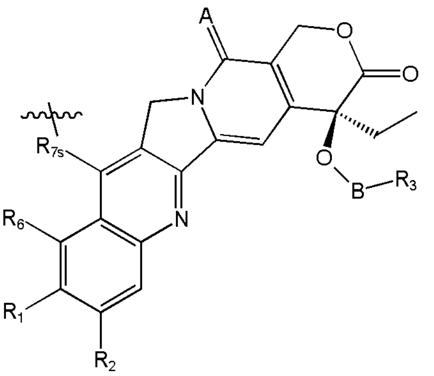

Formula 4-1 ,

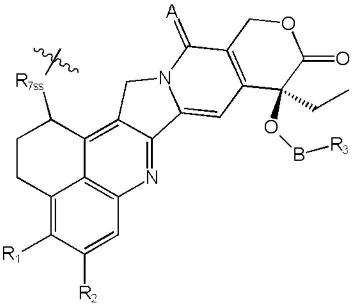

Formula 4-5 ,

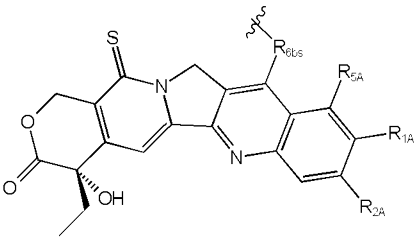

Formula 5-1 ,

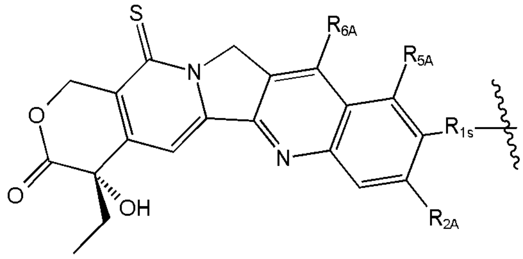

Formula 5-3 ,

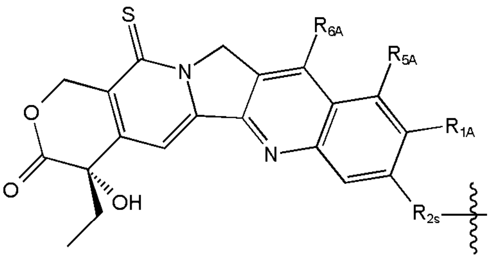

Formula 5-4 , and

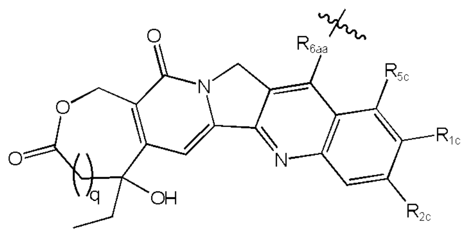

Formula 7-1 ;

wherein,
$R_1$, $R_2$, $R_3$, $R_6$, $R_7$, A, B, $R_{1c}$, $R_{2c}$, $R_{5c}$, $R_{1A}$, $R_{2A}$, $R_{3A}$, $R_{4A}$, and q as defined in any one of claims 2, 3, or 4;
$R_{6A}$ is selected from hydrogen, $-CH(CH_3)N(R_{3A})(R_{4A})$, and $-CH_2N(R_{3A})(R_{4A})$;
$R_{5A}$ is hydrogen or $C_{1-6}$ alkyl;
$R_{1s}$, $R_{2s}$, $R_{7s}$, and $R_{7ss}$ are each independently $C_{1-8}$ alkylene-NR-, $C_{1-8}$ haloalkylene NR-, -OR, -SR, -NR-, -C(O)NR-, $-SO_2NR$-, or $-O-CH_2C(O)NR-$; preferably, $R_{1s}$, $R_{2s}$, $R_{7s}$, and $R_{7ss}$ are each independently -NR-, $-NR-CH_2-$, or $-O-CH_2C(O)NR-$; preferably, $R_{1s}$, $R_{2s}$, $R_{7s}$, and $R_{7ss}$ are each independently -NH-, $-NH-CH_2-$, or $-O-CH_2C(O)NH-$;
each occurence of $R_{6bs}$ is independently selected from $-CH_2N(R_{a1})-$, $-CH_2N(R_{a1})-C_{2-5}$ alkylene-O-, $-CH_2N(R_{a1})C(O)C(R_{a1})(R_{b1})O-$, $-CH_2N(R_{a1})C(S)C(R_{a1})(R_{b1})O-$, - $CH_2N(R_{a1})C(O)-C_{3-8}$ cycloalkylene-O-, $-CH_2N(R_{a1})C(O)-C_{2-5}$alk-

ylene-O-, - $CH_2N(R_{a1})C(O)C(R_{a1})(R_{b1})N(R_{a1})$-, -$CH_2N(R_{a1})C(O)$-$C_{3-8}$ cycloalkylene -$C(R_{a1})(R_{b1})$-O-, -$C_{2-5}$ alkylene-O-, and -$CH_2N(R_{a1})C(O)O$-$C_{2-5}$ alkylene-O-;

wherein each occurrence of R, $R_{a1}$ and $R_{b1}$ are independently selected from hydrogen, protium, deuterium, tritium, halogen, -$NO_2$, -CN, -OH, -SH, -$NH_2$, -C(O)H, -$CO_2$H, -C(O)C(O)H, -C(O)$CH_2$C(O)H, -S(O)H, -S$(O)_2$H, -C(O)$NH_2$, -$SO_2NH_2$, - OC(O)H, -N(H)$SO_2$H, $C_{1-6}$ alkyl, $C_{2-6}$ alkenyl, $C_{2-6}$ alkynyl, 3-, 4-, 5-, 6-, 7-, or 8-membered cycloalkyl, 3- to 8-membered heterocyclyl containing 1, 2, or 3 heteroatoms independently selected from N, O, and S, 6- to 10-membered aryl, and 5- to 10-membered heteroaryl containing 1, 2, or 3 heteroatoms independently selected from N, O, and S; wherein the $C_{1-6}$ alkyl, $C_{2-6}$ alkenyl, $C_{2-6}$ alkynyl, 3-, 4-, 5-, 6-, 7-, or 8-membered cycloalkyl, 3- to 8-membered heterocyclyl, 6- to 10-membered aryl, or 5- to 10-membered heteroaryl is unsubstituted or substituted with one or more substituents independently selected from halogen, -$NO_2$, -CN, -OH, -$NH_2$, -C(O)H, -$CO_2$H, - C(O)C(O)H, -C(O)$CH_2$C(O)H, -S(O)H, -S$(O)_2$H, -C(O)$NH_2$, -$SO_2NH_2$, -OC(O)H, - N(H)$SO_2$H, $C_{1-6}$ alkyl, $C_{2-6}$ alkenyl, $C_{16}$ alkynyl, and hydroxyl-$C_{1-6}$ alkylene; and

$R_{6aa}$ is selected from - $C_{1-8}$ alkylene- N($R_{a2}$)-, -$CH_2N(R_{a2})C(O)C(R_{a2})(R_{b2})$O-, - $CH_2N(R_{a2})C(O)$-$C_{3-8}$ cycloalkylene-O-, -$C_{2-5}$ alkylene-O-, and -$CH_2N(R_{a2})C(O)O$-$C_{2-5}$ alkylene-O-; preferably, $R_{6aa}$ is -$CH_2$-$NH_2$, or -$(CH_2)_4$-O-;

wherein, each occurrence of $R_{a2}$ and R $_{b2}$ are independently hydrogen, protium, deuterium, tritium, halogen, --$NO_2$, -CN, -OH, -SH, -$NH_2$, -C(O)H, -$CO_2$H, - C(O)C(O)H, -C(O)$CH_2$C(O)H, -S(O)H, -S$(O)_2$H, -C(O)$NH_2$, -$SO_2NH_2$, -OC(O) H, - N(H)$SO_2$H, or $C_{1-6}$ alkylene-hydroxyl; preferably, each occurrence of $R_{a2}$ and $R_{b2}$ are independently hydrogen, F, Cl, Br, I, -$CH_2$OH, and -$C_2H_4$OH.

In some embodiments, the drug moiety D is selected from the following structures:

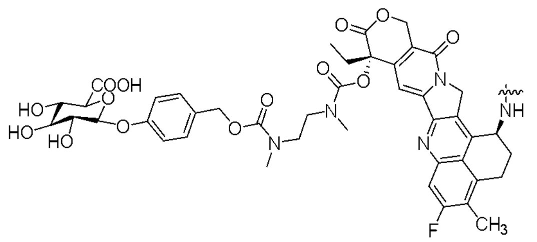

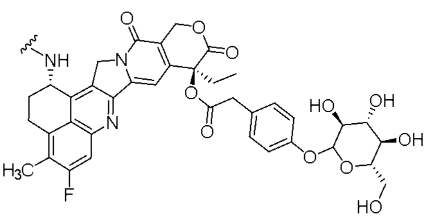

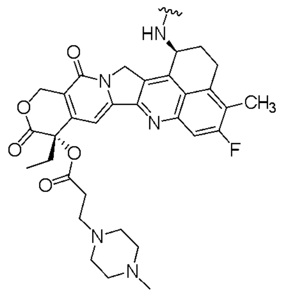

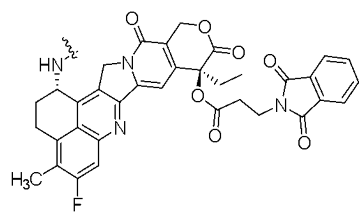

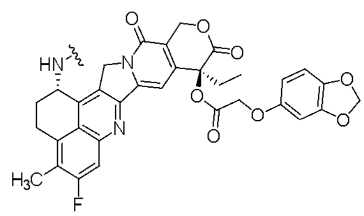

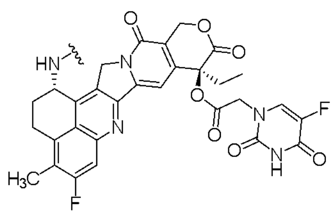

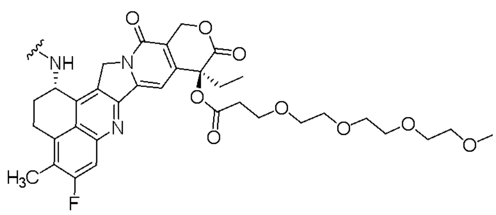

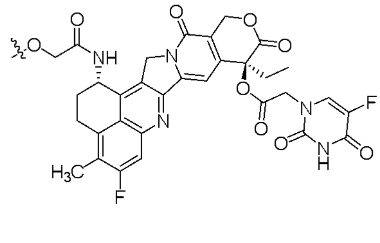

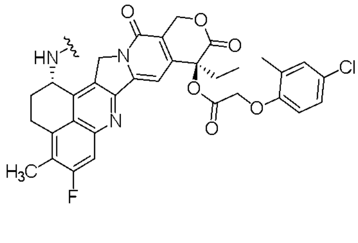

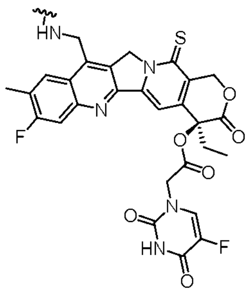

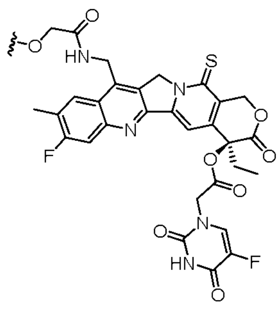

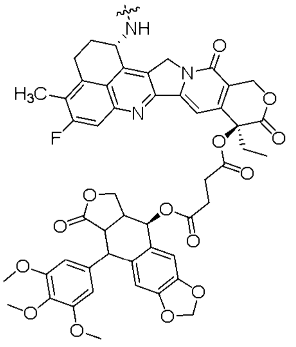

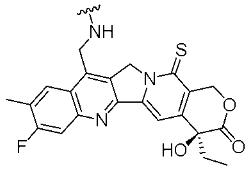

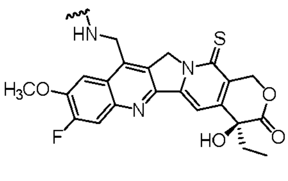

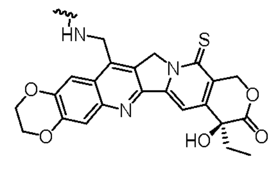

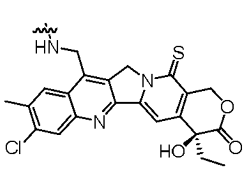

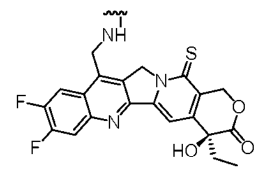

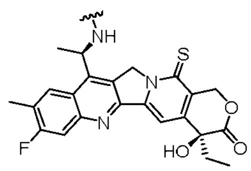

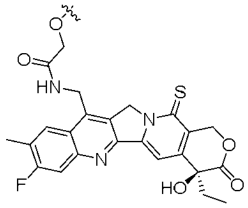

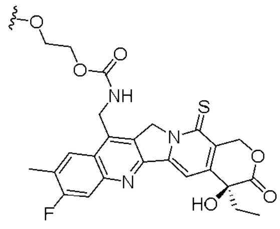

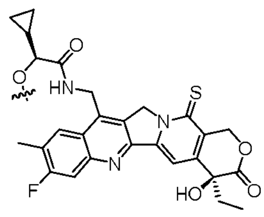

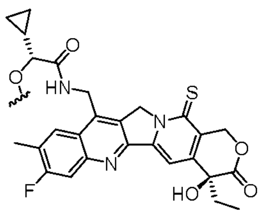

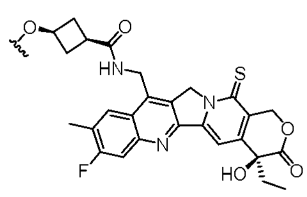

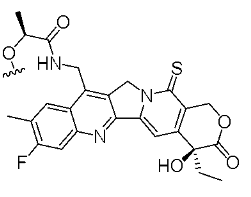

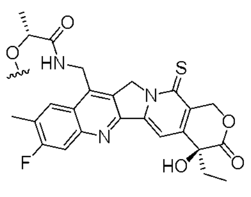

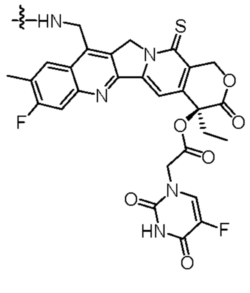

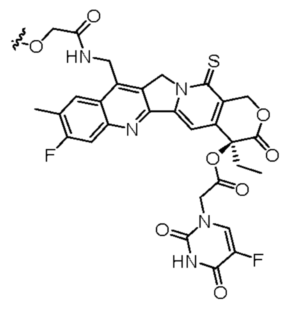

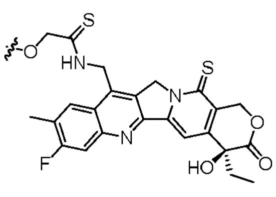

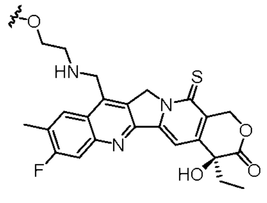

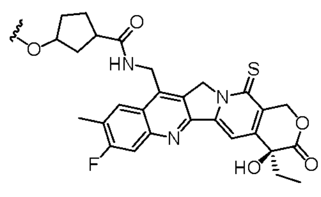

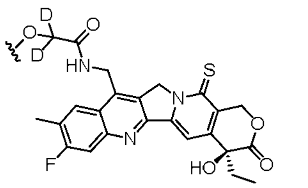 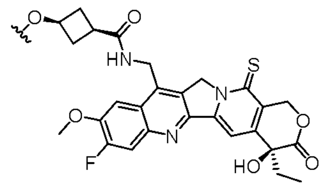 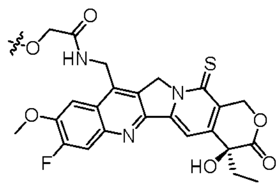

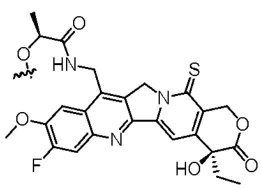 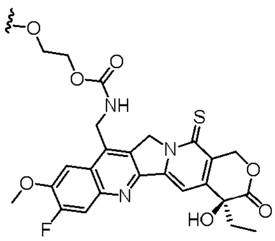 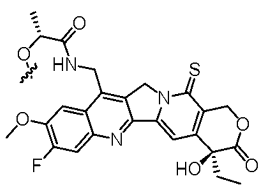 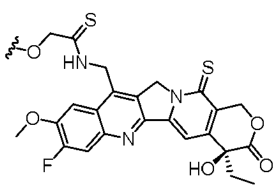

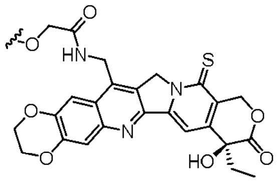 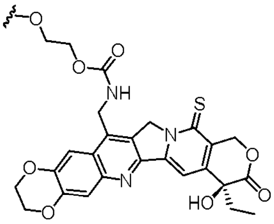 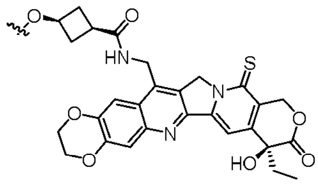

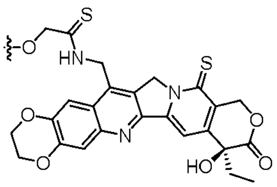 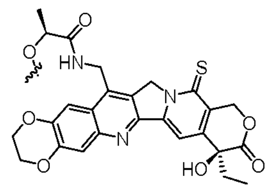 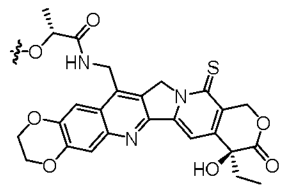

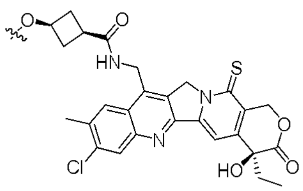 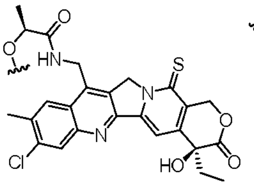 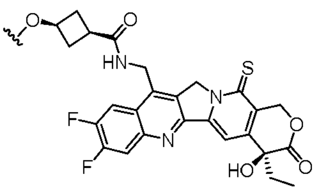 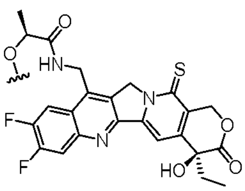

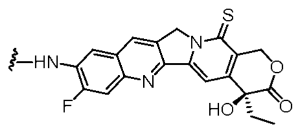

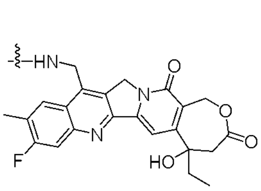 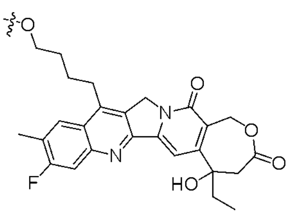 and 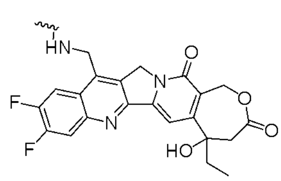 ,

wherein

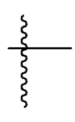

represents the attachment point.

In some embodiments, the linker moiety L is $L^1$-$L^2$-$L^3$-$L^4$;

$L^1$ is selected from -(succinimidyl-3-yl-N)-, -(succinimidyl-3-yl-N)-W-C(=O)-,

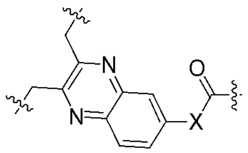, 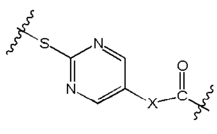, 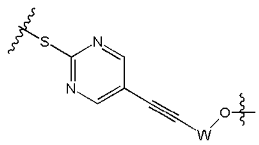, 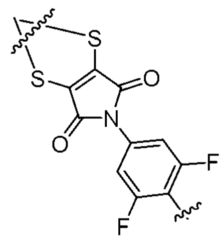,

and

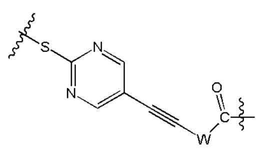;

wherein, W is selected from $C_{1-10}$ alkylene, $C_{1-10}$ alkylene-cycloalkylene, $C_{1-10}$ heteroalkylene, $C_{1-10}$ alkylene-heterocyclylene, or $C_{1-10}$ heteroalkylene-cycloalkylene; preferably, W is $C_{1-8}$ alkylene, $C_{1-8}$ alkylene-cycloalkylene, or $C_{1-8}$ heteroalkylene, the heteroalkylene contains 1 to 3 heteroatoms independently selected from N, O, and S; wherein the alkylene, cycloalkylene, and heteroalkylene are unsubstituted or each independently optionally further substituted with one or more substituents selected from halogen, hydroxyl, -CN, amino, alkyl, haloalkyl, deuterated alkyl, alkoxy, and cycloalkyl; preferably, the alkylene, cycloalkylene, and heteroalkylene are unsubstituted or each independently optionally further substituted with one or more substituents selected from halogen, hydroxyl, -CN, amino, $C_{1-6}$ alkyl, $C_{1-6}$ haloalkyl, deuterated $C_{1-6}$ alkyl, $C_{1-6}$ alkoxy, and $C_{5-8}$ cycloalkyl; more preferably, the alkylene, cycloalkylene, and heteroalkylene are unsubstituted or optionally substituted with one or more substituents selected from halogen, -OH, -CN, $C_{1-3}$ alkyl, and -$OC_{1-3}$ alkyl; preferably, the alkylene, cycloalkylene, and heteroalkylene are unsubstituted or optionally substituted with one or more substituents selected from Cl, Br, F, -OH, -CN, methyl, and -$OCH_3$;

X is selected from a single bond, $C_{1-10}$ alkylene, $C_{1-10}$ alkylene-cycloalkylene, $C_{1-10}$ heteroalkylene, $C_{1-10}$ alkylene-heterocyclylene, or $C_{1-10}$ heteroalkylene-cycloalkylene; preferably, X is selected from a single bond, $C_{1-8}$ alkylene, $C_{1-8}$ alkylene-cycloalkylene, and $C_{1-8}$ heteroalkylene;

$L^2$ is selected from -$(CH_2CH_2O)_rCH_2CH_2C$(=O)-, -$(CH_2CH_2O)_rC$(=O)-, - $NR_{1L}(CH_2CH_2O)_rC$(=O)-, -$(CH_2CH_2O)_rCH_2C$(=O)-, - $NR_{1L}(CH_2CH_2O)_rCH_2CH_2C$(=O)-, -$NR_{1L}(CH_2CH_2O)_rCH_2C$(=O)-, -$NR_{1L}CH_2$-$Ar^1$-$(CH_2CH_2O)_rCH_2CH_2NR_{1L}C$(=O)$CH_2OCH_2C$(=O)-, -$NR_{1L}(CH_2CH_2O)_r$ $CH_2$-$Ar^1$-$(CH_2CH_2O)_r$ $CH_2CH_2C$(=O)-, -$S(CH_2)_rC$(=O)-, -O-$(CH_2CH_2O)_rCH_2$-$Ar^1$-$(CH_2CH_2O)_r$ $CH_2CH_2C$(=O)-,

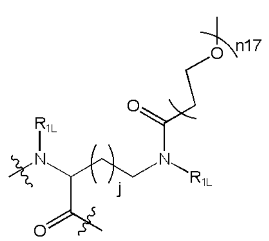,

or a single bond, wherein r is an integer selected from 12, 3, 4, 5, 6, 7, 8, 9, 10, 11, 12, 13, 14, 15, 16, 17, 18, 19, or 20; each occurrence of n17 is independently an integer selected from 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30; each occurrence of j is independently selected from 0, 1, 2, 3, 4, 5, and 6; preferably, r is an integer selected from 1, 2, 3, 4, 5, 6, 7, or 8;

$Ar^1$ is selected from 6- to 10-membered aryl, 5- to 10-membered heteroaryl containing 1 to 3 heteroatoms independently selected from N, O, P, and S, 3- to 10-membered cycloalkyl, and 3- to 10-membered heterocyclyl containing 1 to 3 heteroatoms independently selected from N, O, P, and S; preferably, $Ar^1$ is selected from

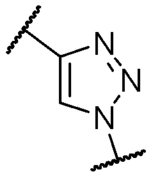, 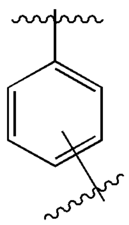, 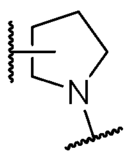, 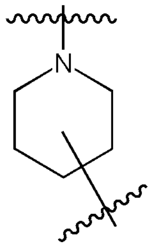, 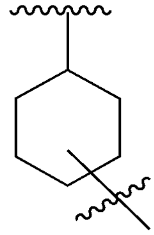,

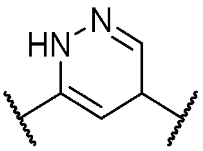, 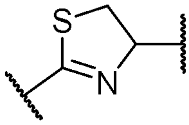, 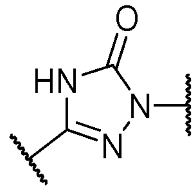, 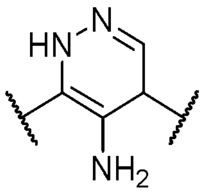,

and

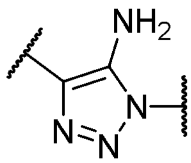;

$L^3$ is a peptide residue consisting of 2 to 7 amino acids, preferably $L^3$ is a peptide residue consisting of 2, 3, 4, 5, or 6 amino acids, wherein the amino acids are unsubstituted or optionally further substituted with one or more substituents selected from halogen, hydroxyl, -CN, amino, alkyl, haloalkyl, deuterated alkyl, alkoxy, and cycloalkyl; preferably, optionally further substituted with one or more substituents selected from halogen, hydroxyl, -CN, amino, $C_{1-6}$ alkyl, $C_{1-6}$ haloalkyl, deuterated $C_{1-6}$ alkyl, $C_{1-6}$ alkoxy, and $C_{5-8}$ cycloalkyl.

$L^4$ is selected from $-NR_{2L}(CR_{3L}R_{4L})_t-Z-(CR_{3L}R_{4L})_t-C(=O)-$, $-NR_{2L}(CR_{3L}R_{4L})_t-$, $-NR_{2L}(CR_{3L}R_{4L})_t-Z-(CR_{3L}R_{4L})_t-Z-C(=O)-$,

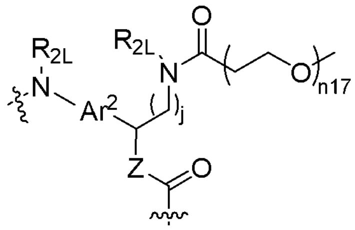,

$-NR_2-Ar^2-(CR_{3L}R_{aL})_t-Z-C(=O)-$, or a single bond, wherein each occurrence of t is independently an integer of 0, 1, 2, 3, 4, 5, or 6; each occurrence of Z is independently a single bond, O, S, or -NH-; $Ar^2$ is arylene or heteroarylene, preferably selected from 6-membered arylene or 5- to 8-membered heteroarylene, wherein the heteroarylene containing 1, 2, or 3 heteroatoms independently selected from N, O, and S; the arylene or heteroarylene is unsubstituted or optionally substituted with one or more substituents selected from H, halogen, -OH, -CN, $C_{1-6}$ alkyl, $-OC_{1-6}$ alkyl, $-NH_2$, $-NH(C_{1-6}$ alkyl), and $-N(C_{1-6}$ alkyl$)_2$;

$R_{1L}$ and $R_{2L}$ are the same or different and are independently at each occurrence selected from hydrogen, halogen, $C_{1-6}$ alkyl, $C_{1-6}$ haloalkyl, deuterated $C_{1-6}$ alkyl, and $C_{1-6}$ alkylene-OH;

$R_{3L}$ and $R_{4L}$ are the same or different and are independently at each occurrence selected from hydrogen, halogen, $C_{1-6}$ alkyl, $C_{1-6}$ haloalkyl, deuterated $C_{1-6}$ alkyl, and - $C_{1-6}$ alkylene-OH; and

the $L^1$ end is attached to the ligand, and the $L^4$ end is attached to the drug moiety.

In some embodiments, $L^3$ is a peptide residue consisting of 2 to 6 amino acids selected from glycine, phenylalanine, alanine, valine, lysine, citrulline, serine, glutamic acid, and aspartic acid; preferably a dipeptide residue, tripeptide residue, or tetrapeptide residue selected from alanine, phenylalanine, glycine, lysine, and citrulline; preferably,

$L^3$ is a peptide residue selected from:glycine-phenylalanine-glycine, alanine-alanine-alanine-glycine, alanine-alanine-alanine, glycine-glycine-phenylalanine-glycine, valine-citrulline, and valine-alanine;

Wherein, the peptide residue is unsubstituted or optionally further substituted with one or more substituents selected from halogen, hydroxyl, -CN, amino, alkyl, haloalkyl, deuterated alkyl, alkoxy, and cycloalkyl; preferably, optionally further substituted with one or more substituents selected from halogen, hydroxyl, -CN, amino, and $C_{1-6}$ alkyl.

In some embodiments, the linker moiety L is selected from:

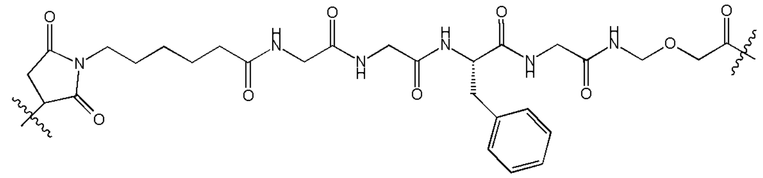

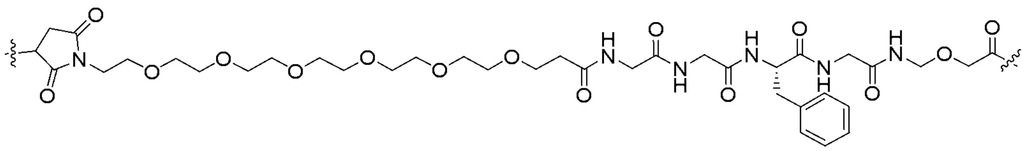

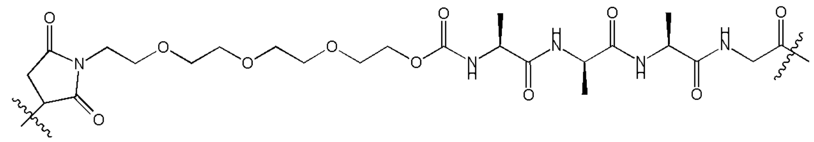

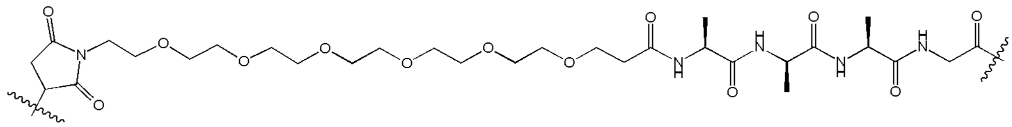

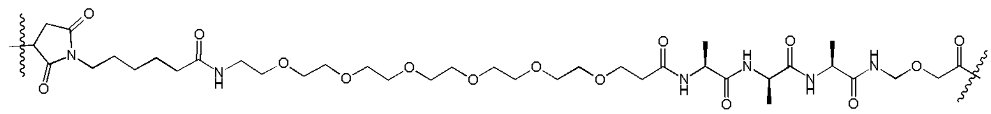

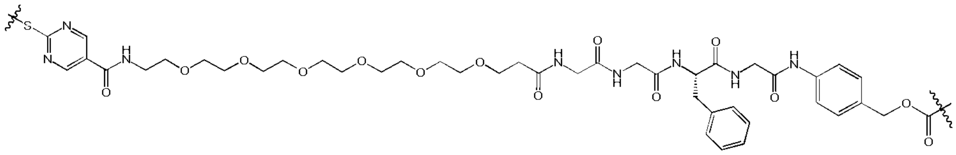

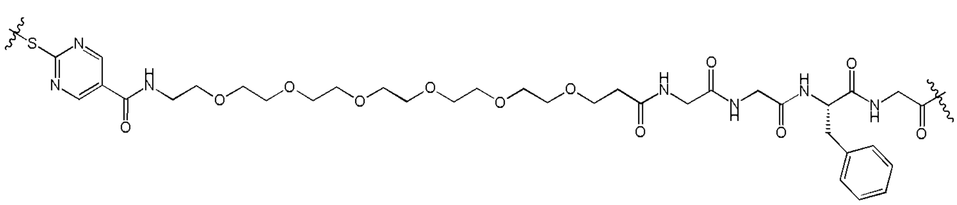

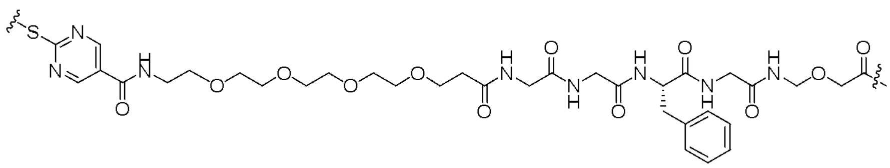

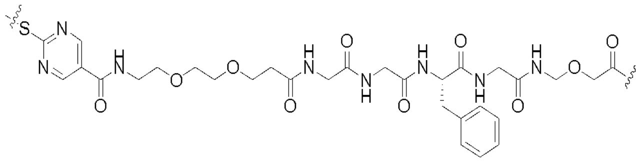

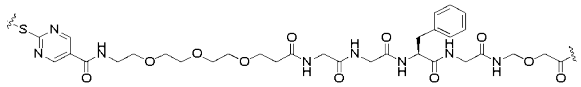

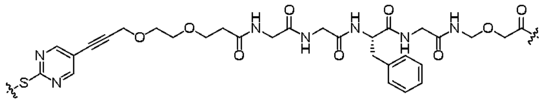

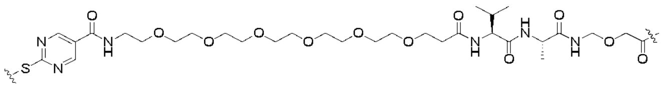

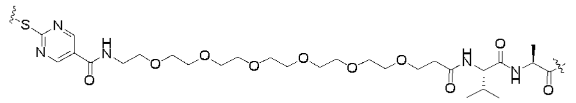

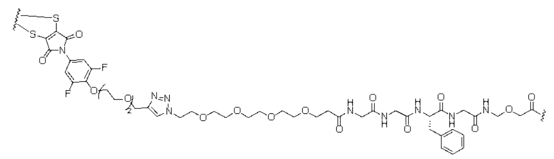

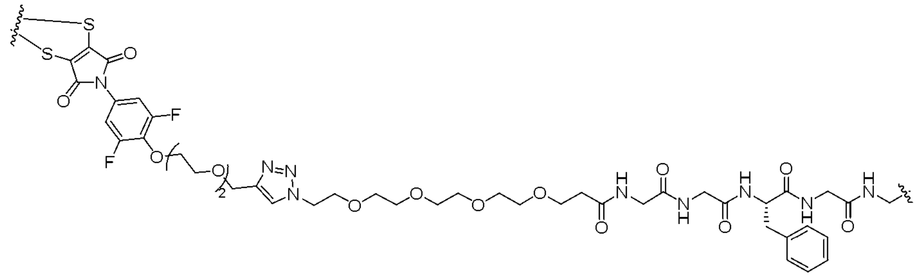

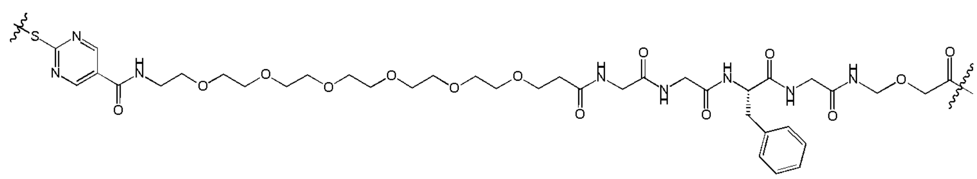

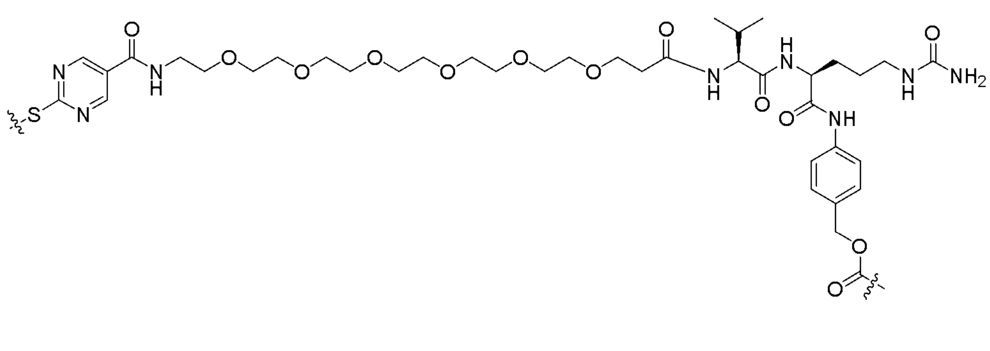

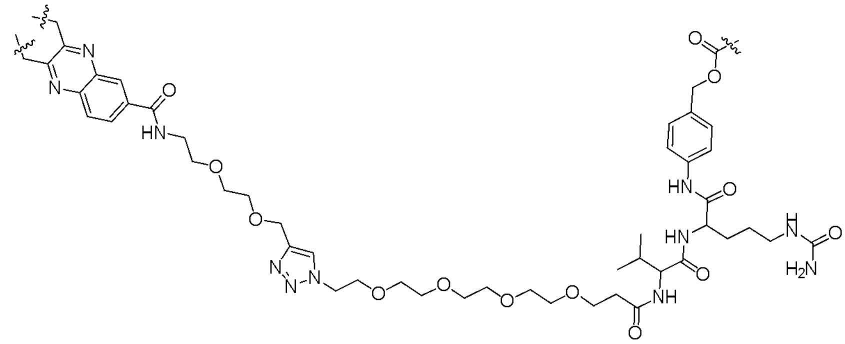

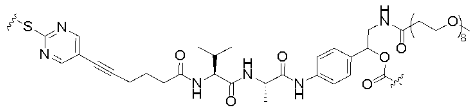

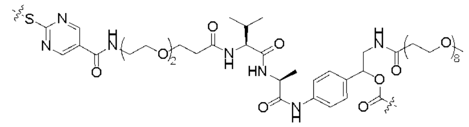

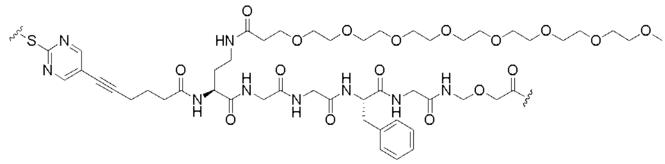

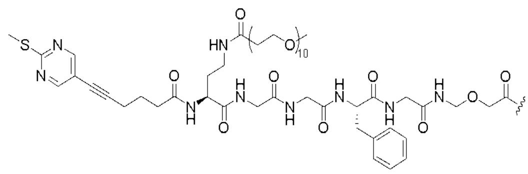

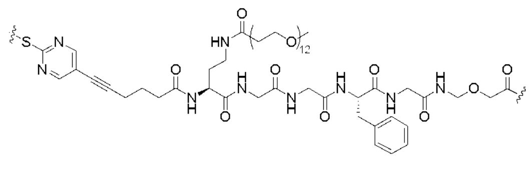

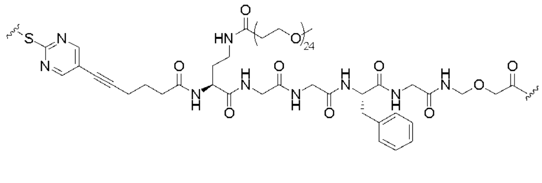

and

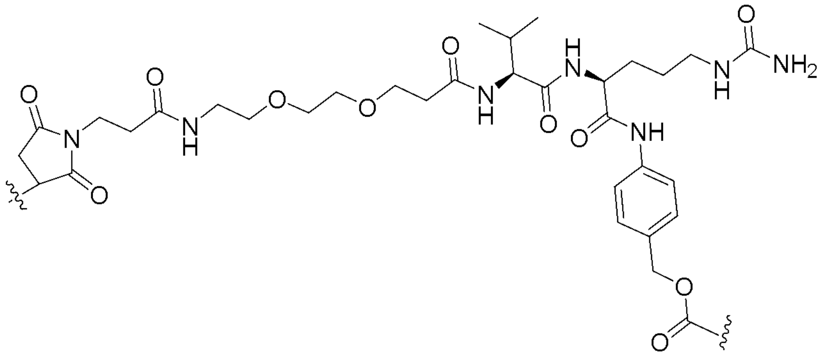

,

wherein

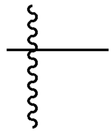

represents the attachment point.

In some embodiments, the ligand-drug conjugate is selected from the following structures:

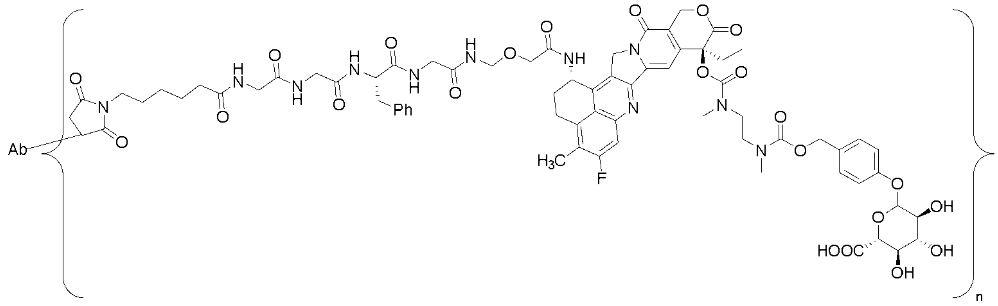

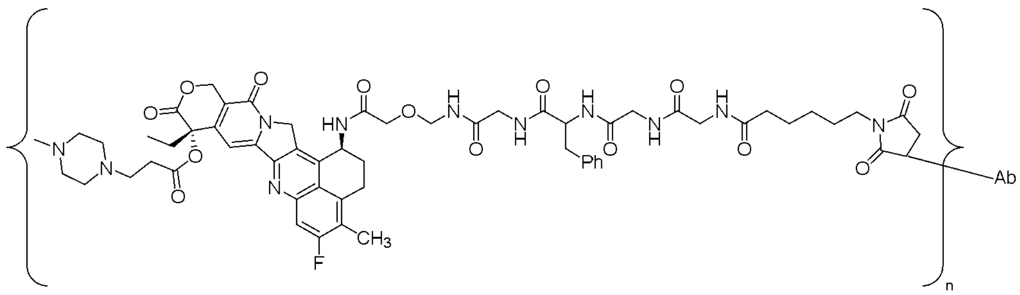

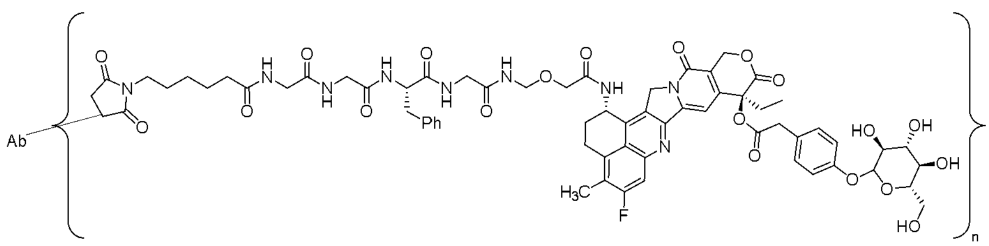

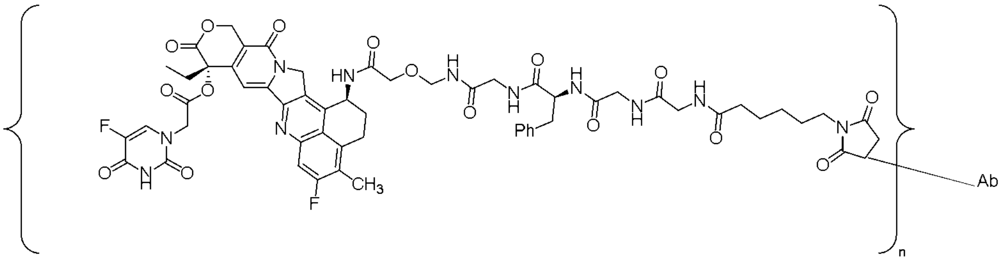

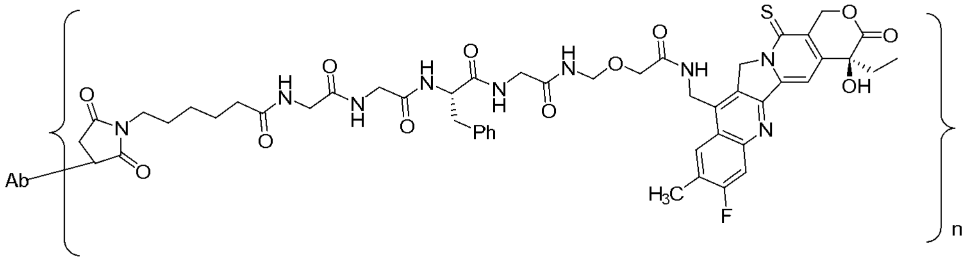

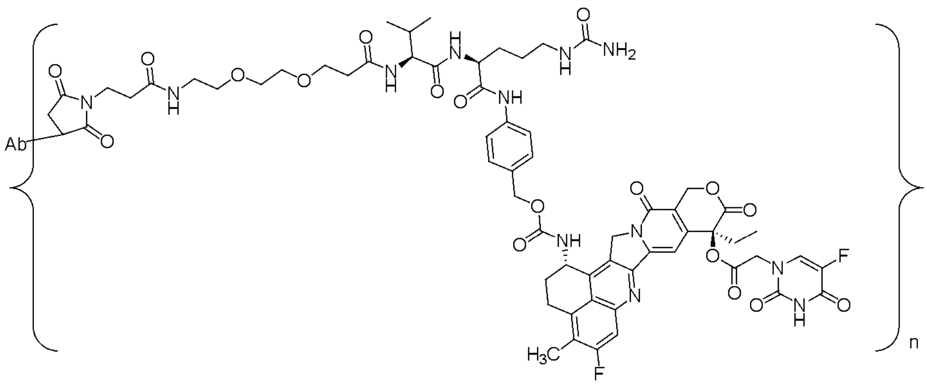

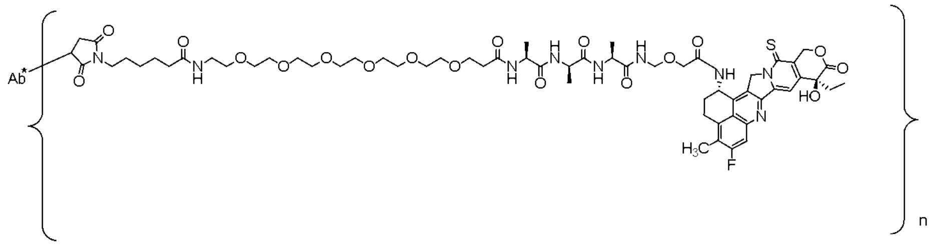

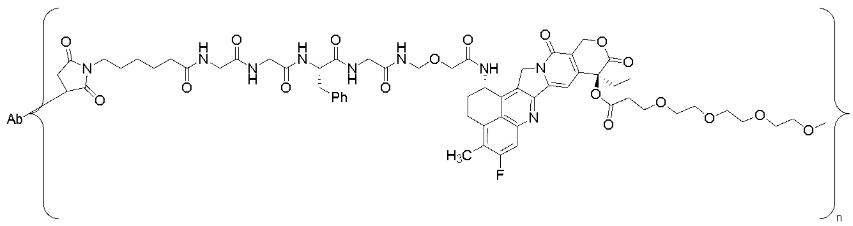

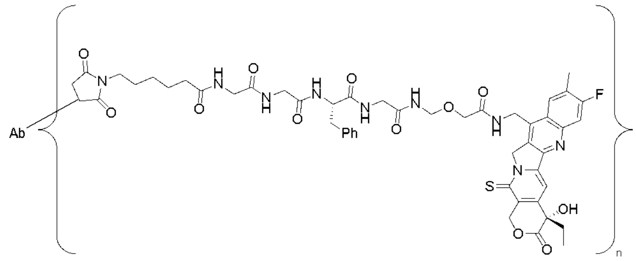

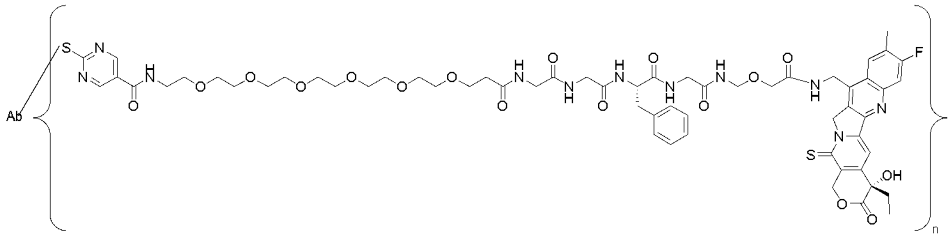

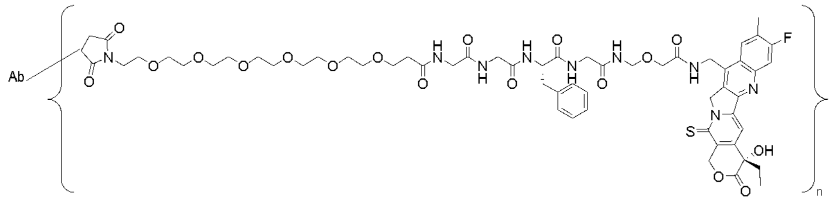

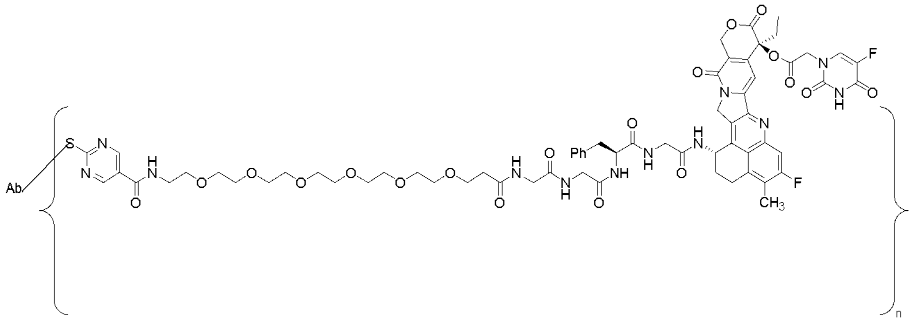

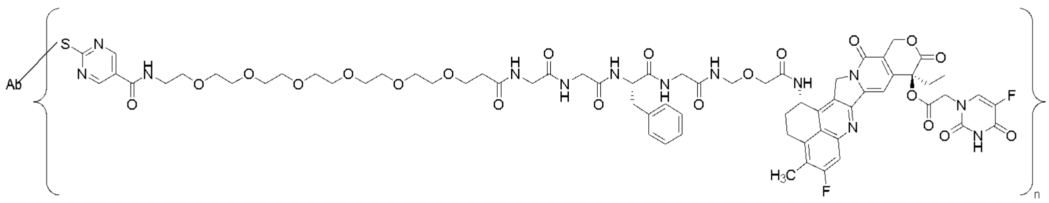

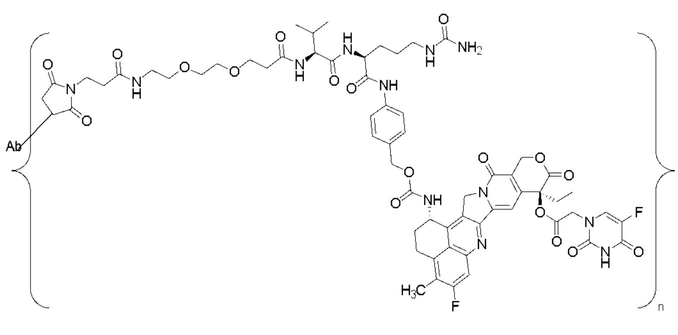

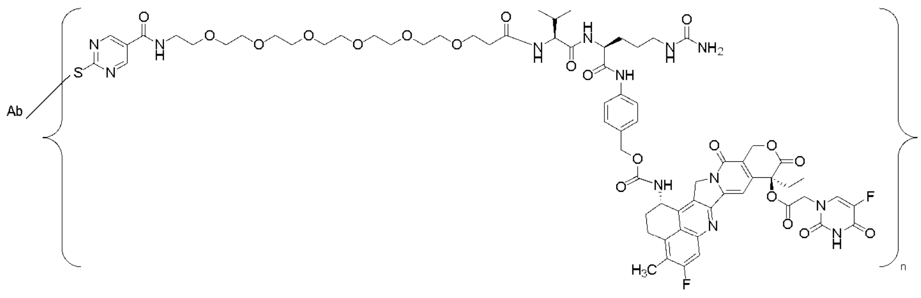

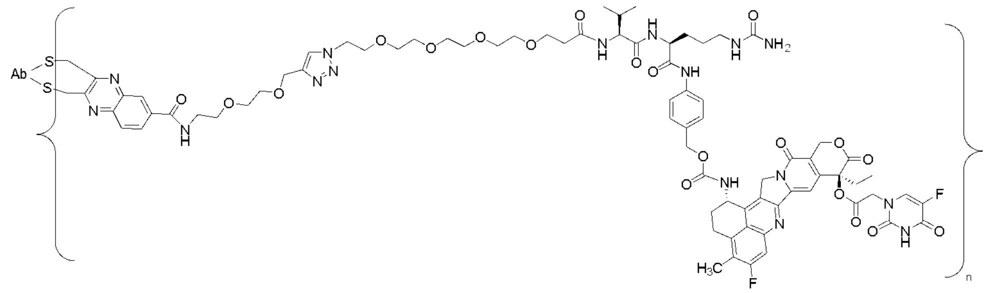

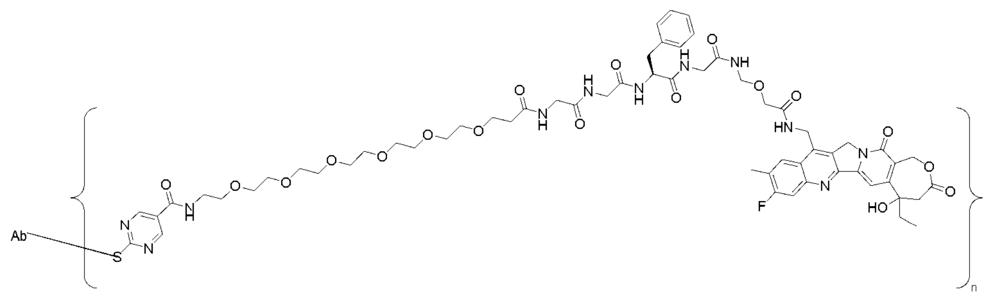

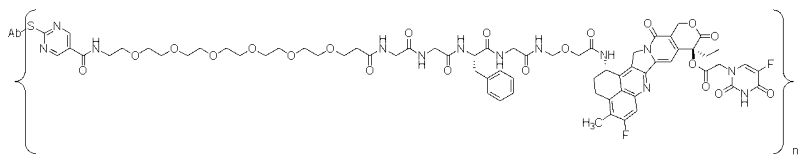

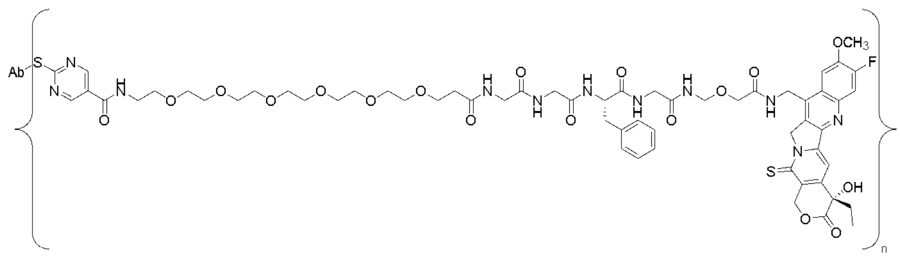

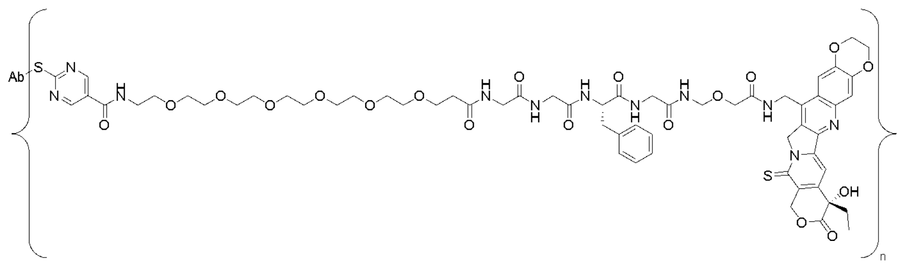

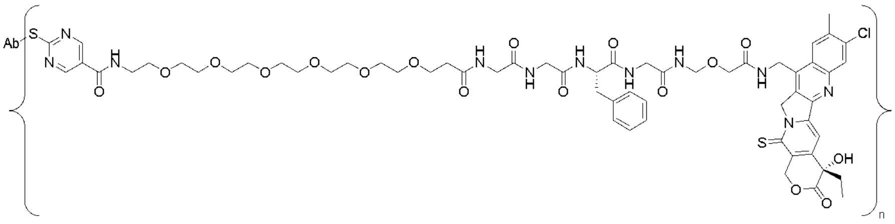

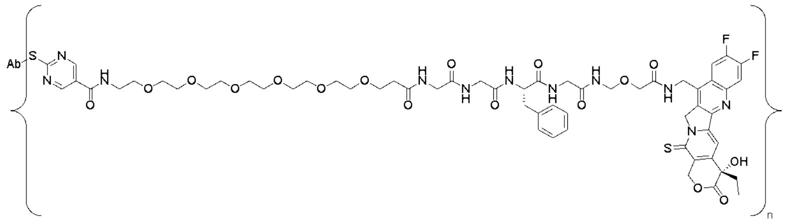

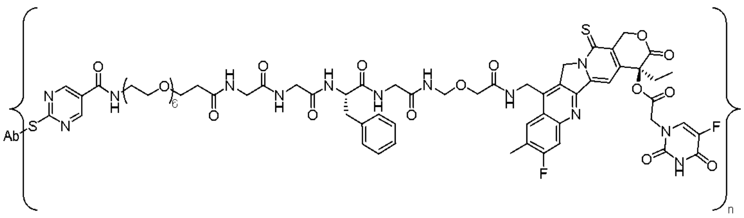

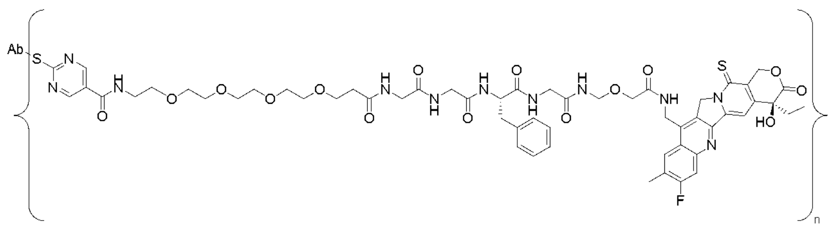

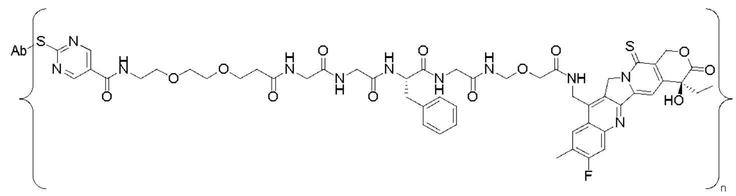

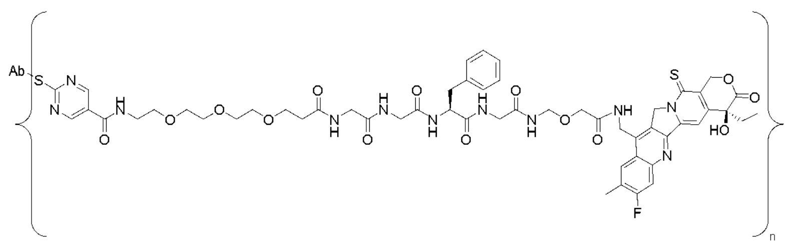

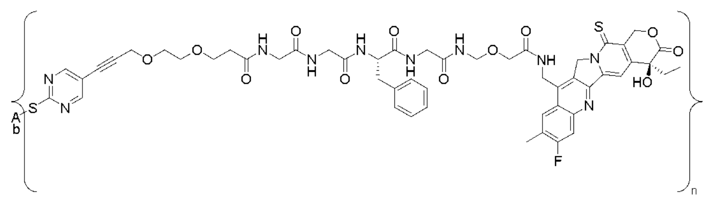

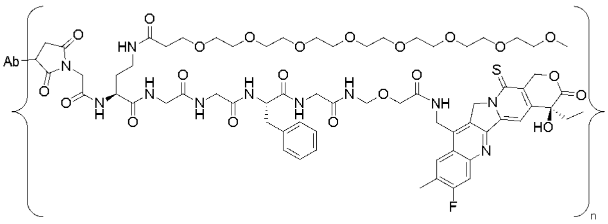

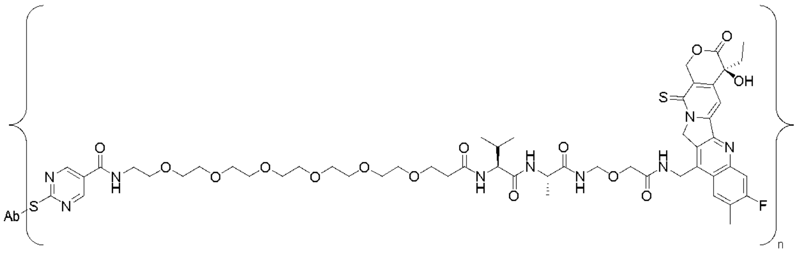

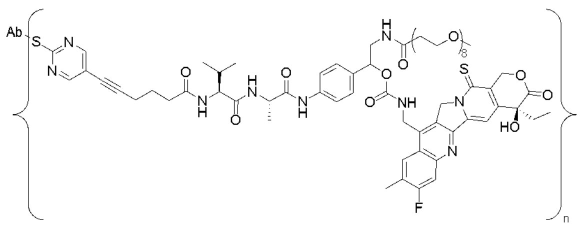

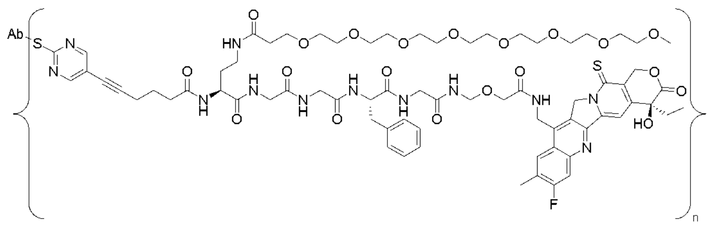

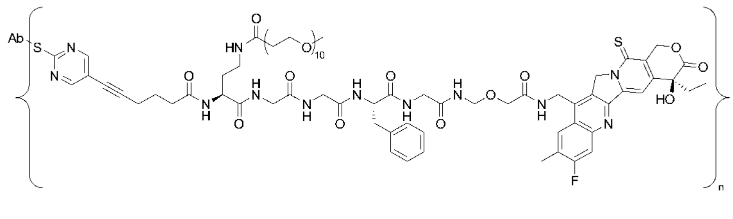

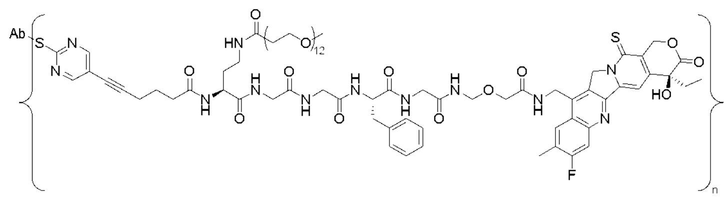

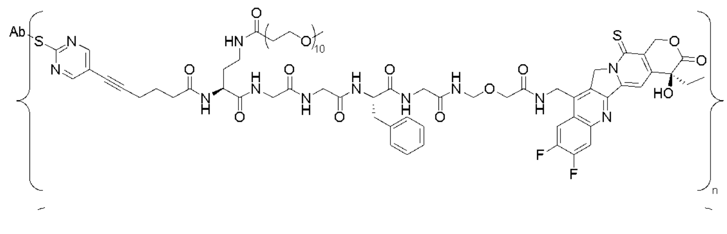

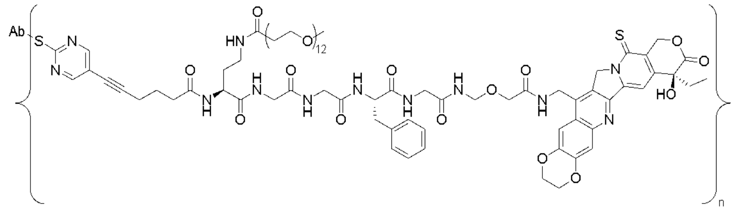

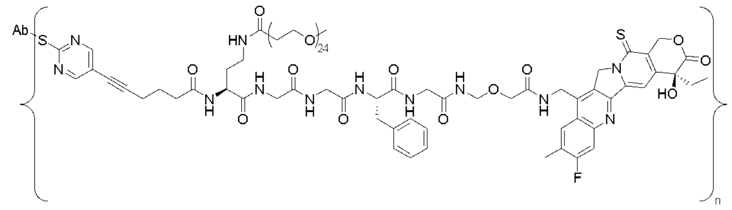

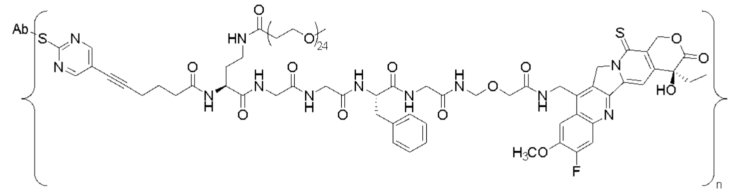

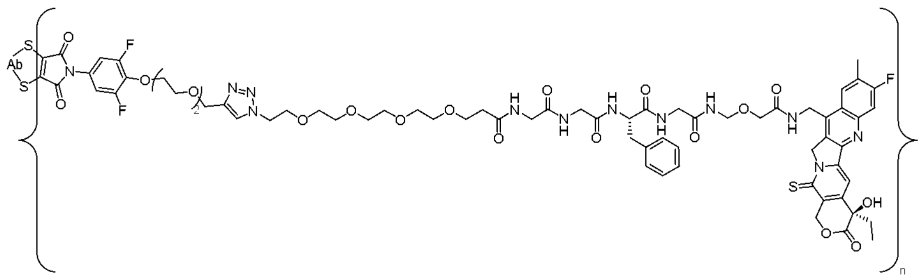

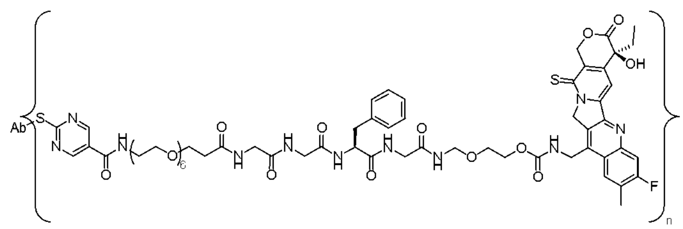

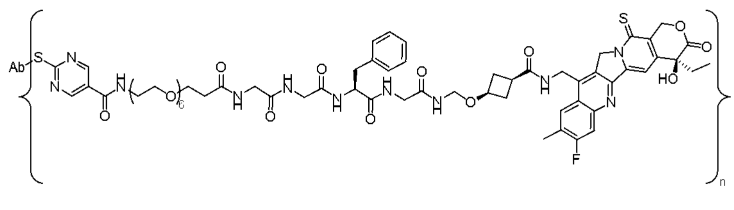

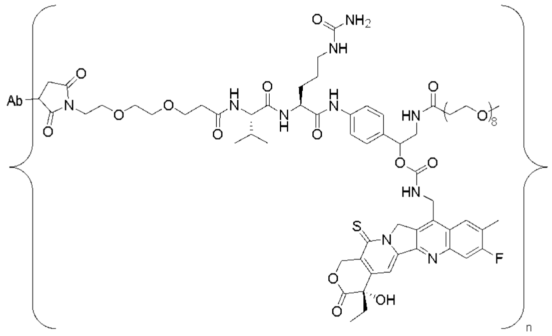

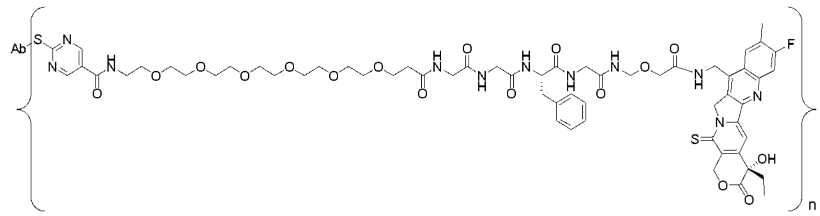

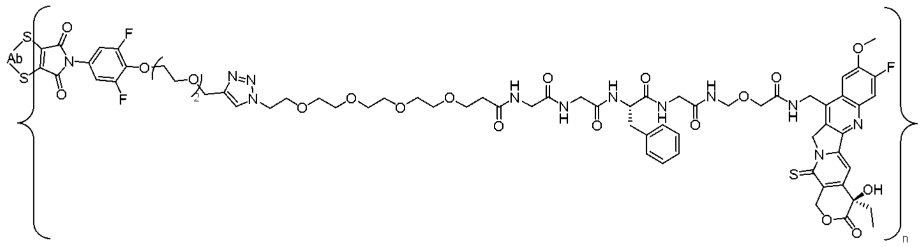

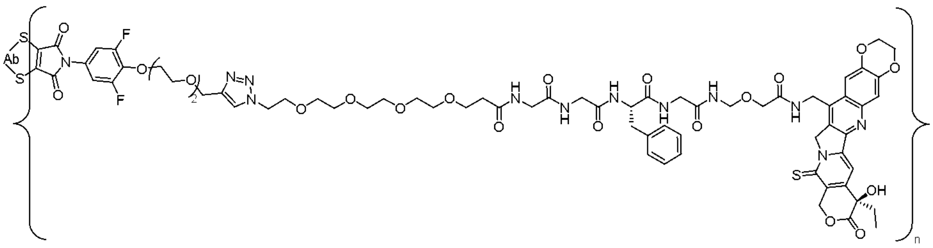

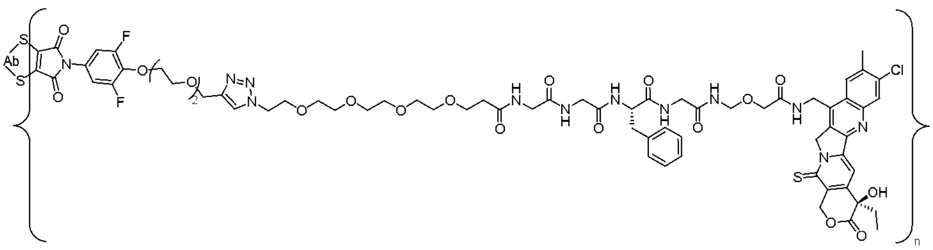

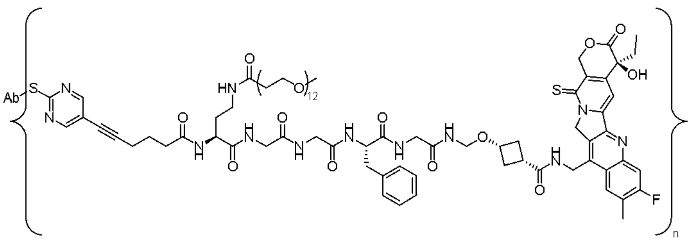

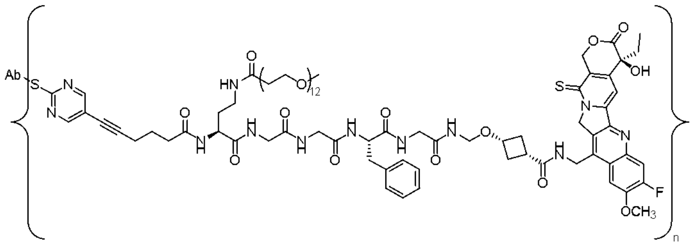

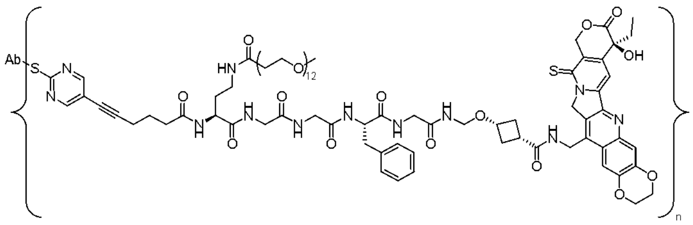

and

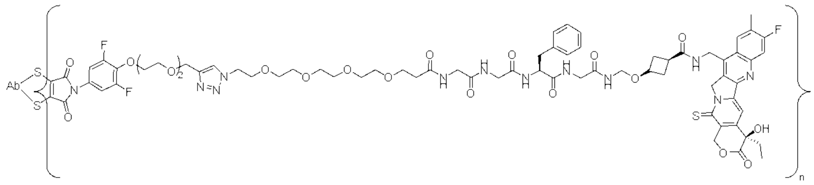

wherein, n is an integer or decimal from 1 to 10; preferably, n is an integer or decimal from 3 to 8; Ab is a ligand.

In some embodiments, the Ab is an antibody or an antigen-binding fragment thereof, or a polypeptide, wherein the antibody is selected from a chimeric antibody, a humanized antibody, and a fully human antibody;

preferably, the antibody or antigen-binding fragment thereof is selected from an anti-TROP-2 antibody, an anti-HER2 (ErbB2) antibody, an anti-NECTIN4 antibody, an anti-EGFR antibody, an anti-B7-H3 antibody, an anti-c-Met antibody, an anti-HER3 (ErbB3) antibody, an anti-HER4 (ErbB4) antibody, an anti-LIV-1 antibody, an anti-ROR1 antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody an anti-A33 antibody, an anti-Cripto antibody, an anti-EphA2 antibody, an anti-G250 antibody, an anti-MUC1 antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-Mesothelin antibody, or an antigen-binding fragment thereof;

preferably, the antibody or antigen-binding fragment thereof is an anti-TROP-2 antibody, an anti-NECTIN4 antibody, an anti-B7-H3 antibody, an anti-HER2 (ErbB2) antibody, an anti-HER3 (ErbB3) antibody, an anti-LIV-1 antibody, an anti-ROR1 antibody, or an antigen-binding fragment thereof;

preferably, the antibody or antigen-binding fragment thereof is an anti-HER2 (ErbB2) antibody, an anti-NECTIN4 antibody, an anti-B7-H3 antibody or an antigen-binding fragment thereof;

preferably, the antibody or antigen-binding fragment thereof is Trastuzumab, Ifinatamab, or PADCEV.

The third aspect of the present invention provides a compound having the structure represented by Formula 5, or a pharmaceutically acceptable salt thereof,

$L_j$- $L_2$-$L_3$ -$L_4$ -D (Formula 5) ;

wherein -$L_2$-, -$L_3$-, -$L_4$- and -D are as defined in the second aspect of the present invention ;

$L_j$ is selected from

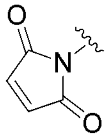 , 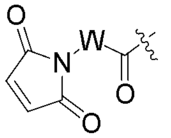 , 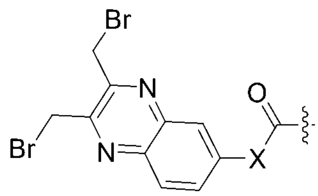 ,

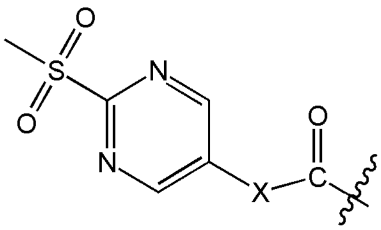 , 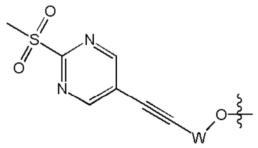 , 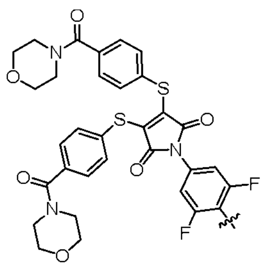 ,

and

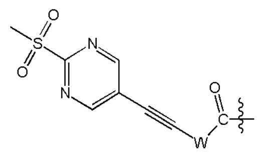 ;

W and X are as defined in the second aspect of the present invention.

In some embodiments, the compound is selected from the following structures:

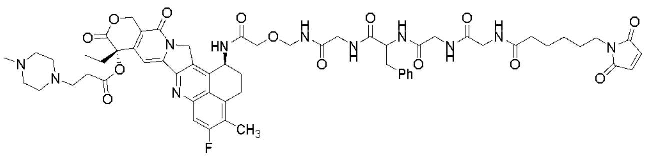

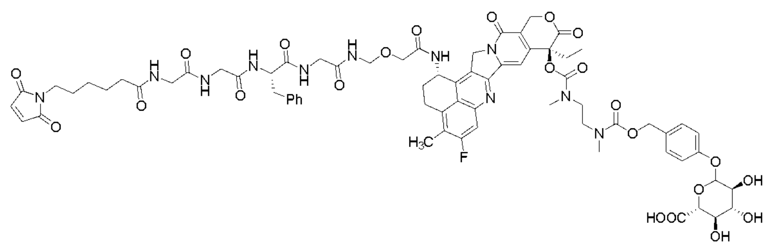

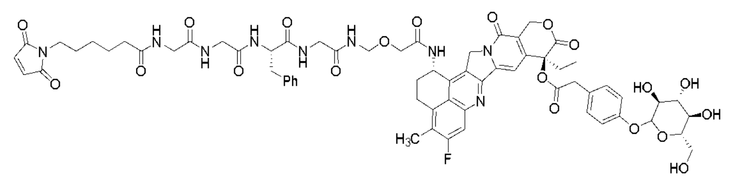

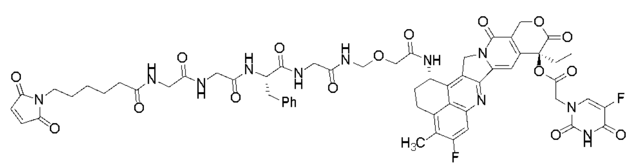

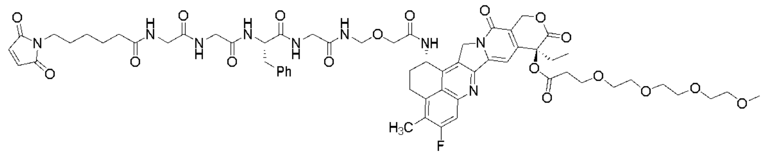

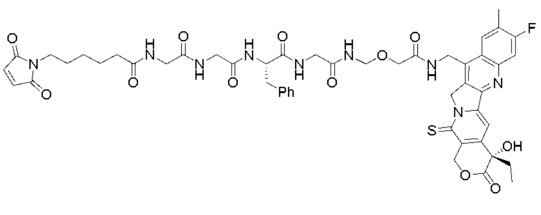

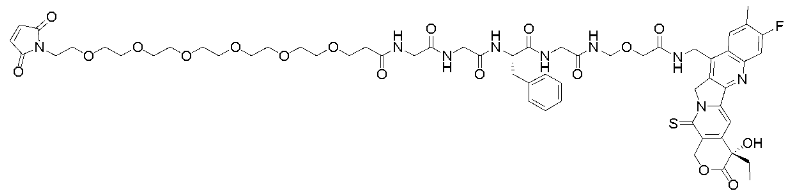

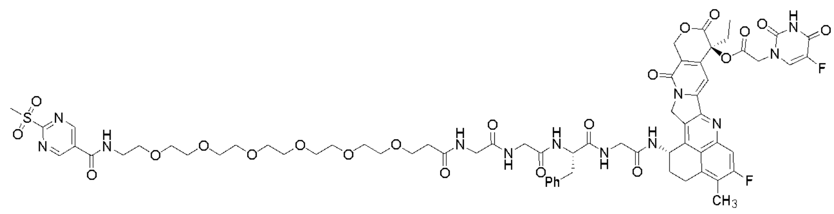

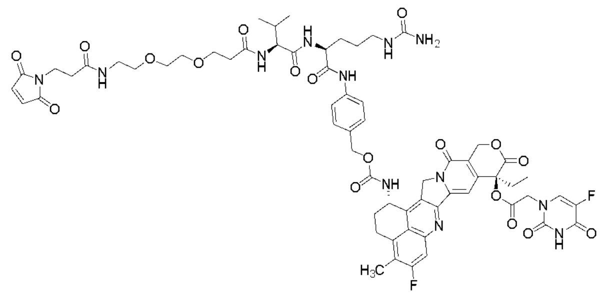

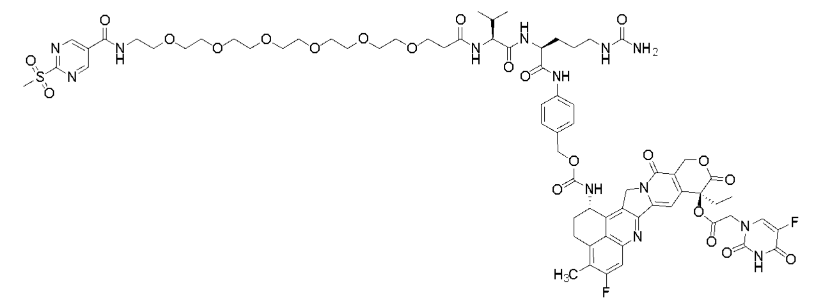

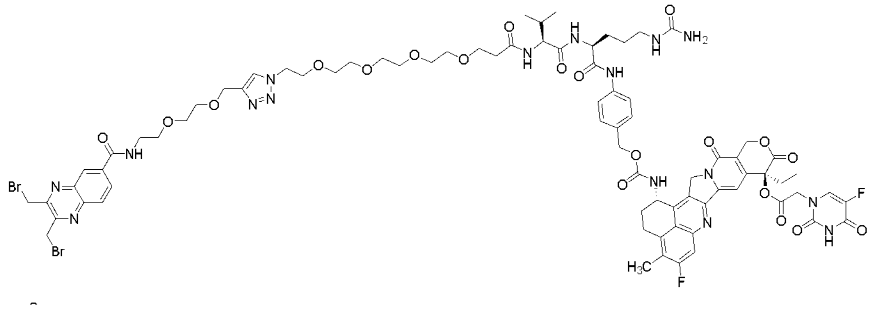

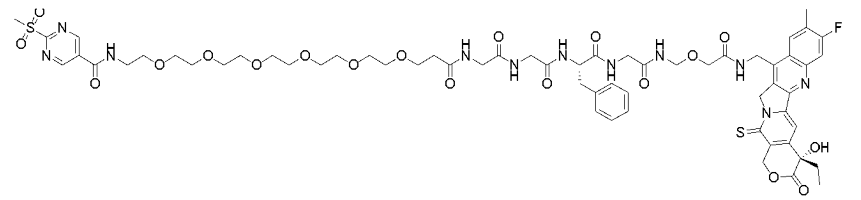

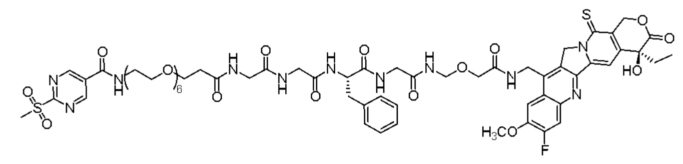

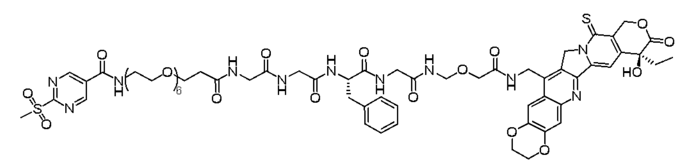

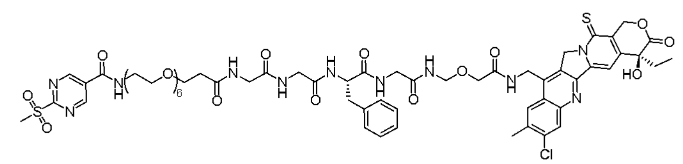

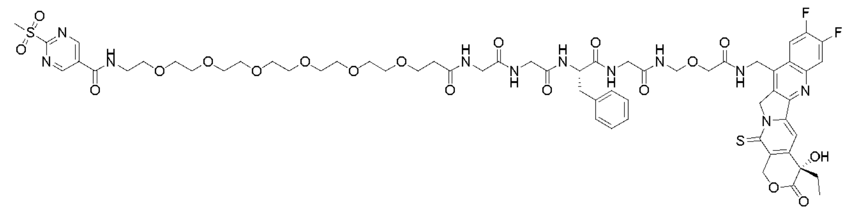

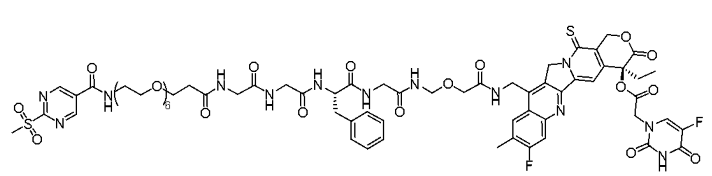

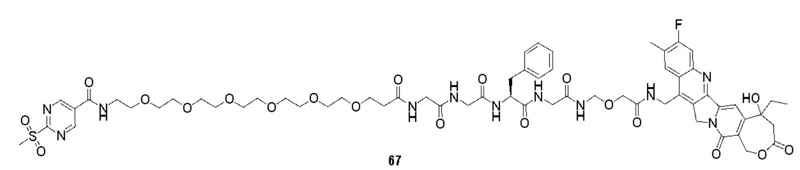

67

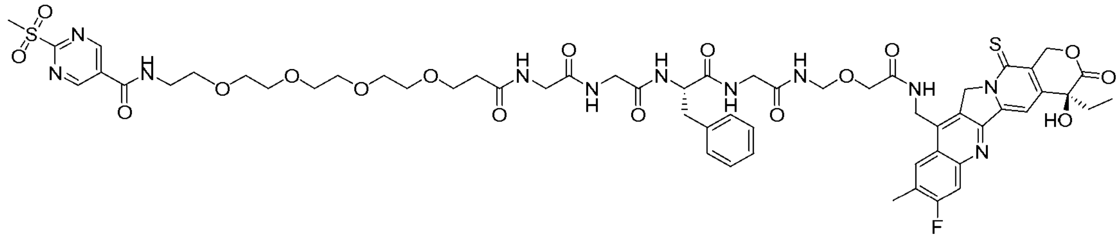

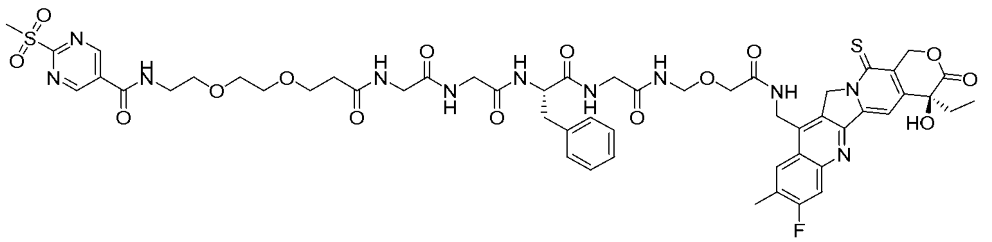

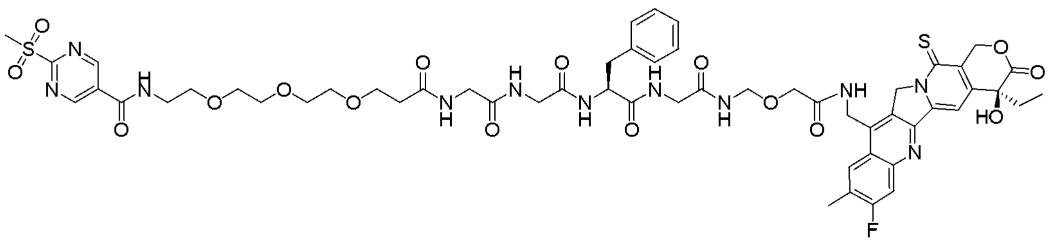

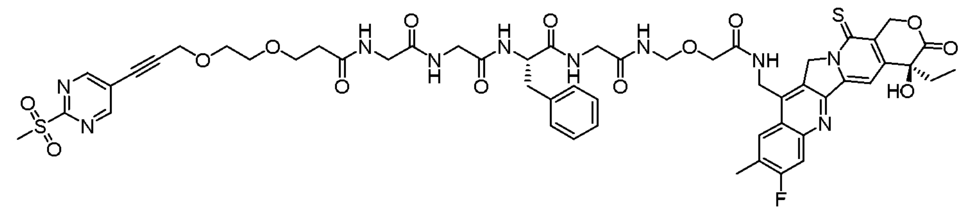

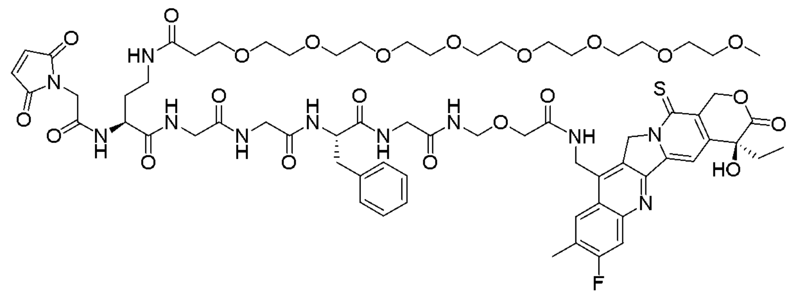

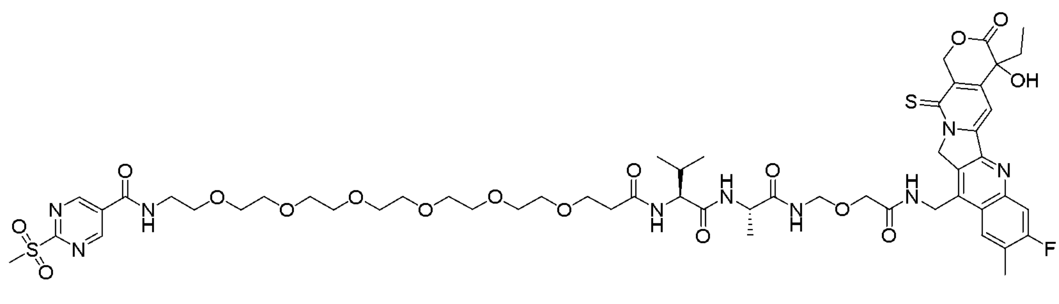

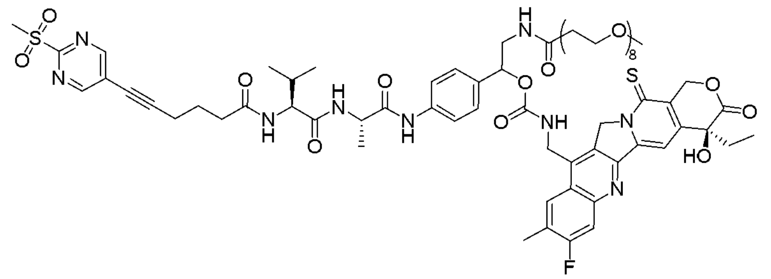

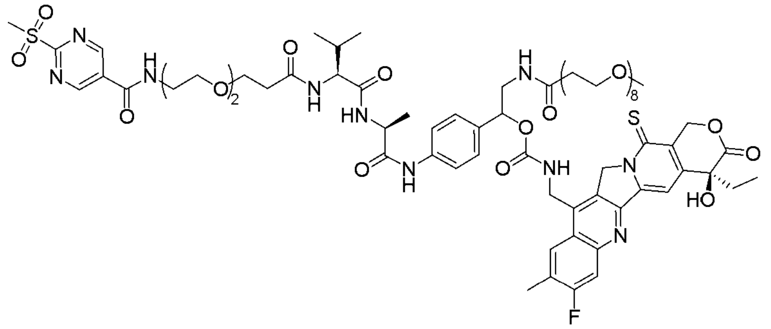

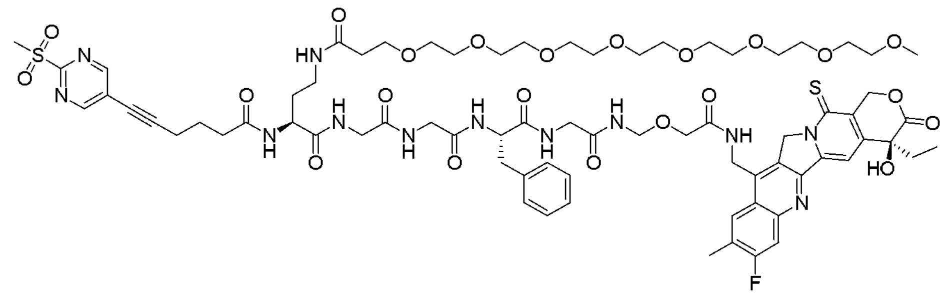

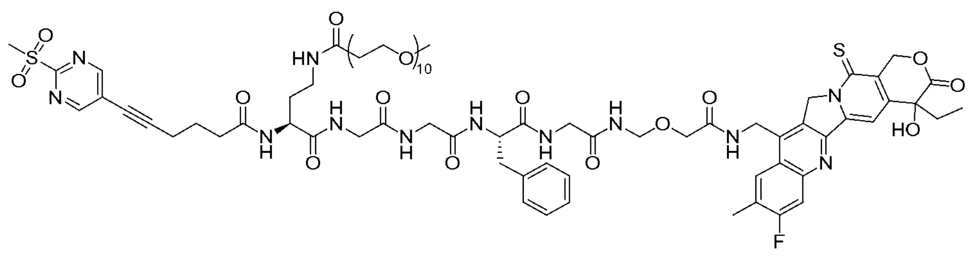

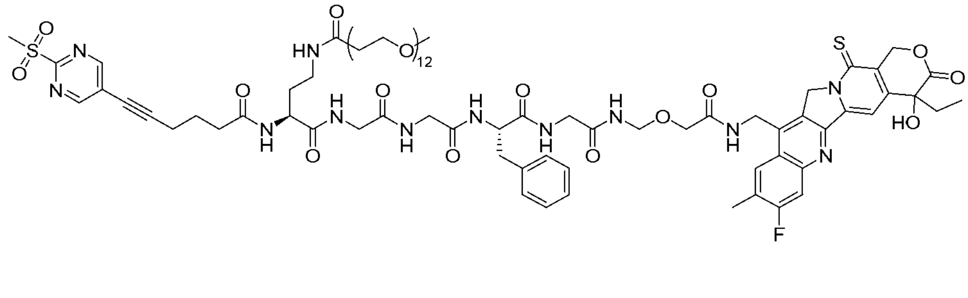

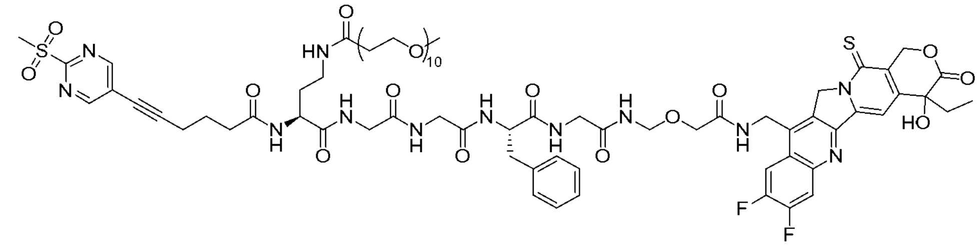

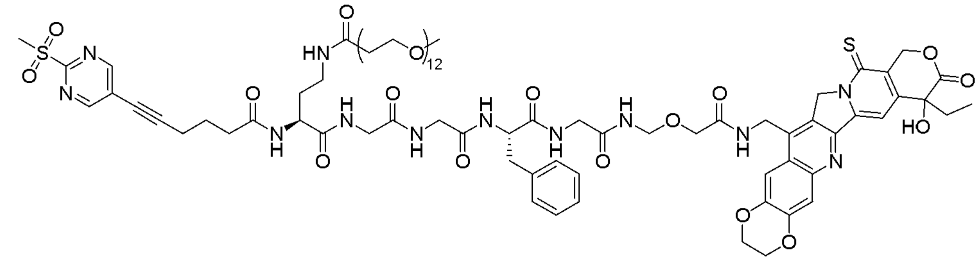

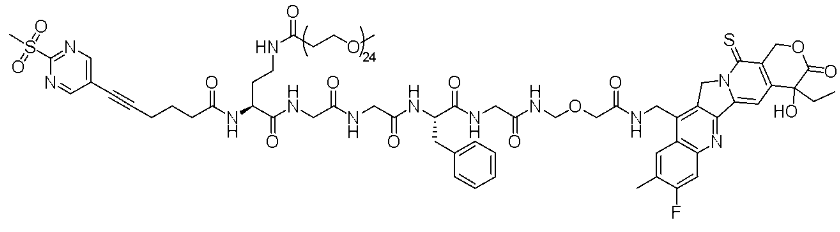

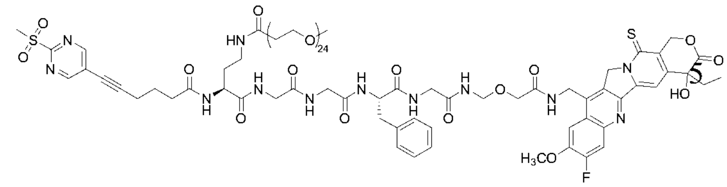

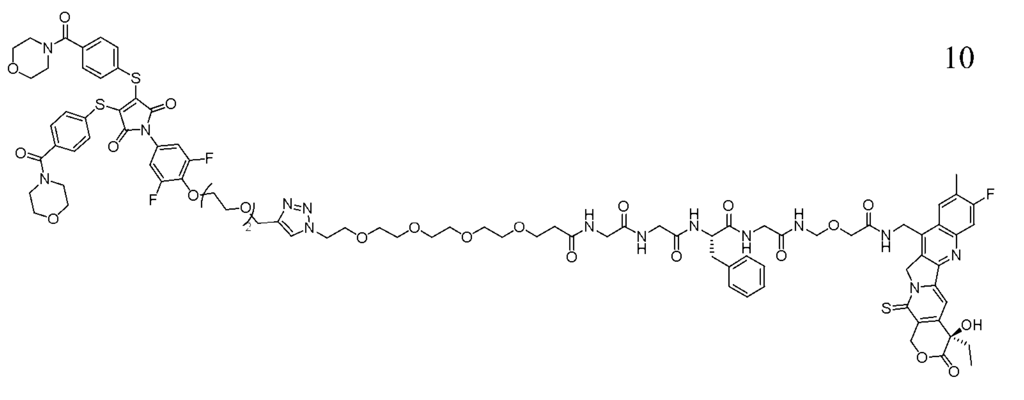

10

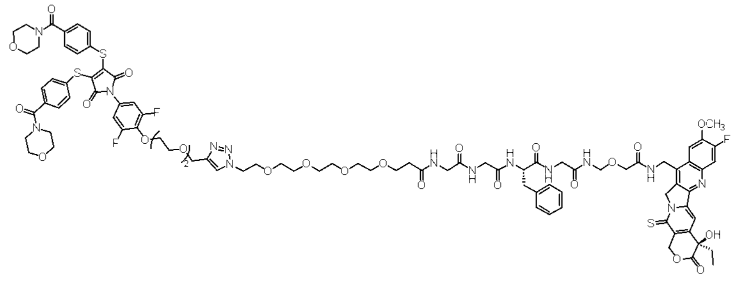

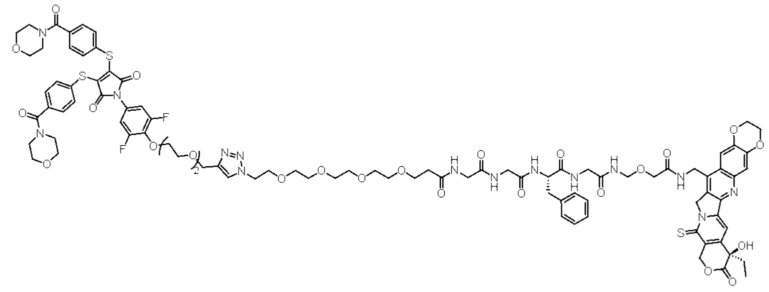

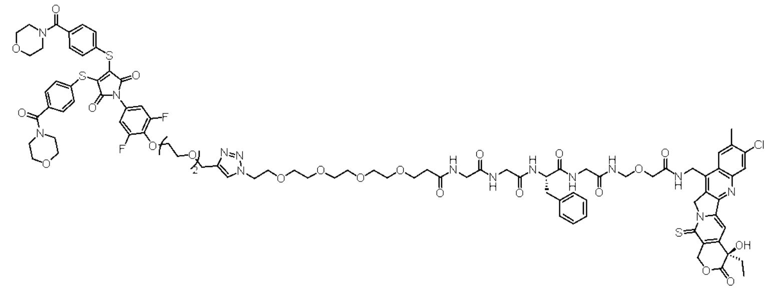

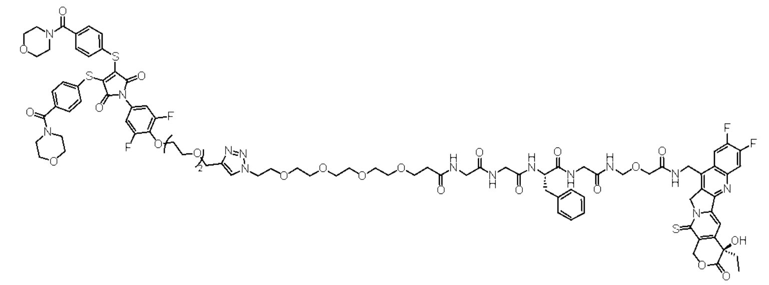

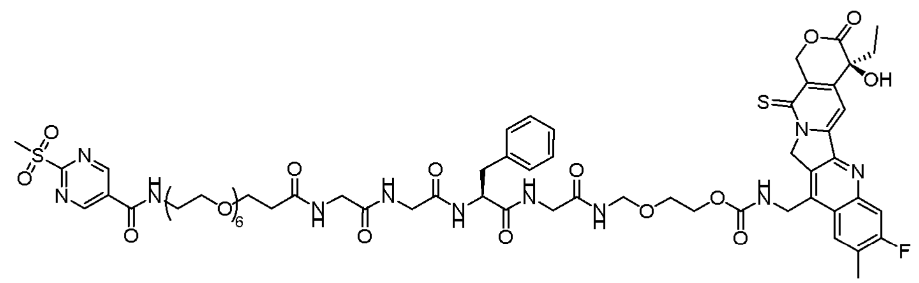

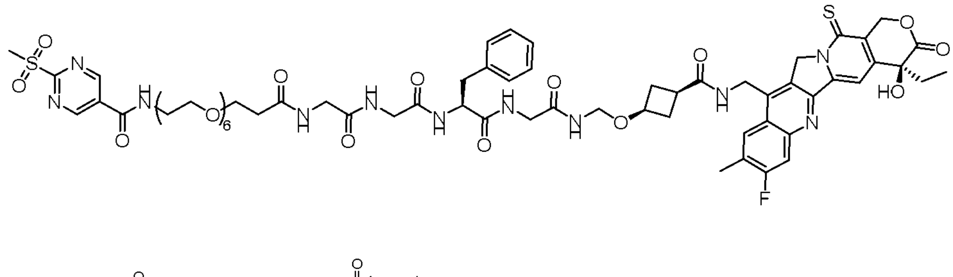

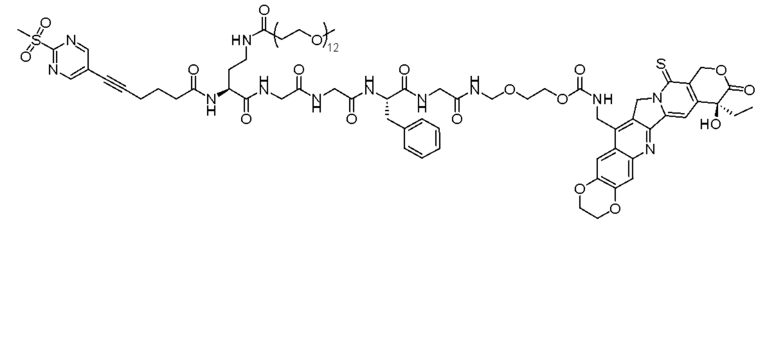

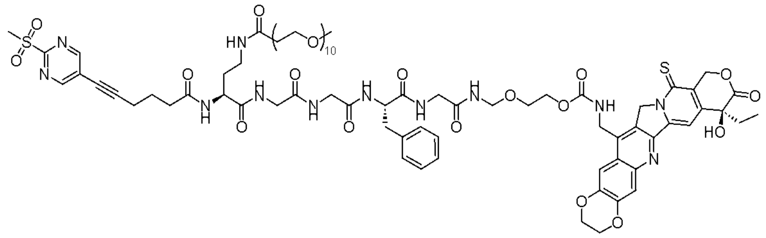

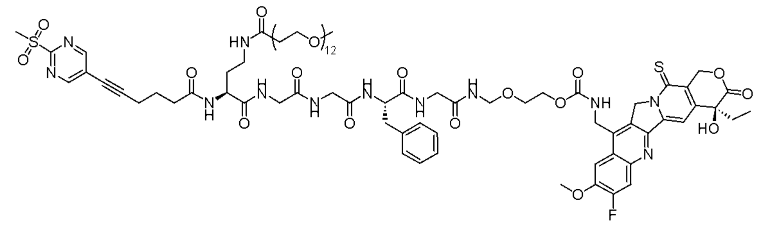

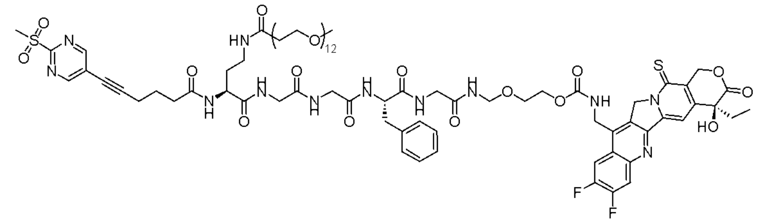

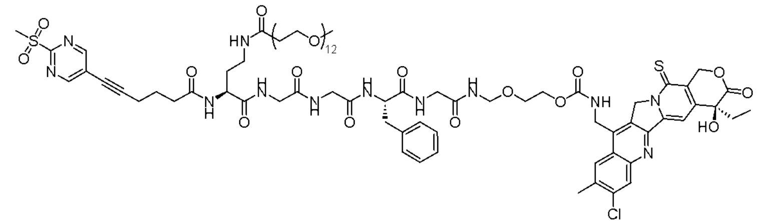

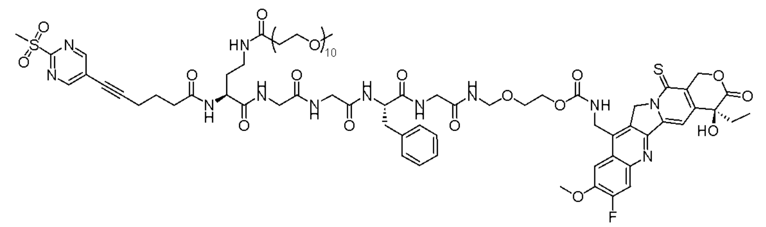

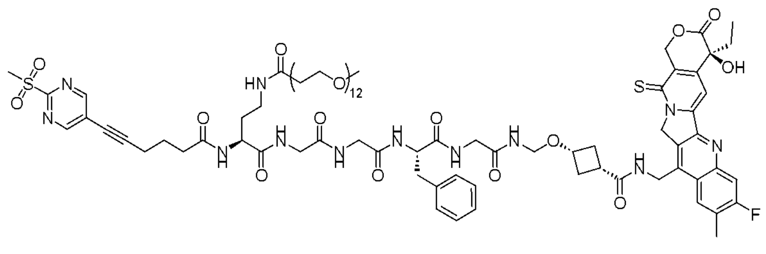

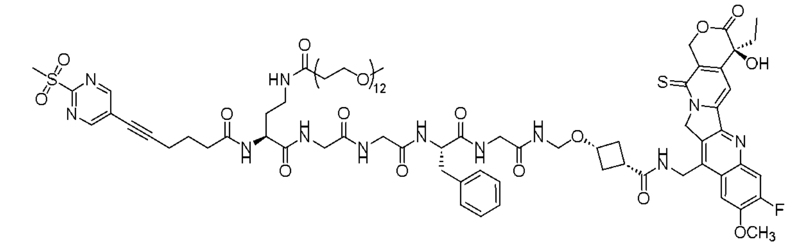

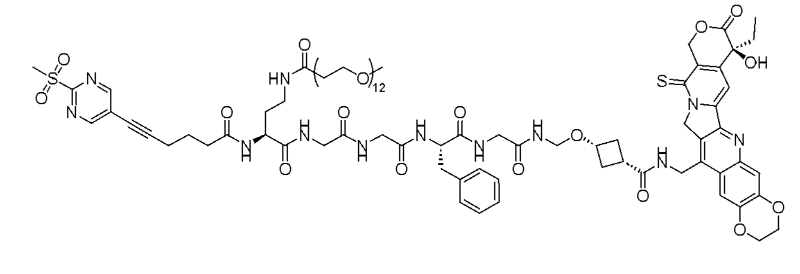

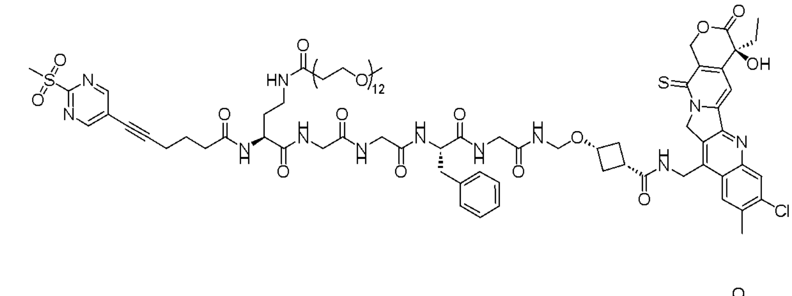

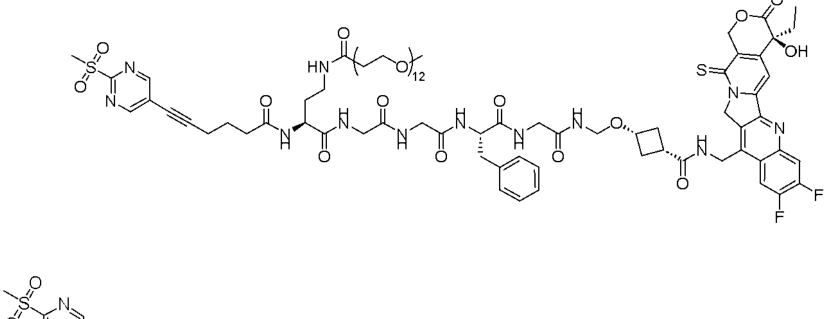

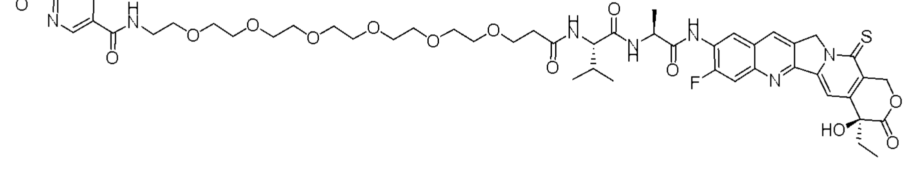

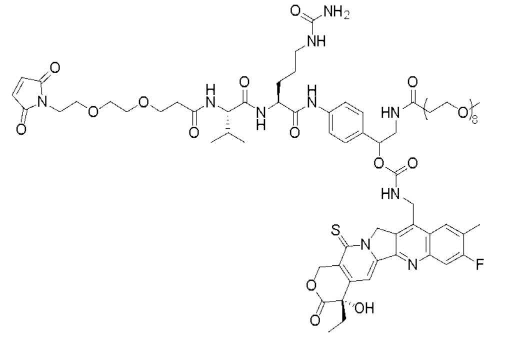

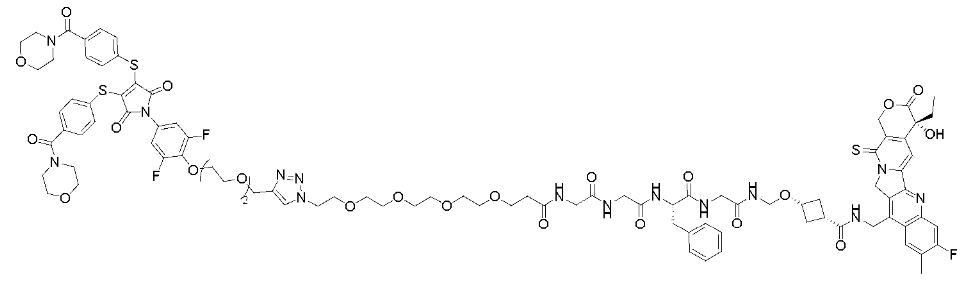

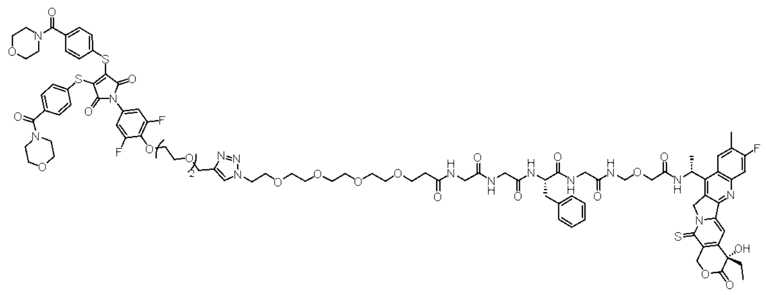

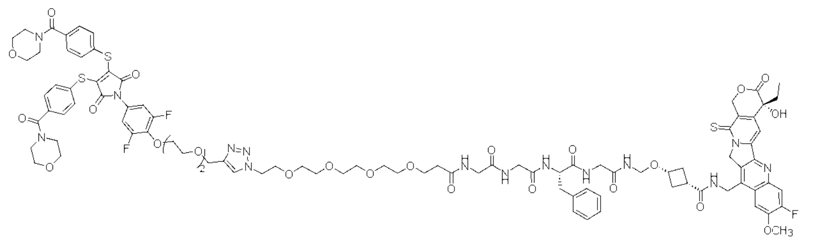

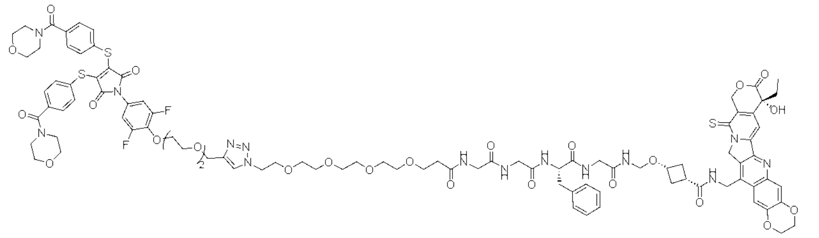

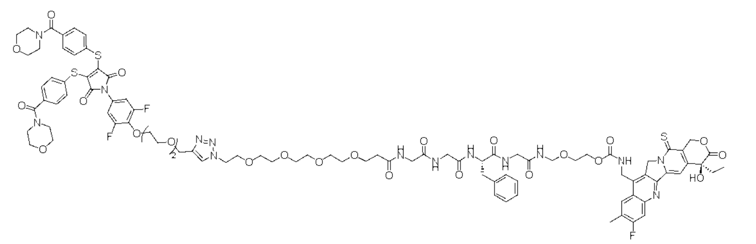

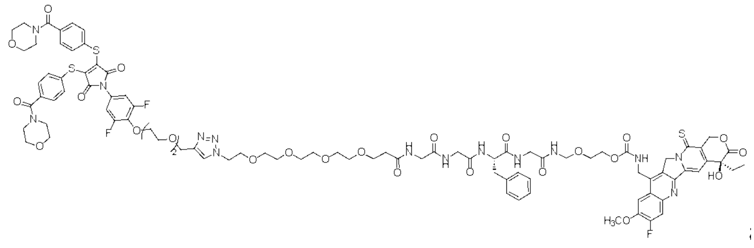

;

and

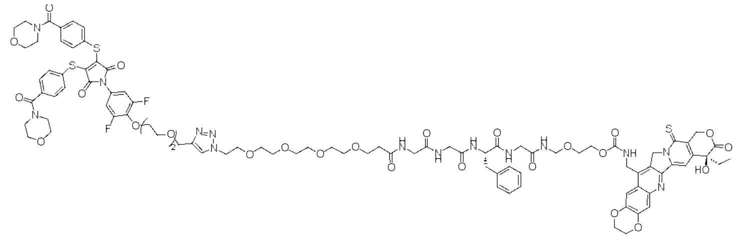

.

The fourth aspect of the present invention provides a pharmaceutical composition, wherein the pharmaceutical composition comprises a therapeutically effective amount of the compound or the ligand-drug conjugate or a pharmaceutically acceptable salt thereof according to any one of the first, second, third aspects, or a pharmaceutically acceptable carrier.

The fifth aspect of the present invention provides a pharmaceutical composition, comprising the use of the compound or ligand-drug conjugate or a pharmaceutically acceptable salt thereof according to any one of the first, second or third aspects of the present invention, or the pharmaceutical composition according to the fourth aspect, in the preparation of a medicament for treating or preventing tumors;

preferably, wherein the tumor is a cancer associated with B7-H3 expression, HER2 expression, or NECTIN4 expression;
preferably, the cancer is selected from breast cancer, gastric cancer, melanoma, and lung cancer.

The sixth aspect of the present invention provides a method of preventing or treating a tumor, comprising administering to a subject in need thereof an effective amount of the compound or the ligand-drug conjugate or a pharmaceutically acceptable salt thereof according to any one of the first, second, or third aspects of the present invention , or the pharmaceutical composition according to the fourth aspect ;

preferably, wherein the tumor is a cancer associated with B7-H3 expression, HER2 expression, or NECTIN4 expression;
preferably, the cancer is selected from breast cancer, gastric cancer, melanoma, and lung cancer.

The sixth aspect of the present invention provides the compound or the ligand-drug conjugate or a pharmaceutically acceptable salt thereof according to any one of the first, second, or third aspects of the present invention, or the pharmaceutical composition according to the fourth aspect, for use in the prevention or treatment of a tumor;
preferably, wherein the tumor is a cancer associated with B7-H3 expression, HER2 expression, or NECTIN4 expression;
preferably, the cancer is selected from breast cancer, gastric cancer, melanoma, and lung cancer.

The sixth aspect of the present invention provides a use of the compound according to the first aspect of the present invention, characterized in that it is used as a toxin in an antibody-drug conjugate for the preparation of the antibody-drug conjugate.

## DESCRIPTION OF THE DRAWINGS

FIG. 1 shows the inhibitory activity of B7H3-ADC-27 in B7H3-negative Raji cell lines in Test Example 6;

FIG. 2 shows the inhibitory activity of B7H3-ADC-27 in B7H3-positive A375 and B7H3-negative Raji mixed cell lines in Test Example 6;

FIG. 3 shows the inhibitory activity of B7H3-ADC-1 in B7H3-negative Raji cells in Test Example 6;

FIG. 4 shows the inhibitory activity of B7H3-ADC-1 in a mixed cell line of B7H3-positive A375 and B7H3-negative Raji cells in Test Example 6;

FIG. 5 shows the antitumor activity of the ADC molecule in an animal model co-harboring B7H3-negative and positive cells in Test Example 6;

FIG. 6 shows the tumor growth curve of mice in Test Example 7;

FIG. 7 shows the body weight change curve of mice in Test Example 7;

FIG. 8 shows the tumor growth curve of mice in Test Example 8;

FIG. 9 shows the body weight change curve of mice in Test Example 8;

FIG. 10 shows the body weight change curve of mice in Test Example 9;

FIG. 11 shows the tumor growth curve of mice in Test Example 10;

FIG. 12 shows tumor tissues from mice in Test Example 10.

## DETAILED DESCRIPTION

The following provides a clear and complete description of the technical solutions of the present invention. It should be understood that the described embodiments represent a portion of the embodiments of the present invention, not all of them. All other embodiments obtained by those skilled in the art based on the embodiments of the present invention without performing creative labor fall within the scope of protection of the present invention.

Definitions and Terms

Unless otherwise defined below, all technical and scientific terms used herein are intended to have the same meanings as those commonly understood by those skilled in the art.

Compounds of the present invention may exist in free form or, where appropriate, in the form of pharmaceutically acceptable derivatives. In the present invention, pharmaceutically acceptable derivatives include, but not limited to, pharmaceutically acceptable salts, prodrugs, stereoisomers (include but not limited to, diastereoisomers and enantiomers), tautomers, solvates, polymorphs, and isotopic compounds, which, upon administration to a patient in need thereof, are capable of providing, directly or indirectly, the compounds of the present invention or the metabolites. Therefore, references herein to "the compounds of the invention" are also intended to encompass the aforementioned derivative forms of the compounds.

The term "pharmaceutically acceptable salt" refers to a salt that retains the biological efficacy of the free acid and base of a particular compound without undesirable biological effects. Examples of pharmaceutically acceptable salts include, but not limited to: (1) acid addition salts, such as those formed with inorganic acids like hydrochloric acid, sulfuric acid, hydrobromic acid, nitric acid, phosphoric acid, and the like; or with organic acids such as malic acid, fumaric acid, maleic acid, benzoic acid, phenylacetic acid, succinic acid, tartaric acid, citric acid, methanesulfonic acid, ethane sulfonic acid, hydroxyacetic acid, cinnamic acid, pyruvic acid, formic acid, acetic acid, propionic acid, oxalic acid, malonic acid, acrylic acid, mandelic acid, and the like; or (2) Base addition salts, including salts formed with alkali metals such as lithium, sodium, potassium, and the like; salts formed with alkaline earth metals such as calcium, magnesium, and the like; and salts formed with organic bases such as ammonium, choline, diethylamine, lysine, ethylenediamine, tert-butylamine, tert-octylamine, trimethylolpropane, N-methylglucosamine, triethanolamine, dehydroabietine, and the like. Other pharmaceutically acceptable salts are known to those skilled in the art.

The pharmaceutical prodrugs of the compounds of the present invention are included within the scope of protection of the present invention. Typically, the pharmaceutical prodrugs refer to functional derivatives that readily convert into the desired compounds within the body. Therefore, the term "administration" in the therapeutic methods provided by the present invention encompasses the application of the compounds disclosed herein, or the administration to subjects of compounds not explicitly disclosed herein that can convert into the disclosed compounds within the body to treat the various diseases described. Conventional methods for selecting and preparing suitable prodrug derivatives are documented in books such as Design of Prodrugs (H. Bundgaard, Elsevier, 1985).

The compounds described herein may comprise one or more asymmetric centers and may result in yield of diastereomers and optical isomers. The invention encompasses all possible diastereomers and their racemic mixtures, their substantially pure resolved enantiomers, all possible geometric isomers, and their pharmaceutically acceptable salts.

The stereochemical configuration at any particular position of the compounds of the invention is not precisely defined. The present invention encompasses all stereoisomers of the compounds and their pharmaceutically acceptable salts. Moreover, mixtures of stereoisomers and isolated specific stereoisomers are also included herein. Products obtained during the synthesis of such compounds, or through racemization or diastereomerization methods known to those skilled in the art, may be mixtures of stereoisomers.

When the compounds of the present invention exist as tautomers, unless specifically stated otherwise, the present invention includes any possible tautomers and their pharmaceutically acceptable salts, as well as mixtures

thereof.

When the compounds of the present invention and their pharmaceutically acceptable salts exist as solvates or polymorphs, the invention includes any possible solvate and polymorphic forms. The type of solvent forming the solvate is not specifically limited, provided the solvent is pharmacologically acceptable. For example, water, ethanol, propanol, acetone, and similar solvents may be employed.

The present invention further encompasses all pharmaceutically acceptable isotopic compounds identical to the compounds of the invention except for one or more atoms substituted with atoms having the same atomic number but differing atomic mass or mass number from the naturally predominant atomic mass or mass number. Examples of isotopes suitable for inclusion in the compounds of the present invention include (but not limited to) isotopes of hydrogen (e.g., $^{2}H$, $^{3}H$); isotopes of carbon (e.g., $^{13}C$ and $^{14}C$); isotopes of chlorine (e.g., $^{37}Cl$); isotopes of iodine (e.g., $^{125}I$); isotopes of nitrogen (e.g., $^{13}N$ and $^{15}N$); isotopes of oxygen (e.g., $^{17}O$ and $^{18}O$); isotopes of phosphorus (e.g., $^{32}P$), and isotopes of sulfur (e.g., $^{34}S$).

The term "ligand" refers to a macromolecular compound capable of recognizing and binding to antigens or receptors associated with target cells. The function of the ligand is to deliver the drug to the target cell population that binds to the ligand. In the embodiments of the present invention, the ligand is represented as Ab. The ligand can form a bond with the linking unit through a heteroatom on the ligand. Preferably, the ligand is an antibody or its antigen-binding fragment or a peptide. The antibody is selected from chimeric antibodies, humanized antibodies, fully humanized antibodies, or murine antibodies; preferably, it is a monoclonal antibody.

The term "ligand-binding moiety" refers to a group capable of binding to the ligand portion.

The term "drug-binding moiety" refers to a group capable of binding to the drug portion.

The term "drug" refers to cytotoxic drugs, denoted as D, which are chemical molecules capable of strongly disrupting the normal growth of tumor cells.

The terms "linker unit," "linker fragment," "linking unit," "linker moiety," or "linker" refer to a chemical structural fragment or bond connected to a ligand at one end and a drug at the other end. It may also connect to other linkers before linking to the drug.

The term "ligand-drug conjugate" refers to a ligand connected via a stable linker to a biologically active drug. In this disclosure, "ligand-drug conjugate" is preferably an antibody-drug conjugate (ADC), which connects a monoclonal antibody or antibody fragment via a stable linker to a biologically active toxic drug.

The three-letter and single-letter amino acid codes used herein are as described in J. Biol. Chem. 243, p. 3558 (1968).

The term "antibody" refers to immunoglobulin, a tetrameric protein structure composed of two identical heavy chains and two identical light chains linked by interchain disulfide bonds. The amino acid composition and sequence of the constant regions in immunoglobulin heavy chains differ, resulting in distinct antigenic properties. Immunoglobulins are classified into five classes, also known as immunoglobulin isotypes accordingly: IgM, IgD, IgG, IgA, and IgE. Their corresponding heavy chains are μ-chain, δ-chain, γ-chain, α-chain, and ε-chain, respectively. Within the same Ig class, further subclasses are defined by differences in hinge region amino acid composition and the number/position of heavy chain disulfide bonds. For example, IgG is subdivided into IgG1, IgG2, IgG3, and IgG4. Light chains are classified asκorλchains based on their constant region. Each of the five Ig classes can possess either κor λchains. The antibodies described herein are preferably specific antibodies targeting cell surface antigens on target cells. Non-limiting examples include the following antibodies: an anti-TROP-2 antibody, anti-HER2 (ErbB2) antibody, anti-EGFR antibody, anti-NECTIN4 antibody, anti-B7-H3 antibody, anti-c-Met antibody, anti-HER3 (ErbB3) antibody, anti-HER4 (ErbB4) antibody, anti-LIV-1 antibody, anti-ROR1 antibody, anti-CD20 antibody, anti-CD22 antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-MUCl antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody, anti-Mesothelin antibody, or their antigen-binding fragments; More preferably, the antibody or its antigen-binding fragment is an anti-B7-H3 antibody, anti-NECTIN4 antibody, anti-HER2 (ErbB2) antibody, or an antigen-binding fragment thereof.

The antibodies of the present invention include a murine antibody, a chimeric antibody, a humanized antibody, and a fully humanized antibody, preferably, a humanized antibody and a full humanized antibody.

The term "murine antibody" in the present invention refers to an antibody prepared using mice according to the knowledge and skills of the art. Preparation involves injecting a specific antigen into a test subject, followed by isolating a hybridoma expressing an antibody with the desired sequence or functional characteristics.

The term "chimeric antibody" refers to an antibody created by fusing the variable region of a mouse antibody with the constant region of a human antibody, thereby reducing the immune response triggered by mouse-derived antibodies. To establish chimeric antibodies, one must first create a hybridoma secreting mouse-specific monoclonal antibodies. Variable region genes are then cloned from mouse hybridoma cells. Depending on requirements, constant region genes from human antibodies are also cloned. The mouse variable region gene and human constant region gene are fused into a

chimeric gene and inserted into an expression vector. Finally, the chimeric antibody molecule is expressed in either a eukaryotic or prokaryotic system.

The term "humanized antibody" also known as a CDR-grafted antibody, refers to antibodies produced by grafting mouse CDR sequences onto human antibody variable region frameworks-specifically, different types of human germline antibody framework sequences. This approach overcomes the heterologous reactions induced by chimeric antibodies due to their substantial mouse protein content.

The term "fully humanized antibody," "fully human antibody," or "completely humanized antibody," also known as "fully humanized monoclonal antibody," denotes antibodies where both the variable and constant regions are of human origin. This eliminates immunogenicity and adverse toxic effects.

The term "antigen-binding fragment" refers to one or more fragments of an antibody that retain the ability to specifically bind an antigen. It has been demonstrated that fragments derived from full-length antibodies can be utilized for antigen-binding functions. Examples of binding fragments contained within an "antigen-binding fragment" include: (i) Fab fragments, monovalent fragments composed of VL, VH, CL, and CH1 domains; (ii) F(ab')2 fragments, which are bivalent fragments comprising two Fab fragments connected by disulfide bridges across the hinge region; (iii) Fd fragments, composed of VH and CH1 domains; (iv) Fv fragments, composed of the VH and VL domains of a single antibody arm; (v) Single-domain or dAb fragment (Ward et al., 1989, Nature 341:544-546), composed of the VH domain; and (vi) Isolated complementarity-determining region (CDR) or (vii) Optionally, a combination of two or more isolated CDRs joined by synthetic junctions. Furthermore, although the two domains VL and VH of the Fv fragment are encoded by separate genes, they may be joined by synthetic junctions using recombinant methods, thereby enabling the production of a single protein chain in which the VL and VH regions are paired to form a monovalent molecule (referred to as a single-chain Fv (scFv); see, e.g., Bird et al. (1988) Science 242:423-426; and Huston et al. (1988) Proc. Natl. Acad. Sci. USA 85:5879-5883). Such single-chain antibodies are also intended to be included within the term "antigen-binding fragment" of an antibody. Such antibody fragments are obtained using conventional techniques known to those skilled in the art, and functional screening of the fragments is performed in the same manner as for intact antibodies. The antigen-binding portion may be produced by recombinant DNA technology or by enzymatic or chemical cleavage of intact immunoglobulins.

Fab is an antibody fragment with a molecular weight of approximately 50,000 and antigen-binding activity, obtained by treating IgG antibody molecules with the protease papain (which cleaves the amino acid residue at position 224 of the heavy chain). Approximately half of the N-terminal side of the heavy chain and the entire light chain are linked together by disulfide bonds.

F(ab')2 is an antibody fragment with a molecular weight of approximately 100,000 and antigen-binding activity, obtained by digesting the lower portion of the two disulfide bonds in the IgG hinge region with the enzyme pepsin. It comprises two Fab regions connected at the hinge position.

Fab' is an antibody fragment with a molecular weight of approximately 50,000 and antigen-binding activity obtained by cleaving the disulfide bonds in the hinge region of the aforementioned F(ab')2.

Furthermore, the Fab' can be produced by inserting DNA encoding the Fab' fragment of the antibody into a prokaryotic expression vector or eukaryotic expression vector and introducing the vector into a prokaryotic or eukaryotic organism to express Fab'.

The terms "single-chain antibody," "single-chain Fv," or "scFv" refer to molecules comprising an antibody heavy chain variable domain (or region; VH) and an antibody light chain variable domain (or region; VL) connected via a hinge. Such scFv molecules may exhibit the general structure: NH2-VL-hinge-VH-COOH or NH2-VH-hinge-VL-COOH. Suitable prior art linkers comprise the repeating GGGGS amino acid sequence or variants thereof, such as those employing 1-4 repeating variants(Holliger et al. (1993), Proc. Natl. Acad. Sci. USA 90:6444-6448). Additional linkers suitable for use in the present disclosure are described by Alfthan et al. (1995), Protein Eng. 8:725-731; Choi et al. (2001), Eur. J. Immunol. 31:94-106; Hu et al. (1996), Cancer Res. 56:3055-3061; Kipriyanov et al. (1999), J. Mol. Biol. 293:41-56, and Roovers et al. (2001), and Cancer Immunol.

The term "CDR" refers to one of the six hypervariable regions within the variable domain of an antibody that primarily mediates antigen binding. One of the most commonly used definitions for these six CDRs is provided by Kabat E.A. et al. (1991), Sequences of proteins of immunological interest. NIH Publication 91-3242. As used herein, the Kabat definition of CDRs applies only to CDR1, CDR2, and CDR3 (CDR L1, CDR L2, CDR L3 or L1, L2, L3) of the light chain variable domain, and CDR2 and CDR3 (CDR H2, CDR H3 or H2, H3) of the heavy chain variable domain.

The term "vector" refers to a nucleic acid molecule capable of transporting another nucleic acid to which it is attached. In one embodiment, the vector is "plasmid," a circular double-stranded DNA ring to which additional DNA segments can be attached. In another embodiment, the vector is the viral vector, wherein additional DNA segments can be attached to the viral genome. The vectors disclosed herein may replicate autonomously within host cells into which they are introduced (e.g., bacterial vectors possessing bacterial replication origins and non-integrating mammalian vectors) or may integrate into the host cell genome upon introduction, thereby replicating with the host genome (e.g., integrating mammalian vectors).

The term "alkyl" refers to a saturated aliphatic hydrocarbon group, which is a straight-chain or branched group

comprising 1 to 20 carbon atoms, preferably comprising 1 to 12 carbon atoms, more preferably containing 1 to 10 carbon atoms, and most preferably comprising 1 to 6 carbon atoms. The term "$C_{1-6}$ alkyl" refers to a saturated straight-chain or branched-chain hydrocarbon group having 1 to 6 carbon atoms (e.g., 1, 2, 3, 4, 5, or 6 carbon atoms). For example, "$C_{1-6}$ alkyl" may be methyl, ethyl, n-propyl, isopropyl, n-butyl, isobutyl, sec-butyl, tert-butyl, n-pentyl, isopentyl, neopentyl, or n-hexyl, and the like.

The term "cycloalkyl" refers to a saturated or partially unsaturated mono- or polycyclic ring hydrocarbon substituent, wherein the cycloalkyl ring contains 3 to 20 carbon atoms, preferably 3 to 12 carbon atoms, more preferably 3 to 10 carbon atoms, and most preferably 3 to 8 carbon atoms. Non-limiting examples of monocyclic cycloalkyl include cyclopropyl, cyclobutyl, cyclopentyl, cyclopentylenyl, cyclohexyl, cyclohexenyl, cyclohexadienyl, cycloheptyl, cycloheptatriene, cyclooctyl, and the like; polycyclic cycloalkyl includes spirocyclic, fused-ring, and bridged-ring groups.

The term "heterocyclyl" refers to a saturated or partially unsaturated monocyclic or polycyclic ring hydrocarbon substituents containing 3 to 20 ring atoms, wherein one or more (e.g., 1, 2, or 3) ring atoms are selected from nitrogen, oxygen, or sulfur, with the remaining ring atoms being carbon. Preferably, they contain 3 to 12 ring atoms, with 1 to 4 being heteroatoms; more preferably, the naphthenyl ring comprises 3 to 10 ring atoms. Non-limiting examples of monocyclic heterocyclic groups include pyrrolidine, piperidine, piperazine, morpholine, thio-morpholine, and homopiperazine. Polycyclic heterocyclyl includes spirocyclic, fused-ring, and bridged-ring heterocyclyl groups.

The term "aryl" refers to a 6- to 14-membered monocyclic or fused polycyclic (i.e., rings sharing adjacent carbon pairs) group with a conjugated $\pi$-electron system, preferably 6- to 10-membered, such as phenyl and naphthyl, preferably phenyl. The aromatic ring may be fused to a heteroaromatic, heterocyclic, or aliphatic ring, wherein the ring connected to the parent structure is the aromatic ring.

The term "heteroaryl" refers to a heteroaromatic system comprising 1 to 4 heteroatoms and 5 to 14 ring atoms, wherein the heteroatoms are selected from oxygen, sulfur, and nitrogen. The heteroaryl group is preferably 5- to 10-membered, more preferably 5- or 6-membered, such as furanyl, thiophene, pyridine, pyrrole, N-alkylpyrrole, pyrimidine, pyrazine, imidazole, or tetrazole. The heteroaryl ring may be fused to an aryl, heterocyclic, or cycloalkyl ring, with the ring connected to the parent structure being the heteroaryl ring.

The term "haloalkyl" refers to an alkyl group substituted with one or more halogen atoms.

The term "deuterated alkyl" refers to an alkyl group substituted with one or more deuterium atoms.

The term "halogen" refers to fluorine, chlorine, bromine, or iodine.

The term "drug moiety" or designated as "D" broadly refers to any compound possessing desired biological activity and reactive functional groups for preparing the conjugates described herein.

The term "cytotoxic compound" refers to a class of drugs effective in killing tumor cells and inhibiting their proliferation. This includes a topoisomerase I (TOP1) inhibitor, a microtubule polymerization inhibitor, a topoisomerase II (TOP2) inhibitor, a dihydrofolate reductase inhibitor, a thymidylate synthase inhibitor, a purine nucleoside synthetase inhibitor, a nucleotide reductase inhibitor, a DNA polymerase inhibitor, a RNA polymerase II inhibitor, and other compounds capable of inhibiting cell proliferation. A topoisomerase I (TOP1) inhibitor include but are not limited to a camptothecin derivative such as SN-38, Dxd, Dx-8951; tubulin polymerization inhibitors such as eribulin, MMAE, MMAF, miconazole, and the like (structures as follows)

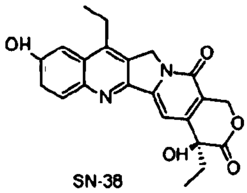
SN-38

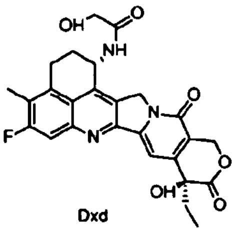
Dxd

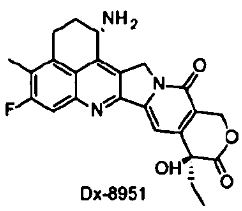
Dx-8951

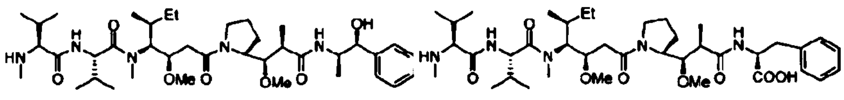
MMAE MMAF

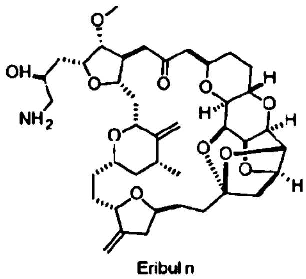

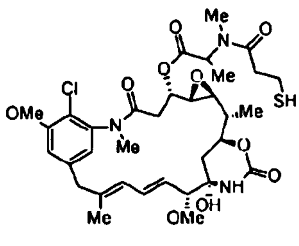

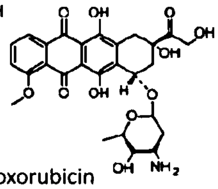

Eribulin Maytansincid Doxorubicin

The term "camptothecin derivatives" refers to pyrroloquinoline alkaloid derivatives, such as

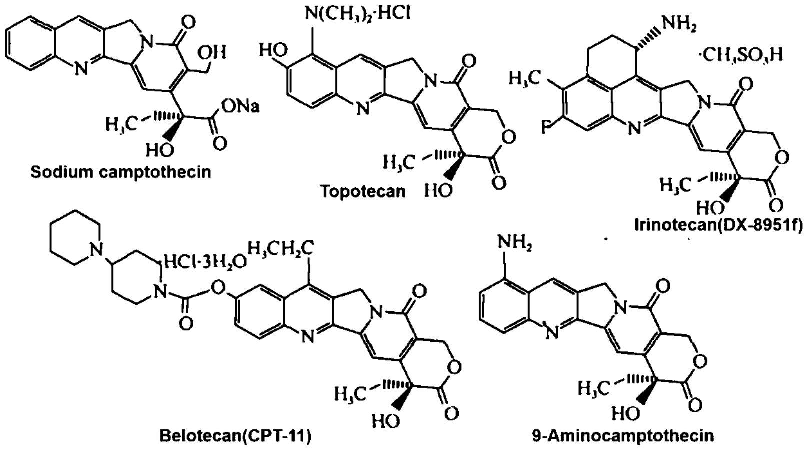

Sodium camptothecin Topotecan Irinotecan(DX-8951f) Belotecan(CPT-11) 9-Aminocamptothecin

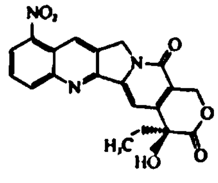

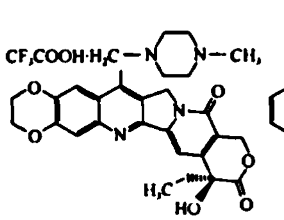

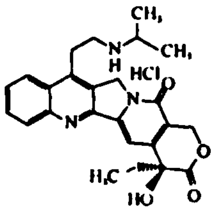

Rubitecan (9-Nitrocamptothecin) Lurtotecan(GG-211) CKD-602

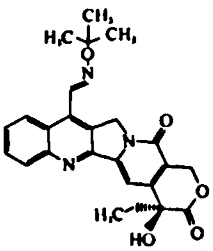

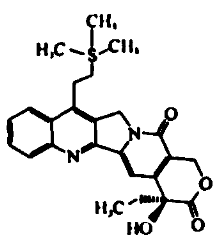

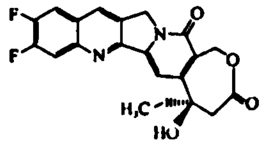

Gimatecan(ST1481) karenitecin(BNP-1350) BN-80915

,

and the like.

The terms "substituted" and "substitution" refer to the selective replacement of one or more (e.g., one, two, three, or four) hydrogen atoms on the specified atom by the indicated group, provided that the normal valence of the specified atom in the current context is not exceeded and the substitution forms a stable compound. Combinations of substituents and/or variables are permitted only when such combinations form stable compounds.

If a substituent is described as "optionally ...substituted," the substituent may (1) be unsubstituted, or (2) be substituted. If an atom or group is described as optionally substituted by one or more substituents from a list, one or more hydrogens on that atom or group may be replaced by independently selected, optional substituents. If substituents are described as "independently selected from" or "each independently being," each substituent is selected independently of the others. Thus, each substituent may be the same as or different from another (or others). For example, when a

substituent or substitution position, or different substituents or substitution positions, involve the selection of R groups (such as but not limited to R2, R3, Rh, Ri, Rx, and/or Ry) that may have the same or different symbols, each R is selected independently, meaning it may be the same or different. The same applies to the selection of numerical values such as d, g, m, and n.

Unless otherwise specified, the point of attachment of a substituent, as used herein, may originate from any suitable position on the substituent.

When a substituent bond is depicted as traversing a ring to connect two atoms, such a substituent may bond to any ring-forming atom within the substituable ring.

The terms "comprising", "comprise", "including", "having", "containing", or "involving", and other variations thereof used herein are inclusive or open-ended and do not exclude other elements or method steps not specifically listed. Those skilled in the art will understand that such term used above as "comprising" encompass the meaning of "consisting of."

In the present invention, "pharmaceutically acceptable carrier" refers to a diluent, excipient, adjuvant, or vehicle that is administered together with an active ingredient and that, within the scope of reasonable medical judgment, is suitable for contact with human and/or other animal tissues without causing excessive toxicity, irritation, allergic reactions, or other problems or complications inconsistent with a reasonable benefit/risk ratio. The terms "active ingredient," "therapeutic agent," "active substance," or "active agent" refer to a chemical entity that can effectively treat one or more symptoms of a target disease or condition.

As used herein, the term "effective amount" (e.g., " therapeutically effective amount" or "preventively effective amount") refers to the amount of active ingredient that, upon administration, achieves the desired effect to a certain degree, such as alleviating one or more symptoms of the condition being treated or preventing the onset of the condition or its symptoms.

Unless otherwise specified, the term "treat" or "treatment" as used herein means reversing, alleviating, or inhibiting the progression of the disease or condition to which the term applies, or one or more symptoms thereof, or preventing the disease or condition or one or more symptoms thereof.

Example

The experimental methods in the following examples are conventional methods unless otherwise specified. The chemical raw materials, reagents, etc., used in the following examples are all commercially available products unless otherwise specified. The abbreviations appeared in this text and their meanings are shown below:

Table 1 Abbreviations and Their Meanings

| Abbreviation | Meaning |
|---|---|
| EXD | Exatecan mesylate |
| DMF | N,N-Dimethylformamide |
| HATU | 2-(1H-7-azabenzotriazol- 1-yl)-1,1,3,3-tetramethyluronium hexafluorophosphate |
| DIEA | N,N-Diisopropylethylamine |
| EDCI•HCl | 1-(3-Dimethylaminopropyl)-3-ethylcarbodiimide hydrochloride |
| DCC | N,N'-Dicyclohexylcarbodiimide |
| HOSu | N-Hydroxysuccinimide |
| DMAP | 4-Dimethylaminopyridine |
| TFA | Trifluoroacetic acid |
| HOBt | 1-Hydroxybenzotriazole |
| EEDQ | 2-Ethoxy-1-ethoxycarbonyl-1,2-dihydroquinoline |
| NPC | Bis(4-nitrophenyl) carbonate |
| EMCA | 6-Maleimidocaproic acid |

Cell Lines and Their Sources

Unless otherwise stated, the cell lines used in the Examples of the present invention can be obtained through commercial purchase channels. Specifically:

The SK-BR-3 cell line was purchased from Nanjing Beiruiji Biotechnology Co., Ltd.;
The NCI-N87 cell line was purchased from Nanjing Beiruiji Biotechnology Co., Ltd.;
The T47D cell line was purchased from Nanjing Kebai Biotechnology Co., Ltd.;
The MDA-MB-453 cell line was purchased from Nanjing Kebai Biotechnology Co., Ltd.;
The A375 cell line was purchased from ATCC;
The NCI-H1703 cell line was purchased from Nanjing Kebai Biotechnology Co., Ltd.

**Example** 1:

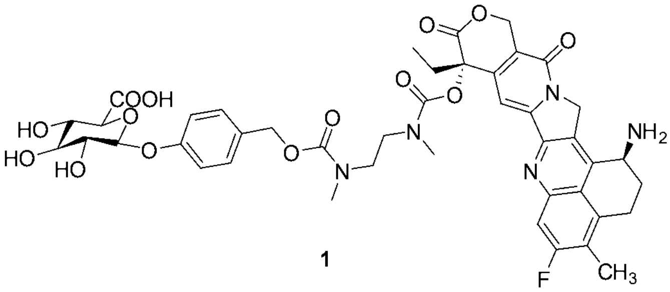

1

Synthetic route:

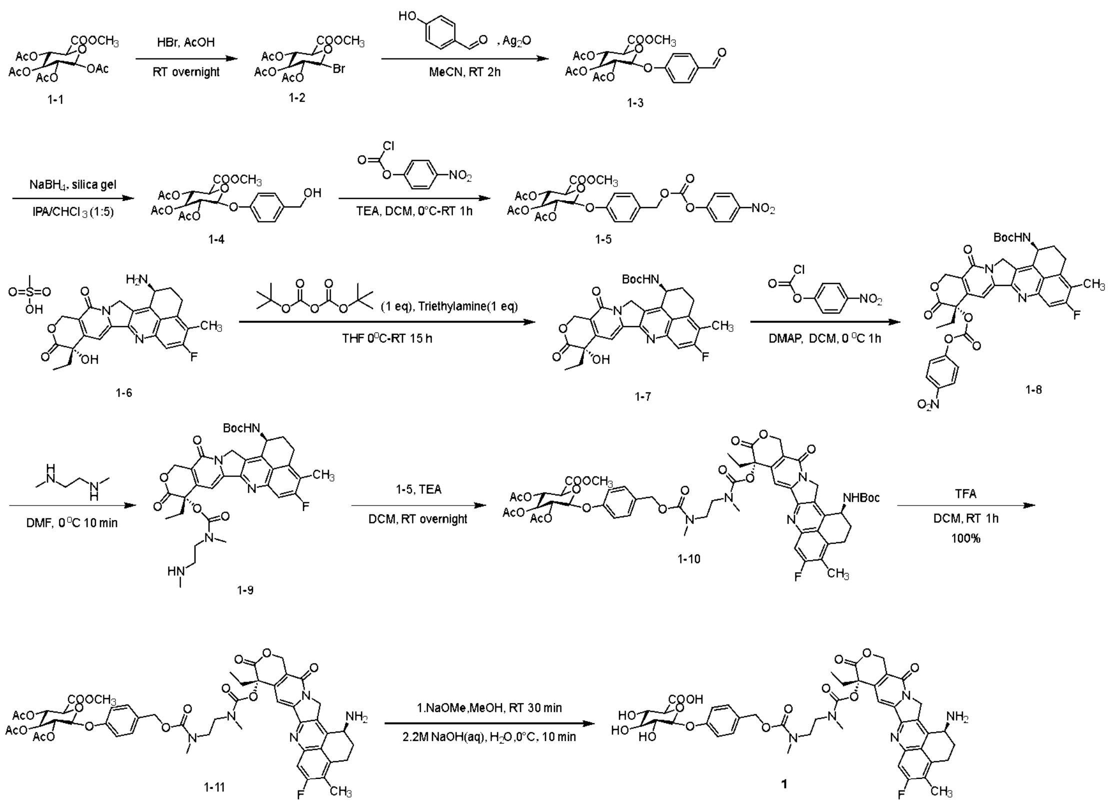


Synthesis of Intermediate 1-2:

In an ice-water bath, 20 mL of a solution of HBr (33%) in AcOH was added to a single-necked flask. Methyl (1,2,3,4-tetra-O-acetyl-β-D-glucopyranosyl)uronate (1-1, 5g, 13.2 mmol) was slowly added to the system. After being stirred for 10 minutes, the mixture was warmed to room temperature and stirred overnight. The reaction mixture gradually

became clear. Upon completion, the reaction mixture was diluted with DCM and extracted with water for 3 times(50 mL × 3). The organic phase was then washed with saturated $NaHCO_3$ aqueous solution and saturated NaCl aqueous solution (three times each) and then combined. The organic phase was dried over anhydrous $Na_2SO_4$ and concentrated to obtain a compound 1-2 (4.3g, 82%).

Synthesis of Intermediate 1-3:

The compound 1-2 (4.9 g, 12.3 mmol) was dissolved in 30 mL of acetonitrile. 4-Hydroxybenzaldehyde (1.8 g, 14.9 mmol) and silver oxide (4.2g, 18.1 mmol) were added sequentially. The mixture was stirred overnight. TLC analysis indicated completion of the reaction. The mixture was filtered through Celite and washed with ethyl acetate. The filtrate was spin-dried and concentrated under reduced pressure. The crude product was redissolved in ethyl acetate and washed with saturated $NaHCO_3$ aqueous solution and saturated NaCl aqueous solution (three times each) and then the organic phases were combined. The combined organic phase was dried over anhydrous $Na_2SO_4$ and concentrated. Purification by column chromatography (petroleum ether:ethyl acetate = 4:1 ~ 2:1) was performed to obtain a compound 1-3 (2.7 g, 50% yield). MS (ESI): m/z found $[M+18]^+$ = 456.1.

Synthesis of Intermediate 1-4:

The compound 1-3 (6g, 13.6 mmol) was dissolved in dry THF. $NaBH_4$ (1g, 27.2 mmol) was added at 0°C. After reacting for 1 hour, TLC analysis indicated complete consumption of the starting material. The reaction mixture was diluted with DCM and washed with saturated NaCl aqueous solution (200 mL × 4), and the organic phases were combined. The combined organic phase was dried over anhydrous $Na_2SO_4$ and concentrated to obtain a compound 1-4 (5g, 83%).

Synthesis of Intermediate 1-5:

In an ice-water bath, the compound 1-4 (63 mg, 0.143 mmol) was dissolved in DCM. 4-Nitrophenyl chloroformate (29 mg, 0.143 mmol) and triethylamine (40 μL, 0.286 mmol) were added sequentially. The reaction mixture was maintained in the ice-water bath for 10 minutes, then warmed to room temperature and stirred for 1 hour. TLC analysis indicated that the compound 1-4 was completely consumed. The reaction mixture was reserved for further use.

Synthesis of Intermediate 1-7:

In an ice-water bath, exatecan mesylate (1-6, 500 mg, 0.941 mmol) was dissolved in DMF. Di-tert-butyl dicarbonate (226 mg, 1.035 mmol) and triethylamine (262 μL, 1.881 mmol) were added sequentially. The mixture was stirred at 0°C for 15 minutes, then warmed to room temperature and reacted overnight. TLC analysis indicated completion of the reaction. The reaction mixture was diluted with ethyl acetate and washed with water (three times), and the organic phases were combined. The combined organic phase was dried over anhydrous $Na_2SO_4$ and concentrated to obtain a compound 1-7 (600 mg, 100%).

Synthesis of Intermediate 1-8:

In an ice-water bath, the compound 1-7 (100 mg, 0.187 mmol) was dissolved in DCM. 4-Nitrophenyl chloroformate (151 mg, 0.747 mmol) and 4-dimethylaminopyridine (91 mg, 0.747 mmol) were added sequentially. The reaction proceeded at 0°C for 1 hour. TLC analysis indicated completion of the reaction. 0.1 N HCl solution was added. After extraction and washing, the organic phase was washed with water three times and was washed with saturated NaCl aqueous solution once, dried over anhydrous $Na_2SO_4$, and concentrated. Purification by PTLC was performed to obtain a compound 1-8 (110 mg, 84%).

Synthesis of Intermediate 1-9:

In an ice-water bath, the compound 1-8 (110 mg, 0.157 mmol) was dissolved in DMF. N,N-Dimethylethylenediamine (13.85 mg, 0.157 mmol) was added, and the reaction proceeded at 0°C for 10 minutes. TLC analysis indicated completion of the reaction. The reaction mixture was diluted with ethyl acetate and washed with water three times, and the organic phases were combined. The combined organic phase was dried over anhydrous $Na_2SO_4$ and concentrated. Purification by PTLC was perfomed to obtain a compound 1-9 (62 mg, 75%).

Synthesis of Intermediate 1-10:

At room temperature, the compound 1-9 (62 mg, 0.143 mmol) was added to the reaction mixture containing intermediate 1-5. The reaction proceeded at room temperature overnight. TLC analysis indicated completion of the reaction. The organic phase was washed with water three times and washed with saturated NaCl aqueous solution once, dried over anhydrous $Na_2SO_4$, and concentrated. Purification by PTLC was perfomed to obtain a compound 1-10 (50 mg, 45%).

Synthesis of Intermediate 1-11:

In an ice-water bath, the compound 1-10 (30 mg, 0.027 mmol) was dissolved in ultra-dry DCM (5 mL). Trifluoroacetic acid (0.5 mL) was added, and the reaction proceeded for 30 minutes. TLC analysis indicated completion of the reaction. The reaction mixture was diluted with ethyl acetate and neutralized with saturated $NaHCO_3$ solution. It was then washed with water three times and the organic phases were combined. The combined organic phase was dried over anhydrous $Na_2SO_4$ and concentrated to obtain a compound 1-11 (27 mg, 98%).

Synthesis of Compound 1:

At room temperature, the compound 1-11 (36 mg, 0.035 mmol) was dissolved in methanol. Sodium methoxide (0.15 mg, 0.003 mmol) was added, and the reaction proceeded at room temperature. TLC analysis indicated completion of the reaction. The reaction mixture was then placed in an ice-water bath, and 2N NaOH solution (2.2 mg, 0.054 mmol) was added. The reaction proceeded at 0°C for 1 hour. TLC analysis indicated completion of the reaction. The reaction mixture was neutralized to pH 7 with 1 N HCl and concentrated. Purification by HPLC was perfomed to obtain a compound 1 (15 mg, 48%). MS (ESI): m/z found $[M+H]^+$ = 876.4.

Example 2:

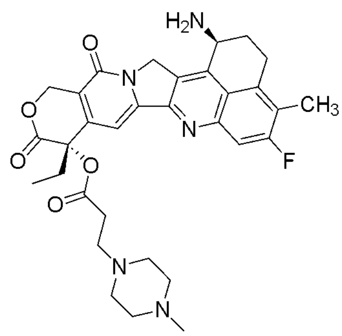

2

Synthetic route:

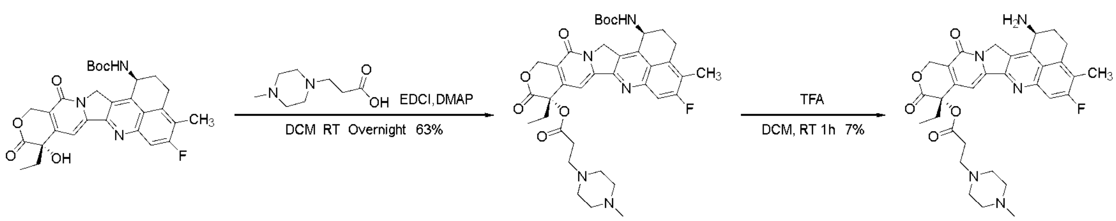


1-7 2-1 2

Synthesis of Intermediate 2-1:

At room temperature, the compound 1-7 (50 mg, 0.093 mmol) was dissolved in ultra-dry dichloromethane (5 mL). 4-Methyl-1-piperazinepropanoic acid (32 mg, 0.182 mmol), 1-(3-dimethylaminopropyl)-3-ethylcarbodiimide hydrochloride (80 mg, 0.449 mmol), and 4-dimethylaminopyridine (8 mg, 0.060 mmol) were added sequentially. The reaction proceeded overnight. LCMS monitoring indicated completion of the reaction. The reaction mixture was concentrated and purified by PTLC to obtain a compound 2-1 (40 mg, 63%). MS (ESI): m/z found $[M+H]^+$ = 690.3.

Synthesis of Compound 2:

In an ice-water bath, the compound 2-1 (40 mg, 0.019 mmol) was dissolved in ultra-dry dichloromethane (5 mL). Trifluoroacetic acid (TFA, 0.5 mL) was added, and the reaction proceeded for 1 hour. LCMS monitoring indicated complete consumption of the starting material. The reaction mixture was diluted with ethyl acetate and extracted with a saturated sodium bicarbonate ($NaHCO_3$) solution. The organic phase was washed with a saturated sodium chloride (NaCl) aqueous solution, dried over anhydrous sodium sulfate ($Na_2SO_4$), and concentrated. The crude product was purified by preparative HPLC to obtain a compound 2 (0.79 mg, 7% ). MS (ESI): m/z found $[M+H]^+$ = 590.3.

Example 3:

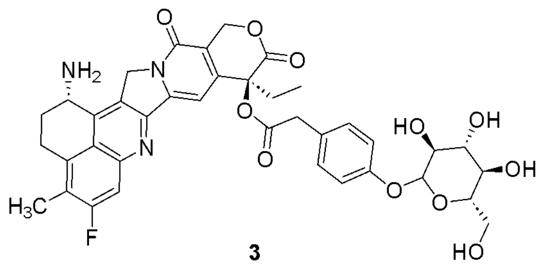

3

Synthetic route:

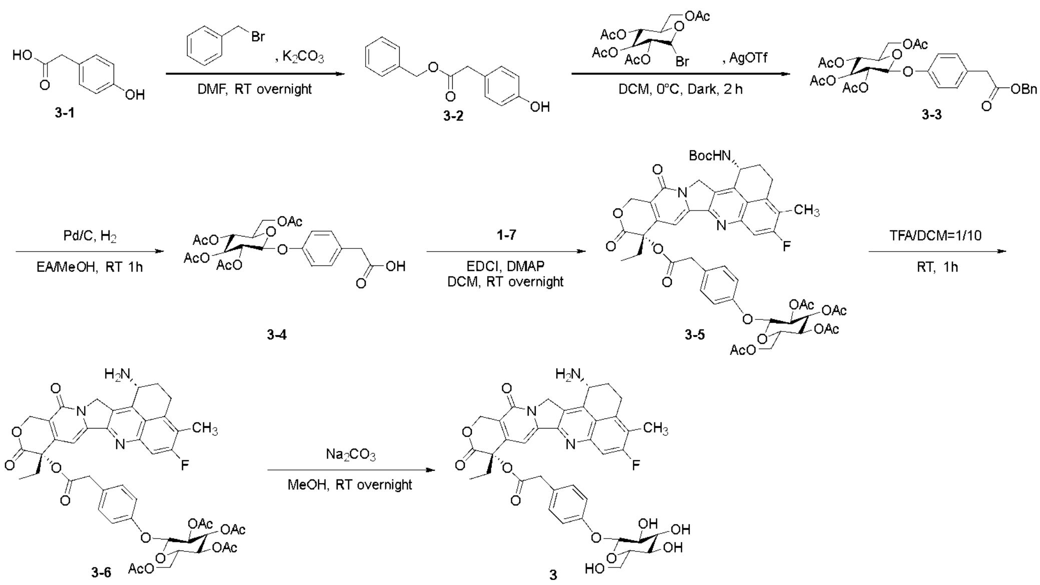


Synthesis of Intermediate 3-2:

At room temperature, p-hydroxyphenylacetic acid (1.0 g, 6.57 mmol) was dissolved in DMF (30 mL). Benzyl bromide (1.1 g, 6.57 mmol) and potassium carbonate (0.9 g, 6.57 mmol) were added sequentially. The reaction proceeded overnight. LCMS monitoring indicated completion of the reaction. The reaction mixture was diluted with ethyl acetate and extracted with water. The organic phase was washed with saturated sodium chloride aqueous solution (200 mL × 4), and the organic phases were combined, dried over anhydrous sodium sulfate, and concentrated. Purification by normal-phase

column chromatography was perfomed to obtain a compound 3-2 (1.7 g, 100% ). MS (ESI): m/z found $[M+18]^+$ = 260.2.

Synthesis of Intermediate 3-3:

In an ice-water bath, the compound 3-2 (1.0 g, 4.128 mmol) was dissolved in DCM (50 mL). The flask was wrapped in aluminum foil to protect from light. Silver trifluoromethanesulfonate (1.54 g, 6.004 mmol) and 2,3,4,6-tetra-O-acetyl-α-D-glucopyranosyl bromide (2.5 g, 6.004 mmol) were added sequentially. The reaction proceeded at 0°C for 2 hours. TLC analysis indicated complete consumption of the starting material. The reaction mixture was quenched with N,N-diisopropylethylamine (DIPEA), filtered, and the filtrate was concentrated. Purification by normal-phase column chromatography was perfomed to obtain a compound 3-3 (1.5 g, 62% ). MS (ESI): m/z found $[M+18]^+$ = 590.2.

Synthesis of Intermediate 3-4:

At room temperature, the compound 3-3 (1.5 g, 2.56 mmol) was dissolved in a mixture of ultra-dry ethyl acetate (10 mL) and methanol (2 mL). An appropriate amount of palladium on carbon (Pd/C) was added. The atmosphere was replaced with hydrogen gas ($H_2$), and the reaction proceeded for 1 hour. TLC analysis indicated completion of the reaction. The reaction mixture was filtered, and the filtrate was concentrated. Purification by normal-phase column chromatography was perfomed to obtain a compound 3-4 (678 mg, 55% ).

Synthesis of Intermediate 3-5:

At room temperature, the compound 1-7 (50 mg, 0.093 mmol) was dissolved in DCM (5 mL). The compound 3-4 (88 mg, 0.182 mmol), 1-(3-dimethylaminopropyl)-3-ethylcarbodiimide hydrochloride (87 mg, 0.449 mmol), and 4-dimethylaminopyridine (8 mg, 0.060 mmol) were added sequentially. The reaction proceeded overnight. LCMS monitoring indicated completion of the reaction. The reaction mixture was concentrated and purified by PTLC to obtain a compound 3-5 (72 mg, 77% ). MS (ESI): m/z found $[M+H]^+$ = 1000.4.

Synthesis of Intermediate 3-6:

In an ice-water bath, the compound 3-5 (72 mg, 0.072 mmol) was dissolved in ultra-dry DCM (5 mL). Trifluoroacetic acid (0.5 mL) was added, and the reaction proceeded for 1 hour. LCMS monitoring indicated complete consumption of the starting material. The reaction mixture was diluted with ethyl acetate and extracted with saturated sodium bicarbonate solution. The organic phase was washed with saturated sodium chloride aqueous solution, dried over anhydrous sodium sulfate, and concentrated to obtain a compound 3-6 (90 mg, 100%). MS (ESI): m/z found $[M+H]^+$ = 900.3.

Synthesis of Compound 3:

At room temperature, the compound 3-6 (32 mg, 0.036 mmol) was dissolved in methanol (5 mL). Sodium carbonate (23 mg, 0.216 mmol) was added, and the reaction proceeded at room temperature overnight. LCMS monitoring indicated completion of the reaction. The reaction mixture was filtered, and the filtrate was concentrated. Purification by preparative HPLC (Prep-HPLC) was perfomed to obtain a compound 3 (1.6 mg, 6%). MS (ESI): m/z found $[M+H]^+$ = 734.1.

Example 4:

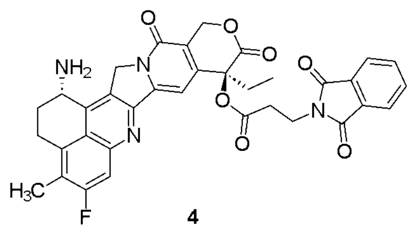

4

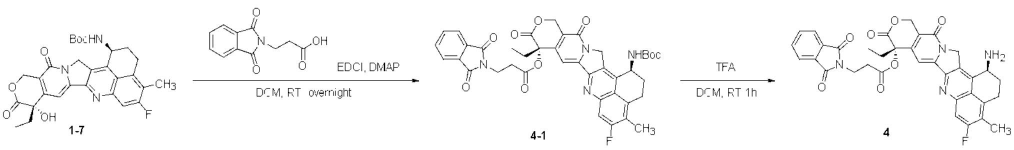


Synthesis of Intermediate 4-1:

At room temperature, the compound 1-7 (50 mg, 0.093 mmol) was dissolved in ultra-dry dichloromethane (5 mL). 3-(Phthalimido)propanoic acid (40 mg, 0.182 mmol), 1-(3-dimethylaminopropyl)-3-ethylcarbodiimide hydrochloride (80 mg, 0.449 mmol), and 4-dimethylaminopyridine (8 mg, 0.060 mmol) were added sequentially. The reaction proceeded overnight. LC-MS monitoring indicated completion of the reaction. The reaction mixture was concentrated and purified by preparative thin-layer chromatography (PTLC) to obtain a compound 4-1 (14 mg, 20.5% ). MS (ESI): m/z found $[M+H]^+$ = 737.2.

Synthesis of Compound 4:

In an ice-water bath, the compound 4-1 (14 mg, 0.019 mmol) was dissolved in ultra-dry dichloromethane (5 mL). Trifluoroacetic acid (0.5 mL) was added, and the reaction proceeded for 1 hour. LC-MS monitoring indicated complete consumption of the starting material. The reaction mixture was diluted with ethyl acetate and extracted with a saturated sodium bicarbonate solution. The organic phase was washed with a saturated sodium chloride aqueous solution, dried over anhydrous sodium sulfate, and concentrated. The crude product was purified by preparative HPLC(Prep-HPLC) to obtain a compound 4 (1.9 mg, 16%). MS (ESI): m/z found $[M+H]^+$ = 637.2.

Example 5:

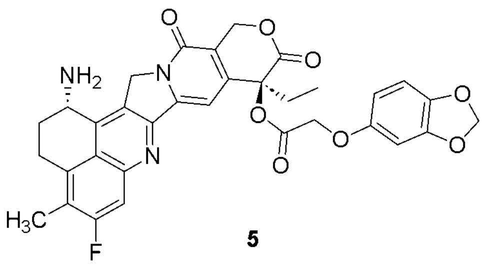

**5**

Synthetic route:

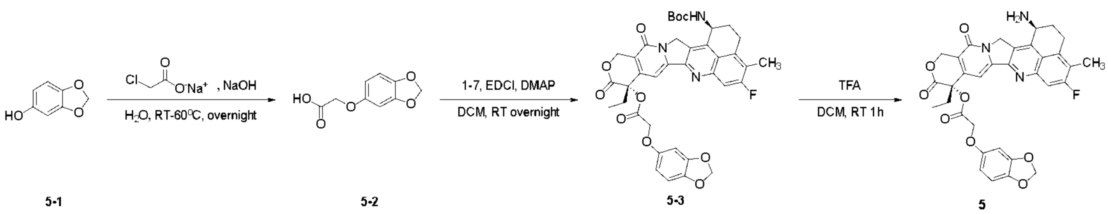


Synthesis of Intermediate 5-2:

At room temperature, sodium chloroacetate (928 mg, 8.0 mmol) was dissolved in water (5 mL), and sodium hydroxide (319 mg, 8.0 mmol) was dissolved in water (5 mL). The two solutions were mixed, and then 3,4-methylenedioxyphenol (1.0 g, 7.2 mmol) was added. The reaction proceeded for 10 minutes, then heated to 60°C and the reaction proceeded overnight. LCMS monitoring indicated completion of the reaction. The reaction mixture was adjusted to pH 2 with hydrochloric acid. The precipitated solid was filtered and the filtrate was recrystallized with petroleum ether and ethyl acetate. Concentration was perfomed to obtain a compound 5-2 (485 mg, 34%), MS (ESI): m/z found $[M+H]^+$ = 197.0.

Synthesis of Intermediate 5-3:

At room temperature, the compound 1-7 (50 mg, 0.093 mmol) was dissolved in ultra-dry dichloromethane (5 mL). The compound 5-2 (36 mg, 0.19 mmol), 1-(3-dimethylaminopropyl)-3-ethylcarbodiimide hydrochloride (80 mg, 0.45 mmol), and 4-dimethylaminopyridine (8 mg, 0.06 mmol) were added sequentially. The reaction proceeded overnight. LC-MS monitoring indicated completion of the reaction. The reaction mixture was concentrated and purified by PTLC to obtain a compound 5-3 (53 mg, 80%), MS (ESI): m/z found $[M+H]^+$ = 714.2.

Synthesis of Compound 5:

In an ice-water bath, the compound 5-3 (53 mg, 0.08 mmol) was dissolved in ultra-dry dichloromethane (5 mL). Trifluoroacetic acid (0.5 mL) was added, and the reaction proceeded for 1 hour. LCMS monitoring indicated complete consumption of the starting material. The reaction mixture was dried and concentrated, then purified by PTLC to obtain a compound 5 (34 mg, 72%), MS (ESI): m/z found $[M+H]^+$ = 614.2.

Example 6:

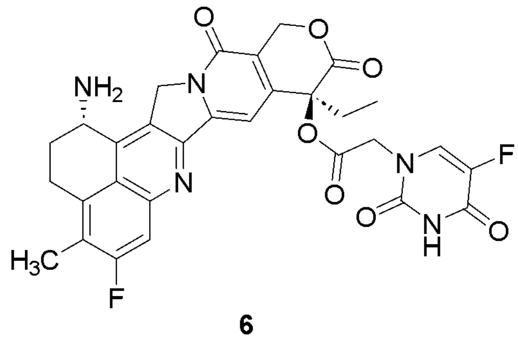

**6**

Synthetic route:

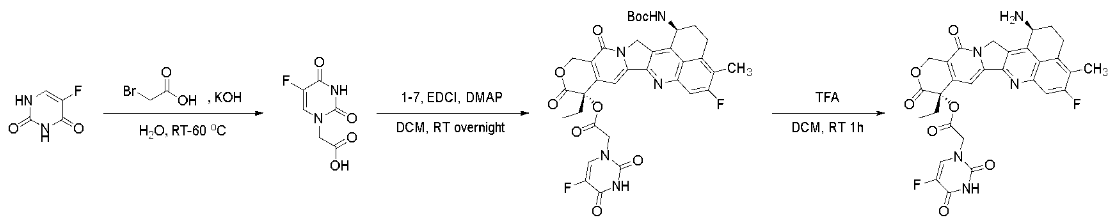


**6-1** **6-2** **6-3** **6**

Synthesis of Intermediate 6-2:

At room temperature, potassium hydroxide (1.8 g, 30.8 mmol) was dissolved in water (20 mL). 5-Fluorouracil (6-1, 1.0 g, 7.7 mmol) was added, and the reaction proceeded at 60°C for 1 hour. After the reaction mixture was cooled to room temperature, bromoacetic acid (1.5 g, 10.8 mmol) was added, and the reaction mixture was heated to 60°C and the reaction proceeded for 5 hours. After the reaction mixture was cooled to room temperature, hydrochloric acid was added to adjust the pH to 2, and the reaction mixture was stirred overnight at room temperature. The precipitated solid was filtered and the filtrate was concentrated to obtain a compound 6-2 (710 mg, 49%), MS (ESI): m/z found $[M+H]^+$ = 189.0.

Synthesis of Intermediate 6-3:

At room temperature, the compound 1-7 (50 mg, 0.09 mmol) was dissolved in ultra-dry dichloromethane (5 mL). The compound 6-2 (35 mg, 0.19 mmol), 1-(3-dimethylaminopropyl)-3-ethylcarbodiimide hydrochloride (80 mg, 0.45 mmol), and 4-dimethylaminopyridine (8 mg, 0.06 mmol) were added sequentially. The reaction proceeded overnight.

LCMS monitoring indicated completion of the reaction. The reaction mixture was concentrated and purified by PTLC to obtain a compound 6-3 (50 mg, 83%), MS (ESI): m/z found $[M+H]^+$ = 706.2.

Synthesis of Compound 6:

In an ice-water bath, the compound 6-3 (50 mg, 0.07 mmol) was dissolved in ultra-dry dichloromethane (5 mL). Trifluoroacetic acid (0.5 mL) was added, and the reaction proceeded for 1 hour. LCMS monitoring indicated complete consumption of the starting material. The reaction mixture was dried and concentrated, then purified by Prep-HPLC to obtain a compound 6 (34 mg, 70%), MS (ESI): m/z found $[M+H]^+$ = 606.2.

Example 7:

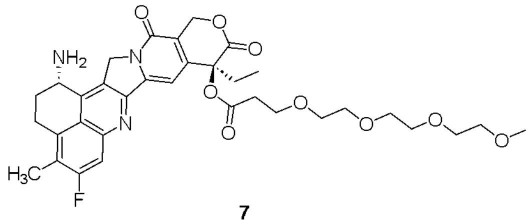

7

Synthetic route:

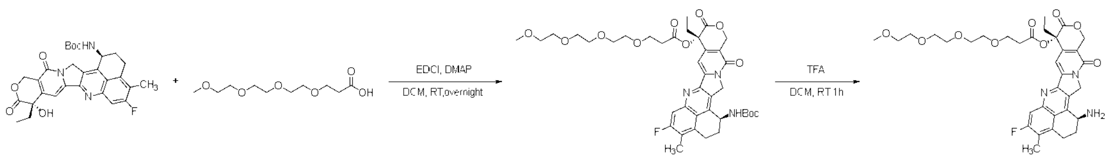


1-7 7-1 7-2 7

Synthesis of Intermediate 7-2:

At room temperature, the compound 1-7 (50 mg, 0.09 mmol) was dissolved in ultra-dry dichloromethane (5 mL). 4,7,10,13-Tetraoxatetradecanoic acid (43 mg, 0.182 mmol), 1-(3-dimethylaminopropyl)-3-ethylcarbodiimide hydrochloride (80 mg, 0.449 mmol), and 4-dimethylaminopyridine (7.4 mg, 0.060 mmol) were added sequentially. The reaction proceeded overnight. LC-MS monitoring indicated completion of the reaction. The reaction mixture was concentrated and purified by PTLC to obtain a compound 7-2 (48 mg, 68%), MS (ESI): m/z found $[M+H]^+$ = 637.2.

Synthesis of Compound 7:

In an ice-water bath, the compound 7-2 (48 mg, 0.064 mmol) was dissolved in ultra-dry dichloromethane (5 mL). Trifluoroacetic acid (1.0 mL) was added, and the reaction proceeded for 1 hour. LC-MS monitoring indicated complete consumption of the starting material. The reaction mixture was dried and concentrated, then purified by Prep-HPLC to obtain a compound 7 (35.87 mg, 86%), MS (ESI): m/z found $[M+H]^+$ = 654.0.

Example 8:

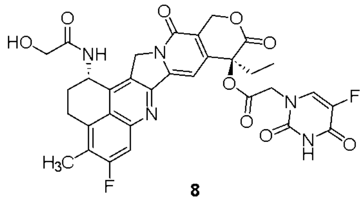

8

Synthetic route:

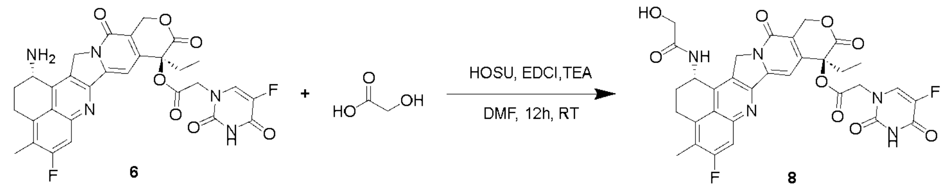


Synthesis of Example 8:

Glycolic acid (19 mg, 0.248 mmol) was dissolved in DMF (3 mL). HOSU (28.5 mg, 0.248 mmol) and EDCI (48 mg, 0.248 mmol) were added, and the reaction proceeded at room temperature for 1 hour. The compound 6 (100 mg, 0.165 mmol) and TEA (18.4 mg, 0.182 mmol) were then added, and the reaction proceeded at room temperature overnight. After LC-MS monitoring indicated complete consumption of the starting material, the reaction mixture was purified by prep-HPLC and lyophilized to obtain a white solid product (28.4 mg, 26.0%). MS (ESI): m/z found $[M+H]^+$= 664.0.

Example 9:

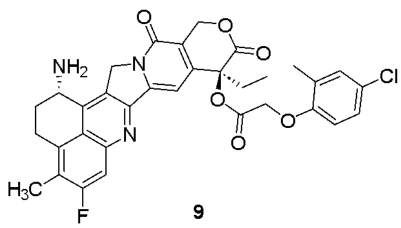

9

Synthetic route:

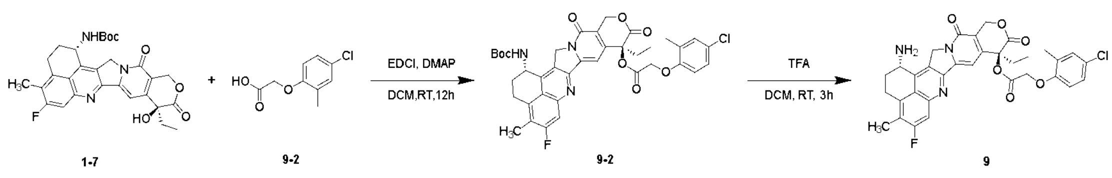


Synthesis of Intermediate 9-2:

The compound 1-7 (30 mg, 0.056 mmol) was dissolved in DCM (3 mL). 2-Methyl-4-chlorophenoxyacetic acid (20 mg, 0.098 mmol), EDCI (33 mg, 0.169 mmol), and DMAP (4.1 mg, 0.034 mmol) were added. The reaction proceeded at room temperature overnight. After LC-MS monitoring indicated completion of the reaction, the reaction mixture was extracted with water/DCM, and concentrated under reduced pressure and purified by preparative TLC (DCM:MeOH = 10:1) to obtain a white solid compound 9-2(31 mg, 77.2%). MS (ESI): m/z found $[M+H]^+$ = 718.0.

Synthesis of Compound 9:

The compound 9-2 (30 mg, 0.042 mmol) was dissolved in DCM (3 mL). TFA (0.3 mL) was added, and the reaction proceeded at room temperature for 3 hours. After LC-MS monitoring indicated completion of the reaction, the reaction mixture was distilled under reduced pressure and spin-dried. Purification by preparative HPLC and lyophilization were perfomed to obtain a white solid product (11.9 mg, 45.9%). MS (ESI): m/z found $[M+H]^+$ = 618.0.

Example 10:

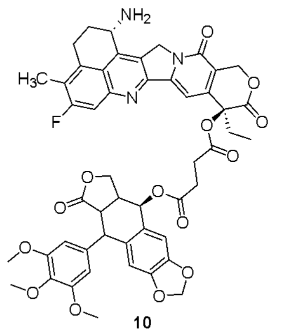

10

Synthetic route:

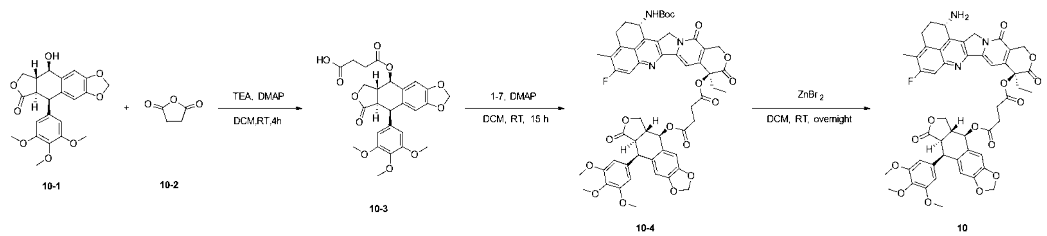


Synthesis of Intermediate 10-3:

At room temperature, podophyllotoxin (10-1, 100 mg, 0.241 mmol) was dissolved in DCM (10 mL). Triethylamine (49 mg, 0.482 mmol), succinic anhydride (25 mg, 0.241 mmol), and DMAP (3.0 mg, 0.024 mmol) were added sequentially. The reaction proceeded at room temperature for 4 hours. TLC monitoring indicated completion of the reaction. The reaction mixture was spin-dried and concentrated and purified by column chromatography (DCM:MeOH = 10:1) to obtain a compound 10-3 (125 mg, 99% ). MS (ESI): m/z found $[M-H]^-$ = 513.0.

Synthesis of Intermediate 10-4:

The compound 10-3 (50 mg, 0.093 mmol) was dissolved in dry DCM. The compound 1-7 (94 mg, 0.182 mmol) was added, and the reaction proceeded at room temperature overnight. TLC monitoring indicated complete consumption of the starting material. The reaction mixture was spin-dried and concentrated and purified by column chromatography (DCM:MeOH = 10:1) to obtain a compound 10-4 (37 mg, 39% ). MS (ESI): m/z found $[M+H]^+$ = 1032.2.

Synthesis of Compound 10:

At room temperature, the compound 10-4 (10 mg, 0.0097 mmol) was dissolved in dry DCM (5 mL). Zinc bromide (4.4 mg, 0.0194 mmol) was added, and the reaction proceeded at room temperature for 15 hours. LC-MS monitoring indicated completion of the reaction. The reaction mixture was spin-dried and concentrated. The residue was dissolved in a small amount of DMF and purified by prep-HPLC to obtain a compound 10 (2.86 mg, 32% ). MS (ESI): m/z found $[M+H]^+$ = 932.4.

Example 11:

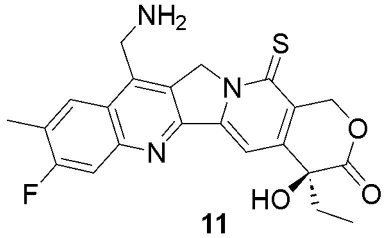

11

Synthetic route:

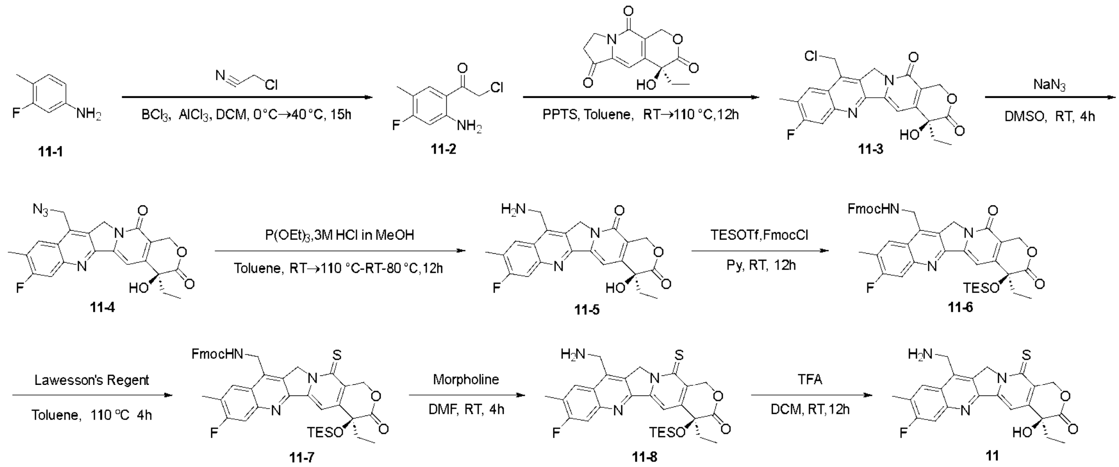


Synthesis of Intermediate 11-2:

In an ice-water bath, boron trichloride (32 mL, 31.96 mmol) was dissolved in DCM (80 mL). 3-Fluoro-4-methylaniline (11-1, 5 g, 39.95 mmol), chloroacetonitrile (3.53 g, 46.95 mmol), and aluminum chloride (6.9 g, 51.94 mmol) were added sequentially. The reaction mixture was stirred at 0°C for 10 minutes, warmed to room temperature, stirred for another 10 minutes, and then heated to 40°C overnight. TLC monitoring indicated completion of the reaction. The reaction mixture was cooled in an ice-water bath, and water (30 mL) was added. After being stirred in the ice bath for 10 minutes, 2N HCl solution was added, and the reaction mixture was stirred for 1 hour. The reaction mixture was diluted with DCM, washed with water for 3 times, and the organic phases were combined. The combined organic phase was dried over anhydrous $Na_2SO_4$, and concentrated. Purification by column chromatography (DCM:MeOH = 10:1) was perfomed to obtain a compound 11-2 (2.15 g, 27%). MS (ESI): m/z found $[M+H]^+$ = 202.0.

Synthesis of Intermediate 11-3:

At room temperature, the compound 11-2 (500 mg, 2.487 mmol) was dissolved in toluene (5 mL). (S)-4-Ethyl-4-hydroxy-7,8-dihydro-1H-pyrano[3,4-f]indolizine -3,6,10(4H)-trione (624 mg, 2.369 mmol) and PPTS (30 mg, 0.118 mmol) were added. The atmosphere was purged with nitrogen three times and the reaction mixture was heated to 110°C, the reaction proceeded overnight. LC-MS monitoring indicated completion of the reaction. The reaction mixture was spin-dried and concentrated, dissolved in a small amount of DMF, and purified by reverse-phase column chromatography ($H_2O$:$CH_3CN$ = 45:55) to obtain a compound 11-3 (467 mg, 46%). MS (ESI): m/z found $[M+H]^+$ = 429.0.

Synthesis of Intermediate 11-4:

At room temperature, the compound 11-3 (754 mg, 1.758 mmol) was dissolved in dry DMSO (6 mL). Sodium azide (114 mg, 1.758 mmol) was added, and the reaction proceeded at room temperature for 4 hours. LC-MS monitoring

indicated completion of the reaction. The reaction mixture was cooled in an ice bath, water (50 mL) was added, and was stirred for 10 minutes. The reaction mixture was filtered and the filter cake was dried to obtain a compound 11-4 (603 mg, 78%). MS (ESI): m/z found $[M+H]^+$ = 436.0.

Synthesis of Intermediate 11-5:

At room temperature, the compound 11-4 (600 mg, 1.378 mmol) was dissolved in toluene (10 mL). Triethyl phosphite (590 μL, 3.445 mmol) was added, and the reaction mixture was heated to 110°C, and the reaction proceeded for 4 hours. After the reaction mixture was cooled to room temperature, 3N HCl in methanol (5 mL) was added, and the reaction mixture was heated to 80°C, and the reaction proceeded overnight. LC-MS monitoring indicated completion of the reaction. The reaction mixture was concentrated and purified by reverse-phase column chromatography to obtain a compound 11-5 (360 mg, 63%). MS (ESI): m/z found $[M+H]^+$ = 410.0.

Synthesis of Intermediate 11-6:

At room temperature, the compound 11-5 (64 mg, 0.156 mmol) was dissolved in dry pyridine (5 mL). TESOTf (340 μL, 1.56 mmol) was added, and the reaction proceeded overnight. TLC monitoring indicated completion of the reaction. FmocCl (81 mg, 0.312 mmol) was added, and the reaction proceeded for 2 hours. TLC monitoring indicated completion of the reaction. The reaction mixture was dried, diluted with DCM, washed with water for three times and washed with saturated NaCl solution once, dried over anhydrous $Na_2SO_4$, and concentrated. Purification by column chromatography (DCM:MeOH = 95:5) was perfomed to obtain a compound 11-6 (105 mg, 90% yield).

Synthesis of Intermediate 11-7:

At room temperature, the compound 11-6 (105 mg, 0.141 mmol) was dissolved in toluene (5 mL). Lawesson's reagent (143 mg, 0.352 mmol) was added, and the reaction mixture was heated to 110°C, and the reaction proceeded for 4 hours. TLC monitoring indicated completion of the reaction. The reaction mixture was concentrated, dissolved in a small amount of DCM, and purified by column chromatography (DCM:MeOH = 95:5) to obtain a compound 11-7 (102 mg, 95%).

Synthesis of Intermediate 11-8:

At room temperature, the compound 11-7 (102 mg, 0.134 mmol) was dissolved in dry DMF (3 mL). Morpholine (300 μL) was added, and the reaction proceeded for 4 hours. TLC monitoring indicated completion of the reaction. The reaction mixture was purified by reverse-phase column chromatography ($H_2O$:$CH_3CN$ = 45:55) to obtain a compound 11-8 (47 mg, 65% yield). MS (ESI): m/z found $[M+H]^+$ = 540.2.

Synthesis of Compound 11:

At room temperature, the compound 11-8 (47 mg, 0.0871 mmol) was dissolved in dry DCM (5 mL). Trifluoroacetic acid (1 mL) was added, and the reaction proceeded at room temperature overnight. LC-MS monitoring indicated completion of the reaction. The reaction mixture was purified by reverse-phase column chromatography ($H_2O$:$CH_3CN$ = 45:55) to obtain a compound 11 (32 mg, 86%). MS (ESI): m/z found $[M+H]^+$ = 426.0.

Example **12:**

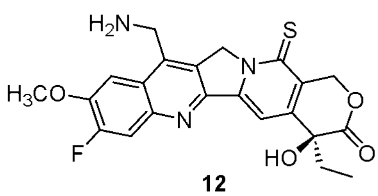

**12**

Synthetic route:

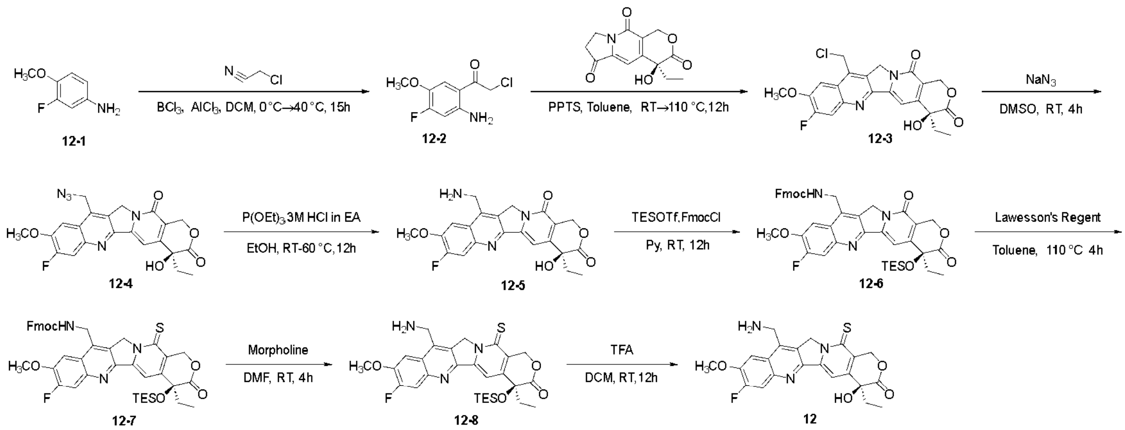


Synthesis of Intermediate 12-2:

DCM (50 mL) was added to an erlenmeyer flask and cooled in an ice bath. Boron trichloride (11.34 mL, 11.34 mmol) was added, and the reaction mixture was kept in the ice bath for 10 minutes. 3-Fluoro-4-methoxyaniline (12-1, 2 g, 14.17 mmol) was added, and the reaction mixture was kept in the ice bath for another 10 minutes. Chloroacetonitrile (1.05 mL, 16.65 mmol) was added, followed by keeping in the ice bath for 10 minutes. Aluminum chloride (2.46 g, 18.42 mmol) was added, and the reaction mixture was stirred at room temperature for 10 minutes. The reaction mixture was heated to 40°C, and the reaction proceeded overnight. After LCMS monitoring indicated completion of the reaction, water (15 mL) was added under an ice bath, and the reaction mixture was stirred for 10 minutes. Hydrochloric acid (4 mL, 2 M) was added, and the reaction mixture was stirred at room temperature for 1.5 hours. The reaction mixture was filtered under suction, and the filtrate was extracted with water/DCM. Purification by normal-phase column chromatography (PE:EA = 2:1) was performed to obtain a yellow solid product (668.8 mg, 21.7%). MS (ESI): m/z found $[M+H]^+$ = 218.15.

Synthesis of Intermediate 12-3:

The compound 12-2 (668.8 mg, 3.07 mmol) was dissolved in toluene (15 mL). 7-Ethyl-10-hydroxycamptothecin intermediate (808.2 mg, 3.07 mmol) and PPTS (38.7 mg, 0.154 mmol) were added. The reaction proceeded at 110°C overnight. After LC-MS monitoring indicated completion of the reaction, the reaction mixture was filtered under suction. The solid was washed with ethanol and dried to obtain a pale yellow solid product (974.4 mg, 71.5%). MS (ESI): m/z found $[M+H]^+$ = 445.25.

Synthesis of Intermediate 12-4:

The compound 12-3 (974.4 mg, 2.19 mmol) was dissolved in DMSO (15 mL). Sodium azide (142.4 mg, 2.19 mmol) was added, and the reaction proceeded at room temperature for 3 hours. After LCMS monitoring indicated completion of the reaction, water (8 mL) was added. The reaction mixture was filtered under suction and dried to obtain a brown solid product (801.4 mg). MS (ESI): m/z found $[M+H]^+$ = 452.30.

Synthesis of Intermediate 12-5:

The compound 12-4 (801.4 mg, 1.78 mmol) was dissolved in ethanol (10 mL). Triethyl phosphite (739.54 mg, 4.45 mmol) was added, and the reaction proceeded at 50°C for 4 hours. After the reaction mixture was cooled to room temperature, a solution of HCl in ethyl acetate (19 mL) was added, and the reaction proceeded at 60°C overnight. After LCMS monitoring indicated completion of the reaction, the reaction mixture was filtered under suction to obtain a pale yellow solid product (550.8 mg, 72.8%). MS (ESI): m/z found $[M+H]^+$ = 426.30.

Synthesis of Intermediate 12-6:

The compound 12-5 (550.8 mg, 1.29 mmol) was dissolved in pyridine (10 mL). TESOTf (2.8 mL, 12.9 mmol) was added, and the reaction proceeded at room temperature overnight. After LCMS monitoring indicated completion of the reaction, FmocCl (667.4 mg, 2.58 mmol) was added, and the reaction proceeded at room temperature for 3 hours. TLC monitoring indicated completion of the reaction. The pyridine was spin-dried, and extracted with $H_2O$/DCM. Purification by normal-phase column chromatography was performed to obtain a yellow solid product (467.5 mg, 47.6%).

Synthesis of Intermediate 12-7:

Under nitrogen protection, the compound 12-6 (467.5 mg, 0.61 mmol) was dissolved in toluene (10 mL). Lawesson's reagent (744.1 mg, 1.84 mmol) was added, and the reaction proceeded at 110°C for 4 hours. After TLC monitoring indicated completion of the reaction, the reaction mixture was distilled under reduced pressure and spin-dried, and purified by normal-phase column chromatography to obtain a yellow solid product (389.3 mg, 82.1%).

Synthesis of Intermediate 12-8:

The compound 12-7 (389.3 mg, 0.5 mmol) was dissolved in DMF (5 mL). Morpholine (2 mL) was added, and the reaction proceeded at room temperature for 5 hours. After LCMS monitoring indicated completion of the reaction, the reaction mixture was distilled under reduced pressure and concentrated. The residue was extracted with saturated NaCl solution/DCM and purified by normal-phase column chromatography (DCM:MeOH = 47:3) to obtain a brown solid product (134.6 mg, 48.4%). MS (ESI): m/z found $[M+H]^+$ = 556.45.

Synthesis of Compound 12:

The compound 12-8 (134.6 mg, 0.24 mmol) was dissolved in DCM (5 mL). TFA (1 mL) was added, and the reaction proceeded at room temperature overnight. After LCMS monitoring indicated completion of the reaction, the reaction mixture was spin-dried. Purification by reverse-phase column chromatography ($H_2O$:ACN = 4:1) and lyophilization were performed to obtain an orange-red solid product (54.9 mg, 51.8%). MS(ESI):m/z found $[M+1]^+$=442.30. $^1H$ NMR (400 MHz, DMSO-$d_6$) δ 8.15 (s, 1H), 8.04 (d, *J* = 12.1 Hz, 1H), 7.79 (d, *J* = 8.6 Hz, 2H), 6.70 (s, 1H), 5.93 (d, *J* = 16.6 Hz, 1H), 5.76 (s, 2H), 5.53 (d, *J* = 16.6 Hz, 1H), 4.48 (s, 2H), 4.11 (s, 3H), 1.90 (dt, *J* = 10.5, 6.8 Hz, 2H), 0.86 (t, *J* = 7.3 Hz, 3H).

Example 13:

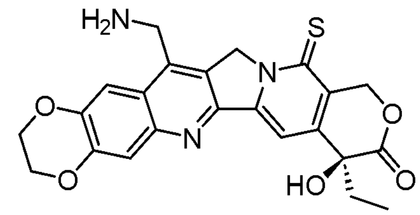

**13**

Synthetic route:

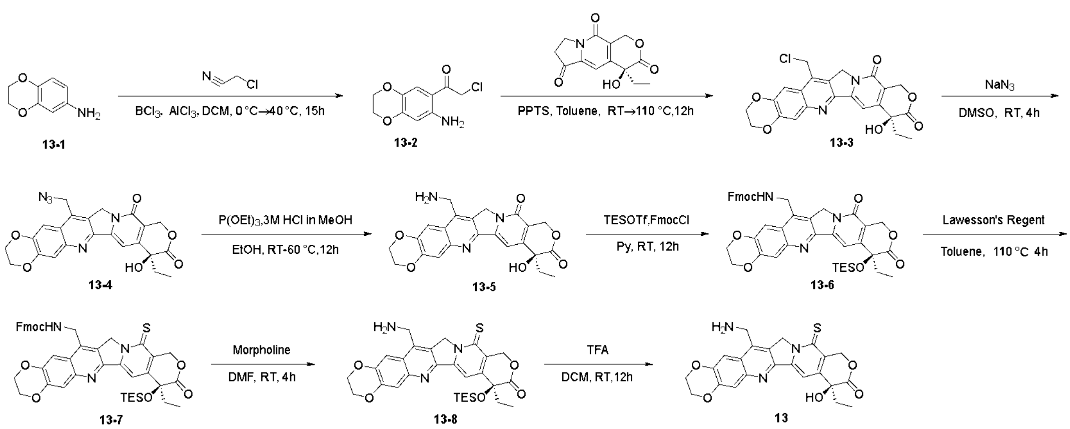

13-1 13-2 13-3 13-4 13-5 13-6 13-7 13-8 13

Synthesis of Intermediate 13-2:

In an ice-water bath, boron trichloride (26 mL, 26 mmol) was dissolved in dichloromethane (80 mL). 6-Amino-1,4-benzodioxane (13-1, 5 g, 33 mmol), chloroacetonitrile (3.01 g, 40 mmol), and aluminum chloride (5.7 g, 43 mmol) were added sequentially. The reaction mixture was stirred at 0°C for 10 minutes, warmed to room temperature and the reaction proceeded for another 10 minutes, then the reaction mixture was heated to 40°C and the reaction proceeded overnight. TLC monitoring indicated completion of the reaction. The reaction mixture was cooled in an ice-water bath, and water (30 mL) was added. After being stirred in the ice bath for 10 minutes, 2N HCl solution was added, and the reaction mixture was stirred for 1 hour. The reaction mixture was diluted with dichloromethane, washed with water for three times, the organic phases were combined and dried over anhydrous $Na_2SO_4$, and concentrated. Purification by column chromatography (DCM:MeOH = 10:1) was perfomed to obtain a compound 13-2 (1.8 g, 24%). MS (ESI): m/z found $[M+H]^+$ = 228.00.

Synthesis of Intermediate 13-3:

At room temperature, the compound 13-2 (2.6 g, 11.42 mmol) was dissolved in toluene (60 mL). (S)-4-Ethyl-4-hydroxy-7,8-dihydro-1H-pyrano[3,4-f]indolizine-3,6,10(4H)-trione (3.0 g, 11.42 mmol) and PPTS (144 mg, 0.57 mmol) were added. The atmosphere was purged with nitrogen for three times and the reaction mixture was heated to 110°C, and the reaction proceeded overnight. LC-MS monitoring indicated completion of the reaction. After being cooled, the reaction mixture was purified by slurry washing with ethanol to obtain a compound 13-3 (2.74 g, 54%). MS (ESI): m/z found $[M+H]^+$ = 455.30.

Synthesis of Intermediate 13-4:

At room temperature, the compound 13-3 (2.74 g, 6.02 mmol) was dissolved in dry DMSO (6 mL). Sodium azide (392 mg, 6.02 mmol) was added, and the reaction proceeded at room temperature for 4 hours. LC-MS monitoring indicated completion of the reaction. The reaction mixture was cooled in an ice bath, water (50 mL) was added, and the reaction mixture was filtered after being stirred for 10 minutes. The filter cakewas collected and dried to obtain a compound 13-4 (2.32 g, 83%). MS (ESI): m/z found $[M+H]^+$ = 462.30.

Synthesis of Intermediate 13-5:

At room temperature, the compound 13-4 (2.32 g, 5.019 mmol) was dissolved in ethanol (20 mL). Triethyl phosphite (2.15 mL, 12.548 mmol) was added, and the reaction mixture was heated to 50°C and the reaction proceeded for 4 hours. After the reaction mixture was cooled to room temperature, 3N HCl in methanol (30 mL) was added, and the reaction mixture was heated to 60°C, and the reaction proceeded overnight. LC-MS monitoring indicated completion of the reaction. After the reaction mixture was cooled to room temperature, the precipitated solid was filtered and washed with ethanol to obtain a compound 13-5 (1.657 g, 73%). MS (ESI): m/z found $[M+H]^+$ = 436.0.

Synthesis of Intermediate 13-6:

At room temperature, the compound 13-5 (1.657 g, 3.805 mmol) was dissolved in dry pyridine (15 mL). Triethylsilyl trifluoromethanesulfonate (8.2 mL, 38.05 mmol) was added, and the reaction proceeded overnight. TLC monitoring indicated completion of the reaction. 9-Fluorenylmethyl chloroformate (1.97 g, 7.610 mmol) was added, and the reaction proceeded for 2 hours. TLC monitoring indicated completion of the reaction. The reaction mixture was dried, diluted with dichloromethane, washed with water for three times ($3 \times 50$ mL) and washed with saturated NaCl solution once ($1 \times 50$ mL), dried over anhydrous $Na_2SO_4$, and concentrated. Purification by column chromatography (DCM:MeOH = 95:5) was perfomed to obtain a compound 13-6 (1.58 g, 58% yield).

Synthesis of Intermediate 13-7:

At room temperature, the compound 36-6 (1.58 g, 2.047 mmol) was dissolved in toluene (5 mL). Lawesson's reagent (2.5 g, 6.140 mmol) was added, and the reaction mixture was heated to 100°C, and the reaction proceeded for 4 hours. TLC monitoring indicated completion of the reaction. The reaction mixture was concentrated, dissolved in a small amount of dichloromethane, and purified by column chromatography (DCM:MeOH = 95:5) to obtain a compound 13-7 (499 mg, 31% yield).

Synthesis of Intermediate 13-8:

At room temperature, the compound 13-7 (499 mg, 0.633 mmol) was dissolved in dry DMF (5 mL). Morpholine (2 mL) was added, and the reaction proceeded for 4 hours. TLC monitoring indicated completion of the reaction. The reaction mixture was diluted with dichloromethane, washed with water for three times and washed with saturated NaCl solution once (1 × 20 mL), dried over anhydrous $Na_2SO_4$, and concentrated. Purification by normal-phase column chromatography (DCM:MeOH = 95:5) was perfomed to obtain a compound 13-8 (317 mg, 88% yield). MS (ESI): m/z found $[M+H]^+$ = 566.20.

Synthesis of Compound 13:

At room temperature, the compound 13-8 (257 mg, 0.453 mmol) was dissolved in dry dichloromethane (5 mL). Trifluoroacetic acid (2 mL) was added, and the reaction proceeded at room temperature overnight. LC-MS monitoring indicated completion of the reaction. The reaction mixture was purified by reverse-phase column chromatography ($H_2O$:$CH_3CN$ = 45:55) to obtain a compound 13 (112 mg, 55%). MS (ESI): *m/z* found $[M+H]^+$= 452.35. $^1H$ NMR (600 MHz, DMSO-$d_6$) δ 8.32 (s, 2H), 7.92 (s, 1H), 7.79 (s, 1H), 7.71 (s, 1H), 6.71 (s, 1H), 5.94 (d, *J*= 16.5 Hz, 1H), 5.77 (d, *J* = 2.0 Hz, 2H), 5.54 (d, *J* = 16.5 Hz, 1H), 4.69 (d, *J* = 5.9 Hz, 2H), 4.54 - 4.46 (m, 4H), 1.97 - 1.85 (m, *J* = 7.2 Hz, 2H), 0.87 (t, *J* = 7.3 Hz, 3H).

Example **14:**

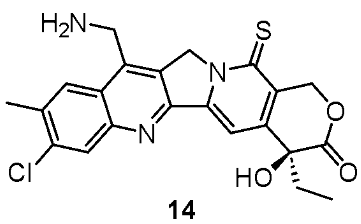

**14**

Synthetic route:

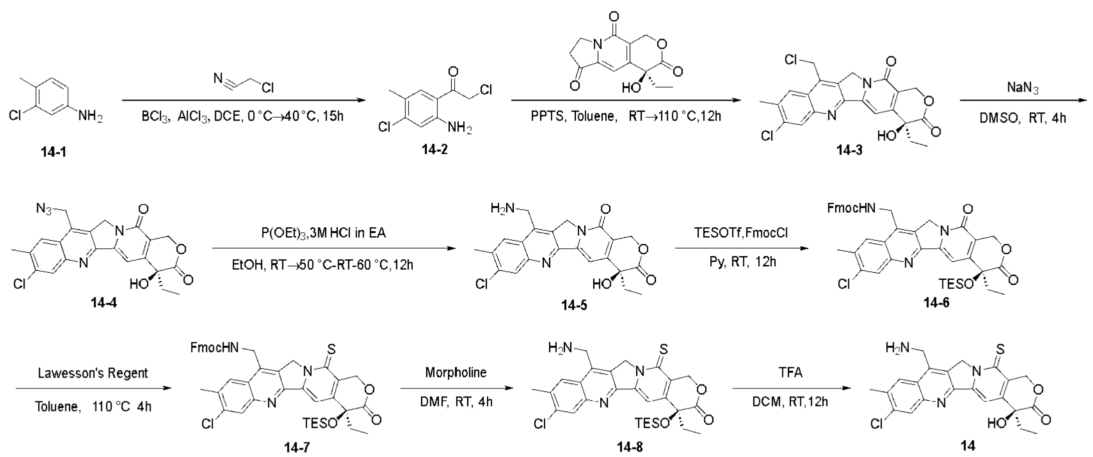


Synthesis of Intermediate 14-2:

DCE (300 mL) was added to an erlenmeyer flask and cooled in an ice bath. Boron trichloride (56.5 mL, 56.5 mmol) was added, and the reaction mixture was kept in the ice bath for 10 minutes. 3-Chloro-p-toluidine (14-1, 10 g, 70.6 mmol) was added, and the reaction mixture was kept in the ice bath for another 10 minutes. Chloroacetonitrile (5.25 mL, 82.96 mmol) was added, and the reaction mixture was kept in the ice bath for 10 minutes. Aluminum chloride (12.24 g, 91.78 mmol) was added, and the reaction mixture was stirred at room temperature for 10 minutes and then heated to 80°C, and

the reaction proceeded overnight. After LCMS monitoring indicated completion of the reaction, water (80 mL) was added under ice bath, and the reaction mixture was stirred for 10 minutes. Hydrochloric acid (20 mL, 2 M) was added, and the reaction mixture was stirred at room temperature for 1.5 hours. The reaction mixture was filtered under suction, and the filtrate was extracted with water/DCM. Purification by normal-phase column chromatography (PE:EA = 2:1) was performed to obain a yellow solid product (2.96 g, 19.2%). MS (ESI): m/z found $[M+H]^+$ = 218.0.

Synthesis of Intermediate 14-3:

The compound 14-2 (2.96 g, 13.57 mmol) was dissolved in toluene (65 mL). 7-Ethyl-10-hydroxycamptothecin intermediate (3.57 g, 13.57 mmol) and PPTS (170.6 mg, 0.679 mmol) were added. The reaction proceeded at 110°C overnight. After LCMS monitoring indicated completion of the reaction, the reaction mixture was filtered under suction. The solid was washed with ethanol and dried to obtain a pale yellow solid product (5 g, 82.8%). MS (ESI): m/z found $[M+H]^+$ = 445.0.

Synthesis of Intermediate 14-4:

The compound 14-3 (5 g, 11.22 mmol) was dissolved in DMSO (80 mL). Sodium azide (729.4 mg, 11.22 mmol) was added, and the reaction proceeded at room temperature for 3 hours. After LCMS monitoring indicated completion of the reaction, water (80 mL) was added. The reaction mixture was filtered under suction and dried to obtain a brown solid product (4.88 g). MS (ESI): m/z found $[M+H]^+$ = 452.0.

Synthesis of Intermediate 14-5:

The compound 14-4 (4.88 g, 10.8 mmol) was dissolved in ethanol (50 mL). Triethyl phosphite (4.63 mL, 27 mmol) was added, and the reaction proceeded at 50°C for 4 hours. After the reaction mixture was cooled to room temperature, a solution of HCl in ethyl acetate (60 mL) was added, and the reaction proceeded at 60°C overnight. After LCMS monitoring indicated completion of the reaction, the reaction mixture was filtered under suction to obtain a pale yellow solid product (4.39 g, 95.4%). MS (ESI): m/z found $[M+H]^+$ = 426.0.

Synthesis of Intermediate 14-6:

The compound 14-5 (4.39 g, 10.31 mmol) was dissolved in pyridine (40 mL). TESOTf (22.19 mL, 103.1 mmol) was added, and the reaction proceeded at room temperature overnight. After LCMS monitoring indicated completion of the reaction, FmocCl (5.34 g, 20.62 mmol) was added, and the reaction proceeded at room temperature for 3 hours. TLC monitoring indicated completion of the reaction. The pyridine was spin-dried, and the residue was extracted with $H_2O$/DCM. Purification by normal-phase column chromatography was performed to obtain a yellow solid product (2.94 g, 97%).

Synthesis of Intermediate 14-7:

Under nitrogen protection, the compound 14-6 (2.94 g, 3.86 mmol) was dissolved in toluene (30 mL). Lawesson's reagent (4.68 g, 11.58 mmol) was added, and the reaction proceeded at 110°C for 4 hours. After TLC monitoring indicated completion of the reaction, the reaction mixture was distilled under reduced pressure and spin-dried, and purified by normal-phase column chromatography to obtain a yellow solid product (1.51 g, 50.3%).

Synthesis of Intermediate 14-8:

The compound 14-7 (1.5 g, 1.97 mmol) was dissolved in DMF (5 mL). Morpholine (2 mL) was added, and the reaction proceeded at room temperature for 5 hours. After LCMS monitoring indicated completion of the reaction, the reaction mixture was distilledunder reduced pressure and concentrated. The residue was extracted with saturated NaCl solution/DCM and purified by normal-phase column chromatography (DCM:MeOH = 20:1) to obtain a brown solid product (536.8 mg, 49%). MS (ESI): m/z found $[M+H]^+$ = 556.2.

Synthesis of Compound 14:

The compound 14-8 (536.8 mg, 0.97 mmol) was dissolved in DCM (10 mL). TFA (2 mL) was added, and the reaction proceeded at room temperature overnight. After LCMS monitoring indicated completion of the reaction, the reaction mixture was spin-dried. Purification by reverse-phase column chromatography ($H_2O$:ACN = 3:1) and lyophilization were performed to obtain an orange-red solid product (348.2 mg, 81.2%). MS(ESI):m/z found $[M+H]^+$=442.30. $^1H$ NMR (400 MHz, DMSO-$d_6$) δ 8.40 (s, 1H), 8.31 (d, $J$ = 2.6 Hz, 1H), 8.15 (s, 2H), 7.81 (s, 1H), 6.73 (s, 1H), 5.94 (d, $J$ = 16.6 Hz, 1H), 5.77 (s, 2H), 5.54 (d, $J$ = 16.7 Hz, 1H), 4.52 (s, 1H), 2.61 (s, 3H), 1.90 (dt, $J$ = 9.8, 6.7 Hz, 2H), 0.87 (t, $J$ = 7.3 Hz, 4H).

Example 15:

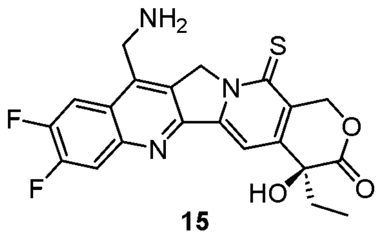

15

Synthetic route:

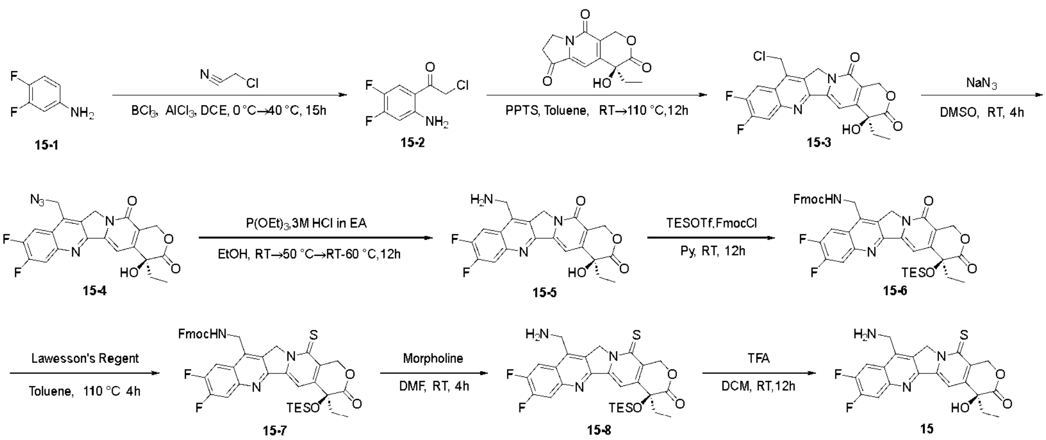


Synthesis of Intermediate 15-2:

1,2-Dichloroethane (DCE, 300 mL) was added to an erlenmeyer flask and cooled in an ice bath. Boron trichloride (61.6 mL, 61.6 mmol) was added, and the reaction mixture was kept in the ice bath for 10 minutes. 3,4-Difluoroaniline (15-1, 10 g, 77.46 mmol) was added, and the reaction mixture was kept in the ice bath for another 10 minutes. Chloroacetonitrile (5.76 mL, 91.02 mmol) was added, and the reaction mixture was kept in the ice bath for 10 minutes. Aluminum chloride (13.4 g, 100.7 mmol) was added, and the reaction mixture was stirred at room temperature for 10 minutes, and the reaction mixture was heated to 80°C, and the reaction proceeded overnight. After LCMS monitoring indicated completion of the reaction, water (80 mL) was added under ice bath, and the reaction mixture was stirred for 10 minutes. Hydrochloric acid (20 mL, 2 M) was added, and the reaction mixture was stirred at room temperature for 1.5 hours. The reaction mixture was filtered under suction, and the filtrate was extracted with water/DCM. Purification by normal-phase column chromatography (PE:EA = 2:1) was performed to obtain a yellow solid product (5.79 g, 36.4%). MS (ESI): m/z found $[M+H]^+$ = 206.0.

Synthesis of Intermediate 15-3:

The compound 15-2 (5.79 g, 28.16 mmol) was dissolved in toluene (150 mL). 7-Ethyl-10-hydroxycamptothecin intermediate (7.4 g, 28.16 mmol) and PPTS (354.3 mg, 1.41 mmol) were added. The reaction proceeded at 110°C overnight. After LC-MS monitoring indicated completion of the reaction, the reaction mixture was filtered under suction. The solid was washed with ethanol and dried to obtain a pale yellow solid product (6.87 g, 56.4%). MS (ESI): m/z found $[M+H]^+$ = 433.0.

Synthesis of Intermediate 15-4:

The compound 15-3 (6.87 g, 15.87 mmol) was dissolved in DMSO (105 mL). Sodium azide (1.03 mg, 15.87 mmol) was added, and the reaction proceeded at room temperature for 3 hours. After LCMS monitoring indicated completion of the reaction, water (80 mL) was added. The reaction mixture was filtered under suction and dried to obtain a brown solid product (5.45 g). MS (ESI): m/z found $[M+H]^+$ = 440.0.

Synthesis of Intermediate 15-5:

The compound 15-4 (5.45 g, 12.4 mmol) was dissolved in ethanol (75 mL). Triethyl phosphite (5.35 mL, 31.03 mmol) was added, and the reaction proceeded at 50°C for 4 hours. After the reaction mixture was cooled to room temperature, a solution of HCl in ethyl acetate (120 mL) was added, and the reaction proceeded at 60°C overnight. After LCMS monitoring indicated completion of the reaction, the reaction mixture was filtered under suction to obtain a pale yellow solid product (3.39 g, 51.67%). MS (ESI): m/z found $[M+H]^+$ = 414.0.

Synthesis of Intermediate 15-6:

The compound 15-5 (3.39 g, 8.2 mmol) was dissolved in pyridine (33 mL). Triethylsilyl trifluoromethanesulfonate (TESOTf, 17.65 mL, 82 mmol) was added, and the reaction proceeded at room temperature overnight. After LCMS monitoring indicated completion of the reaction, FmocCl (4.24 g, 16.4 mmol) was added, and the reaction proceeded at room temperature for 3 hours. TLC monitoring indicated completion of the reaction. The pyridine was spin-dried, and the residue was extracted with $H_2O$/DCM. Purification by normal-phase column chromatography was performed to obtain a yellow solid product (2.59 g, 42.1%).

Synthesis of Intermediate 15-7:

Under nitrogen protection, the compound 15-6 (2.59 g, 3.45 mmol) was dissolved in toluene (65 mL). Lawesson's reagent (4.19 g, 10.35 mmol) was added, and the reaction proceeded at 110°C for 4 hours. After TLC monitoring indicated completion of the reaction, the reaction mixture was distilled under reduced pressure and spin-dried, and purified by normal-phase column chromatography to obtain a yellow solid product (1.69 g, 64%).

Synthesis of Intermediate 15-8:

The compound 15-7 (1.69 g, 2.21 mmol) was dissolved in DMF (10 mL). Morpholine (4 mL) was added, and the reaction proceeded at room temperature for 5 hours. After LCMS monitoring indicated completion of the reaction, the reaction mixture was distilled under reduced pressure and concentrated. The residue was extracted with saturated NaCl solution/DCM and purified by normal-phase column chromatography (DCM:MeOH = 25:2) to obtain a brown solid product (486.8 mg, 40.6%). MS (ESI): m/z found $[M+H]^+$ = 544.4.

Synthesis of Compound 15:

The compound 15-8 (486.8 mg, 0.89 mmol) was dissolved in DCM (10 mL). Trifluoroacetic acid (TFA, 2 mL) was added, and the reaction proceeded at room temperature overnight. After LCMS monitoring indicated completion of the reaction, the reaction mixture was spin-dried. Purification by reverse-phase column chromatography ($H_2O$:ACN = 5:1) and lyophilization were performed to obtain an orange-red solid product (128.8 mg, 51.8%). MS(ESI):m/z found $[M+H]^+$=430.0. $^1H$ NMR (600 MHz, DMSO-$d_6$) $\delta$ 8.53 (dd, $J$= 12.2, 8.5 Hz, 1H), 8.33 (dd, $J$= 11.4, 7.9 Hz, 1H), 7.84 (s, 1H), 6.73 (s, 1H), 5.94 (d, $J$ = 16.5 Hz, 1H), 5.81 (d, $J$ = 2.0 Hz, 2H), 5.55 (d, $J$ = 16.5 Hz, 1H), 4.56 (s, 2H), 1.95 - 1.86 (m, $J$ = 7.3 Hz, 2H), 0.87 (t, $J$ = 7.3 Hz, 3H).

Example 16:

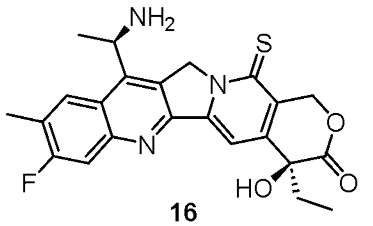

16

Synthetic route:

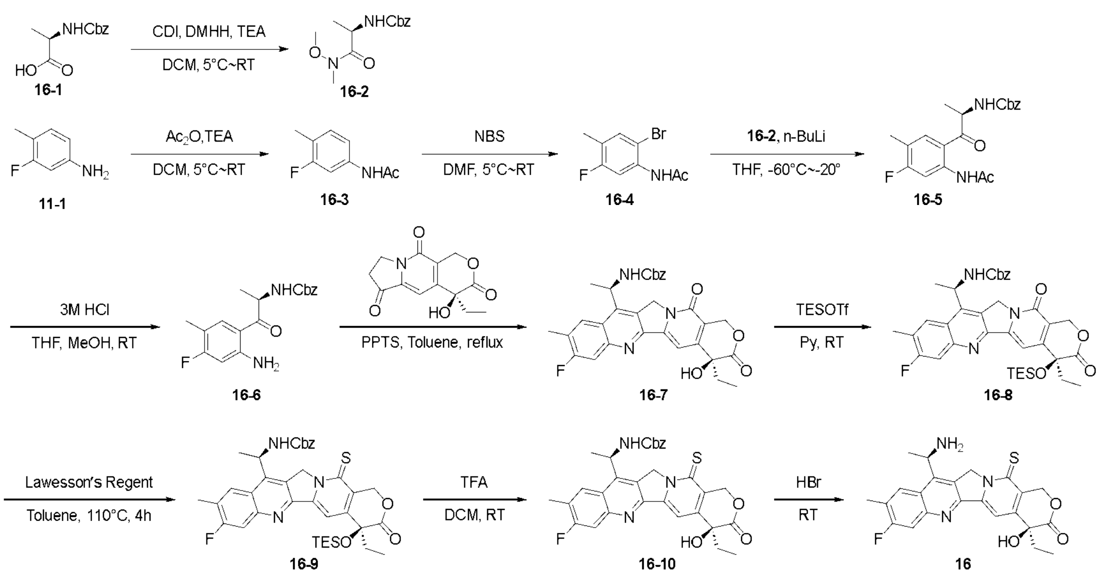


Synthesis of Intermediate 16-2:

In an ice bath, compound 16-1 (10 g, 0.045 mol) was dissolved in DCM (100 mL). CDI (9.5 g, 0.058 mol) was added and the reaction mixture was stirred for 1 hour. N,O-Dimethylhydroxylamine hydrochloride (6.5 g, 0.067 mol) and triethylamine (13.6 g, 0.013 mol) were then added. The reaction mixture was warmed to room temperature and stirred overnight. TLC indicated complete consumption of the starting material. Water (50 mL) was added to quench the reaction, and the reaction mixture was extracted with DCM for three times, and the organic phases were combined. The combined organic phase was washed with 1N HCl once, saturated $NaHCO_3$ solution once, and saturated NaCl solution once, then dried over anhydrous $Na_2SO_4$. After filtration and spin dried, a white solid was obtained (11 g, 92%). LC-MS (ESI): m/z found $[M+H]^+$ = 267.2.

Synthesis of Intermediate 16-3:

In an ice-water bath, 3-fluoro-4-methylaniline (7.8 g, 0.062 mol) was dissolved in DCM (50 mL). Acetic anhydride (12.7 g, 0.124 mol) and triethylamine (20.2 g, 0.2 mol) were added. The reaction mixture was warmed to room temperature and the reaction proceeded for 3 hours. TLC indicated complete consumption of the starting material. Water was added to quench the reaction, and the reaction mixture was extracted with DCM, and the organic phases were combined. The combined organic phase was dried over anhydrous $Na_2SO_4$, filtered, and concentrated. The residue was slurried with petroleum ether to obtain a brown solid 16-3 (11 g, 94.7%). MS (ESI): m/z found $[M+H]^+$ = 168.0.

Synthesis of Intermediate 16-4:

In an ice-water bath, the compound 16-3 (11 g, 0.065 mol) was dissolved in DMF (110 mL). NBS (16.3 g, 0.092 mol) was added portionwise. The reaction mixture was stirred at room temperature overnight. TLC indicated complete consumption of the starting material. The system was poured into ice-water and stirred for 0.5 hours. The reaction mixture was filter, and the filter cake was washed with saturated $NaHCO_3$ solution, and dried to obtain a white solid 16-4 (12 g,

74.5%). MS (ESI): m/z found $[M+H]^+$ = 246.0.

Synthesis of Intermediate 16-5:

The compound 16-4 (2 g, 8.13 mmol) was dissolved in THF. The atmosphere was purged with nitrogen and the system was cooled to -60°C. n-Butyllithium (7.15 mL, 17.9 mmol) was added dropwise. The reaction mixture was stirred at this temperature for 1 hour. A solution of compound 16-2 (3.2 g, 12.19 mmol) in THF was added dropwise at -60°C. The system was then lowered to -20°C and stirred for 1 hour. TLC indicated complete consumption of the starting material. The reaction was quenched with saturated $NH_4Cl$ solution at -20°C and the reaction mixture was extracted with ethyl acetate, and the organic phases were combined. The combined organic phase was dried over anhydrous $Na_2SO_4$, filtered, and purified by medium-pressure normal-phase chromatography to obtain a yellow oily compound (340 mg, 11.23%). LC-MS (ESI): m/z found $[M+H]^+$ = 373.2.

Synthesis of Intermediate 16-6:

The compound 16-5 (150 mg, 0.4 mmol) was dissolved in a mixture of THF (5 mL) and MeOH (5 mL). 3N HCl aqueous solution (5 mL) was added. The reaction mixture was heated to 70°C, and the reaction proceeded overnight. TLC indicated complete consumption of the starting material. After the reaction mixture was cooled to room temperature, the reaction mixture was extracted with ethyl acetate, and the organic phases were combined. The combined organic phase was dried over anhydrous $Na_2SO_4$ and spin-dried to obtain a yellow oily compound 16-6 (110 mg, 82.67%). LC-MS (ESI): m/z found $[M+H]^+$ = 331.2.

Synthesis of Intermediate 16-7:

At room temperature, the compound 16-6 (97.19 mg, 0.35 mmol) was dissolved in toluene (3 mL). (S)-4-Ethyl-4-hydroxy-7,8-dihydro-1H-pyrano[3,4-f]indolizine-3,6,10(4H)-trione (110 mg, 0.33 mmol) and PPTS (4.4 mg, 0.02 mmol) were added. The atmosphere was purged with nitrogen for three times, and the reaction mixture was heated to 110°C, and the reaction proceeded overnight. LC-MS indicated complete consumption of the starting material. After the system was cooled to room temperature, the system was filtered directly. The filter cake was washed with ethanol and dried to obtain a brown solid 16-7 (41 mg, 25%). MS (ESI): m/z found $[M+H]^+$ = 558.45.

Synthesis of Intermediate 16-8:

At room temperature, the compound 16-7 (41 mg, 0.07 mmol) was dissolved in dry pyridine (3 mL). TESOTf (194.6 mg, 0.7 mmol) was added. The reaction proceeded overnight. TLC indicated completion of the reaction. The reaction mixture was dried, diluted with DCM, and washed with water for three times and saturated NaCl solution once. The reaction mixture was dried over anhydrous $Na_2SO_4$, concentrated, and purified by column chromatography (petroleum ether:ethyl acetate = 65:35) to obtain a black solid 16-8 (50 mg, 99%). MS (ESI): m/z found $[M+H]^+$ = 672.0.

Synthesis of Intermediate 16-9:

At room temperature, the compound 16-8 (50 mg, 0.074 mmol) was dissolved in toluene (3 mL). Lawesson's reagent (75.25 mg, 0.186 mmol) was added. The reaction mixture was heated to 110°C, and the reaction proceeded for 4 hours. TLC indicated completion of the reaction. The reaction mixture was concentrated dissolved in a small amount of dichloromethane, and purified by column chromatography (petroleum ether:ethyl acetate = 75:25) to obtain a yellow oily compound 16-9 (30 mg, 58.59%). MS (ESI): m/z found $[M+H]^+$ = 688.0.

Synthesis of Intermediate 16-10:

At room temperature, the compound 16-9 (30 mg, 0.0445 mmol) was dissolved in dry DCM (5 mL). Trifluoroacetic acid (TFA, 1 mL) was added. The reaction proceeded at room temperature overnight. LC-MS indicated completion of the reaction. The system was spin-dried to obtain a yellow oily compound 16-10 (14 mg, 54.82%), which was used directly in the next step.

Synthesis of Compound 16:

At room temperature, the compound 16-10 (7 mg, 0.012 mmol) was dissolved in a solution of HBr in AcOH (2 mL). The reaction proceeded at room temperature overnight. LC-MS indicated complete consumption of the starting material.

The reaction mixture was spin-dried directly and purified by reverse-phase column chromatography ($H_2O$:$CH_3CN$ = 65:35) to obtain a compound 16 (0.38 mg, yield 7.09%). $^1H$ NMR (600 MHz, DMSO-$d_6$) δ 8.64 (d, *J*= 5.6 Hz, 3H), 8.49 (d, J= 7.8 Hz, 1H), 8.06 (d, *J*= 10.3 Hz, 1H), 7.85 (s, 1H), 5.93 (t, *J*= 17.2 Hz, 2H), 5.78 (dd, *J* = 19.5, 3.5 Hz, 1H), 5.56 (dd, *J* = 16.5, 4.0 Hz, 1H), 5.50 (s, 1H), 2.58 (s, 3H), 1.92 (ttd, *J*= 14.1, 7.1, 2.9 Hz, 2H), 1.83 (t, *J*= 6.2 Hz, 3H), 0.87 (t, *J*= 7.3 Hz, 3H).

Example 17:

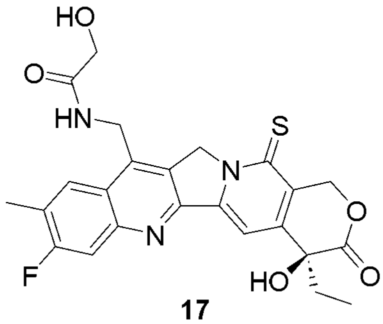

**17**

Synthetic route:

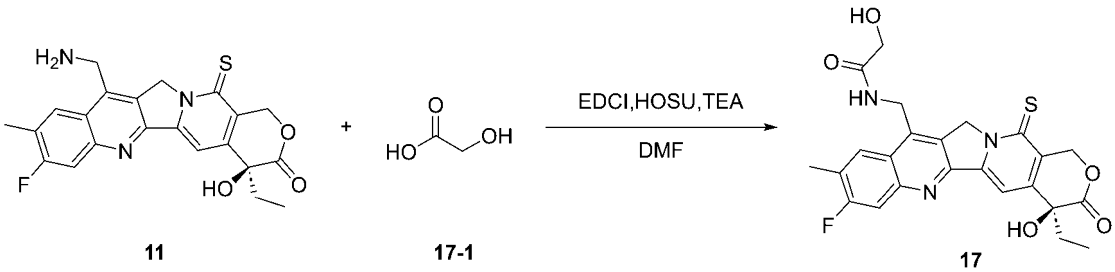


**11** **17-1** **17**

Synthesis of Compound 17:

At room temperature, glycolic acid (1.4 mg, 0.0176 mmol) was dissolved in dry DMF (3 mL). HOSU (2.1 mg, 0.0176 mmol) and EDCI (3.4 mg, 0.0176 mmol) were added, and the reaction proceeded for 1 hour. The compound 11 (5.0 mg, 0.0118 mmol) and triethylamine (1.4 mg, 0.0130 mmol) were then added, and the reaction proceeded at room temperature overnight. LC-MS monitoring indicated completion of the reaction. The reaction mixture was purified by pre-HPLC to obtain a compound 17 (3.49 mg, yield 61% ). MS (ESI): m/z found $[M+H]^+$ = 484.0.

Example 18:

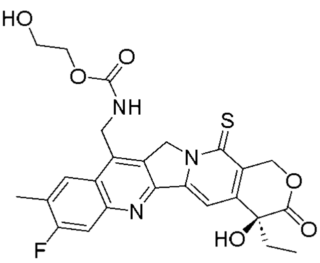

**18**

Synthetic route:

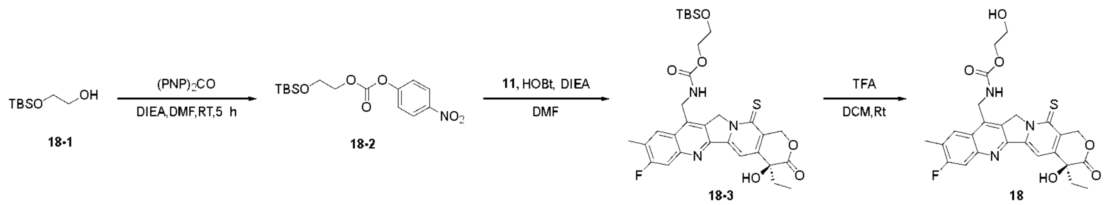


Synthesis of Intermediate 18-2:

tert-Butyl(2-hydroxyethoxy)dimethylsilane (18-1, 500 mg, 2.84 mmol) and bis(4-nitrophenyl) carbonate (1.7 g, 5.67 mmol) were dissolved in DMF (5 mL). DIEA (704 μL, 4.25 mmol) was added, and the reaction proceeded at room temperature for 5 hours. After TLC monitoring indicated completion of the reaction, the reaction mixture was purified by normal-phase column chromatography (PE:EA = 10:1) to obtain a pale yellow oily compound 18-2 (640 mg, 66.1%). MS (ESI): m/z found $[M+H]^+$ = 342.0.

Synthesis of Intermediate 18-3:

At room temperature, the compound 11 (5 mg, 0.0118 mmol) was dissolved in dry DMF (3 mL). The compound 18-2 (4.1 mg, 0.0118 mmol), 1-hydroxybenzotriazole (HOBt, 1.8 mg, 0.01298 mmol), and N,N-diisopropylethylamine (DIPEA, 6.2 μL, 0.0354 mmol) were added. The reaction proceeded at room temperature for 4 hours. LC-MS monitoring indicated complete consumption of the starting material. The reaction mixture was purified by prep-HPLC to obtain a compound 18-3 (4 mg, 54%). MS (ESI): m/z found $[M+H]^+$ = 628.2.

Synthesis of Compound 18:

At room temperature, the compound 18-3 (4 mg, 0.0064 mmol) was dissolved in dry DCM (5 mL). Trifluoroacetic acid (TFA, 1 mL) was added, and the reaction proceeded at room temperature for 1 hour. LC-MS monitoring indicated complete consumption of the starting material. The reaction mixture was dried under reduced pressure. The residue was dissolved in a small amount of DMF and purified by pre-HPLC to obtain a compound 18 (1.36 mg, 41%). MS (ESI): m/z found $[M+H]^+$= 514.35.

Example 19:

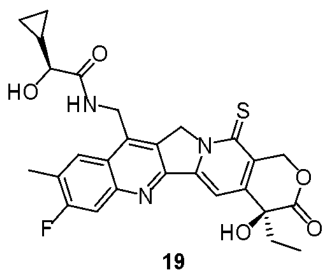

19

Synthetic route:

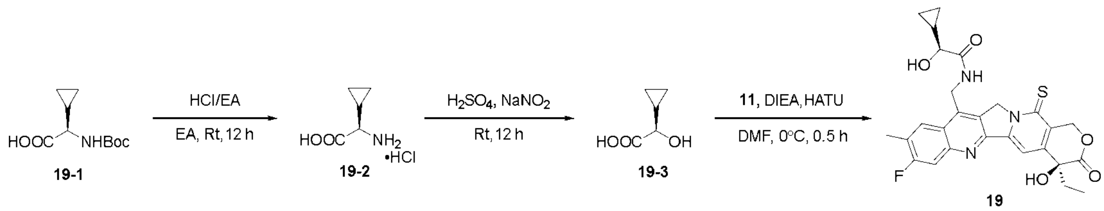


19-1 19-2 19-3 19

Synthesis of Intermediate 19-2:

Boc-L-cyclopropylglycine (19-1, 500 mg, 2.32 mmol) was dissolved in ethyl acetate (EA, 4 mL). A solution of HCl/EA (3 M, 7.4 mL) was added, and the reaction proceeded at room temperature overnight. There is a large amount of white solid precipitated in the reaction mixture. The reaction mixture was filtered under suction, and the filter cake was dried to obtain a solid crude product 19-2 (320 mg). MS (ESI): m/z found $[M+H]^+$ = 116.2.

Synthesis of Intermediate 19-3:

The compound 19-2 was dissolved in dilute sulfuric acid (2 N, 4.4 mL). The solution was cooled in an ice bath, and a solution of sodium nitrite (4.4 mol/L, 5 mL) was added dropwise. The reaction mixture was then warmed to room temperature, and the reaction proceeded overnight. Sodium chloride was added to make the solution saturated. The filtrate was extracted with water/EA. The organic phase was dried over anhydrous sodium sulfate and spin-dried to obtain a crude product (80 mg).

Synthesis of Compound 19:

The compound 11 (15 mg, 0.036 mmol) was dissolved in DMF (2 mL) and cooled to 0°C in an ice bath. The intermediate 19-3 (9 mg, 0.072 mmol), DIEA (6 μL, 0.034 mmol), and HATU (5.3 mg, 0.014 mmol) were added. The reaction proceeded in the ice bath for 0.5 hours. After LC-MS monitoring indicated completion of the reaction, the reaction mixture was distilled under reduced pressure and concentrated. Purification by preparative HPLC and lyophilization were perfomed to obtain a yellow solid product 19 (0.82 mg, 8.9%). MS (ESI): m/z found $[M+H]^+$ = 524.40.

Example 20:

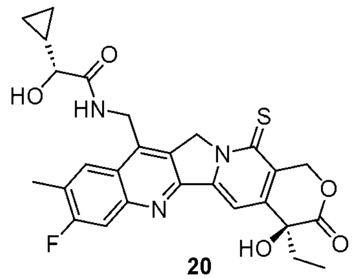

20

Synthetic route:

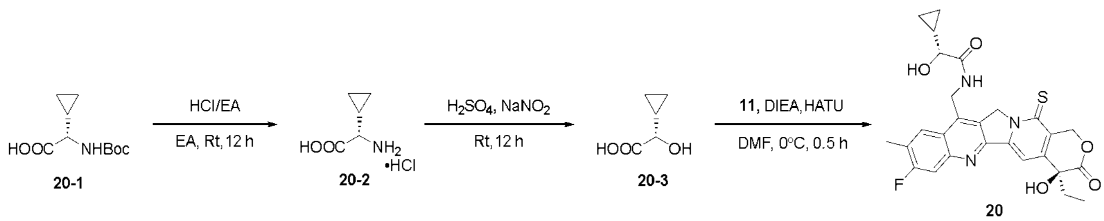


20-1 20-2 20-3 20

Synthesis of Intermediate 20-2:

Boc-D-cyclopropylglycine (20-1, 500 mg, 2.32 mmol) was dissolved in ethyl acetate (EA, 4 mL). A solution of HCl/EA (3 M, 7.4 mL) was added, and the reaction proceeded at room temperature overnight. There is a large amount of white solid precipitated in the reaction mixture. The reaction mixture was filtered under suction, and the filter cake was dried to obtain a solid crude product 20-2 (300 mg). MS (ESI): m/z found $[M+H]^+$ = 116.2.

Synthesis of Intermediate 20-3:

The compound 20-2 was dissolved in dilute sulfuric acid (2 N, 4.4 mL). The reaction mixture was cooled in an ice bath, and a solution of sodium nitrite (4.4 mol/L, 5 mL) was added dropwise. The reaction mixture was then warmed to room temperature, and the reaction proceeded overnight. Sodium chloride was added to make the solution saturated. The filtrate was extracted with water/EA, and dried over by anhydrous sodium sulfate and spin-dried to obtain a crude product (90 mg).

Synthesis of Compound 20:

The 11 (20 mg, 0.047 mmol) was dissolved in DMF (3 mL) and cooled to 0°C in an ice bath. The intermediate 20-3 (9 mg, 0.072 mmol), DIEA (6 μL, 0.034 mmol), and HATU (5.3 mg, 0.014 mmol) were added. The reaction proceeded in the ice bath for 0.5 hours. After LC-MS monitoring indicated completion of the reaction, the reaction mixture was distilled under reduced pressure and concentrated. Purification by preparative HPLC and lyophilization were perfomed to obtain a yellow solid product (1.44 mg, 15.6%). MS(ESI):m/z found $[M+H]^+$=524.00. $^1H$ NMR (600 MHz, DMSO-$d_6$) δ 8.29 (d, *J* = 7.1 Hz, 1H), 7.97 (d, *J*= 10.6 Hz, 1H), 7.81 (s, 1H), 6.71 (d, *J* = 2.0 Hz, 1H), 6.23 (dd, *J* = 8.6, 6.4 Hz, 1H), 5.92 (dd, *J* = 16.5, 3.0 Hz, 1H), 5.53 (d, *J* = 16.4 Hz, 3H), 5.45 (d, *J* = 19.1 Hz, 1H), 5.03 (dd, *J*= 16.6, 13.4 Hz, 1H), 4.93 (tt, *J* = 7.0, 3.9 Hz, 1H), 2.54 (d, *J* = 5.6 Hz, 3H), 2.05 - 1.95 (m, 2H), 1.89 (dd, *J* = 9.1, 6.0 Hz, 2H), 1.72 (d, *J* = 12.6 Hz, 1H), 0.86 (t, *J* = 7.3 Hz, 5H).

Example 21:

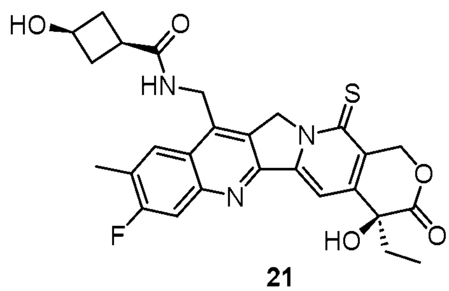

**21**

Synthetic route:

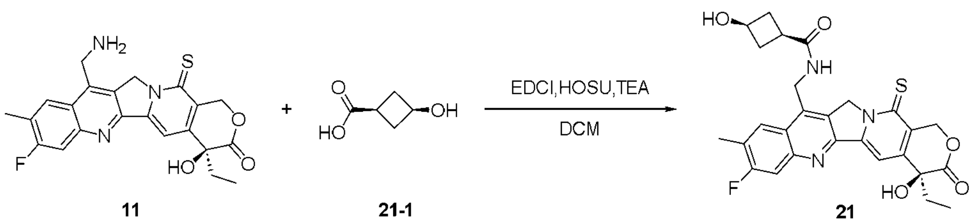


11 21-1 21

Synthesis of Compound 21:

In an ice bath, cis-3-hydroxycyclobutanecarboxylic acid (4.09 mg, 0.0353 mmol) was dissolved in dry DCM (5 mL). N-Hydroxysuccinimide (NHS, 4.06 mg, 0.0353 mmol) and 1-(3-dimethylaminopropyl)-3-ethylcarbodiimide hydrochloride (EDC·HCl, 6.77 mg, 0.0353 mmol) were added. The reaction proceeded for 1 hour. The compound 11 (10.00 mg,

0.0235 mmol) and triethylamine (TEA, 3.6 μL, 0.0259 mmol) were then added, and the reaction proceeded at room temperature for 2 hours. LCMS monitoring indicated completion of the reaction. The reaction mixture was purified by prep-HPLC to obtain a compound 21 (3.54 mg, 30% ).MS (ESI): m/z found[M+H]+=524.3 $^1$H NMR (400 MHz, DMSO-$d_6$) δ 8.68 (t, *J* = 5.8 Hz, 1H), 8.37 (d, *J* = 8.2 Hz, 1H), 7.96 (d, *J* = 10.7 Hz, 1H), 7.80 (s, 1H), 6.71 (s, 1H), 5.93 (d, *J*= 16.6 Hz, 1H), 5.67 (s, 2H), 5.53 (d, *J* = 16.7 Hz, 1H), 5.09 (d, *J* = 7.0 Hz, 1H), 4.89 (d, *J* = 5.8 Hz, 2H), 3.92 (q, *J* = 7.6 Hz, 1H), 2.53 (s, 3H), 2.03 - 1.97 (m, 7H), 0.85 (s, 3H).

Example **22:**

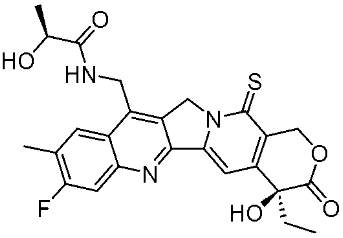

**22**

Synthetic route:

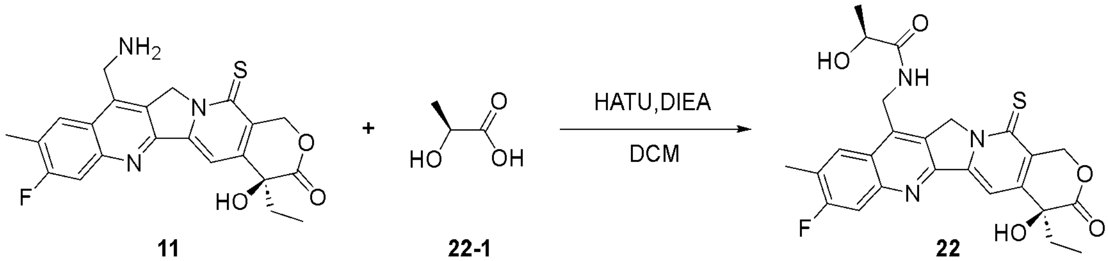


**11** **22-1** **22**

Synthesis of Compound 22:

In an ice bath, the compound 11 (15 mg, 0.035 mmol) was dissolved in dry DCM (5 mL). (S)-2-Hydroxypropanoic acid (6.4 mg, 0.071 mmol), O-(7-azabenzotriazol-1-yl)-N,N,N',N'-tetramethyluronium hexafluorophosphate (16 mg, 0.042 mmol), and N,N-diisopropylethylamine (18 μL, 0.106 mmol) were added. The reaction proceeded at room temperature for 2 hours. LCMS monitoring indicated completion of the reaction. The reaction mixture was purified by pre-HPLC to obtain a compound 22 (2.37 mg, 14% yield).MS (ESI): *m/z* found [M+H]+= 498.35. $^1$H NMR (600 MHz, DMSO-$d_6$) δ 8.78 (t, *J* = 6.1 Hz, 1H), 8.48 (d, *J* = 8.2 Hz, 1H), 7.96 (d, *J* = 10.7 Hz, 1H), 7.81 (s, 1H), 6.71 (s, 1H), 5.94 (d, *J* = 16.5 Hz, 1H), 5.77 (s, 2H), 5.63 (d, *J* = 4.8 Hz, 1H), 5.54 (d, *J* = *16.5* Hz, 1H), 4.88 (qd, *J* = 15.1, 6.1 Hz, 2H), 4.06 - 3.98 (m, 1H), 2.52 (s, 3H), 2.00 (dt, *J* = 18.4, 7.0 Hz, 3H), 1.95 - 1.85 (m, 2H), 0.86 (s, 3H).

Example **23:**

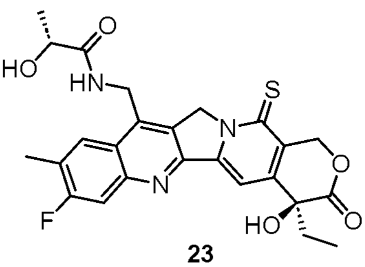

**23**

Synthetic route:

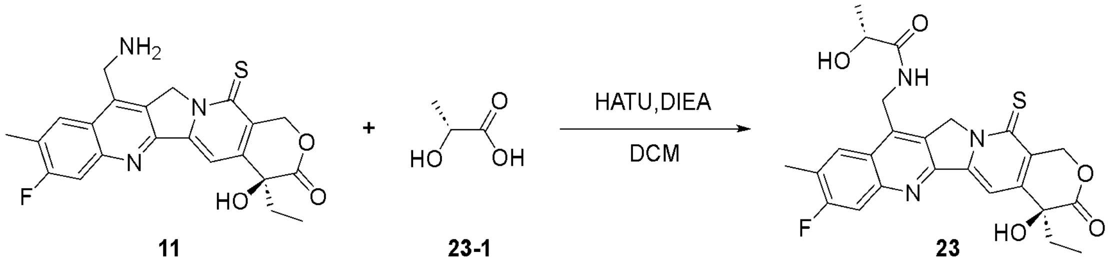


11 23-1 23

Synthesis of Compound 23:

In an ice bath, the compound 11 (15 mg, 0.035 mmol) was dissolved in anhydrous dichloromethane (5 mL). D-2-Hydroxypropanoic acid (6.4 mg, 0.071 mmol), O-(7-azabenzotriazol-1-yl)- N,N,N',N'-tetramethyluronium hexafluorophosphate (16 mg, 0.042 mmol), and N,N-diisopropylethylamine (18 μL, 0.106 mmol) were added. The reaction proceeded at room temperature for 2 hours. LCMS monitoring indicated completion of the reaction. The reaction mixture was purified by prep-HPLC to obtain a compound 23 (4.94 mg, yield 28% ).MS (ESI): *m/z* found $[M+H]^+$= 498.35. $^1H$ NMR (600 MHz, DMSO-$d_6$) δ 8.78 (t, *J* = 6.1 Hz, 1H), 8.48 (d, *J* = 8.1 Hz, 1H), 7.95 (s, 1H), 7.81 (s, 1H), 6.71 (s, 2H), 5.94 (d, *J* = 16.5 Hz, 1H), 5.77 (s, 2H), 5.63 (d, *J* = 4.8 Hz, 1H), 5.54 (d, *J*= 16.5 Hz, 1H), 4.96 - 4.77 (m, 2H), 4.09 - 3.96 (m, 1H), 2.52 (s, 3H), 2.00 (dt, *J* = 18.6, 7.1 Hz, 3H), 1.89 (dq, *J*= 14.3, 7.1 Hz, 2H), 0.86 (d, *J* = 4.5 Hz, 3H).

Example **24:**

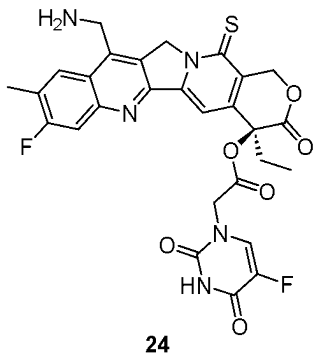

**24**

Synthetic route:

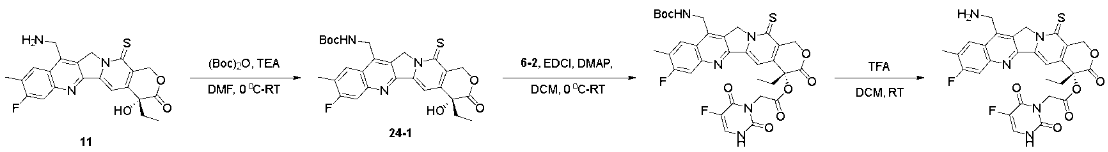


11 24-1 24-2 24

Synthesis of Intermediate 24-1:

In an ice bath, the compound 11 (100 mg, 0.235 mmol) was dissolved in anhydrous N,N-dimethylformamide (10 mL). Di-tert-butyl dicarbonate (56 mg, 0.259 mmol) and triethylamine (33 μL, 0.235 mmol) were added. The reaction proceeded at room temperature for 8 hours. LCMS monitoring indicated completion of the reaction. The reaction mixture was diluted with dichloromethane and washed with water for three times. The organic phase was collected, washed with

saturated brine, dried over anhydrous sodium sulfate, and concentrated to obtain a compound 24-1 (140 mg, 99%). MS (ESI): m/z found $[M+H]^+$ = 426.45.

Synthesis of Intermediate 24-2:

In an ice bath, the compound 24-1 (70 mg, 0.133 mmol) was dissolved in anhydrous dichloromethane (10 mL). The compound 6-2 (49 mg, 0.260 mmol), 4-dimethylaminopyridine (9.8 mg, 0.0798 mmol), and 1-(3-dimethylaminopropyl)-3-ethylcarbodiimide hydrochloride (122 mg, 0.638 mmol) were added. The reaction proceeded at room temperature for 1 hour. LCMS monitoring indicated completion of the reaction. Dimethyl sulfoxide was added to the reaction mixture to achieve a clear solution, followed by the addition of twice the volume of water, resulting in the precipitation of a solid. The filter cake was obtained by filtration and dried to obtain a compound 24-2 (30 mg, 32%). MS (ESI): m/z found $[M+H]^+$ = 696.40.

Synthesis of Compound 24:

At room temperature, the compound 24-2 (30 mg, 0.043 mmol) was dissolved in anhydrous dichloromethane (5 mL). Trifluoroacetic acid (2 mL) was added, and the reaction proceeded at room temperature for 2 hours. LCMS monitoring indicated completion of the reaction. The reaction mixture was concentrated under reduced pressure. The residue was dissolved in a small amount of methanol and purified by pre-HPLC to obtain a compound 24 (16.75 mg, yield 65% ). MS (ESI): *m/z* found $[M+H]^+$= 596.36. $^1$H NMR (600 MHz, DMSO-$d_6$) δ 11.97 (s, 1H), 8.45 (d, *J* = 8.0 Hz, 1H), 8.36 (s, 2H), 8.18 (d, *J* = 6.6 Hz, 1H), 8.00 (d, *J* = 10.5 Hz, 1H), 7.73 (s, 1H), 5.90 (d, *J* = 17.0 Hz, 1H), 5.83 (d, *J* = 9.4 Hz, 2H), 5.77 (s, 1H), 5.65 (d, *J* = 17.0 Hz, 1H), 4.86 (*d, J* = 17.6 Hz, 1H), 4.78 (s, 2H), 3.17 (s, 3H), 2.22 (q, *J* = 7.3 Hz, 2H), 0.91 (t, *J* = 7.4 Hz, 3H).

Example 25:

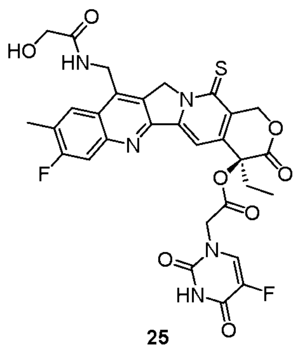

**25**

Synthesis of Compound 25:

In an ice bath, the compound 24 (5 mg, 0.0084 mmol) was dissolved in anhydrous dichloromethane (5 mL). Glycolic acid (1.3 mg, 0.0168 mmol), O-(7-azabenzotriazol-1-yl)- N,N,N',N'-tetramethyluronium hexafluorophosphate (3.8 mg, 0.0101 mmol), and N,N-diisopropylethylamine (3.3 μL, 0.0252 mmol) were added. The reaction proceeded at room temperature for 2 hours. LCMS monitoring indicated completion of the reaction. The reaction mixture was concentrated under reduced pressure. The residue was dissolved in a small amount of N,N-dimethylformamide and purified by pre-HPLC to obtain a compound 25 (0.57 mg, yield 11% ). MS (ESI): m/z found $[M+H]^+$ = 654.35.

Example 26:

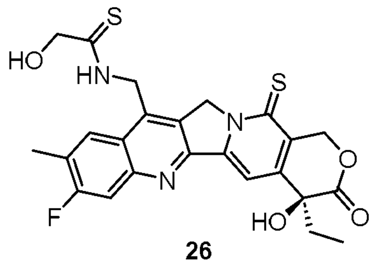

26

Synthetic route:

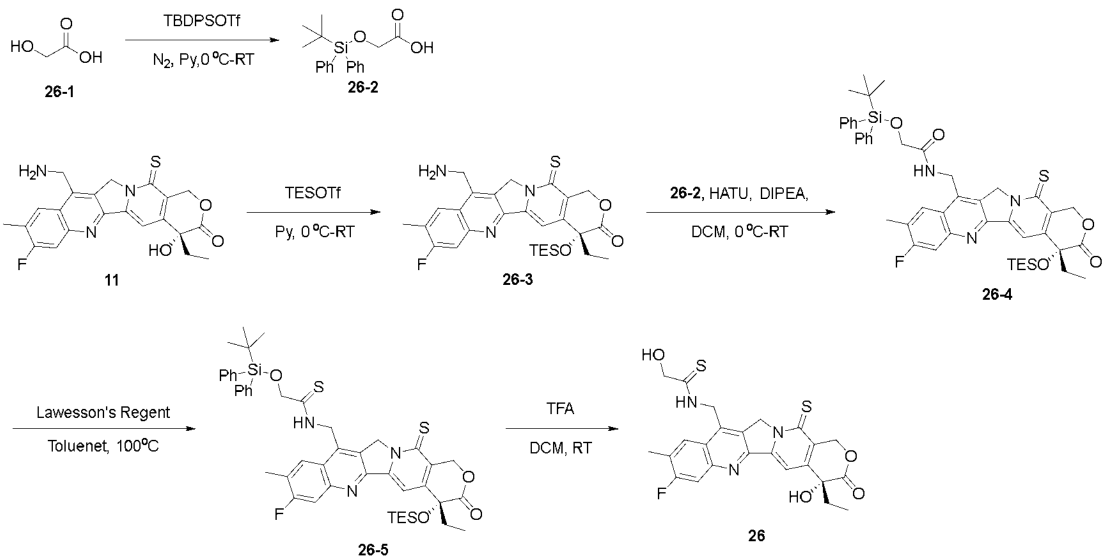


Synthesis of Intermediate 26-2:

In an ice bath, glycolic acid (50 mg, 0.657 mmol) was dissolved in anhydrous pyridine (5 mL). tert-Butyldiphenylsilyl trifluoromethanesulfonate (409 μL, 1.315 mmol) was added. The reaction proceeded at room temperature for 8 hours. LCMS monitoring indicated completion of the reaction. The reaction mixture was concentrated under reduced pressure. The residue was dissolved in a small amount of methanol and purified by reverse-phase column chromatography to obtain a compound 26-2 (100 mg, 48% yield). MS (ESI): m/z found $[M-H]^-$ = 313.10.

Synthesis of Intermediate 26-3:

In an ice bath, the compound 11 (100 mg, 0.235 mmol) was dissolved in anhydrous pyridine (5 mL). Triethylsilyl trifluoromethanesulfonate (504 μL, 2.35 mmol) was added. The reaction proceeded at room temperature for 8 hours. LCMS monitoring indicated completion of the reaction. The reaction mixture was concentrated under reduced pressure. The residue was dissolved in a small amount of N,N-dimethylformamide and purified by reverse-phase column chromatography to obtain a compound 26-3 (55 mg, yield 43% ). MS (ESI): m/z found $[M+H]^+$ = 540.40.

Synthesis of Intermediate 26-4:

In an ice bath, the compound 26-3 (55 mg, 0.102 mmol) was dissolved in anhydrous dichloromethane (5 mL). The compound 26-2 (64 mg, 0.204 mmol), O-(7-azabenzotriazol-1-yl)- N,N,N',N'-tetramethyluronium hexafluorophosphate (47 mg, 0.122 mmol), and N,N-diisopropylethylamine (54 μL, 0.306 mmol) were added. The reaction proceeded at room temperature for 1 hour. LCMS monitoring indicated completion of the reaction. The reaction mixture was concentrated under reduced pressure and purified by normal-phase column chromatography to obtain a compound 26-4 (60 mg, 70% yield ). MS (ESI): m/z found $[M+H]^+$ = 837.45.

Synthesis of Intermediate 26-5:

At room temperature, the compound 26-4 (76 mg, 0.0909 mmol) was dissolved in toluene (5 mL). Lawesson's reagent (110 mg, 0.273 mmol) was added. The atmosphere was purged with nitrogen for three times, and the reaction mixture was heated to 100°C, and the reaction proceeded for 4 hours. TLC monitoring indicated completion of the reaction. The reaction mixture was concentrated under reduced pressure and purified by normal-phase column chromatography to obtain a compound 26-5 (43 mg, yield 55% ).

Synthesis of Compound 26:

At room temperature, the compound 26-5 (43 mg, 0.0505 mmol) was dissolved in anhydrous dichloromethane (5 mL). Trifluoroacetic acid (2 mL) was added, and the reaction proceeded at room temperature for 36 hours. LCMS monitoring indicated completion of the reaction. The reaction mixture was concentrated under reduced pressure. The residue was dissolved in a small amount of N,N-dimethylformamide and purified by prep-HPLC to obtain a compound 26 (16.75 mg, yield 65% ).MS (ESI): *m/z* found $[M+H]^+$= 500.35. $^1$H NMR (600 MHz, DMSO-$d_6$) δ 10.64 (t, *J* = 5.9 Hz, 1H), 8.46 (d, *J*= 8.1 Hz, 1H), 7.98 (d, *J* = 10.6 Hz, 1H), 7.81 (s, 1H), 6.71 (s, 1H), 6.10 (s, 1H), 5.93 (d, *J* = 16.5 Hz, 1H), 5.70 (s, 2H), 5.53 (d, *J* = 16.5 Hz, 1H), 5.48 (d, *J* = 5.9 Hz, 2H), 4.36 (s, 2H), 2.52 (s, 3H), 1.95 - 1.83 (m, 2H), 0.86 (t, *J*= 7.3 Hz, 3H).

Example 27:

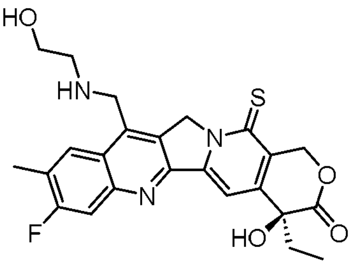

**27**

Synthetic route:

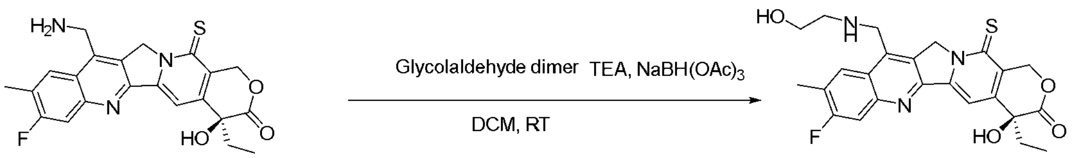


Synthesis of Compound 27:

The compound 11 (15 mg, 0.22mmol) was dissolved in DCM (2 mL). Under a nitrogen atmosphere at 0°C, glycolaldehyde dimer (7.62 mg, 0.40 mmol) and TEA (10.69 mg, 0.65 mmol) were added, and the reaction mixture was stirred for 0.5 hours. Sodium triacetoxyborohydride (14.96 mg, 0.44 mmol) was then added. The reaction mixture was slowly warmed to room temperature, and the reaction proceeded for 12 hours. LC-MS monitoring indicated completion of the reaction. The reaction mixture was extracted with DCM/saturated sodium bicarbonate aqueous solution. The organic phase was dried over anhydrous $Na_2SO_4$, filtered under suction, and spin-dried. Purification by prep-HPLC and lyophilization were perfomed to obtain a yellow solid compound 27 (1.2 mg, 7.25% ).LC-MS(ESI):m/z found $[M+H]^+$=470.35. $^1$H NMR (400 MHz, DMSO-$d_6$) δ 8.39 (d, *J* = 8.3 Hz, 1H), 8.19 (s, 1H), 7.93 (d, *J* = 10.8 Hz, 1H), 7.80 (s, 1H), 6.71 (s, 1H), 5.93 (d, *J* = 16.6 Hz, 1H), 5.66 (s, 2H), 5.53 (d, *J* = 16.6 Hz, 1H), 4.41 (s, 2H), 3.53 (t, *J* = 5.8 Hz, 2H), 2.75 - 2.63 (m, 3H), 2.53 (s, 3H), 1.89 (dt, *J* = 10.1, 6.6 Hz, 3H), 0.86 (t, *J* = 7.3 Hz, 3H).

Example 28:

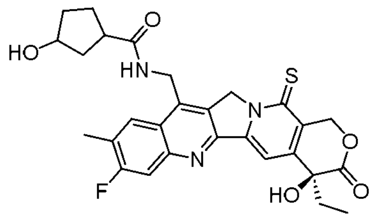

**28**

Synthesis of Compound 28:

In an ice bath, the compound 11 (15 mg, 0.035 mmol) was dissolved in anhydrous N,N-dimethylformamide (5 mL). 3-Hydroxycyclopentanecarboxylic acid (9.2 mg, 0.071 mmol), O-(7-azabenzotriazol-1-yl)-N,N,N',N'-tetramethyluronium hexafluorophosphate (16 mg, 0.042 mmol), and N,N-diisopropylethylamine (18 μL, 0.106 mmol) were added. The reaction proceeded at room temperature for 2 hours. LCMS monitoring indicated completion of the reaction. The reaction mixture was purified by prep-HPLC to obtain a compound 28 (3.03 mg, yield 16% ).MS (ESI): *m/z* found $[M+H]^+$= 538.40. $^1H$ NMR (600 MHz, DMSO-$d_6$) δ 8.75 (t, *J* = 5.9 Hz, 1H), 8.37 (d, *J* = 8.2 Hz, 1H), 7.96 (d, *J* = 10.7 Hz, 1H), 7.80 (s, 1H), 6.72 (s, 1H), 5.93 (d, *J* = 16.5 Hz, 1H), 5.68 (d, *J* = 3.7 Hz, 2H), 5.53 (d, *J* = 16.5 Hz, 1H), 5.02 (s, 1H), 4.89 (td, *J* = 5.5, 2.8 Hz, 2H), 4.48 (s, 1H), 2.87 (p, *J* = 8.4 Hz, 1H), 2.53 (s, 3H), 2.03 - 1.96 (m, 2H), 1.94 - 1.88 (m, 2H), 1.70 (ddd, *J* = 8.6, 6.9, 4.4 Hz, 2H), 1.46 (dq, *J* = 14.8, 7.6, 7.0 Hz, 2H), 0.88 - 0.84 (m, 3H).

Example **29:**

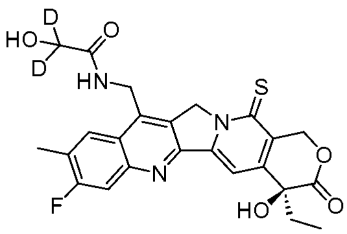

**29**

Synthetic route:

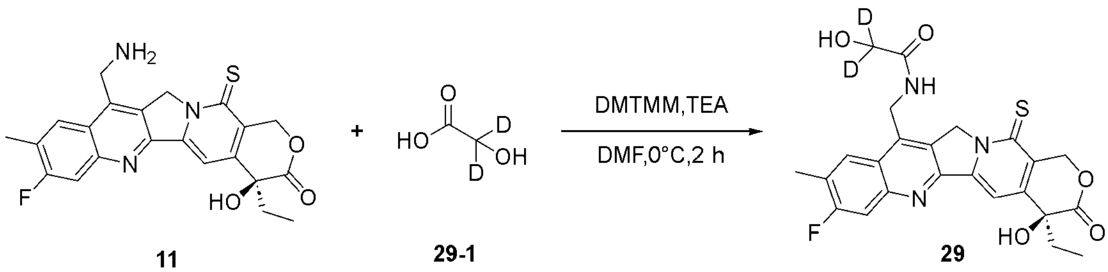


**11** **29-1** **29**

Synthesis of Compound 29:

The compound 11 (10 mg, 0.023 mmol) was dissolved in DMF (1 mL), and the reaction mixture was cooled to 0°C in an ice bath. Compound 29-1 (4 mg, 0.047 mmol), triethylamine (8 μL, 0.059 mmol), and DMMTM (13 mg, 0.047 mmol) were added. The reaction proceeded in the ice bath for 2 hours. After LC-MS monitoring indicated completion of the

reaction, the reaction mixture was purified by preparative HPLC and lyophilized to obtain a yellow solid compound 29 (5.40 mg, 49.1%).MS(ESI):m/z found $[M+H]^+$=486.30. $^1$H NMR (600 MHz, DMSO-$d_6$) δ 8.86 (*t, J* = 6.1 Hz, 1H), 8.52 (d, *J* = 8.2 Hz, 1H), 7.96 (d, *J* = 10.7 Hz, 1H), 7.81 (s, 1H), 6.72 (s, 1H), 5.94 (d, *J* = 16.5 Hz, 1H), 5.79 (s, 2H), 5.54 (d, *J* = 16.5 Hz, 1H), 4.89 (d, *J* = 6.2 Hz, 2H), 3.93 (s, 1H), 2.53 (s, 3H), 1.94 - 1.86 (m, 2H), 0.86 (t, *J* = 7.3 Hz, 3H).

Example **30:**

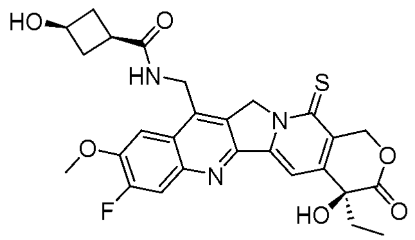

**30**

Synthesis of Compound 30:

The compound 12 (5 mg, 0.011 mmol) was dissolved in dichloromethane (2 mL), and the reaction mixture was cooled to 0°C in an ice bath. cis-3-Hydroxycyclobutanecarboxylic acid (3 mg, 0.023 mmol), DIEA (6 μL, 0.034 mmol), and HATU (5.3 mg, 0.014 mmol) were added. The reaction proceeded in the ice bath for 0.5 hours. After LC-MS monitoring indicated completion of the reaction, the reaction mixture was distilled under reduced pressure and concentrated. Purification by preparative HPLC and lyophilization were perfomed to obtain a yellow solid product 30 (2.4 mg, 40.7% ).LC-MS(ESI):m/z found $[M+H]^+$=540.40. $^1$H NMR (400 MHz, DMSO-$d_6$) δ 8.65 (t, *J* = 6.0 Hz, 1H), 8.04 (d, *J* = 12.1 Hz, 1H), 7.91 (d, *J* = 9.2 Hz, 1H), 7.77 (s, 1H), 6.69 (d, *J* = 11.1 Hz, 2H), 5.93 (d, *J* = 16.6 Hz, 1H), 5.70 (s, 2H), 5.52 (d, *J* = 16.6 Hz, 1H), 5.32 (t, *J* = 4.8 Hz, 1H), 4.96 - 4.86 (m, 2H), 4.09 (s, 3H), 3.91 (q, *J* = 7.7 Hz, 1H), 2.03 - 1.92 (m, 6H), 0.87 (d, *J* = 7.3 Hz, 3H).

Example **31:**

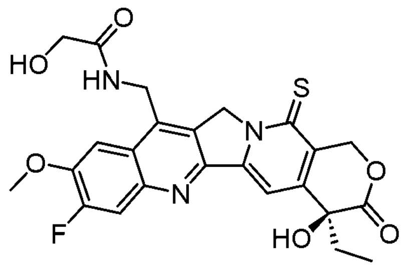

**31**

Synthesis of Compound 31:

The compound 12 (5 mg, 0.011 mmol) was dissolved in dichloromethane (2 mL), and the reaction mixture was cooled to 0°C in an ice bath. Glycolic acid (2 mg, 0.023 mmol), DIEA (6 μL, 0.034 mmol), and HATU (5.3 mg, 0.014 mmol) were added to the reaction mixture. The reaction proceeded at 0°C for 0.5 hours. After completion confirmed by LC-MS monitoring, the reaction mixture was distilled under reduced pressure and concentrated. Purified by preparative HPLC and lyophilized were performed to obtain a yellow solid product 31 (0.77 mg, 14.3%). LC-MS(ESI):m/z found $[M+H]^+$=500.35. $^1$H NMR (400 MHz, DMSO-$d_6$) δ 8.84 (t, *J* = 6.2 Hz, 1H), 8.02 (d, *J* = 12.1 Hz, 1H), 7.97 (d, *J*= 9.2 Hz, 1H), 7.78 (s, 1H), 6.74 (s, 1H), 6.68 (s, 1H), 5.93 (d, *J* = 16.6 Hz, 1H), 5.79 (s, 2H), 5.50 (d, *J* = 16.6 Hz, 1H), 5.31 (t, *J* = 4.9 Hz, 2H), 4.95 - 4.88 (m, 2H), 4.05 (s, 3H), 2.00 (d, *J* = 7.5 Hz, 2H), 0.84 (d, *J* = 4.7 Hz, 3H).

Example **32:**

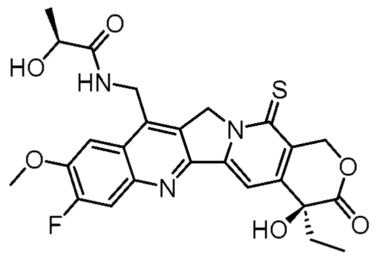

**32**

Synthesis of Compound 32:

The compound 12 (5 mg, 0.011 mmol) was dissolved in dichloromethane (2 mL), and the reaction mixture was cooled to 0°C in an ice bath. L-Lactic acid (2.1 mg, 0.023 mmol), DIEA (6 μL, 0.034 mmol), and HATU (5.3 mg, 0.014 mmol) were added. The reaction proceeded in the ice bath for 0.5 hours. After LC-MS monitoring indicated completion of the reaction, the reaction mixture was distilled under reduced pressure and concentrated. Purification by preparative HPLC and lyophilization were perfomed to obtain a yellow solid product 32 (0.61 mg, 10.9% ).LC-MS(ESI):m/z found $[M+H]^+$=514.35. $^1$H NMR (400 MHz, DMSO-$d_6$) δ 8.80 (t, *J* = 6.1 Hz, 1H), 8.04 (d, *J* = 12.1 Hz, 1H), 7.98 (d, *J* = 9.2 Hz, 1H), 7.78 (s, 1H), 6.68 (s, 2H), 5.93 (d, *J* = 16.6 Hz, 1H), 5.80 (s, 2H), 5.53 (d, *J* = 16.5 Hz, 1H), 5.32 (t, *J* = 4.8 Hz, 2H), 4.96 - 4.85 (m, 2H), 4.06 (s, 3H), 2.00 (d, *J* = 7.8 Hz, 2H), 0.86 (dt, *J*= 7.6, 4.1 Hz, 6H).

Example **33:**

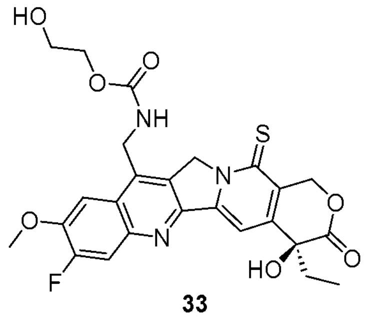

**33**

Synthetic route:

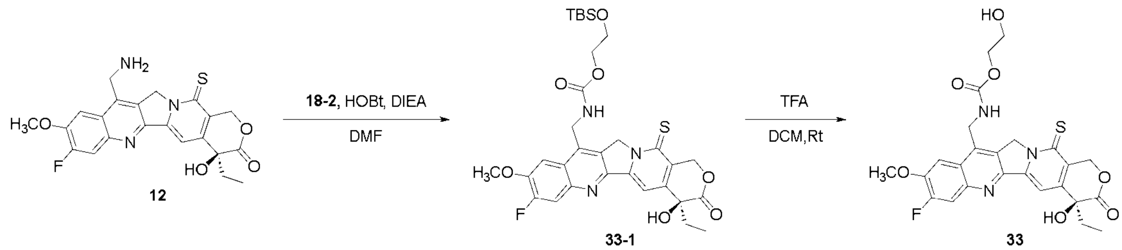


Synthesis of Intermediate 33-1:

The compound 12 (15 mg, 0.034 mmol) was dissolved in DMF (2 mL). The compound 18-2 (12 mg, 0.034 mmol), HOBt (6 mg, 0.044 mmol), and DIEA (6 μL, 0.068 mmol) were added. The reaction proceeded at room temperature for 4

hours. After LC-MS monitoring indicated completion of the reaction, the reaction mixture was purified by preparative HPLC and lyophilized to obtain a yellow solid product 33-1 (12 mg, 54.5% ). LC-MS (ESI): m/z found $[M+H]^+$ = 644.40.

Synthesis of Compound 33:

The compound 33-1 (12 mg, 0.019 mmol) was dissolved in dichloromethane (5 mL). Trifluoroacetic acid (TFA, 1 mL) was added, and the reaction proceeded at room temperature for 1.5 hours. After LC-MS monitoring indicated completion of the reaction, the reaction mixture was purified by preparative HPLC and lyophilized to obtain a yellow solid product 33 (4.76 mg, 47.6% ).LC-MS(ESI):m/z found $[M+H]^+$=530.35. $^1H$ NMR (600 MHz, DMSO-$d_6$) δ 8.22 (t, *J* = 6.1 Hz, 1H), 8.04 (d, *J* = 12.0 Hz, 1H), 7.87 (d, *J* = 9.1 Hz, 1H), 7.77 (s, 1H), 6.70 (s, 1H), 5.93 (d, *J* = 16.4 Hz, 1H), 5.74 (s, 2H), 5.52 (d, *J* = 16.4 Hz, 1H), 4.83 (d, *J* = 6.1 Hz, 2H), 4.72 (s, 1H), 4.11 (s, 3H), 3.99 (t, *J* = 5.1 Hz, 2H), 3.54 (t, *J* = 5.1 Hz, 2H), 1.94 - 1.84 (m, *J* = 7.2 Hz, 2H), 0.87 (t, *J*= 7.3 Hz, 3H).

Example **34:**

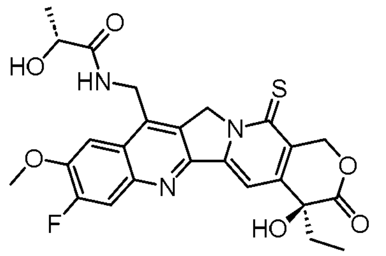

**34**

Synthesis of Compound 34:

The compound 12 (5 mg, 0.011 mmol) was dissolved in dichloromethane (2 mL), and the reaction mixture was cooled to 0°C in an ice bath. D-Lactic acid (2.1 mg, 0.023 mmol), DIEA (6 μL, 0.034 mmol), and HATU (5.3 mg, 0.014 mmol) were added. The reaction proceeded in the ice bath for 0.5 hours. After LC-MS monitoring indicated completion of the reaction, the reaction mixture was distilled under reduced pressure and concentrated. Purification by preparative HPLC and lyophilization were perfomed to obtain a yellow solid product 34 (1.00 mg, 17.9% ).LC-MS(ESI):m/z found $[M+H]^+$=514.35. $^1H$ NMR (600 MHz, DMSO-$d_6$) δ 8.80 (t, *J* = 6.2 Hz, 1H), 8.04 (d, *J* = 12.0 Hz, 1H), 7.99 (d, *J* = 9.2 Hz, 1H), 7.78 (s, 1H), 6.70 (s, 1H), 5.94 (d, *J* = 16.5 Hz, 1H), 5.86 - 5.74 (m, 2H), 5.61 (d, *J* = 4.9 Hz, 1H), 5.53 (d, *J*= 16.4 Hz, 1H), 4.90 (ddd, *J* = 50.7, 15.0, 6.2 Hz, 2H), 4.07 (s, 3H), 4.04 (td, *J* = 6.7, 4.4 Hz, 1H), 1.95 - 1.83 (m, *J*= 7.3 Hz, 2H), 1.23 (s, 3H), 0.87 (t, *J* = 7.3 Hz, 3H).

Example 35:

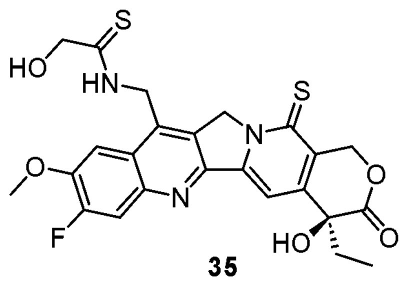

**35**

Synthetic route:

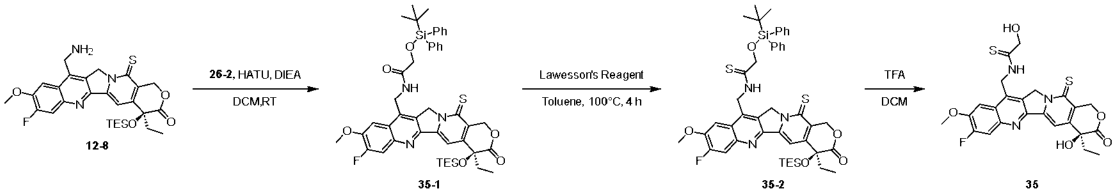


Synthesis of Intermediate 35-1:

The compound 12 (100 mg, 0.180 mmol) was dissolved in dichloromethane (3 mL), and the reaction mixture was cooled to 0°C in an ice bath. The compound 26-2 (113 mg, 0.360 mmol), DIEA (89 μL, 0.540 mmol), and HATU (82 mg, 0.216 mmol) were added. The reaction proceeded at room temperature for 2 hours. After LC-MS monitoring indicated completion of the reaction, the reaction mixture was distilled under reduced pressure and concentrated. Purification by TLC plate (DCM:EA = 1:1) was perfomed to obtain a yellow solid product 35-1 (90 mg, 58.8% ). LC-MS (ESI): m/z found $[M+H]^+$ = 852.60.

Synthesis of Intermediate 35-2:

Under nitrogen protection, the compound 35-1 (90 mg, 0.106 mmol) was dissolved in toluene (3 mL). Lawesson's reagent (171 mg, 0.424 mmol) was added, and the reaction proceeded at 100°C for 4 hours. After TLC monitoring indicated completion of the reaction, the reaction mixture was distilledunder reduced pressure and spin-dried. Purification by TLC plate (PE:EA = 5:1) was perfomed to obtain a yellow solid product 35-2 (45 mg, 48.9% ).

Synthesis of Compound 35:

The compound 35-2 (45 mg, 0.052 mmol) was dissolved in dichloromethane (5 mL). Trifluoroacetic acid (TFA, 5 mL) was added, and the reaction proceeded at room temperature for 3 days. After LC-MS monitoring indicated completion of the reaction, the reaction mixture was distilled under reduced pressure and concentrated. Purification by preparative HPLC and lyophilization were perfomed to obtain a yellow solid product 35 (3.34 mg, 12.5%). LC-MS(ESI):m/z found $[M+H]^+$=516.30. $^1$H NMR (600 MHz, DMSO-$d_6$) δ 10.59 (t, *J* = 5.9 Hz, 1H), 8.06 (d, *J* = 11.9 Hz, 1H), 7.85 (d, *J* = 9.0 Hz, 1H), 7.79 (s, 1H), 6.70 (s, 1H), 6.05 (t, *J* = 5.8 Hz, 1H), 5.93 (d, *J* = 16.4 Hz, 1H), 5.76 (d, *J* = 2.9 Hz, 2H), 5.53 (d, *J* = 16.5 Hz, 1H), 5.50 (dd, *J* = 10.3, 5.9 Hz, 1H), 4.35 (d, *J*= 5.7 Hz, 2H), 4.06 (s, 3H), 2.55 (s, 1H), 1.90 (hept, *J*= 7.1 Hz, 2H), 0.87 (t, *J* = 7.4 Hz, 3H).

Example **36:**

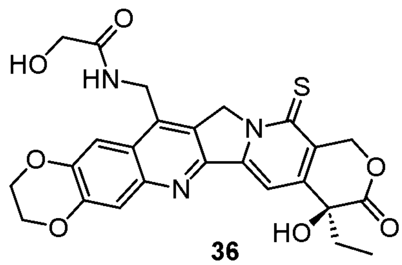

**36**

Synthesis of Compound 36:

In an ice bath, the compound 13 (5 mg, 0.011 mmol) was dissolved in anhydrous dichloromethane (5 mL). Glycolic acid (1.7 mg, 0.022 mmol), O-(7-azabenzotriazol-1-yl)- N,N,N',N'-tetramethyluronium hexafluorophosphate (HATU, 5 mg, 0.013 mmol), and N,N-diisopropylethylamine (DIEA, 5.7 μL, 0.033 mmol) were added. The reaction proceeded at room temperature for 2 hours. LCMS monitoring indicated completion of the reaction. The reaction mixture was purified by prep-HPLC to obtain a compound 36 (1.55 mg, 28%).MS (ESI): *m/z* found $[M+H]^+$= 510.35. $^1$H NMR (600 MHz, DMSO-$d_6$) δ 8.80 (*t, J* = 6.1 Hz, 1H), 7.97 (s, 1H), 7.76 (s, 1H), 7.62 (s, 1H), 6.69 (s, 1H), 5.93 (d, *J* = 16.4 Hz, 1H), 5.77 (d, *J*= 1.9 Hz, 3H), 5.52 (d, *J* = 16.4 Hz, 1H), 4.79 (dd, *J*= 6.1, 2.8 Hz, 2H), 4.46 (s, 6H), 1.92 - 1.88 (m, 2H), 0.86 (t, *J* = 7.3 Hz, 3H).

Example **37:**

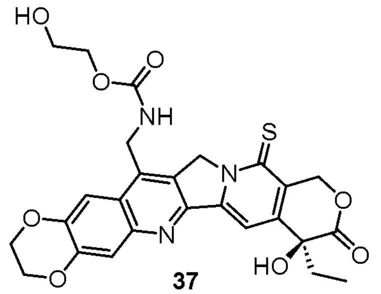

**37**

Synthetic route:

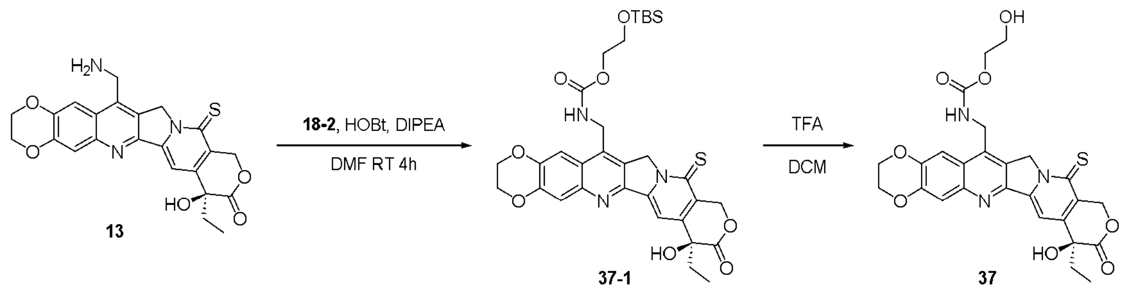


Synthesis of Intermediate 37-1:

At room temperature, the compound 13 (15 mg, 0.033 mmol) was dissolved in anhydrous N,N-dimethylformamide (3 mL). The compound 18-2 (11 mg, 0.033 mmol), 1-hydroxybenzotriazole (HOBt, 5 mg, 0.036 mmol), and N,N-diisopropylethylamine (DIEA, 17 μL, 0.099 mmol) were added. The reaction proceeded at room temperature for 3 hours. LCMS monitoring indicated completion of the reaction. The reaction mixture was purified by prep-HPLC to obtain a compound 37-1 (23 mg, 99% yield). MS (ESI): m/z found $[M+H]^+$ = 654.45.

Synthesis of Compound 37:

At room temperature, the compound 37-1 (23 mg, 0.035 mmol) was dissolved in anhydrous dichloromethane (5 mL). Trifluoroacetic acid (TFA, 2 mL) was added, and the reaction proceeded at room temperature for 2 hours. LCMS monitoring indicated completion of the reaction. The reaction mixture was concentrated under reduced pressure. The residue was dissolved in a small amount of N,N-dimethylformamide and purified by prep-HPLC to obtain a compound 37 (4.21 mg, 22%). MS (ESI): *m/z* found $[M+H]^+$= 540.35. $^1H$ NMR (400 MHz, DMSO-$d_6$) δ 8.84 (t, *J* = 6.2 Hz, 1H), 8.02 (d, *J* = 12.1 Hz, 1H), 7.97 (d, *J* = 9.2 Hz, 1H), 7.78 (s, 1H), 6.74 (s, 1H), 6.68 (s, 1H), 5.93 (d, *J* = 16.6 Hz, 1H), 5.79 (s, 2H), 5.50 (d, *J* = 16.6 Hz, 1H), 5.31 (t, *J* = 4.9 Hz, 2H), 4.95 - 4.88 (m, 2H), 4.05 (s, 3H), 2.00 (d, *J* = 7.5 Hz, 2H), 0.84 (d, *J* = 4.7 Hz, 3H).

Example **38:**

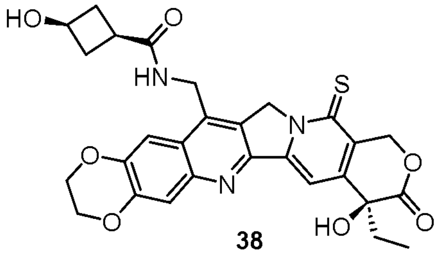

38

Synthesis of Compound 38:

In an ice bath, the compound 13 (5 mg, 0.011 mmol) was dissolved in anhydrous N,N-dimethylformamide (5 mL). cis-3-Hydroxycyclobutanecarboxylic acid (2.6 mg, 0.022 mmol), O-(7-azabenzotriazol-1-yl)-N,N,N',N'-tetramethyluronium hexafluorophosphate (4.9 mg, 0.013 mmol), and N,N-diisopropylethylamine (5.7 μL, 0.033 mmol) were added. The reaction proceeded at room temperature for 2 hours. LCMS monitoring indicated completion of the reaction. The reaction mixture was purified by prep-HPLC to obtain a compound 38 (0.34 mg, 5% yield ).MS (ESI): *m/z* found $[M+H]^+$=550.45. $^1H$ NMR (400 MHz, DMSO-$d_6$) δ 8.62 (*t, J* = 5.9 Hz, 1H), 7.80 (s, 1H), 7.75 (s, 1H), 7.61 (s, 1H), 6.68 (s, 1H), 5.93 (d, *J* = 16.5 Hz, 1H), 5.64 (s, 2H), 5.51 (d, *J* = 16.6 Hz, 1H), 5.09 (s, 1H), 4.76 (d, *J* = 5.8 Hz, 2H), 4.46 (s, 4H), 3.91 (t, *J* = 7.9 Hz, 1H), 2.45 - 2.36 (m, 1H), 2.27 (qt, *J* = 7.4, 3.8 Hz, 2H), 1.90 (dtd, *J* = 18.1, 10.5, 4.4 Hz, 4H), 0.86 (t, *J* = 7.3 Hz, 3H).

Example **39:**

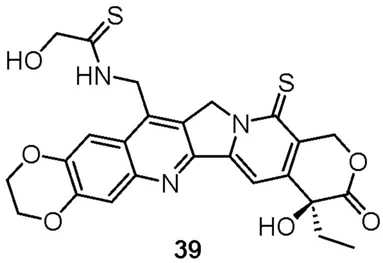

39

Synthetic route:

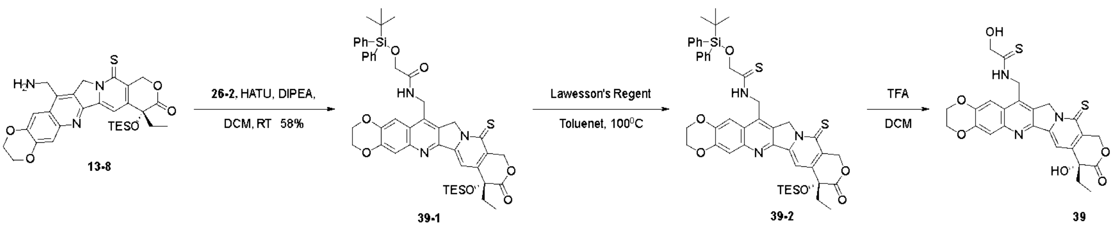


Synthesis of Intermediate 39-1:

In an ice bath, the compound 13-8 (60 mg, 0.106 mmol) was dissolved in anhydrous dichloromethane (5 mL). The compound 26-2 (67 mg, 0.212 mmol), O-(7-azabenzotriazol-1-yl)- N,N,N',N'-tetramethyluronium hexafluorophosphate (HATU, 48 mg, 0.127 mmol), and N,N-diisopropylethylamine (DIEA, 55 μL, 0.318 mmol) were added. The reaction proceeded at room temperature for 1 hour. LCMS monitoring indicated completion of the reaction. The reaction mixture was concentrated under reduced pressure and purified by normal-phase column chromatography to obtain a compound 39-1 (53 mg, 58% ). MS (ESI): m/z found $[M+H]^+$ = 862.55.

Synthesis of Intermediate 39-2:

At room temperature, the compound 39-1 (53 mg, 0.061 mmol) was dissolved in toluene (5 mL). Lawesson's reagent (75 mg, 0.184 mmol) was added. The atmosphere was purged with nitrogen for three times, and the reaction mixture was heated to 100°C, and the reaction proceeded for 4 hours. TLC monitoring indicated completion of the reaction. The reaction mixture was concentrated under reduced pressure and purified by normal-phase column chromatography to obtain a compound 39-2 (37 mg, 69% yield ).

Synthesis of Compound 39:

At room temperature, the compound 39-2 (43 mg, 0.0505 mmol) was dissolved in anhydrous dichloromethane (5 mL). Trifluoroacetic acid (TFA, 2 mL) was added, and the reaction proceeded at room temperature for 48 hours. LCMS monitoring indicated completion of the reaction. The reaction mixture was concentrated under reduced pressure. The residue was dissolved in a small amount of N,N-dimethylformamide and purified by prep-HPLC to obtain a compound 39 (4.08 mg, 18% ).MS (ESI): *m/z* found $[M+H]^+$= 526.35. $^1H$ NMR (600 MHz, DMSO-$d_6$) δ 10.55 (t, *J* = 5.8 Hz, 1H), 7.89 (s, 1H), 7.76 (s, 1H), 7.63 (s, 1H), 6.67 (s, 1H), 6.07 (t, *J* = 5.7 Hz, 1H), 5.92 (d, *J*= 16.4 Hz, 1H), 5.71 (s, 2H), 5.52 (d, *J*= 16.4 Hz, 1H), 5.35 (dd, *J* = 5.9, 3.4 Hz, 2H), 4.46 (s, 4H), 4.32 (d, *J* = 5.4 Hz, 2H), 1.90 (hept, *J* = 7.1 Hz, 2H), 0.88 - 0.85 (m, 3H).

Example **40:**

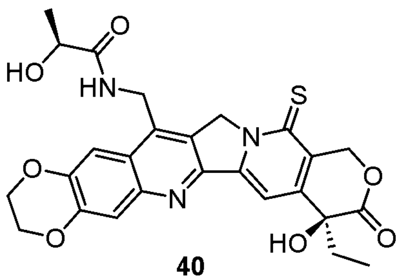

**40**

In an ice bath, the compound 13 (5 mg, 0.011 mmol) was dissolved in anhydrous N,N-dimethylformamide (5 mL). (S)-2-Hydroxypropanoic acid (1.6 μL, 0.022 mmol), O-(7-azabenzotriazol-1-yl)- N,N,N',N'-tetramethyluronium hexafluorophosphate (HATU, 4.9 mg, 0.013 mmol), and N,N-diisopropylethylamine (DIEA, 5.7 μL, 0.033 mmol) were added. The reaction proceeded at room temperature for 2 hours. LCMS monitoring indicated completion of the reaction. The reaction mixture was purified by prep-HPLC to obtain a compound 40 (0.34 mg, yield 5% ).MS (ESI): *m/z* found $[M+H]^+$=524.40. $^1H$ NMR (600 MHz, DMSO-$d_6$) δ 8.73 (t, *J* = 6.1 Hz, 1H), 7.94 (s, 1H), 7.75 (s, 1H), 7.62 (d, *J* = 10.8 Hz, 1H), 6.67 (s, 1H), 5.92 (s, 1H), 5.75 (d, *J* = 3.7 Hz, 2H), 5.60 (d, *J* = 4.9 Hz, 1H), 5.52 (d, *J* = 16.4 Hz, 2H), 4.82 (d, *J* = 6.4 Hz, 1H), 4.77 (dd, *J* = 8.6, 6.1 Hz, 2H), 4.46 (s, 4H), 4.03 - 3.95 (m, 1H), 1.91 - 1.88 (m, 2H), 1.22 (d, *J*= 6.8 Hz, 3H), 0.86 (s, 3H).

Example **41:**

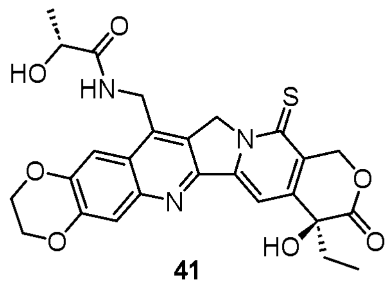

**41**

In an ice bath, the compound 13 (5 mg, 0.011 mmol) was dissolved in anhydrous N,N-dimethylformamide (5 mL). D-2-Hydroxypropanoic acid (1.6 μL, 0.022 mmol), O-(7-azabenzotriazol-1-yl)- N,N,N',N'-tetramethyluronium hexafluorophosphate (HATU, 4.9 mg, 0.013 mmol), and N,N-diisopropylethylamine (DIEA, 5.7 μL, 0.033 mmol) were added. The reaction proceeded at room temperature for 2 hours. LCMS monitoring indicated completion of the reaction. The reaction mixture was purified by prep-HPLC to obtain a compound 41 (1.16 mg, 20% ).MS (ESI): *m/z* found $[M+H]^+$=524.40. $^1H$ NMR (600 MHz, DMSO-$d_6$) δ 8.72 (t, *J* = 6.1 Hz, 1H), 7.94 (s, 1H), 7.75 (s, 1H), 7.61 (d, *J* = 1.2 Hz, 1H), 6.67 (s, 1H), 5.93 (d, *J* = 16.4 Hz, 1H), 5.75 (s, 2H), 5.60 (d, *J* = 4.9 Hz, 1H), 5.52 (d, *J* = 16.4 Hz, 1H), 4.77 (d, *J* = 6.0 Hz, 2H), 4.46 (s, 4H), 4.00 (q, *J*= 6.7, 6.1 Hz, 1H), 1.90 (hept, *J*= 7.3 Hz, 2H), 1.21 (d, *J* = 6.7 Hz, 3H), 0.86 (t, *J* = 7.3 Hz, 3H).

Example **42:**

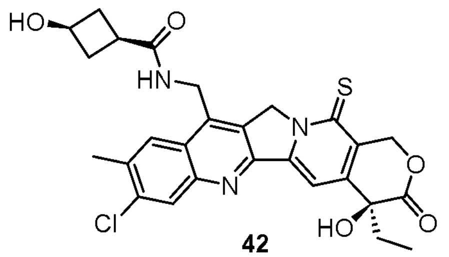

**42**

In an ice bath, the compound 14 (5 mg, 0.0113 mmol) was dissolved in anhydrous N,N-dimethylformamide (5 mL). The cis-3-Hydroxycyclobutanecarboxylic acid (2.6 mg, 0.0226 mmol), O-(7-azabenzotriazol-1-yl)- N,N,N',N'-tetramethyluronium hexafluorophosphate (HATU, 5.2 mg, 0.0136 mmol), and N,N-diisopropylethylamine (DIEA, 5.9 μL, 0.0339 mmol) were added. The reaction proceeded at room temperature for 2 hours. LCMS monitoring indicated completion of the reaction. The reaction mixture was purified by prep-HPLC to obtain a compound 42 (3.03 mg, 50% yield ).MS (ESI): *m/z* found $[M+H]^+$=540.03. $^1H$ NMR (600 MHz, DMSO-$d_6$) δ 8.68 (t, *J* = 5.9 Hz, 1H), 8.41 (s, 1H), 8.32 (s, 1H), 7.80 (s, 1H), 6.71 (s, 1H), 5.93 (d, *J* = 16.5 Hz, 1H), 5.68 (s, 2H), 5.53 (d, *J* = 16.5 Hz, 1H), 5.09 (d, *J* = 7.0 Hz, 1H), 4.89 (d, *J* = 5.9 Hz, 2H), 3.92 (q, *J* = 7.1 Hz, 1H), 2.61 (s, 3H), 2.44 (tt, *J* = 9.8, 7.4 Hz, 1H), 2.29 (ddt, *J*= 11.7, 6.9, 3.6 Hz, 2H), 1.98 - 1.92 (m, 2H), 1.89 (dt, *J* = 14.3, 7.5 Hz, 2H), 0.86 (d, *J* = 7.4 Hz, 3H).

Example **43:**

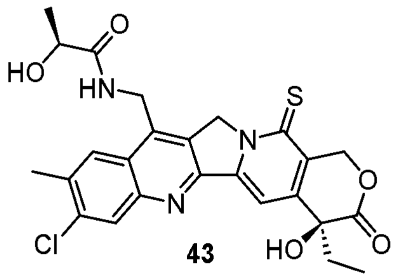

**43**

In an ice bath, the compound 14 (5 mg, 0.0113 mmol) was dissolved in anhydrous N,N-dimethylformamide (5 mL). (S)-2-Hydroxypropanoic acid (1.7 μL, 0.0226 mmol), 4-(4,6-Dimethoxy-1,3,5-triazin-2-yl)-4-methylmorpholinium chloride (DMTMM, 6.3 mg, 0.0226 mmol), and triethylamine (TEA, 3.9 μL, 0.0283 mmol) were added. The reaction proceeded at room temperature for 1 hour. LCMS monitoring indicated completion of the reaction. The reaction mixture was purified by prep-HPLC to obtain a compound 43 (1.76 mg, 30% ).MS (ESI): *m/z* found $[M+H]^+$=514.35. $^1H$ NMR (400 MHz, DMSO-$d_6$) δ 8.78 (t, *J* = 6.0 Hz, 1H), 8.51 (s, 1H), 8.31 (s, 1H), 7.80 (s, 1H), 6.71 (s, 1H), 5.93 (d, *J* = 16.7 Hz, 1H), 5.77 (s, 2H), 5.63 (d, *J* = 4.8 Hz, 1H), 5.53 (d, *J= 16.7* Hz, 1H), 4.97 - 4.81 (m, 2H), 4.06 - 3.95 (m, 1H), 2.60 (s, 3H), 1.90 (dt, *J*= 11.6, 6.9 Hz, 2H), 1.22 (d, *J* = 6.8 Hz, 4H), 0.86 (t, *J* = 7.3 Hz, 3H).

Example **44:**

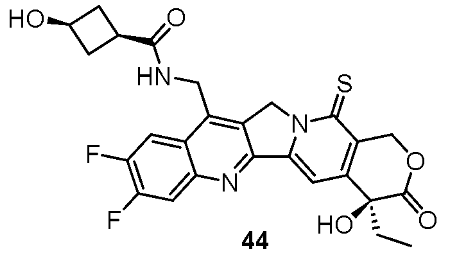

44

In an ice bath, the compound 15 (5 mg, 0.011 mmol) was dissolved in anhydrous N,N-dimethylformamide (5 mL). The cis-3-Hydroxycyclobutanecarboxylic acid (2.6 mg, 0.022 mmol), O-(7-azabenzotriazol-1-yl)- N,N,N',N'-tetramethyluronium hexafluorophosphate (HATU, 4.9 mg, 0.013 mmol), and N,N-diisopropylethylamine (DIEA, 5.7 μL, 0.033 mmol) were added. The reaction proceeded at room temperature for 2 hours. LCMS monitoring indicated completion of the reaction. The reaction mixture was purified by prep-HPLC to obtain a compound 44 (0.34 mg, yield 5%).MS (ESI): *m/z* found $[M+H]^+$=550.45. $^1$H NMR (600 MHz, DMSO-$d_6$) δ 8.69 (t, *J* = 6.0 Hz, 1H), 8.50 (dd, *J*= 12.3, 8.6 Hz, 1H), 8.30 (dd, *J* = 11.3, 8.0 Hz, 1H), 7.81 (s, 1H), 6.72 (s, 1H), 5.94 (d, *J* = 16.5 Hz, 1H), 5.72 (s, 2H), 5.54 (d, *J* = 16.5 Hz, 1H), 5.09 (d, *J* = 7.0 Hz, 1H), 4.86 (d, *J* = 6.0 Hz, 2H), 3.97 - 3.86 (m, 1H), 2.43 (tt, *J* = 9.9, 7.5 Hz, 1H), 2.32 - 2.23 (m, 2H), 1.96 - 1.85 (m, 4H), 0.86 (t, *J* = 7.3 Hz, 3H).

Example **45:**

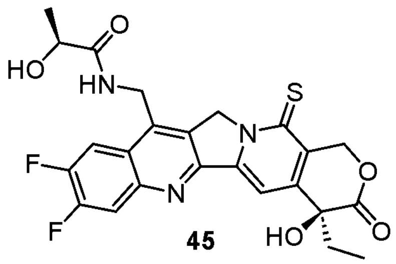

45

In an ice bath, the compound 15 (5 mg, 0.0116 mmol) was dissolved in anhydrous N,N-dimethylformamide (5 mL). (S)-2-Hydroxypropanoic acid (1.7 μL, 0.0232 mmol), O-(7-azabenzotriazol-1-yl)- N,N,N',N'-tetramethyluronium hexafluorophosphate (HATU, 5.3 mg, 0.0139 mmol), and N,N-diisopropylethylamine (DIEA, 6.1 μL, 0.0348 mmol) were added. The reaction proceeded at room temperature for 1 hour. LCMS monitoring indicated completion of the reaction. The reaction mixture was purified by prep-HPLC to obtain a compound 45 (1.75 mg, 30% yield).MS (ESI): *m/z* found $[M+H]^+$=502.30. $^1$H NMR (600 MHz, DMSO-$d_6$) δ 8.85 (t, *J* = 6.1 Hz, 1H), 8.63 (dd, *J* = 12.4, 8.6 Hz, 1H), 8.30 (dd, *J* = 11.3, 8.0 Hz, 1H), 7.82 (s, 1H), 6.72 (s, 1H), 5.94 (d, *J = 16.6* Hz, 1H), 5.81 (s, 2H), 5.64 (d, *J* = 4.8 Hz, 1H), 5.54 (d, *J* = 16.5 Hz, 1H), 4.89 - 4.80 (m, 2H), 4.04 - 3.97 (m, 1H), 1.90 (hept, *J* = 7.1 Hz, 2H), 1.20 (d, *J* = 6.8 Hz, 3H), 0.87 (t, *J* = 7.3 Hz, 3H).

Example **46:**

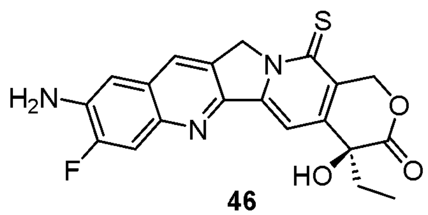

46

Synthetic route:

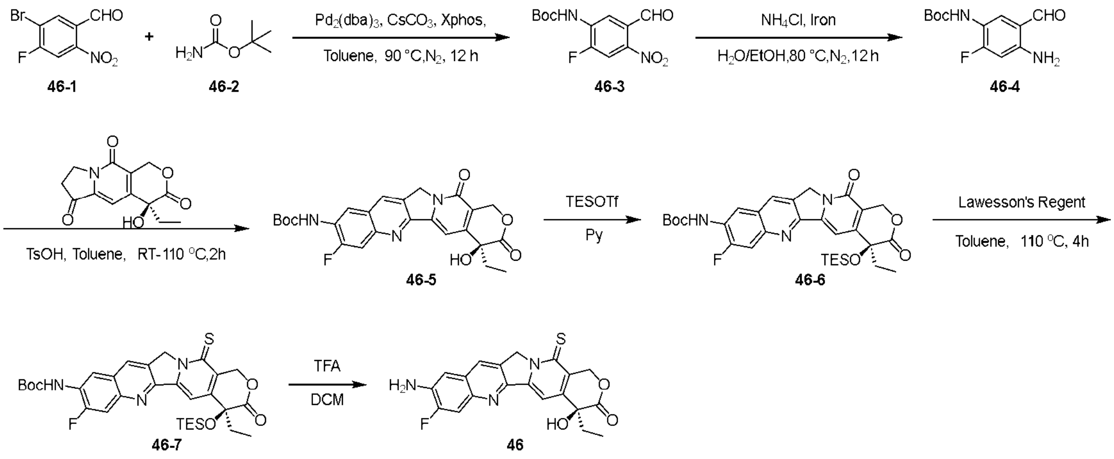


Synthesis of Intermediate 46-3:

At room temperature, 5-bromo-4-fluoro-2-nitrobenzaldehyde (46-1, 1.0 g, 4.032 mmol) was dissolved in toluene (20 mL). tert-Butyl carbamate (567 mg, 4.839 mmol), $Pd_2(dba)_3$ (185 mg, 0.202 mmol), $Cs_2CO_3$ (4.2 g, 8.064 mmol), and XPhos (385 mg, 0.801 mmol) were added. The atmosphere was purged with nitrogen for three times, and the reaction mixture was heated to 90°C, and the reaction proceeded overnight. LC-MS monitoring indicated complete consumption of the starting material. The reaction mixture was diluted with ethyl acetate and washed with water for three times and the organic phases were combined. The combined organic phase was dried over anhydrous $Na_2SO_4$ and concentrated. Purification by column chromatography (petroleum ether:ethyl acetate = 93:7) was perfomed to obtain a compound 46-3 (878 mg, 77% yield ). LC-MS (ESI): m/z found $[M+H]^+$ = 285.2.

Synthesis of Intermediate 46-4:

At room temperature, the compound 46-4 (878 mg, 3.089 mmol) was dissolved in a mixture of water (5 mL) and ethanol (20 mL). Ammonium chloride (268 mg, 5.004 mmol) and iron powder (691 mg, 12.356 mmol) were added. The atmostphere was purged with nitrogen for three times, and the reaction mixture was heated to 80°C, and the reaction proceeded overnight. LC-MS monitoring indicated complete consumption of the starting material. The reaction mixture was filtered to remove the iron powder. The filtrate was concentrated, diluted with ethyl acetate, and washed with water for three times, and the organic phases were combined. The combined organic phase was dried over anhydrous $Na_2SO_4$ and concentrated. Purification by column chromatography (dichloromethane:methanol = 99:1) was perfomed to obtain a compound 46-4 (571 mg, 73% yield ). MS (ESI): m/z found $[M+H]^+$ = 255.4.

Synthesis of Intermediate 46-5:

At room temperature, the compound 46-4 (571 mg, 2.242 mmol) was dissolved in toluene (10 mL). (S)-4-Ethyl-4-hydroxy-7,8-dihydro-1H-pyrano[3,4-f]indolizine-3,6,10(4H)-trione (492 mg, 1.868 mmol) and p-toluenesulfonic acid (TsOH, 33 mg, 0.187 mmol) were added. The reaction mixture was heated to 110°C, and the reaction proceeded for 2 hours. LC-MS monitoring indicated complete consumption of the starting material. After being cooled and concentrated, the residue was recrystallized from diethyl ether to obtain a compound 46-5 (749 mg, 69% yield ). LC-MS (ESI): m/z found $[M+H]^+$ = 482.

Synthesis of Intermediate 46-6:

At room temperature, the compound 46-5 (400 mg, 0.831 mmol) was dissolved in dry pyridine (6 mL). Triethylsilyl trifluoromethanesulfonate (TESOTf, 1.8 mL, 8.31 mmol) was added, and the reaction proceeded overnight. TLC monitoring indicated completion of the reaction. The reaction mixture was dried, diluted with dichloromethane, and washed with water for three times and saturated NaCl solution once. The organic phase was dried over anhydrous $Na_2SO_4$ and concentrated. Purification by column chromatography (petroleum ether:ethyl acetate = 65:35) was perfomed to obtain a compound 46-6 (340 mg, yield 68% ). MS (ESI): m/z found $[M+H]^+$ = 596.2.

Synthesis of Intermediate 46-7:

At room temperature, the compound 46-6 (202 mg, 0.339 mmol) was dissolved in toluene (10 mL). Lawesson's reagent (343 mg, 0.848 mmol) was added. The reaction mixture was heated to 100°C, and the reacton proceeded for 4 hours. TLC monitoring indicated completion of the reaction. The reaction mixture was concentrated and dissolved in a small amount of dichloromethane, and purified by column chromatography (petroleum ether:ethyl acetate = 65:35) to obtain a compound 46-7 (102 mg, yield 95%).

Synthesis of Compound 46:

At room temperature, the compound 46-7 (30 mg, 0.049 mmol) was dissolved in dry dichloromethane (5 mL). Trifluoroacetic acid (TFA, 1 mL) was added, and the reaction proceeded at room temperature overnight. LC-MS monitoring indicated completion of the reaction. The reaction mixture was purified by reverse-phase column chromatography to obtain a compound 46 (20 mg, yield 99% ). LC-MS (ESI): m/z found $[M+H]^+$ = 398.0.

Example **47:**

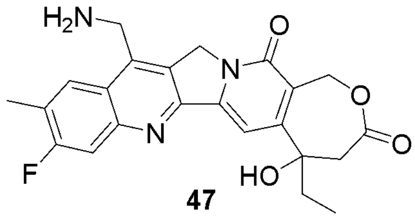

**47**

Synthetic route:

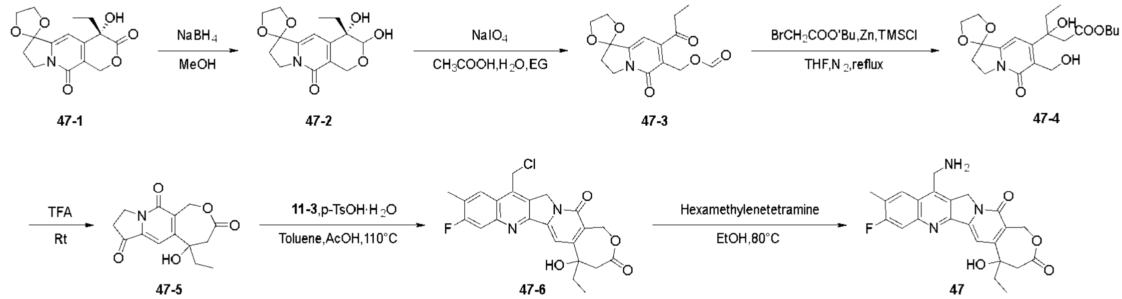


47-1 47-2 47-3 47-4 47-5 47-6 47

Synthesis of Intermediate 47-2:

The compound 47-1 (2.0 g, 6.51 mmol) was dissolved in MeOH (40 mL). $NaBH_4$ (0.172 g, 4.55 mmol) was added in three portions, and the reaction proceeded at room temperature for 30 minutes. The solvent was removed through distilling under reduced pressure. A small amount of $H_2O$ was added, and the pH was adjusted to 4 by dropwise addition of 2M HCl aqueous solution, resulting in a large amount of solid precipitated. The solid was collected by suction filtration, the filter cake was washed with a small amount of cold water, and lyophilized to obtain an off-white solid product 47-2 (1.135 g, 56.4% ). LC-MS: $[M+H]^+$ = 310.2.

Synthesis of Intermediate 47-3:

Glacial acetic acid (60 mL) was added into the compound 47-2 (1.135 g, 3.67 mmol) . A solution of $NaIO_4$ (1.138 g, 5.32 mmol) in $H_2O$ (12 mL) was prepared and added dropwise into the solution of the compound 47-2 in glacial acetic acid at room temperature. The reaction mixure was stirred at room temperature until the reaction was completed. Ethylene glycol (EG, 1 mL) was added to quench the reaction. The reaction mixture was extracted with DCM/$H_2O$. The organic phase was dried over anhydrous $Na_2SO_4$, filtered, and distilled under reduced pressure to remove solvent. Purification by

medium-pressure liquid chromatography (DCM:MeOH = 95:5) and concentration were performed to obtain a white solid product 47-3 (1.0 g, 88.9% yield). LC-MS: $[M+H]^+$ = 308.0.

Synthesis of Intermediate 47-4:

The zinc powder (1.625 g, 24.7 mmol) was suspended in anhydrous THF (30 mL) under $N_2$ atmosphere. TMSCl (0.706 g, 6.5 mmol) was added dropwise at room temperature to activate the zinc for 15 minutes, then the reaction mixture was heated to reflux. The heating bath was removed while maintaining reflux, and tert-butyl bromoacetate (3.6 mL, 24.7 mmol) was added dropwise. Heating was continued until the zinc powder was completely dissolved. A solution of 47-3 in anhydrous THF was then added dropwise, and the reaction mixture was heated under reflux for 2 hours. After the reaction mixture was cooled to room temperature, the reaction mixture was extracted with DCM/saturated $NH_4Cl$ aqueous solution. The organic phase was dried over anhydrous $Na_2SO_4$, filtered, and distilled under reduced pressure to remove solvent. Purification by medium-pressure liquid chromatography (DCM:MeOH = 96:6) and concentration were performed to obtain a pale yellow oily compound 47-4 (1.268 g, 98.6% yield ). LC-MS: $[M+H]^+$ = 396.0.

Synthesis of Intermediate 47-5:

Trifluoroacetic acid (TFA, 7.74 mL) was added dropwise to 47-4 (1.268 g, 3.206 mmol) at room temperature. The reaction proceeded at room temperature overnight. The solvent was removed through distilling under reduced pressure. Purification by medium-pressure liquid chromatography (DCM:MeOH = 98:2) and concentration were perfomed to obtain a light pink solid 47-5 (0.502 g, 56.5% yield ). LC-MS: $[M+H]^+$ = 278.0.

Synthesis of Intermediate 47-6:

The compound 47-5 (0.251 g, 0.905 mmol) was dissolved in a mixture of toluene (3 mL) and glacial acetic acid (3 mL) at room temperature. The compound 11-3 (0.182 g, 0.905 mmol) and p-TsOH·$H_2O$ (9 mg) were added sequentially. The reaction mixture was heated to 110 °C and the reaction proceeded overnight. The solvent was removed through distilling under reduced pressure. Acetone (8 mL) was added, and the reaction mixture was stirred at room temperature for 30 minutes. A pale yellow solid 47-6 was obtained by suction filtrations (0.247 g, 63.4% ). LC-MS: $[M+H]^+$ = 443.0.

Synthesis of Compound 47:

The compound 47-6 (50 mg, 0.113 mmol) was dissolved in EtOH (5 mL). Hexamethylenetetramine (urotropine, 47.6 mg, 0.339 mmol) was added at room temperature, and the reaction mixture was heated to 80°C to reflux for 48 hours. The solvent was removed through distilling under reduced pressure. Purification by reverse-phase column chromatography was perfomed to obtain a yellow solid product 47 (10 mg, 20.9% ). LC-MS: $[M+H]^+$ = 424.0.

Example 48:

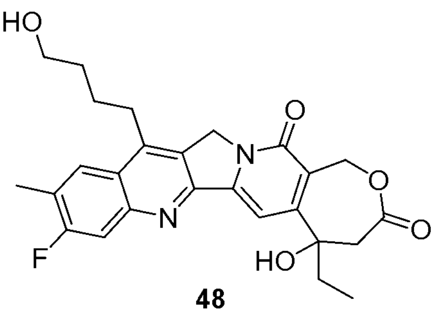

**48**

Synthetic route:

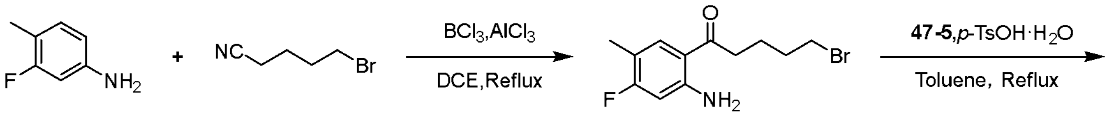


11-1 48-1 48-2

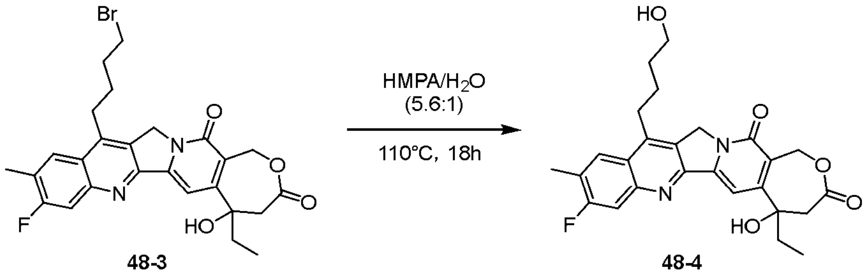


48-3 48-4

Synthesis of Intermediate 48-2:

1,2-Dichloroethane (DCE, 25 mL) was added to a erlenmeyer flask and cooled to 0°C in an ice bath. Boron trichloride ($BCl_3$, 6 mL, 6.39 mmol) was added, and the reaction mixture was stirred in the ice bath for 10 minutes. The compounds 11-1 (1 g, 7.99 mmol), 48-1 (1.502 g, 9.388 mmol), and aluminum chloride ($AlCl_3$, 1.39 g, 10.4 mmol) were added sequentially. The reaction proceeded at room temperature for 10 minutes, then the reaction mixture was heated to 80°C, and the reaction proceeded overnight. The reaction mixture was cooled to room temperature. Water (20 mL) was added slowly under ice bath, and the reaction mixture was stirred for 10 minutes. Hydrochloric acid (2 M, 40 mL) was added, and the reaction mixture was stirred at 0°C for 30 minutes. The reaction mixture was extracted with DCM/$H_2O$ (3 × 30 mL). The organic phase was dried over anhydrous $Na_2SO_4$, filtered, and distilled under reduced pressure to remove solvent. Purification by medium-pressure liquid chromatography (petroleum ether:ethyl acetate = 2:1) was performed to obtain a red oily compound 48-2 (1.85 g, 80.4% ). LC-MS: $[M+H]^+$ = 288.0.

Synthesis of Intermediate 48-3:

At room temperature, the compound 48-2 (100 mg, 0.361 mmol) was dissolved in a mixture of toluene (3 mL) and glacial acetic acid (3 mL). The compound 47-5 (83 mg, 0.288 mmol) and p-TsOH·$H_2O$ (5 mg) were added sequentially. The reaction mixture was heated to 110°C, and the reaction proceeded for 7 hours. The solvent was removed through distilling under reduced pressure. Purification by medium-pressure liquid chromatography was performed to obtain a compound 48-3 (30 mg, 15.7% yield). LC-MS: $[M+H]^+$ = 529.0.

Synthesis of Compound 48:

The compound 48-3 (30 mg, 0.057 mmol) was dissolved in a mixture of hexamethylphosphoramide (HMPA, 1.5 mL) and water ($H_2O$, 0.3 mL). The reaction mixture was heated to 110°C, and the reaction proceeded for 4 hours. Purification by preparative HPLC was perfomed to obtain a compound 27 (2.26 mg, 8.5% yield).LC-MS: $[M+H]^+$=467.35. $^1H$ NMR (600 MHz, DMSO-$d_6$) δ 8.24 (d, $J$ = 8.2 Hz, 1H), 7.85 (d, $J$ = 10.8 Hz, 1H), 7.37 (s, 1H), 6.04 (s, 1H), 5.53 (d, $J$ = 15.1 Hz, 1H), 5.40 (d, $J$ = 15.0 Hz, 1H), 5.28 (d, $J$ = 4.1 Hz, 2H), 4.45 (t, $J$ = 5.1 Hz, 1H), 3.48 (q, $J$ = 6.8, 5.9 Hz, 3H), 3.20 (dq, $J$ = 9.2, 6.0, 5.1 Hz, 2H), 3.05 (d, $J$ = 13.9 Hz, 1H), 2.52 (s, 3H), 1.85 (q, $J$ = 7.4 Hz, 2H), 1.74 (p, $J$ = 7.9 Hz, 2H), 1.62 (p, $J$ = 6.4 Hz, 2H), 0.86 (t, $J$ = 7.4 Hz, 4H).

Example **49:**

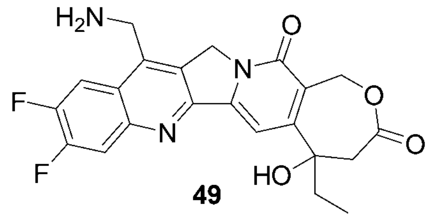

**49**

Synthetic route:

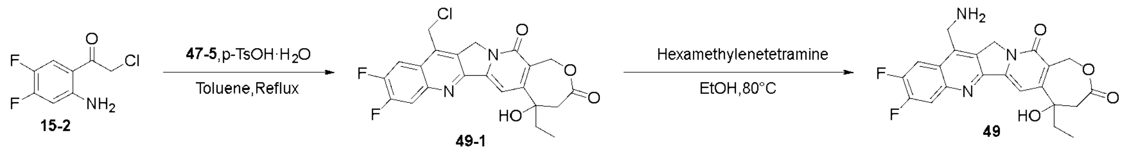


Synthesis of Intermediate 49-1:

At room temperature, the compound 47-5 (100 mg, 0.361 mmol) was dissolved in a mixture of toluene (3 mL) and glacial acetic acid (3 mL). The compound 15-2 (66.6 mg, 0.325 mmol) and p-TsOH·$H_2O$ (5 mg) were added sequentially. The reaction mixture was heated to 110°C, and the reaction proceeded for 5 hours. The solvent was removed through distilling under reduced pressure. Purification by medium-pressure liquid chromatography (dichloromethane:methanol = 94:6) was performed to obtain a yellow solid 49-1 (50 mg, 31.0% yield). LC-MS: $[M+H]^+$ = 447.0.

Synthesis of Compound 49:

The compound 49-1 (50 mg, 0.112 mmol) was dissolved in EtOH (5 mL). Hexamethylenetetramine (46 mg, 0.336 mmol) was added at room temperature, and the reaction mixture was heated to 78°C to reflux for 48 hours. The solvent was removed through distilling under reduced pressure. Purification by HPLC was perfomed to obtain a pale yellow solid product 49 (1 mg, yield 2.1% ). LC-MS: $[M+H]^+$ = 428.0.

Example 50:

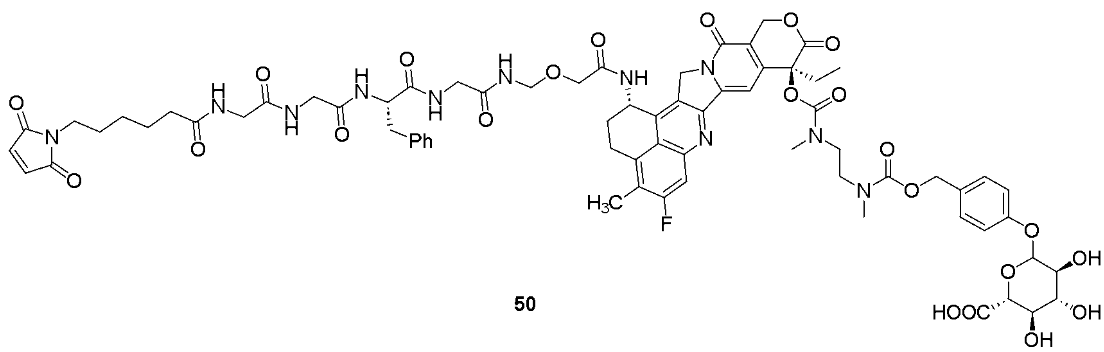

**50**

Synthetic route:

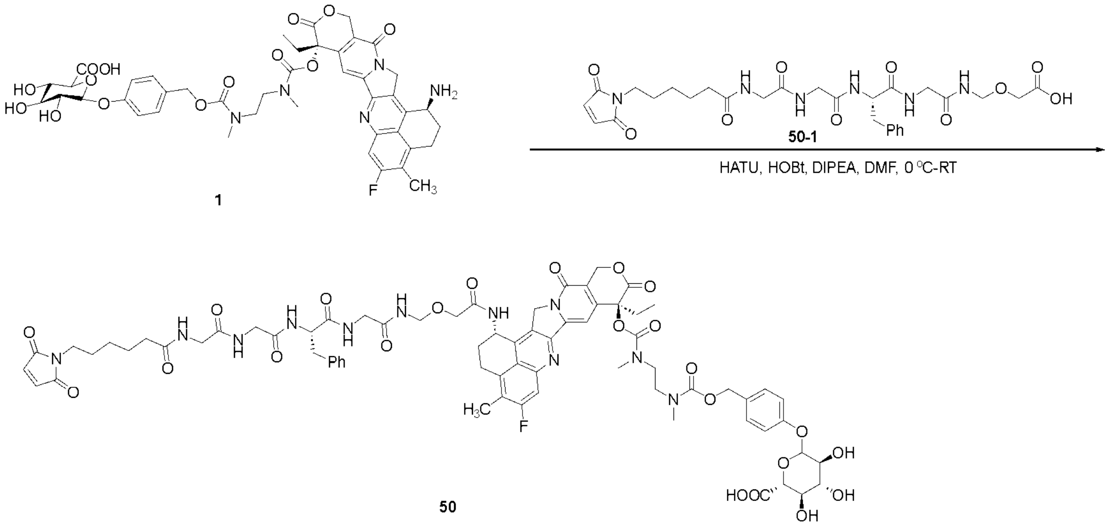


1

50-1

50

Synthesis of Compound 50:

In an ice-water bath, the compound 1 (5 mg, 0.0050 mmol) was dissolved in ultra-dry DMF. The compounds 50-1 (3.5 mg, 0.006 mmol), HATU (2.5 mg, 0.0065 mmol), HOBt (0.9 mg, 0.0065 mmol), and N,N-diisopropylethylamine (1.5 mg, 0.011 mmol) were added sequentially. The reaction proceeded at room temperature for 1 hour. LCMS monitoring indicated completion of the reaction. The reaction mixture was concentrated and purified by prep-HPLC to obtain a compound 50 (1.0 mg, 12% ). MS (ESI): m/z found $[M+H]^+$ = 1475.4.

Example **51:**

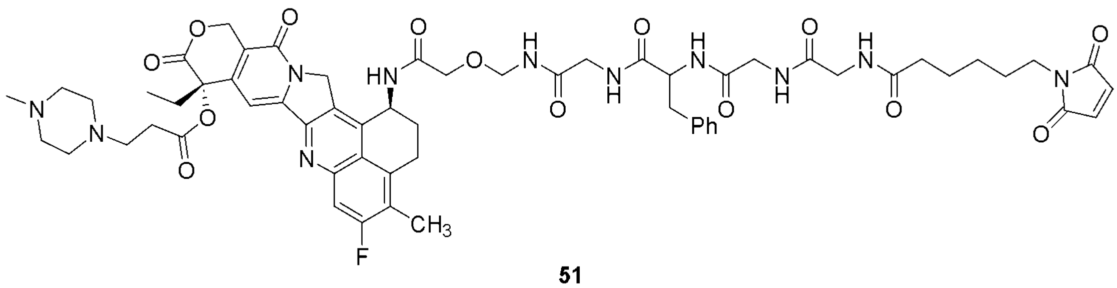

**51**

Synthetic route:

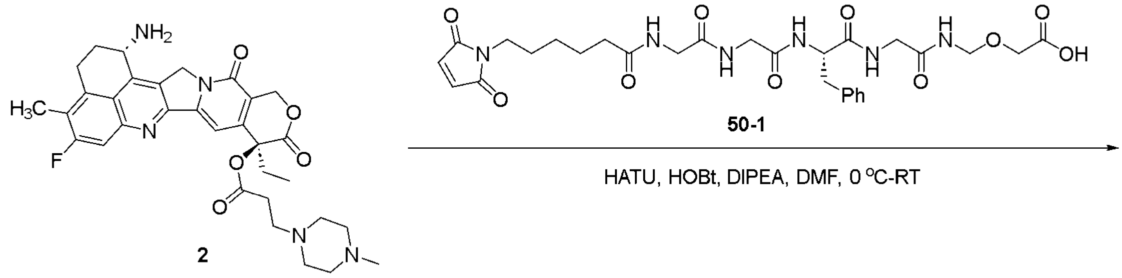


2

50-1

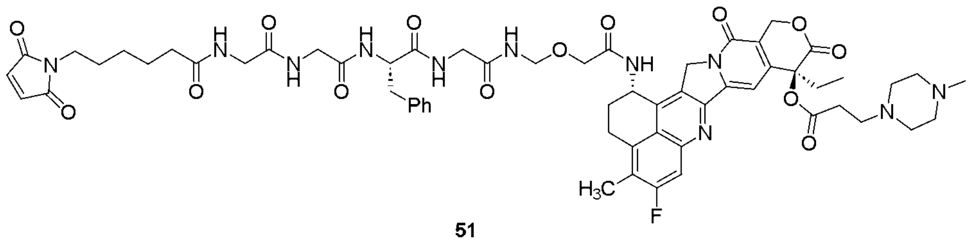

51

Synthesis of Compound 51:

In an ice-water bath, the compound 2 (4.09 mg, 0.0069 mmol) was dissolved in ultra-dry DMF (5 mL). The compound 50-1 (4.28 mg, 0.0069 mmol), HATU (4.56 mg, 0.0079 mmol), HOBt (1.07 mg, 0.0079 mmol), and N,N-diisopropylethylamine (2.00 mg, 0.0150 mmol) were added sequentially. The reaction then proceeded at room temperature for 1 hour. LC-MS monitoring indicated completion of the reaction. The reaction mixture was concentrated and purified by prep-HPLC to obtain a compound 51 (L1-S02) (5.90 mg, 73%). MS (ESI): m/z found $[M/2+H]^+$ = 648.3.

Example 52:

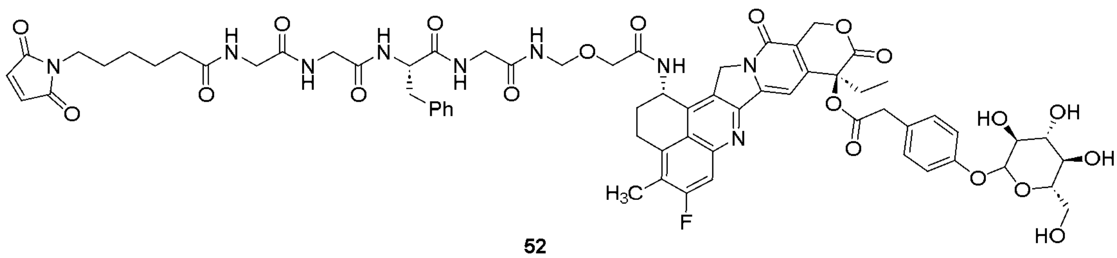

52

Synthetic route:

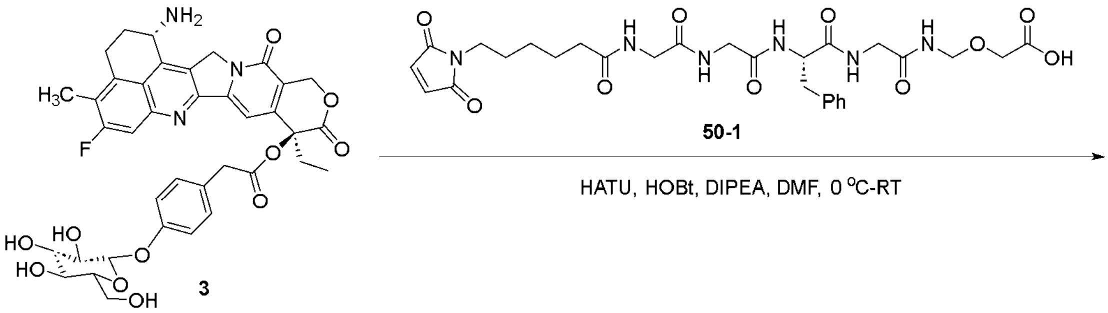


3

50-1

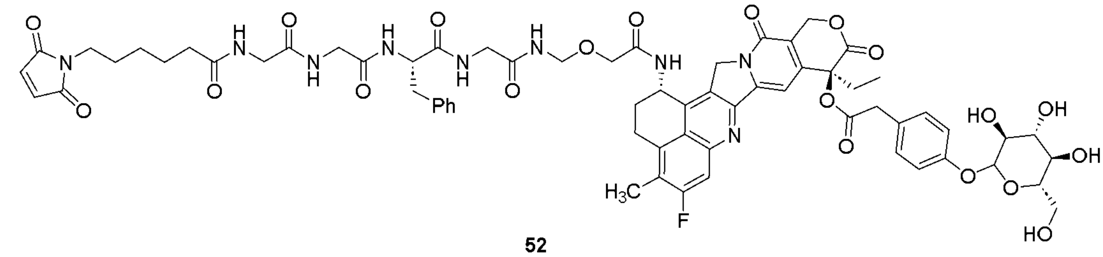

52

Synthesis of Compound 52:

In an ice-water bath, the compound 3 (5.0 mg, 0.0068 mmol) was dissolved in ultra-dry DMF (5 mL). The compound 50-1 (4.2 mg, 0.0068 mmol), 2-(7-Azabenzotriazol-1-yl)-N,N,N',N'-tetramethyluronium hexafluorophosphate (HATU, 4.5 mg, 0.0078 mmol), 1-Hydroxybenzotriazole hydrate (HOBt·$H_2O$, 1.1 mg, 0.0078 mmol), and N,N-Diisopropylethylamine (DIPEA, 1.7 mg, 0.0129 mmol) were added sequentially. The reaction proceeded for 10 minutes, then proceeded at room temperature for 1 hour. LC-MS monitoring indicated completion of the reaction. The reaction mixture was concentrated and purified by prep-HPLC to obtain a compound 52 (L1-3) (2.43 mg, 27%). MS (ESI): m/z found $[M+H]^+$ = 1331.5.

Example **53:**

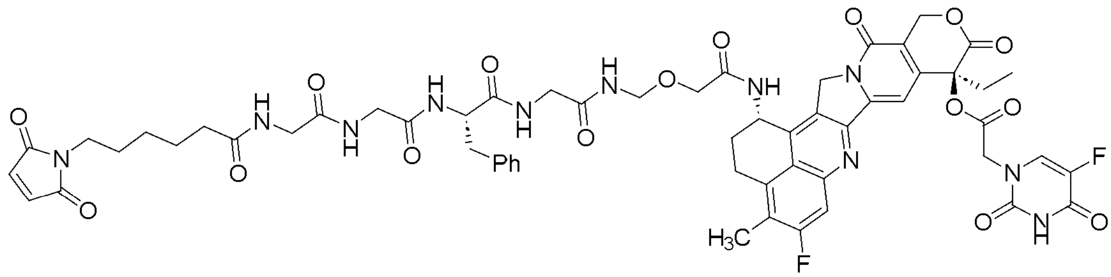

53

Synthetic route:

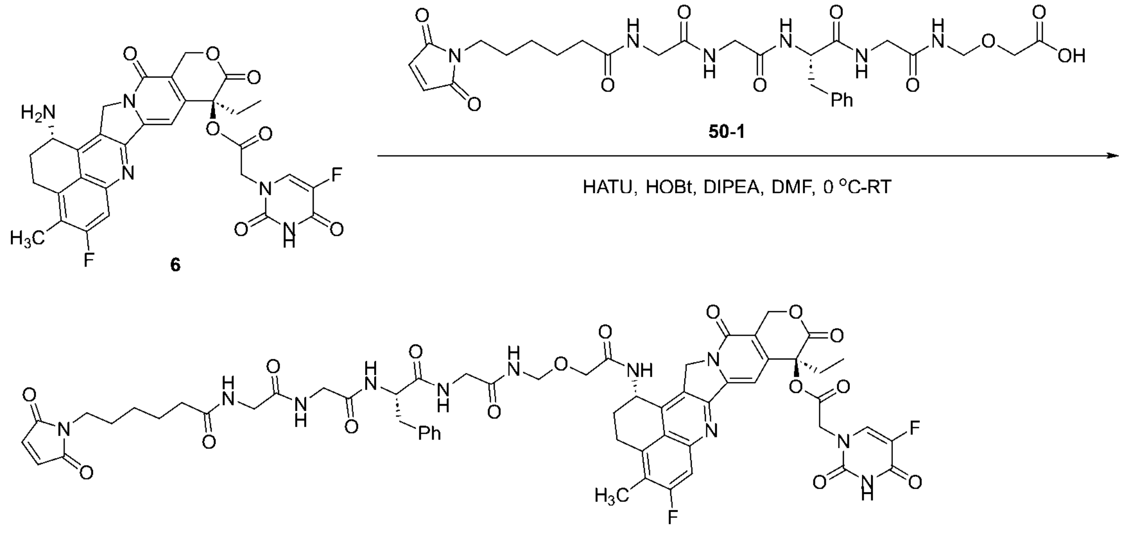


6

50-1

53

Synthesis of Compound 53:

In an ice-water bath, the 6 (5.0 mg, 0.0083 mmol) was dissolved in ultra-dry DMF. The compound 50-1 (5.1 mg, 0.0083 mmol), 2-(7-Azabenzotriazol-1-yl)-N,N,N',N'-tetramethyluronium hexafluorophosphate (HATU, 5.5 mg, 0.0095 mmol), 1-Hydroxybenzotriazole hydrate ($HOBt{\cdot}H_2O$, 1.3 mg, 0.007895 mmol), and N,N-Diisopropylethylamine (DIPEA, 2.1 mg, 0.0158 mmol) were added sequentially. The reaction proceeded for 10 minutes, then proceeded at room temperature for 1 hour. LC-MS monitoring indicated completion of the reaction. The reaction mixture was concentrated and purified by prep-HPLC to obtain a compound (4.08 mg, 41% ). MS (ESI): m/z found $[M+H]^+$ = 1205.4.

Example **54:**

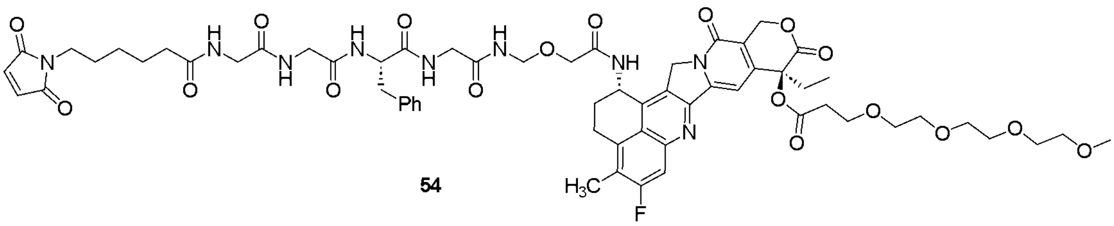

54

Synthetic route:

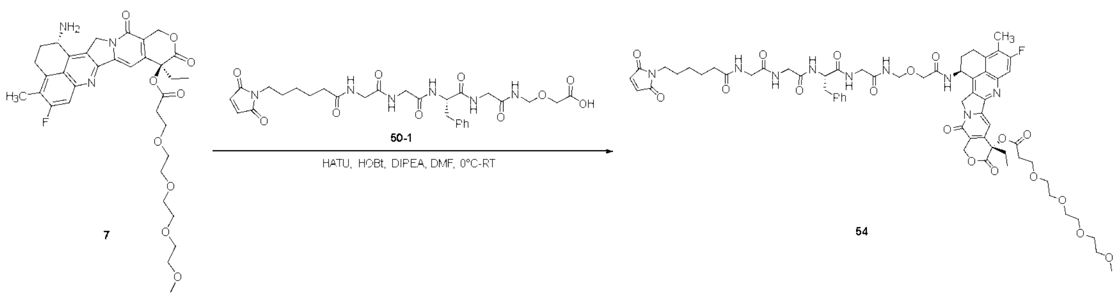


7

50-1

54

Synthesis of Compound 54:

At room temperature (under ice bath conditions), the compound 7 (5 mg, 0.0077 mmol) was dissolved in

anhydrous DMF (2 mL). The compounds 50-1 (4.7 mg, 0.0077 mmol), 2-(7-Azabenzotriazol-1-yl)-N,N,N',N'-tetramethyluronium hexafluorophosphate (HATU, 5.1 mg, 0.0088 mmol), 1-hydroxybenzotriazole hydrate (HOBt·$H_2O$, 1.2 mg, 0.0088 mmol), and N,N-diisopropylethylamine (DIPEA, 1.9 mg, 0.0146 mmol) were added sequentially. The reaction proceeded for 10 minutes, then the reaction mixture was warmed to room temperature, and the reaction proceeded for 2 hours. LC-MS monitoring indicated completion of the reaction. The reaction mixture was purified by prep-HPLC to obtain a compound (4 mg, 42% yield). MS (ESI): m/z found $[M+H]^+$ = 1252.85.

Example **55:**

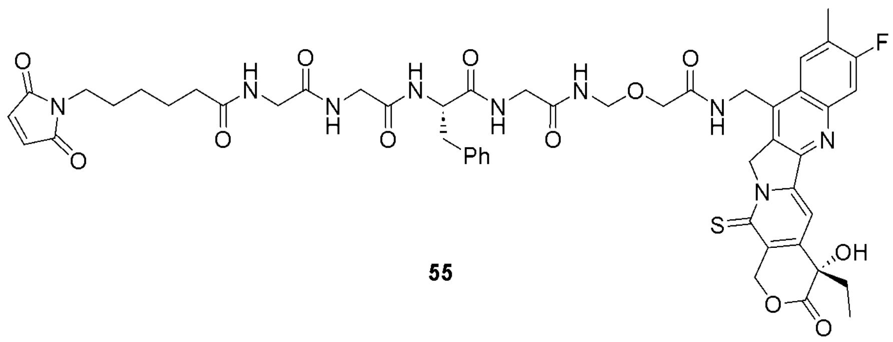

**55**

Synthesis of Compound 55:

In an ice bath, the compound 50-1 (7.2 mg, 0.0118 mmol) was dissolved in ultra-dry DMF (2 mL). 2-(7-Azabenzotriazol-1-yl)-N,N,N',N'-tetramethyluronium hexafluorophosphate (HATU, 7.8 mg, 0.0134 mmol) and 1-hydroxybenzotriazole hydrate (HOBt·$H_2O$, 2 mg, 0.0134 mmol) were added. The reaction proceeded for 10 minutes. The compound 11 (5 mg, 0.0118 mmol) and N,N-diisopropylethylamine (DIEA, 1.5 mg, 0.0224 mmol) were then added. The reaction proceeded for 10 minutes, the reaction mixture was warmed to room temperature, and the reaction proceeded for an additional hour. LC-MS monitoring indicated completion of the reaction. The reaction mixture was purified by prep-HPLC to obtain a compound 55 (2.22 mg, 19%). MS (ESI): m/z found $[M+H]^+$ = 1024.60.

Example **56:**

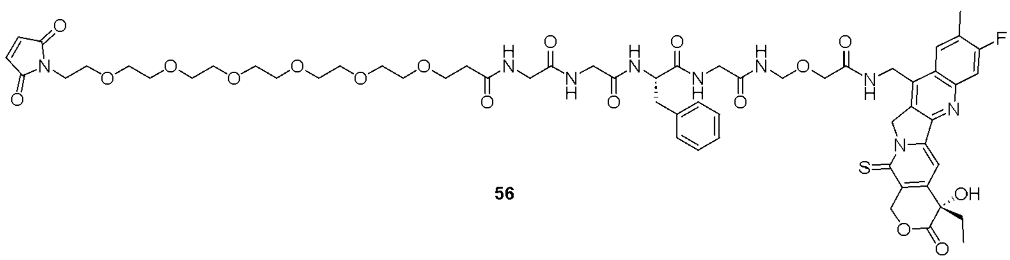

**56**

Synthetic route:

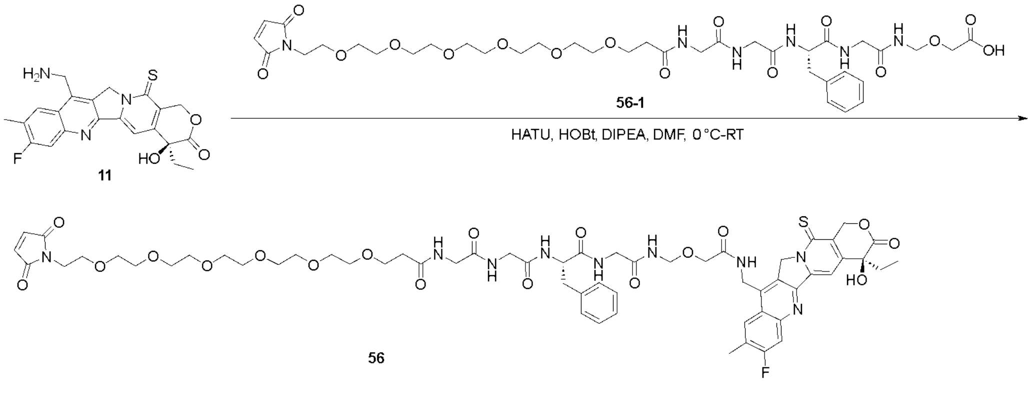

56-1
11
56

Synthesis of Compound 56:

In an ice bath, the compound 56-1 (5.9 mg, 0.0071 mmol) was dissolved in ultra-dry DMF (2 mL). 2-(7-Azabenzotriazol-1-yl)-N,N,N',N'-tetramethyluronium hexafluorophosphate (HATU, 3.8 mg, 0.0066 mmol) and 1-hydroxybenzotriazole hydrate (HOBt·$H_2O$, 1.0 mg, 0.0066 mmol) were added. The reaction proceeded for 10 minutes. The compound 11 (3.0 mg, 0.0071 mmol) and N,N-diisopropylethylamine (DIEA, 1.4 mg, 0.0135 mmol) were then added. The reaction proceeded for 10 minutes, the reaction mixture was warmed to room temperature, and the reaction proceeded for an additional hour. LC-MS monitoring indicated completion of the reaction. The reaction mixture was purified by prep-HPLC to obtain a compound 56 (0.43 mg, 5% ). MS (ESI): m/z found $[M+H]^+$ = 1246.80.

Example **57:**

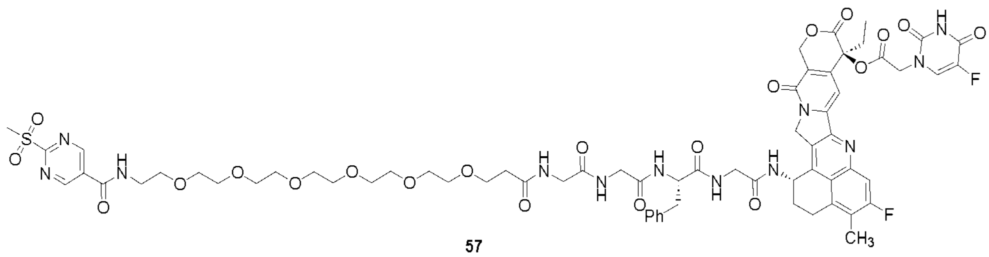
57

Synthetic route:

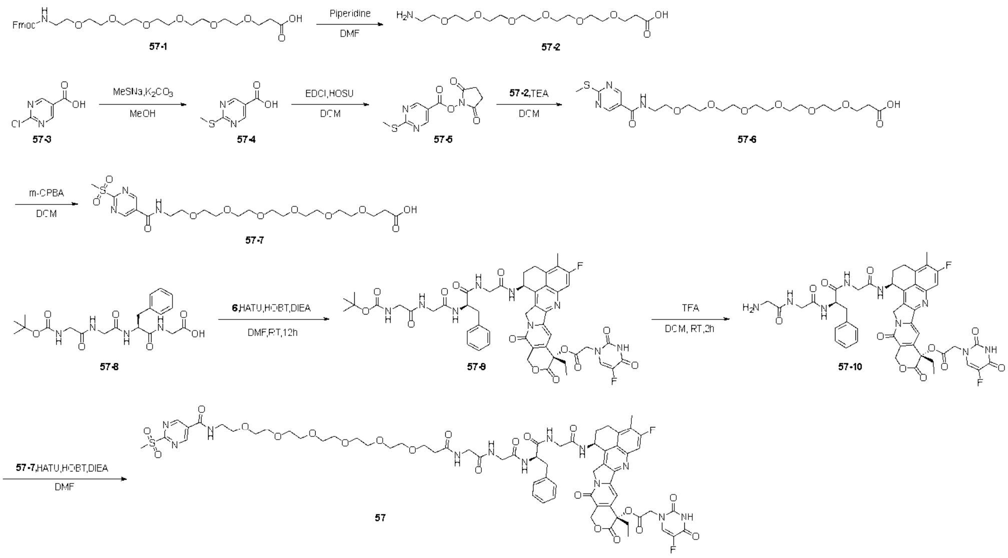


Synthesis of Intermediate 57-2:

5,8,11,14,17,20-Hexaoxa-2-azatricosanedioic acid 1-(9H-fluoren-9-ylmethyl) ester (1.4 g, 1.73 mmol) was dissolved in DMF (10 mL). Piperidine (2.5 mL) was added, and the reaction mixture was stirred at room temperature for 3 hours. LC-MS monitoring indicated complete consumption of starting material 57-1. Diethyl ether was added to the reaction mixture at 0°C, resulting in the precipitation of a white solid. After standing at room temperature for 2 hours, the reaction mixture was filtered to obtain a white solid 57-2 (800 mg, 95% ).

Synthesis of Intermediate 57-4:

2-Chloropyrimidine-5-carboxylic acid (1g, 6.3mmol) and sodium methanethiolate (MeSNa, 860 mg, 12.6 mmol) were dissolved in MeOH (200 mL). $K_2CO_3$ (2.7 g, 12.6 mmol) was added, and the reaction proceeded at room temperature overnight. The reaction mixture was concentrated. Water (50 mL) was added, and the reaction mixture was extracted with ethyl acetate (EA, 3 × 30 mL). 2M HCl was added dropwise to the aqueous phase, resulting in the precipitation of a solid. After standing for 1 hour, the reaction mixture was filtered and lyophilized to obtain a white solid 57-4 (800 mg, 75% ). MS (ESI): m/z found $[M+H]^+$ = 171.2.

Synthesis of Intermediate 57-5:

The compound 57-4 (150 mg, 0.881 mmol) and N-hydroxysuccinimide (NHS, 107.2 mg, 0.925 mmol) were dissolved in DCM (5mL). 1-(3-Dimethylaminopropyl)-3-ethylcarbodiimide hydrochloride (EDC·HCl, 177.3 mg, 0.925 mmol) was added, and the reaction mixture was stirred at room temperature for 3 hours. LC-MS monitoring indicated complete consumption of starting material 57-4. The reaction mixture was used directly in the next step. MS (ESI): m/z found $[M+H]^+$ = 268.2.

Synthesis of Intermediate 57-6:

The compound 57-2 (311.2 mg, 0.881 mmol) was added to the reaction mixture from the previous step (57-5). Triethylamine (122 μL, 0.881 mmol) was added, and the reaction proceeded at room temperature for 5 hours. Purification by flash column chromatography (C-18 reverse phase column, 26% ACN in $H_2O$) was performed to obtain a white solid 57-6 (400 mg, 89% ). MS (ESI): m/z found $[M+H]^+$ = 506.2.

Synthesis of Intermediate 57-7:

The compound 57-6 (400 mg, 0.79 mmol) was dissolved in DCM (5mL). meta-Chloroperoxybenzoic acid (85%, 402 mg, 1.97 mmol) was added, and the reaction proceeded at room temperature for 12 hours. Purification by flash column chromatography (C-18 reverse phase column, 35% ACN in $H_2O$) and concentration were performed to obtain a colorless oily liquid 57-7 (380 mg, 89% ). MS (ESI): m/z found $[M+H]^+$ = 538.2.

Synthesis of Intermediate 57-9:

The compound 57-8 (14.4 mg, 0.032 mmol) was dissolved in DMF (3 mL). 2-(7-Azabenzotriazol-1-yl)-N,N,N',N'-tetramethyluronium hexafluorophosphate (14 mg, 0.038 mmol) and 1-hydroxybenzotriazole (5 mg, 0.038 mmol) were added, and the reaction mixture was stirred at room temperature for 20 minutes. N,N-Diisopropylethylamine (1 μL, 0.064 mmol) and the compound 6 (20 mg, 0.032 mmol) were added, and the reaction mixture was stirred at room temperature for 12 hours. Water (30 mL) was added to the reaction mixture, which was then extracted with DCM (3 × 15 mL), and the organic phases were combined. The combined organic phase was dried over anhydrous sodium sulfate and concentrated. Purification by column chromatography (10% MeOH in DCM) was performed to obtain a white solid 57-9 (30 mg, 90% ). MS (ESI): m/z found $[M+H]^+$ = 1024.55.

Synthesis of Intermediate 57-10:

The compound 57-9 (30mg, 0.029 mmol) was dissolved in DCM (5mL). Trifluoroacetic acid (1 mL) was added, and the reaction mixture was stirred at room temperature for 2 hours. The reaction mixture was concentrated and dissolved in MeOH (2 mL) and purified by flash column chromatography (C-18 reverse phase column, 60% ACN in $H_2O$), and then concentrated to obtain a white solid 57-10 (12 mg, 44% ). MS (ESI): m/z found $[M+H]^+$ = 924.2.

Synthesis of Compound 57:

The compound 57-7 (5mg, 0.0053 mmol) was dissolved in DMF (3 mL). 2-(7-Azabenzotriazol-1-yl)-N,N,N',N'-tetramethyluronium hexafluorophosphate (2.5 mg, 0.0063 mmol) was added, and the reaction mixture was stirred at room temperature for 20 minutes. The compound 57-10 (3 mg, 0.0053 mmol) and N,N-diisopropylethylamine (1.35 μL, 0.0076 mmol) were added, and the reaction mixture was stirred at room temperature for 3 hours. The reaction mixture was purified by preparative HPLC and concentrated to obtain a pale yellow solid (2 mg, 26%). MS (ESI): m/z found $[M+H]^+$ = 1443.8.

Example **58:**

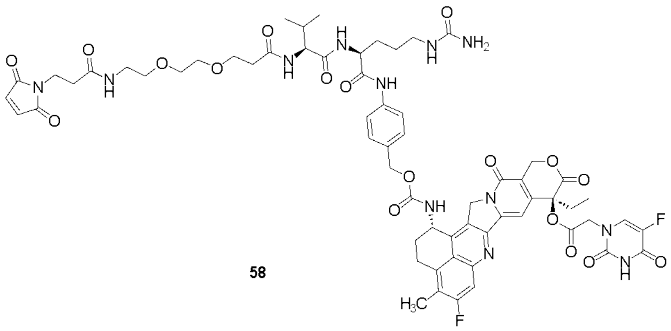

58

Synthetic route:

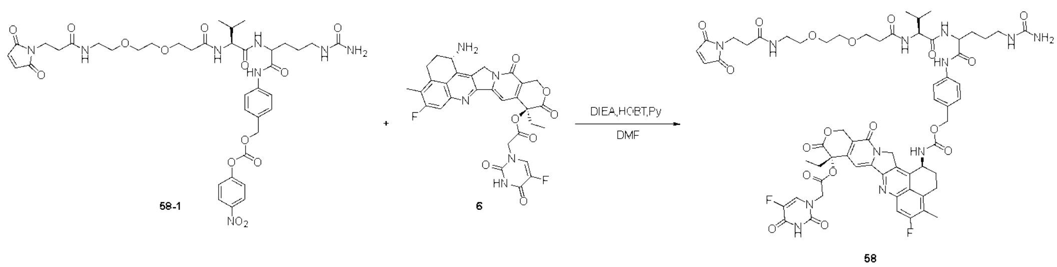


58-1

6

58

Synthesis:

A compound 58-1 (5 mg, 0.0058 mmol) was dissolved in DMF (3 mL). The compound 6 (3.5 mg, 0.0058 mmol), DIEA (1 μL, 0.0058 mmol), HOBT (1.5 mg, 0.0117 mmol), and pyridine (2.31 μL, 0.029 mmol) were added. The reaction mixture was stirred at room temperature for 12 hours. Purification by preparative HPLC (ACN in $H_2O$ with 0.1‰ TFA, 5-55% gradient over 30 minutes) was performed to obtain a yellow solid (2.1 mg, 27% ). MS (ESI): m/z found $[M+H]^+$ = 1321.7.

Example **59:**

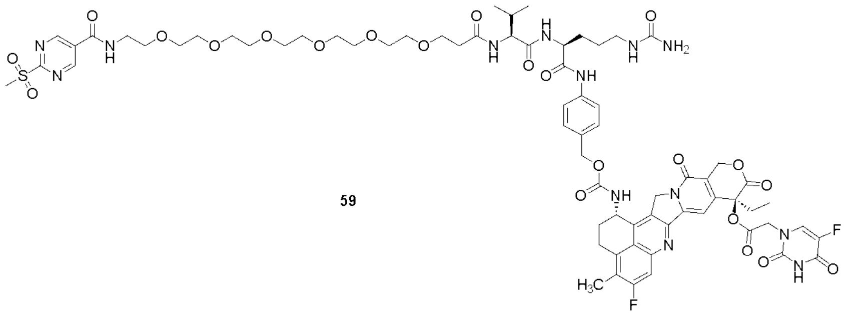

59

Synthetic route:

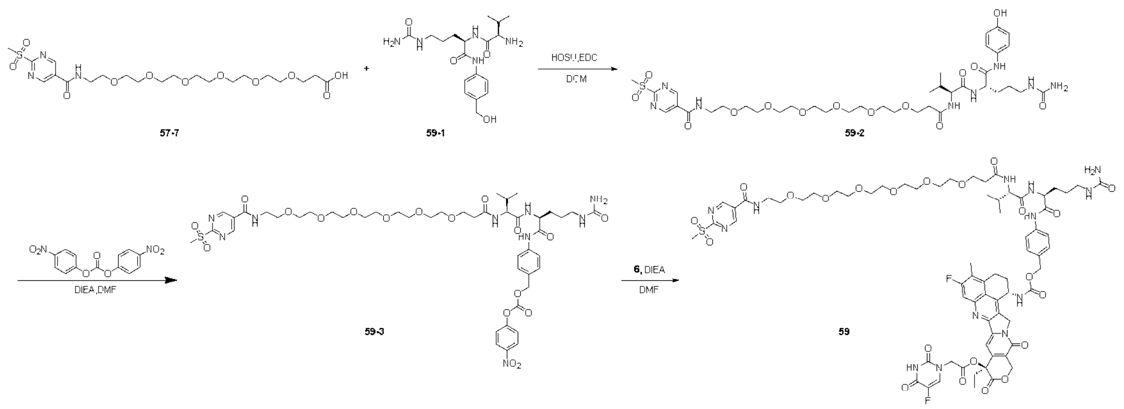


57-7 59-1 59-2 59-3 59

Synthesis of Intermediate 59-2:

The compound 57-7 (40 mg, 0.074 mmol) and N-Hydroxysuccinimide (NHS, 9.5 mg, 0.082 mmol) were dissolved in DCM (5 mL). 1-Ethyl-3-(3-dimethylaminopropyl)carbodiimide hydrochloride (16 mg, 0.082 mmol) was added, and the reaction mixture was stirred at room temperature for 3 hours. LC-MS monitoring indicated completion of the reaction. VAL-CIT-PAB (59-1, 28 mg, 0.082 mmol) and triethylamine (11.3 μL, 0.082 mmol) were added, and the reaction mixture was stirred at room temperature for an additional 2 hours. The reaction mixture was concentrated. The residue was dissolved in MeOH (5 mL) and purified by flash column chromatography (78% ACN in $H_2O$). MS (ESI): m/z found $[M+H]^+$ = 899.2.

Synthesis of Intermediate 59-3:

The compound 59-2 (52 mg, 0.058 mmol) and bis(4-nitrophenyl) carbonate (35 mg, 0.116 mmol) were dissolved in DMF (2.5 mL). N,N-Diisopropylethylamine (14.3 μL, 0.087 mmol) was added, and the reaction proceeded at room temperature for 18 hours. Water (50 mL) was added to the reaction mixture, which was then extracted with DCM (3 × 20 mL), and the organic phases were combined. The combined organic phase was washed with water for three times,, and the organic phases were combined. Purification by flash column chromatography (5% MeOH in DCM) was performed to

obtain a white solid (20 mg, 32%). MS (ESI): m/z found $[M+H]^+$ = 1064.3.

Synthesis of Compound 59:

The compound 59-3 (20 mg, 0.019 mmol) was dissolved in DMF (3 mL). The compound 6 (11 mg, 0.019 mmol) and N,N-diisopropylethylamine (12 μL, 0.075 mmol) were added. The reaction mixture was stirred at room temperature for 3 hours. The reaction mixture was purified by flash column chromatography (8% MeOH in DCM) to obtain a white solid crude product (5 mg). Further purification by preparative HPLC (ACN in $H_2O$ with 0.1‰TFA, 5-55% gradient over 30 minutes) was perfomed to obtain a pale yellow solid (0.84 mg, 3%). MS (ESI): m/z found $[M/2+H]^+$ = 765.15.

Example **60:**

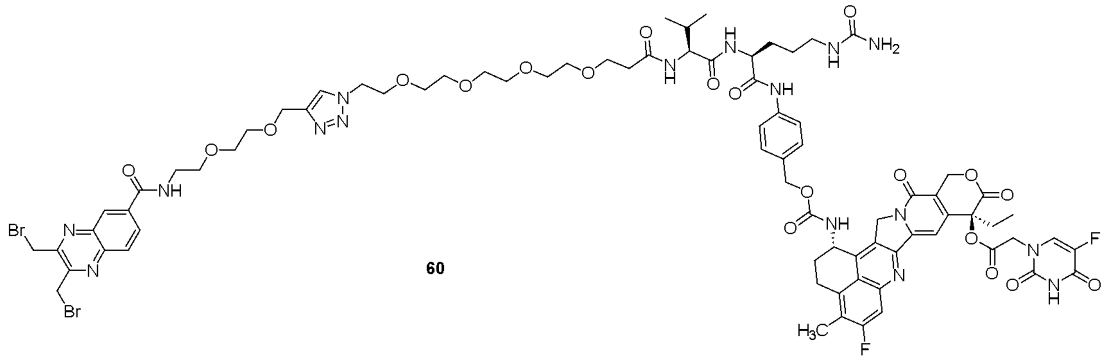

60

Synthetic route:

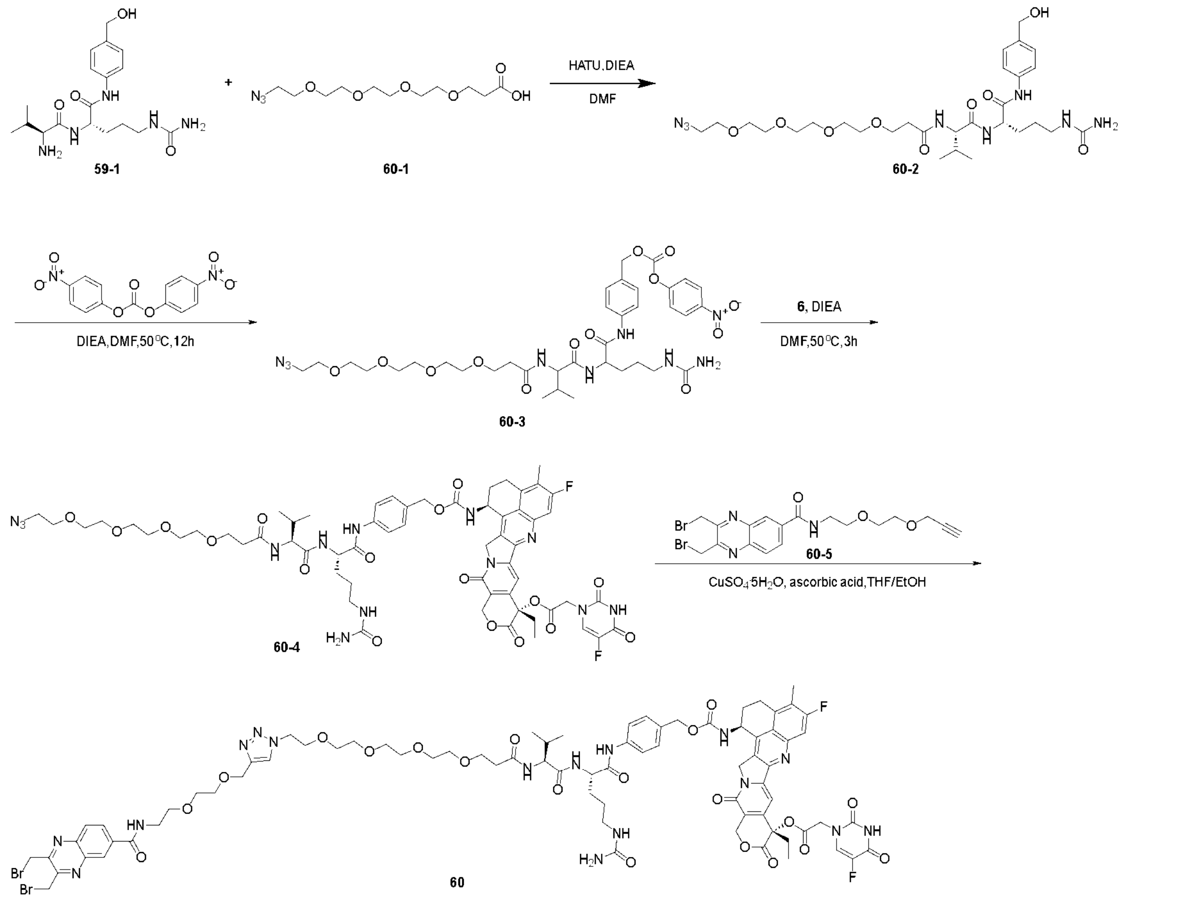


59-1 60-1 60-2 60-3 60-4 60

Synthesis of Intermediate 60-2:

15-Azido-4,7,10,13-tetraoxapentadecanoic acid (76 mg, 0.26mmol) was dissolved in DMF (5mL). 2-(7-Azabenzotriazol-1-yl)-N,N,N',N'-tetramethyluronium hexafluorophosphate (110 mg, 0.34 mmol) was added, and the reaction mixture was stirred at room temperature for 20 minutes. VAL-CIT-PAB (100 mg, 0.26 mmol) and DIEA (54 μL, 0.32 mmol) were added, and the reaction mixture was stirred at room temperature for 12 hours. The reaction mixture was purified by flash column chromatography (C-18 reverse phase column, 75% ACN in $H_2O$) to obtain a white solid (120 mg, 75% ). MS (ESI): m/z found $[M+H]^+$ = 653.4.

Synthesis of Intermediate 60-3:

The compound 60-2 (60 mg, 0.092 mmol) and bis(4-nitrophenyl) carbonate (97 mg, 0.322 mmol) were dissolved in DMF (2.5 mL). N,N-Diisopropylethylamine (35 μL, 0.211 mmol) was added. The reaction mixture was heated to 50°C and the reaction proceeded for 12 hours. Water (30 mL) was added to the reaction mixture, which was then extracted with DCM (3 × 10 mL), and the organic phases were combined. The combined organic phase was dried over anhydrous sodium sulfate and concentrated. Purification by column chromatography (8% MeOH in DCM) was performed to obtain a white solid product (40 mg, 75.3% ). MS (ESI): m/z found $[M+H]^+$ = 818.4.

Synthesis of Intermediate 60-4:

The compound 60-3 (20 mg, 0.024 mmol) was dissolved in DMF (2.5 mL). The compound 6 (15 mg, 0.024 mmol) and N,N-diisopropylethylamine (16 μL, 0.096 mmol) were added. The reaction proceeded at room temperature for 3 hours. Purification by preparative HPLC (ACN in $H_2O$ with 0.1‰ TFA, 5-55% gradient over 30 minutes) was performed to obtain a white solid product (5 mg, 16% ). MS (ESI): m/z found $[M+H]^+$ = 1284.55.

Synthesis of Compound 60:

The compounds 60-4 (3.8 mg, 0.0076 mmol) and 60-5 (5 mg, 0.0038 mmol) were dissolved in a mixture of THF

(0.5 mL) and EtOH (1.5 mL). Copper(II) sulfate pentahydrate (1 mg, 0.0038 mmol) and L-ascorbic acid (0.7 mg, 0.0038 mmol) were added. The reaction mixture was stirred at room temperature under a nitrogen atmosphere for 3 hours. Purification by preparative HPLC (ACN in $H_2O$ with 0.1‰ TFA, 5-55% gradient over 30 minutes) was perfomed to obtain a pale yellow solid (1.8 mg, 26% ). MS (ESI): m/z found $[M+H]^+$ = 1770.75.

Example 61:

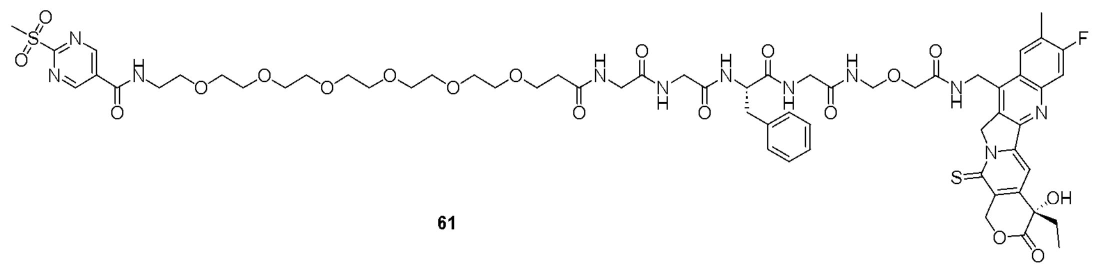

61

Synthetic route:

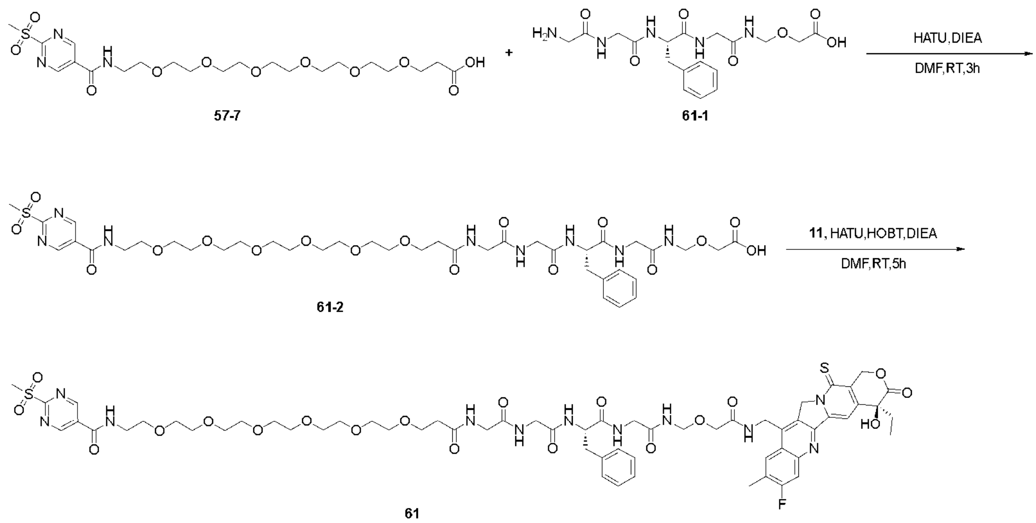


Synthesis of Intermediate 61-2:

The compound 57-7 (100 mg, 0.186 mmol) was dissolved in DMF (3 mL). 2-(7-Azabenzotriazol-1-yl)-N,N,N',N'-tetramethyluronium hexafluorophosphate (35 mg, 0.186 mmol) was added, and the reaction mixture was stirred at room temperature for 30 minutes. N,N-Diisopropylethylamine (38 μL, 0.459 mmol) and the compound 61-1 (80 mg, 0.186 mmol) were added. The reaction proceeded at room temperature for 5 hours. Purification by flash column chromatography (C-18 reverse phase column, 38% ACN in $H_2O$) and concentration were performed to obtain a white solid (90 mg, 51%). MS (ESI): m/z found $[M+Na]^+$ = 965.2.

Synthesis of Compound 61:

The compound 61-2 (5.2 mg, 0.0055 mmol) was dissolved in DMF (2 mL). 2-(7-Azabenzotriazol-1-yl)-N,N,N',N'-tetramethyluronium hexafluorophosphate (HATU, 2.1 mg, 0.0055 mmol) and 1-Hydroxybenzotriazole (0.7 mg, 0.0055 mmol) were added, and the reaction mixture was stirred at room temperature for 20 minutes. The compound 11 (2.34 mg, 0.0055 mmol) and N,N-diisopropylethylamine (1.82 μL, 0.011 mmol) were added. The reaction mixture was stirred at room temperature for 5 hours. Purification by preparative HPLC (5-50% ACN in $H_2O$ with 0.1% TFA over 30 minutes) was perfomed to obtain a yellow solid (2.4 mg, 32% ). MS (ESI): m/z found $[M+H]^+$ = 1350.7.

Example 62:

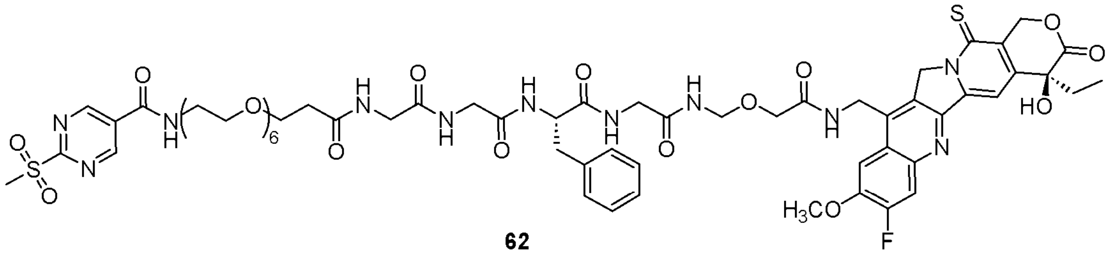

62

The compound 61-2 (10 mg, 0.011 mmol) was dissolved in DMF (1.5 mL). HATU (5 mg, 0.013 mmol), DIEA (2.2 μL, 0.013 mmol), and the compound 12 (5 mg, 0.011 mmol) were added. The reaction proceeded at room temperature for 3 hours. After LC-MS monitoring indicated completion of the reaction, the reaction mixture was purified by preparative HPLC and lyophilized to obtain a yellow solid product (0.50 mg, 3.4%). MS (ESI): m/z found $[M/2+H]^+$ = 684.35.

Example 63:

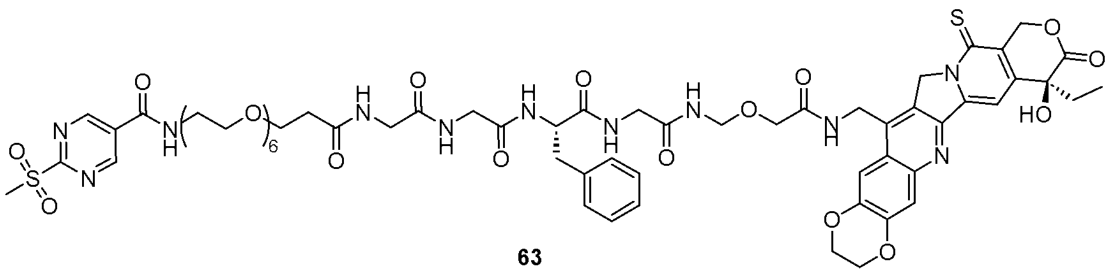

63

In an ice bath, the compound 61-2 (11 mg, 0.012 mmol) was dissolved in anhydrous N,N-dimethylformamide (5 mL). 1-(3-Dimethylaminopropyl)-3-ethylcarbodiimide hydrochloride (4.1 mg, 0.022 mmol), the compound 13 (5 mg, 0.012 mmol), and 2,4,6-trimethylpyridine (8.6 μL, 0.065 mmol) were added. The reaction proceeded at room temperature for 2 hours. LC-MS monitoring indicated completion of the reaction. The reaction mixture was purified by prep-HPLC to obtain a compound 63 (0.7 mg, 4%). MS (ESI): m/z found $[M+H]^+$ = 1376.80.

Example 64:

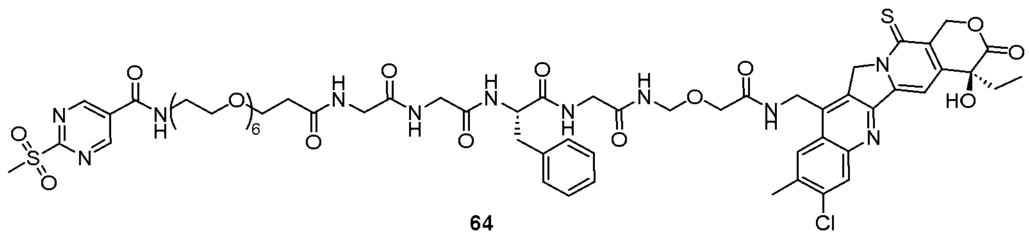

64

Synthesis of Compound 64:

In an ice bath, the compound 61-2 (11 mg, 0.0113 mmol) was dissolved in anhydrous N,N-dimethylformamide (5 mL). 1-(3-Dimethylaminopropyl)-3-ethylcarbodiimide hydrochloride (4.0 mg, 0.0203 mmol), the compound 14 (5 mg, 0.0113 mmol), and 2,4,6-trimethylpyridine (8.0 μL, 0.0610 mmol) were added. The reaction proceeded at room temperature for 2 hours. LCMS monitoring indicated completion of the reaction. The reaction mixture was purified by prep-HPLC to obtain a compound 64 (5.85 mg, 4% yield). MS (ESI): m/z found $[M+H]^+$ = 1366.70.

Example **65:**

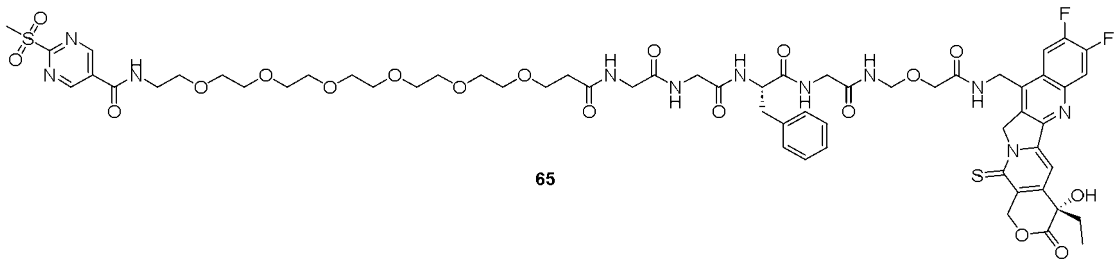

**65**

Synthesis of Compound 65:

In an ice bath, the compound 61-2 (11 mg, 0.0116 mmol) was dissolved in anhydrous N,N-dimethylformamide (5 mL). 1-(3-Dimethylaminopropyl)-3-ethylcarbodiimide hydrochloride (4.0 mg, 0.0209 mmol), the compound 15 (5.0 mg, 0.0116 mmol), and 2,4,6-trimethylpyridine (8.3 μL, 0.0610 mmol) were added. The reaction proceeded at room temperature for 2 hours. LCMS monitoring indicated completion of the reaction. The reaction mixture was purified by prep-HPLC to obtain a compound 65 (3.76 mg, yield 24% ). MS (ESI): m/z found $[M+H]^+$ = 1354.98.

Example 66:

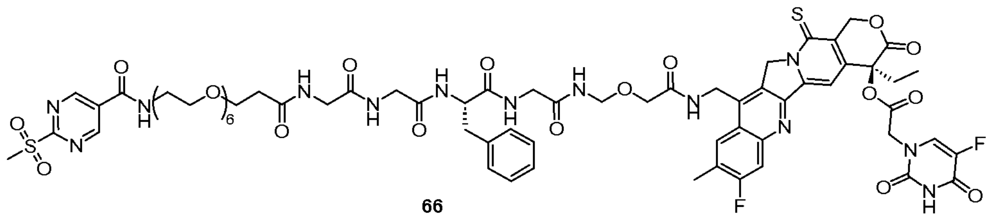

**66**

Synthesis of Compound 66:

In an ice bath, the compound 61-2 (13 mg, 0.0134 mmol) was dissolved in anhydrous N,N-dimethylformamide (5 mL). 1-(3-Dimethylaminopropyl)-3-ethylcarbodiimide hydrochloride (4.6 mg, 0.0134 mmol), the compound 24 (8 mg, 0.0134 mmol), and 2,4,6-trimethylpyridine (9.6 μL, 0.0724 mmol) were added. The reaction proceeded at room temperature for 2 hours. LCMS monitoring indicated completion of the reaction. The reaction mixture was purified by prep-HPLC to obtain a compound 66 (0.9 mg, yield 4%). MS (ESI): m/z found $[M/2+H]^+$ = 761.25.

Example 67:

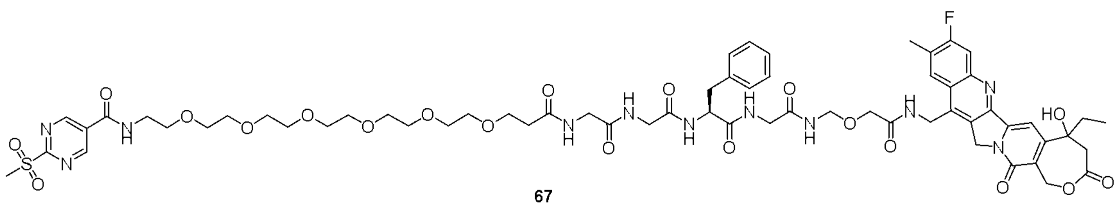

**67**

The compound 61-2 (20 mg, 0.021 mmol) was dissolved in DMF (3 mL). 2-(7-Azabenzotriazol-1-yl)-N,N,N',N'-tetramethyluronium hexafluorophosphate (11.3 mg, 0.030 mmol) and 1-hydroxybenzotriazole (4.02 mg, 0.030 mmol) were added. The reaction mixture was stirred at room temperature for 20 minutes. The 47 (9 mg, 0.021 mmol) and N,N-diisopropylethylamine (6.7 μL) were added. Purification by preparative HPLC (ACN in $H_2O$ with 0.1% HCOOH, 5-55%

gradient over 30 minutes) was performed to obtain a white solid product (2 mg, 7% ). MS (ESI): m/z found $[M+H]^+$ = 1348.76.

Example **68:**

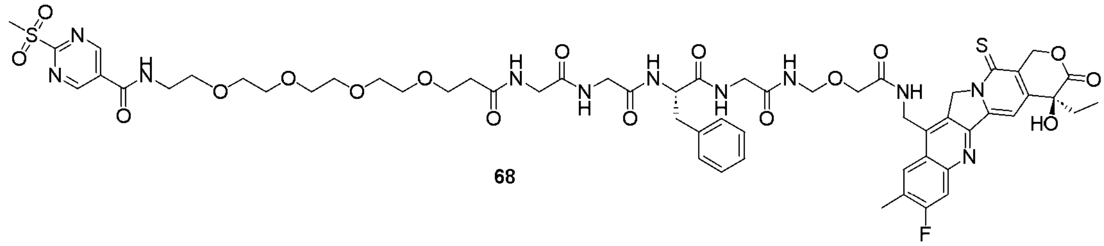

68

Synthetic route:

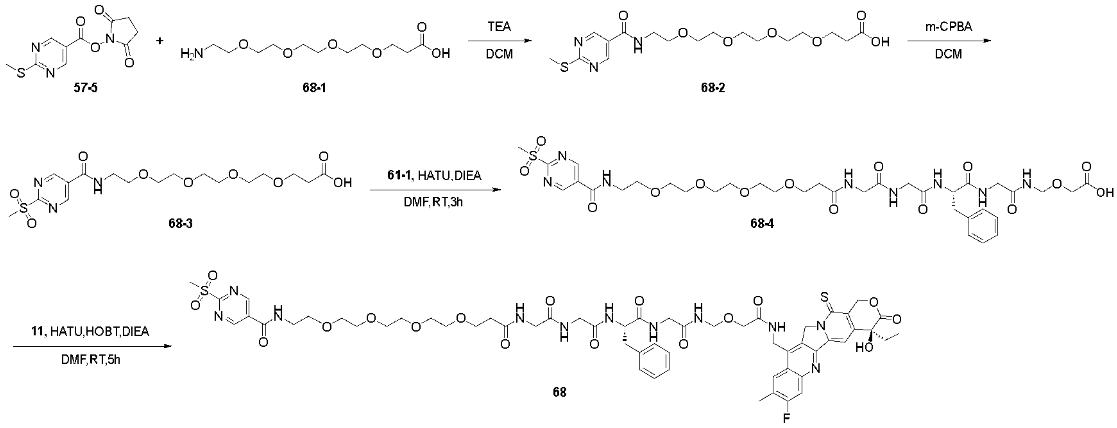


57-5 68-1 68-2 68-3 68-4 68

Synthesis of Intermediate 68-2:

A compound 68-1 (312 mg, 1.18 mmol) was added to the reaction mixture from the previous step (containing 57-5, 200 mg, 1.18 mmol). Triethylamine (163 μL, 1.18 mmol) was added, and the reaction proceeded at room temperature for 5 hours. Purification by flash column chromatography (C-18 reverse phase column, 26% ACN in $H_2O$) was performed to obtain a colorless oily compound (320 mg, 65%). MS (ESI): m/z found $[M+H]^+$ = 418.2.

Synthesis of Intermediate 68-3:

The compound 68-2 (320 mg, 0.767 mmol) was dissolved in DCM (5 mL). meta-Chloroperoxybenzoic acid (85%, 390 mg, 2.26 mmol) was added, and the reaction proceeded at room temperature for 12 hours. Purification by flash column chromatography (C-18 reverse phase column, 35% ACN in $H_2O$) and concentration were performed to obtain a colorless oily liquid (180 mg, 52.2% ). MS (ESI): m/z found $[M+H]^+$ = 450.25.

Synthesis of Intermediate 68-4:

The compound 68-3 (50 mg) was dissolved in DMF (3 mL). 2-(7-Azabenzotriazol-1-yl)-N,N,N',N'-tetramethyluronium hexafluorophosphate (42 mg) was added, and the reaction mixture was stirred at room temperature for 30 minutes. N,N-Diisopropylethylamine (46 μL) and the compound 61-1 (48 mg) were added. The reaction proceeded at room temperature for 5 hours. Purification by reverse phase column chromatography (38% ACN in $H_2O$) and concentration were performed to obtain a white solid (50 mg). MS (ESI): m/z found $[M-H]^-$ = 853.2.

Synthesis of Compound 68:

In an ice bath, the compound 68-3 (10.1 mg, 0.0118 mmol) was dissolved in anhydrous N,N-dimethylformamide (5 mL). O-(7-Azabenzotriazol-1-yl)-N,N,N',N'-tetramethyluronium hexafluorophosphate (7.8 mg, 0.0135 mmol) and 1-hydroxybenzotriazole (1.9 mg, 0.0135 mmol) were added. The mixture proceeded for 10 minutes. The compound 11 (5 mg, 0.0118 mmol) and N,N-diisopropylethylamine (3.9 μL, 0.0224 mmol) were then added. The reaction proceeded at room temperature for 2 hours. LCMS monitoring indicated completion of the reaction. The reaction mixture was purified by prep-HPLC to obtain a compound (2.39 mg, 16%). MS (ESI): m/z found $[M+H]^+$ = 1262.70.

Example 69:

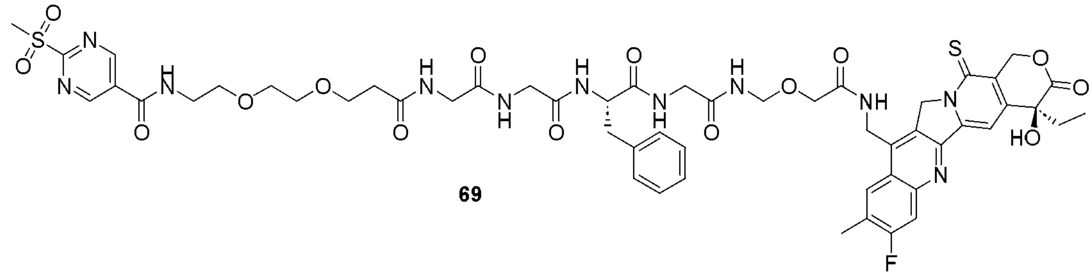

69

Synthetic route:

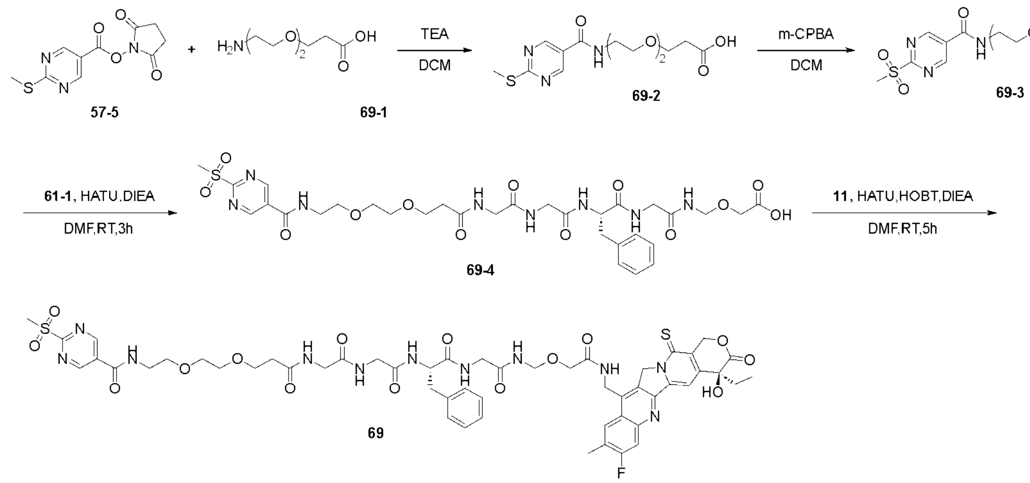


Synthesis of Intermediate 69-2:

The compound 69-1 (114.5 mg, 0.646 mmol) was added to the reaction mixture from the previous step (containing 57-5). Triethylamine (90 μL, 0.646 mmol) was added, and the reaction proceeded at room temperature for 5 hours. Purification by flash column chromatography (C-18 reverse phase column, 28% ACN in $H_2O$) was performed to obtain a colorless oily compound (100 mg, 58% ). MS (ESI): m/z found $[M+H]^+$ = 268.0.

Synthesis of Intermediate 69-3:

The compound 69-2 (100 mg, 0.375 mmol) was dissolved in DCM (3 mL). meta-Chloroperoxybenzoic acid (85%, 154 mg, 0.936 mmol) was added, and the reaction proceeded at room temperature for 12 hours. Purification by flash column chromatography (C-18 reverse phase column, 18% ACN in $H_2O$) and concentration were performed to obtain a colorless oily liquid (35 mg, 25.8% ). MS (ESI): m/z found $[M+H]^+$ = 362.0.

Synthesis of Intermediate 69-4:

The compound 69-3 (35 mg) was dissolved in DMF (3 mL). 2-(7-Azabenzotriazol-1-yl)-N,N,N',N'-tetramethyluronium hexafluorophosphate (36 mg) was added, and the reaction mixture was stirred at room temperature for 30 minutes. N,N-Diisopropylethylamine (40 μL) and the 61-1 (41 mg) were added. The reaction proceeded at room temperature for 5 hours. Purification by HPLC (38% ACN in $H_2O$) and lyophilization were performed to obtain the product. MS (ESI): m/z found $[M-H]^-$ = 765.3.

Synthesis of Compound 69:

In an ice bath, the compound 69-4 (9.1 mg, 0.0118 mmol) was dissolved in anhydrous N,N-dimethylformamide (5 mL). O-(7-Azabenzotriazol-1-yl)-N,N,N',N'-tetramethyluronium hexafluorophosphate (7.8 mg, 0.0135 mmol) and 1-hydroxybenzotriazole (1.9 mg, 0.0135 mmol) were added. The reaction proceeded for 10 minutes. The compound 11 (5 mg, 0.0118 mmol) and N,N-diisopropylethylamine (DIEA, 3.9 μL, 0.0224 mmol) were then added. The reaction proceeded at room temperature for 2 hours. LCMS monitoring indicated completion of the reaction. The reaction mixture was purified by prep-HPLC to obtain a compound (5.26 mg, 37% ). MS (ESI): m/z found $[M+H]^+$ = 1174.83.

Example 70:

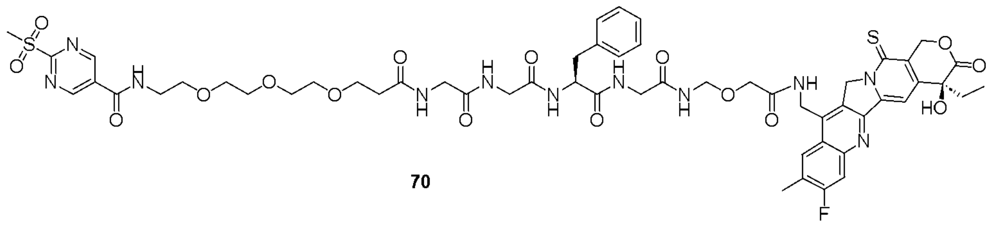

**70**

Following the same procedure as described in Example 69, but with the corresponding starting materials replaced, the title compound was obtained as a yellow solid (5.26 mg, 37% yield ). LC-MS (ESI): m/z found $[M+H]^+$ = 1218.75.

Example 71:

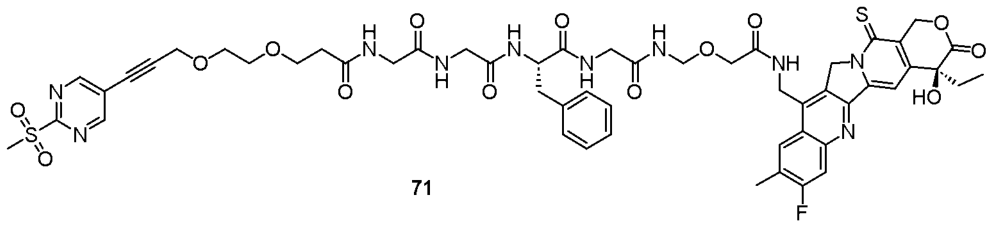

**71**

Synthetic route:

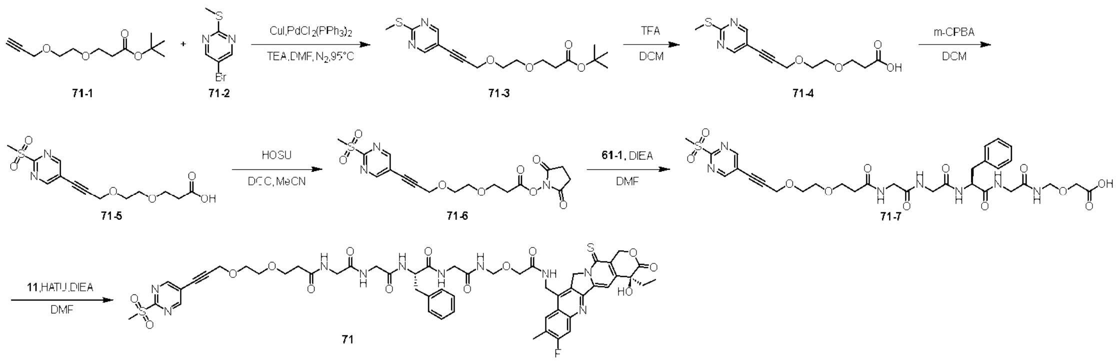


Synthesis of Intermediate 71-3:

Compounds 71-1 (200 mg, 0.876 mmol) and 71-2 (180 mg, 0.876 mmol) were dissolved in DMF. Triethylamine (0.66 mL), copper(I) iodide (CuI, 16.8 mg, 0.088 mmol), and bis(triphenylphosphine)palladium(II) dichloride ($PdCl_2(PPh_3)_2$, 61.8 mg, 0.088 mmol) were added sequentially. Under a nitrogen atmosphere, the reaction mixture was heated to 95°C and the reaction proceeded for 6 hours. LC-MS monitoring indicated completion of the reaction. The reaction mixture was extracted with ethyl acetate/water. The organic phase was washed with saturated NaCl solution, dried over anhydrous $Na_2SO_4$, filtered under suction, and spin-dried. Purification by normal-phase column chromatography (8% EA in PE) and spin-drying were performed to obtain a yellow solid (205 mg, 66.4% ). LC-MS (ESI): m/z found $[M+H]^+$ = 353.0.

Synthesis of Intermediate 71-4:

The compound 71-3 (91 mg, 0.258 mmol) was dissolved in DCM (3 mL). Trifluoroacetic acid (TFA, 1 mL) was added dropwise, and the reaction proceeded at room temperature for 1 hour. LC-MS monitoring indicated completion of the reaction. The solvent was removed through distilling under reduced pressure to obtain a yellow solid product (90.6 mg, 100%). LC-MS (ESI): m/z found $[M+H]^+$ = 297.0.

Synthesis of Intermediate 71-5:

The compound 71-4 (90.6 mg, 0.258 mmol) was dissolved in DCM (5 mL). meta-Chloroperoxybenzoic acid (111.4 mg, 0.645 mmol) was added, and the reaction proceeded at room temperature for 12 hours. Purification by flash column chromatography (C-18 reverse phase column, 16% ACN in $H_2O$) and concentration were performed to obtain a white solid (49 mg, 48.8% ). LC-MS (ESI): m/z found $[M+H]^+$ = 329.0.

Synthesis of Intermediate 71-6:

The compound 71-5 (25 mg, 0.076 mmol) was dissolved in acetonitrile (MeCN). N-Hydroxysuccinimide (HOSu) and N,N'-Dicyclohexylcarbodiimide (DCC) were added at 0°C. The reaction mixture was warmed to room temperature and the reaction proceeded for 2 hours. LC-MS monitoring indicated completion of the reaction. The white insoluble solid was removed by suction filtration . The filtrate was concentrated under reduced pressure to obtain a white solid product (32.3 mg, 99.9% ). LC-MS (ESI): m/z found $[M+H]^+$ = 426.0.

Synthesis of Intermediate 71-7:

The compound 71-6 (32.3 mg, 0.076 mmol) was dissolved in DMF (2 mL). The compound 61-1 (32.2 mg, 0.076 mmol) and N,N-Diisopropylethylamine (25 $\mu$L, 0.152 mmol) were added sequentially. The reaction proceeded at room temperature for 2 hours. After completion, the reaction mixture was purified by reverse-phase column chromatography and lyophilized to obtain the product (49.2 mg, 8.2% ). LC-MS (ESI): m/z found $[M-H]^-$ = 732.25.

Synthesis of Compound 71:

In an ice bath, the compound 71-7 (8.7 mg, 0.0118 mmol) was dissolved in anhydrous N,N-dimethylformamide (5 mL). O-(7-Azabenzotriazol-1-yl)-N,N,N',N'-tetramethyluronium hexafluorophosphate (7.8 mg, 0.0135 mmol) and 1-hydroxybenzotriazole (1.9 mg, 0.0135 mmol) were added. The reaction proceeded for 10 minutes. The compound 11 (5 mg, 0.0118 mmol) and N,N-diisopropylethylamine (3.9 $\mu$L, 0.0224 mmol) were then added. The reaction proceeded at

room temperature for 2 hours. LCMS monitoring indicated completion of the reaction. The reaction mixture was purified by prep-HPLC to obtain a compound 71 (2.61 mg, 19% yield). MS (ESI): m/z found $[M+H]^+$ = 1141.60.

Example 72:

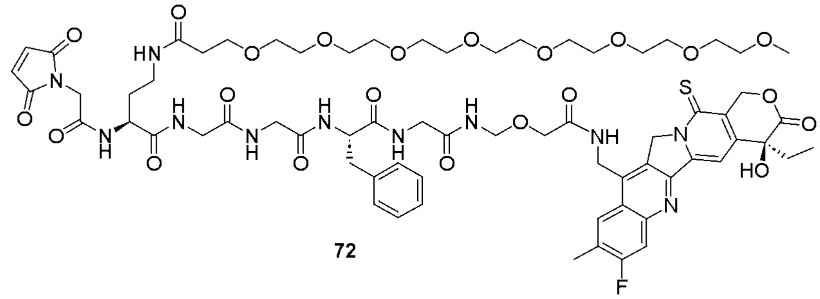

72

Synthetic route:

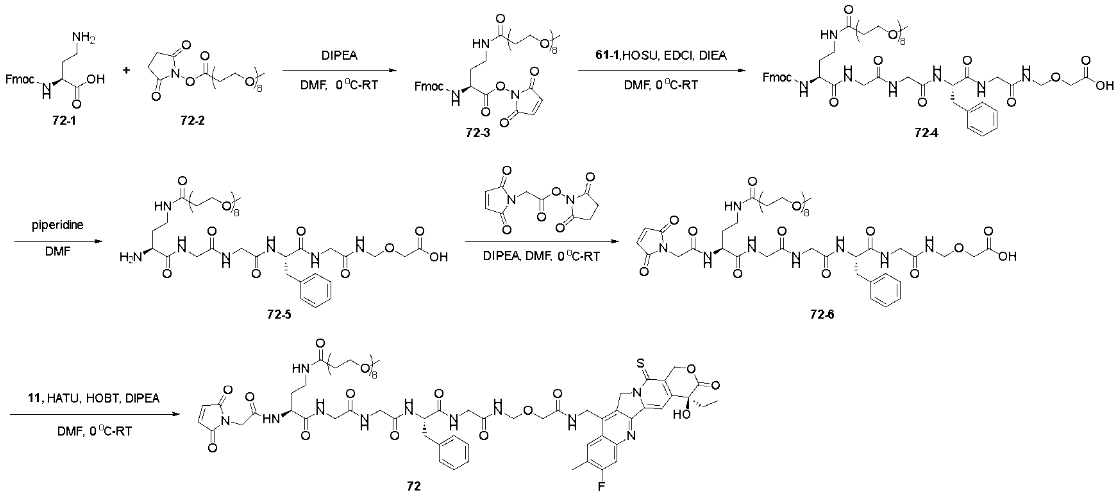


Synthesis of Intermediate 72-3:

In an ice bath, N-2-Fmoc-L-2,4-diaminobutanoic acid (72-1, 334 mg, 0.981 mmol) was dissolved in anhydrous N,N-dimethylformamide (5 mL). Methyl-PEG8-NHS ester (72-2, 500 mg, 0.981 mmol) and N,N-diisopropylethylamine (267 μL, 1.530 mmol) were added. The reaction proceeded at room temperature for 2 hours. LCMS monitoring indicated completion of the reaction. The reaction mixture was purified by reverse-phase column chromatography and lyophilized to obtain a compound (605 mg, 84% ). MS (ESI): m/z found $[M+H]^+$ = 735.50.

Synthesis of Intermediate 72-4:

In an ice bath, the compound 72-3 (100 mg, 0.136 mmol) was dissolved in anhydrous N,N-dimethylformamide (5 mL). N-Hydroxysuccinimide (20 mg, 0.163 mmol) and 1-(3-dimethylaminopropyl)-3-ethylcarbodiimide hydrochloride (32 mg, 0.163 mmol) were added. The reaction proceeded for 1.5 hours. The compound 61-1 (58 mg, 0.136 mmol) and N,N-diisopropylethylamine (24 μL, 0.136 mmol) were then added. The reaction proceeded at room temperature for 2 hours. LCMS monitoring indicated completion of the reaction. The reaction mixture was purified by reverse-phase column chromatography to obtain a compound (65 mg, 42% yield). MS (ESI): m/z found $[M+H]^+$ = 1140.80.

Synthesis of Intermediate 72-5:

At room temperature, the compound 72-4 (485 mg, 0.426 mmol) was dissolved in anhydrous N,N-dimethylformamide (5 mL). Piperidine (421 μL, 4.26 mmol) was added. The atmosphere was purged with nitrogen for three times, and the reaction proceeded at room temperature for 3 hours. LC-MS monitoring indicated completion of the reaction. The reaction mixture was purified by reverse-phase column chromatography to obtain a compound (1.5 mg, 99% yield). MS (ESI): m/z found $[M+H]^+$ = 918.70.

Synthesis of Intermediate 72-6:

In an ice bath, the compound 72-5 (37.8 mg, 0.0412 mmol) was dissolved in anhydrous N,N-dimethylformamide (3 mL). N-Succinimidyl 4-(maleimidobutanoate) (10.4 mg, 0.0412 mmol) and N,N-diisopropylethylamine (6.8 μL, 0.0412 mmol) were added. The reaction proceeded at room temperature for 3 hours. LCMS monitoring indicated completion of the reaction. The reaction mixture was purified by prep-HPLC to obtain a compound (17 mg, 39% yield). MS (ESI): m/z found $[M+H]^+$ = 735.50.

Synthesis of Compound 72:

In an ice bath, the compound 72-6 (12.4 mg, 0.0118 mmol) was dissolved in anhydrous N,N-dimethylformamide (5 mL). O-(7-Azabenzotriazol-1-yl)-N,N,N',N'-tetramethyluronium hexafluorophosphate (7.8 mg, 0.0135 mmol) and 1-hydroxybenzotriazole (1.9 mg, 0.0135 mmol) were added. The reaction proceeded for 10 minutes. The compound 11 (5 mg, 0.0118 mmol) and N,N-diisopropylethylamine (3.9 μL, 0.0224 mmol) were then added. The reaction proceeded at room temperature for 2 hours. LCMS monitoring indicated completion of the reaction. The reaction mixture was purified by prep-HPLC to obtain a compound 72 (2.41 mg, 14% yield ). LC-MS (ESI): m/z found $[M+H]^+$ = 1462.90.

Example **73:**

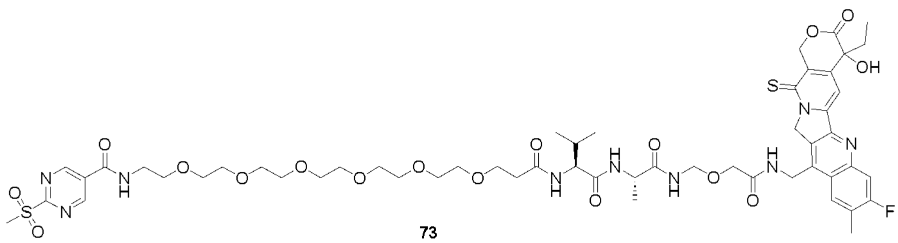

**73**

Synthetic route:

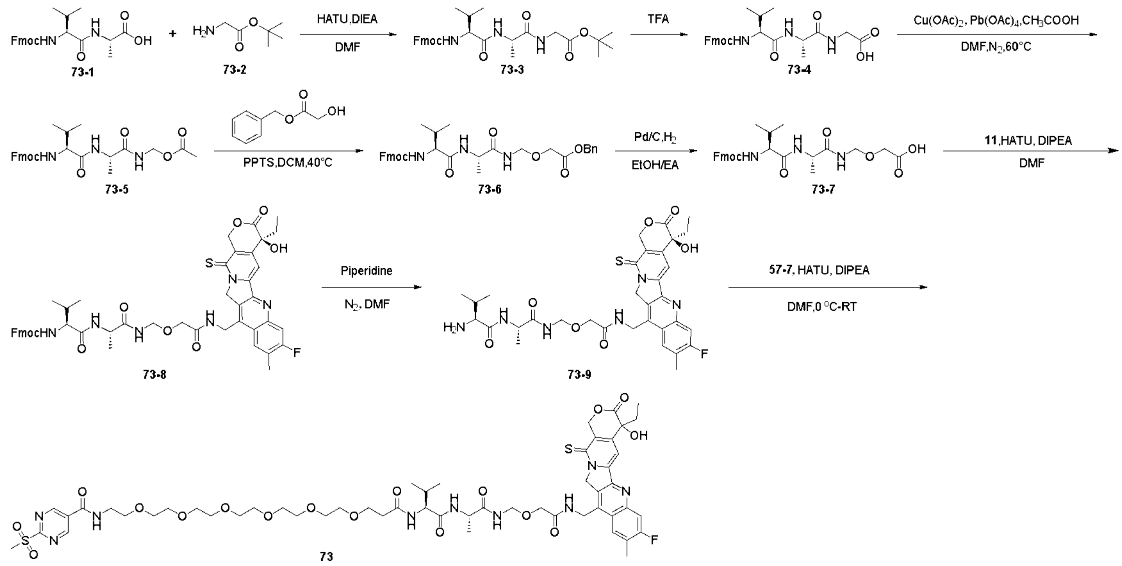


Synthesis of Intermediate 73-3:

A compound 73-1 (410 mg, 1 mmol) was dissolved in DMF. N,N-Diisopropylethylamine (0.188 mL, 1.14 mmol) and 2-(7-Azabenzotriazol-1-yl)-N,N,N',N'-tetramethyluronium hexafluorophosphate (0.433 g, 1.14 mmol) were added sequentially. The reaction proceeded at room temperature for 30 minutes. TLC detection indicated the formation of the active ester intermediate. A compound 73-2 (0.136 mL, 1 mmol) was added, and the reaction proceeded at room temperature for 2 hours. After completion, the reaction mixture was slurried with $H_2O$/DMF, and filtered under suction. The filter cake was lyophilized to obtain the product (501 mg, 95.8% ). LC-MS (ESI): m/z found $[M+H]^+$ = 524.45.

Synthesis of Intermediate 73-4:

The compound 73-3 (501 mg, 0.958 mmol) was dissolved in DCM (10 mL). Trifluoroacetic acid (TFA) was added dropwise at 0°C, and the reaction mixture was warmed to room temperature to proceed reaction. The reaction mixture was concentrated under reduced pressure. The residue was dissolved in a small amount of DMF and purified by reverse-phase column chromatography (39% MeCN in $H_2O$) to obtain a product. The product was Lyophilized to obtain a white solid (370 mg, 82.6% ). LC-MS: $[M+H]^+$ = 468.2.

Synthesis of Intermediate 73-5:

The compound 73-4 (100 mg, 0.214 mmol) was dissolved in DMF. Copper(II) acetate ($Cu(OAc)_2$, 2 mg), lead (IV) acetate ($Pb(OAc)_4$, 110 mg, 0.338 mmol), and acetic acid ($CH_3COOH$, 28.2 µL) were added sequentially. Under a nitrogen atmosphere, the reaction mixture was heated to 60°C, and the reaction proceeded for 4 hours. Some starting material remained. The reaction mixture was extracted with ethyl acetate/water. The organic phase was washed with saturated NaCl solution, dried over anhydrous $Na_2SO_4$, filtered under suction, and spin-dried. Slurry with petroleum ether and suction filtration were performed to obtain a product (65.8 mg, 63.9% ). LC-MS: $[M+H]^+$ = 482.2.

Synthesis of Intermediate 73-6:

The compound 73-5 (646 mg, 1.34 mmol) was dissolved in DCM (14 mL). Pyridinium p-toluenesulfonate (700 mg, 2.81 mmol) was added. The reaction mixture was heated to 40°C and refluxed overnight. Purification by normal-phase column chromatography and concentration under reduced pressure were performed to obtain a white solid (351 mg, 44.6% ). LC-MS: $[M+Na]^+$ = 610.2.

Synthesis of Intermediate 73-7:

At room temperature, the compound 73-6 (100 mg, 0.170 mmol) was dissolved in a mixture of anhydrous ethyl acetate (5 mL) and anhydrous ethanol (10 mL). A suitable amount of palladium on carbon (Pd/C) was added. The atmosphere was replaced with hydrogen gas ($H_2$) for three times, and the reaction proceeded at room temperature for 4 hours. LCMS monitoring indicated completion of the reaction. The reaction mixture was filtered through Celite. The filtrate

was concentrated and purified by reverse-phase column chromatography to obtain a compound 73-7 (25 mg, 30% yield ). MS (ESI): m/z found $[M+H]^+$ = 496.20.

Synthesis of Intermediate 73-8:

At room temperature, the compound 73-7 (5.9 mg, 0.0118 mmol) was dissolved in anhydrous N,N-dimethylformamide (5 mL). O-(7-Azabenzotriazol-1-yl)- N,N,N',N'-tetramethyluronium hexafluorophosphate (7.8 mg, 0.0135 mmol), the compound 11 (5 mg, 0.0118 mmol), and N,N-diisopropylethylamine (3.9 μL, 0.0224 mmol) were added. The reaction proceeded at room temperature for 2 hours. LCMS monitoring indicated completion of the reaction. The reaction mixture was purified by prep-HPLC to obtain a compound (2.3 mg, 22% yield ). LC-MS (ESI): m/z found $[M+H]^+$ = 905.55.

Synthesis of Intermediate 73-9:

At room temperature, the compound 73-8 (2.3 mg, 0.0025 mmol) was dissolved in anhydrous N,N-dimethylformamide (3 mL). Piperidine (2.5 μL, 0.025 mmol) was added. The reaction proceeded at room temperature for 1.5 hours. LC-MS monitoring indicated completion of the reaction. The reaction mixture was purified by prep-HPLC to obtain a compound 73-9 (1.5 mg, 87% yield). MS (ESI): m/z found $[M+H]^+$= 683.45.

Synthesis of Compound 73:

In an ice bath, the compound 73-9 (1.5 mg, 0.0022 mmol) was dissolved in anhydrous N,N-dimethylformamide (2 mL). O-(7-Azabenzotriazol-1-yl)- N,N,N',N'-tetramethyluronium hexafluorophosphate (1.0 mg, 0.0025 mmol), the compound 57-7 (1.4 mg, 0.0022 mmol), and N,N-diisopropylethylamine (DIEA, 0.8 μL, 0.0044 mmol) were added. The reaction proceeded at room temperature for 2 hours. LCMS monitoring indicated completion of the reaction. The reaction mixture was purified by prep-HPLC to obtain a compound(0.95 mg, yield 36% ). MS (ESI): m/z found $[M+H]^+$ = 1096.80.

Example 74:

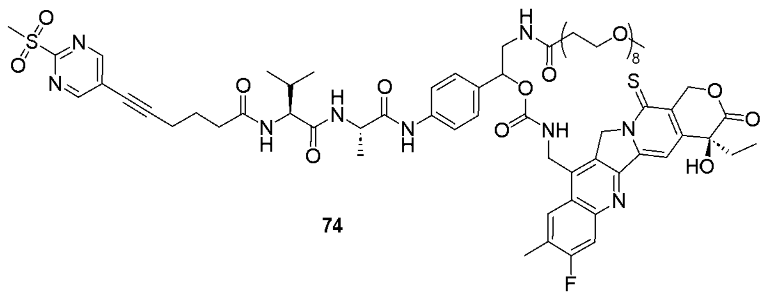

74

Synthetic route:

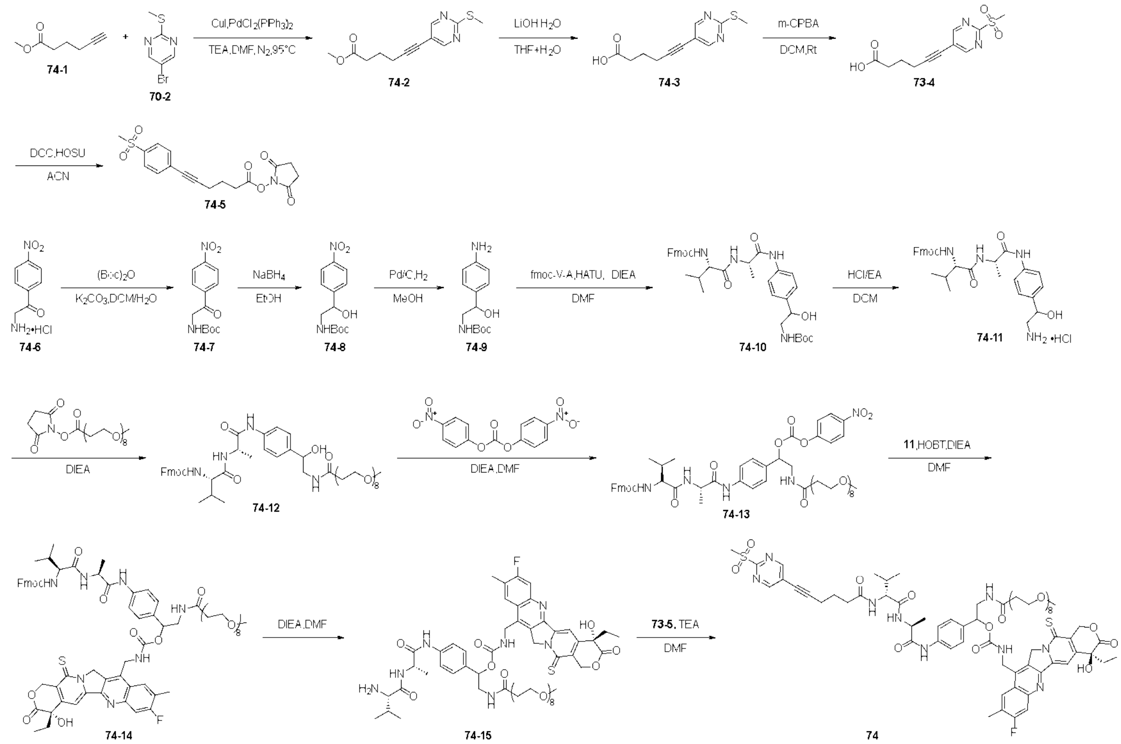


74-1 70-2 74-2 74-3 73-4 74-5 74-6 74-7 74-8 74-9 74-10 74-11 74-12 74-13 74-14 74-15 74

Synthesis of Intermediate 74-2:

The compounds 74-1 (500 mg, 3.96 mmol) and 70-2 (0.813 mg, 3.96 mmol) were dissolved in DMF (3 mL). Triethylamine (3 mL), copper(I) iodide (CuI, 75 mg, 0.396 mmol), and bis(triphenylphosphine)palladium(II) dichloride ($PdCl_2(PPh_3)_2$, 278 mg, 0.396 mmol) were added sequentially. Under a nitrogen atmosphere, the reaction mixture was heated to 95°C, and the reaction proceeded for 6 hours. LC-MS monitoring indicated completion of the reaction. The reaction mixture was extracted with ethyl acetate/water. The organic phase was washed with saturated NaCl solution, dried over anhydrous $Na_2SO_4$, filtered under suction, and spin-dried. Purification by normal-phase column chromatography and concentration under reduced pressure were performed to obtain a white solid (736 mg, 74.2% ). LC-MS (ESI): m/z found $[M+H]^+$ = 251.0.

Synthesis of Intermediate 74-3:

The compound 74-2 (0.375 g, 1.5 mmol) was dissolved in THF (8 mL). Water (4 mL) and lithium hydroxide monohydrate ($LiOH{\cdot}H_2O$, 0.441 g, 10.5 mmol) were added sequentially. The reaction proceeded at room temperature for 4 hours. After completion, the reaction mixture was washed with ethyl acetate twice. The aqueous phase was adjusted to pH ~2 with 1M HCl aqueous solution, resulting in the precipitation of an amount of white solid. The solid was collected by suction filtration and lyophilized to obtain a white solid (273 mg, 77.1% ). LC-MS (ESI): m/z found $[M+H]^+$ = 237.0.

Synthesis of Intermediate 74-4:

The compound 74-3 (540 mg, 0.488 mmol) was dissolved in DCM (40 mL). meta-Chloroperoxybenzoic acid (766 mg, 4.437 mmol) was added. The reaction proceeded at room temperature for 12 hours. Purification by reverse-phase column chromatography and concentration were performed to obtain a white solid (483 mg, 83.9% ). LC-MS (ESI): m/z found $[M+H]^+$ = 269.2.

Synthesis of Intermediate 74-5:

The compound 74-4 (30 mg, 0.112 mmol) was dissolved in acetonitrile (MeCN). N-Hydroxysuccinimide (HOSu) and N,N'-Dicyclohexylcarbodiimide (DCC) were added at 0°C. The reaction mixture was warmed to room temperature, and the reaction proceeded for 2 hours. LC-MS monitoring indicated completion of the reaction. The white insoluble solid was removed by suction filtration. The filtrate was concentrated under reduced pressure to obtain a white solid product (40 mg, 99% ). LC-MS (ESI): m/z found $[M+H]^+$ = 366.20.

Synthesis of Intermediate 74-7:

A compound 74-6 (2 g, 9.23 mmol) was dissolved in DCM (20 mL). A solution of $K_2CO_3$ (3.8 g, 27.7 mmol) in $H_2O$ (8 mL) was prepared and cooled, then added to the reaction mixture. Di-tert-butyl dicarbonate (2 g, 9.23 mmol) was added. The reaction mixture was warmed to room temperature, and the reaction proceeded for 2 hours. The reaction mixture was extracted with DCM/$H_2O$. The organic phase was washed with saturated NaCl solution for three times and concentrated. Purification by normal-phase column chromatography (17% EA in PE) and concentration under reduced pressure were performed to obtain a pale yellow solid product (980 mg, 37.9%). LC-MS (ESI): m/z found $[M-H]^-$ = 279.0.

Synthesis of Intermediate 74-8:

The compound 74-7 (980 mg, 3.5 mmol) was dissolved in EtOH (20 mL). Sodium borohydride (200 mg, 5.28 mmol) was added. Under a nitrogen atmosphere, the reaction proceeded at room temperature for 2 hours. The reaction mixture was extracted with ethyl acetate/water. The organic phase was washed with saturated NaCl solution, dried over anhydrous $Na_2SO_4$, filtered under suction, and spin-dried. Purification by normal-phase column chromatography (25% EA in PE) and spin-drying were performed to obtain a pale yellow solid (590 mg, 60% ). LC-MS (ESI): m/z found $[M-56]^+$ = 227.20.

Synthesis of Intermediate 74-9:

The compound 74-8 (590 mg, 2.09 mmol) was dissolved in MeOH (10 mL). Palladium on carbon (Pd/C) was added. Under a hydrogen atmosphere ($H_2$), the reaction proceeded at room temperature for 4 hours. After completion, the reaction mixture was filtered. The filtrate was spin-dried and purified by normal-phase column chromatography (70% EA). Concentration under reduced pressure was performed to obtain a white solid product (450 mg, 85%). LC-MS (ESI): m/z found $[M-56]^+$= 179.20.

Synthesis of Intermediate 74-10:

Fmoc-Val-Ala (Fmoc-V-A, 732 mg, 1.78 mmol) and 2-(7-Azabenzotriazol-1-yl)-N,N,N',N'-tetramethyluronium hexafluorophosphate (HATU, 814 mg, 2.136 mmol) were dissolved in DMF (4 mL). The reaction mixture was stirred for 5 minutes. The compound 74-9 (450 mg, 1.78 mmol) and N,N-Diisopropylethylamine (DIEA, 692 mg, 5.35 mmol) were added sequentially. The reaction proceeded at room temperature until completion. Purification by reverse-phase C-18 column chromatography (48% MeCN in $H_2O$) and concentration under reduced pressure were performed to obtain a white solid (1.05 g, 91%). LC-MS (ESI): m/z found $[M-100]^+$ = 546.5.

Synthesis of Intermediate 74-11:

The compound 74-10 (200 mg, 0.31 mmol) was dissolved in DCM (2 mL). A solution of HCl in ethyl acetate (0.39 mL) was added. The reaction proceeded at room temperature for 4 hours. After completion, the reaction mixture was concentrated under reduced pressure. The residue was dissolved in a small amount of DMF and purified by reverse-phase column chromatography (50% ACN in $H_2O$). Lyophilization was performed to obtain a white solid (380 mg, 56%). LC-MS (ESI): m/z found $[M+H]^+$ = 545.2.

Synthesis of Intermediate 74-12:

The compound 74-11 (380 mg, 0.698 mmol) was dissolved in DMF (2 mL). m-PEG8-NHS ester (355.5 mg, 0.698 mmol) and N,N-Diisopropylethylamine (DIEA, 270 mg, 2.09 mmol) were added. The reaction proceeded at room temperature until completion. Purification by reverse-phase C-18 column chromatography (42% MeCN in $H_2O$) and lyophilization were performed to obtain s a white solid (350 mg, 53.4%). LC-MS (ESI): m/z found $[M+H]^+$ = 939.7.

Synthesis of Intermediate 74-13:

The compound 74-12 (350 mg, 0.374 mmol) was dissolved in DMF. The reaction mixture was stirred for 5 minutes. Bis(4-nitrophenyl) carbonate ($(PNP)_2CO$, 226.7 mmol) and N,N-Diisopropylethylamine (DIEA, 48 mg, 0.374 mmol) were added. The reaction proceeded at room temperature until completion. Purification by reverse-phase C-18 column chromatography (55% ACN in $H_2O$) and lyophilization were performed to obtain a white solid product. LC-MS (ESI): m/z found $[M/2+H]^+$ = 553.5.

Synthesis of Intermediate 74-14:

The compound 74-13 (50 mg, 0.045 mmol) and 1-Hydroxybenzotriazole (HOBt, 6.75 mg, 0.050 mmol) were dissolved in DMF. The compound 11 (19.25 mg, 0.045 mmol) and N,N-Diisopropylethylamine (DIEA, 17.5 mg, 0.136 mmol) were added sequentially. The reaction proceeded at room temperature for 2 hours. Purification by HPLC (10%-70% MeCN in $H_2O$ over 30 minutes) and lyophilization were performed to obtain a yellow solid product (35 mg, 56%). LC-MS (ESI): m/z found $[M/2+H]^+$ = 696.30.

Synthesis of Intermediate 74-15:

The compound 74-14 (35 mg, 0.025 mmol) was dissolved in DMF. Diethylamine was added. The reaction proceeded at room temperature for 2 hours. Purification by HPLC (10%-60% ACN in $H_2O$ over 30 minutes) and lyophilization were performed to obtain a yellow solid (6 mg, 20.4% ). LC-MS (ESI): m/z found $[M+H]^+$ = 1168.80.

Synthesis of Compound 74:

The compound 73-5 (2 mg, 0.005 mmol) was dissolved in DMF. The compound 74-15 (3 mg, 0.003 mmol) and triethylamine (TEA, 0.66 mg, 0.005 mmol) were added. The reaction proceeded at room temperature for 2 hours. Purification by HPLC and lyophilization were perfomed to obtain a yellow solid (0.6 mg, 16.5%). LC-MS (ESI): m/z found $[M/2+H]^+$ = 710.35.

Example 75:

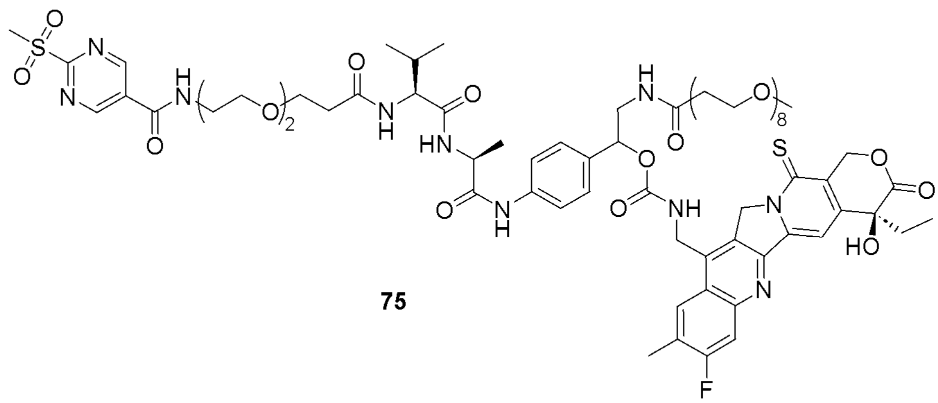

75

Synthetic route:

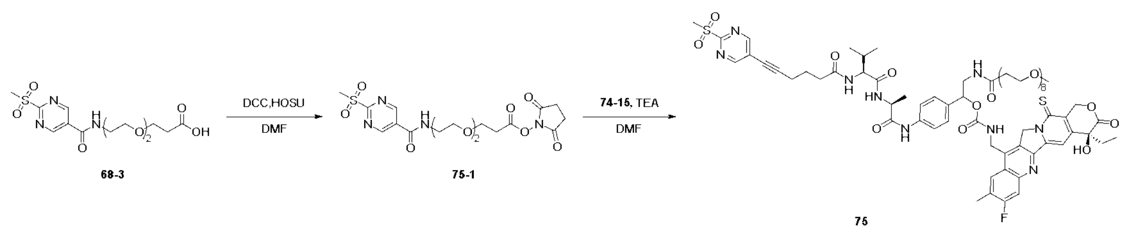


68-3 75-1 75

Synthesis of Intermediate 75-1:

The compound 68-3 (30 mg, 0.083 mmol) was dissolved in acetonitrile (MeCN). N-Hydroxysuccinimide (HOSu) and N,N'-Dicyclohexylcarbodiimide (DCC) were added at 0°C. The reaction mixture was warmed to room temperature, and the reaction proceeded for 2 hours. LC-MS monitoring indicated completion of the reaction. The white insoluble solid was removed by suction filtration. The filtrate was concentrated under reduced pressure to obtain a white solid product (38

mg, 99% ). LC-MS (ESI): m/z found $[M+H]^+$ = 459.1.

Synthesis of Compound 75:

The compound 75-1 (2 mg, 0.005 mmol) was dissolved in DMF. The compound 74-15 (3 mg, 0.003 mmol) and triethylamine (0.66 mg, 0.005 mmol) were added. The reaction proceeded at room temperature for 2 hours. Purification by HPLC and lyophilization were perfomed to obtain a yellow solid (0.63 mg, 16.2% ). LC-MS (ESI): m/z found $[M/2+H]^+$ = 757.05.

Example **76:**

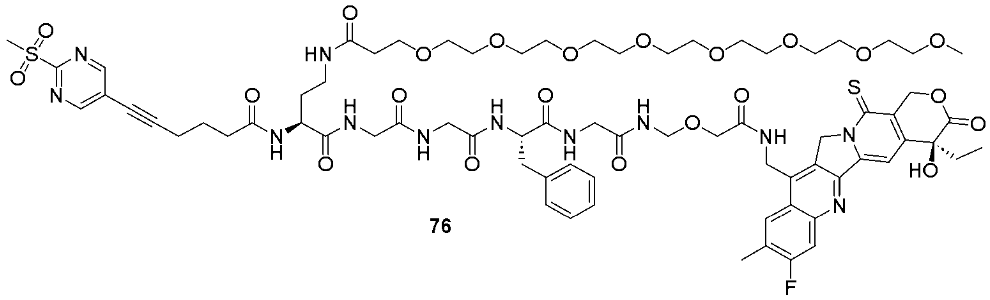

**76**

Synthetic route:

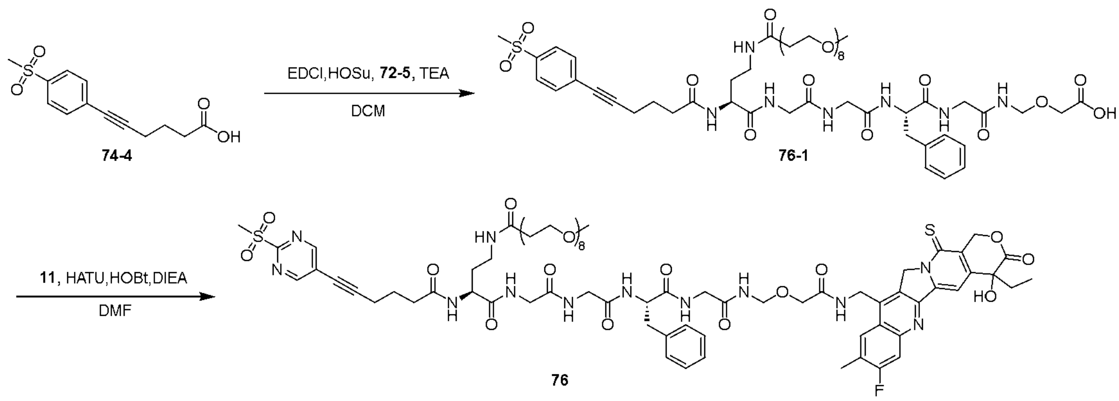


74-4

76-1

76

Synthesis of Intermediate 76-1:

The compound 74-4 (43 mg, 0.177 mmol) was dissolved in DCM (5 mL). N-Hydroxysuccinimide (21.4 mg, 0.186 mmol) and 1-Ethyl-3-(3-dimethylaminopropyl)carbodiimide hydrochloride (EDCI, 35.7 mg, 0.186 mmol) were added. The reaction proceeded at room temperature until the compound 74-4 was no longer reduced. The compound 72-5 (162 mg, 0.177 mmol) and triethylamine (24 μL, 0.172 mmol) were then added, and the reaction was continued at room temperature overnight. After completion, the reaction mixture was concentrated under reduced pressure. The residue was dissolved in MeOH and purified by preparative HPLC to obtain a colorless oily product (54.3 mg, 26.3% ). LC-MS (ESI): m/z found $[M-75]^-$ = 1092.65.

Synthesis of Compound 76:

The compound 76-1 (8.2 mg, 0.007 mmol) was dissolved in DMF. 2-(7-Azabenzotriazol-1-yl)-N,N,N',N'-tetramethyluronium hexafluorophosphate (HATU, 3.06 mg, 0.008 mmol) was added, and the reaction proceeded at room temperature for 30 minutes. The compound 11 (3 mg, 0.007 mmol) and N,N-diisopropylethylamine (DIEA, 1.3 μL, 0.008 mmol) were then added. The reaction proceeded at room temperature for an additional 2 hours. LC-MS monitoring

indicated completion of the reaction. Purification by HPLC (0.05% TFA in water) and lyophilization were performed to obtain a product (3.23 mg, 29.3% ). LC-MS (ESI): m/z found $[M/2+1]^+$ = 788.95.

Example **77:**

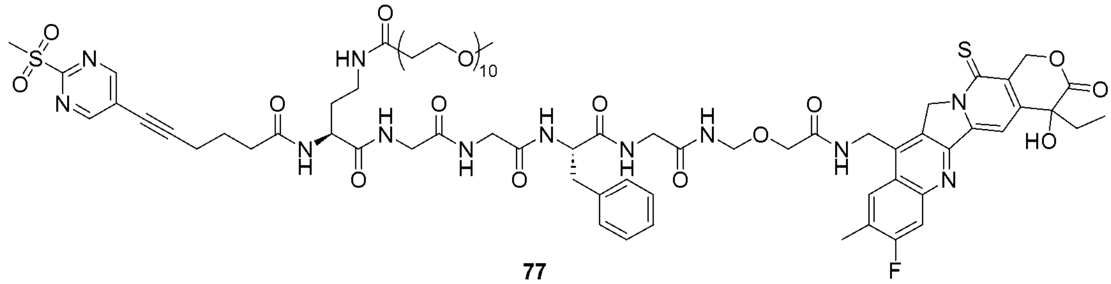

**77**

Synthetic route:

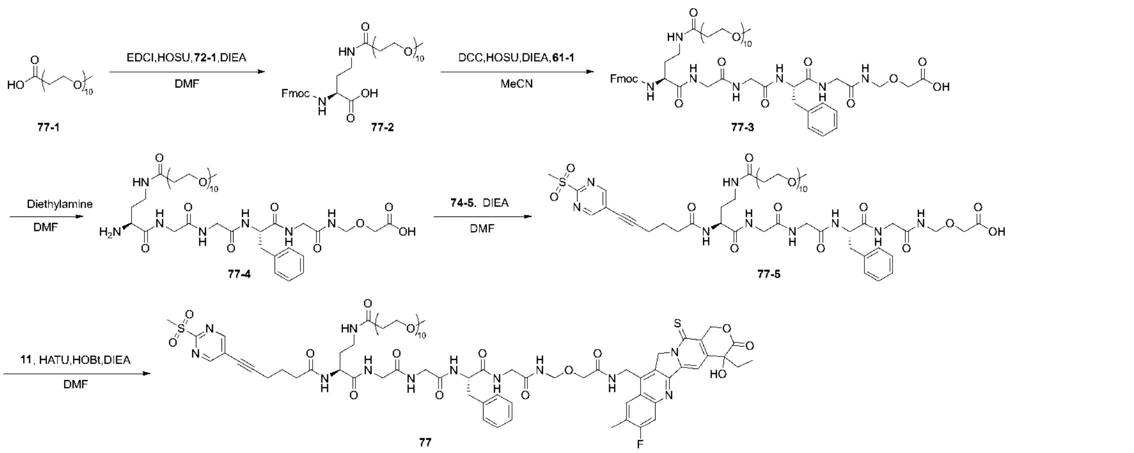


Synthesis of Intermediate 77-2:

A compound 77-1 (300 mg, 0.6 mmol) was dissolved in DMF. 1-Ethyl-3-(3-dimethylaminopropyl)carbodiimide hydrochloride (126 mg, 0.66 mmol) and N-Hydroxysuccinimide (76 mg, 0.66 mmol) were added. The reaction proceeded at room temperature for 2 hours. N,N-Diisopropylethylamine (155 mg, 1.2 mmol) and the compound 72-1 (204 mg, 0.6 mmol) were then added, and the reaction was continued at room temperature for 4 hours. After LC-MS monitoring indicated completion of the reaction, the reaction mixture was purified by reverse-phase C-18 column chromatography (50% ACN in $H_2O$) and concentrated under reduced pressure to obtain a colorless oily product (193 mg, 39% ). LC-MS (ESI): m/z found $[M+H]^+$ = 823.6.

Synthesis of Intermediate 77-3:

The compound 77-2 (308 mg, 0.374 mmol) was dissolved in DMF. N,N'-Dicyclohexylcarbodiimide (DCC, 85 mg, 0.412 mmol) and N-Hydroxysuccinimide (HOSu, 47.4 mg, 0.412 mmol) were added. The reaction proceeded at room temperature for 2 hours. N,N-Diisopropylethylamine (DIEA, 48 mg, 0.374 mmol) and the compound 61-1 (158 mg, 0.374 mmol) were then added, and the reaction was continued at room temperature for 4 hours. Purification by reverse-phase C-18 column chromatography (44% ACN in $H_2O$) and concentration under reduced pressure were performed to obtain a product (2225 mg, 49% ). LC-MS (ESI): m/z found $[M+H]^+$ = 1228.85.

Synthesis of Intermediate 77-4:

The compound 77-3 (225 mg, 0.183 mmol) was dissolved in DMF. Diethylamine (50 μL) was added, and the reaction proceeded at room temperature for 2 hours. Purification by reverse-phase column chromatography (30% ACN in $H_2O$) and concentration under reduced pressure were performed to obtain a product (115 mg, 62% ). LC-MS (ESI): m/z found $[M+H]^+$ = 1006.75.

Synthesis of Intermediate 77-5:

The compound 77-4 (115 mg, 0.114 mmol) was dissolved in DMF. The compound 74-5 (62 mg, 0.171 mmol) and N,N-Diisopropylethylamine (DIEA, 44 mg, 0.342 mmol) were added. The reaction proceeded at room temperature for 2 hours. Purification by reverse-phase column chromatography and lyophilization were performed to obtain a product (100 mg, 70% ). LC-MS (ESI): m/z found $[M+H]^+$ = 1257.00.

Synthesis of Compound 77:

The compound 77-5 (30 mg, 0.024 mmol) and 2-(7-Azabenzotriazol-1-yl)-N,N,N',N'-tetramethyluronium hexafluorophosphate (HATU, 10 mg, 0.026 mmol) were dissolved in DMF. The compound 11 (10 mg, 0.024 mmol) and N,N-Diisopropylethylamine (DIEA, 9.1 mg, 0.072 mmol) were added. The reaction proceeded at room temperature for 2 hours. Purification by prep-HPLC and lyophilization were perfomed to obtain a yellow solid (11 mg, 28% ). LC-MS (ESI): m/z found $[M/2+H]^+$ = 833.05.

Examples 78-80:

Following the synthetic procedure described for Example 77, the corresponding starting materials were substituted to achieve Examples 78-80.

Table 1 Structural formulas and MS (ESI) data for Examples 78-80

| Example | Structural formulas | MS (ESI) $[M/2+H]^+$ |
|---|---|---|
| 78 | 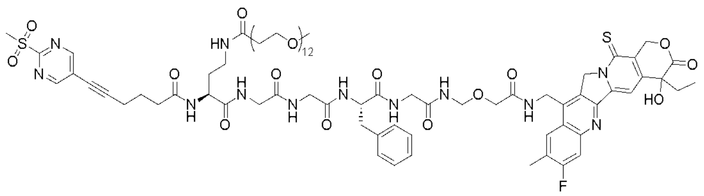 | 877.00 |
| 79 | 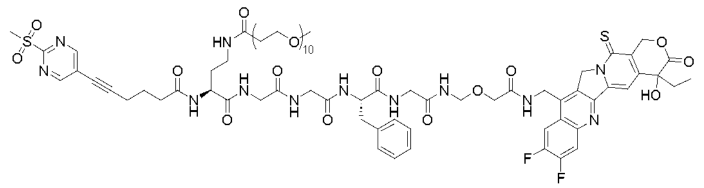 | 834.85 |
| 80 | 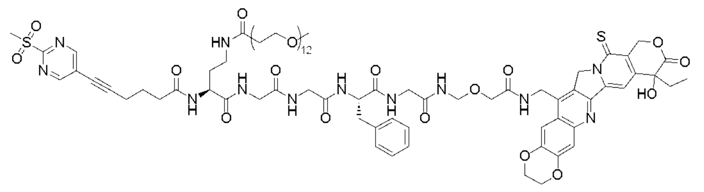 | 890.10 |

Example **81-82:**

Following the synthetic procedure described for Example 72 and Example 76, the corresponding starting materials were substituted to achieve Examples 81-82.

Table 2 Structural formulas and MS (ESI) data for Examples 81-82

| Example | Structural formulas | MS (ESI) [M/2+H]+ |
|---|---|---|
| 81 | 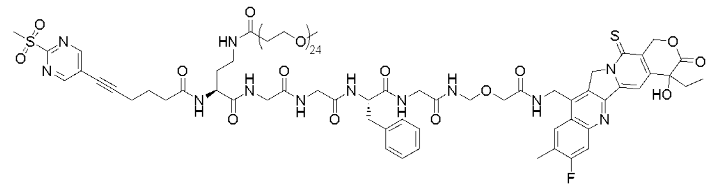 | 761.30 |
| 82 | 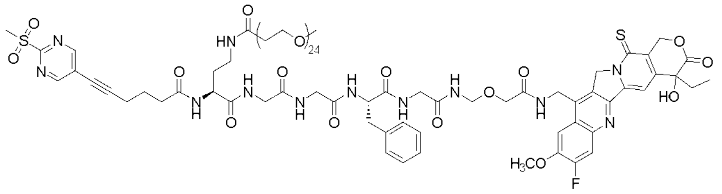 | 766.70 |

Example 83:

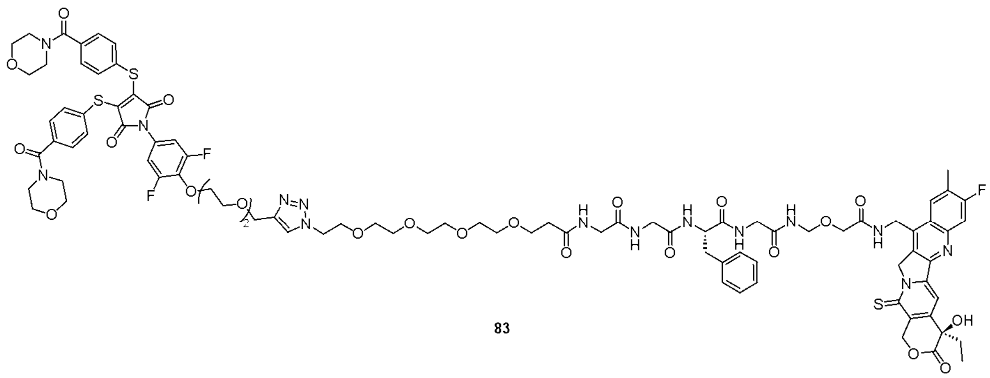

83

Synthetic route:

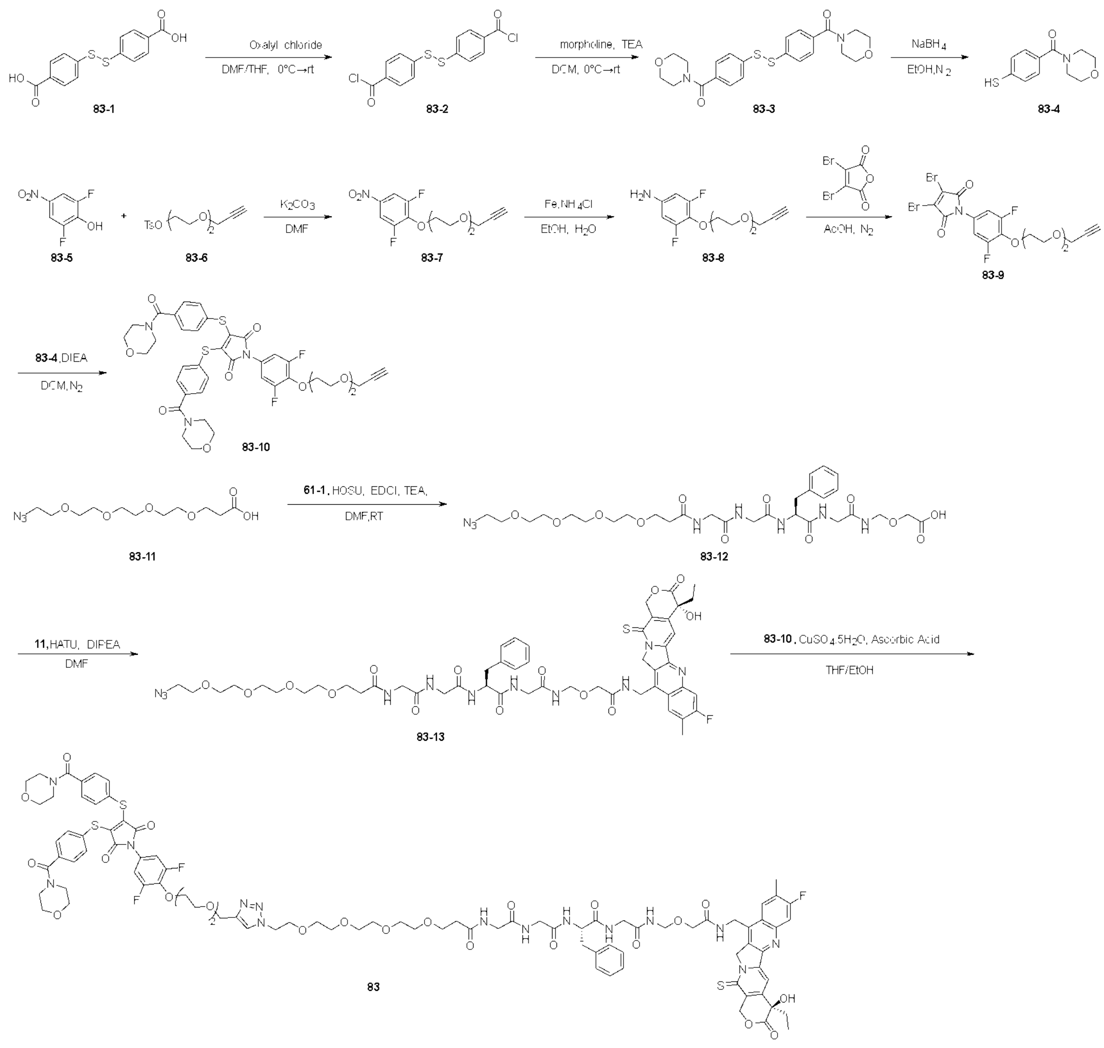


Synthesis of Intermediate 83-3:

A compound 83-1 (100 mg, 0.326 mmol) was dissolved in THF (3 mL). A catalytic amount of DMF was added. Under a nitrogen atmosphere at 0°C, oxalyl chloride (138 μL, 0.038 mmol) was added dropwise. The reaction mixture was then warmed to room temperature, and the reaction proceeded for 1 hour; the solution turned from turbid to clear. The reaction mixture was concentrated under reduced pressure. The acyl chloride intermediate was dissolved in DCM (2 mL). Triethylamine (TEA, 453 μL, 3.26 mmol) and morpholine (143 μL, 1.632 mmol) were added at 0°C. The reaction mixture was warmed to room temperature, and the reaction proceeded for 2 hours. The reaction mixture was extracted with DCM/$H_2O$. The organic phase was washed with saturated NaCl solution, dried over anhydrous $Na_2SO_4$, filtered under suction, and concentrated under reduced pressure. Purification by normal-phase column chromatography (5.9% MeOH in DCM) and concentration under reduced pressure were performed to obtain a light brown oily product (141 mg, 97.3% ). LC-MS (ESI): m/z found $[M+H]^+$ = 445.0.

Synthesis of Intermediate 83-4:

The compound 83-3 (2.89 g, 6.5 mmol) was dissolved in EtOH (25 mL). Sodium borohydride ($NaBH_4$, 1.967 g, 52 mmol) was added in two portions at 0.5-hour intervals. Under a nitrogen atmosphere, the reaction proceeded at room temperature overnight. The reaction was quenched with saturated $NH_4Cl$ aqueous solution and extracted with ethyl acetate. The organic phase was dried over anhydrous $Na_2SO_4$, filtered under suction, and concentrated under reduced pressure. Purification by normal-phase column chromatography (10.3% MeOH in DCM) and concentration under reduced pressure were performed to obtain a pale yellow oily product (1.25 g, 41.1% ). LC-MS (ESI): m/z found $[M+H]^+$ = 224.2.

Synthesis of Intermediate 83-7:

Compounds 83-5 (200 mg, 1.142 mmol) and 83-6 (340.8 mg, 1.142 mmol) were dissolved in DMF (5 mL). Potassium carbonate ($K_2CO_3$, 316 mg, 2.284 mmol) was added. The reaction mixture was heated to 100°C and refluxed overnight. The reaction mixture was extracted with 1N dilute HCl/DCM. The organic phase was dried over anhydrous $Na_2SO_4$, filtered under suction, and concentrated under reduced pressure. Purification by normal-phase column chromatography (15% EA in PE) and concentration under reduced pressure were performed to obtain a pale yellow oily product (234.7 mg, 68.2% ). LC-MS (ESI): m/z found $[M+H]^+$ = 302.2.

Synthesis of Intermediate 83-8:

The compound 83-7 (235 mg, 0.780 mmol) was dissolved in EtOH (8 mL). Water ($H_2O$, 2 mL), iron powder (Fe, 174.3 mg, 3.12 mmol), and ammonium chloride ($NH_4Cl$, 66.8 mg, 1.248 mmol) were added sequentially. The reaction mixture was heated to 80°C and refluxed for 5 hours. The reaction mixture was filtered under suction through Celite. The filtrate was concentrated under reduced pressure. Purification by normal-phase column chromatography (18.8% EA in PE) and concentration under reduced pressure were performed to obtaina pale yellow oily product (127.2 mg, 60.1% ). LC-MS (ESI): m/z found $[M+H]^+$ = 272.2.

Synthesis of Intermediate 83-9:

The compound 83-8 (23 mg, 0.085 mmol) was dissolved in acetic acid (AcOH, 1 mL). 2,3-Dibromomaleic anhydride (21.7 mg, 0.085 mmol) was added. The reaction mixture was heated to 110°C and refluxed for 3 hours. The reaction mixture was distilled under reduced pressure. Purification by normal-phase column chromatography (15.8% EA in PE) and concentration under reduced pressure were performed to obtain a white solid (32.9 mg, 76.0% ). LC-MS (ESI): m/z found $[M+Na]^+$ = 526.8.

Synthesis of Intermediate 83-10:

The compound 83-9 (32.9 mg, 0.065 mmol) was dissolved in DCM (2 mL). The compound 83-4 (28.9 mg, 0.129 mmol) and N,N-Diisopropylethylamine (DIEA, 21.3 μL, 0.129 mmol) were added slowly at 0°C. The reaction mixture was stirred at 0°C for 5 minutes, then warmed to room temperature, and the reaction proceeded for 2 hours. The reaction mixture was distilled under reduced pressure. Purification by normal-phase column chromatography and concentration under reduced pressure were performed to obtain an orange solid (16.3 mg, 31.6% ). LC-MS (ESI): m/z found $[M+H]^+$ = 794.0.

Synthesis of Intermediate 83-12:

In an ice bath, 15-azido-4,7,10,13-tetraoxapentadecanoic acid (83-11, 50 mg, 0.0171 mmol) was dissolved in anhydrous N,N-dimethylformamide (5 mL). N-Hydroxysuccinimide (NHS, 26 mg, 0.257 mmol) and 1-(3-Dimethylaminopropyl)-3-ethylcarbodiimide hydrochloride (EDC·HCl, 50 mg, 0.257 mmol) were added. The reaction proceeded for 1 hour. The compound 61-1 (109 mg, 0.257 mmol) and triethylamine (TEA, 26 μL, 0.189 mmol) were then added. The reaction proceeded at room temperature for 2 hours. LCMS monitoring indicated completion of the reaction. The reaction mixture was purified by prep-HPLC to obtain a compound (65 mg, 54% ). LC-MS (ESI): m/z found $[M+H]^+$ = 697.50.

Synthesis of Intermediate 83-13:

In an ice bath, the compound 83-12 (20 mg, 0.0287 mmol) was dissolved in anhydrous N,N-dimethylformamide (5 mL). 2-(7-Azabenzotriazol-1-yl)-N,N,N',N'-tetramethyluronium hexafluorophosphate (HATU, 12.4 mg, 0.0327 mmol) was added. The reaction proceeded for 20 minutes. The compound 11 (12 mg, 0.0287 mmol) and N,N-Diisopropylethylamine (DIEA, 9.7 μL, 0.0540 mmol) were then added. The reaction proceeded at room temperature for 2 hours. LC-MS monitoring indicated completion of the reaction. The reaction mixture was purified by prep-HPLC to obtain a compound (15 mg, 47% yield ). LC-MS (ESI): m/z found $[M+H]^+$ = 1104.07.

Synthesis of Compound 83:

At room temperature, the compound 83-13 (5 mg, 0.0045 mmol) was dissolved in a mixture of anhydrous tetrahydrofuran (3 mL) and anhydrous ethanol (1 mL). The compound 83-10 (3.6 mg, 0.0045 mmol), copper(II) sulfate pentahydrate (1.12 mg, 0.0045 mmol), and ascorbic acid (Vitamin C, 0.79 mg, 0.0045 mmol) were added. The atmosphere

was purged with nitrogen for three times. The reaction proceeded at room temperature for 2 hours. LCMS monitoring indicated completion of the reaction. The reaction mixture was dried and purified by prep-HPLC to obtain a compound 83 (1.60 mg, 19% yield ). LC-MS (ESI): m/z found $[M/2+H]^+$ = 949.00.

Example **84-88:**

Table 3 Structural formulas and MS (ESI) data for Examples 84-88

| Examples | Structural formulas | MS (ESI) $[M/2+H]^+$ |
|---|---|---|
| 84 | 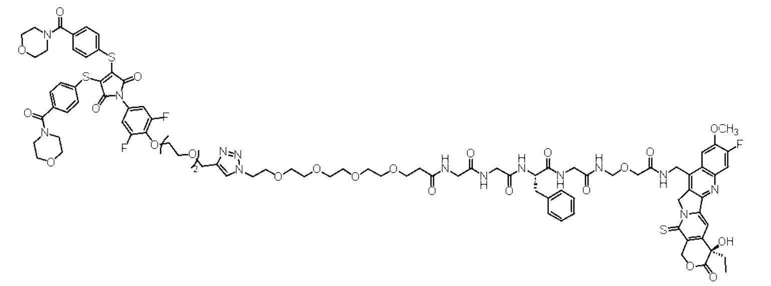 | 958.10 |
| 85 | 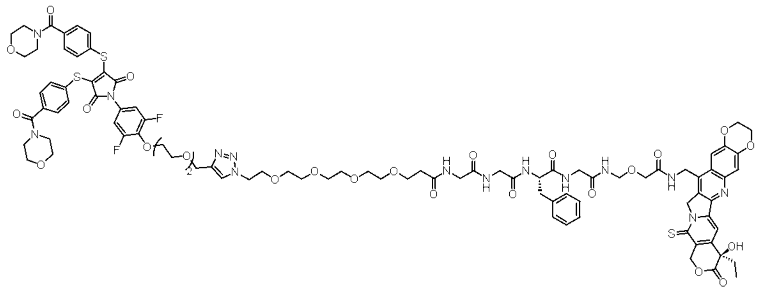 | 963.20 |
| 86 | 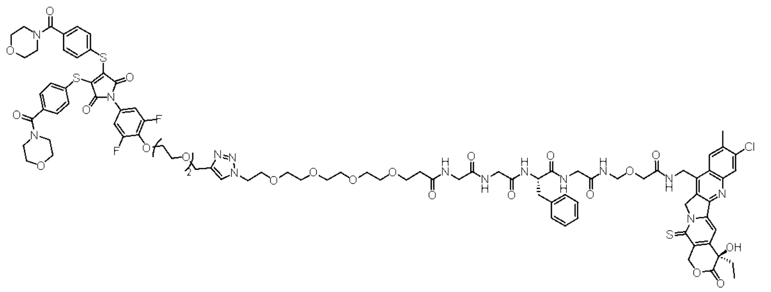 | 957.90 |
| 87 | 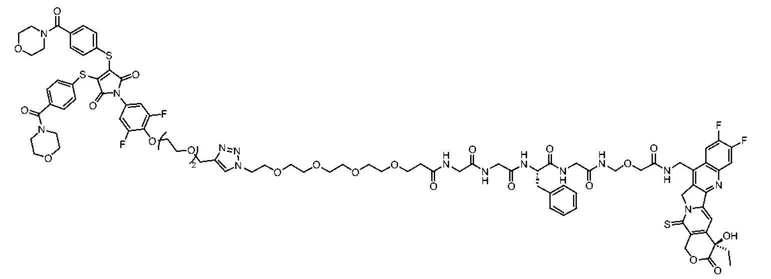 | 951.80 |

(continued)

| Examples | Structural formulas | MS (ESI) [M/2+H] + |
|---|---|---|
| 88 | 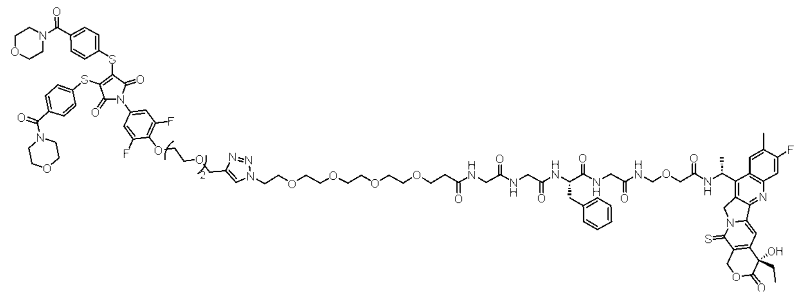 | 957.10 |

Example 89:

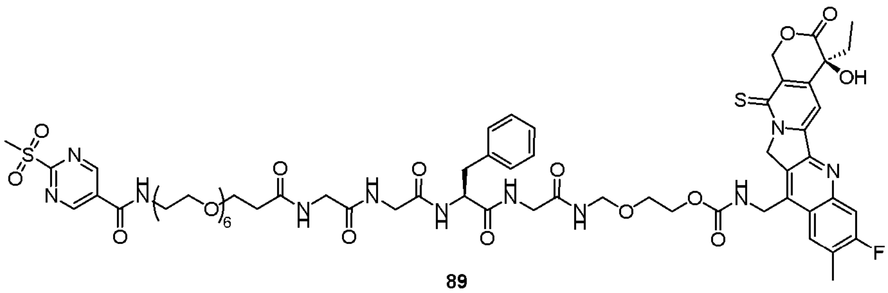

**89**

Synthetic route:

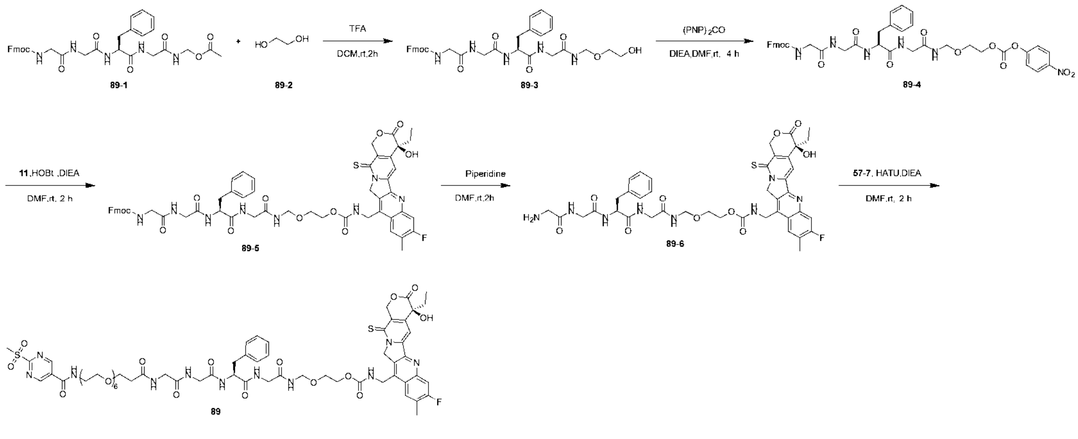


Synthesis of Intermediate 89-3:

At room temperature, the compound 89-1 (200 mg, 0.3176 mmol) was dissolved in DCM (6.67 mL). Trifluoroacetic acid (TFA, 1.33 mL) and ethylene glycol (89-2, 0.333 mL) were added. The reaction proceeded at room temperature for 2 hours. LC-MS monitoring indicated complete consumption of the starting material. The reaction mixture was extracted with DCM/$H_2O$. The organic phase was washed with saturated brine, dried over anhydrous $Na_2SO_4$, and concentrated. Purification by normal-phase column chromatography (MeOH:DCM = 13:87) and concentration were performed to obtain a product (130 mg, 65% yield). LC-MS (ESI): m/z found $[M+H]^+$ = 632.5.

Synthesis of Intermediate 89-4:

At room temperature, the compound 89-3 (125 mg, 0.198 mmol) was dissolved in DMF (2.5 mL). N,N-Diisopropylethylamine (DIEA, 50 mg, 0.396 mmol) and bis(4-nitrophenyl) carbonate (125 mg, 0.396 mmol) were added. The reaction proceeded at room temperature for 4 hours. TLC and LC-MS monitoring indicated no further consumption of starting material. The reaction mixture was extracted with DCM and water. The organic phase was concentrated and purified by normal-phase column chromatography (MeOH:DCM = 1:9), and concentrated to obtain a product (70 mg, 44% yield). LC-MS (ESI): m/z found $[M+H]^+$ = 797.5.

Synthesis of Intermediate 89-5:

At room temperature, the compound 89-4 (70 mg, 0.0878 mmol) was dissolved in DMF (2 mL). 1-Hydroxybenzotriazole (HOBt, 13.5 mg, 0.0966 mmol) was added, and the reaction mixture was stirred for 5 minutes. N,N-Diisopropylethylamine (DIEA, 22.7 mg, 0.1757 mmol) and the compound 11 (38.8 mg, 0.0878 mmol) were then added. The reaction proceeded at room temperature for 2 hours. After LC-MS monitoring indicated no further consumption of starting material, the reaction mixture was filtered. Purification by reverse-phase C-18 column chromatography (55% MeCN in $H_2O$) and concentration were performed to obtain a product (35 mg, 37% ). LC-MS (ESI): m/z found $[M+H]^+$ = 1083.3.

Synthesis of Intermediate 89-6:

At room temperature, the compound 89-5 (35 mg, 0.0323 mmol) was dissolved in DMF (2 mL). Piperidine (14 mg, 0.1615 mmol) was added. The reaction proceeded at room temperature for 2 hours. LC-MS monitoring indicated complete consumption of the starting material. The reaction mixture was filtered and purified by prep-HPLC (5%-50% ACN in $H_2O$ over 30 minutes, 0.1% formic acid). Lyophilization was performed to obtain a product (8.2 mg, 29% yield). MS (ESI): m/z found $[M+H]^+$ = 861.55.

Synthesis of Compound 89:

At room temperature, the compound 57-7 (7.7 mg, 14.3 μmol) was dissolved in DMF (2 mL). 2-(7-Azabenzotriazol-1-yl)-N,N,N',N'-tetramethyluronium hexafluorophosphate (HATU, 5.8 mg, 15.24 μmol) was added, and the reaction mixture was stirred for 5 minutes. N,N-Diisopropylethylamine (DIEA, 3.7 mg, 28.6 μmol) and the compound 89-6 (8.2 mg, 9.525 μmol) were then added. The reaction proceeded at room temperature for 2 hours. After LC-MS monitoring indicated no further consumption of starting material, the reaction mixture was filtered. Purification by prep-HPLC (10%-55% ACN in $H_2O$ over 30 minutes, 0.1% formic acid) and lyophilization were perfomed to obtain a product 89 (3.0 mg, 22% yield). MS (ESI): m/z found $[M+H]^+$ = 1381.7.

Example **90:**

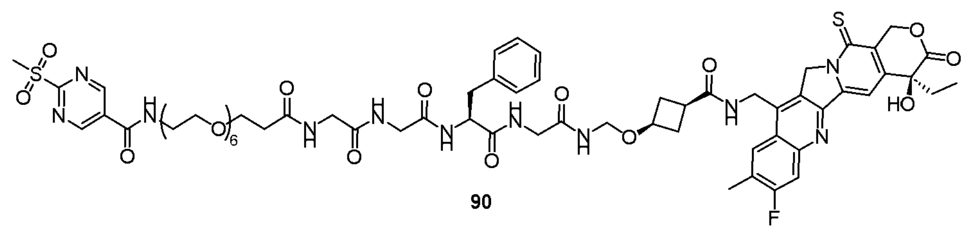

**90**

Synthetic route:

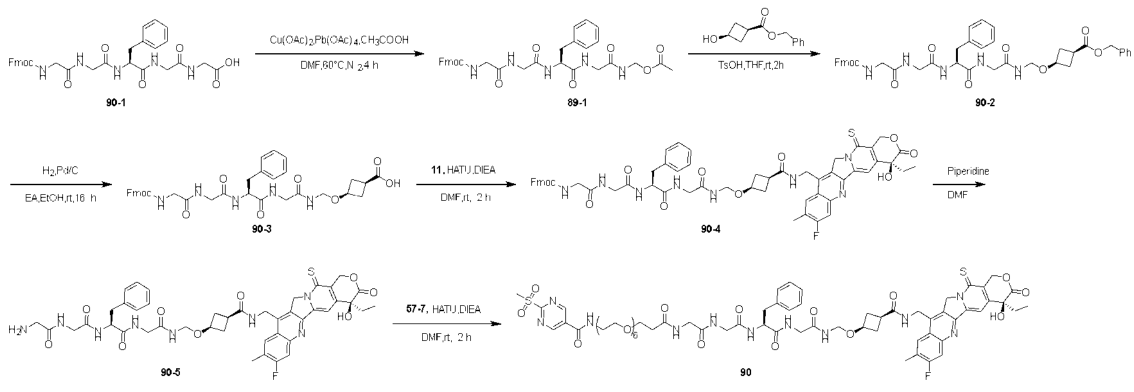


Synthesis of Intermediate 90-1:

At room temperature, the compound 90-1 (465 mg, 0.7553 mmol) was dissolved in DMF (5 mL). Copper(II) acetate (1 mg, 0.0038 mmol), acetic acid (104 mg, 1.737 mmol), and lead (IV) acetate (388 mg, 1.193 mmol) were added. Under a nitrogen atmosphere, the reaction mixture was heated to 60°C, and the reaction proceeded for 4 hours. TLC monitoring indicated complete consumption of the starting material. The reaction mixture was purified by reverse-phase C-18 column chromatography (50% MeCN in $H_2O$), and concentrated to obtaina product (330 mg, 69% yield). LC-MS (ESI): m/z found $[M+H]^+$ = 570.45.

Synthesis of Intermediate 90-2:

At room temperature, the compound 90-1 (244 mg, 0.388 mmol) and p-toluenesulfonic acid (TsOH, 33 mg, 0.194 mmol) were dissolved in THF (3 mL). Benzyl (1s,3s)-3-hydroxycyclobutane-1-carboxylate (160 mg, 0.777 mmol) was added. Under a nitrogen atmosphere, the reaction proceeded at room temperature for 2 hours. LC-MS monitoring indicated complete consumption of the starting material. The reaction mixture was extracted with DCM/$H_2O$. The organic phase was washed with saturated brine, dried over anhydrous $Na_2SO_4$, and concentrated. Purification by normal-phase column chromatography (MeOH:DCM = 1:9) and concentration were performed to obtain a product 1-3 (140 mg, 46.7% yield ). MS (ESI): m/z found $[M+H]^+$ = 776.5.

Synthesis of Intermediate 90-3:

At room temperature, the compound 90-2 (140 mg, 0.18 mmol) was dissolved in a mixture of ethyl acetate (EtOAc, 2 mL) and ethanol (EtOH, 2 mL). Palladium on carbon (Pd/C, 0.096 mg, 0.009 mmol) was added. The atmosphere was replaced with hydrogen gas ($H_2$), and the reaction proceeded at room temperature for 16 hours. After LC-MS monitoring indicated no further consumption of the starting material, the reaction mixture was filtered. The reaction mixture was purified by reverse-phase C-18 column chromatography (47% MeCN in $H_2O$) and concentrated to obtain a product (65 mg, 52.8% yield ). MS (ESI): m/z found $[M+H]^+$ = 686.5.

Synthesis of Intermediate 90-4:

At room temperature, the compound 90-3 (65 mg, 95 μmol) was dissolved in DMF (2 mL). 2-(7-Azabenzotriazol-1-yl)-N,N,N',N'-tetramethyluronium hexafluorophosphate (HATU, 39.6 mg, 104 μmol) was added, and the reaction mixture was stirred for 5 minutes. N,N-Diisopropylethylamine (DIEA, 12.3 mg, 189.6 μmol) and the compound 11 (40.3 mg, 95 μmol) were then added. The reaction proceeded at room temperature for 2 hours. After LC-MS monitoring indicated no further consumption of the starting material, the reaction mixture was filtered. The reaction mixture was purification by reverse-phase C-18 column chromatography (55% MeCN in $H_2O$) and concentrated to obtain a product (50 mg, 48% yield ). MS (ESI): m/z found $[M+H]^+$ = 1093.65.

Synthesis of Intermediate 90-5:

At room temperature, the compound 90-4 (50 mg, 0.0457 mmol) was dissolved in DMF (1.25 mL). Piperidine (19.5 mg, 0.2285 mmol) was added. The reaction proceeded at room temperature for 2 hours. LC-MS monitoring indicated complete consumption of the starting material. The reaction mixture was filtered and purified by prep-HPLC (5%-50% ACN in $H_2O$ over 30 minutes, 0.1% formic acid), and Lyophilizated to obtain a product (13 mg, 32.6% yield ). MS (ESI): m/z found

$[M+H]^+$ = 871.55.

Synthesis of Compound 90:

At room temperature, the compound 57-7 (8.8 mg, 16.4 μmol) was dissolved in DMF (2 mL). 2-(7-Azabenzotriazol-1-yl)-N,N,N',N'-tetramethyluronium hexafluorophosphate (HATU, 6.8 mg, 17.9 μmol) was added, and the reaction mixture was stirred for 5 minutes. N,N-Diisopropylethylamine (DIEA, 5.8 mg, 45 μmol) and the compound 90-5 (13 mg, 15 μmol) were then added. The reaction proceeded at room temperature for 2 hours. After LC-MS monitoring indicated no further consumption of the starting material, the reaction mixture was filtered, purified by prep-HPLC (10%-50% ACN in $H_2O$ over 30 minutes, 0.1% formic acid) and lyophilizated to obtain a product (5.0 mg, 24% yield ). MS (ESI): m/z found $[M+H]^+$ = 1390.75.

Example **91-96:**

Synthetic route:

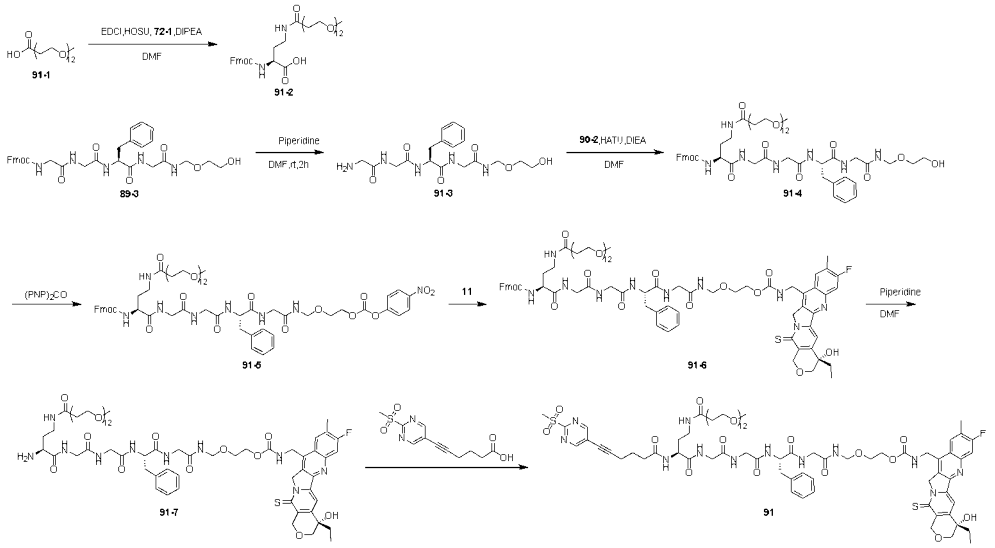


Synthesis of Intermediate 91-2:

The compound 91-1 (1 g, 1.7 mmol) was dissolved in DMF (8 mL). 1-Ethyl-3-(3-dimethylaminopropyl)carbodiimide hydrochloride (EDCI, 360 mg, 1.87 mmol) and N-Hydroxysuccinimide (HOSu, 220 mg, 1.87 mmol) were added. The reaction proceeded at room temperature for 2 hours. N,N-Diisopropylethylamine (DIEA, 320 mg, 2.55 mmol) and the compound 72-1 (520 mg, 1.47 mmol) were then added, and the reaction was continued at room temperature for 4 hours. After completion, the reaction mixture was purified by reverse-phase column chromatography and concentrated under reduced pressure to obtain a colorless oily product (980 mg, 63% ). LC-MS (ESI): m/z found $[M+H]^+$ = 911.7.

In addition to replacing with the corresponding starting materials, compounds 91-96 were synthesized following the procedures described in Example 77 and Example 88. Their structures and LC-MS (ESI) data are shown in Table 4.

Table 4 Structural formulas and MS (ESI) data for Examples 91-96

| Examples | Structural formulas | MS (ESI) $[M/2+H]^+$ |
|---|---|---|
| 91 | 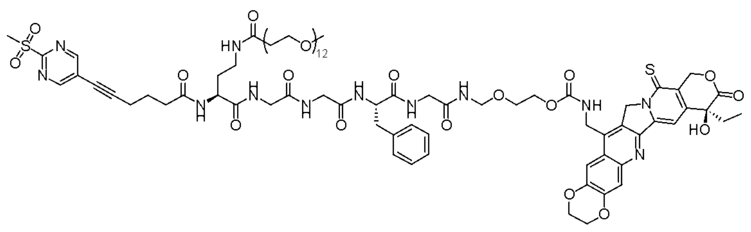 | 904.35 |

(continued)

| Examples | Structural formulas | MS (ESI) [M/2+H]+ |
|---|---|---|
| 92 | 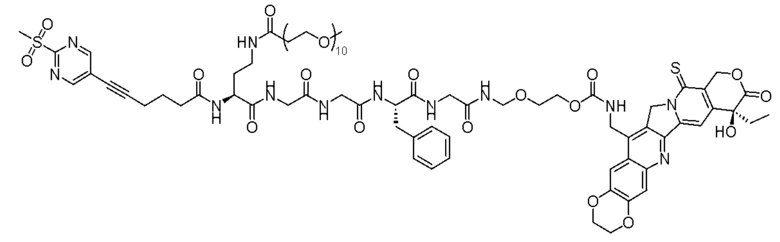 | 860.34 |
| 93 | 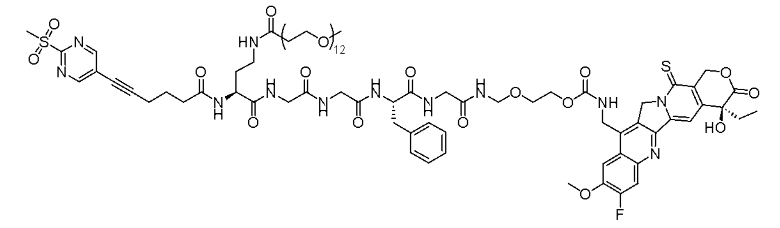 | 899.41 |
| 94 | 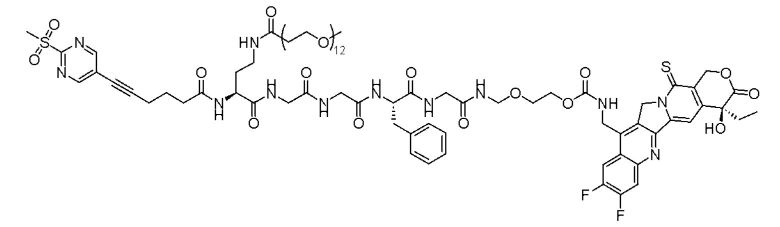 | 893.35 |
| 95 | 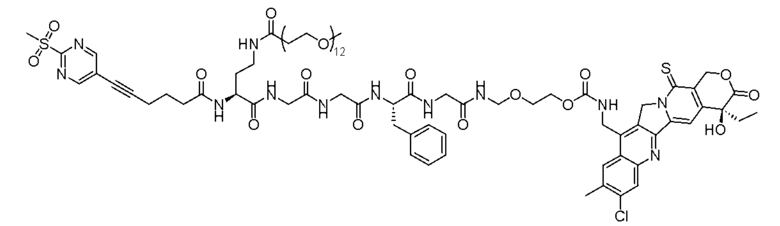 | 899.40 |
| 96 | 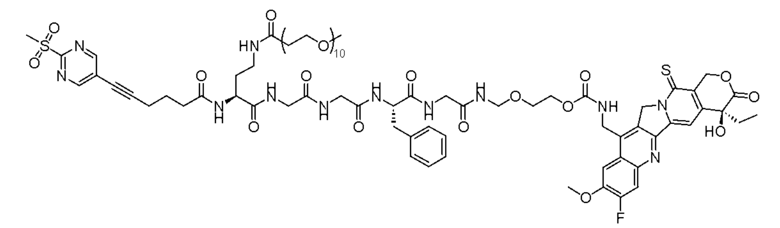 | 855.36 |

Example 97:

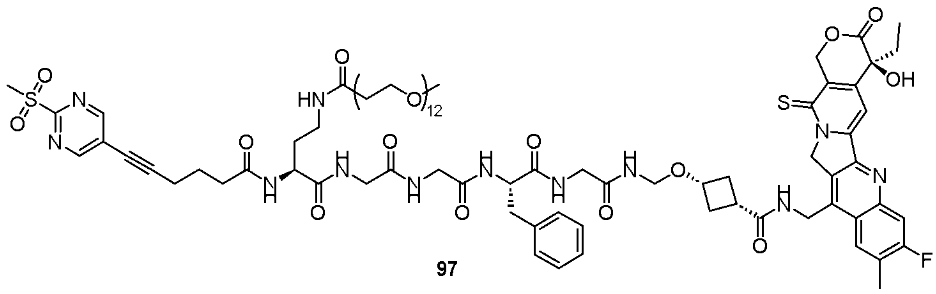

97

Synthetic route:

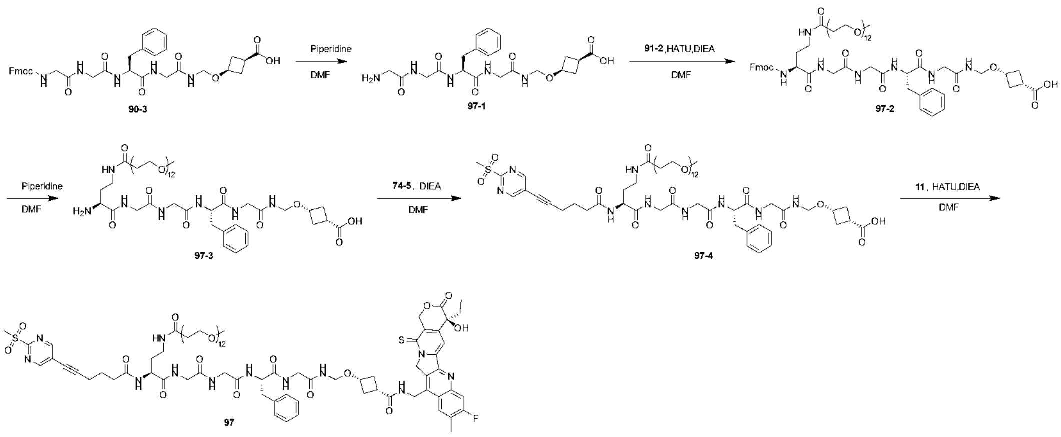


Synthesis of Intermediate 97-1:

The compound 90-3 (270 mg, 0.394 mmol) was dissolved in DMF. Piperidine (167 mg, 1.968 mmol) was added, and the reaction proceeded at room temperature for 2 hours. Purification by reverse-phase column chromatography and concentration under reduced pressure were performed to obtain a white solid product (129 mg, 70% ). LC-MS (ESI): m/z found $[M+H]^+$ = 464.4.

Synthesis of Intermediate 97-2:

The compound 91-2 (253 mg, 0.278 mmol) was dissolved in DMF. 2-(7-Azabenzotriazol-1-yl)-N,N,N',N'-tetramethyluronium hexafluorophosphate (HATU, 116 mg, 0.306 mmol) and N,N-Diisopropylethylamine (DIEA, 72 mg, 0.556 mmol) were added sequentially. The reaction mixture was stirred at room temperature for 30 minutes. The compound 96-1 (129 mg, 0.278 mmol) was then added, and the reaction proceeded at room temperature for an additional 2 hours. Purification by prep-HPLC and concentration under reduced pressure were performed to obtain a colorless oily product (120 mg, 31% ). LC-MS (ESI): m/z found $[M+H]^+$ = 1357.0.

Synthesis of Intermediate 97-3:

The compound 97-2 (120 mg, 0.088 mmol) was dissolved in DMF. Piperidine (38 mg, 0.442 mmol) was added, and the reaction proceeded at room temperature for 2 hours. Purification by prep-HPLC and concentration under reduced pressure were performed to obtain a colorless oily product (61 mg, 61% ). LC-MS (ESI): m/z found $[M+H]^+$ = 1134.85.

Synthesis of Intermediate 97-4:

The compound 97-3 (61 mg, 0.054 mmol) was dissolved in DMF. The compound 74-5 (21.6 mg, 0.059 mmol) and N,N-Diisopropylethylamine (DIEA, 20.85 mg, 0.161 mmol) were added sequentially. The reaction proceeded at room temperature until completion. Purification by prep-HPLC and concentration under reduced pressure were performed to obtain a pale yellow oily product (21 mg, 28% ). LC-MS (ESI): m/z found $[M/2+H]^+$ = 693.3.

Synthesis of Compound 97:

The compound 97-4 (7 mg, 0.005 mmol) and 2-(7-Azabenzotriazol-1-yl)-N,N,N',N'-tetramethyluronium hexafluorophosphate (HATU, 2.1 mg, 0.005 mmol) were dissolved in DMF. N,N-Diisopropylethylamine (DIEA, 2 mg, 0.015 mmol) and the compound 11 (2.36 mg, 0.005 mmol) were added. The reaction proceeded at room temperature for 2 hours. Purification by prep-HPLC and lyophilization were perfomed to obtain a yellow solid (1.6 mg, 17.66% ). LC-MS (ESI): m/z found $[M/2+H]^+$ = 897.15.

Examples 98-101:

In addition to replacing with the corresponding starting materials, examples 98-**101** were synthesized following the procedures described in Example 97. Their structures and LC-MS (ESI) data are shown in Table 5.

Table 5 Structural formulas and MS (ESI) data for Examples **98-101**

| Examples | Structural formulas | MS(ESI) [M/2+H] $^{+}$ |
|---|---|---|
| 98 | 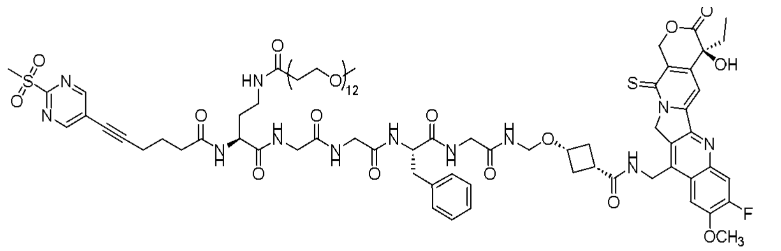 | 905.00 |
| 99 | 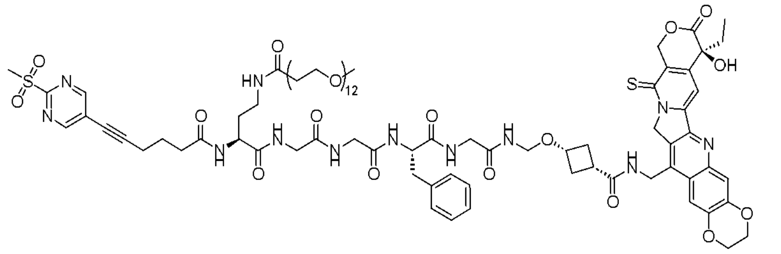 | 910.25 |
| 100 | 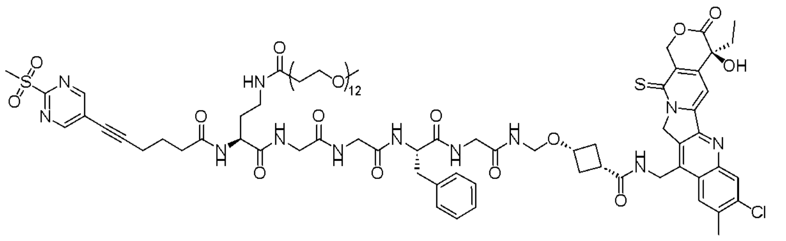 | 905.25 |
| 101 | 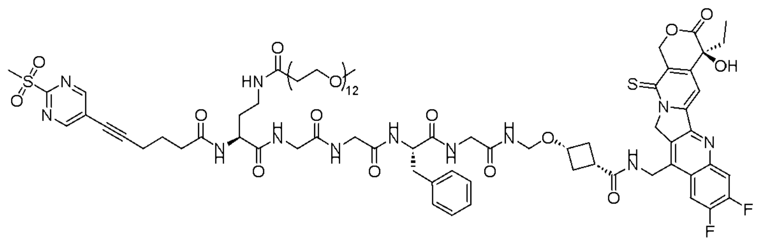 | 899.10 |

Example 102:

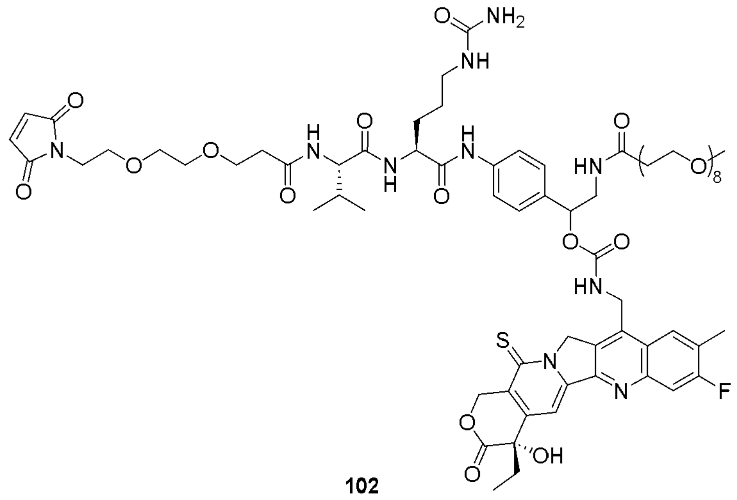

102

Synthetic route:

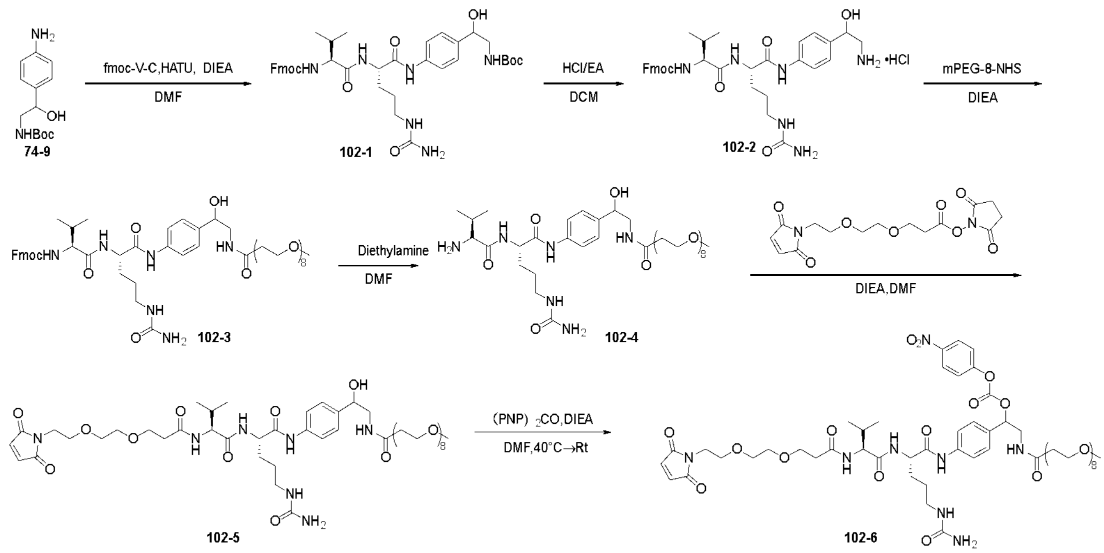


Synthesis of Intermediate 102-1:

Fmoc-V-C (2.36 g, 4.756 mmol) and HATU (2.17 g, 5.707 mmol) were dissolved in DMF (5 mL). After the reaction mixture was stirred for 5 minutes, The compound 74-9 (1.2 g, 4.756 mmol) and DIEA (1.84 g, 14.268 mmol) were added sequentially. The reaction proceeded at room temperature until completion. Purification by reverse-phase C-18 column chromatography (50% MeCN in $H_2O$) and concentration under reduced pressure were performed to obtain a white solid. LC-MS (ESI): m/z found $[M+H]^+$ = 731.6.

Synthesis of Intermediate 102-2:

The compound 102-1 (710 mg, 0.972 mmol) was dissolved in DMF (5 mL). HCl/EA (1.21 mL) was added. The reaction proceeded at room temperature for 4 hours. After completion, concentration under reduced pressure was performed to obtain a white solid (620 mg, 96% ). LC-MS (ESI): m/z found $[M+H]^+$ = 631.2.

Synthesis of Intermediate 102-3:

The compound 102-2 (1.8 g, 2.82 mmol) was dissolved in DMF (5 mL). m-PEG8-NHS ester (1.7 g, 2.82 mmol) and DIEA (0.93 mL, 5.64 mmol) were added. The reaction proceeded at room temperature until completion. Purification by

reverse-phase C-18 column chromatography and lyophilization were performed to obtain a colorless oily product (1.5 g, 51.9% ). LC-MS (ESI): m/z found $[M+H]^+$ = 1025.80.

Synthesis of Intermediate 102-4:

The compound 102-3 (1.5 g, 1.46 mmol) was dissolved in DMF. Diethylamine (150 μL) was added. The reaction proceeded at room temperature for 4 hours. Purification by reverse-phase column chromatography ($H_2O$:ACN = 1:4) was performed to obtain a product. LC-MS (ESI): m/z found $[M+H]^+$ = 803.70.

Synthesis of Intermediate 102-5:

The compound 102-4 (560 mg, 0.7 mmol) was dissolved in DMF (4 mL). Maleimide-PEG2-NHS ester (0.7 mmol) and DIEA (231 μL) were added. The reaction proceeded at room temperature for 3 hours. Purification by reverse-phase column chromatography ($H_2O$:ACN = 7:3) and concentration under reduced pressure were performed to obtain a product (350 mg, 48.1% ). LC-MS (ESI): m/z found $[M+H]^+$ = 1042.75.

Synthesis of Intermediate 102-6:

The compound 102-5 (170 mg, 0.020 mmol) and bis(4-nitrophenyl) carbonate ($(PNP)_2CO$, 199 mg, 0.076 mmol) were dissolved in DMF (3 mL). DIEA (51 μL) was added. The reaction proceeded at 40°C for 2 hours, then at room temperature overnight. Purification by reverse-phase column chromatography and lyophilization were performed to obtain a product (90 mg, 45.9% ).

Synthesis of Compound 102:

The compound 102-6 (30 mg, 0.022 mmol) was dissolved in DMF (2 mL). HOBt (4 mg, 0.029 mmol), DIEA (7.3 μL, 0.044 mmol), and the compound 11 (9 mg, 0.022 mmol) were added. The reaction proceeded at room temperature for 2.5 hours. After LC-MS monitoring indicated completion of the reaction, purification by preparative HPLC and lyophilization were perfomed to obtain a yellow solid product (1.94 mg, 6.5% ). LC-MS (ESI): m/z found $[M/2+H]^+$ = 748.05.

Example 102:

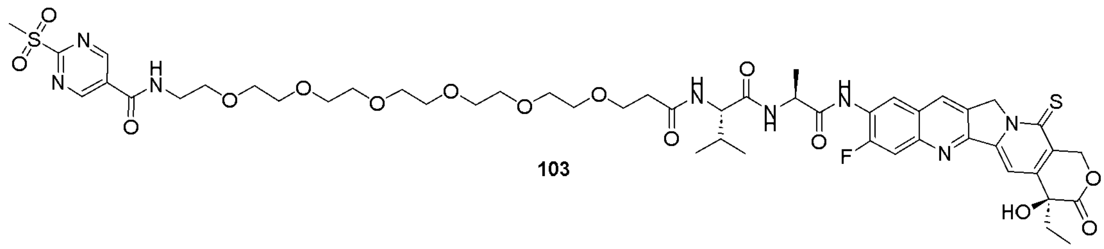

103

Synthetic route:

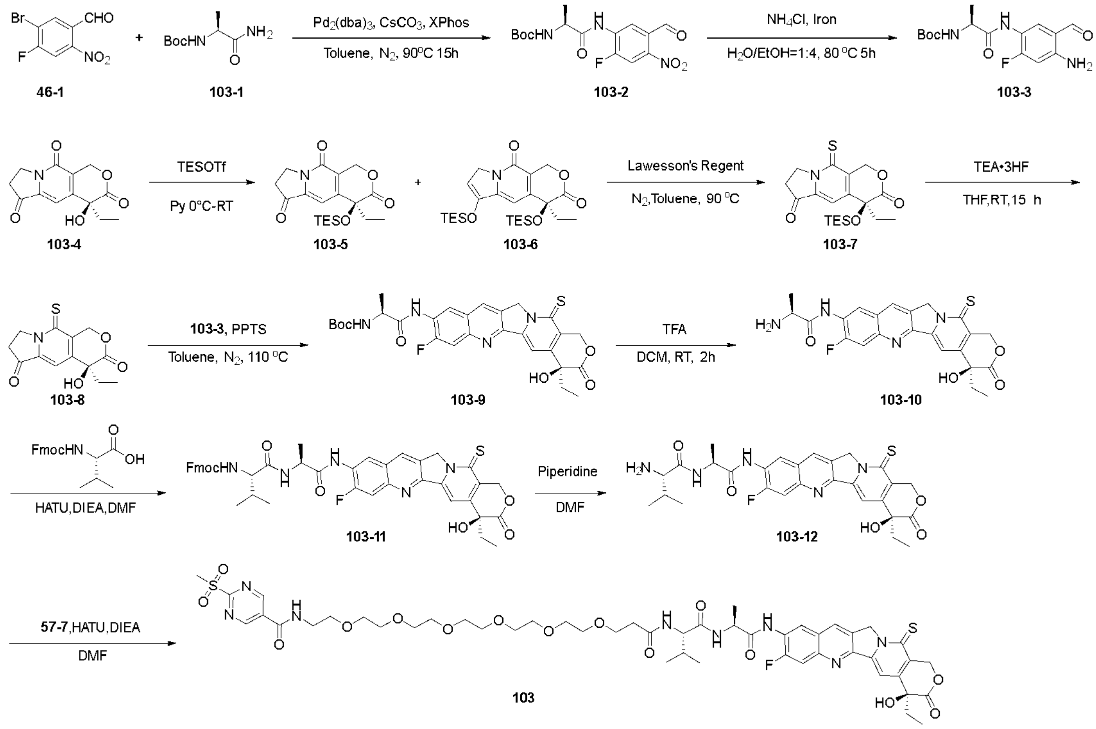


Synthesis of Intermediate 103-2:

At room temperature, the compound 46-1 (500 mg, 2.016 mmol), N-(tert-Butoxycarbonyl)-L-alaninamide (103-1, 456 mg, 2.419 mmol), $Pd_2(dba)_3$ (93 mg, 0.1008 mmol), $Cs_2CO_3$ (1314 mg, 4.032 mmol), and XPhos (192 mg, 0.4032 mmol) were dissolved in toluene. The atmosphere was purged with nitrogen, and the reaction mixture was heated to 90°C, and the reaction proceeded overnight. TLC monitoring indicated completion of the reaction. The reaction mixture was extracted with ethyl acetate/water. The organic phase was washed with saturated NaCl solution, dried over anhydrous $Na_2SO_4$, and concentrated. Purification by normal-phase column chromatography (petroleum ether:ethyl acetate = 77.7:22.3) was performed to obtain a product (430 mg, 60% ). LC-MS (ESI): m/z found $[M-H]^-$ = 354.0.

Synthesis of Intermediate 103-3:

At room temperature, the compound 103-2 (200 mg, 0.563 mmol), iron powder (126 mg, 2.252 mmol), and ammonium chloride (49 mg, 0.912 mmol) were dissolved in a mixture of $H_2O$ (2 mL) and EtOH (8 mL). The reaction mixture was heated to 80°C, and the reaction proceeded for 5 hours. LC-MS monitoring indicated completion of the reaction. The reaction mixture was filtered through Celite. The filtrate was spin-dried, concentrated and purified by normal-phase column chromatography (petroleum ether:ethyl acetate = 81:19) to obtain a compound(103 mg, 57% yield). MS (ESI): m/z found $[M-H]^-$ = 324.0.

Synthesis of Intermediate 103-5/103-6:

In an ice bath, a compound 103-4 (1 g, 3.799 mmol) was dissolved in dry pyridine (10 mL). Triethylsilyl trifluoromethanesulfonate (TESOTf, 8.6 mL, 37.99 mmol) was added. The reaction proceeded in the ice bath for 10 minutes, then the reaction mixture was warmed to room temperature, and the reaction proceeded overnight. LC-MS monitoring indicated completion of the reaction. The reaction mixture was concentrated under reduced pressure to remove a large amount of pyridine. The residue was dissolved in dichloromethane, extracted with water for three times, washed with saturated brine once, dried over anhydrous $Na_2SO_4$, and concentrated under reduced pressure. Purification by normal-phase column chromatography was performed to obtain a mixture of 103-5/103-6 (1.35 g, 79% ). MS (ESI): m/z found $[M+H]^+$ = 378.0 / 492.2.

Synthesis of Intermediate 103-7:

At room temperature, the mixture 103-5/103-6 (440 mg, 0.896 mmol) was dissolved in toluene (10 mL).

Lawesson's reagent (435 mg, 1.075 mmol) was added. The atmosphere was purged with nitrogen, and the reaction mixture was heated to 90°C, and the reaction peoceeded for 4 hours. TLC monitoring indicated completion of the reaction. The reaction mixture was concentrated and purified by normal-phase column chromatography (petroleum ether:ethyl acetate = 95:5) to obtain a compound (153 mg, 43% yield). MS (ESI): m/z found $[M+H]^+$ = 394.0.

Synthesis of Intermediate 103-8:

At room temperature, the compound 103-7 (169 mg, 0.430 mmol) was dissolved in dry tetrahydrofuran (10 mL). Triethylamine trihydrofluoride (175 μL, 1.075 mmol) was added. The reaction proceeded overnight. TLC monitoring indicated completion of the reaction. The reaction mixture was diluted with DCM, washed with water for three times and washed with saturated NaCl solution once, dried over anhydrous $Na_2SO_4$, and concentrated. Purification by column chromatography (petroleum ether:ethyl acetate = 71:29) and distillation under reduced pressure were performed to obtain a product (73 mg, 61% yield).

Synthesis of Intermediate 103-9:

At room temperature, the compounds 103-3 (34 mg, 0.105 mmol), 103-8 (30 mg, 0.105 mmol), and pyridinium p-toluenesulfonate (PPTS, 1.3 mg, 0.005 mmol) were dissolved in toluene (10 mL). The reaction mixture was heated to 110°C, and the reaction proceeded for 5 hours. TLC monitoring indicated completion of the reaction. The reaction mixture was concentrated, dissolved in a small amount of DCM, and purified by column chromatography (petroleum ether:ethyl acetate = 78:22) to obtain a compound (32 mg, 54% yield ). MS (ESI): m/z found $[M+H]^+$ = 569.0.

Synthesis of Intermediate 103-10:

At room temperature, the compound 103-9 (68 mg, 0.120 mmol) was dissolved in dry dichloromethane (5 mL). Trifluoroacetic acid (TFA, 500 μL) was added. The reaction proceeded for 1 hour. TLC monitoring indicated completion of the reaction. The reaction mixture was concentrated, dissolved in a small amount of DMF, and purified by prep-HPLC ($H_2O:CH_3CN$ = 75:25, 0.1% formic acid) to obtain a compound (12 mg, 22% yield ). MS (ESI): m/z found $[M+H]^+$ = 469.30.

Synthesis of Intermediate 103-11:

At room temperature, Fmoc-L-valine (2.7 mg, 0.0079 mmol), HATU (4.5 mg, 0.0079 mmol), and DIPEA (3.3 μL, 0.0189 mmol) were dissolved in dry DMF. After the reaction proceeded for 30 minutes, the compound 103-10 (3.0 mg, 0.0064 mmol) was added. The reaction proceeded at room temperature overnight. LC-MS monitoring indicated completion of the reaction. The reaction mixture was purified by prep-HPLC ($H_2O:CH_3CN$ = 41.2:58.8) and lyophilized to obtain a compound (2.74 mg, 54% yield). MS (ESI): m/z found $[M+H]^+$ = 790.50.

Synthesis of Intermediate 103-12:

At room temperature, the compound 103-11 (30 mg, 0.038 mmol) was dissolved in DMF (3 mL). Piperidine (1 mL) was added. The reaction proceeded at room temperature for 2 hours. After completion, purification by prep-HPLC and lyophilization were performed to obtain a yellow solid product (12 mg, 56% ). LC-MS (ESI): m/z found $[M+H]^+$ = 568.32.

Synthesis of Compound 103:

The compound 57-7 (12 mg, 0.22 mmol) was dissolved in DMF. HATU (9 mg, 0.023 mmol) and DIEA (8 mg, 0.062 mmol) were added. The reaction proceeded at room temperature for 2 hours. Purification by prep-HPLC and lyophilization were perfomed to obtain a compound 103. LC-MS (ESI): m/z found $[M+H]^+$ = 1087.4.

Example 104:

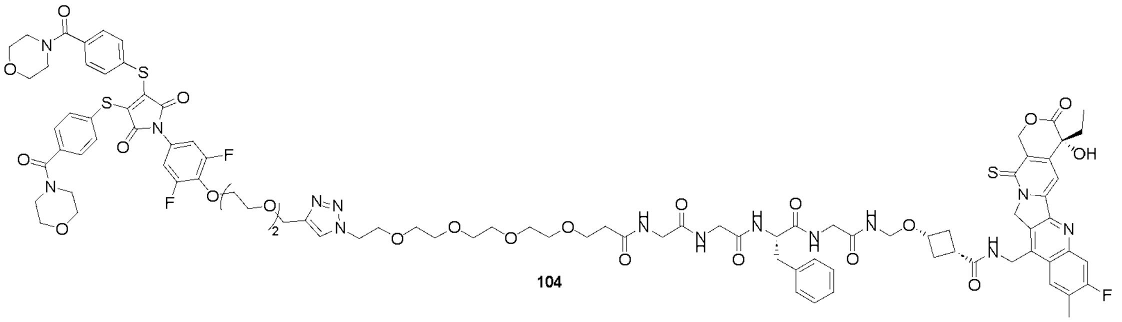

104

Synthetic route:

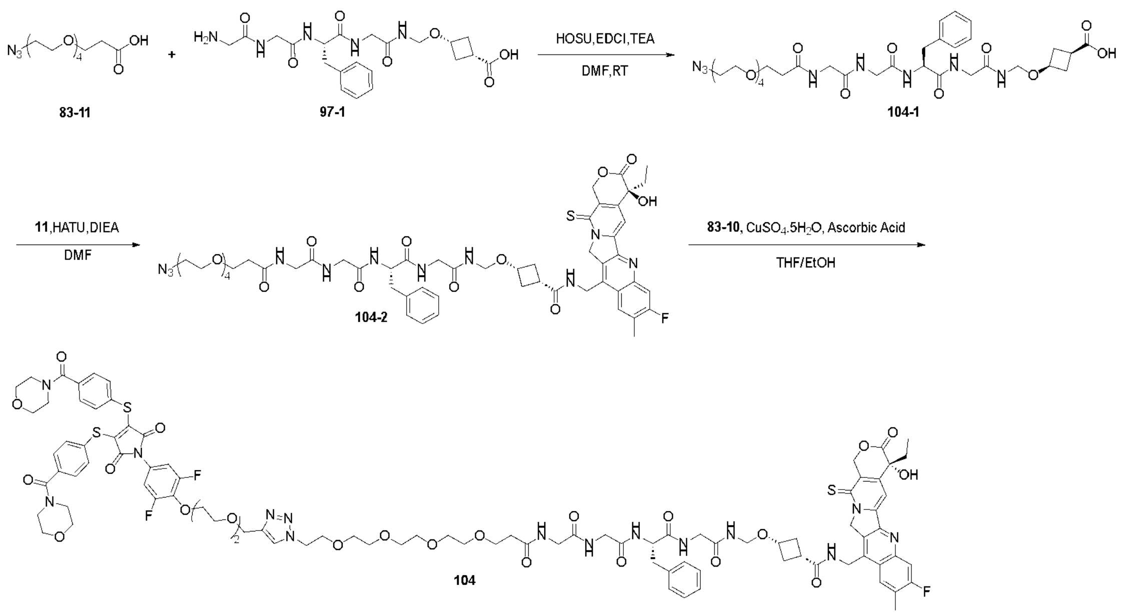


83-11 97-1 104-1 104-2 104

Synthesis of Intermediate 104-1:

At room temperature, the compound 83-11 (31 mg, 0.106 mmol) was dissolved in DMF (5 mL). N-Hydroxysuccinimide (HOSu, 24 mg, 0.127 mmol) and 1-Ethyl-3-(3-dimethylaminopropyl)carbodiimide hydrochloride (EDCI, 16 mg, 0.127 mmol) were added. After the reacton proceeded for 2 hours, the compound 97-1 (49 mg, 0.106 mmol) and triethylamine (TEA, 16 μL, 0.117 mmol) were added sequentially. The reaction proceeded at room temperature for 1 hour. After LC-MS monitoring indicated completion of the reaction, the reaction mixture was purified by prep-HPLC and lyophilized to obtain a colorless oily product (21 mg, 27% ). LC-MS (ESI): m/z found $[M-H]^-$ = 735.40.

Synthesis of Intermediate 104-2:

In an ice bath, the compound 104-1 (21 mg, 0.029 mmol) was dissolved in DMF (3 mL). 2-(7-Azabenzotriazol-1-yl)-N,N,N',N'-tetramethyluronium hexafluorophosphate (HATU, 13 mg, 0.034 mmol) and N,N-Diisopropylethylamine (DIEA, 9.9 μL, 0.057 mmol) were added. After the reaction proceeded for 15 minutes, the compound 11 (12 mg, 0.029 mmol) was added. After completion, purification by prep-HPLC and concentration under reduced pressure were performed to obtain a yellow solid (17 mg, 52% ). LC-MS (ESI): m/z found $[M+H]^+$ = 1144.80.

Synthesis of Compound 104:

The compounds 83-10 (9.8 mg, 0.012 mmol) and 104-2 (17 mg, 0.015 mmol) were dissolved in a mixture of THF/EtOH (1:3). Copper(II) sulfate pentahydrate ($CuSO_4 \cdot 5H_2O$, 3.1 mg, 0.012 mmol) and ascorbic acid (2.2 mg, 0.012 mmol) were added sequentially. Under a nitrogen atmosphere, the reaction proceeded at room temperature for 1.5 hours.

Purification by prep-HPLC and concentration under reduced pressure were perfomed to obtain a yellow solid (6.93 mg, 29.8% ). LC-MS (ESI): m/z found $[M/2+H]^+$ = 970.20.

Example 105-106:

In addition to replacing with the corresponding starting materials, examples **105-106** were synthesized following the procedures described in Example 104. Their structures and LC-MS (ESI) data are shown in Table 6.

Table 6 Structural formulas and MS (ESI) data for Examples **105-106**

| Examples | Structural formulas | MS (ESI) $[M/2+H]^+$ |
|---|---|---|
| 105 | 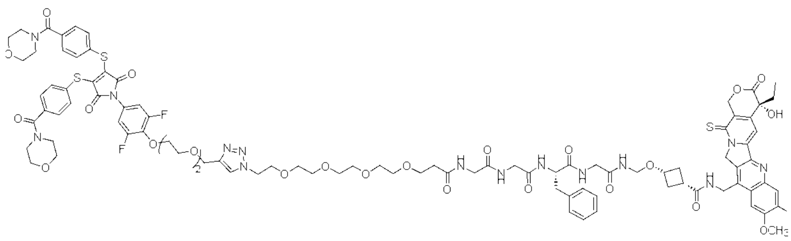 | 955.3 |
| 106 | 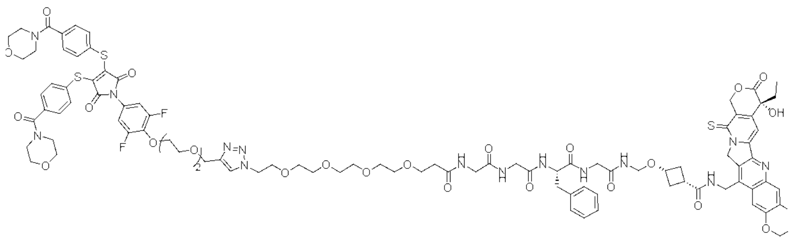 | 950.5 |

Example 107-109:

Synthetic route:

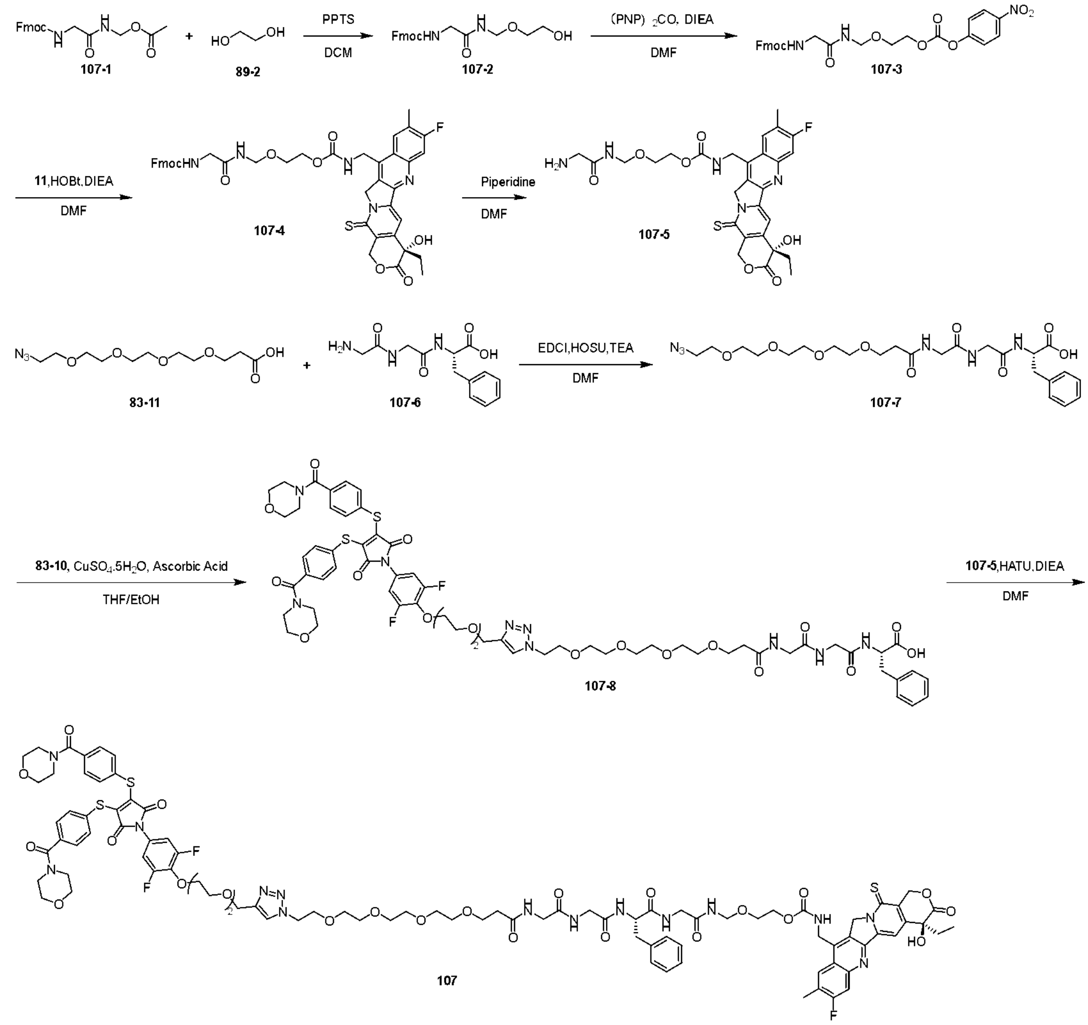


Synthesis of Intermediate 107-2:

At room temperature, the compound 107-1 (200 mg, 0.543 mmol) was dissolved in DCM (5 mL). the compound 89-2 (303 μL, 5.429 mmol) and pyridinium p-toluenesulfonate (PPTS, 273 mg, 1.086 mmol) were added sequentially. The reaction proceeded at room temperature overnight. After completion, the reaction mixture was concentrated under reduced pressure. The residue was dissolved in a small amount of DMF and purified by reverse-phase column chromatography. Concentration under reduced pressure was performed to obtain a white solid product (200 mg, 99% ). LC-MS (ESI): m/z found $[M+Na]^+$ = 393.0.

Synthesis of Intermediate 107-3:

In an ice bath, the compound 107-2 (20 mg, 0.059 mmol) was dissolved in DMF (2 mL). Bis(4-nitrophenyl) carbonate ($(PNP)_2CO$, 6.6 mg, 0.216 mmol) and N,N-diisopropylethylamine (DIEA, 8.3 μL, 0.054 mmol) were added sequentially. The reaction mixture was stirred in the ice bath for 10 minutes, then warmed to room temperature, and the reaction proceeded overnight. Purification by prep-HPLC and concentration under reduced pressure were performed to obtain a white solid product (9 mg, 31%). 1H NMR (600 MHz, DMSO-d6) δ 8.73 (t, J = 6.7 Hz, 1H), 8.31 (d, J = 9.1 Hz, 2H), 7.90 (d, J = 7.6 Hz, 2H), 7.73 (dd, J = 18.5, 8.3 Hz, 2H), 7.63 - 7.54 (m, 3H), 7.42 (t, J = 7.4 Hz, 2H), 7.33 (t, J = 7.4 Hz, 2H), 4.61 (d, J = 6.7 Hz, 2H), 4.38 - 4.33 (m, 2H), 4.29 (d, J = 7.1 Hz, 2H), 4.23 (dd, J = 14.1, 6.8 Hz, 1H), 3.70 - 3.63 (m, 4H).

Synthesis of Intermediate 107-4:

The compound 107-3 (9 mg, 0.018 mmol) was dissolved in DMF (3 mL). 1-Hydroxybenzotriazole (HOBt, 2.8 mg, 0.018 mmol) was added, and the reaction proceeded at room temperature for 15 minutes. The compound 11 (8 mg, 0.018 mmol) and N,N-diisopropylethylamine (DIEA, 5.9 μL, 0.034 mmol) were then added sequentially. The reaction proceeded

at room temperature for additional 2 hours. Purification by prep-HPLC and lyophilization were performed to obtain a compound. LC-MS (ESI): m/z found $[M+H]^+$ = 822.50.

Synthesis of Intermediate 107-7:

In an ice bath, the compound 83-11 (100 mg, 0.343 mmol) was dissolved in DMF (3 mL). 1-Ethyl-3-(3-dimethylaminopropyl)carbodiimide hydrochloride (EDCI, 79 mg, 0.412 mmol) and N-Hydroxysuccinimide (HOSu, 47 mg, 0.412 mmol) were added sequentially. The reaction mixture was warmed to room temperature, and the reaction proceeded for 1 hour. The compound 107-6 (96 mg, 0.343 mmol) and triethylamine (TEA, 52 μL, 0.377 mmol) were then added. The reaction proceeded at room temperature for 2 hours. After substantial completion, purification by prep-HPLC and lyophilization were performed to obtain a colorless oily product (28.1 mg, 15% ). LC-MS (ESI): m/z found $[M+Na]^+$ = 553.50.

Synthesis of Intermediate 107-8:

The compound 83-10 (34 mg, 0.042 mmol) and the compound 107-7 (28.1 mg, 0.051 mmol) were dissolved in a mixture of THF (2 mL) and EtOH (6 mL). Copper(II) sulfate pentahydrate ($CuSO_4 \cdot 5H_2O$, 11 mg, 0.042 mmol) and ascorbic acid (7.4 mg, 0.042 mmol) were added sequentially. Under a nitrogen atmosphere, the reaction proceeded at room temperature for 2 hours. Purification by prep-HPLC and lyophilization were performed to obtain a yellow solid. LC-MS (ESI): m/z found $[M+H]^+$ = 1346.80.

In addition to replacing with the corresponding starting materials, the remaining intermediates and Examples 107-109 were synthesized following the procedures described in Example 77 and Example 88. Their structures and LC-MS data are shown in Table 7.

Table 7 Structural formulas and MS (ESI) data for Examples 105-106

| Example | Structural formulas | MS (ESI) $[M/2+H]^+$ |
|---|---|---|
| 107 | 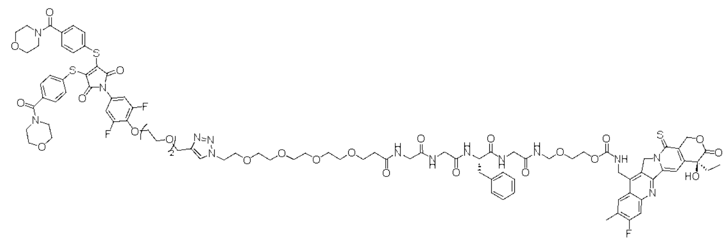 | 964.6 |
| 108 | 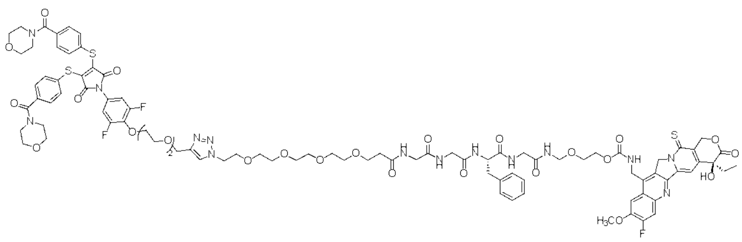 | 972.5 |
| 109 | 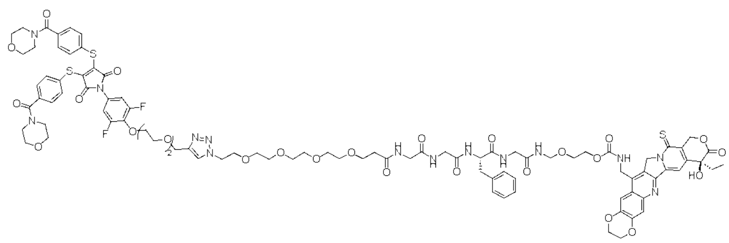 | 977.6 |

Example 10: HER2 ADC Conjugation

The interchain disulfide bonds of Trastuzumab (10 mg/mL) were reduced by adding 1 mM EDTA (Invitrogen, Cat#: AM9260G) and 8 molar equivalents of TECP (Thermo, Cat#: 77720) to the solution. The mixture was stirred at 37°C, 250 rpm for 2.5 hours.

The Light Chain Sequence of Trastuzumab (SEQ ID NO.1) :

DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGQGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GEC

The Heavy Chain Sequence of Trastuzumab (SEQ ID NO.2) :

EVQLVESGGGLVQPGGSLRLSCAASGFNIKDTYIHWVRQAPGKGLEWVA RIYPTNGYTRYADSVKGRFTISADTSKNTAYLQMNSLRAEDTAVYYCSRWGG DGFYAMDYWGQGTLVTVSSASTKGPSVFPLAPSSKSTSGGTAALGCLVKDYF PEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPE

VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDKS RWQQGNVFSCSVMHEALHNHYTQKSLSLSPGK

The mixture was cooled to the target temperature of 4°C, and 10 drug equivalents of the compound per mole were added as a 10% (v/v) DMSO solution (Sigma, Cat. No.: D2660). After 3 hours, the Antibody-Drug Conjugate (ADC) was buffer-exchanged into PBS using ultrafiltration. The numbers and specific structures of the resulting ADCs are listed in Table 8.

Table 8: Identification Numbers and Structural Formulas of Ligand-Drug Conjugate

| ADC Number | ADC Structural Formulas |
|---|---|
| HER2-ADC-1 | 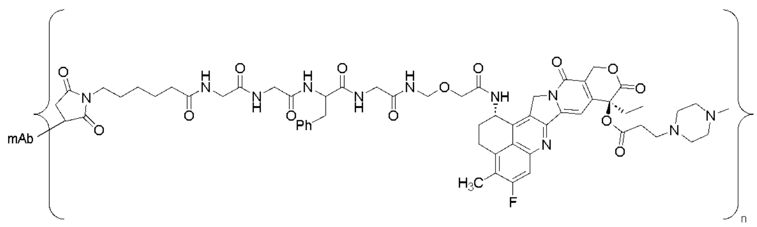 |
| HER2-ADC-2 | 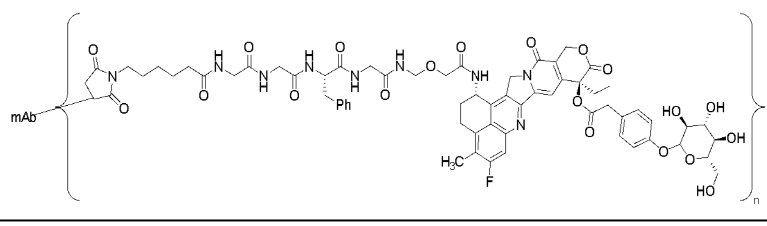 |

(continued)

| ADC Number | ADC Structural Formulas |
|---|---|
| HER2-ADC-3 | 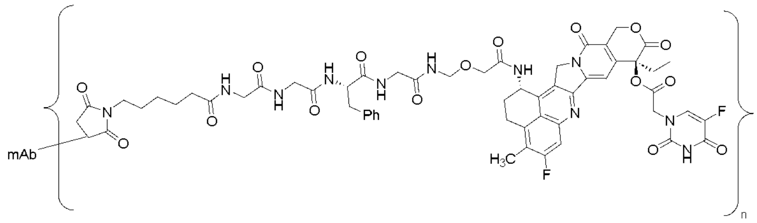 |
| HER2-ADC-4 | 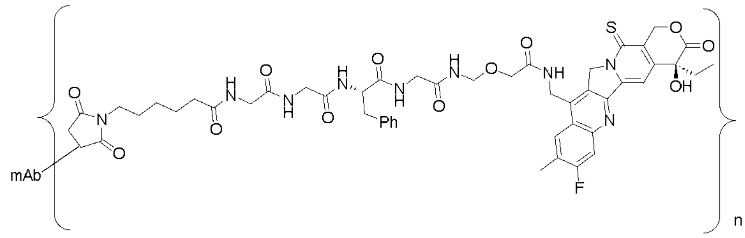 |
| HER2-ADC-5 | 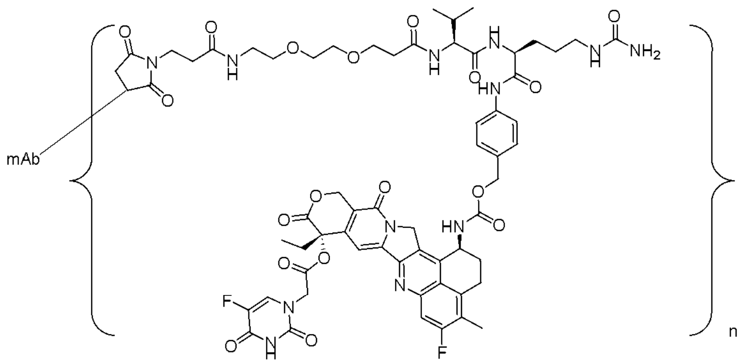 |
| HER2-ADC-6 | 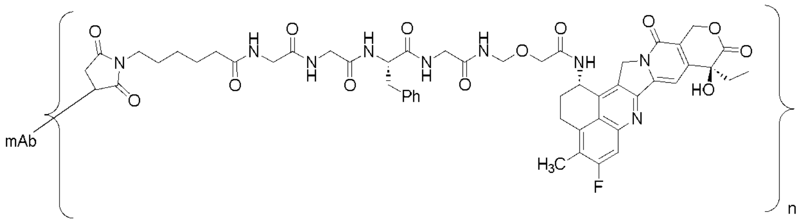 |
| Note: In Table 8, mAb refers to Trastuzumab in this example. At each occurrence, n represents an integer or decimal between 1 and 10, represents the average number of conjugated units per antibody for each selected drug-linker compound, and n can also be expressed as DAR. | |

Following ultrafiltration, the concentration of the ADC conjugate was determined by measuring the absorbance at 280 nm and 365 nm. Whether the conjugation successful or not was confirmed by HPLC-HIC analysis. Antibody purity was assessed by SEC-HPLC, and the DAR value was determined by LC-MS. The results are shown in Table 9.

Table 9: Conjugation Results of HER2 Antibody-based ADCs

| ADC Number | DAR | ADC Number | DAR |
|---|---|---|---|
| HER2-ADC-1 | 8.00 | HER2-ADC-2 | 7.85 |
| HER2-ADC-3 | 7.84 | HER2-ADC-4 | 8.0 |
| HER2-ADC-5 | 8.0 | HER2-ADC-6 | 8.0 |

Example 111: NECTIN-4 ADC Conjugation

The interchain disulfide bonds of the PADCEV monoclonal antibody (10 mg/mL) were reduced by adding 1 mM EDTA (Invitrogen, Cat#: AM9260G) and 8 molar equivalents of TECP (Thermo, Cat#: 77720) to the solution. The mixture was stirred at 37°C, 250 rpm for 2.5 hours.

The Light Chain Sequence of PADCEV (SEQ ID NO.3) :

DIQMTQSPSSVSASVGDRVTITCRASQGISGWLAWYQQKPGKAPKFLIYA
ASTLQSGVPSRFSGSGSGTDFTLTISSLQPEDFATYYCQQANSFPPTFGGGTKVE
IKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNR
GEC

The Heavy Chain Sequence of PADCEV (SEQ ID NO.4) :

EVQLVESGGGLVQPGGSLRLSCAASGFTFSSYNMNWVRQAPGKGLEWV
SYISSSSSTIYYADSVKGRFTISRDNAKNSLSLQMNSLRDEDTAVYYCARAYYY
GMDVWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSN
TKVDKRVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQ
QGNVFSCSVMHEALHNHYTQKSLSLSPGK

The mixture was cooled to the target temperature of 4°C, and 10 drug equivalents of the compound per mole were added as a 10% (v/v) DMSO solution (Sigma, Cat.#:D2660). After 3 hours, the antibody-drug conjugate was buffer-exchanged into PBS using ultrafiltration. The names and specific structures of the conjugates are shown in Table 10.

Table 10 Names and Structural Formulas of Ligand-Drug Conjugates

| ADC Number | ADC Structural Formulas |
|---|---|
| NECTIN 4-ADC-1 | 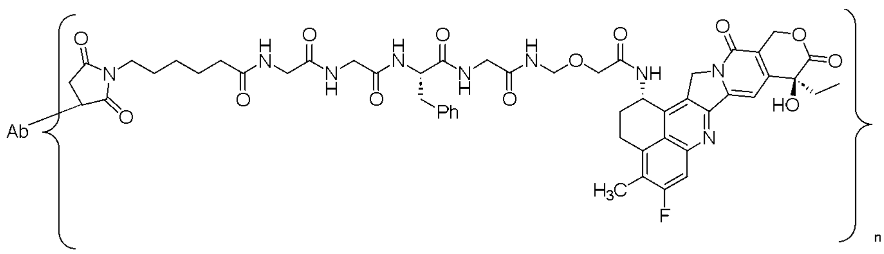 |
| NECTIN 4-ADC-2 | 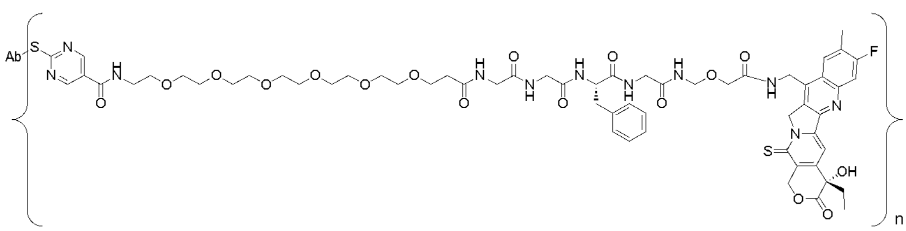 |

(continued)

| ADC Number | ADC Structural Formulas |
|---|---|
| NECTIN 4-ADC-3 | 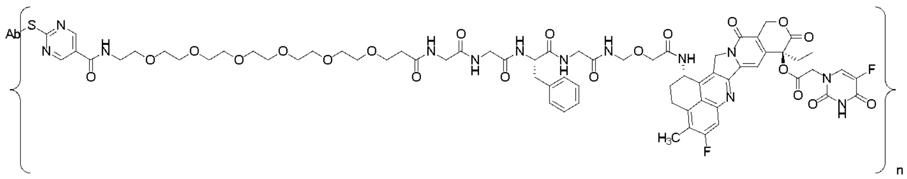 |
| NECTIN4 -ADC-4 | 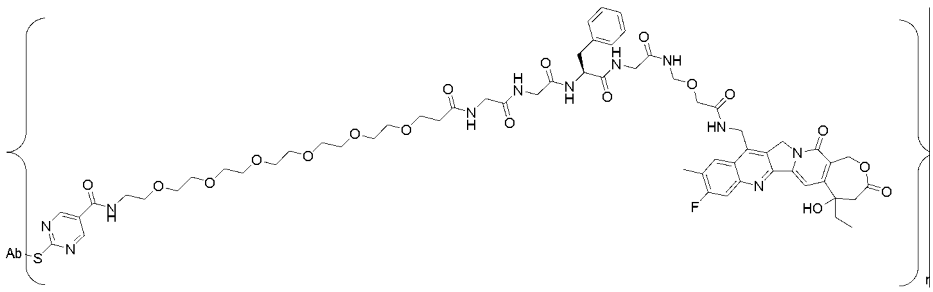 |
| Note: In Table 10, Ab refers to the PADCEV antibody in this example. At each occurrence, n represents an integer or decimal between 1 and 10, indicating the average number of conjugated units per antibody for each selected drug-linker compound. n is also expressed as DAR. | |

After ultrafiltration was completed, the concentration of the ADC conjugate was determined using absorbance values at 280 nm and 365 nm. Whether the conjugation successful or not was confirmed by HPLC-HIC analysis. Antibody purity was assessed by SEC-HPLC, and the DAR value was determined by LC-MS. The results are shown in Table 11.

Table 11 Conjugation Results of the NECTIN4 Antibody-Based ADC

| ADC Number | DAR | ADC Number | DAR |
|---|---|---|---|
| NECTIN4-ADC-1 | 8.0 | NECTIN4-ADC-2 | 7.99 |
| NECTIN4-ADC-3 | 7.62 | NECTIN4-ADC-4 | 7.51 |

Example 112: B7H3 ADC Conjugation

1. Solution Preparation:

    1) 0.5 M EDTA was diluted to 1 mM EDTA-PBS buffer: 20 μL EDTA + 10 mL PBS.
    2) 0.5 M TCEP was diluted to 5 mM: 10 μL TCEP + 990 μL EDTA-PBS.
    3) A 10% DMSO solution was prepared: 100μL DMSO + 900μL EDTA-PBS.
    4) The antibody Ifinatamab was diluted to 10 mg/mL.

2. TCEP Reduction for Disulfide Bond Cleavage:
For synthesizing an ADC with a target DAR of 8, the antibody and TCEP were mixed at a molar ratio of 1:8.
3. The mixture was incubated at 37°C for 2 hours in a shaker (210-230 rpm) and then placed on ice.
4. Linker-Payload Dissolution:
The linker-payload (referring to the linker and small-molecule drug moity of the ligand-drug conjugate, excluding the ligand itself) was dissolved in pure DMSO to a concentration of 5 mg/mL.
5. The linker-payload was added into the antibody at a molar ratio of 1:10 (antibody : linker).
6. The mixture was homogenized for 2 hours using a rotary mixer.
7. The mixture was desalted using a desalting column.
8. For the samples obtained after desalination, the concentration of the ADC conjugate was determined using the BCA method.
9. Whether the conjugation successful or not was confirmed by HPLC-HIC analysis.

10. Antibody purity was analyzed by SEC-HPLC.
11. The DAR value was measured by LC-MS.

The Heavy Chain Sequence of B7H3-targeting antibody Ifinatamab SEQ ID NO: 5

QVQLVQSGAEVKKPGSSVKVSCKASGYTFTNYVMHWVRQAPGQGLEW MGYINPYNDDVKYNEKFKGRVTITADESTSTAYMELSSLRSEDTAVYYCARW GYYGSPLYYFDYWGQGTLVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKRVEPKSCDKTHTCPPCPAPELLGGPSVFLFPPKPKDTLMISR TPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREEM TKNQVSLTCLVKGFYPSDIAVEWESNGQPENNYKTTPPVLDSDGSFFLYSKLT VDKSRWQQGNVFSCSVMHEALHNHYTQKSLSLSPGK

The Light Chain Sequence of B7H3-targeting antibody Ifinatamab SEQ ID NO: 6

EIVLTQSPATLSLSPGERATLSCRASSRLIYMHWYQQKPGQAPRPLIYATSN LASGIPARFSGSGSGTDFTLTISSLEPEDFAVYYCQQWNSNPPTFGQGTKVEIKR TVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC

The names and specific structures of the resulting ADC are shown in Table 12.

Table 12 Names and Structural Formulas of Ligand-Drug Conjugates

| ADC Number | ADC Structural Formulas |
|---|---|
| **B7H3-ADC-1** | 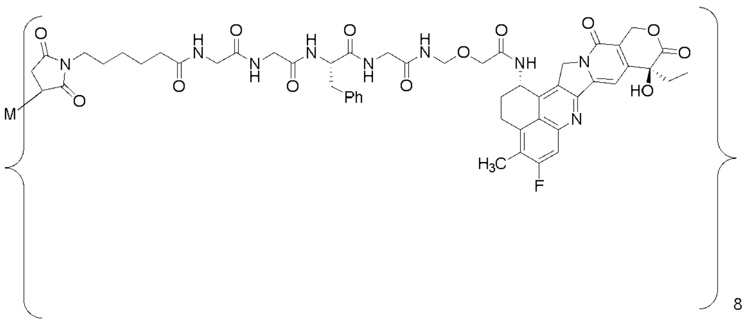 |
| **B7H3-ADC-2** | 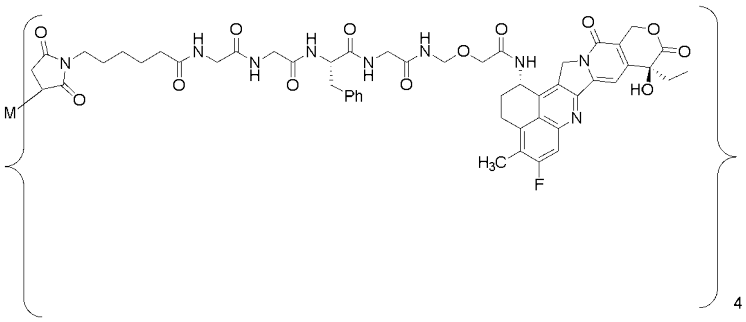 |

(continued)

| ADC Number | ADC Structural Formulas |
|---|---|
| **B7H3-ADC-3** | 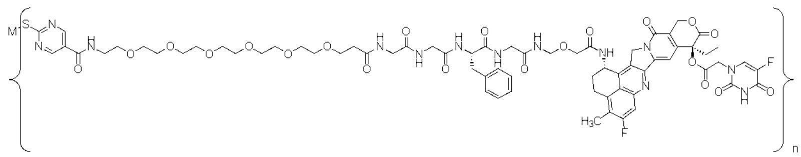 |
| **B7H3-ADC-4** | 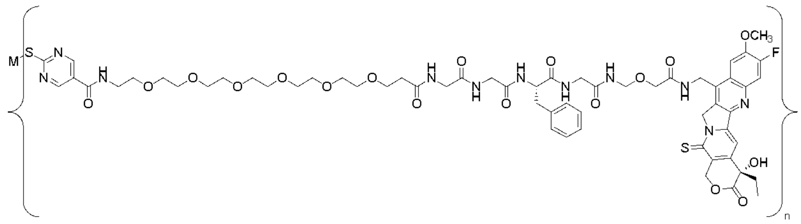 |
| **B7H3-ADC-5** | 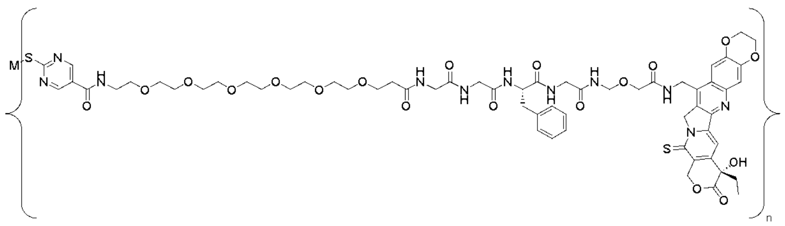 |
| **B7H3-ADC-6** | 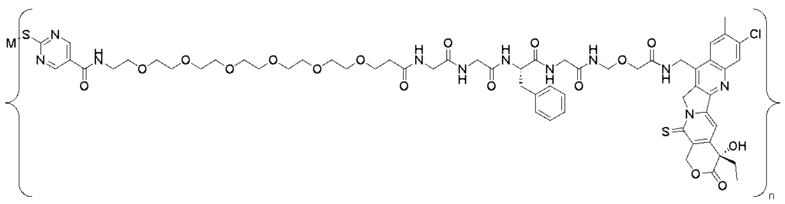 |
| **B7H3-ADC-7** | 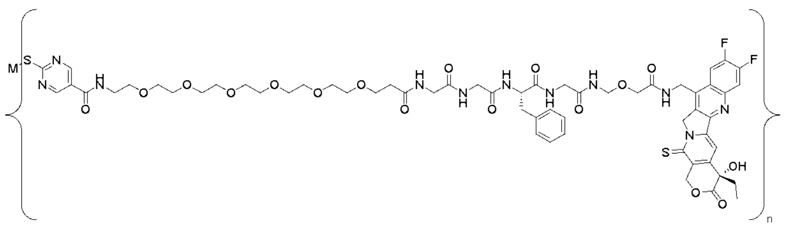 |
| **B7H3-ADC-8** | 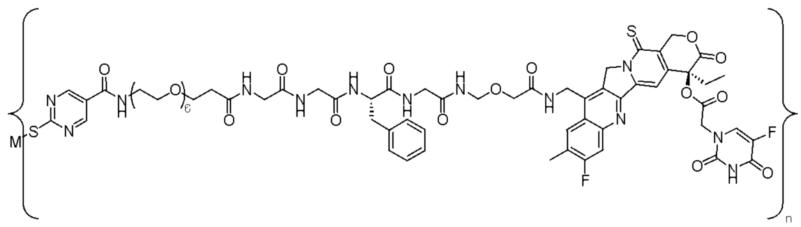 |
| **B7H3-ADC-9** | 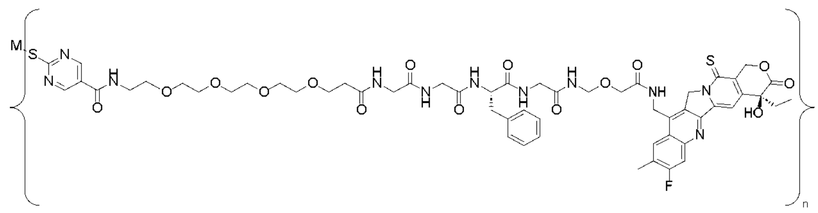 |

(continued)

| ADC Number | ADC Structural Formulas |
|---|---|
| **B7H3-ADC-10** | 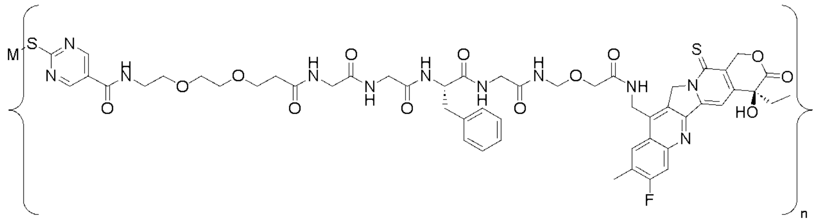 |
| **B7H3-ADC-11** | 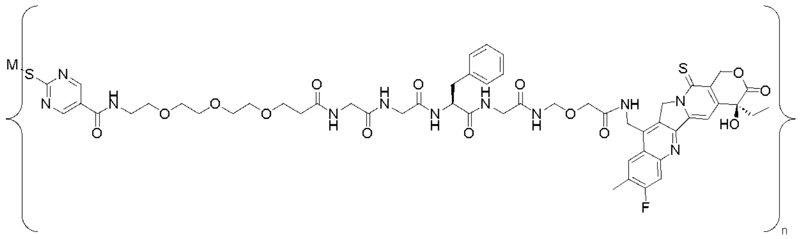 |
| **B7H3-ADC-12** | 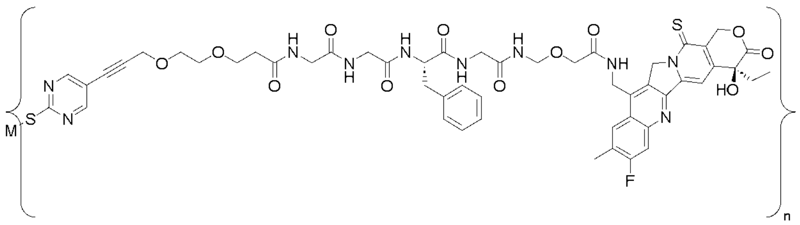 |
| **B7H3-ADC-13** | 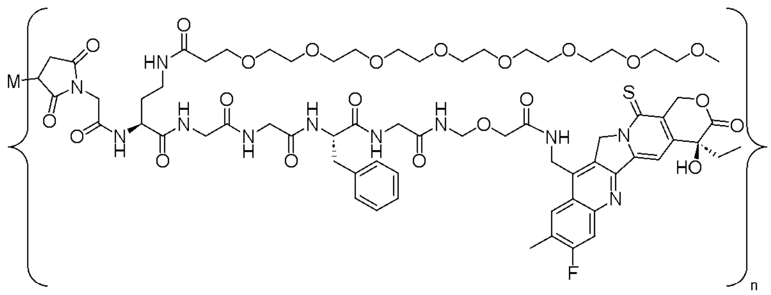 |
| **B7H3-ADC-14** | 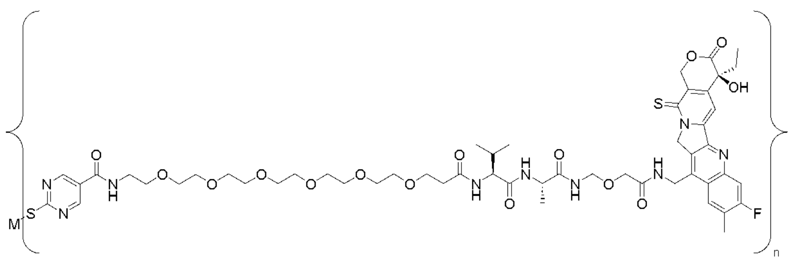 |
| **B7H3-ADC-15** | 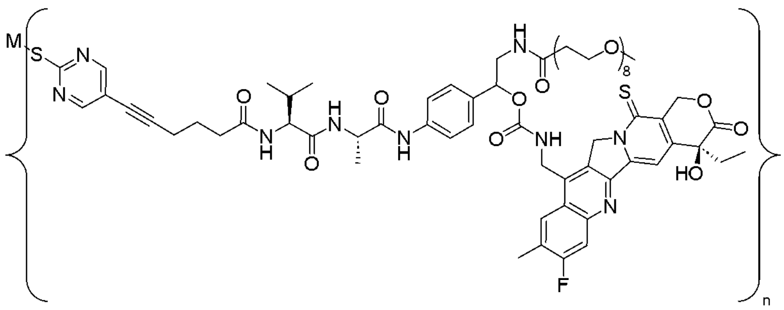 |

(continued)

| ADC Number | ADC Structural Formulas |
|---|---|
| **B7H3-ADC-16** | 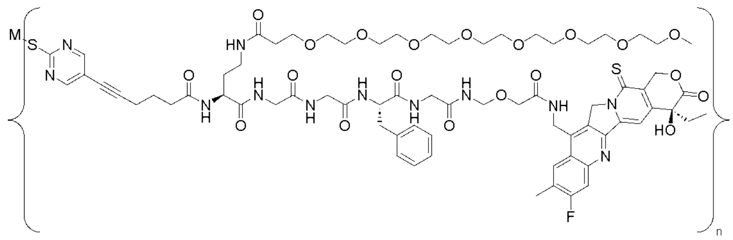 |
| **B7H3-ADC-17** | 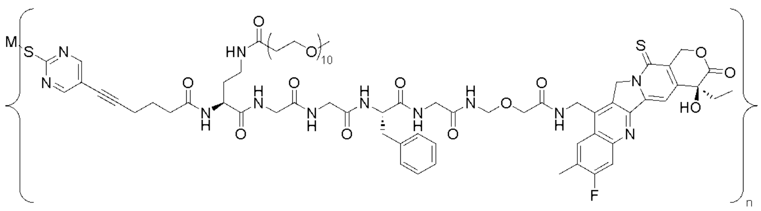 |
| **B7H3-ADC-18** | 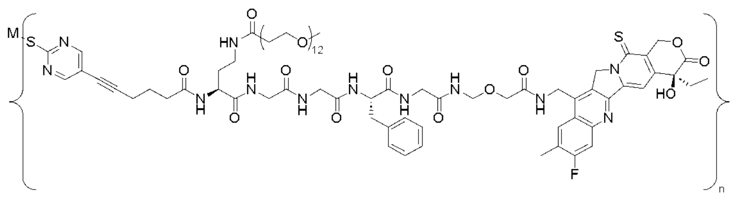 |
| **B7H3-ADC-19** | 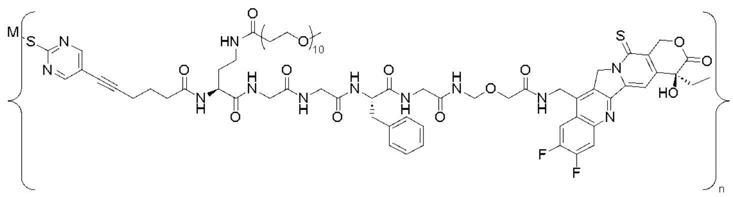 |
| **B7H3-ADC-20** | 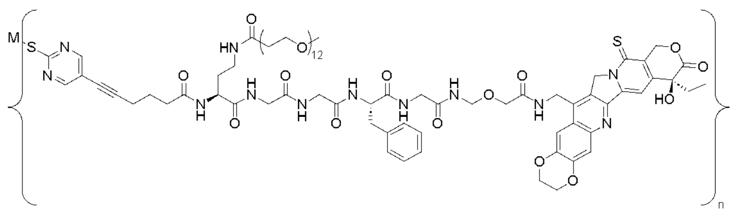 |
| **B7H3-ADC-21** | 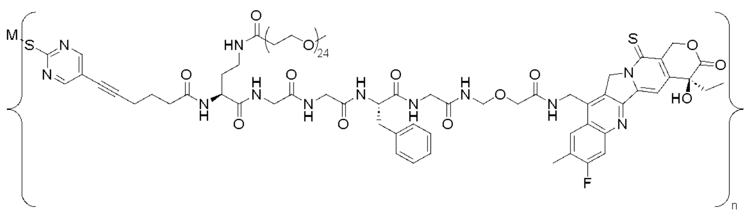 |
| **B7H3-ADC-22** | 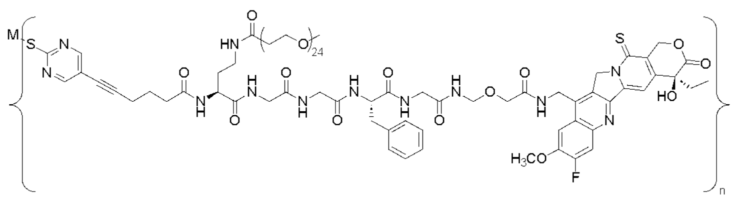 |

(continued)

| ADC Number | ADC Structural Formulas |
|---|---|
| **B7H3-ADC-23** | 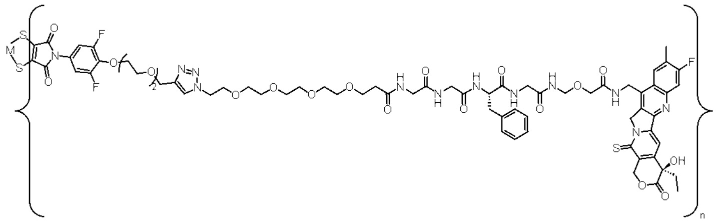 |
| **B7H3-ADC-24** | 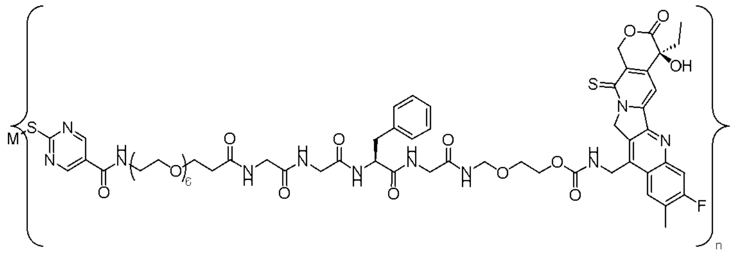 |
| **B7H3-ADC-25** | 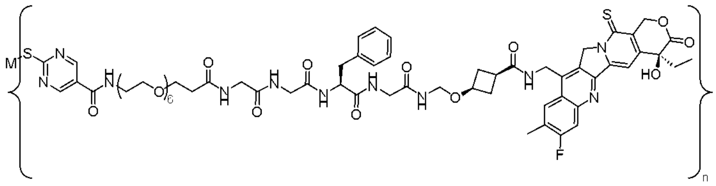 |
| **B7H3-ADC-26** | 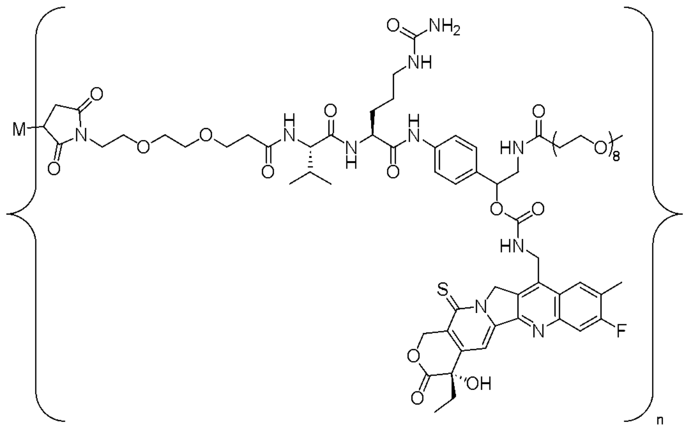 |
| **B7H3-ADC-27** | 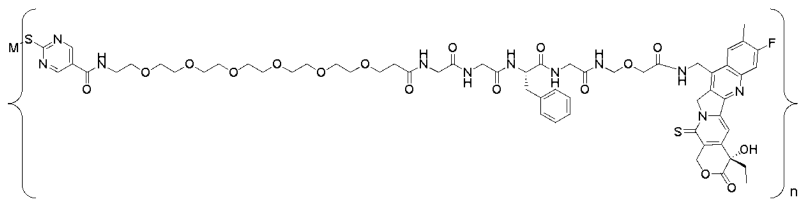 |
| **B7H3-ADC-28** | 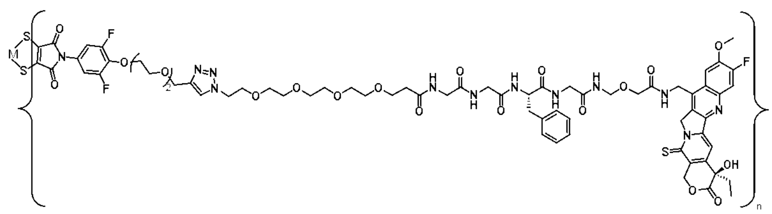 |

(continued)

| ADC Number | ADC Structural Formulas |
|---|---|
| **B7H3-ADC-29** | 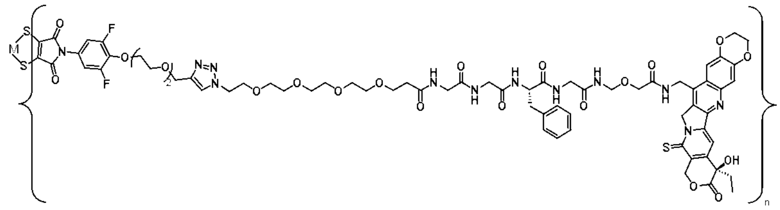 |
| **B7H3-ADC-30** | 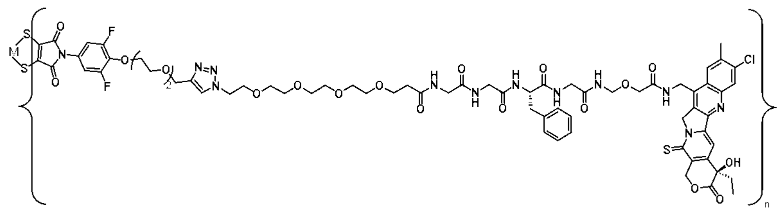 |
| **B7H3-ADC-31** | 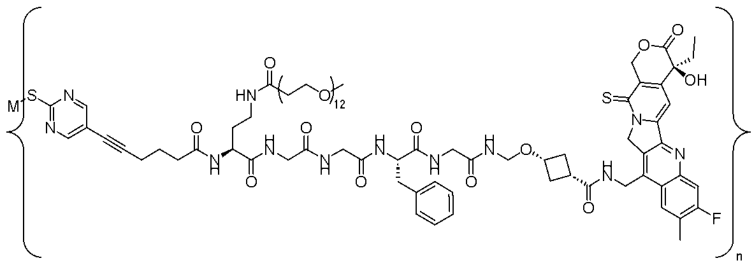 |
| **B7H3-ADC-32** | 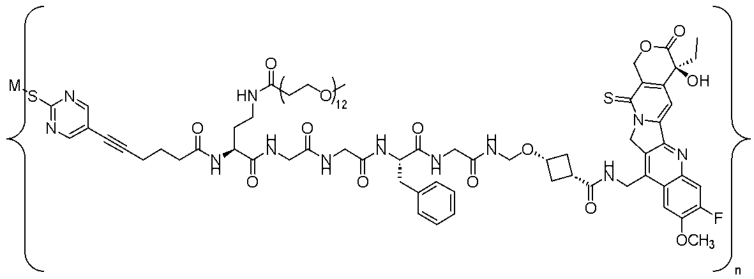 |
| **B7H3-ADC-33** | 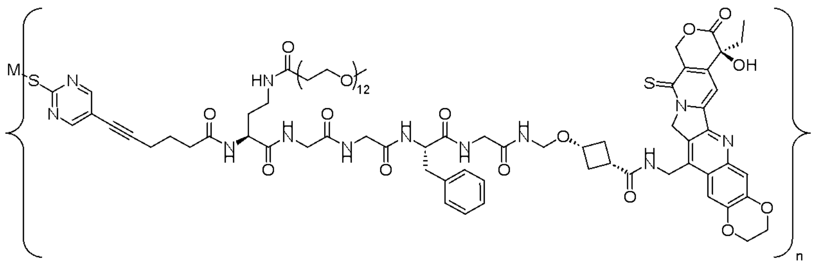 |
| B7H3-ADC-34 | 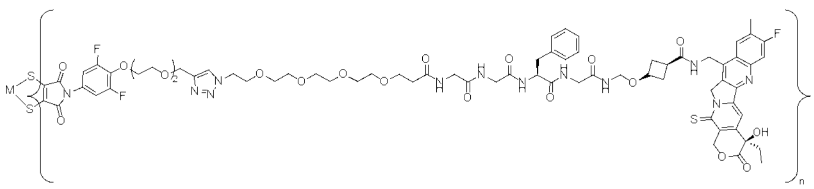 |

After ultrafiltration was completed, the concentration of the ADC conjugate was determined using absorbance values at 280 nm and 365 nm. Whether the conjugation successful or not was confirmed by HPLC-HIC analysis. Antibody purity was assessed by SEC-HPLC, and the DAR value was determined by LC-MS. The results are shown in Table 13.

Table 13 Conjugation Results of the B7H3 Antibody-Based ADC

| ADC Number | DAR | ADC Number | DAR |
|---|---|---|---|
| **B7H3-ADC-1** | 8 | **B7H3-ADC-2** | 4 |
| **B7H3-ADC-3** | 7.66 | **B7H3-ADC-4** | 8 |
| **B7H3-ADC-5** | 8 | **B7H3-ADC-6** | 7.71 |
| **B7H3-ADC-7** | 8 | **B7H3-ADC-8** | 7.66 |
| **B7H3-ADC-9** | 7.26 | **B7H3-ADC-10** | 7.47 |
| **B7H3-ADC-11** | 7.34 | **B7H3-ADC-12** | 7.16 |
| **B7H3-ADC-13** | 7.47 | **B7H3-ADC-14** | 6.79 |
| **B7H3-ADC-15** | 8 | **B7H3-ADC-16** | 8 |
| **B7H3-ADC-17** | 8 | **B7H3-ADC-18** | 8 |
| **B7H3-ADC-19** | 8 | **B7H3-ADC-20** | 8 |
| **B7H3-ADC-21** | 8 | **B7H3-ADC-22** | 8 |
| **B7H3-ADC-23** | 4 | **B7H3-ADC-24** | 8 |
| **B7H3-ADC-25** | 8 | **B7H3-ADC-26** | 1.82 |
| **B7H3-ADC-27** | 7.57 | **B7H3-ADC-28** | 4 |
| **B7H3-ADC-29** | 3.22 | **B7H3-ADC-30** | 4 |
| **B7H3-ADC-31** | 7.68 | **B7H3-ADC-32** | 7.96 |
| **B7H3-ADC-33** | 7.92 | B7H3-ADC-34 | 4 |

Test Example 1 In vitro proliferation inhibition assay of the camptothecin derivatives (Payload) of the present invention on tumor cells

The control compounds used in the experiment were DXD (synthesized from EXD according to the synthesis method of patent EP2907824B1. EXD was purchased from MedChemExpress, catalog number: HY-13631A) and SN38 (purchased from Bide Pharmatech, catalog number BD8481). Their structural formulas are

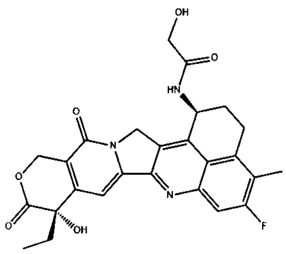

and

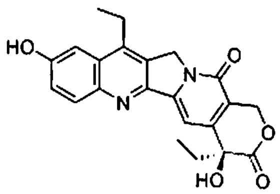

respectively.

The cell lines used in this experiment were human breast cancer cells SK-BR-3, T47D, MDA-MB-453, human malignant melanoma cells A375, human lung squamous carcinoma cells NCI-H1703, and human gastric cancer cells NCI-N87. Cell suspensions were prepared in fresh cell culture medium containing 10% FBS and then diluted to a density of $5 \times 10^4$ cells/mL, $10 \times 10^4$ cells/mL, 100 μL per well was added to a 96-well cell culture plate (Thermo catalog number: 167425), and cultured at 37°C with 5% carbon dioxide for 24 h.

The test compounds of the present invention and the control compounds were each prepared to 10 mM stock solutions in DMSO, and the drugs were diluted with 5% FBS 1640 culture medium in a gradient of 50, 10, 2, 0.4, 0.08, 0.016, 0.0032, 0.00064, 0.000128, and 0 μM, with two replicates for each well.

50 μL of cell supernatant was pipetted from each 96-well plate, followed by the addition of 50 μL of compound dilutions at final molar masses of 25, 5, 1, 0.2, 0.04, 0.008, 0.0016, 0.00032, 0.000064, and 0 μM. After mixing, the cells were cultured in a constant temperature $CO_2$ incubator for 4 days. Cell supernatant was then added with 100 μL of detection reagent (5% FBS1640 medium and CCK-8 solution in a 10:1 ratio) and incubated in an incubator for 4 hours. Chemiluminescence was read on a microplate reader (TECAN, Spark), and data were analyzed using Graphpad Prism 8 software.

The experimental results are shown in Table 14.

Table 14 $IC_{50}$ values of the compounds of the present invention for inhibition of cancer cell proliferation *in vitro*

| Compounds | NCI-N87 $IC_{50}$ (nM) | SK-BR-3 $IC_{50}$ (nM) | T47D $IC_{50}$ (nM) | MDA-MB-453 $IC_{50}$ (nM) | A375 $IC_{50}$ (nM) | NCI-H1703 $IC_{50}$ (nM) |
|---|---|---|---|---|---|---|
| 2 | 3.749 | 1.561 | ND | ND | ND | ND |
| 3 | 1.911 | 1.037 | ND | ND | ND | ND |
| 4 | 380.8 | 182.6 | ND | ND | ND | ND |
| 6 | 2.228 | 1.226 | 0.7415 | 4.238 | 0.1641 | 0.5896 |
| 7 | ND | ND | 244.9 | 570.2 | 0.7142 | 0.064 |
| 9 | ND | ND | 0.8037 | 3.863 | ~0.0010 51 | 0.1493 |
| 11 | 1.563 | 0.906 | ND | ND | ND | ND |
| 17 | 0.999 | 0.675 | 0.1721 | 1.144 | ND | ND |
| 46 | 0.229 | 0.0155 | ND | ND | ND | ND |
| 47 | ND | ND | 0.9556 | 4.37 | ND | ND |
| 48 | ND | ND | 0.8317 | 17.73 | ND | ND |
| 49 | ND | ND | 11.01 | 24.47 | ND | ND |
| EXD | 1.445 | 0.866 | 0.7287 | 1.017 | ~0.0550 5 | 0.007893 |
| DXD | 5.966 | 3.621 | 1.021 | 2.87 | 0.1305 | 0.4723 |
| SN38 | 5.380 | 2.508 | 2.113 | 32.8 | ND | ND |
| Note: "~" indicates estimated $IC_{50}$ value.<br>Conclusion: The camptothecin derivatives provided by the present invention exhibit superior proliferation inhibition activity relative to DXD and SN38 on human breast cancer cells SK-BR-3, T47D, MDA-MB-453, human malignant melanoma cells A375, human lung squamous carcinoma cells NCI-H1703 and human gastric cancer cells NCI-N87, indicating that they have a potential as a drug. | | | | | | |

Test Example 2 In vitro proliferation inhibition assay of the camptothecin derivatives (Payload) of the present invention on tumor cells

1. Instruments and Reagents

| Name | Model and level | Manufacturer |
|---|---|---|
| DMSO | D2650 | Sigma |
| One hundred thousandth analytical balance | XPR105 | METTLER |
| Cell Titer-Glo Reagent | G7573 | Promega |
| 96-well white plate | 236108 | Thermo |
| $CO_2$ cell culture incubator | IEY0006 | NUAIRE |
| Biosafety cabinet | IEY0005 | ESCO |
| Plate Shaker | MTS2/4 | IKA |
| Microplate Reader | CEY0017 | TECAN Spark |
| Cell Counter | CEY0018 | Countstar |

2. Experimental Procedure

a. Plating & culture: MDA-MB-453 (purchased from: Xiehe, catalog number: 1101HUM-PUMCO00016)/T47D (Kebai, CBP60397)/HT1376 cells (purchased from: Kebai, catalog number: CBP60310) were cultured into 96-well plates by a density of $1\times10^3$ /$2\times10^3$ /$1\times10^3$ cells per100 μL, and 200 μL of PBS was added to the outermost circle (to reduce the

volatilization of the culture medium), and cultured at 37°C, 5% $CO_2$ incubator for 24 h;

b. Drug Addition & Incubation: The 96-well flat-bottom plate was retrieved. The culture medium in the wells was aspirated, and 100 μL of the ADC drug/payload (diluted in culture medium) was added to each well. The plate was then incubated at 37°C, 5% $CO_2$ incubator for 5 days.

3. Detection and data processing

a. The 96-well plate was retrieved. The culture medium was removed, and 100 μL of detection reagent (CCK8:medium= 1:9) was added to each well. The plate was then placed in a 37°C, 5% $CO_2$ incubator for 4 hours;

b. The optical density (OD) at 450 nm was measured using a microplate reader (model: CEY0017), and the data was recorded with Excel.

c. Graphpad Prism 9.0 was used to analyze and organize the data.

The experimental results are shown in Table 15.

Table 15 $IC_{50}$ values of the camptothecin derivatives of the present invention for inhibition of cancer cell proliferation *in vitro*

| The name of compound | T47D $IC_{50}$ (nM) | MDA-MB-453 $IC_{50}$ (nM) | HT1376 $IC_{50}$ (nM) |
|---|---|---|---|
| **EXD** | 0.221 | 0.902 | 8.712 |
| **11** | 0.141 | 0.645 | 3.852 |
| **12** | 0.093 | 0.312 | 1.969 |
| **13** | 0.059 | 0.797 | 10.860 |
| **14** | 0.250 | 5.227 | 13.840 |
| **15** | 0.157 | 1.098 | 9.761 |
| **16** | 0.141 | 0.481 | 3.865 |
| **19** | 0.268 | 1.008 | 9.358 |
| **20** | 0.245 | 8.060 | 0.917 |
| **21** | 0.108 | 0.294 | 11.600 |
| **22** | 0.116 | 0.362 | 5.379 |
| **23** | 0.117 | 0.343 | 5.942 |
| **24** | 3.595 | 78.370 | 25.410 |
| **25** | 2.597 | 102.900 | 8.393 |
| **26** | <0.003 | 1.004 | 0.014 |
| **27** | 0.152 | 0.170 | 2.499 |
| **28** | 0.150 | 6.168 | 42.410 |
| **29** | 0.014 | 0.183 | 5.976 |
| **30** | 0.391 | 1.011 | 66.730 |
| **31** | 0.142 | 0.337 | 19.460 |
| **32** | 0.089 | 0.417 | 15.350 |
| **33** | 0.392 | 0.599 | 45.430 |
| **34** | 0.151 | 0.652 | 10.130 |
| **35** | - | ~ 1.220 | 4.943 |
| **36** | 0.119 | 0.029 | 10.290 |
| **37** | 0.054 | 0.013 | 13.540 |
| **38** | 0.234 | 0.121 | 38.400 |
| **39** | 0.047 | 0.482 | 8.692 |

(continued)

| The name of compound | T47D $IC_{50}$ (nM) | MDA-MB-453 $IC_{50}$ (nM) | HT1376 $IC_{50}$ (nM) |
|---|---|---|---|
| **40** | 0.053 | 0.021 | 18.240 |
| **41** | 0.231 | 0.943 | 24.120 |
| **42** | 0.845 | 4.179 | 40.220 |
| **43** | 0.089 | 1.640 | 9.939 |
| **44** | 0.195 | 0.674 | 5.050 |
| **45** | 2.793 | 4.970 | 3.785 |
| Note: "-" means not tested. Conclusion: The camptothecin derivatives provided by the present invention exhibit superior proliferation inhibition activity relative to EXD on human breast cancer cells T47D, MDA-MB-453, and human bladder cancer cells HT1376, indicating that they have a potential as a drug. | | | |

Test Example 3 In vitro proliferation inhibition assay of the ligand-drug conjugates of the present invention on tumor cells targeting HER2

The cell lines used in the experiment were HER2-high-expressing human breast cancer cell line SK-BR-3 and human gastric cancer cell line NCI-N87. Cell suspensions were prepared in fresh cell culture medium containing 10% FBS and then diluted to a density of $5\times10^4$ cells/mL, $10\times10^4$ cells/mL respectively. 100 μL per well was added to a 96-well cell culture plate (Thermo catalog number: 167425), and cultured at 37°C with 5% carbon dioxide for 24 h.

The ADC samples were prepared at 10 μM stock solutions in PBS as the initial concentration, and a five-fold gradient dilution was performed using PBS, for a total of nine concentrations. 50 μL of culture medium was aspirated from each well, and 50 μL of the above ADC solution was added, resulting in an initial ADC concentration of 5 μM in each well and a final volume of 100 μL per well. The cells were cultured at 37°C in 5% $CO_2$ for 3 days. 100 μL of CTG (Celltiter-Glo® Luminescent Cell Viability Assay, Promega, Catalog Number G7573) was added per well. The cells were mixed on a decolorizing shaker at room temperature for 30 minutes, incubated for 10 minutes, and chemiluminescence was read on a microplate reader (TECAN, Spark). Data were analyzed using Graphpad Prism 5 software. The experimental results are shown in Table 16.

Table 16 $IC_{50}$ values of the ligand-drug conjugates of the present invention for inhibition of cancer cell proliferation *in vitro*

| Compounds | NCI-N87/$IC_{50}$ (μg/mL) | SK-BR-3/$IC_{50}$ (μg/mL) |
|---|---|---|
| HER2-ADC-1 | 0.444 | 0.143 |
| HER2-ADC-2 | 2.750 | - |
| HER2-ADC-3 | 0.313 | 0.107 |
| HER2-ADC-4 | 0.04 | 0.02 |
| HER2-ADC-5 | 0.10 | ND |
| HER2-ADC-6 | 0.11 | 0.04 |
| Conclusion: The ligand-drug conjugates targeting HER2 of the present invention have significant proliferation inhibition activity on SK-BR-3 cells and NCI-N87 cells. | | |

Test Example 4 In vitro proliferation inhibition assay of the ligand-drug conjugates of the present invention on tumor cells targeting NECTIN-4

The cell lines used in the experiment were human breast cancer T47D and MDA-MB-453 cell lines that highly expressed NECTIN4. Cell suspensions were prepared in fresh cell culture medium containing 10% FBS and then diluted to a density of $5\times10^4$ cells/mL, $10\times10^4$ cells/mL. 100 μL per well was added to a 96 well cell culture plate (Thermo catalog number: 167425) and cultured at 37 °C with 5% carbon dioxide for 24 hours.

The ADC samples were prepared at 10 μM stock solutions in PBS as the initial concentration, and a five-fold gradient dilution was performed using PBS, for a total of nine concentrations. 50 μL of culture medium was aspirated from

each well, and 50 μL of the above ADC solution was added, resulting in an initial ADC concentration of 5 μM in each well and a final volume of 100 μL per well. The cells were cultured at 37°C in 5% $CO_2$ for 3 days. 100 μL of CTG (Celltiter-Glo® Luminescent Cell Viability Assay, Promega, Catalog No. G7573) was added per well. The cells were mixed on a decolorizing shaker at room temperature for 30 minutes, incubated for 10 minutes, and chemiluminescence was read on a microplate reader (TECAN, Spark). Data were analyzed using Graphpad Prism 5 software. The experimental results are shown in Table 17.

Table 17 $IC_{50}$ values of the ligand-drug conjugates of the present invention for inhibition of cancer cell proliferation *in vitro*

| Compound | T47D/$IC_{50}$ (nM) | MDA-MB-453 /$IC_{50}$ (nM) |
|---|---|---|
| NECTIN4-ADC-1 | 13.61 | 60.31 |
| NECTIN4-ADC-2 | 0.0183 | 9.196 |
| NECTIN4-ADC-3 | 11.32 | 38.82 |
| NECTIN4-ADC-4 | 8.257 | ~200 |
| Conclusion: The ligand-drug conjugates targeting NECTIN-4 of the present invention have obvious proliferation inhibition activity on T47D cells and MDA-MB-453 cells. | | |

Test Example 5 In vitro proliferation inhibition assay of the ligand-drug conjugates of the present invention on tumor cells targeting B7H3

The cell lines used in the experiment were human A375 (ATCC, Catalog No.: CRL-1619), NCI-H358 (ATCC, Catalog Number: CRL-5807), NCI-H1975 (Kebai, Catalog Number : CBP60121), and NCI-H345 cell lines (BIOBW Beijing Bio-Tech, Catalog Number : bio-133296). Cell suspensions were prepared in fresh cell culture medium containing 10% FBS and then diluted to a density of $2\times10^4$ cells/mL, $4\times10^4$ cells/mL, $2\times10^4$ cells/mL and $2\times10^4$ cells/mL respectively. 100 μL per well was added to a 96-well cell culture plate (Thermo catalog number: 167425), and cultured at 37°C with 5% carbon dioxide for 24 h.

The ADC sample was prepared at 10 μM in PBS. This was the initial concentration, and a five-fold gradient dilution was performed using PBS, for a total of nine concentrations. 50 μL of culture medium was aspirated from each well, and 50 μL of the above ADC solution was added, resulting in an initial ADC concentration of 5 μM in each well and a final volume of 100 μL per well. The cells were cultured at 37°C in 5% $CO_2$ for 3 days. 100 μL of CTG (Luminescent Cell Viability Assay, Promega, catalog number: G7573) was added in each well, mixed on a decolorization shaker at room temperature for 30 minutes, and incubated for 10 minutes. Chemiluminescence was read on a microplate reader (TECAN, Spark). Data were analyzed using Graphpad Prism 5 software. The results are shown in Table 18.

Table 18 $IC_{50}$ values of the ligand-drug conjugates of the present invention for inhibition of cancer cell proliferation *in vitro*

| Compounds | **NCI-H345** $IC_{50}$ (nM) | **NCI-H358** $IC_{50}$ (nM) | A375 $IC_{50}$ (nM) | NCI-H1975 $IC_{50}$ (nM) |
|---|---|---|---|---|
| B7H3-ADC-1 | 127.3 | 0.5860 | 16 | 180.7 |
| B7H3-ADC-2 | 446.7 | 90.6000 | 102.0000 | - |
| B7H3-ADC-3 | 21.50 | 4.619 | 5.836 | |
| B7H3-ADC-4 | 31.2124 | 0.5788 | 1.5661 | - |
| B7H3-ADC-5 | 6.6632 | 0.1779 | 0.6102 | - |
| B7H3-ADC-6 | 27.3796 | 0.7980 | 0.7126 | - |
| B7H3-ADC-7 | 9.658 | 0.2423 | 0.9322 | - |
| B7H3-ADC-8 | 22.2497 | 6.1288 | 4.8507 | - |
| B7H3-ADC-10 | - | <0.01024 | <0.01024 | |
| B7H3-ADC-13 | - | <0.01024 | <0.01024 | |
| B7H3-ADC-15 | - | 0.06 | 0.22 | 14.15 |
| B7H3-ADC-16 | 40.0294 | 0.4587 | 0.4747 | - |

(continued)

| Compounds | **NCI-H345** $IC_{50}$ (nM) | **NCI-H358** $IC_{50}$ (nM) | A375 $IC_{50}$ (nM) | NCI-H1975 $IC_{50}$ (nM) |
|---|---|---|---|---|
| B7H3-ADC-17 | 14.3835 | 0.2448 | 0.3879 | - |
| B7H3-ADC-18 | 22.4788 | 0.6996 | 0.1924 | - |
| B7H3-ADC-19 | 6.4175 | 33.3372 | 3.2463 | - |
| B7H3-ADC-20 | 7.6700 | 0.5333 | 0.9606 | - |
| B7H3-ADC-21 | - | 0.78 | 0.33 | 74.96 |
| B7H3-ADC-22 | 10.2859 | 1.8586 | 1.3713 | - |
| B7H3-ADC-23 | 58.4069 | 3.5681 | 6.6564 | - |
| B7H3-ADC-24 | 18.3663 | 0.2086 | 0.2063 | - |
| B7H3-ADC-25 | 19.3587 | 0.1822 | 0.2447 | - |
| B7H3-ADC-26 | 43.9795 | 26.3849 | 26.6232 | - |
| B7H3-ADC-27 | 6.62 | 0.06091 | 0.06347 | 23.45 |
| B7H3-ADC-28 | 194.16 | 36.37 | 105.80 | - |
| B7H3-ADC-29 | 630.32 | 408.25 | 2.04 | - |
| B7H3-ADC-30 | 251.94 | 1.56 | 57.49 | - |
| B7H3-ADC-31 | 37.45 | 438.90 | 4.59 | - |
| B7H3-ADC-32 | 15.34 | 27.60 | 4.19 | - |
| B7H3-ADC-33 | 63.34 | 1.79 | 11.39 | - |
| Note: "-" means not tested.<br>Conclusion: The ligand-drug conjugates provided by the present invention exhibit excellent proliferation inhibition activity on human A375, NCI-H358, NCI-H1975, and NCI-H345 cell lines with high B7H3 expression, indicating their drug potential. | | | | |

Test Example 6: Bystander Killing Effect

*1) In vitro* bystander effect:

1. Cells (Raji(-) or A375(+):Raji(-)=1:1, $1\times10^5$ cells of each type ) were spread into a 6-well plate, with 3 ml of 10% FBS 1640 medium per well.
2. The calculated amount of ADC B7H3-ADC-1 and B7H3-ADC-27 were added to the culture medium and add them to the corresponding wells, bringing the total volume in each well to 4 ml.
3. On the 4th day after drug addition, the supernatant was collected and the cells on the plate were digested. The cells were collected in the same centrifuge tube as the supernatant and centrifuged at 300 $\times$ g for 5 min.
4. FACS was added to wash cells one time.
5. M30 (1ug/100ul) antibody was incubated in a 4°C refrigerator for 1 hour. (antibody + cells = 100ul + 100ul)
6. FACS was used to wash the cells twice, PE dye (1:200) was added and incubated for 30 minutes.
7. PBS was used to wash the cells three times, NIR dye was added (1:500) and incubated for 15 minutes.
8. The cells were washed once with PBS, were resuspended in PBS and were analyzed by flow cytometry (using PE and APC-cy7 channels)

Conclusion: The results are shown in Figures 1, 2, 3, and 4, demonstrating that neither the ADC of the present invention nor the positive control ADC exhibited significant killing activity on B7H3-negative cell lines. Under the co-cultured condition of B7H3-positive and negative cells, the ADC of the present invention efficiently killed both positive and negative cells, demonstrating a bystander killing effect superior to that of the positive control drug.

*2) In vivo* bystander effect:

Experimental methods:

B7H3-positive A375 cells and B7H3-negative MDA-MB-453 cells were resuspended in 100 ul PBS, added equal volumes of Matrigel in a 1:1 ratio and mixed thoroughly. 200 ul of the mixture was inoculated subcutaneously into nude mice.

When grown to around 200mm$^3$, tail vein administration, volume 200ul;

Tumor tissue was removed after administration 15 and 30 days, washed three times with PBS, cut into small pieces, and digested at 37°C for 30 min with 3 ml of trypsin. The tissue was then sieved through a 70 um cell sieve. Undigested tissue fragments were ground and digested with 6 ml of culture medium containing 10% FBS. The tissue was centrifuged at 200 g for 5 min, resuspended in 1 ml of PBS, and the cells were counted.

The cells were diluted to $2\times10^6$ /ml, and 50 ul ($1\times10^5$/well) of the cells were plated in a U-bottom 96-well plate;

M30 antibody was diluted to 20 ug/ml, and 50 ul (1 ug/well) was added to the cells, mixed, and incubated at 4°C for 1 hour;

After Washing twice with FACS buffer by centrifugation, 100 ul of anti-human Fc.PE fluorescent secondary antibody (1:200 dilution) was added, and incubated at 4°C for 30 min;

Following Washing twice by centrifugation with FACS washing solution, 70ul of FACS was added to resuspend, and 50ul was taken to detect PE fluorescence intensity by flow cytometry.

Conclusion: The results are shown in Figure 5, indicating that the ADCs of the present invention exhibit superior tumor inhibition activity compared to positive drug ADCs in an animal model in which both B7H3-negative and positive cells coexist. The tumor (volume) inhibition rate (TGI) of the control ADC **B7H3-ADC-1** 2mpk was 58.37%, and that of B7H3-ADC-2 2mpk was 54.81%. The TGI of the ADC provided by the present invention, **B7H3-ADC-1** 2mpk, was 90.46%, and that of 1mpk was 71.95%, indicating that the ADCs of the present invention have a bystander effect that is significantly superior to that of positive drugs.

Test Example 7 Antitumor Efficacy of the B7H3 Ligand-Drug Conjugates of the Present Invention in the A375 CDX Model

Experimental steps:

1. Human malignant melanoma cells A375 (ATCC) ($5\times10^6$ / 200μl / mouse, containing 50% growth factor-reduced Matrigel). After cell inoculation, the tumors grew for 9 days and the tumor volume reached 200 mm$^3$. After the experiment, the animals were randomly divided into groups (D0), with 5 animals in each group, for a total of 5 groups.
2. **B7H3-ADC-1** and **B7H3-ADC-27** were administered via tail vein injection at two doses, 2 mg/kg and 4 mg/kg, respectively, as a single dose. Tumor volume and body weight were measured twice weekly and the data were recorded. Tumor volume (V): V = 1/2 × major diameter × minor diameter$^2$. The tumor growth curve was prepared using GrapHPadPrism 8.0.2.2.263 software. The tumor growth curve is shown in Figure 6 , and the changes in mouse weight are shown in Figure 7.

| Groups | Tumor volume (mm$^3$) (On the 21st day of administration) | TGI |
|---|---|---|
| PBS | 917.62 | - |
| B7H3-ADC-1 2mg | 524.65 | 42.73% |
| B7H3-ADC-27 2mg | 32.98 | 96.41 % |
| B7H3-ADC-1 4mg | 104.20 | 88.65% |
| B7H3-ADC-27 4mg | 8.86 | 99.04% |
| Conclusion: The ADC B7H3-ADC-27 provided by the present invention can significantly inhibit the growth of tumors in A375 model mice at both 2mpk and 4mpk doses, and its anti-tumor activity is significantly better than that of the positive control ADC. In addition, no animal deaths or significant weight loss were observed in all treatment groups during the observation period, indicating that **B7H3-ADC-27** has no obvious toxicity. | | |

Test Example 8 Antitumor efficacy of the NECTIN4 ligand-drug conjugate of the present invention in the MDA-MB-453CDX model

1. Human breast cancer cells MDA-MB-453 (Xiehe) ($1\times10^7$ After cell inoculation, the tumor grew for 9 days and the tumor volume reached 150 $mm^3$. After the experiment, the animals were randomly divided into three groups (D0), with 5 animals in each group, for a total of 3 groups.
2. **NECTIN4-ADC-1** and **NECTIN4-ADC-2** were administered via tail vein injection at a single dose of 5 mg/kg. Tumor volume and body weight were measured twice weekly and recorded. Data were analyzed using Excel 2016 statistical software: mean was calculated as average; SD was calculated as STDEV; and SEM was calculated as STDEV/SQRT. Tumor growth curves were generated using GraphPad Prism 8.0.2.2.263 software.

$$\text{Tumor volume (V): } V = 1/2 \times L \text{ long diameter} \times L \text{ short diameter}^2$$

$$\text{Relative tumor volume (RTV): } RTV = VT/V0$$

$$\text{Relative tumor proliferation rate T/C (\%)} = TRTV/CRTV \times 100\%$$

$$\text{Tumor inhibition rate (\%)} = (CRTV\text{-}TRTV)/CRTV\ (\%)$$

Where V0 and VT are the tumor volumes at the start and end of the experiment, respectively. CRTV and TRTV are the relative tumor volumes of the blank control group (PBS) and the experimental group at the end of the experiment, respectively. The changes in tumor volume of the mice are shown in Figure 8, and the changes in mouse body weight are shown in Figure 9.

| Groups | Tumor volume ($mm^3$) (On the 32st day of administration) | TGI |
|---|---|---|
| NECTIN4-ADC-1 5mg/kg | 298.85 | 59.99% |
| NECTIN4-ADC-2 5mg/kg | 2.49 | 99.66% |
| PBS | 746.80 | - |
| Conclusion: The body weight of mice increased steadily, with no significant safety risks. Furthermore, **NECTIN4-ADC-2** (5 mg/kg) was significantly more effective than the control drug **NECTIN4-ADC-1** (5 mg/kg). | | |

Test Example 9 Toxicity Test of Ligand-Drug Conjugates of the Present Invention

1. Experimental purpose:

The potential toxicity reaction of ADC administered five times via tail vein injection as a single dose in Balb/c mice was studied, and the dosage and frequency of administration were preliminarily determined to provide a reference for subsequent formal toxicology experiments.

2. Experimental Animals

Species/strain/grade: Balb/c mice, SPF grade

The Source of Experimental animals: Weitonglihua. Age: 6-8 weeks. Number and gender: 8 females.

3. Experimental Design

3.1 Trial Grouping

| | Groups | Dosage (mg/kg) | Route of administration | Dosing frequency | Duration of administration (week) | Number of animals |
|---|---|---|---|---|---|---|
| A | B7H3-ADC-27 | 70 | IV | Q1W | 5 | 4 |
| B | PBS | - | IV | Q1W | 5 | 4 |

3.2 Experimental methods

Administration route and frequency: intravenous injection, once a week, for a total of 5 doses, Day 0, 7, 14, 21, 28

Experimental duration: 6 weeks

Dosing volume: 170μL-210μL

3.3 Detection indicators:

① General clinical observation: Conducted once before grouping and once a day during the trial.
② Body weight: Measured once before administration and twice weekly thereafter. Animals in the main study groups were weighed once prior to the scheduled euthanasia.
③ Histopathological examination: Based on the gross observation results and possible toxic target organs, the heart, liver, spleen, lung, kidney, and small intestine were prepared and examined. The specific experimental procedures are as follows:

a. Sample: Tissue sample, approximately 3mm thick.
b. Dehydration: Dehydration with graded alcohol: 60%, 70%, 80%, 90%, 95% I, 95% II, 100% I, 100% II for 1 hour per tank; xylene I for 30 minutes, xylene II for 20 minutes; paraffin wax immersion: paraffin I and paraffin II for 20 minutes per tank.
c. Embedding: Paraffin embedding.
d. Section: The thickness of the section is about 4 μm.
e. Hematoxylin eosin (HE) staining: ① Dewaxing and hydrating: dewaxing in xylene I and xylene II for 15 min each; 100% anhydrous ethanol, 95% ethanol, 80% ethanol, and 70% ethanol for 10 min each; rinsing with running water for 2 min; ② Hematoxylin staining for 10 min, followed by rinsing with running water; ③ 0.5% eosin staining for 5 min, followed by rinsing with running water; ④ Gradient alcohol dehydration: 70%, 80%, 95%, and 100%, 10 s each; ⑤ Xylene clearing: xylene I and xylene II for 5 min each; ⑥ Seal with neutral resin glue.
f. Observe under light microscope and take microphotographs.

3.4 Data Analysis

The experimental results are expressed as mean ± standard error, and one-way analysis of variance was performed. P values less than 0.05 were considered statistically significant (*p<0.05).

4. Experimental results:

| group | ear mark | lung | heart | liver | spleen, | kidney | intestine |
|---|---|---|---|---|---|---|---|
| PBS | 1084 | - | - | - | + | - | - |
| | 1093 | - | - | - | - | - | - |

(continued)

| group | ear mark | lung | heart | liver | spleen, | kidney | intestine |
|---|---|---|---|---|---|---|---|
| B7H3-ADC-27 | 395 | - | - | - | - | - | - |
| | 1081 | - | - | - | - | - | - |
| | 1089 | - | - | - | + | - | - |
| | 1091 | - | - | - | + | - | - |
| HE,-: no obvious lesions, +: minor lesions, ++: mild, +++: moderate, ++++: severe. | | | | | | | |

The changes in mouse body weight are shown in Figure10.

Conclusion: For the test substance **B7H3-ADC-27,** compared with the vehicle group, at a dose of 70 mg/kg, the animal body weight did not decrease significantly, and showed a slow growth state ($p < 0.05$), indicating that the ligand-drug conjugates provided by the present invention have no obvious toxicity.

Test Example 10 Antitumor efficacy of the B7H3 ligand-drug conjugate of the present invention in the NCI-H358 CDX model

1. Experimental Procedure

BALB/c-Nude mice were subcutaneously inoculated with human non-small cell lung cancer cells NCI-H358 (3×106/200 μL/mouse, in 50% low-growth factor artificial basement membrane matrix gel) in the right flank. After cell inoculation, the tumor grew for 5 days and the tumor volume reached 130 $mm^3$. After about 24 hours, the animals were randomly divided into 13 groups (D0), with 5 animals per group. Drugs were administered via tail vein injection, with high and low doses given once. Tumor volume and body weight were measured twice weekly and the data were recorded.

2. Data Analysis

Data were analyzed using Excel 2016 statistical software, with averages calculated as "average." Tumor growth curves were generated using GraphHPad Prism 8.0.2.2.263 software.

$$\text{Tumor volume (V): } V = 1/2 \times L \text{ long diameter} \times L \text{ short diameter}^2$$

$$\text{Relative tumor volume (RTV)} = VT/V0$$

$$\text{Relative tumor proliferation rate T/C (\%)} = TRTV/CRTV \times 100\%$$

$$\text{Tumor inhibition rate (\%)} = (CRTV-TRTV)/CRTV\ (\%)$$

Where V0, VT are the tumor volumes at the start and end of the experiment, respectively. CRTV and TRTV are the relative tumor volumes of the blank control (PBS) and experimental groups at the end of the experiment, respectively. The changes in tumor volume in the mice are shown in Figure 11 and Figure 12.

Conclusion: The experimental group ADCs **B7H3-ADC-15, B7H3-ADC-21, B7H3-ADC-23 (DAR4),** and **B7H3-ADC-27** provided by the present invention can significantly inhibit the growth of tumors in NCI-H358 model mice, whether in the low-dose group or the high-dose group, and their anti-tumor activity is significantly better than that of the positive control ADCs **(B7H3-ADC-1, B7H3-ADC-2(DAR4)).**

The present invention has been described through the above-described embodiments. However, it should be understood that the above-described embodiments are for illustrative and illustrative purposes only and are not intended to limit the present invention to the described embodiments. Furthermore, it will be understood by those skilled in the art that the present invention is not limited to the above-described embodiments and that further variations and modifications may be made based on the teachings of the present invention, all of which fall within the scope of the present invention. The scope of protection of the present invention is defined by the appended claims and their equivalents.

## Claims

**1.** A compound represented by Formula 1, or a pharmaceutically acceptable salt thereof,

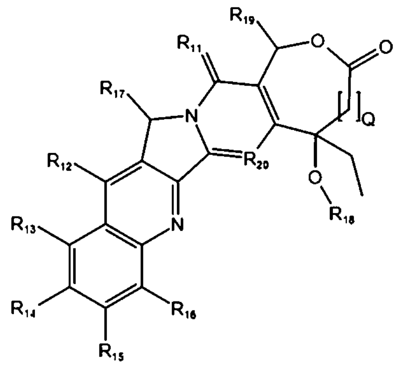

Formula 1

wherein,

each occurrence of $R_{16}$, $R_{17}$, and $R_{19}$ is each independently selected from hydrogen, halogen, $-NO_2$, -CN, -OR, -SR, $-N(R_a)(R_b)$, -C(O)R, $-CO_2R$, -C(O)C(O)R, - $C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, and $C_{1-6}$ alkyl, $C_{2-6}$ alkenyl, and $C_{2-6}$ alkynyl, optionally substituted with R; preferably, each occurrence of $R_{16}$, $R_{17}$, and $R_{19}$ is each independently selected from hydrogen and $C_{1-6}$ alkyl optionally substituted with R; more preferably, each occurrence of $R_{16}$, $R_{17}$, and $R_{19}$ is each independently selected from hydrogen and $C_{1-6}$ alkyl; more preferably, each occurrence of $R_{16}$, $R_{17}$, and $R_{19}$ is each independently hydrogen;

$R_{20}$ is selected from N and $CR_{20a}$;

$R_{20a}$ is selected from hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, $-C(O)CH_2OH$, - S(O)H, $-S(O)_2H$, $-C(O)NH_2$, $-SO_2NH_2$, -OC(O)H, $-N(H)SO_2H$, and $C_{1-6}$ alkyl; preferably, $R_{20a}$ is hydrogen or $C_{1-6}$ alkyl;

$R_{11}$ is selected from O and S;

Q is selected from 0, 1, 2, 3, and 4;

$R_{18}$ is selected from H and $-B-R_3$;

when Q is 0, and $R_{18}$ is $-B-R_3$, wherein $R_{14}$ and $R_{15}$ are each independently selected from hydrogen, halogen, $C_{1-8}$ alkyl, and $C_{1-8}$ haloalkyl; preferably, $R_{14}$ and $R_{15}$ are each independently selected from hydrogen, F, Cl, Br, I, $C_{1-3}$ alkyl, and $C_{1-3}$ haloalkyl; preferably, $R_{14}$ and $R_{15}$ are each independently $-CH_3$ or F; preferably, $R_{14}$ is $-CH_3$, and/or $R_{15}$ is -F;

B is selected from -C(O)- and -P(O)(OH)-; preferably, B is -C(O)-;

$R_3$ is selected from $-(CH_2CH_2O)_aC_{1-6}$ alkyl,

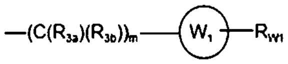

,

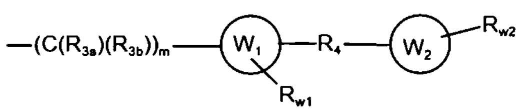

,

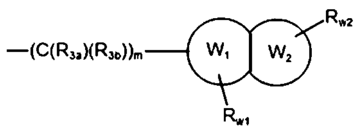

,

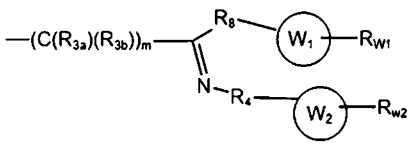

,

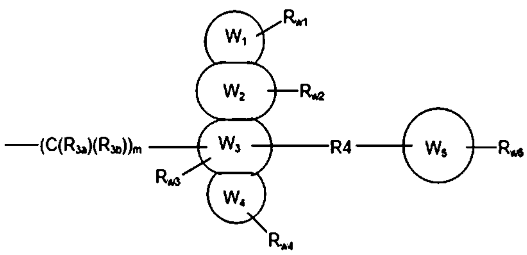

,

and

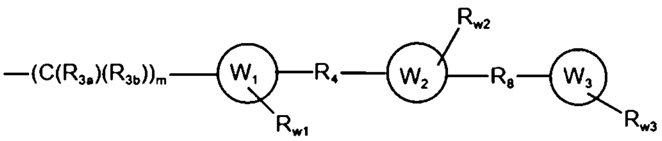

,

wherein, when $-(C(R_{3a})(R_{3b}))_m$ contains methylene units, n methylene units of the $-(C(R_{3a})(R_{3b}))_m-$ are each independently replaced by $-N(R_5)C(O)-$, -C(O)-, -OC(O)-, $-NR_5-$, -O-, -S-, -SO-, $-SO_2-$, $-P(R_5)-$, $-P(=O)(R_5)-$, $-N(R_5)SO_2-$, -C(=S)-, $-C(=NR_5)-$, -N=N-, or -N=CH-;
a is an integer greater than or equal to 1; preferably, a is an integer from 1 to 20; more preferably, a is selected from 1, 2, 3, 4, 5, 6, 7, and 8;
each occurrence of $R_4$ and $R_8$ is each independently a single bond or selected from -O-, $C_{1-8}$ alkylene, $C_{1-8}$ haloalkylene, $-N(R_5)C(O)-$, -C(O)-, -OC(O)-, $-NR_5-$, -S-, -SO-, $-SO_2-$, $-P(R_5)-$, $-P(=O)(R_5)-$, $-N(R_5)SO_2-$, -C(=S)-, $-C(=NR_5)-$, -N=N-, and $-N= CR_5-$;
each occurrence of ring $W_1$, ring $W_2$, ring $W_3$, ring $W_4$, and ring $W_5$ is each independently selected from 6- to 10-membered aryl or arylene, 5- to 10-membered heteroaryl or heteroarylene, 3- to 10-membered cycloalkyl or cycloalkylene, and 3- to 10-membered heterocyclyl or heterocyclylene; preferably, the heteroaryl, heteroarylene, heterocyclylene, and heterocyclyl each independently contain 1, 2, 3, or 4 heteroatoms independently selected from N, O, P, and S; and when $R_3$ is

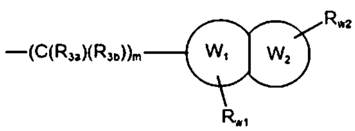

,

$W_1$ and $W_2$ are fused together; when $R_3$ is

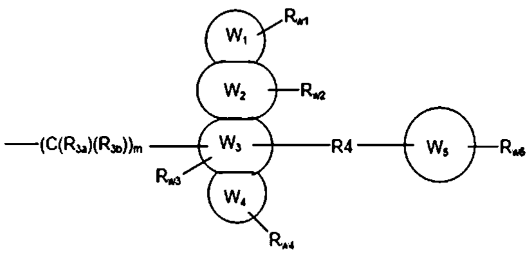

,

$W_1$ and $W_2$ are fused together, $W_2$ and $W_3$ are fused together, and $W_3$ and $W_4$ are fused together;
when ring $W_1$, ring $W_2$, ring $W_3$, ring $W_4$, and ring $W_5$ is each independently selected from 3- to 10-membered cycloalkyl, cycloalkylene, heterocyclylene, and heterocyclyl, the 3- to 10-membered cycloalkyl, cycloalkylene, heterocyclylene, and heterocyclyl are optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is each independently selected from hydrogen, protium, deuterium, tritium, halogen, =O, = S, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, and $C_{1-6}$ alkyl optionally substituted with R; preferably, each occurrence of $R_{w1}$, $R_{w2}$, $R_{w3}$, $R_{w4}$, and $R_{w5}$ is each independently selected from hydrogen, deuterium, F, Cl, Br, I, hydroxyl, =O, =S, $-NH_2$, $-NO_2$, -CN, -COOH, $-OC_{1-3}$ alkyl, $-C_{1-3}$ alkyl, and $C_{1-3}$ alkylene hydroxyl;

when ring $W_1$, ring $W_2$, ring $W_3$, ring $W_4$, and ring $W_5$ is each independently selected from 6- to 10-membered aryl, 6- to 10-membered arylene, 5- to 10-membered heteroarylene, and 5- to 10-membered heteroaryl, the aryl, arylene, heteroarylene, and heteroaryl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is each independently selected from hydrogen, protium, deuterium, tritium, halogen, $-NO_2$, - CN, -OR, -SR, $-N(R_a)(R_b)$, -C(O)R, $-CO_2R$, -C(O)C(O)R, $-C(O)CH_2C(O)R$, -S(O)R, - $S(O)_2R$, $-C(O)N(R_a)(R_b)$, $-SO_2N(R_a)(R_b)$, -OC(O)R, $-N(R)SO_2R$, and $C_{1-6}$ alkyl optionally substituted with R; preferably, each occurrence of $R_{w1}$, $R_{w2}$, $R_{w3}$, $R_{w4}$, and $R_{w5}$ is each independently selected from hydrogen, deuterium, F, Cl, Br, I, hydroxyl, - $NH_2$, $-NO_2$, -CN, $-OC_{1-3}$ alkyl, -COOH, and $C_{1-3}$ alkyl;

$R_{13}$ is selected from hydrogen, halogen, $C_{1-8}$ alkyl, and $C_{1-8}$ haloalkyl; preferably, $R_{13}$ is hydrogen; $R_{12}$ is selected from $C_{1-8}$ alkylene-$N(R_a)(R_b)$, $C_{1-8}$ haloalkylene-$N(R_a)(R_b)$, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, - N(R) $SO_2R$, and $C_{1-6}$ alkyl optionally substituted with R; preferably, $R_{12}$ is selected from $C_{1-3}$ alkylene-$N(R_a)(R_b)$ and $C_{1-3}$ haloalkylene-$N(R_a)(R_b)$; preferably, $R_{12}$ is selected from $-CH_2N(R_a)(R_b)$; or $R_{13}$ and $R_{12}$ together with the carbon atoms to which they are attached form a saturated or unsaturated 5- to 10-membered cycloalkyl, or form a 5- to 10-membered heterocyclyl containing 1, 2, or 3 heteroatoms each independently selected from N, O, P, and S, wherein the cycloalkyl or heterocyclyl is optionally substituted with one or more substituents independently selected from halogen, $-NO_2$, -CN, -OR, -SR, $-N(R_a)(R_b)$, -C(O)R, $-CO_2R$, -C(O)C(O)R, $-C(O)CH_2C(O)R$, -S(O) R, -S(O)2R, $-C(O)N(R_a)(R_b)$, $-SO_2N(R_a)(R_b)$, -OC(O)R, -N(R)SO2R, and $C_{1-6}$ alkyl optionally substituted with R; preferably, $R_{13}$ and $R_{12}$ together with the carbon atoms to which they are attached form a 6-membered cycloalkyl, wherein the cycloalkyl is substituted with $-N(R_a)(R_b)$;

each occurrence of $R_{3a}$, $R_{3b}$, and $R_5$ is each independently hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ alkyl optionally substituted with R; and

m and n are each independently an integer greater than or equal to 1, and m≥n; preferably, m and n are each independently 1, 2, 3, 4, 5, 6, 7, 8, 9, or 10, and m≥n; preferably, m is 1, 2, 3, 4, 5, 6, 7, 8, 9, or 10, -n is 0, 1, 2, 3, 4, or 5, and m≥n;

when Q is 1,2,3, or 4, and $R_{18}$ is H, $R_{13}$, $R_{14}$, and $R_{15}$ are each independently selected from hydrogen, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, and $C_{1-6}$ alkyl optionally substituted with R; preferably, $R_{13}$, $R_{14}$, and $R_{154}$ are each independently selected from hydrogen, F, Cl, Br, I, $C_{1-3}$ alkyl, $-NO_2$, -CN, and $-O-C_{1-3}$ alkyl; or $R_{14}$ and $R_{15}$ together with the carbon atoms to which they are attached form a saturated or unsaturated 5 to 10 membered cycloalkyl, or a 5-10 membered heterocyclyl containing 1, 2, or 3 heteroatoms independently selected from N, O, and S, wherein the cycloalkyl or heterocyclyl is unsubstituted or substituted with one or more substituents independently selected from halogen, $-NO_2$, -CN, -OR, -SR, - $N(R_a)(R_b)$, -C(O)R, $-CO_2R$, -C(O) C(O)R, $-C(O)CH_2C(O)R$, -S(O)R, $-S(O)_2R$, - $C(O)N(R_a)(R_b)$, $-SO_2N(R_a)(R_b)$, -OC(O)R, $-N(R)SO_2R$, and $C_{1-6}$ alkyl optionally substituted with R; preferably, $R_{14}$ and $R_{15}$ together with the carbon atoms to which they are attached form a saturated or unsaturated 5- to 6-membered heterocyclyl containing two oxygen atoms;

$R_{12}$ is $C_{1-8}$ alkylene-hydroxyl, $C_{1-8}$ alkylene-amino, $-C(R_a)(R_b)-N(R_a)-R_{4c}$, - $C(R_a)(R_b)-N(R_a)-C(O)-R_{4c}$, or $-C(R_a)(R_b)-N(R_a)-C(O)O-R_{4c}$; preferably, $R_{12}$ is $-CH_2-NH_2$, $-CH_2-NH-C(O)-R_{4c}$, $-CH_2-NH-C(O)O-R_{4c}$, or $C_4H_8OH$; preferably, $R_{12}$ is $-CH_2-NH_2$, or $-(CH_2)_4-OH$; and

each occurrence of R, $R_a$, $R_b$, and $R_{4c}$ is each independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, $-C(O)CH_2OH$, -S(O)H, $-S(O)_2H$, $-C(O)NH_2$, $-SO_2NH_2$, -OC(O)H, $-N(H)SO_2H$, $C_{1-6}$ alkyl, or $C_{1-6}$ alkylene hydroxyl; preferably, each occurrence of R, $R_a$, $R_b$, and $R_{4c}$ is each independently hydrogen, F, Cl, Br, I, $CH_2OH$, or $C_2H_4OH$;

when Q is 0, $R_{11}$ is S, and $R_{18}$ is H, $R_{12}$ is selected from hydrogen and - $CH_2N(R_{3A})(R_{4A})$;

$R_{14}$ and $R_{15}$ are each independently selected from hydrogen, halogen, $-NO_2$, -CN, -OR', $-N(R_{a1})(R_{b1})$, -C(O)R', $-CO_2R'$, -C(O)C(O)R', $-C(O)CH_2C(O)R'$, - $C(O)N(R_{a1})(R_{b1})$, $-SO_2N(R_{a1})(R_{b1})$, -OC(O)R', $-N(R')SO_2R'$, and $C_{1-6}$ alkyl; preferably, $R_{14}$ and $R_{15}$ are each independently selected from halogen, amino, -OR', and $C_{1-6}$ alkyl; more preferably, $R_{14}$ and $R_{15}$ are each independently selected from fluorine, chlorine, amino, $-O-C_{1-6}$ alkyl, and $C_{1-6}$ alkyl; or $R_{14}$ and $R_{15}$ together form a saturated or unsaturated 4- to 10-membered cycloalkyl, or a 5- or 6-membered heterocyclyl containing 1 to 3 heteroatoms each independently selected from N, O, and S, wherein the cycloalkyl or heterocyclyl is unsubstituted or substituted with one or more substituents independently selected from halogen, $-NO_2$, -CN, -OR, -SR, $-N(R_{a1})(R_{b1})$, -C(O)R, $-CO_2R$, -C(O)C(O)R, $-C(O)CH_2C(O)R$, -S(O)R, $-S(O)_2R$, $-C(O)N(R_{a1})(R_{b1})$, - $SO_2N(R_{a1})(R_{b1})$, -OC(O)R, $-N(R)SO_2R$, and $C_{1-6}$ alkyl, $C_{2-6}$ alkenyl, and $C_{2-6}$ alkynyl, each optionally substituted with R; preferably, the cycloalkyl or heterocyclyl is unsubstituted;

$R_{13}$ is hydrogen, $-C_{1-6}$ alkyl, or $-C_{1-6}$ alkylene-hydroxyl; preferably, $R_{5A}$ is hydrogen, $C_{1-6}$ alkyl, or $-(CH_2)_4$-hydroxyl;

each occurrence of $R_{3A}$ and $R_{4A}$ is each independently selected from hydrogen, halogen, -OR', $-N(R_{a1})(R_{b1})$, -C(O)R', -C(S)R', $-C(S)C(R_{a1})(R_{b1})OH$, - $C(O)CH_2N(R_{a1})(R_{b1})$, $-CO_2R'$, -C(O)C(O)R', $-C(O)CH_2C(O)R'$, $-C(O)C(R_{a1})(R_{b1})OH$, -C(O)-$C_{3-6}$ cycloalkylene-OH, -C(O)-$C_{1-6}$ alkylene-OH, -$C_{1-6}$ alkylene -OH, -C(O)-$C_{3-6}$ cycloalkylene-$C_{1-6}$ alkylene-OH, -C(O)O-$C_{1-6}$ alkylene-OH, -S(O)R', $-S(O)_2R$, - $C(O)N(R_{a1})(R_{b1})$, $-SO_2N(R_{a1})(R_{b1})$, -OC(O)R', $-N(R')SO_2R'$, and $C_{1-6}$ alkyl, $C_{1-6}$ alkenyl, and $C_{1-6}$ alkynyl, each optionally substituted with R'; preferably, $R_{4A}$ is selected from hydrogen or $C_{1-6}$ alkyl;

wherein each occurrence of R', $R_{a1}$, and $R_{b1}$ is independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, -S(O)H, $-S(O)_2H$, $-C(O)NH_2$, $-SO_2NH_2$, -OC(O)H, - $N(H)SO_2H$, $C_{1-6}$ alkyl, $C_{2-6}$ alkenyl, or $C_{2-6}$ alkynyl, 3-, 4-, 5-, 6-, 7-, or 8-membered cycloalkyl, 3- to 8-membered heterocyclyl containing 1, 2, or 3 heteroatoms independently selected from N, O, and S, 6- to 10-membered aryl, or 5- to 10-membered heteroaryl containing 1, 2, or 3 heteroatoms independently selected from N, O, and S; the $C_{1-6}$ alkyl, $C_{2-6}$ alkenyl, $C_{2-6}$ alkynyl, 3-, 4-, 5-, 6-, 7-, 8-membered cycloalkyl, 3- to 8-membered heterocyclyl, 6- to 10-membered aryl, or 5- to 10-membered heteroaryl is unsubstituted or substituted with one or more substituents independently selected from halogen, $-NO_2$, -CN, -OH, $-NH_2$, -C(O)H, $-CO_2H$, -C(O)C(O)H, $-C(O)CH_2C(O)H$, - S(O)H, $-S(O)_2H$, $-C(O)NH_2$, $-SO_2NH_2$, -OC(O)H, $-N(H)SO_2H$, $C_{1-6}$ alkyl, $C_{2-6}$ alkenyl or $C_{1-6}$ alkynyl, and hydroxyl-$C_{1-6}$ alkylene;

when $R_{14}$ is $-CH_3$, $R_{15}$ is fluorine, and $R_{13}$ is hydrogen, $R_{12}$ is

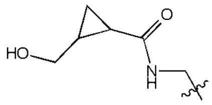,

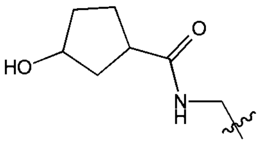, 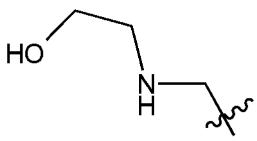; 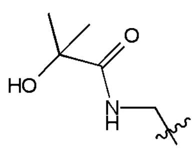, 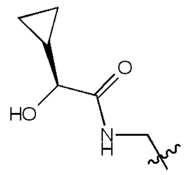,

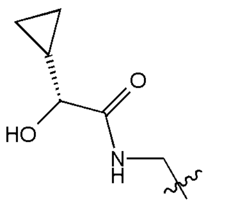, 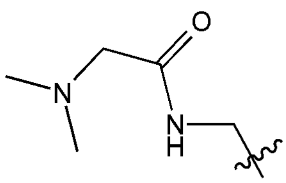, 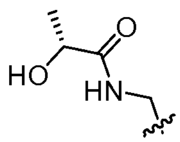, 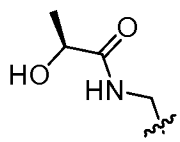

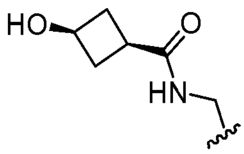 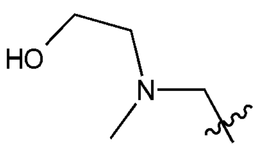; 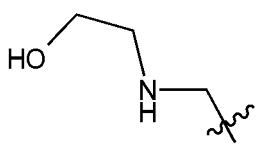, 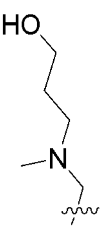, 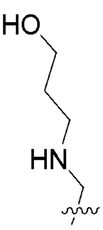,

or

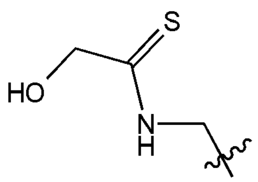,

and

when $R_{14}$ is -O-$CH_3$, $R_{15}$ is fluorine, and $R_{13}$ is hydrogen, $R_{12}$ is not

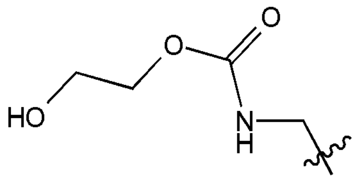 , 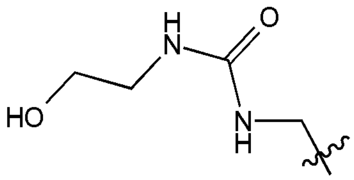 , 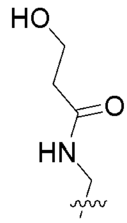 ;

and

when $R_{14}$ and $R_{15}$ form a 1,4-dioxane ring and $R_{13}$ is hydrogen, $R_{12}$ is -$CH_2NH_2$, - $CH_2NHC_2H_4OH$, -$CH_2N(CH_3)C_2H_4OH$, -$CH_2NHC_3H_6OH$,

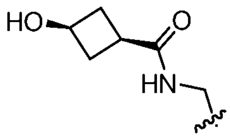 ,

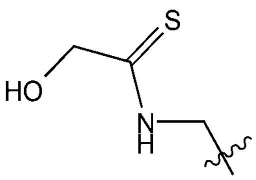 , 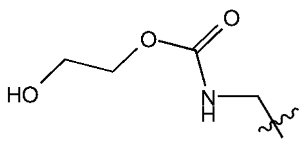 , 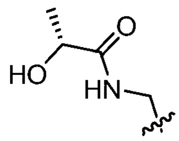 , 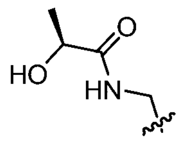 ,

or

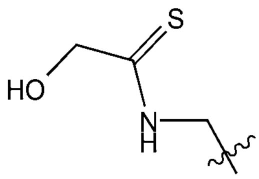 ;

and

when $R_{14}$ is fluorine, $R_{15}$ is fluorine, and $R_{13}$ is hydrogen, $R_{12}$ is not hydrogen; and

when $R_{14}$ is -$CH_3$, $R_{15}$ is chlorine, and $R_{13}$ is hydrogen, $R_{12}$ is selected from

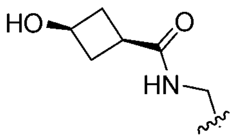 , 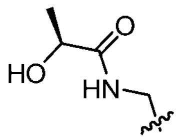 , or 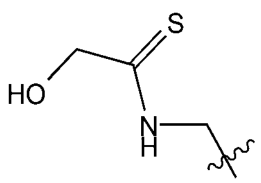 ;

and

when $R_{14}$ and $R_{15}$ form a 1,3-dioxolane ring, and $R_{13}$ is hydrogen, $R_{12}$ is selected from -$CH_2NHC_2H_4OH$, -$CH_2N(CH_3)C_2H_4OH$, -$CH_2NHC_3H_6OH$, or

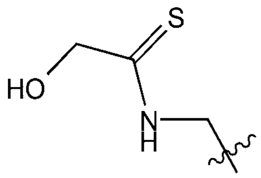

;

and

when $R_{13}$ is selected from $-(CH_2)_4$-OH, and $R_{12}$ is hydrogen, $R_{14}$ and $R_{15}$ are fluorine, or $R_{14}$ and $R_{15}$ form a 5-membered or 6-membered heterocyclyl containing two oxygen atoms.

**2.** The compound or a pharmaceutically acceptable salt thereof according to claim 1, wherein the structure of Formula 1 is selected from the following structures:

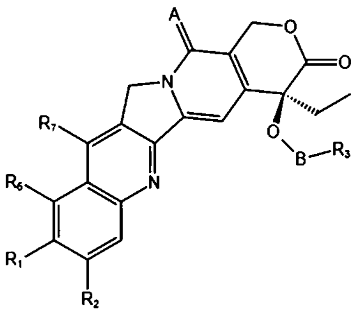

Formula 2A ,

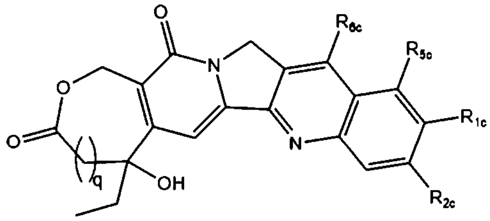

Formula 2B , and

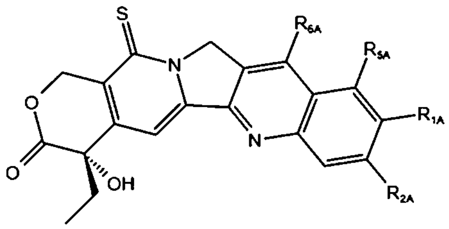

Formula 2C ;

when Formula 1 is Formula 2A:

wherein, $R_1$ and $R_2$ are each independently selected from hydrogen, halogen, $C_{1-8}$ alkyl, and $C_{1-8}$ haloalkyl; preferably, $R_1$ and $R_2$ are each independently selected from hydrogen, F, Cl, Br, I, $C_{1-3}$ alkyl, and $C_{1-3}$ haloalkyl; preferably, $R_1$ and $R_2$ are each independently $-CH_3$ or F; preferably, $R_1$ is $-CH_3$, and/or $R_2$ is F;
A is selected from oxygen and sulfur;
B is selected from -C(O)- and -P(O)(OH)-; preferably, B is -C(O)-;
$R_3$ is selected from $-(CH_2CH_2O)_aC_{1-6}$ alkyl,

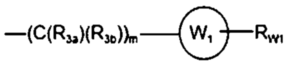

,

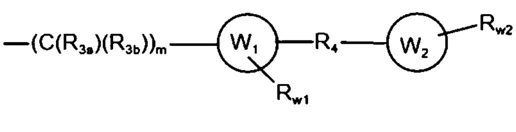

,

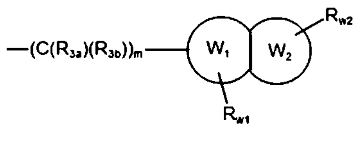

,

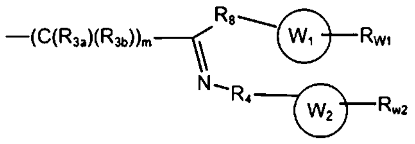
,
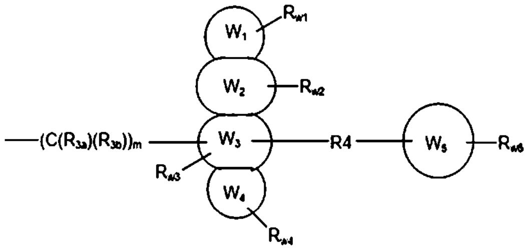
,

and

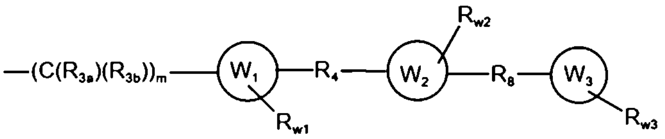
,

wherein when $-(C(R_{3a})(R_{3b}))_m$-contains methylene units, the n methylene units of the $-(C(R_{3a})(R_{3b}))_m$- are each independently replaced by $-N(R_5)C(O)-$, -C(O)-, -OC(O)-, $-NR_5-$, -O-, -S-, -SO-, $-SO_2-$, $-P(R_5)-$, $-P(=O)(R_5)-$, $-N(R_5)SO_2-$, -C(=S)-, $-C(=NR_5)-$, -N=N-, or -N=CH-;
a is an integer greater than or equal to 1; preferably, a is an integer from 1 to 20; more preferably, a is selected from 1, 2, 3, 4, 5, 6, 7, and 8;
each occurrence of $R_4$ and $R_8$ is each independently a single bond or independently selected from -O-, $C_{1-8}$ alkylene, $C_{1-8}$ haloalkylene, $-N(R_5)C(O)-$, -C(O)-, -OC(O)-, - $NR_5$-, -S-, -SO-, $-SO_2-$, $-P(R_5)-$, $-P(=O)(R_5)-$, $-N(R_5)SO_2-$, -C(=S)-, $-C(=NR_5)-$, -N =N-, and $-N=CR_5-$;
each occurrence of ring $W_1$, ring $W_2$, ring $W_3$, ring $W_4$, and ring $W_5$ is each independently selected from 6-10 membered aryl or arylene, 5-10 membered heteroaryl or heteroarylene, 3-10 membered cycloalkyl or cycloalkylene, and 3-10 membered heterocyclyl or heterocyclylene; preferably, the heteroaryl, heteroarylene, heterocyclylene, and heterocyclyl each independently contain 1, 2, 3, or 4 heteroatoms independently selected from N, O, P, and S; and when $R_3$ is

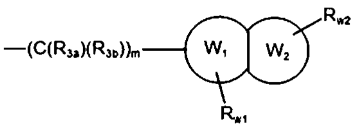
,

$W_1$ and $W_2$ are fused together; when $R_3$ is

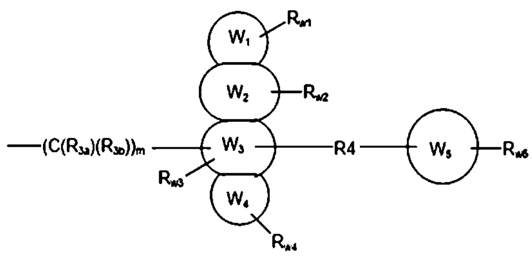
,

$W_1$ and $W_2$ are fused together, $W_2$ and $W_3$ are fused together, and $W_3$ and $W_4$ are fused together;
when ring $W_1$, ring $W_2$, ring $W_3$, ring $W_4$, and ring $W_5$ is each independently selected from 3-10 membered cycloalkyl, cycloalkylene, heterocyclylene, and heterocyclyl, wherein the 3- to 10-membered cycloalkyl, cycloalkylene, heterocyclylene, and heterocyclyl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is independently selected from hydrogen, protium, deuterium, tritium, halogen, =O, =S, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, and $C_{1-6}$ alkyl optionally substituted with R; preferably, each occurrence of $R_{w1}$, $R_{w2}$, $R_{w3}$, $R_{w4}$, and $R_{w5}$ is each independently selected from hydrogen, deuterium, F, Cl, Br, I, hydroxyl, =O, =S, $-NH_2$, $-NO_2$, -CN, -COOH, - $OC_{1-3}$ alkyl, $-C_{1-3}$ alkyl, and $C_{1-3}$ alkylene-hydroxyl;

when ring $W_1$, ring $W_2$, ring $W_3$, ring $W_4$, and ring $W_5$ is each independently selected from 6- to 10-membered aryl, 6- to 10-membered arylene, 5- to 10-membered heteroarylene, and 5- to 10-membered heteroaryl, wherein the aryl, arylene, heteroarylene, and heteroaryl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is each independently selected from hydrogen, protium, deuterium, tritium, halogen, $-NO_2$, -CN, -OR, -SR, $-N(R_a)(R_b)$, -C(O)R, $-CO_2R$, -C(O)C(O)R, - $C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, and $C_{1-6}$ alkyl optionally substituted with R; preferably, each occurrence of $R_{w1}$, $R_{w2}$, $R_{w3}$, $R_{w4}$, and $R_{w5}$ is each independently selected from hydrogen, deuterium, F, Cl, Br, I, hydroxyl, $-NH_2$, $-NO_2$, -CN, $-OC_{1-3}$ alkyl, -COOH, and $C_{1-3}$ alkyl;

$R_6$ is selected from hydrogen, halogen, $C_{1-8}$ alkyl, and $C_{1-8}$ haloalkyl; preferably, $R_6$ is hydrogen; $R_7$ is selected from $C_{1-8}$ alkylene-$N(R_a)(R_b)$, $C_{1-8}$ haloalkylene-$N(R_a)(R_b)$, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, - N(R) $SO_2R$, $-O-CH_2C(O)N(R_a)(R_b)$, and $C_{1-6}$ alkyl optionally substituted with R; preferably, $R_7$ is selected from $C_{1-3}$ alkylene-$N(R_a)(R_b)$ and $C_{1-3}$ haloalkylene-$N(R_a)(R_b)$; preferably, $R_7$ is selected from $-CH_2N(R_a)(R_b)$; or $R_6$ and $R_7$ together with the carbon atoms to which they are attached form a saturated or unsaturated 5- to 10-membered cycloalkyl, or 5- to 10-membered heterocyclyl containing 1, 2, or 3 heteroatoms each independently selected from N, O, P, and S, wherein the cycloalkyl or heterocyclyl is optionally substituted with one or more substituents independently selected from halogen, $-NO_2$, -CN, -OR, -SR, $-N(R_a)(R_b)$, -C(O)R, $-CO_2R$, -C(O)C(O)R, - C(O) $CH_2C(O)R$, -S(O)R, $-S(O)_2R$, $-C(O)N(R_a)(R_b)$, $-SO_2N(R_a)(R_b)$, -OC(O)R, - $N(R)SO_2R$, and $C_{1-6}$ alkyl optionally substituted with R; preferably, $R_6$ and $R_7$ together with the carbon atoms to which they are attached form a 6-membered cycloalkyl, wherein the cycloalkyl is substituted with $-N(R_a)(R_b)$;

each occurrence of $R_{3a}$, $R_{3b}$, and $R_5$ is each independently hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ alkyl optionally substituted with R;

each occurrence of R, $R_a$, and $R_b$ is each independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, $-C(O)CH_2OH$, -S(O)H, $-S(O)_2H$, -C(O) $NH_2$, $-SO_2NH_2$, -OC(O)H, $-N(H)SO_2H$, $C_{1-6}$ alkylene-hydroxyl, or $C_{1-6}$ alkyl; and

m and n are each independently an integer greater than or equal to 1, and $m \geq n$; preferably, m and n are each independently 1, 2, 3, 4, 5, 6, 7, 8, 9, or 10, and $m \geq n$; preferably, m is 1, 2, 3, 4, 5, 6, 7, 8, 9, or 10, -n is 0, 1, 2, 3, 4, or 5, and $m \geq n$;

when Formula 1 is Formula 2B:

wherein, q is 1, 2, or 3;

$R_{1c}$, $R_{2c}$, and $R_{5c}$ are each independently selected from hydrogen, halogen, $-NO_2$, -CN, -OR, -SR, $-N(R_a)$ $(R_b)$, -C(O)R, $-CO_2R$, -C(O)C(O)R, $-C(O)CH_2C(O)R$, -S(O)R, -S(O)2R, $-C(O)N(R_a)(R_b)$, $-SO_2N(R_a)(R_b)$, -OC(O)R, -N(R)SO2R, and $C_{1-6}$ alkyl optionally substituted with R; preferably, $R_{1c}$, $R_{2c}$, and $R_{5c}$ are each independently selected from hydrogen, F, Cl, Br, I, $C_{1-3}$ alkyl, $-NO_2$, -CN, and $-O-C_{1-3}$ alkyl; or $R_{1c}$ and $R_{2c}$ together with the carbon atoms to which they are attached form a saturated or unsaturated 5- to 10-membered cycloalkyl, or a 5- to 10-membered heterocyclyl containing 1, 2, or 3 heteroatoms independently selected from N, O, and S, wherein the cycloalkyl, or heterocyclyl is unsubstituted or substituted with one or more substituents independently selected from halogen, $-NO_2$, -CN, -OR, -SR, $-N(R_a)(R_b)$, -C(O)R, - $CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, and $C_{1-6}$ alkyl optionally substituted with R; preferably, $R_{1c}$ and $R_{2c}$ together with the carbon atoms to which they are attached form a saturated or unsaturated 5- to 6-membered heterocyclyl containing two oxygen atoms;

$R_{6c}$ is $C_{1-8}$ alkylene-hydroxyl, $C_{1-8}$ alkylene-amino, $-C(R_a)(R_b)-N(R_a)-R_{4c}$, - $C(R_a)(R_b)-N(R_a)-C(O)-R_{4c}$, or $-C(R_a)(R_b)-N(R_a)-C(O)O-R_{4c}$; preferably, $R_{6c}$ is $-CH_2-NH_2$, $-CH_2-NH-C(O)-R_{4c}$, $-CH_2-NH-C(O)O-R_{4c}$, or $C_4H_8OH$; preferably, $R_{6c}$ is $-CH_2-NH_2$, or $-(CH_2)_4-OH$; and

each occurrence of R, $R_a$, $R_b$, and $R_{4c}$ is each independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, $-C(O)CH_2OH$, -S(O)H, $-S(O)_2H$, $-C(O)NH_2$, $-SO_2NH_2$, -OC(O)H, $-N(H)SO_2H$, $C_{1-6}$ alkyl, or $C_{1-6}$ alkylene-hydroxyl; preferably, each occurrence of R, $R_a$, $R_b$, and $R_{4c}$ is each independently hydrogen, F, Cl, Br, I, $CH_2OH$, or $C_2H_4OH$;

when Formula 1 is Formula 2C:

$R_{6A}$ is selected from hydrogen and $-CH_2N(R_{3A})(R_{4A})$;

$R_{1A}$ and $R_{2A}$ are each independently selected from hydrogen, halogen, $-NO_2$, -CN, -OR', $-N(R_{a1})(R_{b1})$, -C(O)R', $-CO_2R'$, -C(O)C(O)R', $-C(O)CH_2C(O)R'$, - $C(O)N(R_{a1})(R_{b1})$, $-SO_2N(R_{a1})(R_{b1})$, -OC(O)R', $-N(R')SO_2R'$, and $C_{1-6}$ alkyl; preferably, $R_{1A}$ and $R_{2A}$ are each independently selected from halogen,

amino, -OR', and $C_{1-6}$ alkyl; more preferably, $R_{1A}$ and $R_{2A}$ are each independently selected from fluorine, chlorine, amino, -O-$C_{1-6}$ alkyl, and $C_{1-6}$ alkyl;

or $R_{1A}$ and $R_{2A}$ together form a saturated or unsaturated 4- to 10-membered cycloalkyl, or 5-, or 6-membered heterocyclyl containing 1 to 3 heteroatoms independently selected from N, O, and S, wherein the cycloalkyl, or heterocyclyl is unsubstituted or substituted with one or more substituents selected from halogen, -$NO_2$, -CN, -OR, -SR, -N($R_{a1}$)($R_{b1}$), -C(O)R, -$CO_2$R, -C(O)C(O)R, -C(O)$CH_2$C(O)R, -S(O)R, -S(O)2R, -C(O)N($R_{a1}$)($R_{b1}$), -$SO_2$N($R_{a1}$)($R_{b1}$), -OC(O)R, -N(R)SO2R, and $C_{1-6}$ alkyl, $C_{1-6}$ alkenyl, and $C_{1-6}$ alkynyl optionally substituted with R; preferably, the cycloalkyl or heterocyclyl is unsubstituted;

$R_{5A}$ is hydrogen, or $C_{1-6}$ alkyl, or -$C_{1-6}$ alkylene-hydroxyl; preferably, $R_{5A}$ is hydrogen, $C_{1-6}$ alkyl, or -$(CH_2)_4$-hydroxyl;

each occurrence of $R_{3A}$ and $R_{4A}$ is independently selected from hydrogen, halogen, -OR', -N($R_{a1}$)($R_{b1}$), -C(O)R', -C(S)R', -C(S)C($R_{a1}$)($R_{b1}$)OH, -C(O)$CH_2$N($R_{a1}$)($R_{b1}$), - $CO_2$R', -C(O)C(O)R', -C(O)$CH_2$C(O) R', -C(O)C($R_{a1}$)($R_{b1}$)OH, -C(O)-$C_{3-6}$ cycloalkylene-OH, -C(O)-$C_{1-6}$ alkylene-OH, -C(O)-$C_{3-6}$ cycloalkylene-$C_{1-6}$ alkylene-OH, -C(O)O-$C_{1-6}$ alkylene-OH, -S(O)R', -S$(O)_2$R, -C(O)N($R_{a1}$)($R_{b1}$), -$SO_2$N($R_{a1}$) ($R_{b1}$), -OC(O)R', -N(R')$SO_2$R', and $C_{1-6}$ alkyl optionally substituted with R', $C_{2-6}$ alkenyl, and $C_{2-6}$ alkynyl; preferably, $R_{4A}$ is selected from hydrogen, or $C_{1-6}$ alkyl;

wherein each occurrence of R', $R_{a1}$, and $R_{b1}$ is independently hydrogen, protium, deuterium, tritium, halogen, -$NO_2$, -CN, -OH, -SH, -$NH_2$, -C(O)H, -$CO_2$H, - C(O)C(O)H, -C(O)$CH_2$C(O)H, -S(O)H, -S$(O)_2$H, -C(O)$NH_2$, -$SO_2NH_2$, -OC(O)H, - N(H)$SO_2$H, $C_{1-6}$ alkyl, $C_{2-6}$ alkenyl, or $C_{2-6}$ alkynyl, 3, 4, 5, 6, 7, or 8-membered cycloalkyl, 3- to 8-membered heterocyclyl containing 1, 2, or 3 heteroatoms independently selected from N, O, and S, 6- to 10-membered aryl, or 5- to 10-membered heteroaryl containing 1, 2, or 3 heteroatoms independently selected from N, O, and S; the $C_{1-6}$ alkyl, $C_{2-6}$ alkenyl, $C_{2-6}$ alkynyl, 3-, 4-, 5-, 6-, 7-, or 8-membered cycloalkyl, 3-to 8-membered heterocyclyl, 6- to 10-membered aryl, or 5- to 10-membered heteroaryl is unsubstituted or substituted with one or more substituents selected from halogen, - $NO_2$, -CN, -OH, -$NH_2$, -C(O)H, -$CO_2$H, -C(O)C(O)H, -C(O)$CH_2$C(O)H, -S(O)H, - S$(O)_2$H, -C(O)$NH_2$, -$SO_2NH_2$, -OC(O)H, -N(H)$SO_2$H, $C_{1-6}$ alkyl, $C_{2-6}$ alkenyl, or $C_{1-6}$ alkynyl, and hydroxyl-$C_{1-6}$ alkylene;

wherein, when $R_{1A}$ is -$CH_3$, $R_{2A}$ is fluorine, and $R_{5A}$ is hydrogen, $R_{6A}$ is

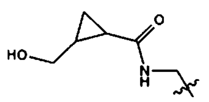 , 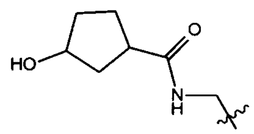 , 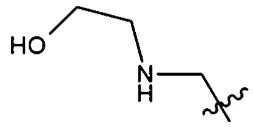 ; 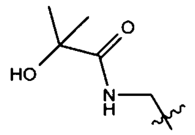 ,

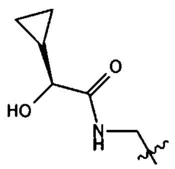 , 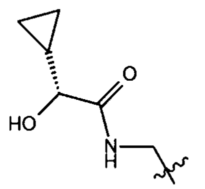 , 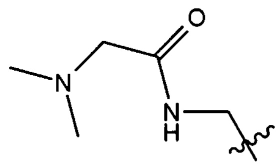 , 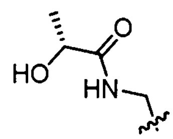 ,

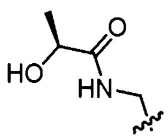 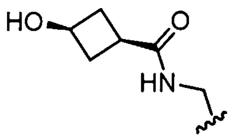 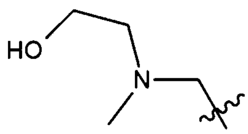 ; 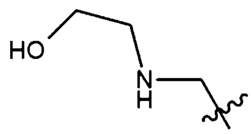 ,

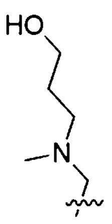 , 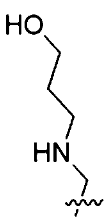 , or 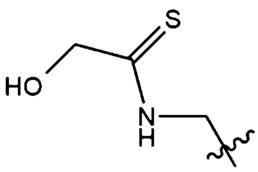 ;

and

when $R_{1A}$ is -O-$CH_3$, $R_{2A}$ is fluorine, and $R_{5A}$ is hydrogen, $R_{6A}$ is not

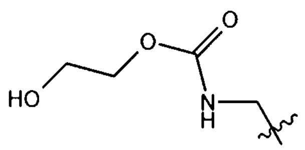
,
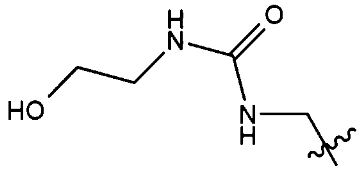
, or
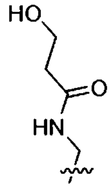
;

and

when $R_{1A}$ and $R_{2A}$ form a 1,4-dioxane, and $R_{5A}$ is hydrogen, $R_{6A}$ is -$CH_2NH_2$, - $CH_2NHC_2H_4OH$, -$CH_2N(CH_3)C_2H_4OH$, -$CH_2NHC_3H_6OH$,

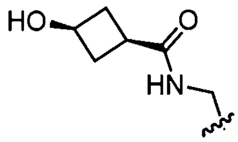
,

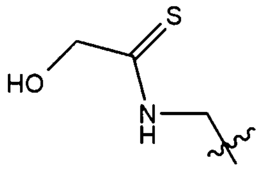
,
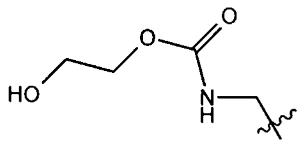
,
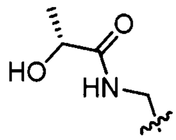
,
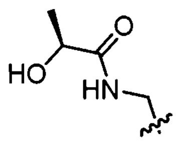
,

or

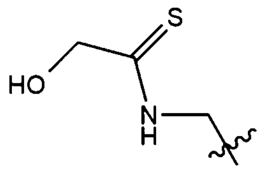
;

and

when $R_{1A}$ is fluorine, $R_{2A}$ is fluorine, and $R_{5A}$ is hydrogen, $R_{6A}$ is not hydrogen; and

when $R_{1A}$ is -$CH_3$, $R_{2A}$ is chlorine, and $R_{5A}$ is hydrogen, $R_{6A}$ is selected from

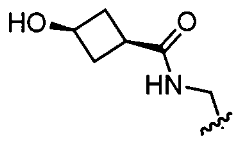
,
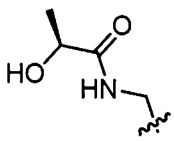
, or
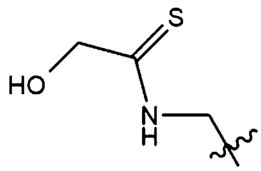
;

and

when $R_{1A}$ and $R_{2A}$ form a 1,3-dioxolane, and $R_{5A}$ is hydrogen, $R_{6A}$ is selected from -$CH_2NHC_2H_4OH$, -$CH_2N(CH_3)C_2H_4OH$, -$CH_2NHC_3H_6OH$, or

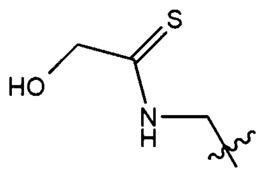
;

and

wherein, when $R_{5A}$ is selected from -$(CH_2)_4$-hydroxyl, $R_{6A}$ is hydrogen, $R_{1A}$ and $R_{2A}$ are fluorine, or $R_{1A}$ and $R_{2A}$ form a 5- or 6-membered heterocyclyl containing two oxygen atoms.

**3.** The compound or a pharmaceutically acceptable salt thereof according to claim 1 or 2, wherein, when Formula 1 is Formula 2A:

$R_1$ and $R_2$ are each independently selected from hydrogen, F, Cl, Br, I, $C_{1-3}$ alkyl, and $C_{1-3}$ haloalkyl; preferably, $R_1$ and $R_2$ are each independently -$CH_3$ or F; preferably, $R_1$ is -$CH_3$, and/or, $R_2$ is F; and/or
a is an integer from 1 to 20; more preferably, a is selected from 1, 2, 3, 4, 5, 6, 7, and 8; and/or
$R_6$ is hydrogen, and $R_7$ is selected from $C_{1-3}$ alkylene-N($R_a$)($R_b$), and $C_{1-3}$ haloalkylene-N($R_a$)($R_b$); preferably, $R_7$ is selected from -$CH_2$N($R_a$)($R_b$); or $R_6$ and $R_7$ together with the carbon atoms to which they are attached form a 6-membered cycloalkyl, wherein the cycloalkyl is substituted with -N($R_a$)($R_b$); and/or
m and n are each independently 1, 2, 3, 4, 5, 6, 7, 8, 9, or 10, and $m \geq n$; preferably, m is 1, 2, 3, 4, 5, 6, 7, 8, 9, or 10, -n is 0, 1, 2, 3, 4, or 5, and $m \geq n$; and/or
each occurrence of ring $W_1$, ring $W_2$, ring $W_3$, ring $W_4$, and ring $W_5$ is each independently selected from 6-, or 8-membered aryl, or heteroarylene, 5-, 6-, 7-, or 8-membered heteroaryl or heteroarylene containing 1, 2, 3, or 4 heteroatoms independently selected from N, O, and S, 4-, 5-, 6-, 7-, or 8-membered cycloalkyl, or cycloalkylene, and 4-, 5-, 6-, 7-, or 8-membered heterocyclyl or heterocyclylene containing 1, 2, 3, or 4 heteroatoms independently selected from N, O, and S, , when each occurrence of ring $W_1$, ring $W_2$, ring $W_3$, ring $W_4$, and ring $W_5$ is each independently selected from 4- to 8-membered cycloalkyl, cycloalkylene, heterocyclylene, and heterocyclyl, the 4-, 5-, 6-, 7-, or 8-membered cycloalkyl, cycloalkylene, heterocyclylene, and heterocyclyl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is each independently selected from hydrogen, deuterium, F, Cl, Br, I, hydroxyl, =O, =S, -$NH_2$, -$NO_2$, -CN, -COOH, -$OC_{1-3}$ alkyl, -$C_{1-3}$ alkyl, and $C_{1-3}$ alkylene-hydroxyl; when each occurrence of ring $W_1$, ring $W_2$, ring $W_3$, ring $W_4$, and ring $W_5$ is each independently selected from 6-, or 8-membered aryl, 6-, or 8-membered arylene, 5-, 6-, 7-, or 8-membered heteroarylene, and heteroaryl, the aryl, arylene, heteroarylene, and heteroaryl are each optionally substituted with one or more substituents selected from $R_{w1}$, $R_{w2}$, $R_{w3}$, $R_{w4}$, or $R_{w5}$, and each occurrence of $R_{w1}$, $R_{w2}$, $R_{w3}$, $R_{w4}$, and $R_{w5}$ is independently selected from hydrogen, deuterium, F, Cl, Br, I, hydroxyl, -$NH_2$, -$NO_2$, -CN, -$OC_{1-3}$ alkyl, -COOH, and $C_{1-3}$ alky; preferably, each occurrence of ring $W_1$, ring $W_2$, ring $W_3$, ring $W_4$, and ring $W_5$ is each independently selected from phenyl or phenylene , pyridinyl, or pyridinylene, pyrazolyl, or pyrazolylene, or

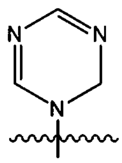

,

optionally substituted with hydrogen, deuterium, F, Cl, Br, I, hydroxyl, -$NH_2$, -$NO_2$, -CN, -$OC_{1-3}$ alky, -COOH, or $C_{1-3}$ alky , or tetrahydropyranyl or tetrahydropyranylene , dioxolanyl, dioxolanylene, tetrahydrofuranyl, or tetrahydrofuranylene,

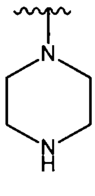

, or

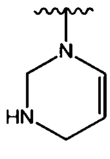

,

optionally substituted with hydrogen, deuterium, F, Cl, Br, I, hydroxyl, =O, =S, -$NH_2$, -$NO_2$, - CN, -COOH, -$OC_{1-3}$ alkyl, -$C_{1-3}$ alkyl, or $C_{1-3}$ alkylene-hydroxyl,

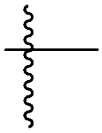

represents the attachment point; and/or
when $R_3$ is

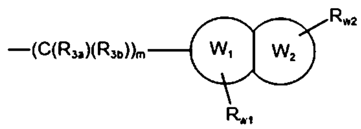,

ring $W_1$ and ring $W_2$ are fused to form the following fused ring structure:

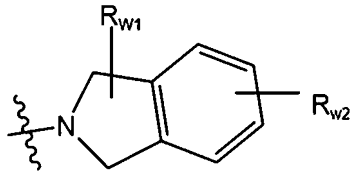,

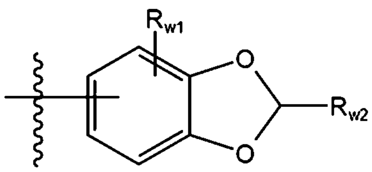, 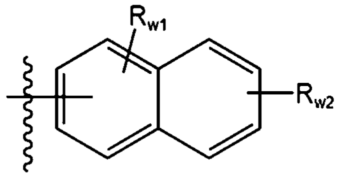

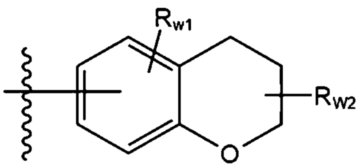, 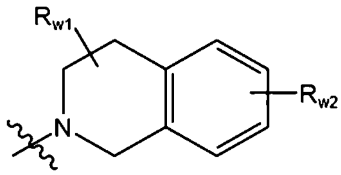, or 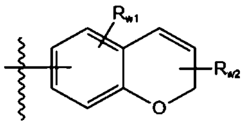,

wherein, each occurrence of $R_{w1}$ and $R_{w2}$ is each independently selected from hydrogen, deuterium, F, Cl, Br, I, hydroxyl, =O, =S, $-NH_2$, $-NO_2$, -CN, -COOH, $-OC_{1-3}$ alkyl, $-C_{1-3}$ alkyl, and $C_{1-3}$ alkylene-hydroxyl,

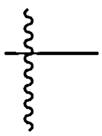

represents the attachment point;
when Formula 1 is Formula 2B:

$R_{1c}$, $R_{2c}$, and $R_{5c}$ are each independently selected from hydrogen, F, Cl, Br, I, $C_{1-3}$ alkyl, $-NO_2$, -CN, and -O-$C_{1-3}$ alkyl; or $R_{1c}$ and $R_{2c}$ together with the carbon atoms to which they are attached form a saturated or unsaturated 5-6 membered heterocyclyl containing two oxygen atoms; and/or
$R_{6c}$ is $-CH_2-NH_2$, $-CH_2-NH-C(O)-R_{4c}$, $-CH_2-NH-C(O)O-R_{4c}$, or $C_4H_8OH$; preferably, $R_{6c}$ is $-CH_2-NH_2$ or $-(CH_2)_4-OH$; and/or
each occurrence of R, $R_a$, $R_b$, and $R_{4c}$ is each independently selected from hydrogen, F, Cl, Br, I, $CH_2OH$, and $C_2H_4OH$;
when Formula 1 is Formula 2C:

$R_{1A}$ and $R_{2A}$ are each independently selected from halogen, amino, -OR', and $C_{1-6}$ alkyl; more preferably, $R_{1A}$ and $R_{2A}$ are each independently selected from fluorine, chlorine, amino, -O-$C_{1-6}$ alkyl, and $C_{1-6}$ alkyl; and/or

each occurrence of $R_{3A}$ is each independently selected from hydrogen, -C(O)R', - C(S)R', -C(S)C($R_{a1}$)($R_{b1l}$)OH, -C(O)$CH_2$N($R_{a1}$)($R_{b1}$), -$CO_2$R', -C(O)C($R_{a1}$)($R_{b1}$)OH, - C(O)-$C_{3-6}$ cycloalkylene-OH, -C(O)-$C_{1-6}$ alkylene-OH, -C(O)-$C_{3-6}$ cycloalkylene-$C_{1-6}$ alkylene-OH, -C(O)O-$C_{1-6}$ alkylene-OH, and $C_{1-6}$ alkyl, $C_{1-6}$ alkenyl, and $C_{1-6}$ alkynyl optionally substituted with R'; and/or

preferably, $R_{4A}$ is selected from hydrogen or $C_{1-6}$ alkyl; and/or

each occurrence of R', $R_{a1}$, and $R_{b1}$ is each independently hydrogen, fluorine, chlorine, -OH, $C_{1-6}$ alkyl, $C_{2-6}$ alkenyl,or $C_{2-6}$ alkynyl, 3, 4, 5, or 6-membered cycloalkyl, or 3, 4, 5, or 6-membered heterocycloalkyl containing heteroatoms independently selected from N, O, and S, the $C_{1-6}$ alkyl, $C_{2-6}$ alkenyl, $C_{2-6}$ alkynyl, 3, 4, 5,or 6-membered cycloalkyl, or 3, 4, 5, or 6-membered heterocyclyl is unsubstituted or substituted with 1 or 2 substituents independently selected from fluorine, chlorine, - $NO_2$, -CN, -OH, -$NH_2$, $C_{1-6}$ alkyl, $C_{2-6}$ alkenyl, $C_{2-6}$ alkynyl, and hydroxyl-$C_{1-6}$ alkylene.

**4.** The compound or a pharmaceutically acceptable salt thereof according to any one of claims 1-3, wherein the compound is selected from:

wherein $R_3$ is selected from the following groups:

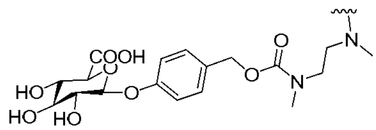,
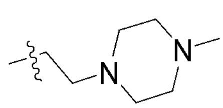,
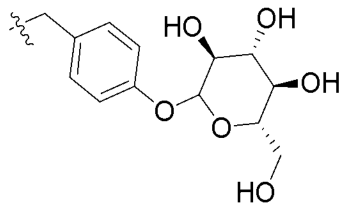,

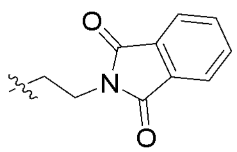,
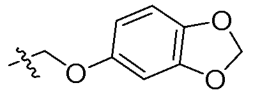,
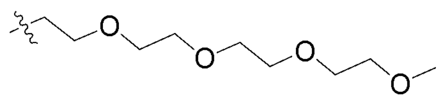,

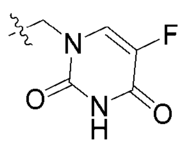,
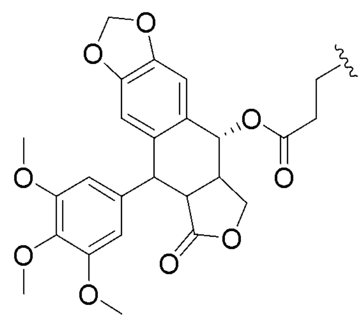,
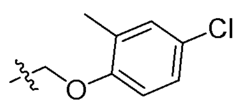,
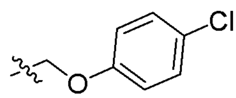,

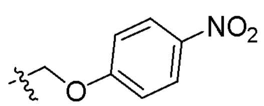,
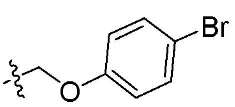,
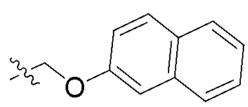,

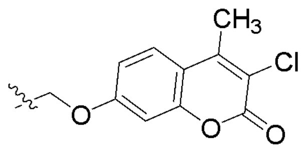,
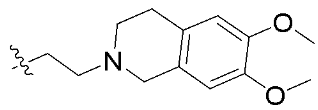,
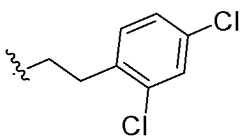,
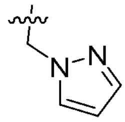,

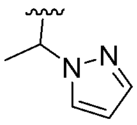,
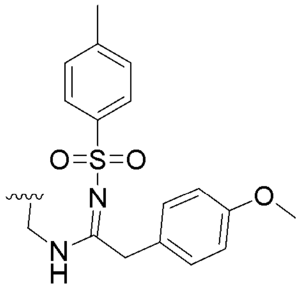,
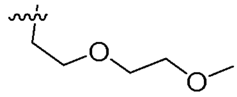,
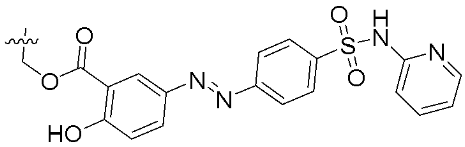,
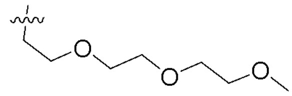,
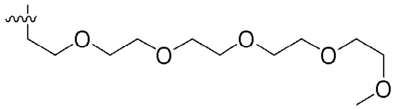,
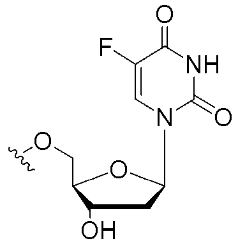,
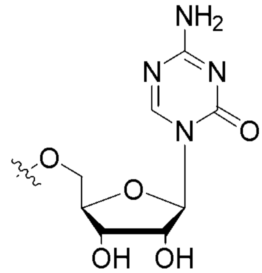,

and

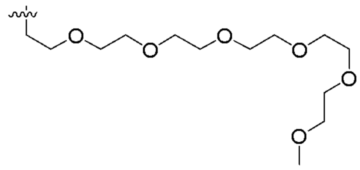,

wherein

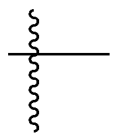

represents the attachment point.

**5.** A compound represented by Formula 2, or a pharmaceutically acceptable salt thereof,

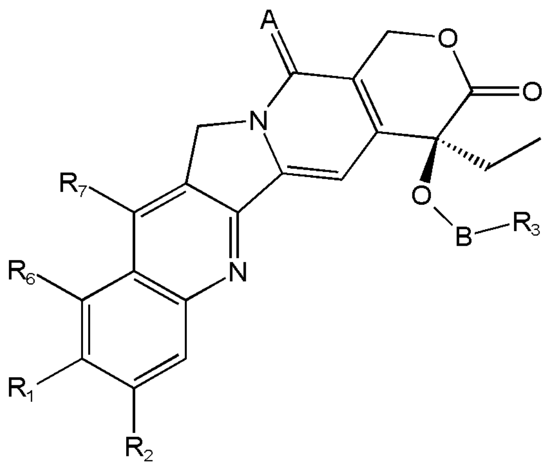

Formula 2

wherein, $R_1$ and $R_2$ are each independently selected from hydrogen, halogen, $C_{1-8}$ alkyl, and $C_{1-8}$ haloalkyl;
A is selected from oxygen or sulfur;
B is selected from -C(O)- or -P(O)(OH)-;
$R_3$ is selected from $-(C(R_{3a})(R_{3b}))_m-R_{3c}$,

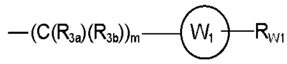

,

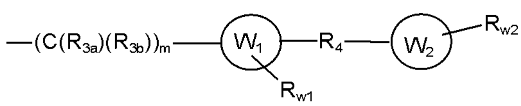

,

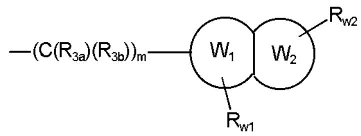

,

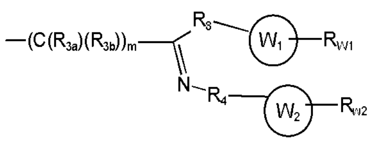

,

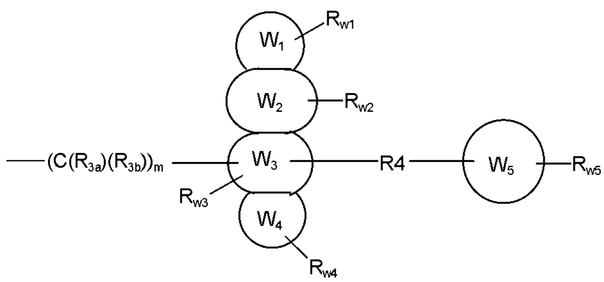

,

or

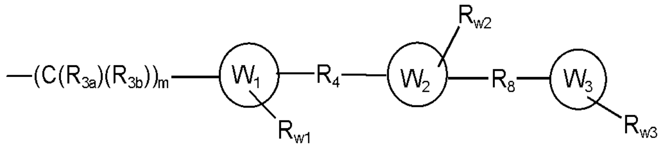

, wherein when the $-(C(R_{3a})(R_{3b}))_m-$ contains methylene units, the n methylene units of the $-(C(R_{3a})(R_{3b}))_m-$ are each independently replaced by $-N(R_5)C(O)-$, $-C(O)N(R_5)-$, -C(O)-, -OC(O)-, - C(O)O-, $-NR_5-$, -O-, -S-, -SO-, $-SO_2-$, $-P(R_5)-$, $-P(=O)(R_5)-$, $-N(R_5)SO_2-$, $-SO_2N(R_5)-$, -C( = S)-, $-C( = NR_5)-$, -N = N-, -CH = N-, or -N = CH-, when $R_3$ is

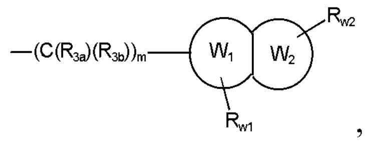

,

$W_1$ and $W_2$ are fused together;

$R_4$ and $R_8$ are each independently a single bond or selected from -O-, $C_{1-8}$ alkylene, $C_{1-8}$ haloalkylene, $-N(R_5)C(O)-$, $-C(O)N(R_5)-$, -C(O)-, -OC(O)-, -C(O)O-, $-NR_5-$, -S-, - SO-, $-SO_2-$, $-P(R_5)-$, $-P(=O)(R_5)-$, $-N(R_5)SO_2-$, $-SO_2N(R_5)-$, -C(=S)-, $-C(=NR_5)-$, -N =N-, -CH=N-, or -N=CH-;

ring $W_1$, ring $W_2$, ring $W_3$, ring $W_4$, and ring $W_5$ are each independently selected from 6- to 10-membered aryl, 5- to 10-membered heteroaryl, 3- to 10- membered cycloalkyl, and 3- to 10-membered heterocyclyl;

when ring $W_1$, ring $W_2$, ring $W_3$, ring $W_4$, and ring $W_5$ are each independently selected from cycloalkyl or heterocyclyl, the cycloalkyl or heterocyclyl is substituted with one or more $R_{w1}$, $R_{w2}$, $R_{w3}$ , $R_{w4}$, or $R_{w5}$, each occurrence of $R_{w1}$, $R_{w2}$ , $R_{w3}$, $R_{w4}$ , or $R_{w5}$ is each independently selected from hydrogen, protium, deuterium, tritium, halogen, =O, =S, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, or $C_{1-6}$ aliphatic group optionally substituted with R;

when ring $W_1$, ring $W_2$, ring $W_3$, ring $W_4$, and ring $W_5$ are each independently selected from aryl or heteroaryl, the aryl or heteroaryl is substituted with one or more $R_{w1}$, $R_{w2}$, or $R_{w3}$, $R_{w4}$ , or $R_{w5}$, each occurrence of $R_{w1}$, $R_{w2}$, $R_{w3}$, $R_{w4}$ , or $R_{w5}$ is each independently selected from hydrogen, protium, deuterium, tritium, halogen, $-NO_2$, - CN, -OR, -SR, $-N(R_a)(R_b)$, -C(O)R, $-CO_2R$, -C(O)C(O)R, $-C(O)CH_2C(O)R$, -S(O)R, - $S(O)_2R$, $-C(O)N(R_a)(R_b)$, $-SO_2N(R_a)(R_b)$, -OC(O)R, $-N(R)SO_2R$, or $C_{1-6}$ aliphatic group optionally substituted with R;

$R_6$ is selected from hydrogen, halogen, $C_{1-8}$ alkyl, $C_{1-8}$ haloalkyl; preferably, $R_6$ is selected from hydrogen;

$R_7$ is selected from $C_{1-8}$ alkylene-$N(R_a)(R_b)$, $C_{1-8}$ haloalkylene-$N(R_a)(R_b)$, halogen, $-NO_2$, -CN, -OR, -SR, $-N(R_a)(R_b)$, -C(O)R, $-CO_2R$, -C(O)C(O)R, $-C(O)CH_2C(O)R$, - S(O)R, $-S(O)_2R$, $-C(O)N(R_a)(R_b)$, $-SO_2N(R_a)(R_b)$, -OC(O)R, $-N(R)SO_2R$, or $C_{1-6}$ aliphatic group optionally substituted with R; preferably, $R_7$ is selected from $C_{1-8}$ alkylene-$N(R_a)(R_b)$, or $C_{1-8}$ haloalkylene-$N(R_a)(R_b)$; preferably, $R_7$ is selected from - $CH_2N(R_a)(R_b)$;

or $R_6$ and $R_7$ together with the carbon atoms to which they are attached form a saturated or unsaturated 5- to 10-membered cycloalkyl or 6- to 10-membered heterocyclyl containing 1 to 3 heteroatoms each independently selected from N, O, and S, wherein the cycloalkyl or heterocyclyl is substituted with one or more substituents independently selected from halogen, $-NO_2$, -CN, -OR, -SR, $-N(R_a)(R_b)$, -C(O)R, - $CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R; preferably, $R_6$ and $R_7$ together with the carbon atoms to which they are attached form a 6-membered cycloalkyl, the cycloalkyl is substituted with $-N(R_a)(R_b)$;

each occurrence of $R_{3a}$, $R_{3b}$, $R_{3c}$, and $R_5$ is independently hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ aliphatic group optionally substituted with R;

each occurrence of R, $R_a$, and $R_b$ is each independently hydrogen, protium, deuterium, tritium, halogen, $-NO_2$, -CN, -OH, -SH, $-NH_2$, -C(O)H, $-CO_2H$, - C(O)C(O)H, $-C(O)CH_2C(O)H$, $-C(O)CH_2OH$, -S(O)H, $-S(O)_2H$, $-C(O)NH_2$, $-SO_2NH_2$, -OC(O)H, $-N(H)SO_2H$, or $C_{1-6}$ aliphatic group;

m and n are integers greater than or equal to 0;

when $R_3$ is $-(C(R_{3a})(R_{3b}))_m-R_{3c}$, $-(C(R_{3a})(R_{3b}))_m-$ contains methylene units, the n methylene units of the $-(C(R_{3a})(R_{3b}))_m-$ are each independently replaced by - $N(R_5)C(O)-$, $-C(O)N(R_5)-$, -C(O)-, -OC(O)-, -C(O)O-, $-NR_5-$, -O-, -S-, -SO-, $-SO_2-$, - $P(R_5)-$, $-P(=O)(R_5)-$, $-N(R_5)SO_2-$, $-SO_2N(R_5)-$, -C(=S)-, $-C(=NR_5)-$, -N=N-, -CH= N- or -N=CH-; preferably, $R_3$ is $-(CH_2)_a(CH_2CH_2O)_b(CH_3)_c$, or $C_{1-8}$ aliphatic group optionally substituted with R, m = a + 3 * b + c, b is not 0, a and b are integers greater than 0; when $R_3$ is

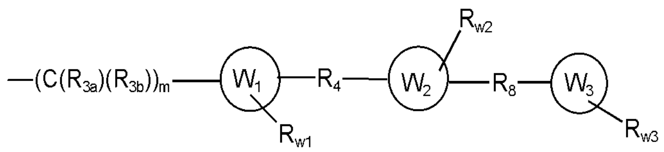

,

$-(C(R3_a)(R_{3b}))_m-$contains methylene units, the n methylene units of the $-(C(R_{3a})(R_{3b}))_m-$ are each independently replaced by $-N(R_5)C(O)-$, $-C(O)N(R_5)-$, -C(O)-, -OC(O)-, -C(O)O-, - $NR_5-$, -O-, or -S-, wherein ring $W_1$, ring $W_2$, and ring $W_3$ are each independently selected from 6- to 10-membered aryl, 5- to 8-membered heteroaryl, 4- to 8-membered cycloalkyl, and 4- to 8-membered heterocyclyl; preferably, ring $W_1$ is selected from phenyl or a 4- to 8-

membered heterocyclyl; ring $W_2$ is selected from phenyl or 4- to 8-membered heterocyclyl, ring $W_3$ is selected from phenyl or 5- to 8-membered heteroaryl; preferably, ring $W_1$ is phenyl or oxolanyl, ring $W_2$ is phenyl or oxolanyl, ring $W_3$ is phenyl or pyridinyl;

when ring $W_1$, ring $W_2$, and ring $W_3$ are selected from cycloalkyl or heterocyclyl, the cycloalkyl or heterocyclyl is substituted with one or more $R_{w1}$, $R_{w2}$, or $R_{w3}$, each occurrence of $R_{w1}$, $R_{w2}$, or $R_{w3}$ is each independently selected from hydrogen, protium, deuterium, tritium, halogen,=O, =S, $-NO_2$, -CN, -OR, -SR, $-N(R_a)(R_b)$, -C(O)R, -$CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, -N(R) $SO_2R$, or $C_{1-6}$ aliphatic group optionally substituted with R; when ring $W_1$, ring $W_2$, or ring $W_3$ is selected from 6- to 10-membered aryl or 5- to 8-membered heteroaryl, the aryl or heteroaryl is substituted with one or more $R_{w1}$, $R_{w2}$, or $R_{w3}$, each occurrence of $R_{w1}$, $R_{w2}$, or $R_{w3}$ is each independently selected from hydrogen, protium, deuterium, tritium, halogen, $-NO_2$, -CN, -OR, -SR, $-N(R_a)(R_b)$, - C(O)R, $-CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R; when $R_3$ is

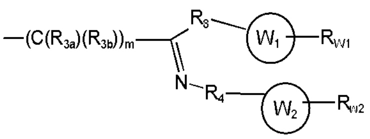

,

and $-(C(R_{3a})(R_{3b}))_m-$ contains methylene units, the n methylene units of the $(C(R_{3a})(R_{3b}))_m-$ are each independently replaced by $-N(R_5)C(O)-$, $-C(O)N(R_5)-$, -C(O)-, -OC(O)-, -C(O)O-, $-NR_5-$, -O-, or -S-; wherein ring $W_1$ is selected from 6- to 10-membered aryl, 4- to 8-membered cycloalkyl, 4- to 8-membered heterocyclyl, and 5- to 8-membered heteroaryl, when ring $W_1$ is selected from 4- to 8-membered cycloalkyl or 4- to 8-membered heterocyclyl, the cycloalkyl or heterocyclyl is substituted with one or more $R_{w1}$, each occurrence of $R_{w1}$ is each independently selected from hydrogen, protium, deuterium, tritium, halogen, - (= O), -( = S), $-NO_2$, -CN, -OR, -SR, $-N(R_a)(R_b)$, -C(O)R, $-CO_2R$, -C(O)C(O)R, - $C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, or $C_{1-6}$ aliphatic group optionally substituted with R; when ring $W_1$ is selected from 6- to 10-membered aryl or 5- to 8-membered heteroaryl, the aryl or heteroaryl is substituted with one or more $R_{w1}$, each occurrence of $R_{w1}$ is independently selected from hydrogen, protium, deuterium, tritium, halogen, $-NO_2$, -CN, -OR, -SR, - $N(R_a)(R_b)$, -C(O)R, $-CO_2R$, -C(O)C(O)R, $-C(O)CH_2C(O)R$, -S(O)R, $-S(O)_2R$, - $C(O)N(R_a)(R_b)$, $-SO_2N(R_a)(R_b)$, -OC(O)R, $-N(R)SO_2R$, or $C_{1-6}$ aliphatic group optionally substituted with R;

when $R_3$ is

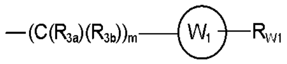

,

and $-(C(R_{3a})(R_{3b}))_m-$ contains methylene units, the n methylene units of the $-(C(R_{3a})(R_{3b}))_m-$ are each independently replaced by - $N(R_5)C(O)-$, $-C(O)N(R_5)-$, -C(O)-, -OC(O)-, -C(O)O-, $-NR_5-$, -O-, -S-, -SO-, $-SO_2-$, - $P(R_5)-$, $-P(=O)(R_5)-$, $-N(R_5)SO_2-$, $-SO_2N(R_5)-$, -C(=S)-, $-C(=NR_5)-$, -N=N-, -CH= N-, or -N=CH-;

preferably, $R_3$ is

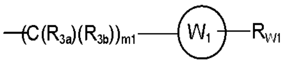

, or

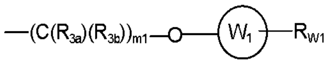

,

m1 is an integer from 1 to 6; ring $W_1$ is selected from 6- to 10-membered aryl, 4- to 8-membered cycloalkyl, 4- to 8-membered saturated or unsaturated heterocyclyl containing 1 to 3 heteroatoms optionally selected from N, O, and S, or 5- to 8-membered heteroaryl containing 1 to 3 heteroatoms optionally selected from N, O, and S;

when ring $W_1$ is selected from 4- to 8-membered cycloalkyl or 4- to 8-membered heterocyclyl, the cycloalkyl or heterocyclyl is substituted with one or more $R_{w1}$, each occurrence of $R_{w1}$ is independently selected from hydrogen, protium, deuterium, tritium, halogen, =O, =S, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, or $C_{1-6}$ aliphatic group optionally substituted with R;

when ring $W_1$ is selected from 6- to 10-membered aryl or 5- to 8-membered heteroaryl, the phenyl or 5- to 8-membered heteroaryl is substituted with one or more $R_{w1}$, each occurrence of $R_{w1}$ is independently selected from hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ aliphatic

group optionally substituted with R;

preferably, when ring $W_1$ is a 5- to 8-membered heteroaryl group substituted with one or more $R_{w1}$, the heteroaryl group is pyridinyl or pyrazolyl; when ring $W_1$ is selected from 4- to 8-membered heterocyclyl, containing 1, 2, or 3 heteroatoms, each occurrence of the heteroatoms is independently selected from N, O, or S; preferably, ring $W_1$ is

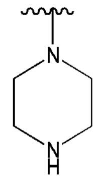 and 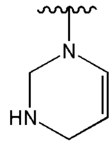 ;

ring $W_1$ is substituted with one or more $R_{w1}$, each occurrence of $R_{w1}$ is independently selected from hydrogen, protium, deuterium, tritium, halogen, =O, = S, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, or $C_{1-6}$ aliphatic group optionally substituted with R;

when $R_3$ is

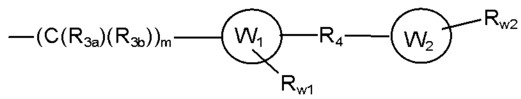

,

**and** $-(C(R_{3a})(R_{3b}))_m-$ contains methylene units, the n methylene units of the $-(C(R_{3a})(R_{3b}))_m-$ are each independently replaced by $-N(R_5)C(O)-$, $-C(O)N(R_5)-$, -C(O)-, -OC(O)-, -C(O)O-, $-NR_5-$, -O-, -S-, -SO-, $-SO_2-$, $-P(R_5)-$, $-P(=O)(R_5)-$, $-N(R_5)SO_2-$, $-SO_2N(R_5)-$, -C(=S)-, $-C(=NR_5)-$, -N=N-, -CH=N-, or -N=CH-; wherein ring $W_1$ and ring $W_2$ are each independently selected from substituted or unsubstituted 6- to 10-membered aryl, 5- to 8-membered heteroaryl, 4- to 8-membered cycloalkyl, or 4- to 8-membered heterocyclyl; preferably, ring $W_1$ is selected from phenyl or 4- to 8-membered heterocyclyl substituted with one or more $R_{w1}$, and ring $W_2$ is selected from 4- to 8-membered heterocyclyl substituted with one or more $R_{w2}$; preferably, ring $W_1$ is phenyl or oxolanyl substituted with one or more $R_{w1}$, ring $W_2$ is selected from

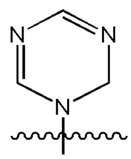

,

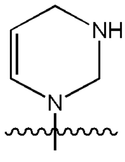 , and 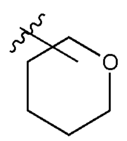 ,

and each substituted with one or more $R_{w2}$;

when ring $W_1$ or ring $W_2$ is selected from cycloalkyl or heterocyclyl, the cycloalkyl or heterocyclyl is substituted with one or more $R_{w1}$ or $R_{w2}$, each occurrence of $R_{w1}$ or $R_{w2}$ is each independently selected from hydrogen, protium, deuterium, tritium, halogen, = O, = S, $-NO_2$, -CN, -OR, -SR, $-N(R_a)(R_b)$, -C(O)R, $-CO_2R$, -C(O)C(O)R, - $C(O)CH_2C(O)R$, -S(O)R, $-S(O)_2R$, $-C(O)N(R_a)(R_b)$, $-SO_2N(R_a)(R_b)$, -OC(O)R, - $N(R)SO_2R$, or $C_{1-6}$ aliphatic group optionally substituted with R;

when ring $W_1$ or ring $W_2$ is selected from 6- to 10-membered aryl or 5- to 8-membered heteroaryl, the aryl or heteroaryl is substituted with one or more $R_{w1}$ or $R_{w2}$, each occurrence of $R_{w1}$ or $R_{w2}$ is each independently selected from hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ aliphatic group optionally substituted with R, $R_4$ is a single bond or selected from -O-, $C_{1-8}$ alkylene, $C_{1-8}$ haloalkylene, $-N(R_5)C(O)-$, - $C(O)N(R_5)-$, -C(O)-, -OC(O)-, -C(O)O-, $-NR_5-$, -S-, -SO-, $-SO_2-$, $-P(R_5)-$, -P(=O)$(R_5)-$, $-N(R_5)SO_2-$, $-SO_2N(R_5)-$, -C(=S)-, $-C(=NR_5)-$, -N=N-, -CH=N-, or -N=CH-;

when $R_3$ is

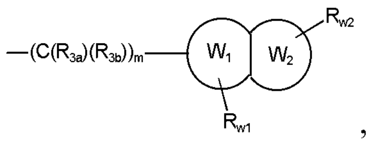

,

$-(C(R_{3a})(R_{3b}))_m-$ contains methylene units, the n methylene units of the $-(C(R_{3a})(R_{3b}))_m-$ are each independently replaced by $-N(R_5)C(O)-$, $-C(O)N(R_5)-$, $-C(O)-$, $-OC(O)-$, $-C(O)O-$, $-NR_5-$, -O-, -S-, -SO-, $-SO_2-$, - $P(R_5)-$, $-P(=O)(R_5)-$, $-N(R_5)SO_2-$, $-SO_2N(R_5)-$, -C(=S)-, $-C(=NR_5)-$, -N=N-, -CH= N-, or -N=CH-; ring $W_1$ and ring $W_2$ are each independently selected from 6- to 10-membered aryl, 5- to 8-membered heteroaryl, 4- to 8-membered cycloalkyl, or 4- to 8-membered heterocyclyl; preferably, ring $W_1$ and ring $W_2$ are fused to form a fused ring structure selected from any one of the following structures:

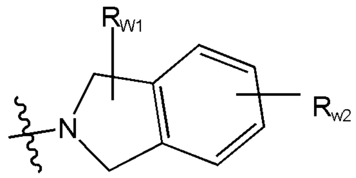

,

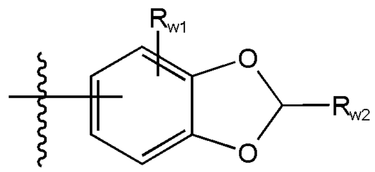

,

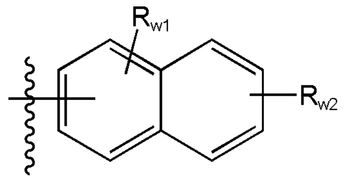

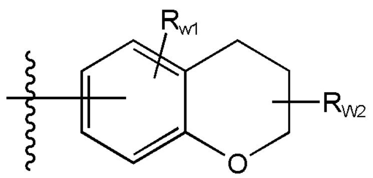

,

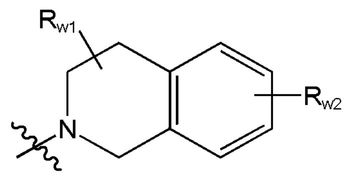

,

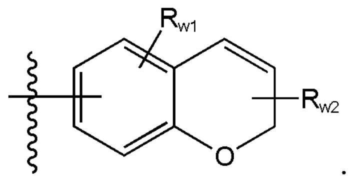

;

when ring $W_1$ or ring $W_2$ is a 4- to 8-membered cycloalkyl or 4- to 8-membered heterocyclyl, the cycloalkyl or heterocyclyl is substituted with one or more $R_{w1}$ or $R_{w2}$, each occurrence of $R_{w1}$ or $R_{w2}$ is independently selected from hydrogen, protium, deuterium, tritium, halogen, =O, =S, $-NO_2$, -CN, -OR, -SR, $-N(R_a)(R_b)$, -C(O)R, - $CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R;

when ring $W_1$ or ring $W_2$ is a 6- to 10-membered aryl or 5- to 8-membered heteroaryl, the aryl or heteroaryl is substituted with one or more $R_{w1}$ or $R_{w2}$, each occurrence of $R_{w1}$ or $R_{w2}$ is each independently selected from hydrogen, protium, deuterium, tritium, halogen, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, - OC(O)R, $-N(R)SO_2R$, or $C_{1-6}$ aliphatic group optionally substituted with R;

when $R_3$ is

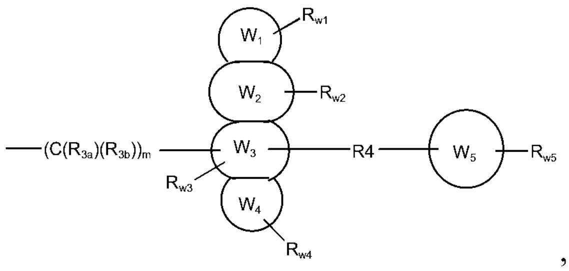

,

$-(C(R_{3a})(R_{3b}))_m$ contains methylene units, the n methylene units of the $-(C(R_{3a})(R_{3b}))_m$- are each independently replaced by $-N(R_5)C(O)-$, $-C(O)N(R_5)-$, -C(O)-, -OC(O)-, -C(O)O-, $-NR_5-$, -O-, -S-, - SO-, $-SO_2-$, $-P(R_5)-$, -P(=O)$(R_5)$-, $-N(R_5)SO_2-$, $-SO_2N(R_5)-$, -C(=S)-, $-C(=NR_5)-$, -N =N-, -CH=N-, or -N=CH-; ring $W_1$, $W_2$, $W_3$, and $W_4$ are fused together in sequence, ring $W_1$ and ring $W_4$ are each independently selected from 4- to 8-membered heterocyclyl, the heterocyclyl is substituted with one or more $R_{w1}$ or $R_{w4}$, each occurrence of $R_{w1}$ or $R_{w4}$ is independently selected from hydrogen, protium, deuterium, tritium, halogen, = O, = S, $-NO_2$, -CN, -OR, -SR, $-N(R_a)(R_b)$, -C(O)R, $-CO_2R$, - C(O)C(O)R, $-C(O)CH_2C(O)R$, -S(O)R, $-S(O)_2R$, $-C(O)N(R_a)(R_b)$, $-SO_2N(R_a)(R_b)$, -OC(O)R, $-N(R)SO_2R$, or $C_{1-6}$ aliphatic group optionally substituted with R; ring $W_5$ and ring $W_2$ is 6- to 10-membered aryl or 5- to 8-membered heteroaryl, the aryl or heteroaryl is substituted with one or more $R_{w2}$, each occurrence of $R_{w2}$ is independently selected from hydrogen, protium, deuterium, tritium, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, $-S(O)_2R$, $-C(O)N(R_a)(R_b)$, -$SO_2N(R_a)(R_b)$, -OC(O)R, $-N(R)SO_2R$, or $C_{1-6}$ aliphatic group optionally substituted with R; ring $W_3$ is 4- to 8-membered cycloalkyl, the cycloalkyl is substituted with one or more $R_{w3}$, each occurrence of $R_{w3}$ is independently selected from hydrogen, protium, deuterium, tritium, halogen, =O, =S, $-NO_2$, -CN, -OR, -SR, $-N(R_a)(R_b)$, -C(O)R, -$CO_2R$, -C(O)C(O)R, $-C(O)CH_2C(O)R$, -S(O)R, $-S(O)_2R$, $-C(O)N(R_a)(R_b)$, - $SO_2N(R_a)(R_b)$, -OC(O)R, -N(R)$SO_2R$, or $C_{1-6}$ aliphatic group optionally substituted with R; $R_4$ is a single bond or selected from -O-, $C_{1-8}$ alkylene, $C_{1-8}$ haloalkylene, - $N(R_5)C(O)-$, $-C(O)N(R_5)-$, -C(O)-, -OC(O)-, -C(O)O-, $-NR_5-$, -S-, -SO-, $-SO_2-$, $-P(R_5)-$, $-P(=O)(R_5)-$, $-N(R_5)SO_2-$, $-SO_2N(R_5)-$, -C(=S)-, $-C(=NR_5)-$, -N=N-, -CH=N-, or - N = CH-;

m is an integer from 1 to 20;

n is an integer from 0 to 8.

**6.** A compound represented by Formula 1C, or a pharmaceutically acceptable salt thereof,

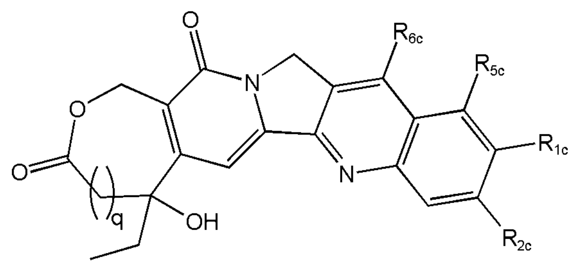

Formula 1C

wherein, q is 1, 2, or 3;

$R_{1c}$, $R_{2c}$, $R_{5c}$ are each independently selected from hydrogen, halogen, $-NO_2$, -CN, -OR, -SR, $-N(R_a)(R_b)$, -C(O)R, $-CO_2R$, -C(O)C(O)R, $-C(O)CH_2C(O)R$, -S(O)R, - $S(O)_2R$, $-C(O)N(R_a)(R_b)$, $-SO_2N(R_a)(R_b)$, -OC(O)R, -N(R)$SO_2R$, or $C_{1-6}$ aliphatic group optionally substituted with R;

or $R_{1c}$ and $R_{2c}$ together with the carbon atoms to which they are attached form a saturated or unsaturated 5- to 10-membered cycloalkyl or 6- to 10-membered heterocyclyl containing 1 to 3 heteroatoms each independently selected from N, O, and S, wherein the cycloalkyl or heterocyclyl is substituted with one or more substituents independently selected from halogen, $-NO_2$, -CN, -OR, -SR, $-N(R_a)(R_b)$, -C(O)R, - $CO_2R$, -C(O)C(O)R, -C(O)$CH_2C(O)R$, -S(O)R, $-S(O)_2R$, $-C(O)N(R_a)(R_b)$, - $SO_2N(R_a)(R_b)$, -OC(O)R, $-N(R)SO_2R$, or $C_{1-6}$ aliphatic group optionally substituted with R;

$R_{6c}$ is $C_{1-8}$ alkyl-hydroxyl, $C_{1-8}$ alkylamino, $-C(R_a)(R_8)-N(R_a)-R_{4c}$, $-C(R_a)(R_b)-N(R_a)-C(O)-R_{4c}$, or $-C(R_a)(R_b)-N(R_a)-C(O)O-R_{4c}$;

Each occurence of R, $R_a$, $R_b$, and $R_{4c}$ are independently selected from hydrogen, protium, deuterium, tritium,

halogen, $-NO_2$, -CN, -OH, -SH, $-NH_2$, -C(O)H, $-CO_2H$, - C(O)C(O)H, $-C(O)CH_2C(O)H$, -S(O)H, $-S(O)_2H$, -C(O) $NH_2$, $-SO_2NH_2$, -OC(O)H, - $N(H)SO_2H$, $C_{1-6}$ aliphatic group, 3- to 8-membered cycloalkyl, 3- to 8-membered heterocyclyl, 6- to 10-membered aryl, or 5- to 10-membered heteroaryl, the $C_{1-6}$ aliphatic group, 3- to 8-membered cycloalkyl, 3- to 8-membered heterocyclyl, 6- to 10-membered aryl, or 5- to 10-membered heteroaryl is unsubstituted or substituted with one or more substituents independently selected from halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, -OC(O) H, $-N(H)SO_2H$, $C_{1-6}$ aliphatic group, $C_{1-6}$ alkylene-hydroxyl.

**7.** The compound or a pharmaceutically acceptable salt thereof according to any one of claims 1-6, wherein the compound is selected from:

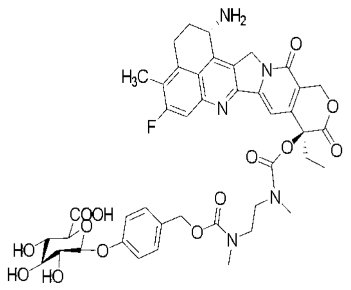
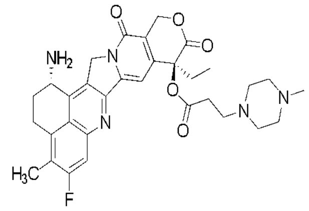

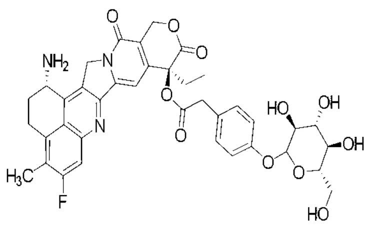
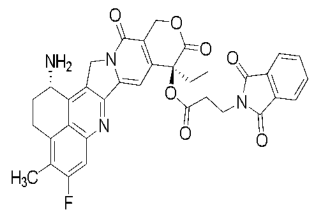

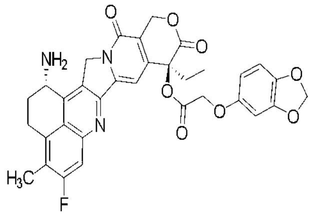
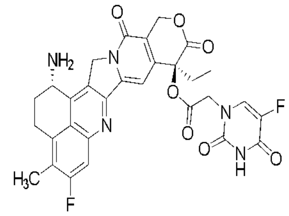

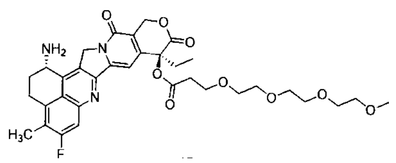
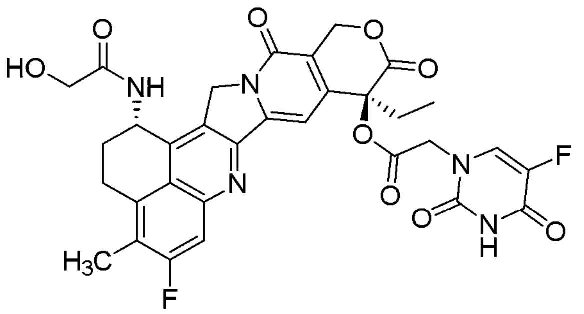
,

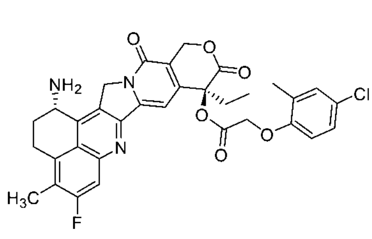

,

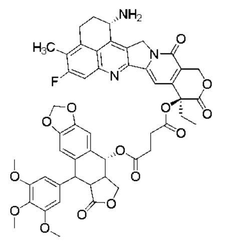

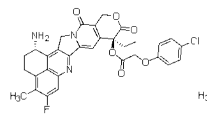

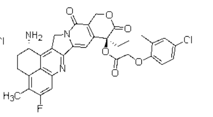

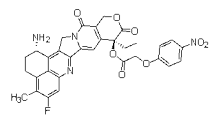

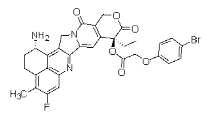

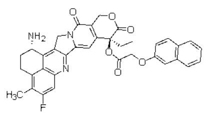

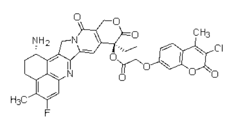

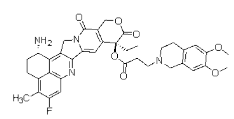

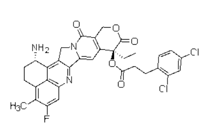

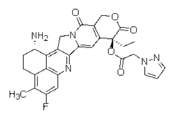

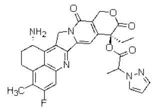

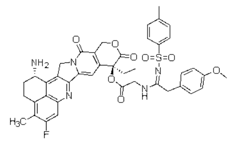

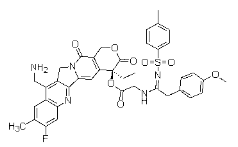

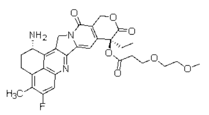

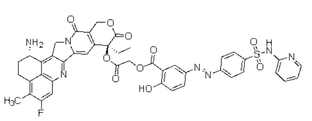

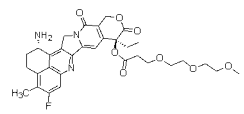

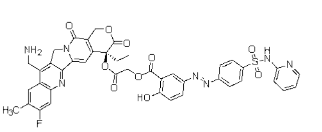

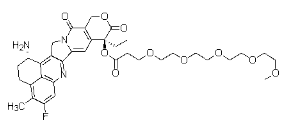

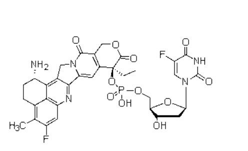

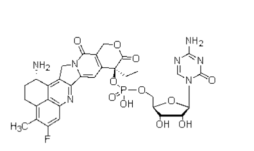

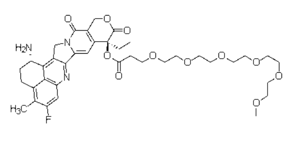

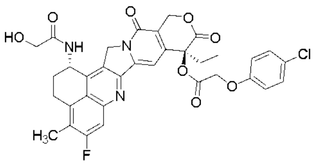

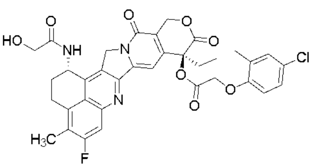

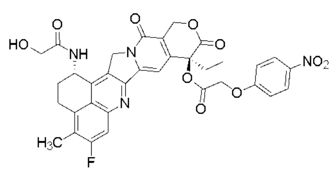

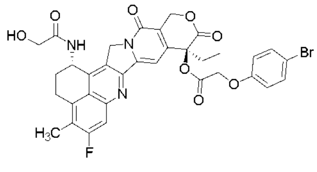

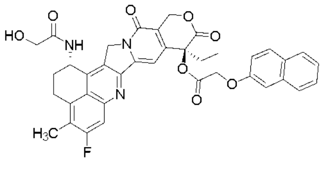

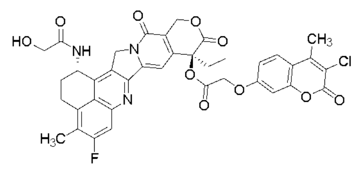

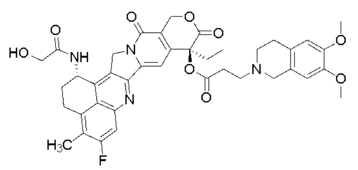

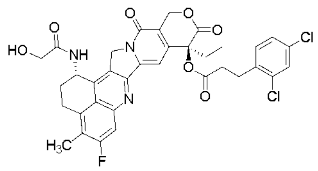

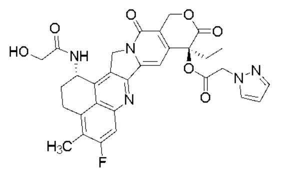

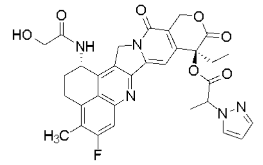

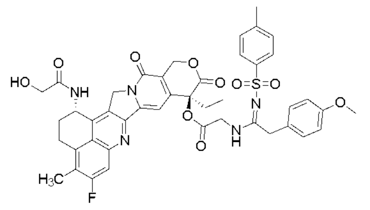

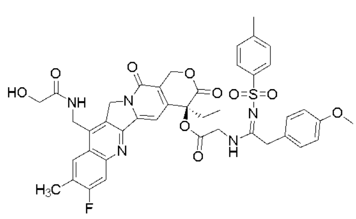

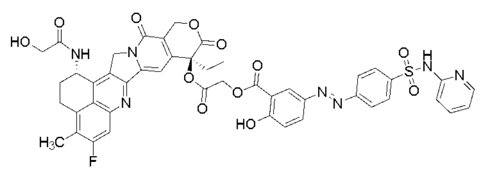

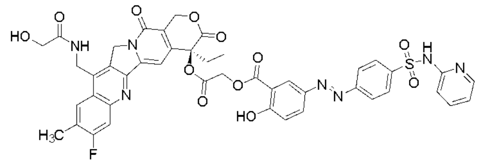

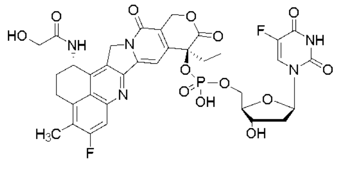

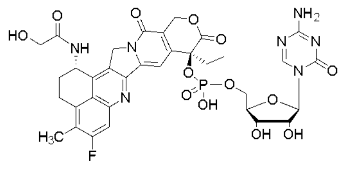

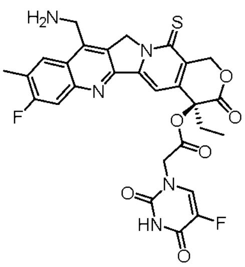,
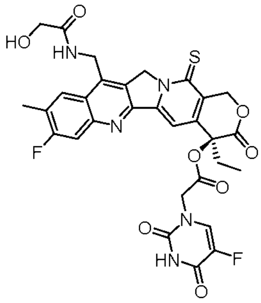。

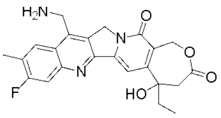,
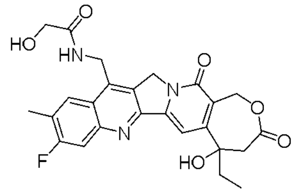,
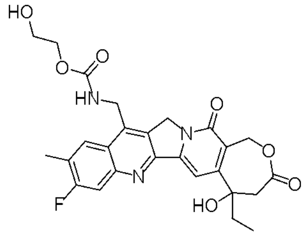,

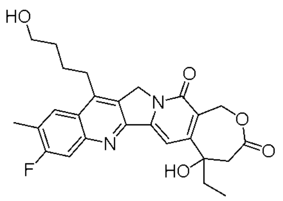,
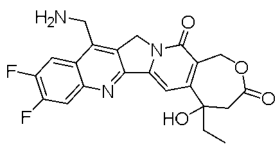,
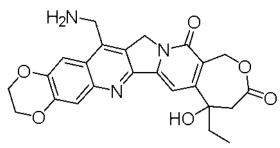,

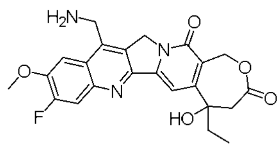,
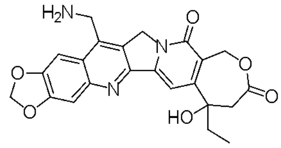,

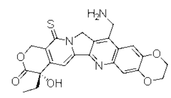
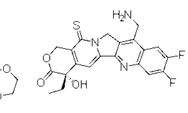
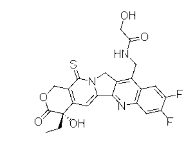
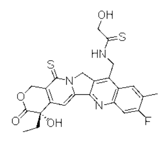

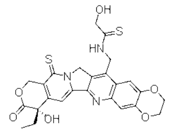
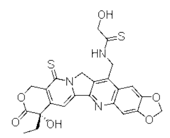
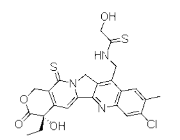
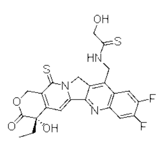

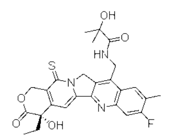
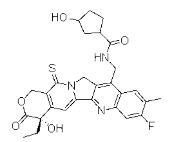
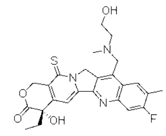

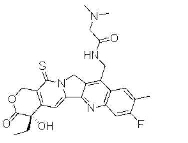
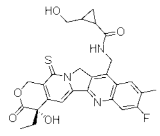
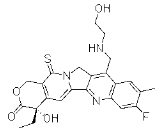

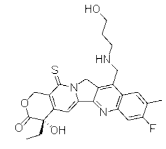 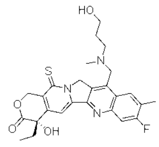

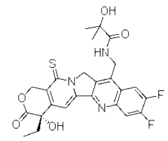 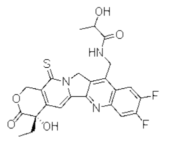 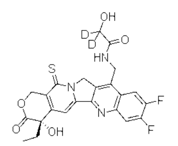 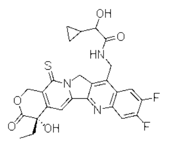

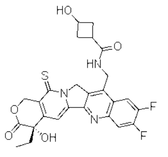 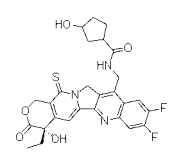 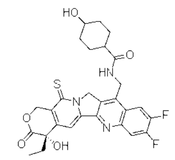 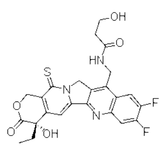

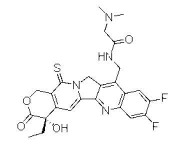 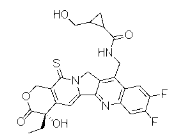 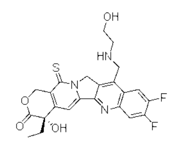 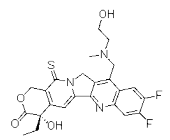

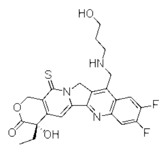 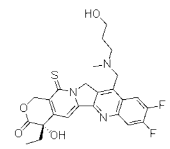 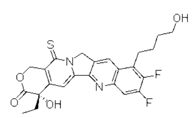 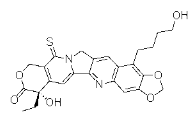

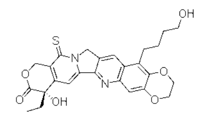 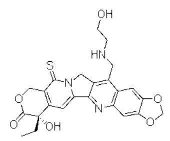 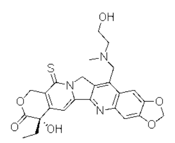 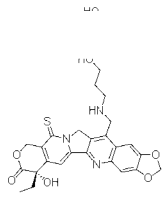

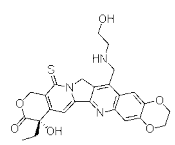 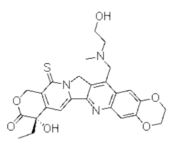 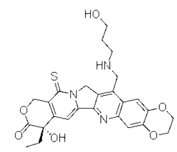

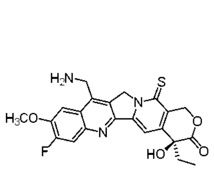 , 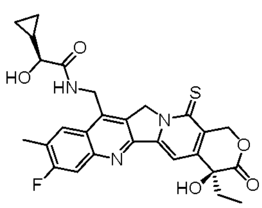 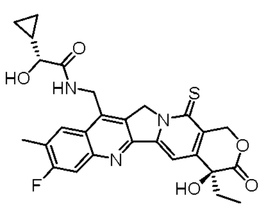

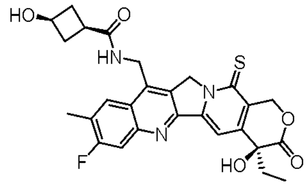 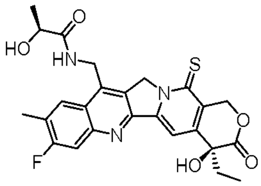 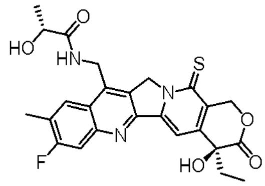

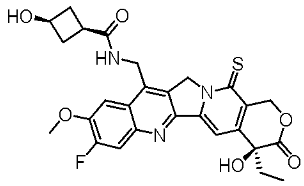
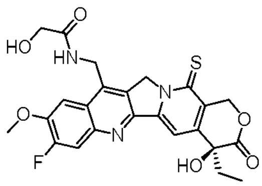
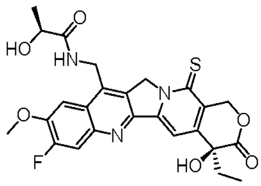
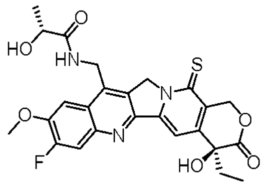

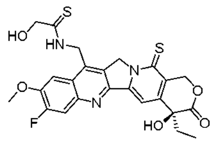
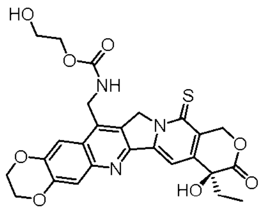
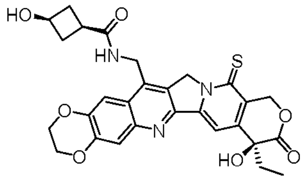
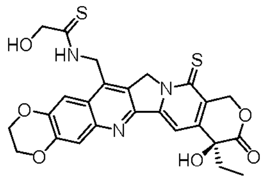

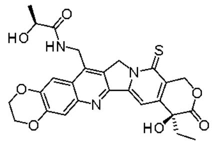
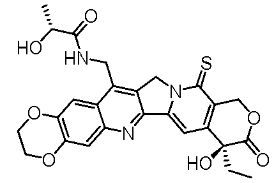
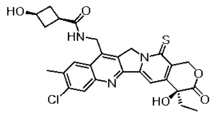
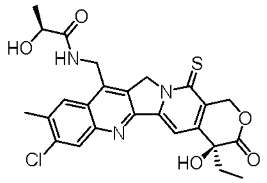

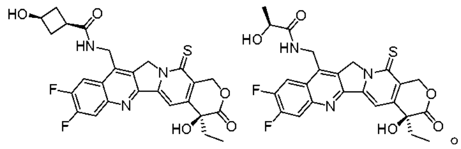
。

**8.** A ligand-drug conjugate represented by Formula 3, or a pharmaceutically acceptable salt thereof,

$$Ab\left(L-D\right)_n$$

Formula 3 ;

wherein, Ab is a ligand; L is a linker moiety; D is a drug moiety;
n is any integer or decimal from 1 to 15; preferably, n is any integer or decimal from 1 to 13;preferably, n is any integer or decimal from 3 to 10;
wherein the drug moiety D is selected from the following structures:

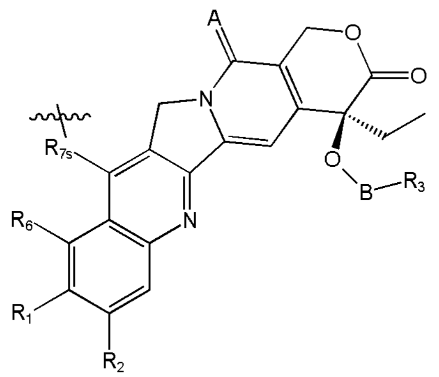
Formula 4-1 ,

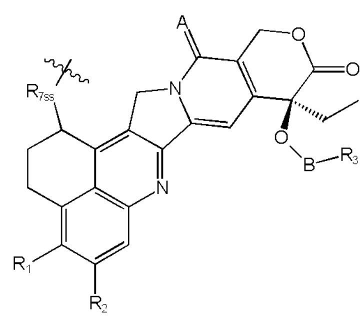
Formula 4-5 ,

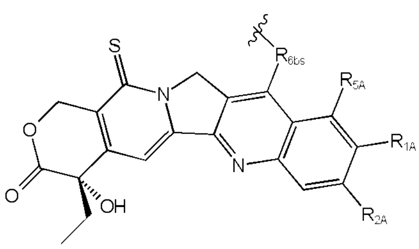 Formula 5-1 , 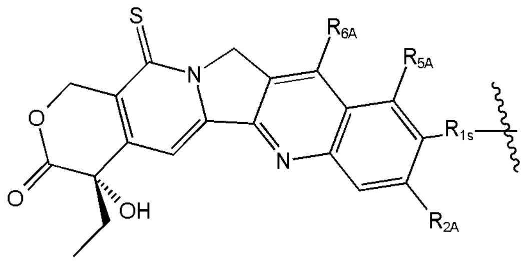 Formula 5-3 ,

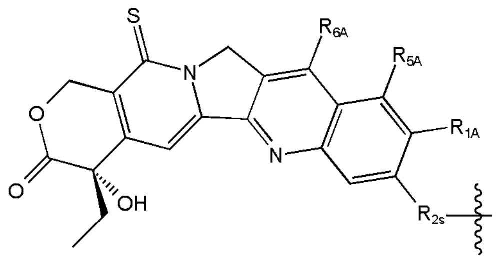 Formula 5-4 , and 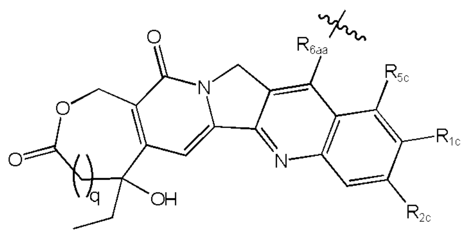 Formula 7-1 ;

wherein,
$R_1$, $R_2$, $R_3$, $R_6$, $R_7$, A, B, $R_{1c}$, $R_{2c}$, $R_{5c}$, $R_{1A}$, $R_{2A}$, $R_{3A}$, $R_{4A}$, and q as defined in any one of claims 2, 3, 4, 5, 6, or 7;
$R_{6A}$ is selected from hydrogen, $-CH(CH_3)N(R_{3A})(R_{4A})$, and $-CH_2N(R_{3A})(R_{4A})$;
$R_{5A}$ is hydrogen or $C_{1-6}$ alkyl;
$R_{1s}$, $R_{2s}$, $R_{7s}$, and $R_{7ss}$ are each independently $C_{1-8}$ alkylene-NR-, $C_{1-8}$ haloalkylene NR-, -OR, -SR, -NR-, -C(O)NR-, $-SO_2NR$-, or $-O-CH_2C(O)NR-$; preferably, $R_{1s}$, $R_{2s}$, $R_{7s}$, and $R_{7ss}$ are each independently -NR-, $-NR-CH_2-$, or $-O-CH_2C(O)NR-$; preferably, $R_{1s}$, $R_{2s}$, $R_{7s}$, and $R_{7ss}$ are each independently -NH-, $-NH-CH_2-$, or $-O-CH_2C(O)NH-$;
Each occurrence of $R_{6bs}$, is independently selected from $-CH_2N(Ra_1)-$, $-CH_2N(R_{a1})-C_{2-5}$ alkylene-O-, $-CH_2N(R_{a1})C(O)C(R_{a1})(R_{b1})O-$, $-CH_2N(R_{a1})C(S)C(R_{a1})(R_{b1})O-$, - $CH_2N(R_{a1})C(O)-C_{3-8}$ cycloalkylene-O-, $-CH_2N(R_{a1})C(O)-C_{2-5}$alkylene-O-, - $CH_2N(R_{a1})C(O)C(R_{a1})(R_{b1})N(R_{a1})-$, $-CH_2N(R_{a1})C(O)-C_{3-8}$ cycloalkylene $-C(R_{a1})(R_{b1})-O-$, $-C_{2-5}$ alkylene-O-, $-CH_2N(R_{a1})C(O)O-C_{2-5}$ alkylene-O-;
Wherein each occurrence of R, $R_{a1}$ and $R_{b1}$ is independently selected from hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, - OC(O)H, $-N(H)SO_2H$, $C_{1-6}$ alkyl, $C_{2-6}$ alkenyl, $C_{2-6}$ alkynyl, 3-, 4-, 5-, 6-, 7-, or 8-membered cycloalkyl, 3- to 8-membered heterocyclyl containing 1, 2, or 3 heteroatoms independently selected from N, O, and S, 6- to 10-membered aryl, and 5- to 10-membered heteroaryl containing 1, 2, or 3 heteroatoms independently selected from N, O, and S; wherein the $C_{1-6}$ alkyl, $C_{2-6}$ alkenyl, $C_{2-6}$ alkynyl, 3-, 4-, 5-, 6-, 7-, or 8-membered cycloalkyl, 3- to 8-membered heterocyclyl, 6- to 10-membered aryl, or 5- to 10-membered heteroaryl is unsubstituted or substituted with one or more substituents independently selected from halogen, $-NO_2$, -CN, -OH, $-NH_2$, -C(O)H, $-CO_2H$, - C(O)C(O)H, $-C(O)CH_2C(O)H$, -S(O)H, $-S(O)_2H$, $-C(O)NH_2$, $-SO_2NH_2$, -OC(O)H, - $N(H)SO_2H$, $C_{1-6}$ alkyl, $C_{2-6}$ alkenyl, or $C_{1-6}$ alkynyl, and hydroxyl-$C_{1-6}$ alkylene; and
$R_{6aa}$ is selected from $-C_{1-8}$ alkylene- $N(R_{a2})-$, $-CH_2N(R_{a2})C(O)C(R_{a2})(R_{b2})O-$, - $CH_2N(R_{a2})C(O)-C_{3-8}$ cycloalkylene-O-, $-C_{2-5}$ alkylene-O-, and $-CH_2N(R_{a2})C(O)O-C_{2-5}$ alkylene-O-; preferably, $R_{6aa}$ is $-CH_2-NH_2$, or $-(CH_2)_4-O-$;
wherein, each occurrence of $R_{a2}$ and $R_{b2}$ is each independently selected from hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, - OC(O)H, $-N(H)SO_2H$, or $C_{1-6}$ alkylene-hydroxyl; preferably, each occurrence of $R_{a2}$ and $R_{b2}$ is each independently selected from hydrogen, F, Cl, Br, I, $-CH_2OH$, or - $C_2H_4OH$.

**9.** The ligand-drug conjugate or a pharmaceutically acceptable salt thereof according to claim 8, wherein the drug moiety D is selected from the following structures:

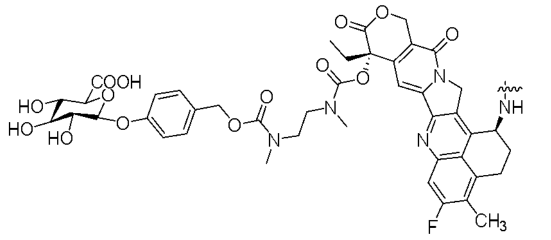

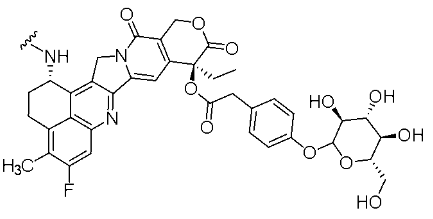

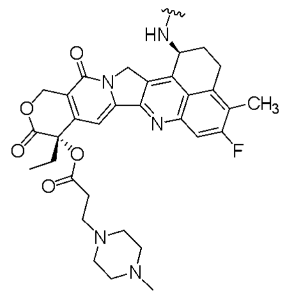

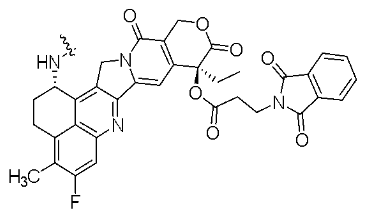

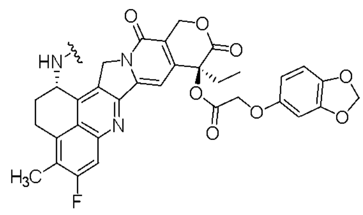

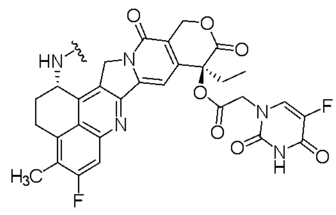

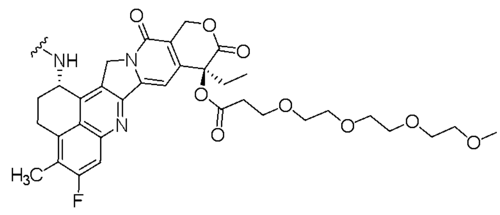

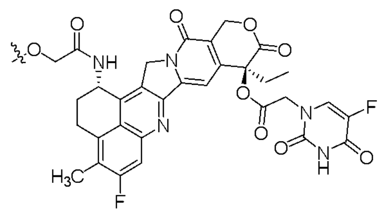

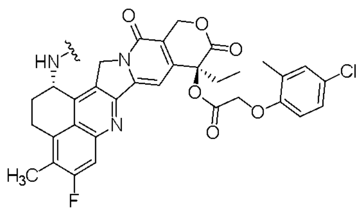

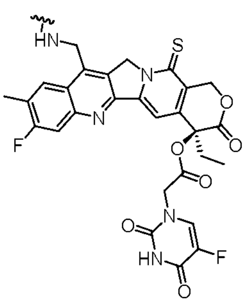

,

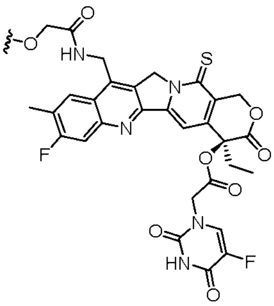

,

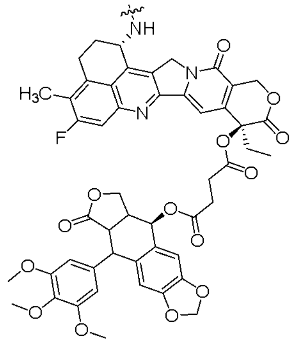

,

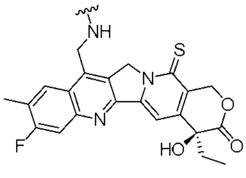

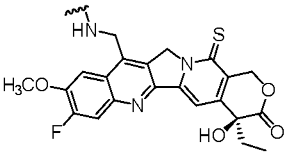

,

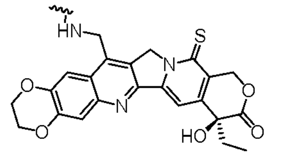

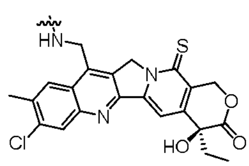

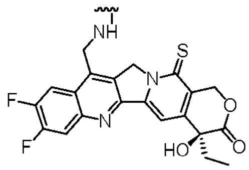

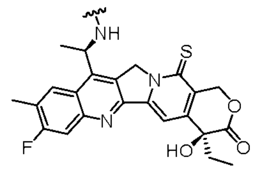

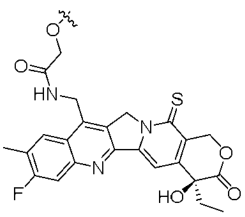

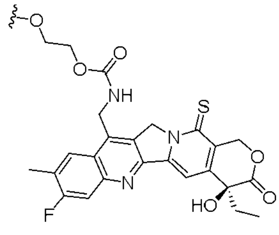

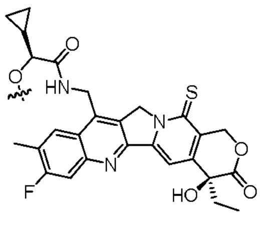

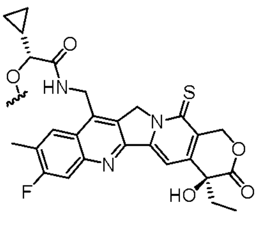

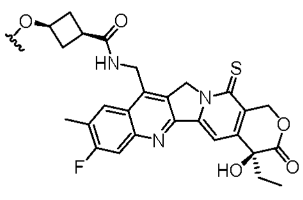

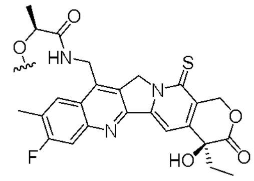

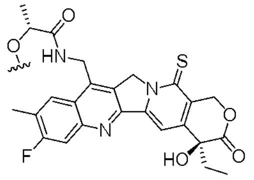

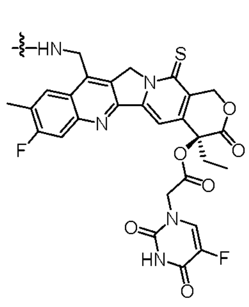

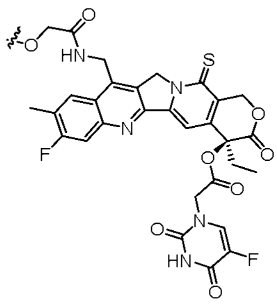

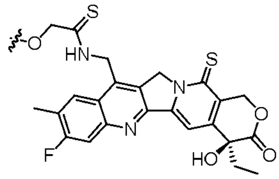

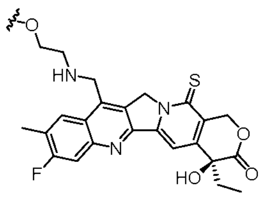

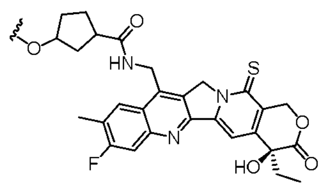

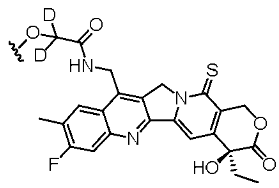
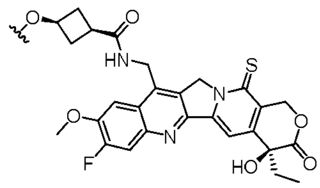
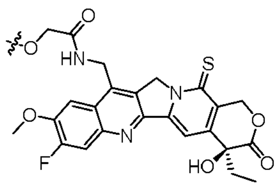

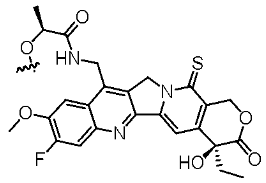
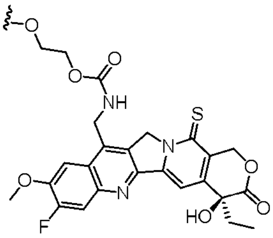
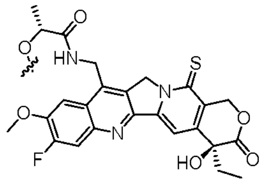
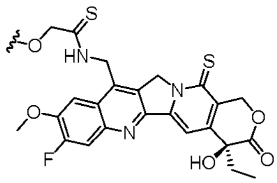

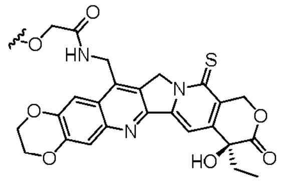
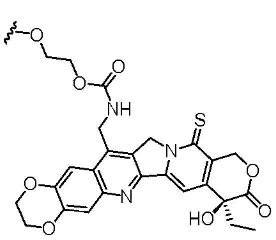
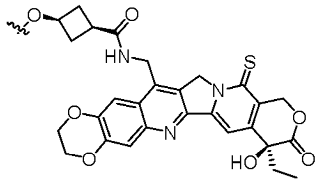

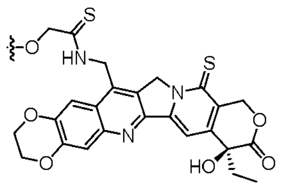
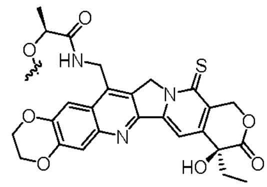
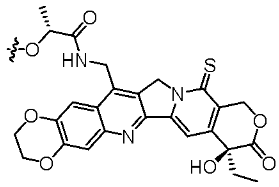

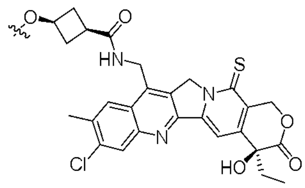
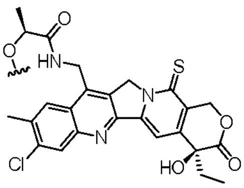
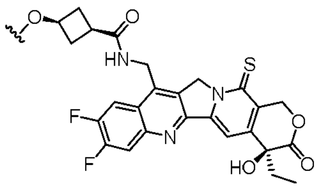
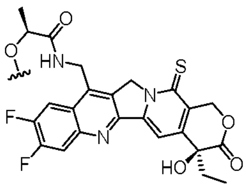

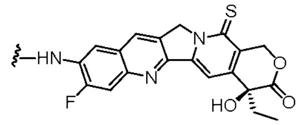

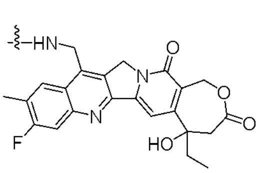
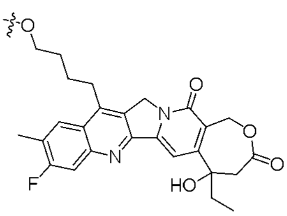
, and
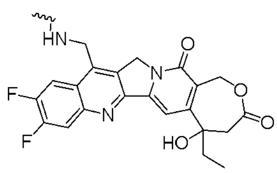
,

wherein

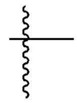

represents the attachment point.

**10.** The ligand-drug conjugate or a pharmaceutically acceptable salt thereof according to claim 8 or 9,

wherein the linker L is $L^1$-$L^2$-$L^3$-$L^4$;
$L^1$ is selected from -(succinimidyl-3-yl-N)-, -(succinimidyl-3-yl-N)-W-C(=O)-,

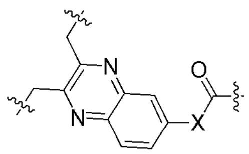,
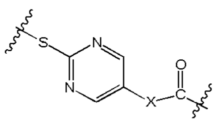,
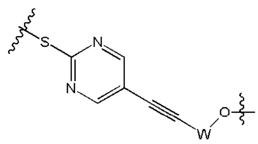,
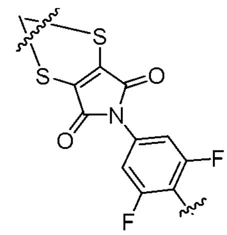,

and

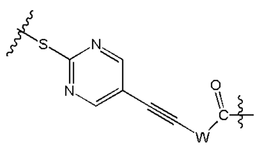;

wherein, W is selected from $C_{1-10}$ alkylene, $C_{1-10}$ alkylene-cycloalkylene, $C_{1-10}$ heteroalkylene, $C_{1-10}$ alkylene-heterocyclylene, or $C_{1-10}$ heteroalkylene-cycloalkylene; preferably, W is $C_{1-8}$ alkylene, $C_{1-8}$ alkylene-cycloalkylene, or $C_{1-8}$ heteroalkylene, wherein the heteroalkylene contains 1 to 3 heteroatoms independently selected from N, O, and S, wherein the alkylene, cycloalkylene, and heteroalkylene are unsubstituted or each independently optionally further substituted with one or more substituents selected from halogen, hydroxyl, -CN, amino, alkyl, haloalkyl, deuterated alkyl, alkoxy, and cycloalkyl; preferably, the alkylene, cycloalkylene, and heteroalkylene are unsubstituted or each independently optionally further substituted with one or more substituents selected from halogen, hydroxyl, -CN, amino, $C_{1-6}$ alkyl, $C_{1-6}$ haloalkyl, deuterated $C_{1-6}$ alkyl, $C_{1-6}$ alkoxy, and $C_{5-8}$ cycloalkyl; more preferably, the alkylene, cycloalkylene, and heteroalkylene are unsubstituted or optionally substituted with one or more substituents selected from halogen, -OH, -CN, $C_{1-3}$ alkyl, and -$OC_{1-3}$ alkyl; preferably, the alkylene, cycloalkylene, and heteroalkylene are unsubstituted or optionally substituted with one or more substituents selected from Cl, Br, F, -OH, -CN, methyl, and -$OCH_3$;
X is selected from a single bond, $C_{1-10}$ alkylene, $C_{1-10}$ alkylene-cycloalkylene, $C_{1-10}$ heteroalkylene, $C_{1-10}$ alkylene-heterocyclylene, or $C_{1-10}$ heteroalkylene-cycloalkylene; preferably, X is selected from a single bond, $C_{1-8}$ alkylene, $C_{1-8}$ alkylene-cycloalkylene, and $C_{1-8}$ heteroalkylene;
$L^2$ is selected from -$(CH_2CH_2O)_rCH_2CH_2C$(=O)-, -$(CH_2CH_2O)_rC$(=O)-, -$NR_{1L}(CH_2CH_2O)_rC$(=O)-, -$(CH_2CH_2O)_rCH_2C$(=O)-, -$NR_{1L}(CH_2CH_2O)_rCH_2CH_2C$(=O)-, -$NR_{1L}(CH_2CH_2O)_rCH_2C$(=O)-, -$NR_{1L}CH_2$-$Ar^1$-$(CH_2CH_2O)_rCH_2CH_2NR_{1L}C$(=O)$CH_2OCH_2C$(=O)-, -$NR_{1L}(CH_2CH_2O)_rCH_2$-$Ar^1$-$(CH_2CH_2O)_r CH_2CH_2C$(=O)-, -$S(CH_2)_rC$(=O)-, -O-$(CH_2CH_2O)_rCH_2$-$Ar^1$-$(CH_2CH_2O)_r$ $CH_2CH_2C$(=O)-,

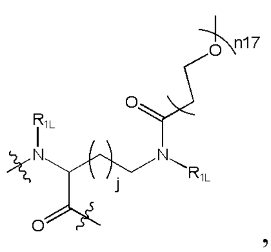,

or a single bond, wherein r is an integer selected from 1, 2, 3, 4, 5, 6, 7, 8, 9, 10, 11, 12, 13, 14, 15, 16, 17, 18, 19, or

20; each occurrence of n17 is each independently 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30; each occurrence of j is each independently selected from 0, 1, 2, 3, 4, 5, 6; preferably, r is an integer selected from 1, 2, 3, 4, 5, 6, 7, or 8;

$Ar^1$ is selected from 6- to 10-membered aryl, 5- to 10-membered heteroaryl containing 1 to 3 heteroatoms independently selected from N, O, P, and S, 3- to 10-membered cycloalkyl, and 3- to 10-membered heterocyclyl containing 1 to 3 heteroatoms independently selected from N, O, P, and S; preferably, $Ar^1$ is selected from

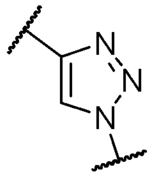, 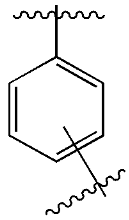, 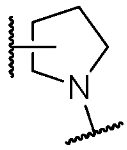, 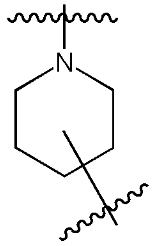, 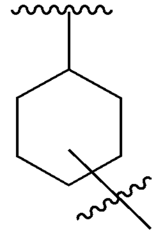,

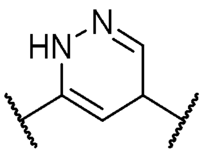, 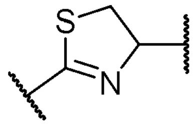, 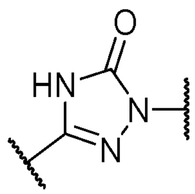, 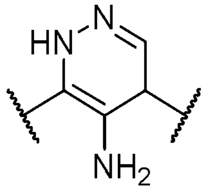,

and

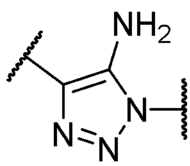;

$L^3$ is a peptide residue consisting of 2 to 7 amino acids, preferably $L^3$ is a peptide residue consisting of 2, 3, 4, 5, or 6 amino acids, wherein the amino acids are unsubstituted or optionally further substituted with one or more substituents selected from halogen, hydroxyl, -CN, amino, alkyl, haloalkyl, deuterated alkyl, alkoxy, and cycloalkyl; preferably, optionally further substituted with one or more substituents selected from halogen, hydroxyl, -CN, amino, $C_{1-6}$ alkyl, $C_{1-6}$ haloalkyl, deuterated $C_{1-6}$ alkyl, $C_{1-6}$ alkoxy, and $C_{5-8}$ cycloalkyl.

$L^4$ is selected from $-NR_{2L}(CR_{3L}R_{4L})_t-Z-(CR_{3L}R_{4L})_t-C(=O)-$, $-NR_{2L}(CR_{3L}R_{4L})_t-$, $-NR_{2L}(CR_{3L}R_{4L})_t-Z-(CR_{3L}R_{4L})_t-Z-C(=O)-$,

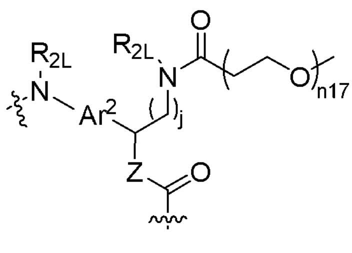,

$-NR_2-Ar^2-(CR_{3L}R_{4L})_t-Z-C(=O)-$, or a single bond, wherein each occurrence of t is each independently an integer of 0, 1, 2, 3, 4, 5, or 6; each occurrence of Z is each independently a single bond, O, S, or -NH-; $Ar^2$ is arylene or heteroarylene, preferably selected from 6-membered arylene or 5- to 8-membered heteroarylene, wherein the heteroarylene containing 1, 2, or 3 heteroatoms independently selected from N, O, and S; the arylene or heteroarylene is unsubstituted or optionally substituted with one or more substituents selected from H, halogen, -OH, -CN, $C_{1-6}$ alkyl, $-OC_{1-6}$ alkyl, $-NH_2$, $-NH(C_{1-6}$ alkyl), and $-N(C_{1-6}$ alkyl$)_2$;

$R_{1L}$ and $R_{2L}$ are the same or different and are each independently at each occurrence selected from hydrogen, halogen, $C_{1-6}$ alkyl, $C_{1-6}$ haloalkyl, deuterated $C_{1-6}$ alkyl, and $C_{1-6}$ alkylene-OH;

$R_{3L}$ and $R_{4L}$ are the same or different and are each independently at each occurrence selected from hydrogen, halogen, $C_{1-6}$ alkyl, $C_{1-6}$ haloalkyl, deuterated $C_{1-6}$ alkyl, and $C_{1-6}$ alkylene-OH; and
the $L^1$ end is attached to the ligand, and the $L^4$ end is attached to the drug moiety.

**11.** The ligand-drug conjugate or a pharmaceutically acceptable salt thereof according to any one of claims 8-10, wherein,

$L^3$ is a peptide residue consisting of 2 to 6 amino acids selected from glycine, phenylalanine, alanine, valine, lysine, citrulline, serine, glutamic acid, and aspartic acid; preferably a dipeptide residue, tripeptide residue, or tetrapeptide residue selected from alanine, phenylalanine, glycine, lysine, and citrulline; more preferably, $L^3$ is a peptide residue selected from: glycine-phenylalanine-glycine, alanine-alanine-alanine-glycine, alanine-alanine-alanine, glycine-glycine-phenylalanine-glycine, valine-citrulline, and valine-alanine;
wherein the peptide residue is unsubstituted or optionally further substituted with one or more substituents selected from halogen, hydroxyl, -CN, amino, alkyl, haloalkyl, deuterated alkyl, alkoxy, and cycloalkyl; preferably, optionally further substituted with one or more substituents selected from halogen, hydroxyl, -CN, amino, and $C_{1-6}$ alkyl.

**12.** The ligand-drug conjugate or a pharmaceutically acceptable salt thereof according to any one of claims 8-11, wherein the linker L is selected from:

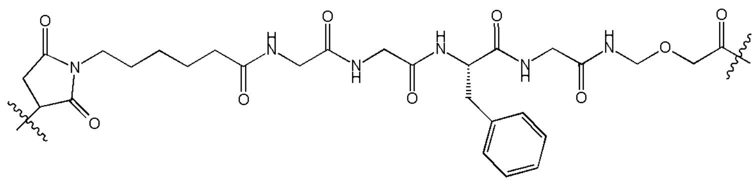

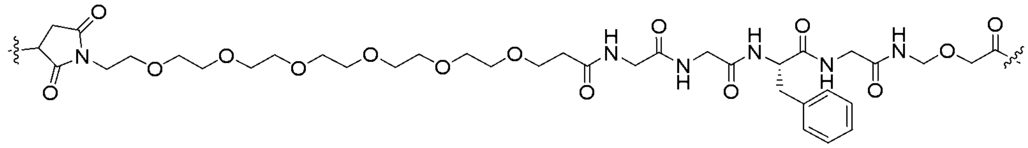

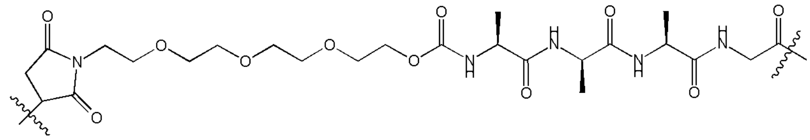

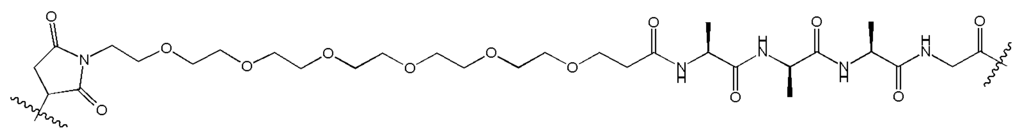

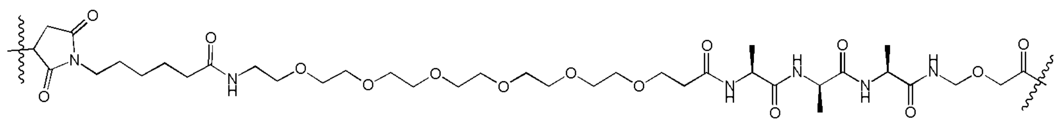

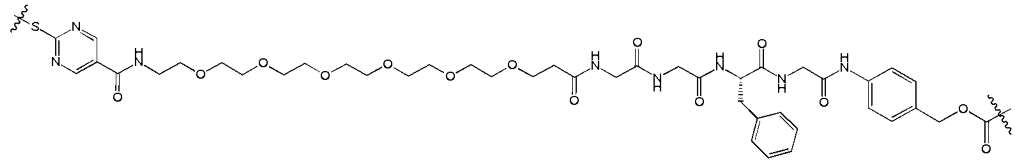

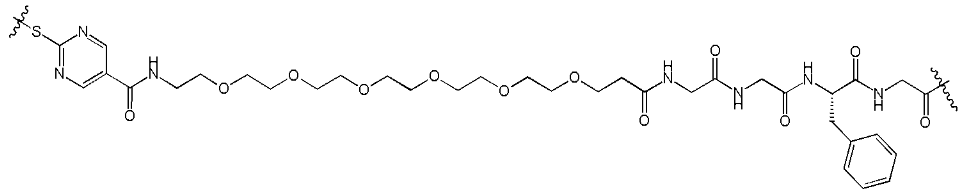

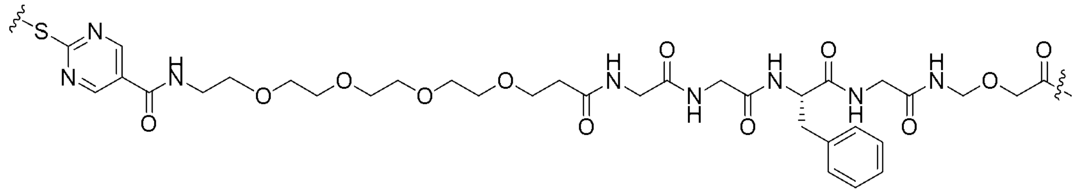

,

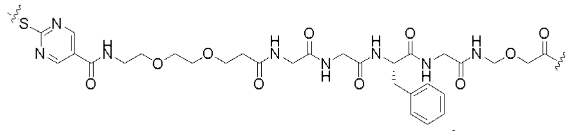

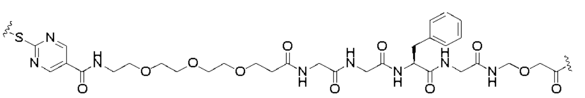

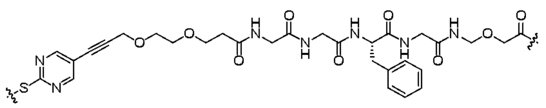

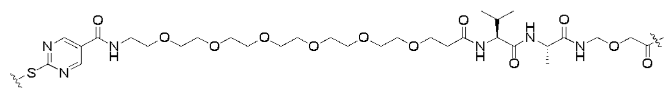

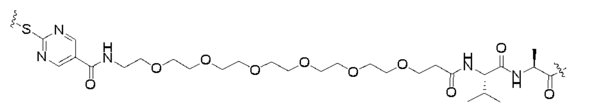

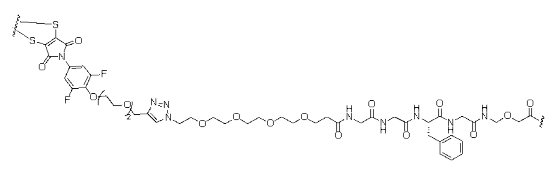

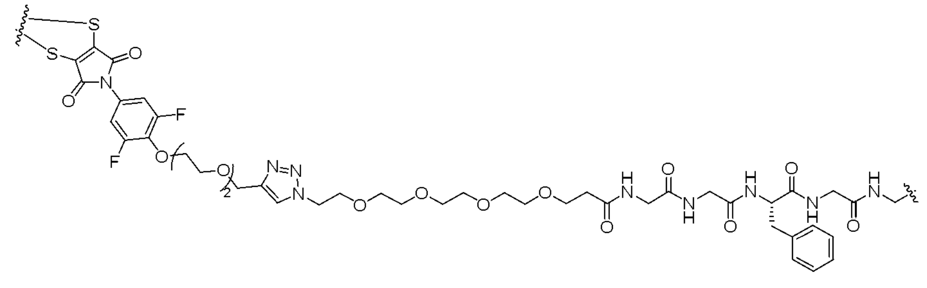

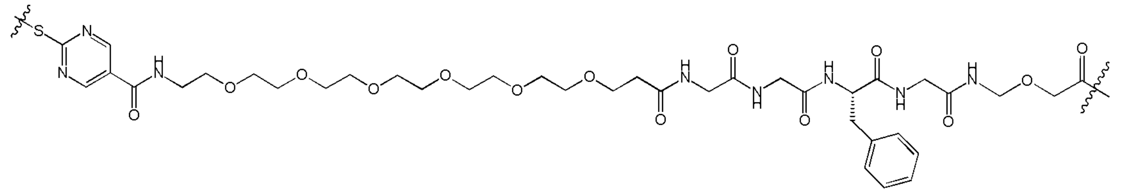

,

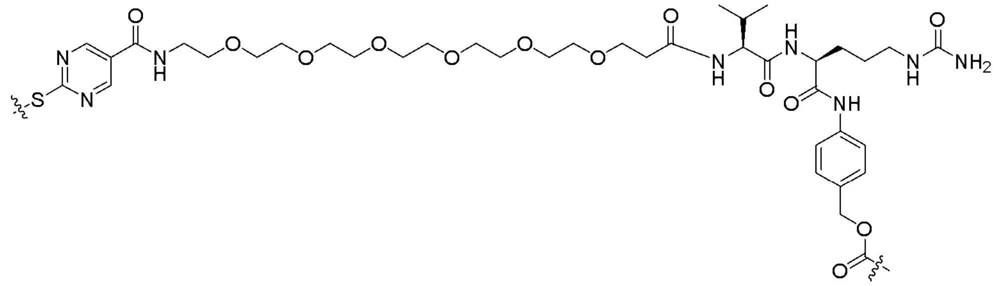

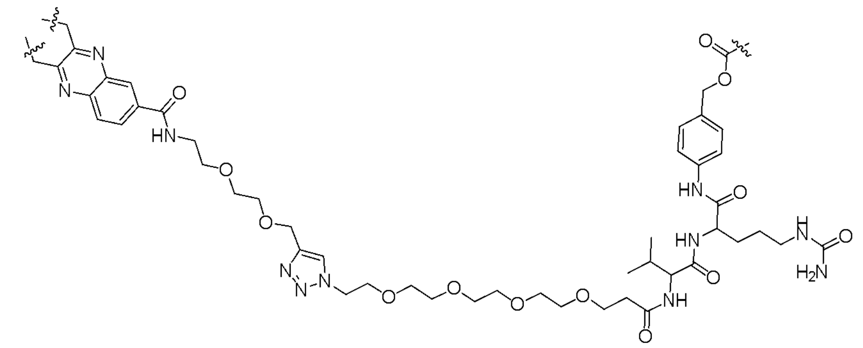

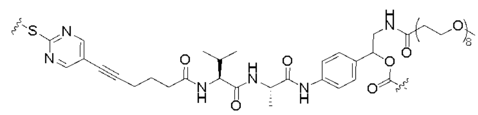

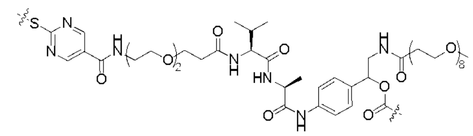

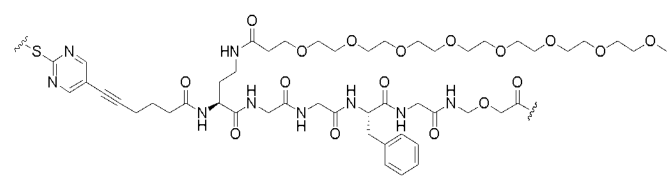

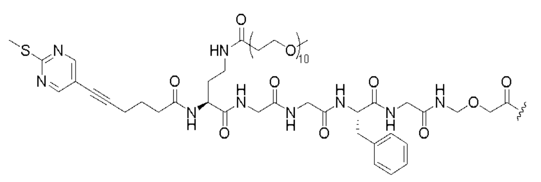

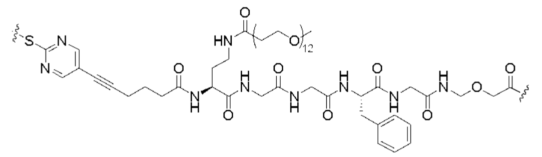

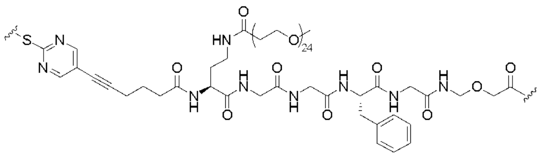

and

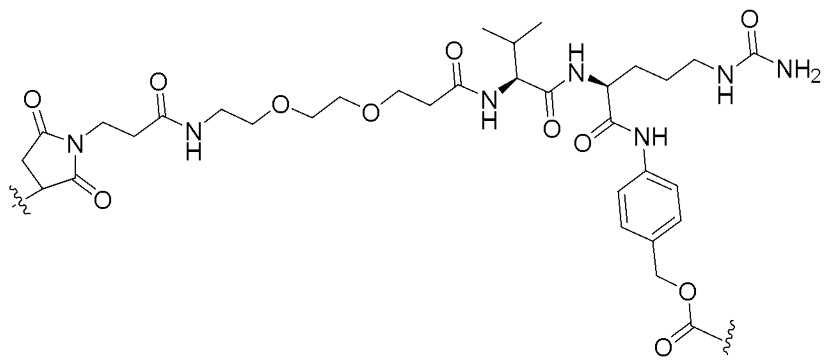

,

wherein

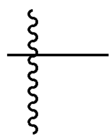

represents the attachment point.

**13.** The ligand-drug conjugate or a pharmaceutically acceptable salt thereof according to any one of claims 8-12, wherein the ligand-drug conjugate is selected from the following structures:

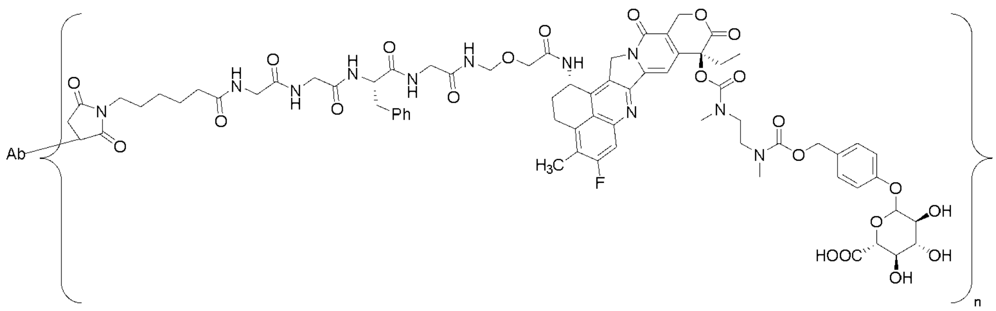

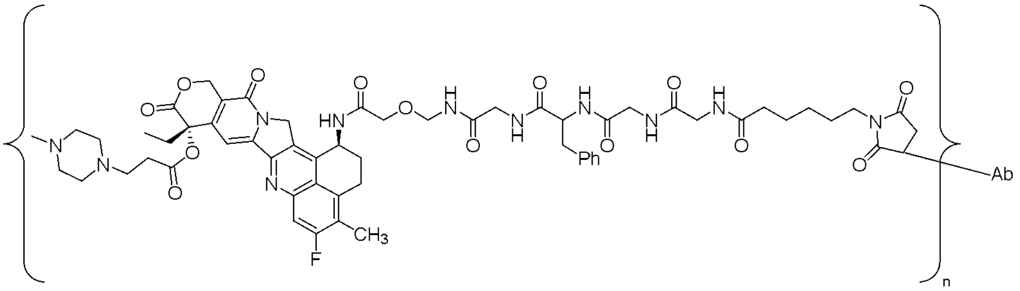

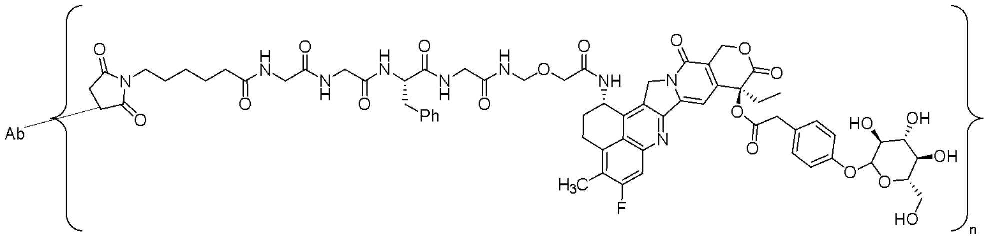

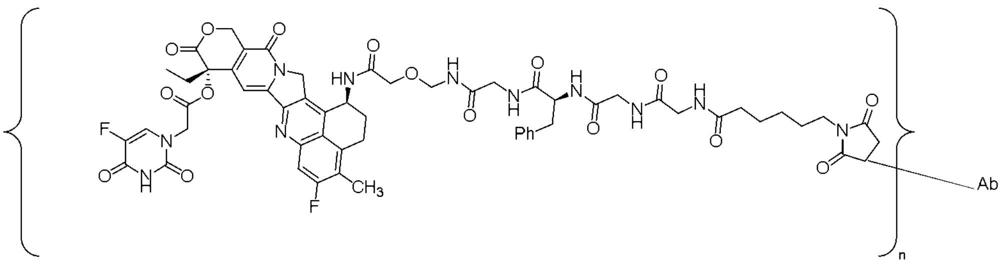

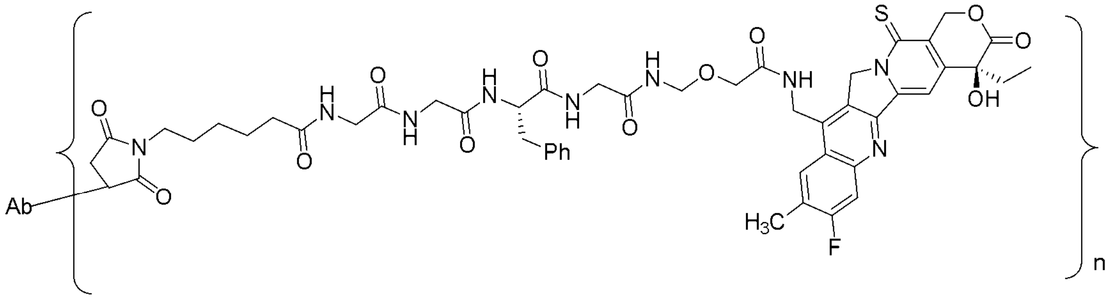

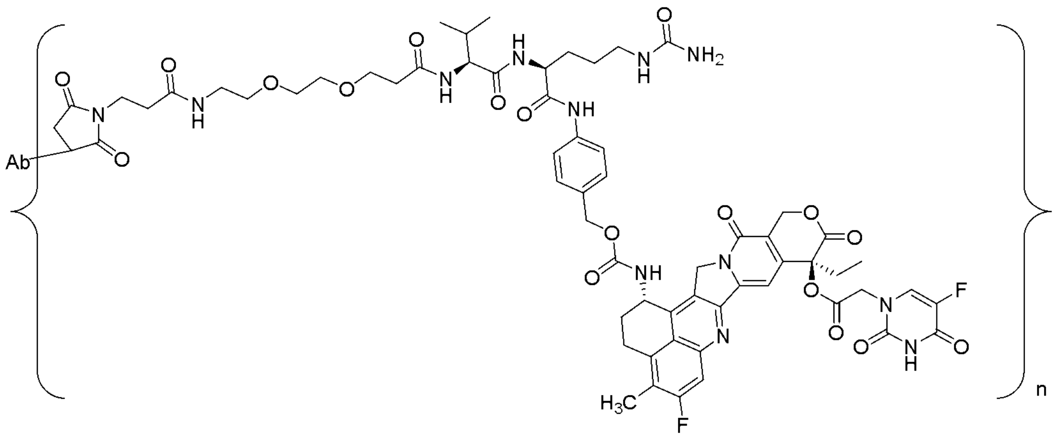

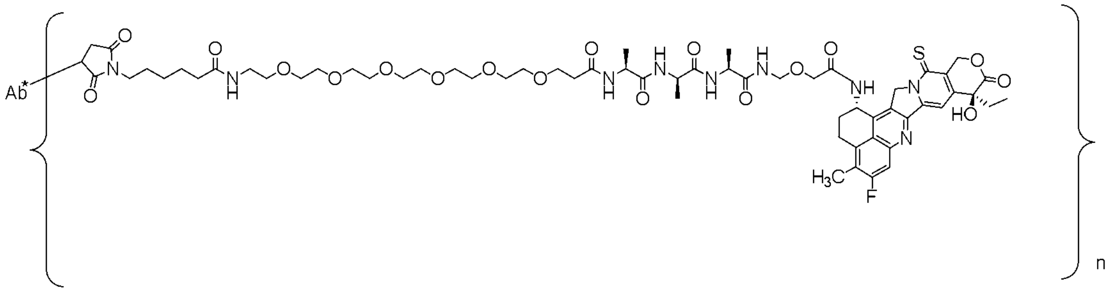

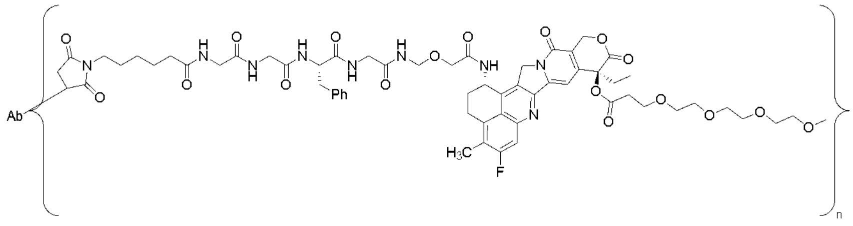

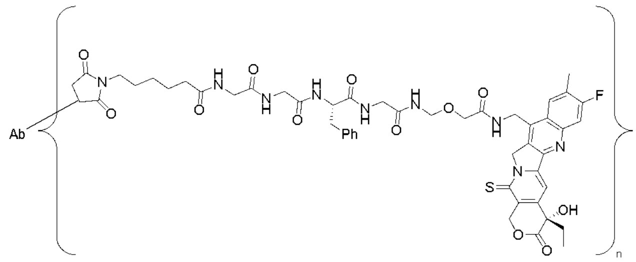

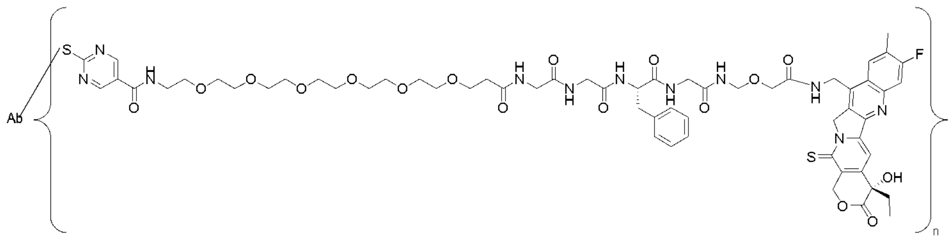

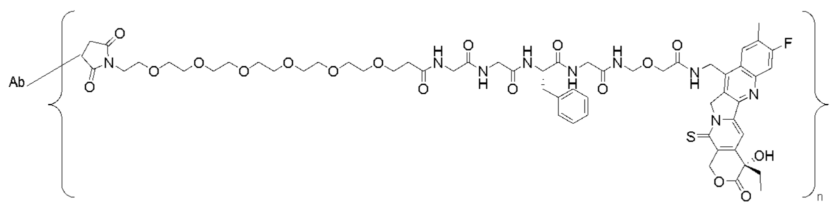

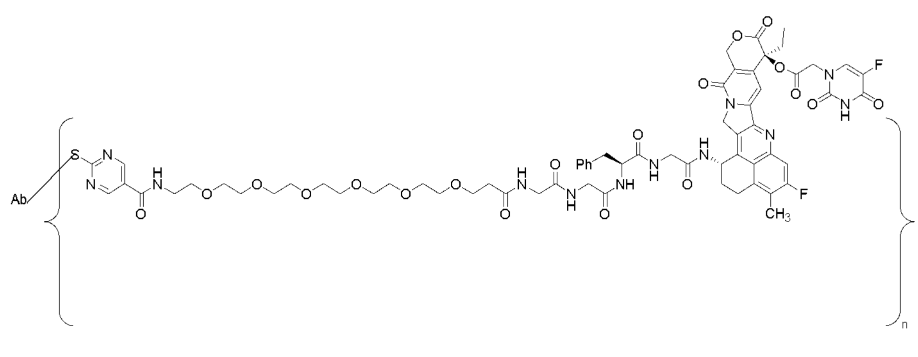

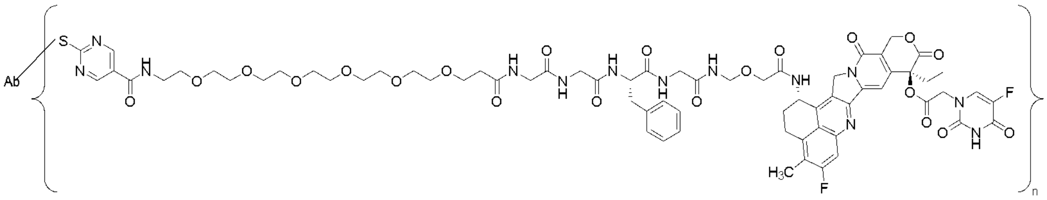

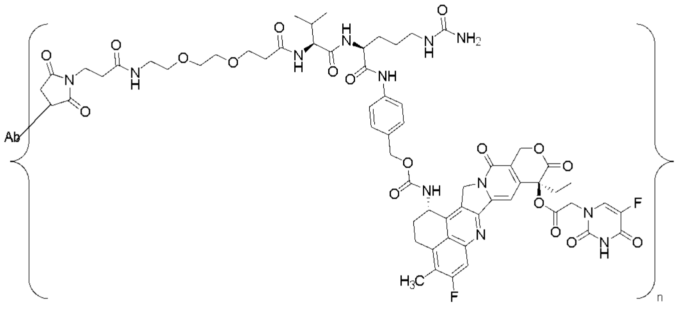

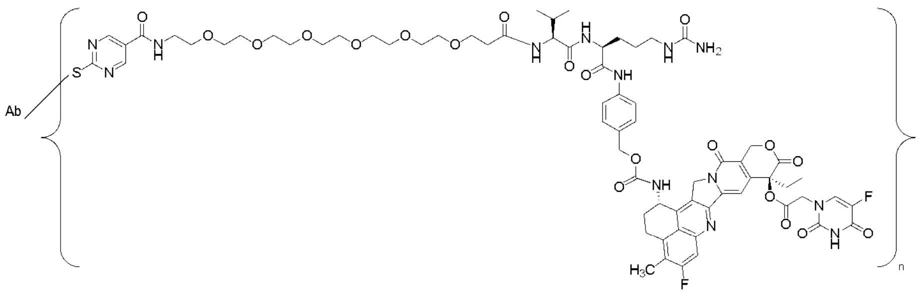

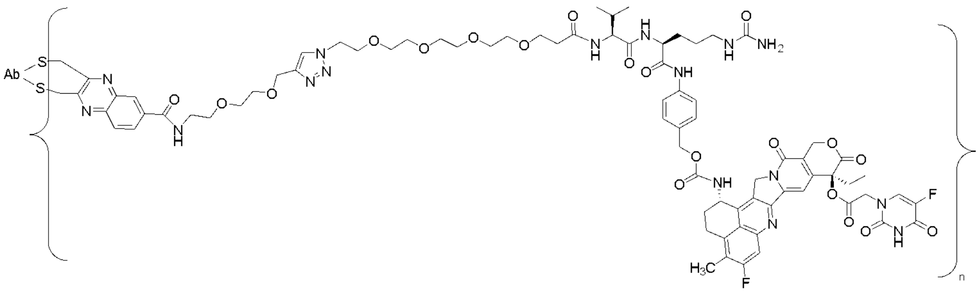

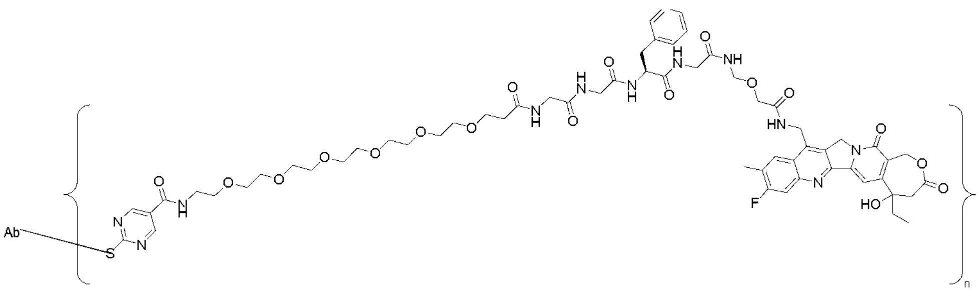

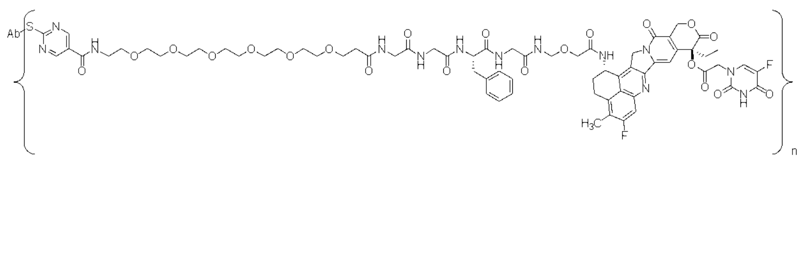

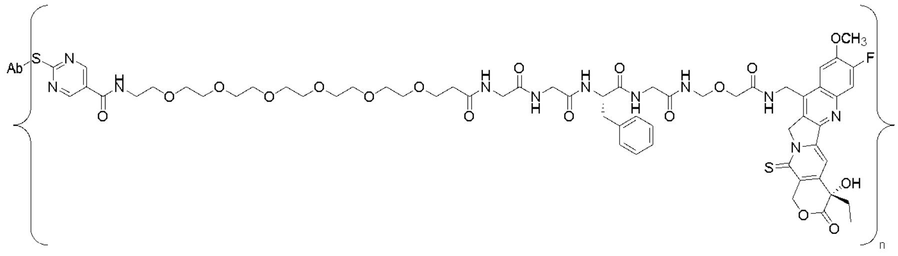

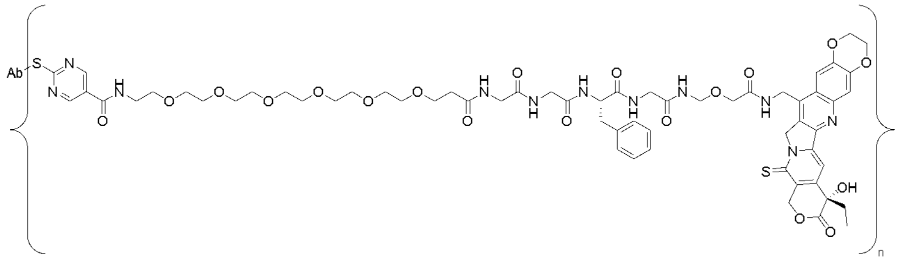

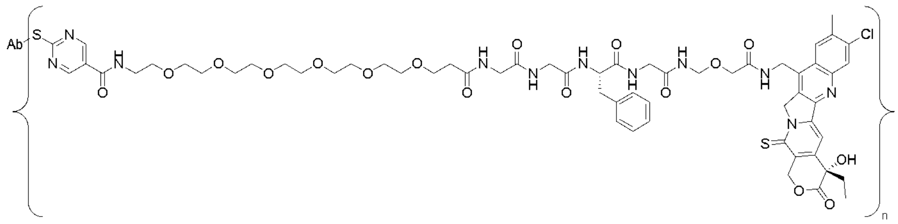

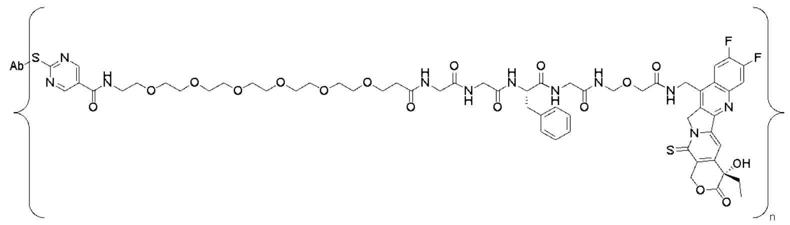

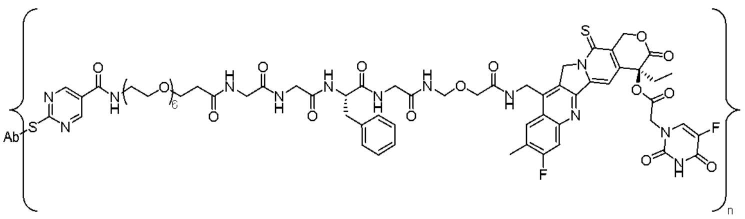

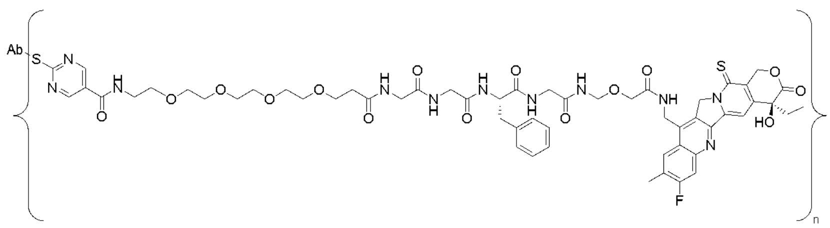

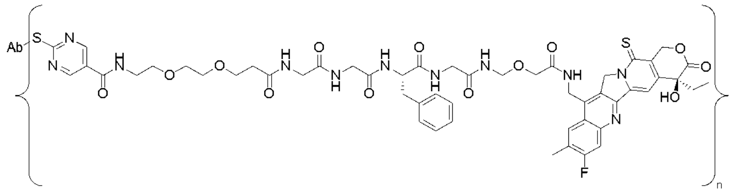

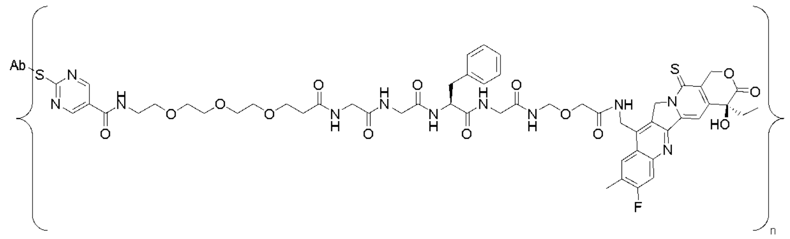

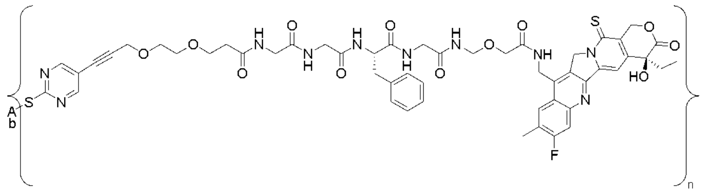

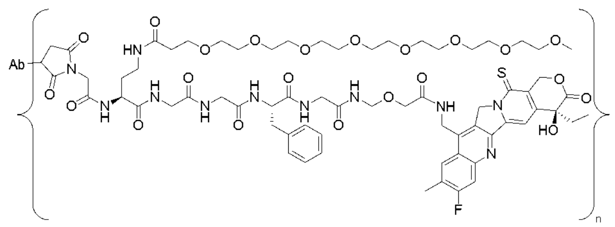

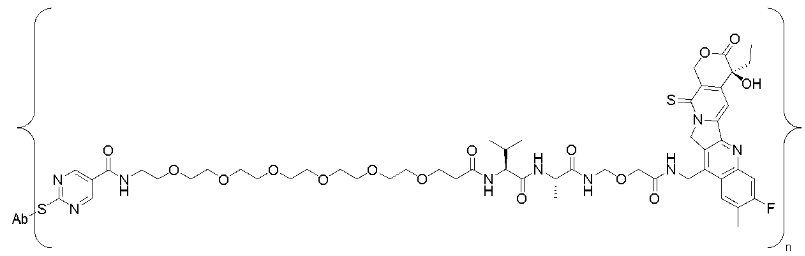

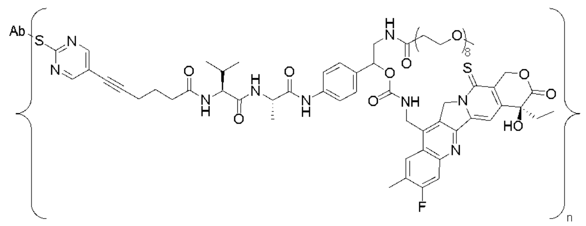

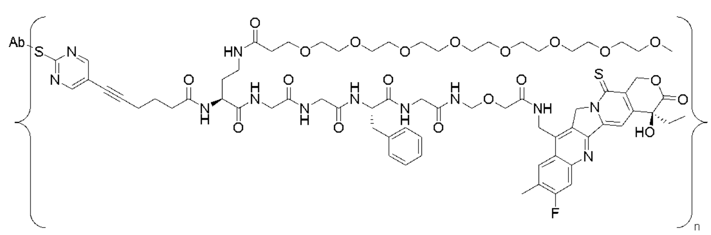

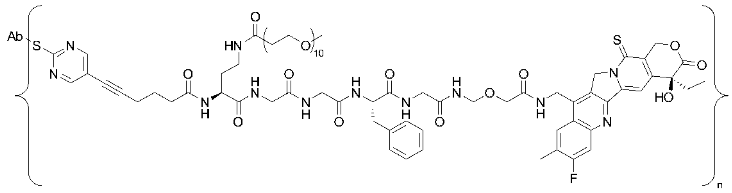

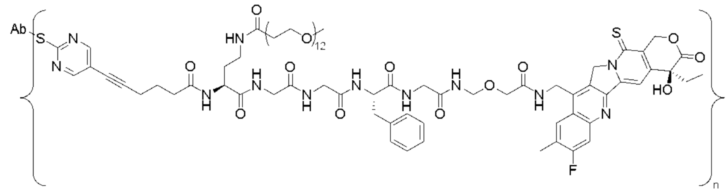

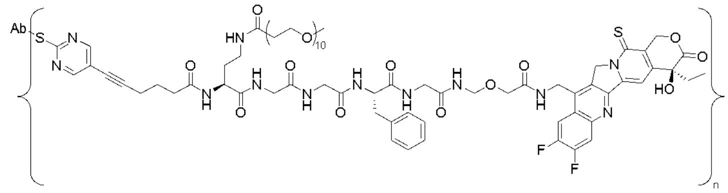

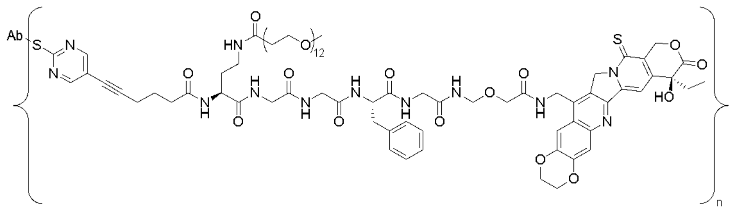

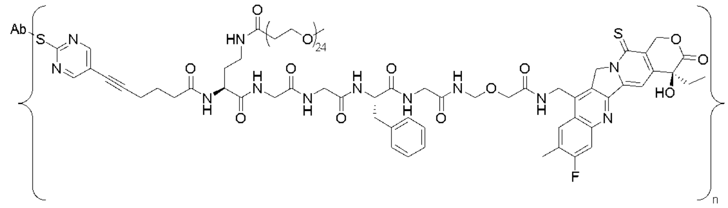

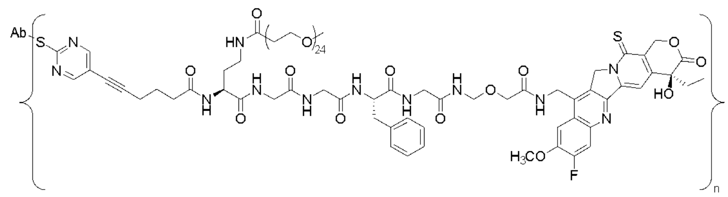

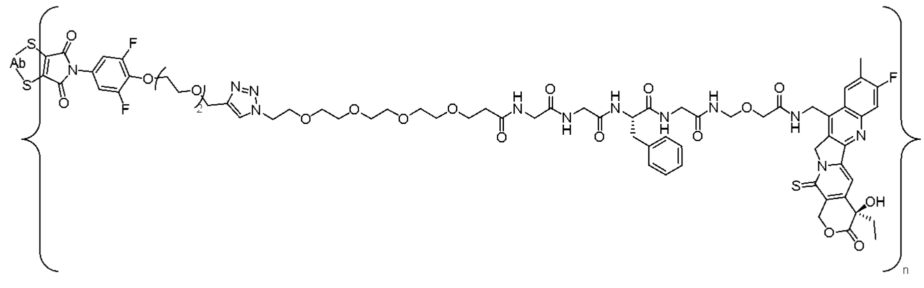

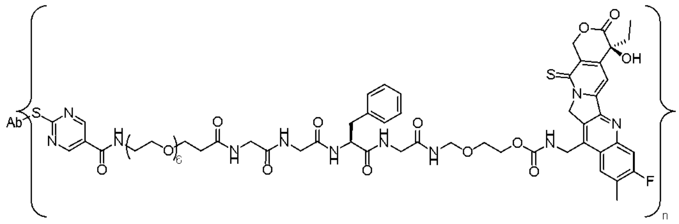

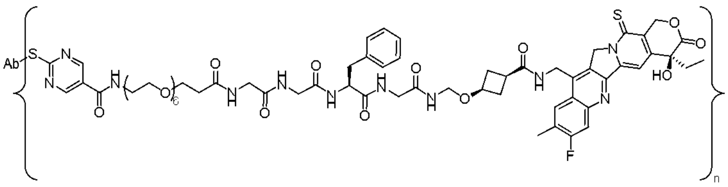

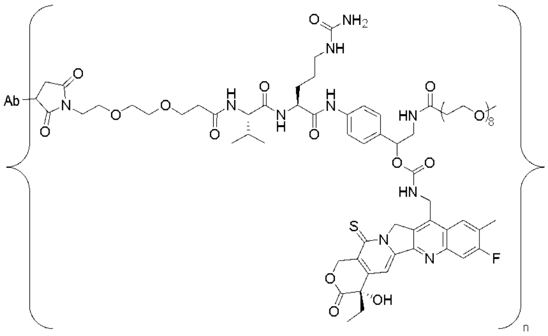

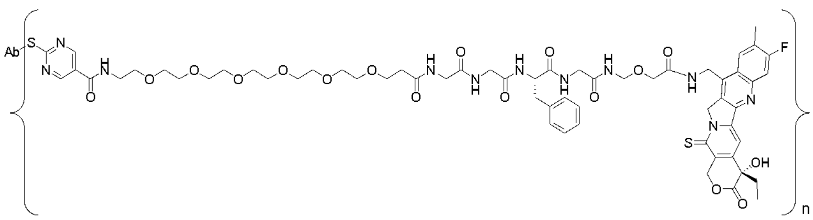

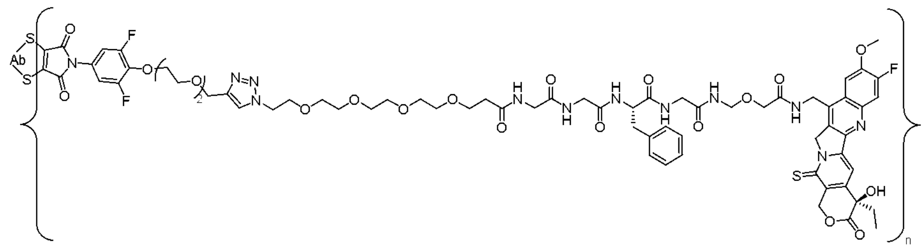

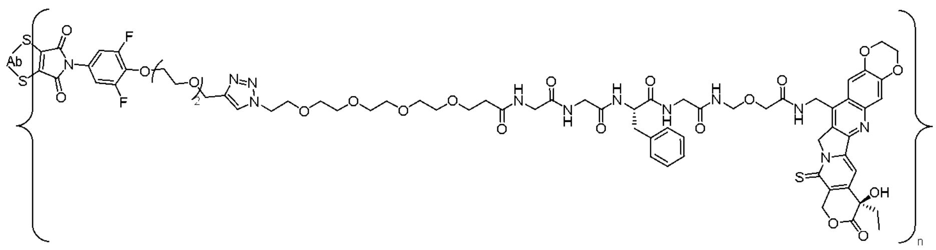

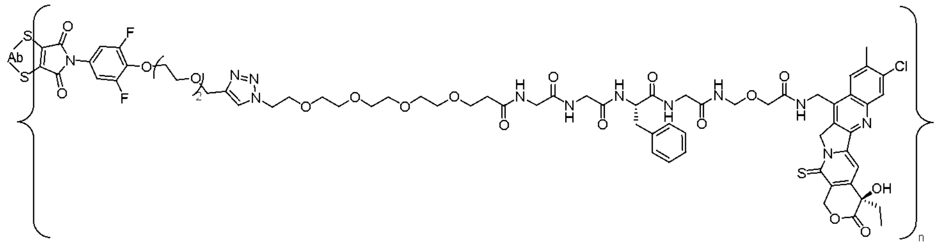

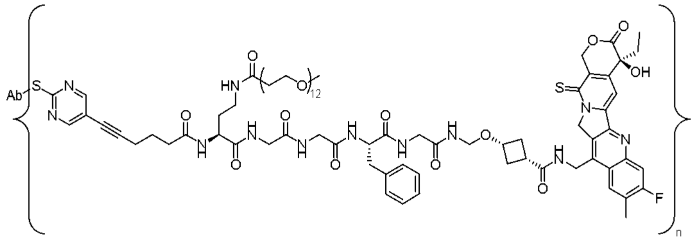

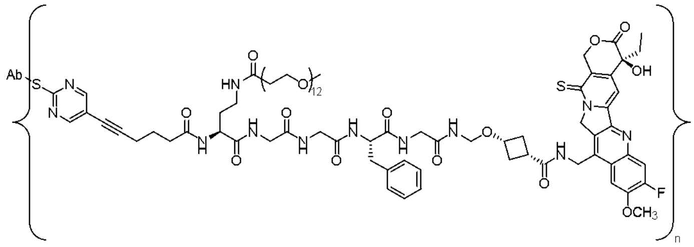

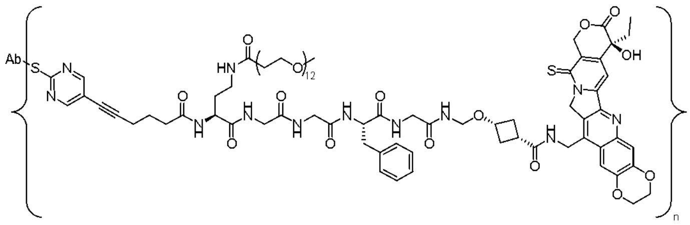

and

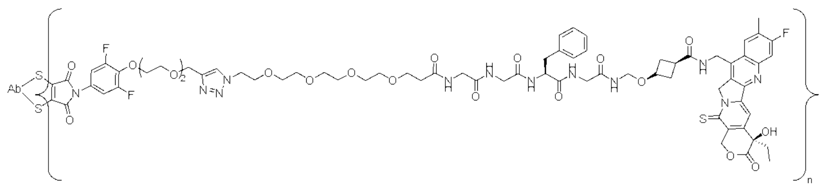

,

wherein, n is an integer or decimal from 1 to 10; preferably, n is an integer or decimal from 3 to 8; Ab is a ligand.

**14.** A compound represented by Formula 5, or a pharmaceutically acceptable salt thereof,

$L_j$- $L_2$-$L_3$ -$L_4$ -D (Formula 5) ;

wherein -$L_2$-, -$L_3$-, -$L_4$- and -D are as defined in any one of claims 8-13;
$L_j$ is selected from

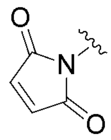, 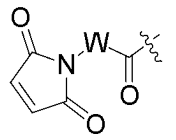, 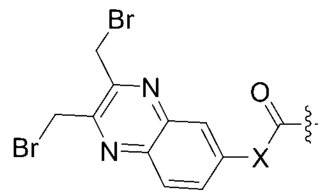,

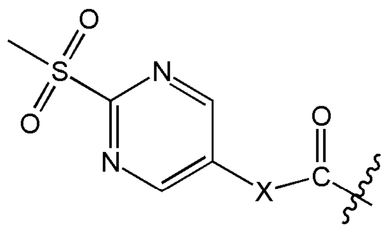, 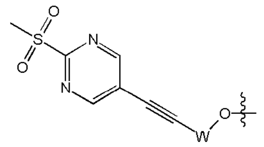, 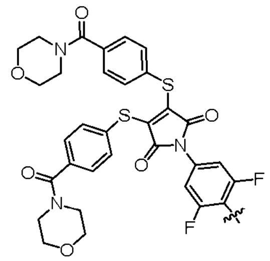,

and

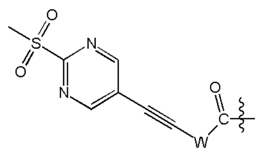;

and W, and X are as defined in any one of claims 8-13.

**15.** The compound according to claim 11, wherein the compound is selected from the following structures:

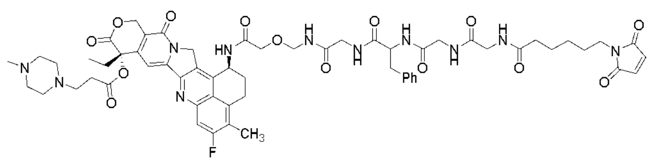

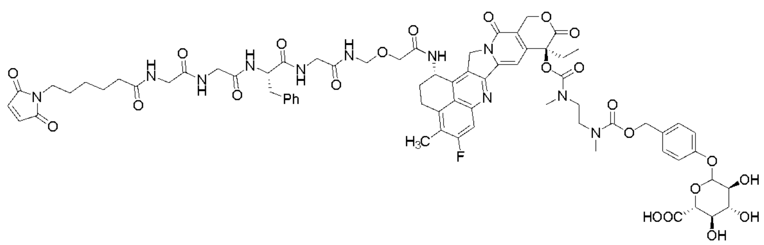

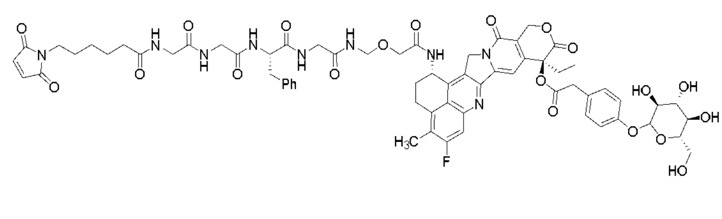

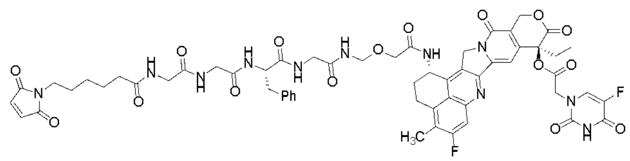

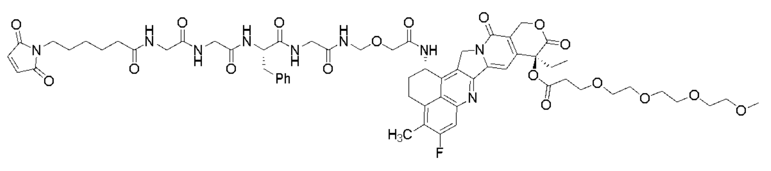

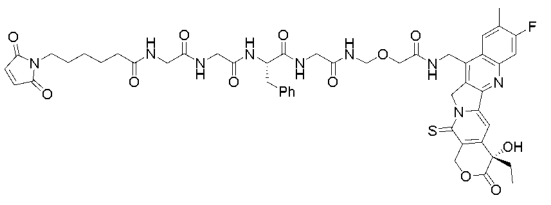

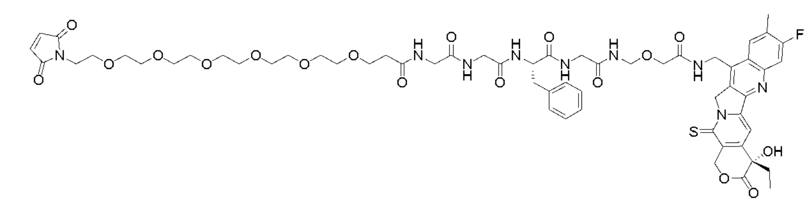

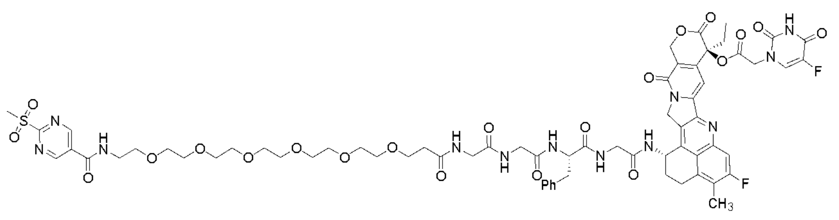

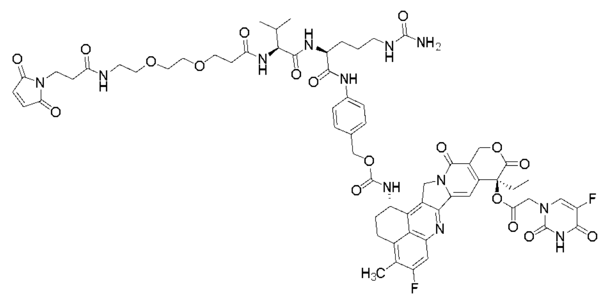

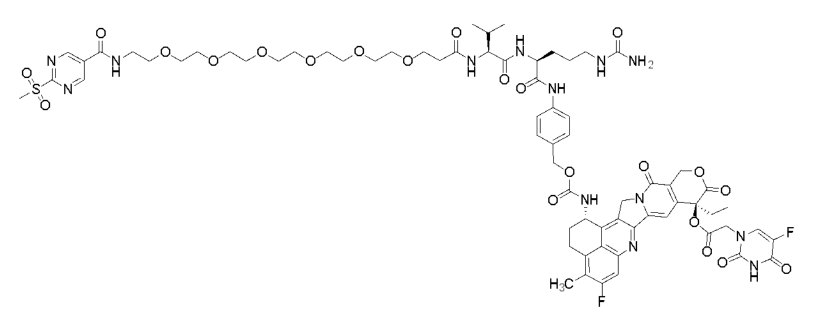

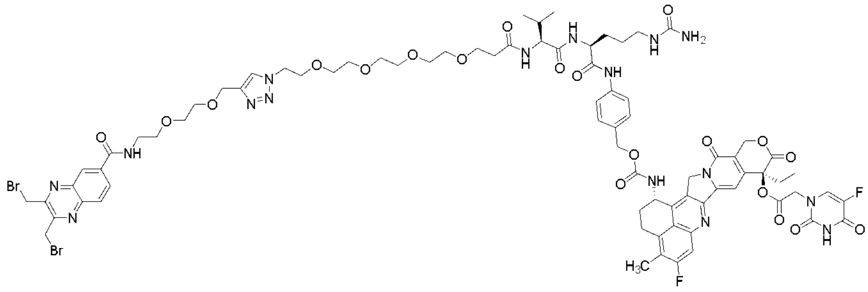

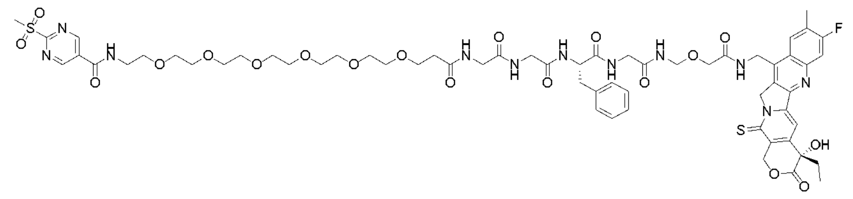

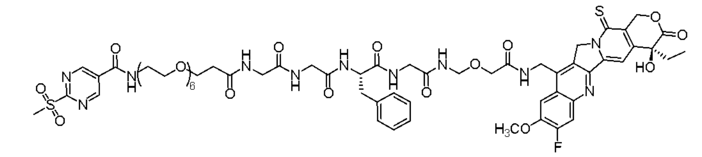

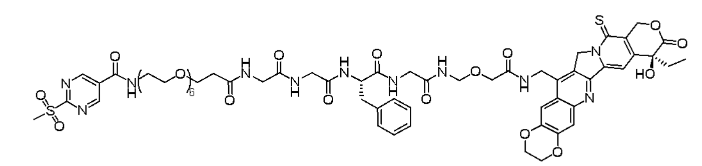

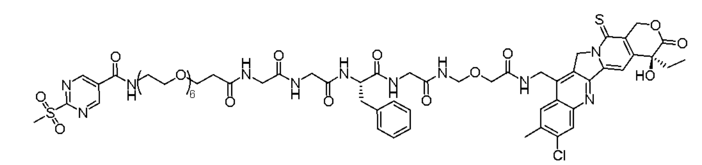

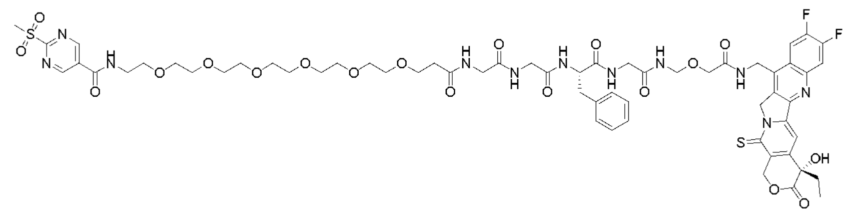

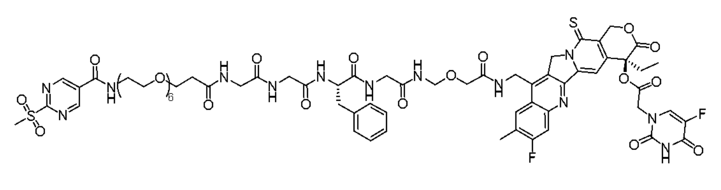

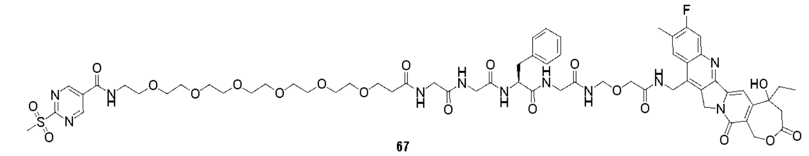

67

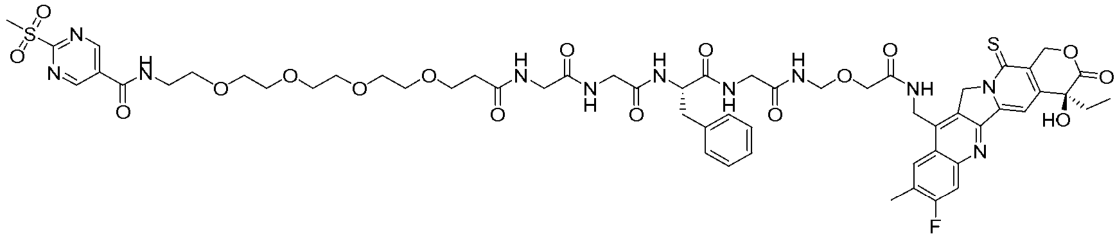

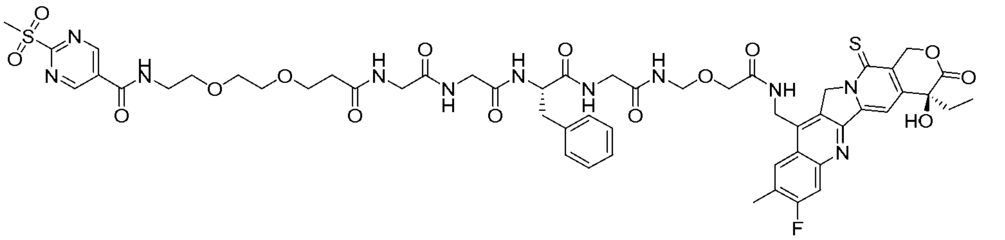

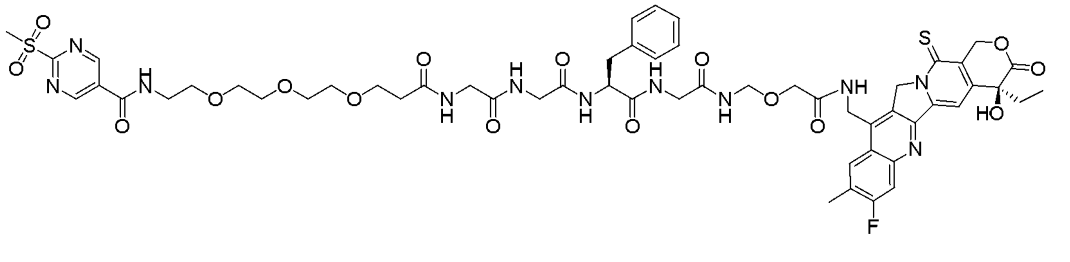

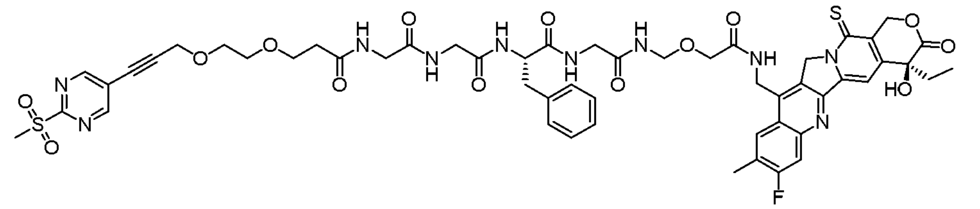

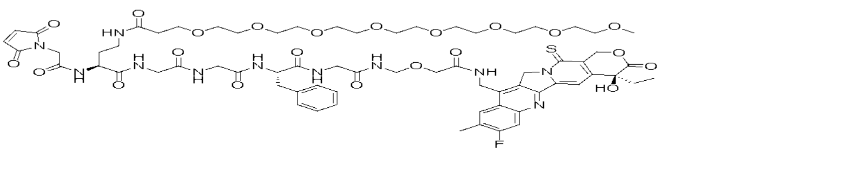

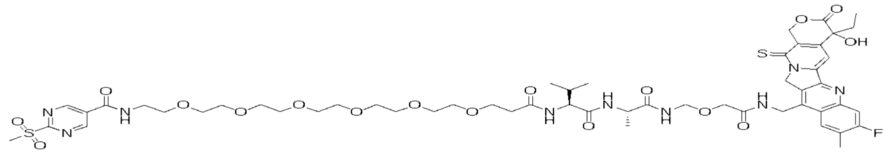

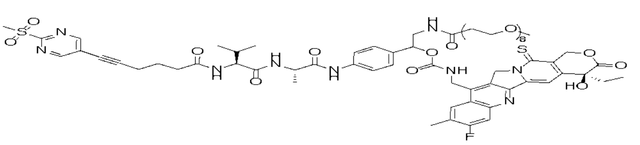

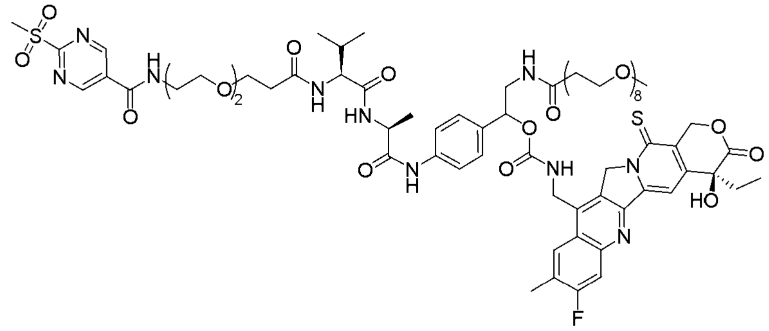

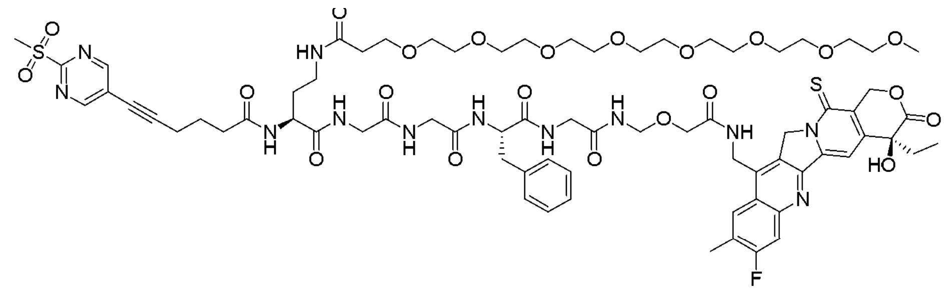

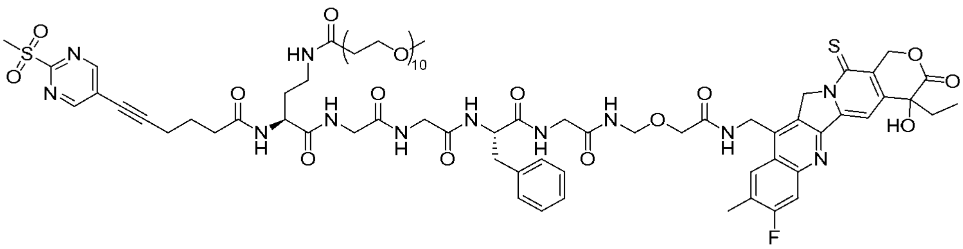

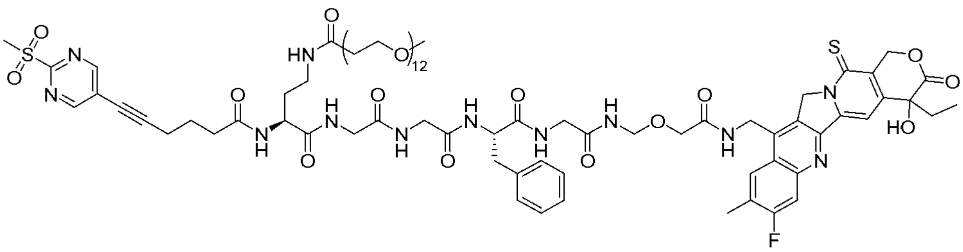

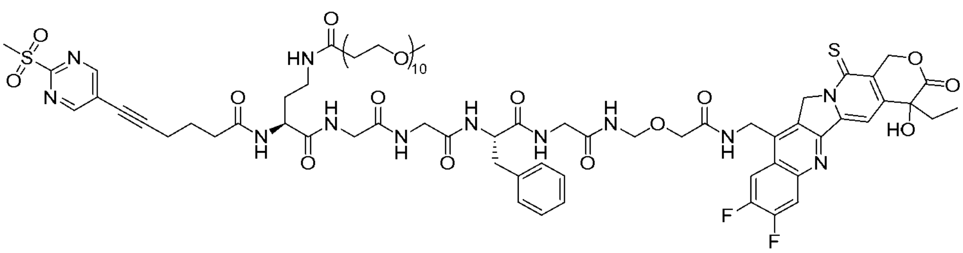

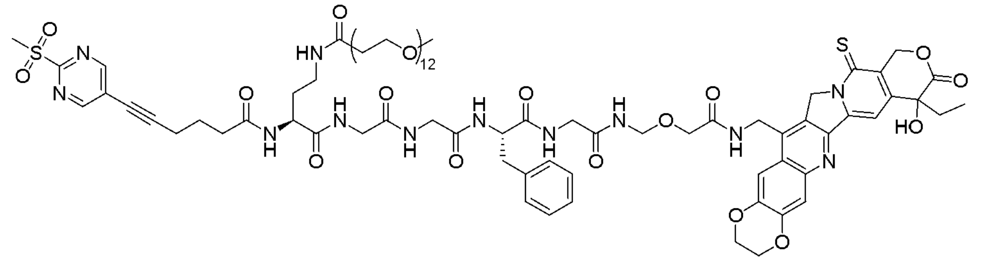

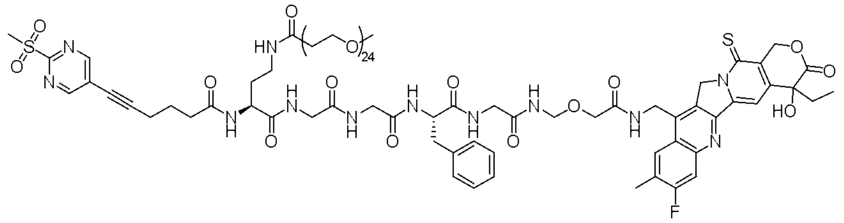

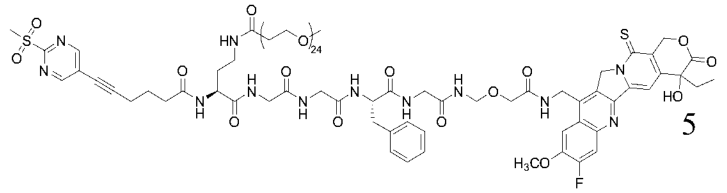

5

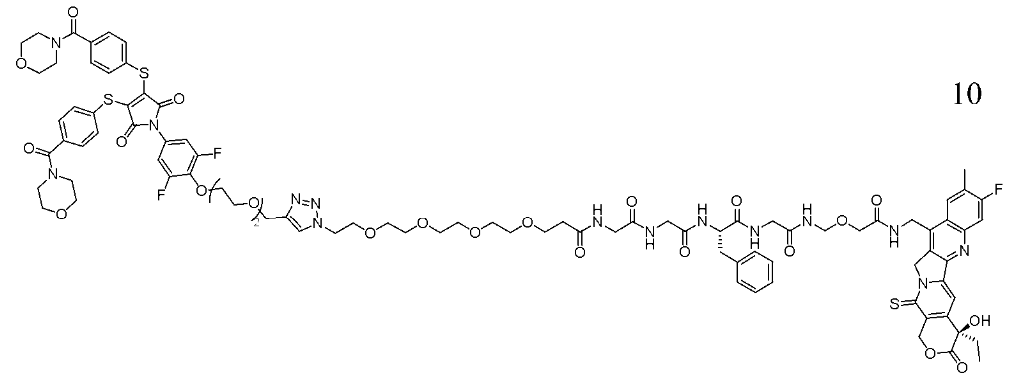

10

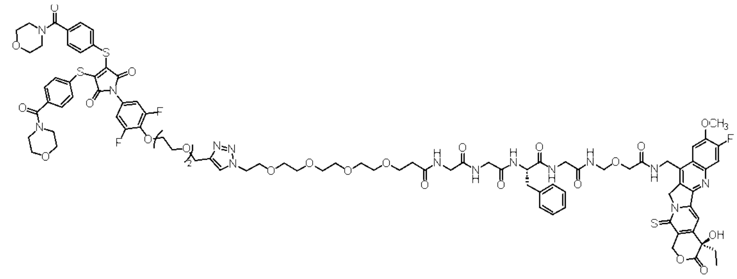

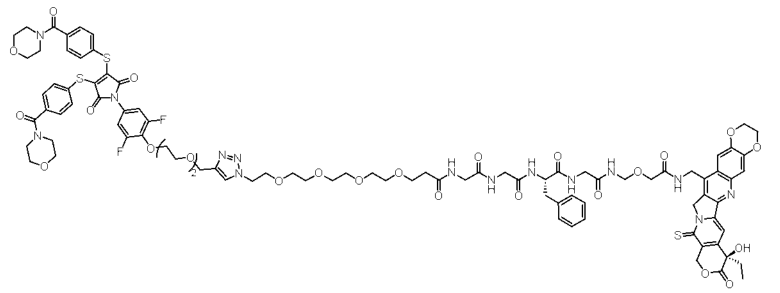

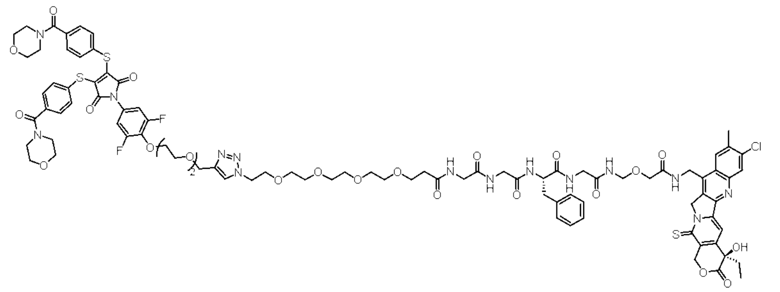

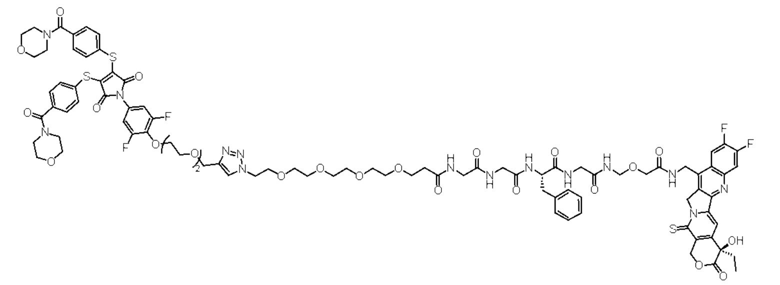

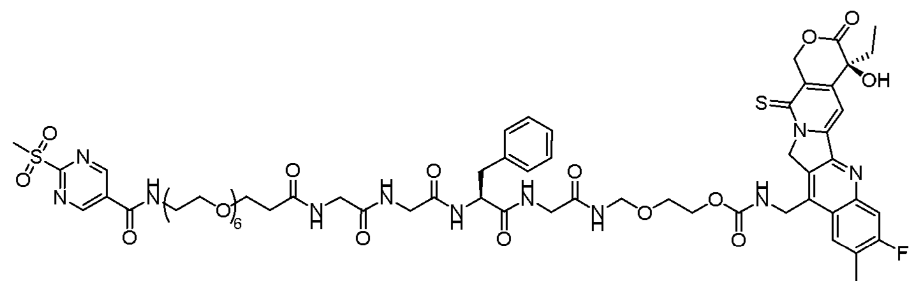

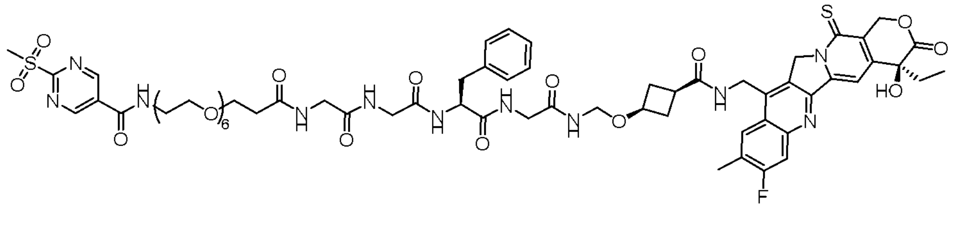

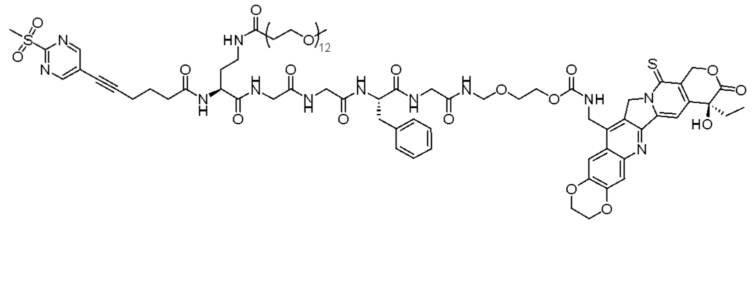

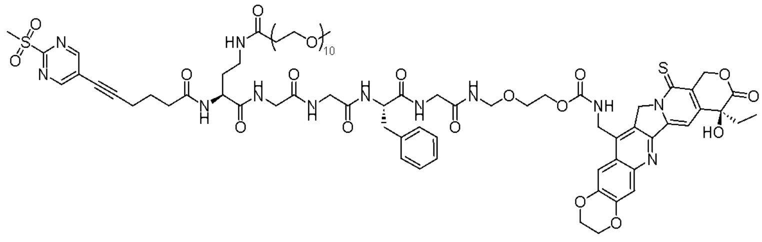

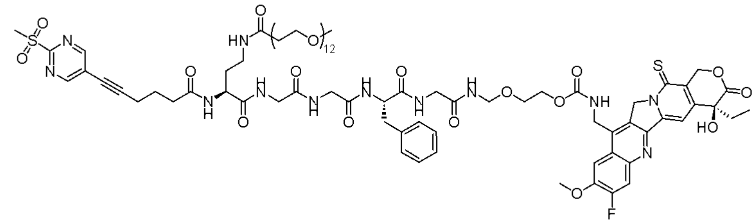

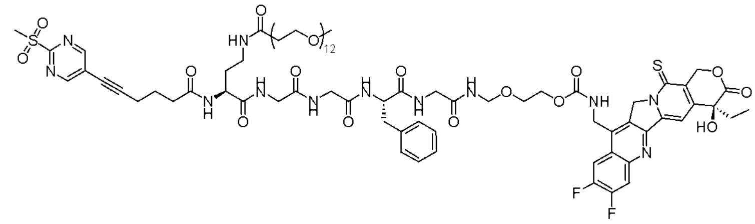

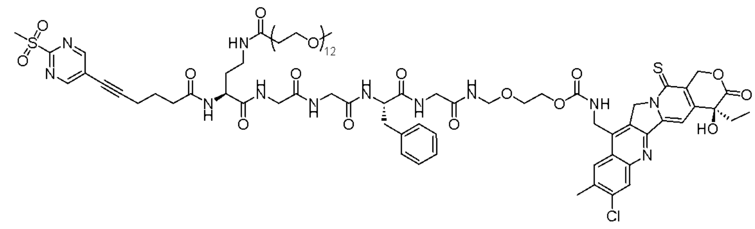

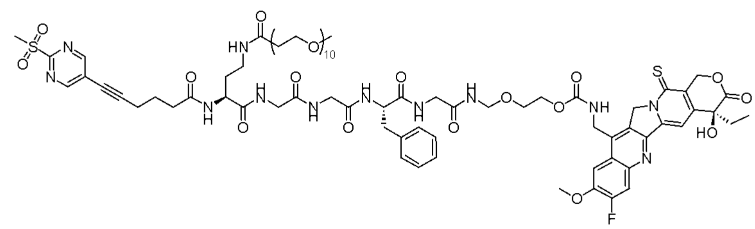

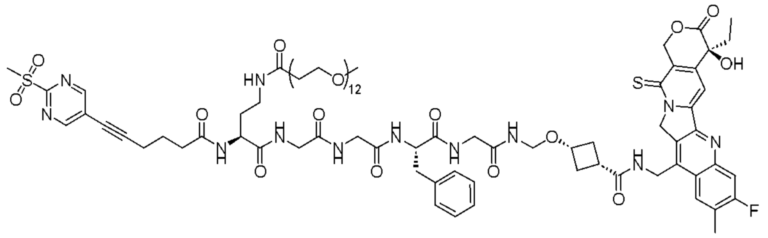

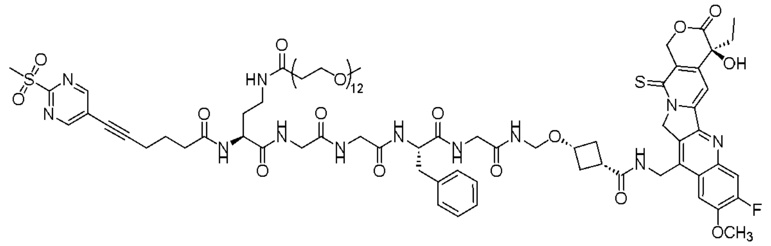

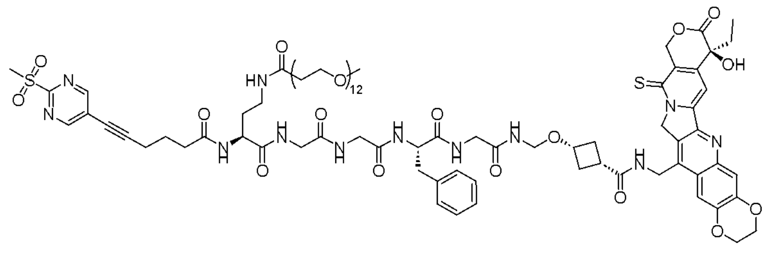

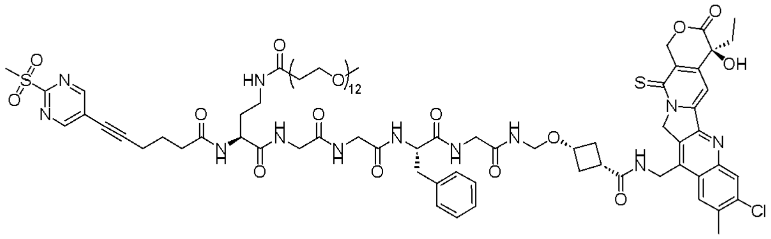

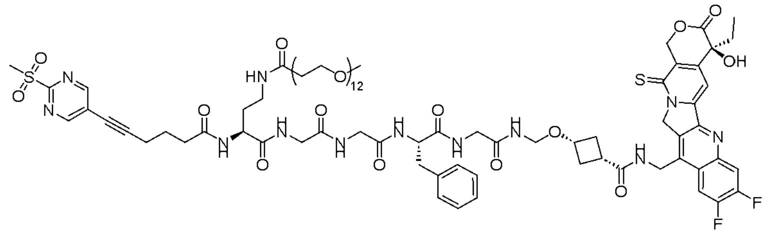

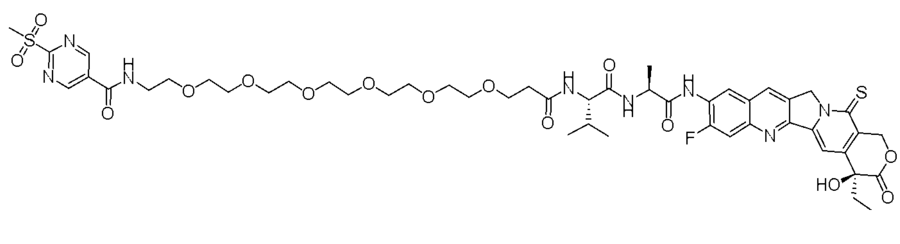

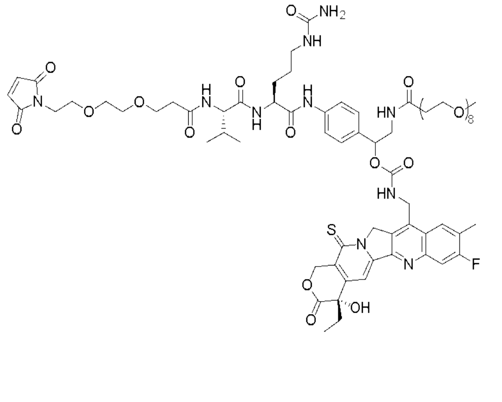

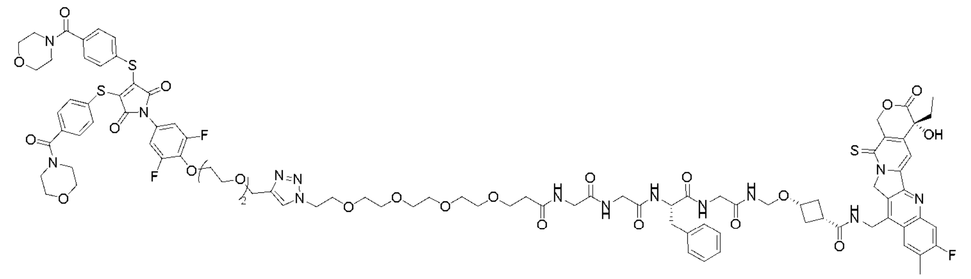

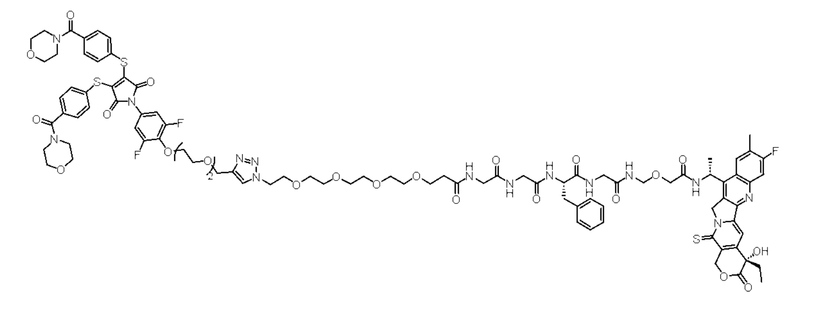

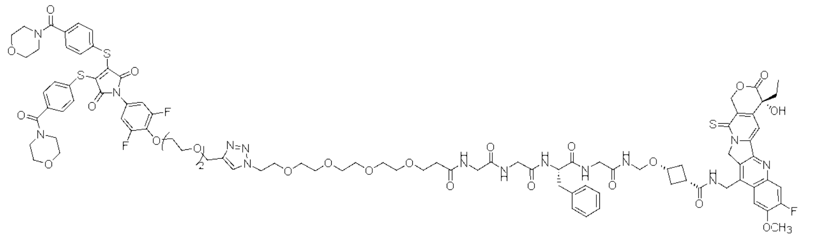

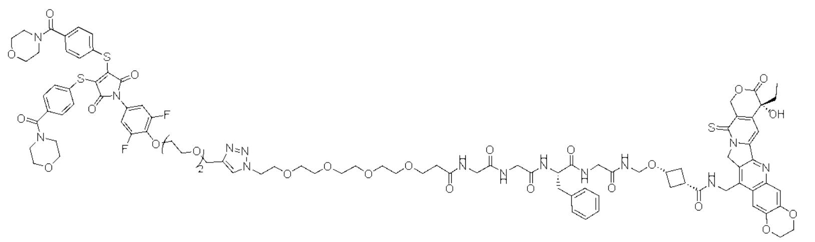

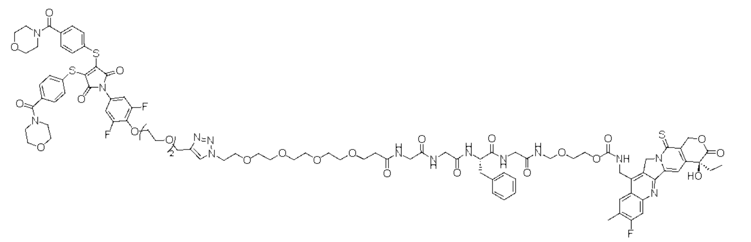

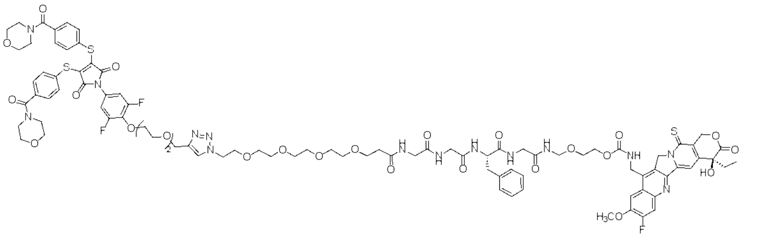

and

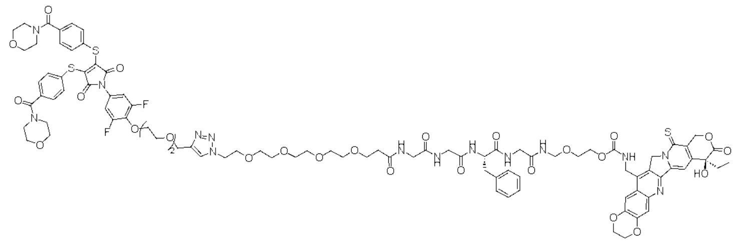
.

**16.** The ligand-drug conjugate or a pharmaceutically acceptable salt thereof according to any one of claims 8-13, wherein the Ab is an antibody or an antigen-binding fragment thereof, or a polypeptide, wherein the antibody is selected from a chimeric antibody, a humanized antibody, and a fully human antibody;

preferably, the antibody or antigen-binding fragment thereof is selected from an anti-TROP-2 antibody, an anti-HER2 (ErbB2) antibody, an anti-NECTIN4 antibody, an anti-EGFR antibody, an anti-B7-H3 antibody, an anti-c-Met antibody, an anti-HER3 (ErbB3) antibody, an anti-HER4 (ErbB4) antibody, an anti-LIV-1 antibody, an anti-ROR1 antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody an anti-A33 antibody, an anti-Cripto antibody, an anti-EphA2 antibody, an anti-G250 antibody, an anti-MUC1 antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-Mesothelin antibody, or an antigen-binding fragment thereof;
preferably, the antibody or antigen-binding fragment thereof is an anti-TROP-2 antibody, anti-NECTIN4 antibody, anti-B7-H3 antibody, anti-HER2 (ErbB2) antibody, anti-HER3 (ErbB3) antibody, anti-LIV-1 antibody, anti-ROR1 antibody, or an antigen-binding fragment thereof;
preferably, the antibody or antigen-binding fragment thereof is an anti-HER2 (ErbB2) antibody, an anti-NECTIN4 antibody, an anti-B7-H3 antibody, or an antigen-binding fragment thereof;
preferably, the antibody or antigen-binding fragment thereof is Trastuzumab, Ifinatamab, or PADCEV.

**17.** A pharmaceutical composition, wherein the pharmaceutical composition comprises a therapeutically effective amount of the compound or the ligand-drug conjugate or a pharmaceutically acceptable salt thereof according to any one of claims 1-16, and a pharmaceutically acceptable carrier.

**18.** Use of the compound or the ligand-drug conjugate or a pharmaceutically acceptable salt thereof according to any one of claims 1-16, or the pharmaceutical composition according to claim 17, in the manufacture of a medicament for treating or preventing a tumor;

preferably, wherein the tumor is a cancer associated with B7-H3 expression, HER2 expression, or NECTIN4 expression;
preferably, the cancer is selected from breast cancer, gastric cancer, melanoma, and lung cancer.

**19.** A method of preventing or treating a tumor, comprising administering to a subject in need thereof an effective amount of the compound or the ligand-drug conjugate or a pharmaceutically acceptable salt thereof according to any one of claims 1-16, or the pharmaceutical composition according to claim 17;

preferably, wherein the tumor is a cancer associated with B7-H3 expression, HER2 expression, or NECTIN4 expression;
preferably, the cancer is selected from breast cancer, gastric cancer, melanoma, and lung cancer.

**20.** The compound or the ligand-drug conjugate or a pharmaceutically acceptable salt thereof according to any one of claims 1-16, or the pharmaceutical composition according to claim 17, for use in the prevention or treatment of a tumor; preferably, wherein the tumor is a cancer associated with B7-H3 expression, HER2 expression, or NECTIN4 expression; preferably, the cancer is selected from breast cancer, gastric cancer, melanoma, and lung cancer.

**21.** Use of the compound according to any one of claims 1-7, **characterized in that** the compound is used as a toxin in an antibody-drug conjugate for the preparation of the antibody-drug conjugate.

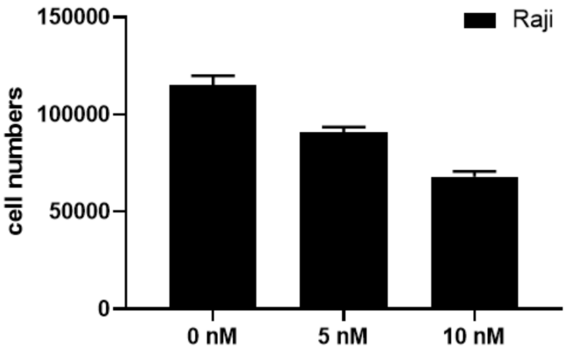


Figure 1

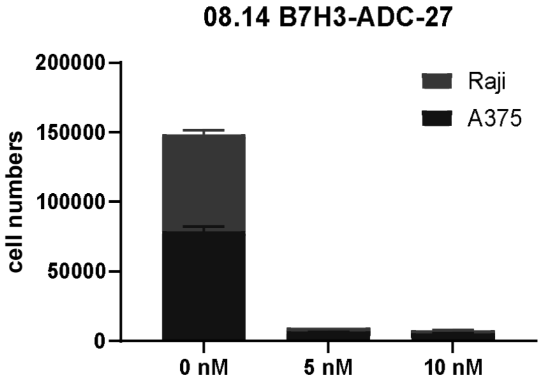


Figure 2

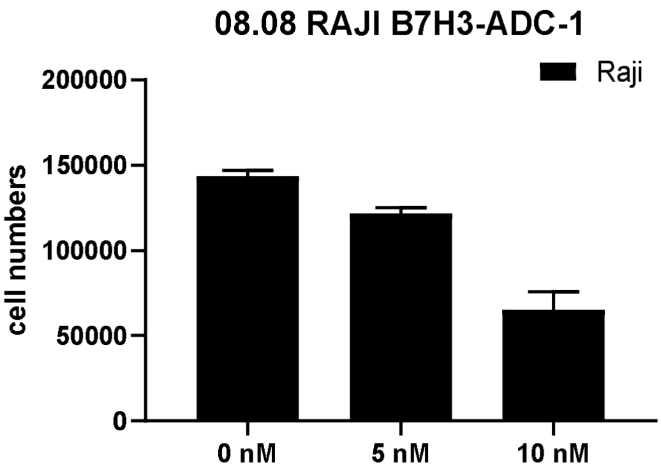


Figure 3

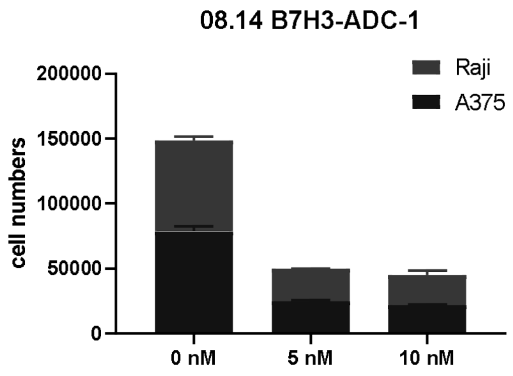


Figure 4

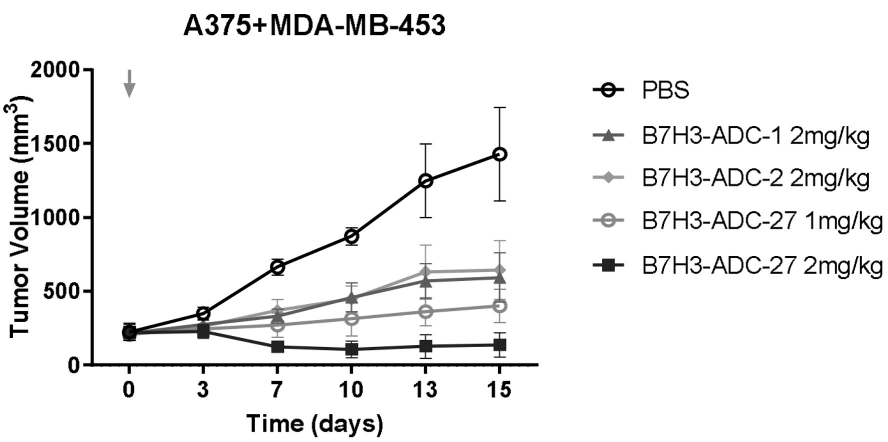


Figure 5

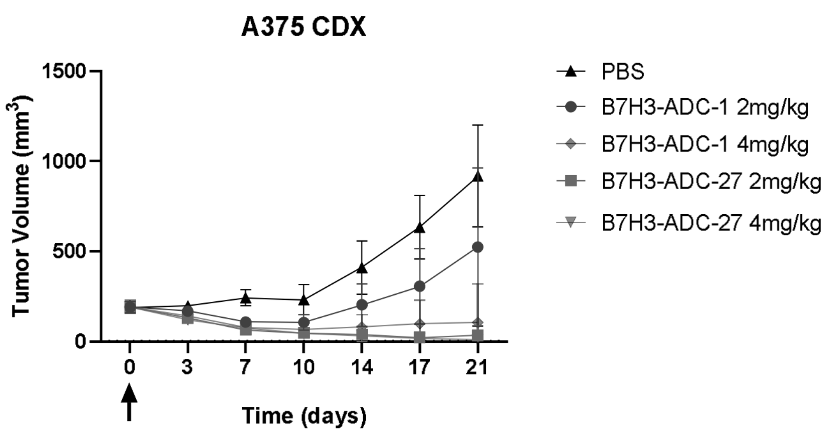


Figure 6

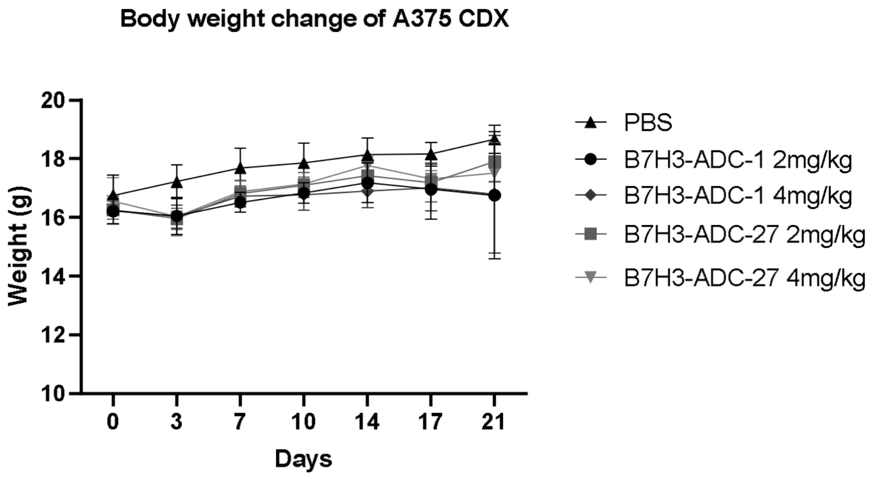


Figure 7

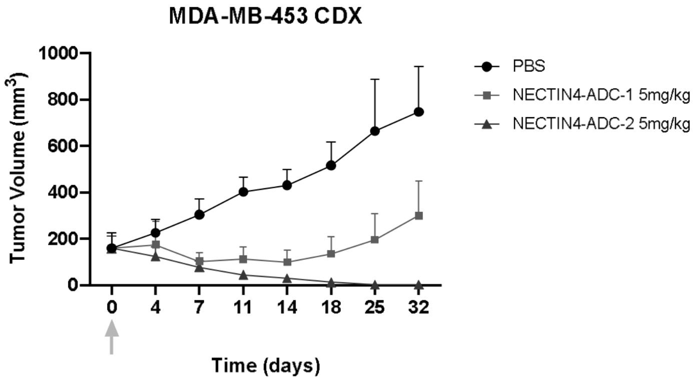


Figure 8

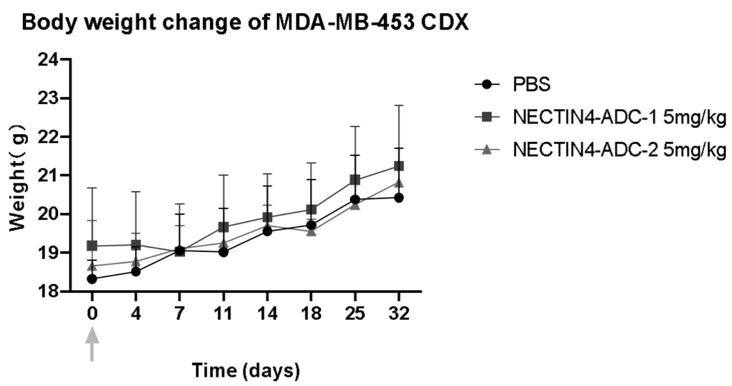


Figure 9

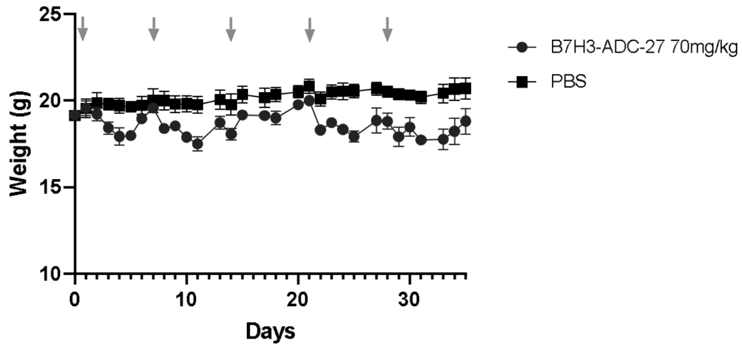


Figure 10

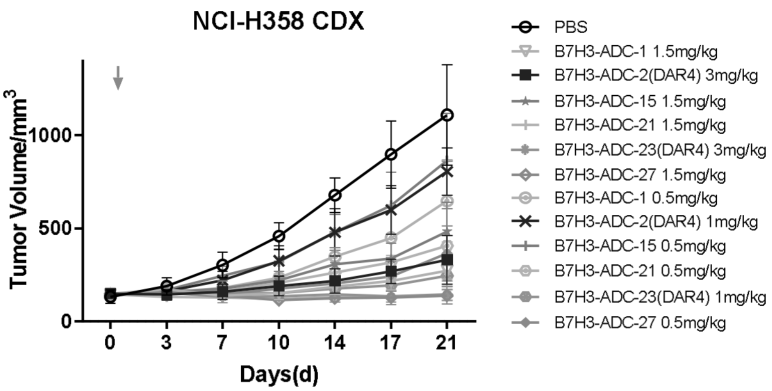


Figure 11

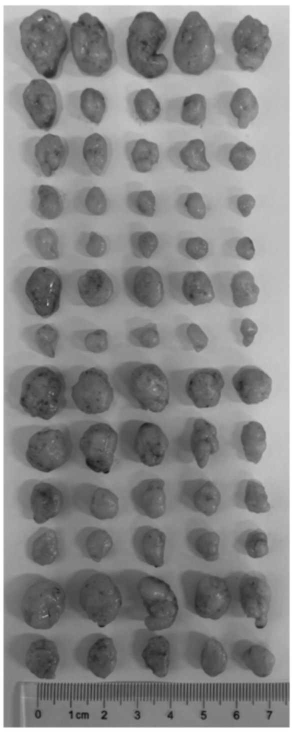

PBS

B7H3-ADC-2 3 mpk

B7H3-ADC-1 1.5 mpk

B7H3-ADC-27 1.5 mpk

B7H3-ADC-23 3 mpk

B7H3-ADC-15 3 mpk

B7H3-ADC-21 3 mpk

B7H3-ADC-2 1 mpk

B7H3-ADC-1 0.5 mpk

B7H3-ADC-27 0.5 mpk

B7H3-ADC-23 1 mpk

B7H3-ADC-15 0.5 mpk

B7H3-ADC-21 0.5 mpk

Figure 12

## INTERNATIONAL SEARCH REPORT

International application No.
**PCT/CN2024/091808**

**A. CLASSIFICATION OF SUBJECT MATTER**

C07D491/22(2006.01)i； A61K31/4745(2006.01)n； A61P35/00(2006.01)n

According to International Patent Classification (IPC) or to both national classification and IPC

**B. FIELDS SEARCHED**

Minimum documentation searched (classification system followed by classification symbols)

IPC： C07D, A61K, A61P

Documentation searched other than minimum documentation to the extent that such documents are included in the fields searched

Electronic data base consulted during the international search (name of data base and, where practicable, search terms used)

CNTXT, CNKI, ENTXTC, VEN, REGISTRY, CAPLUS: 喜树碱, 依喜替康, 伊立替康, 偶联, 配体, 肿瘤, camptothecin, exatecan, conjugate, lignad, antitumor

**C. DOCUMENTS CONSIDERED TO BE RELEVANT**

| Category* | Citation of document, with indication, where appropriate, of the relevant passages | Relevant to claim No. |
|---|---|---|
| X | CN 101979392 A (SECOND MILITARY MEDICAL UNIVERSITY OF PLA) 23 February 2011 (2011-02-23)<br>description, paragraphs 41 and 60-61 | 1-2, 4, 6 |
| X | CN 1241191 A (SOCIETE DE CONSEILS DE RECHERCHES ET D'APPLICATIONS SCIENTIFIQUES) 12 January 2000 (2000-01-12)<br>claims 1-4 and 6-8 | 1-2, 4, 6, 17-18 |
| X | US 2010120816 A1 (INDENA SPA) 13 May 2010 (2010-05-13)<br>description, pages 4-5, and tables | 1-2, 4, 17-18 |
| X | WO 2023030364 A1 (SHANGHAI BEST LINK BIOSCIENCE LLC) 09 March 2023 (2023-03-09)<br>claims 1-17 | 1-7, 17-18, 21 |
| X | WO 2020063676 A1 (JIANGSU HENGRUI MEDICINE CO., LTD. et al.) 02 April 2020 (2020-04-02)<br>claims 1-46 | 1-18, 21 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 September 2024** | **04 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/091808**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/091808**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19-20**
because they relate to subject matter not required to be searched by this Authority, namely:

Claims 19-20 relate to a disease prevention or treatment method, which falls within subject matter for which no search is required as defined in PCT Rule 39(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/091808**

| Patent document cited in search report | Publication date (day/month/year) | Patent family member(s) | Publication date (day/month/year) |
|---|---|---|---|
| CN 101979392 A | 23 February 2011 | None | |
| CN 1241191 A | 12 January 2000 | FR 2757514 A1 | 26 June 1998 |
| | | FR 2757514 B1 | 12 February 1999 |
| | | PT 946567 E | 30 January 2004 |
| | | CA 2275351 A1 | 02 July 1998 |
| | | CA 2275351 C | 14 October 2008 |
| | | HUP 9904210 A2 | 28 May 2000 |
| | | HUP 9904210 A3 | 28 September 2000 |
| | | BR 9714170 A | 29 February 2000 |
| | | CZ 220599 A3 | 15 September 1999 |
| | | CZ 299795 B6 | 26 November 2008 |
| | | NZ 335939 A | 28 April 2000 |
| | | WO 9828305 A1 | 02 July 1998 |
| | | AU 5326598 A | 17 July 1998 |
| | | AU 734485 B2 | 14 June 2001 |
| | | HK 1024695 A1 | 20 October 2000 |
| | | MY 119473 A | 31 May 2005 |
| | | ES 2206761 T3 | 16 May 2004 |
| | | NO 992998 D0 | 18 June 1999 |
| | | NO 992998 L | 18 August 1999 |
| | | NO 326464 B1 | 08 December 2008 |
| | | ZA 9711129 B | 03 May 1999 |
| | | EP 0946567 A1 | 06 October 1999 |
| | | EP 0946567 B1 | 27 August 2003 |
| | | JP 2001505922 A | 08 May 2001 |
| | | JP 3576175 B2 | 13 October 2004 |
| | | DK 0946567 T3 | 22 December 2003 |
| | | KR 20000069608 A | 25 November 2000 |
| | | KR 100516874 B1 | 26 September 2005 |
| | | PL 334125 A1 | 14 February 2000 |
| | | PL 188181 B1 | 31 December 2004 |
| | | RU 2194051 C2 | 10 December 2002 |
| | | ATE 248177 T1 | 15 September 2003 |
| | | DE 69724453 D1 | 02 October 2003 |
| | | DE 69724453 T2 | 09 June 2004 |
| | | JP 2004115535 A | 15 April 2004 |
| | | AR 008543 A1 | 19 January 2000 |
| | | UA 57758 C2 | 15 July 2003 |
| | | MXPA 99005776 A | 31 August 2004 |
| US 2010120816 A1 | 13 May 2010 | IL 196653 A0 | 18 November 2009 |
| | | IL 196653 A | 30 April 2015 |
| | | MX 2009000827 A | 03 February 2009 |
| | | KR 20090033456 A | 03 April 2009 |
| | | KR 101412157 B1 | 25 June 2014 |
| | | PT 2044079 E | 09 March 2016 |
| | | RU 2009102243 A | 27 July 2010 |
| | | RU 2450008 C2 | 10 May 2012 |
| | | SI 2044079 T1 | 29 February 2016 |
| | | HK 1135691 A1 | 11 June 2010 |
| | | CA 2658900 A1 | 31 January 2008 |

Form PCT/ISA/210 (patent family annex) (July 2022)

**INTERNATIONAL SEARCH REPORT**
**Information on patent family members**

International application No.
**PCT/CN2024/091808**

| Patent document cited in search report | | | Publication date (day/month/year) | Patent family member(s) | | | Publication date (day/month/year) |
|---|---|---|---|---|---|---|---|
| | | | | CA | 2658900 | C | 15 September 2015 |
| | | | | PL | 2044079 | T3 | 30 June 2016 |
| | | | | ITMI | 20061473 | A1 | 27 January 2008 |
| | | | | DK | 2044079 | T3 | 29 February 2016 |
| | | | | US | 8217053 | B2 | 10 July 2012 |
| | | | | WO | 2008011994 | A1 | 31 January 2008 |
| | | | | JP | 2009544634 | A | 17 December 2009 |
| | | | | JP | 5313894 | B2 | 09 October 2013 |
| | | | | NO | 20090251 | L | 16 January 2009 |
| | | | | NO | 341812 | B1 | 29 January 2018 |
| | | | | EP | 2044079 | A1 | 08 April 2009 |
| | | | | EP | 2044079 | B1 | 09 December 2015 |
| | | | | ES | 2561356 | T3 | 25 February 2016 |
| | | | | BRPI | 0714553 | A2 | 26 March 2013 |
| | | | | BRPI | 0714553 | B1 | 17 November 2020 |
| | | | | BRPI | 0714553 | B8 | 25 May 2021 |
| | | | | AU | 2007278500 | A1 | 31 January 2008 |
| | | | | AU | 2007278500 | A2 | 09 September 2010 |
| | | | | AU | 2007278500 | B2 | 07 June 2012 |
| | | | | HUE | 027890 | T2 | 28 November 2016 |
| WO | 2023030364 | A1 | 09 March 2023 | None | | | |
| WO | 2020063676 | A1 | 02 April 2020 | ZA | 202102544 | B | 25 October 2023 |
| | | | | CA | 3114137 | A1 | 02 April 2020 |
| | | | | BR | 112021004656 | A2 | 01 June 2021 |
| | | | | JP | 2022511351 | A | 31 January 2022 |
| | | | | EP | 3858386 | A1 | 04 August 2021 |
| | | | | EP | 3858386 | A4 | 12 October 2022 |
| | | | | KR | 20210066833 | A | 07 June 2021 |
| | | | | MX | 2021003382 | A | 27 May 2021 |
| | | | | US | 2021353764 | A1 | 18 November 2021 |
| | | | | AU | 2019349207 | A1 | 08 April 2021 |
| | | | | TW | 202027795 | A | 01 August 2020 |
| | | | | TWI | 826543 | B | 21 December 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2907824 B1

### Non-patent literature cited in the description


- **H. BUNDGAARD**. Design of Prodrugs. Elsevier, 1985
- *J. Biol. Chem.*, 1968, vol. 243, 3558
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol*, 2001
- **KABAT E.A. et al.** Sequences of proteins of immunological interest.. NIH Publication, 1991, 91-3242